# EUROPEAN PATENT APPLICATION

(11) **EP 4 800 018 A1**
(43) Date of publication of application: **02.09.2026**
(21) Application number: 24881790.0
(22) Date of filing: 28.10.2024
(51) Int. Cl.: C07D 513/22, A61K 31/33, A61P 35/00

(54) **PAN-KRAS INHIBITOR AND USE THEREOF IN MEDICINES**

(30) Priority: 27.10.2023 CN 202311405853; 20.11.2023 CN 202311543631; 29.12.2023 CN 202311863699; 10.01.2024 CN 202410034527; 01.02.2024 CN 202410145027; 18.04.2024 CN 202410468223; 22.05.2024 CN 202410644373; 11.06.2024 CN 202410748871; 21.10.2024 CN 202411471487
(71) Applicant: Betta Pharmaceuticals Co., Ltd., Hangzhou, Zhejiang 311100 (CN)
(72) Inventor: WU, Wenmao, Hangzhou, Zhejiang 311100 (CN); DING, Lieming, Hangzhou, Zhejiang 311100 (CN); LI, Shusen, Hangzhou, Zhejiang 311100 (CN); ZHAO, Zhichang, Hangzhou, Zhejiang 311100 (CN); ZHANG, Zhan, Hangzhou, Zhejiang 311100 (CN); WANG, Wei, Hangzhou, Zhejiang 311100 (CN); LI, Boyan, Hangzhou, Zhejiang 311100 (CN); TIAN, Kai, Hangzhou, Zhejiang 311100 (CN); ZHU, Xiaoguan, Hangzhou, Zhejiang 311100 (CN); YANG, Xiang, Hangzhou, Zhejiang 311100 (CN); ZHOU, Yuzhen, Hangzhou, Zhejiang 311100 (CN); WU, Hao, Hangzhou, Zhejiang 311100 (CN); WANG, Jiabing, Beijing 100176 (CN); LAN, Hong, Hangzhou, Zhejiang 311100 (CN); ZHOU, Quan, Hangzhou, Zhejiang 311100 (CN)
(74) Representative: Altmann Stößel Dick Patentanwälte PartG mbB
(86) International application number: PCT/CN2024/127637
(87) International publication number: WO 2025/087431

(57) **Abstract**

The present invention relates to a compound of formula (I), or a stereoisomer, a tautomer, a deuterated compound or a pharmaceutically acceptable salt thereof, a preparation method therefor, a pharmaceutical composition comprising the compound, and the use thereof as a drug for treating and/or preventing KRAS-mediated diseases.

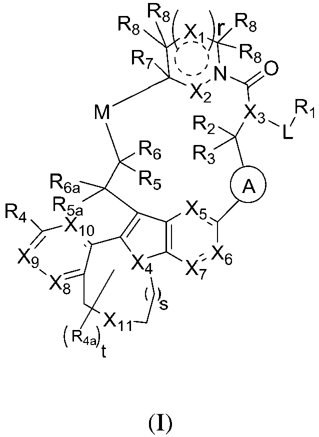

## Description

### TECHNICAL FIELD

The present application relates to the field of medical technology, specifically to a compound represented by formula (I), or a stereoisomer, a tautomer, a deuterated compound or a pharmaceutically acceptable salt thereof, as well as a preparation method therefor, a pharmaceutical composition thereof, and use thereof as a medicament for treating cancer.

### BACKGROUND

Currently, drug research and development targeting KRAS mutations is one of the hotspots in new drug research. The KRAS gene is the most frequently mutated oncogene in human cancers. According to statistics, KRAS gene mutations are found in nearly 90% of pancreatic cancers, 30-40% of colon cancers, and over 30% of lung adenocarcinomas. However, due to the lack of a pocket suitable for small molecule inhibitor binding on the surface of the KRAS protein, the development of small molecule drugs targeting KRAS had no major breakthroughs for 40 years. It was not until recent years, through the unremitting efforts of researchers, that KRAS G12C inhibitors were launched.

Following the discovery of KRAS G12C inhibitors, scientists have turned their attention to other KRAS mutants and to developing pan-KRAS targeted therapies capable of targeting multiple, or even all, KRAS mutants. Pan-KRAS targeted therapies have the potential to treat a broad patient population, including cancers harboring KRAS G12D, G12V, G13D, G12R, G12A, G12S mutations, as well as KRAS wild-type amplification, and cancers that have developed resistance to KRAS G12C inhibitors.

Based on the importance of KRAS aberrant activation in cancer progression and the prevalence of KRAS gene mutations in human cancers, there remains a significant unmet clinical need for the development of safe and effective pan-KRAS inhibitors.

### SUMMARY

An object of the present application is to provide a safe and effective pan-KRAS inhibitor, particularly an inhibitor for use in the treatment of tumors such as intestinal cancer, lung cancer, pancreatic cancer, cholangiocarcinoma, gastric cancer, and the like.

The present application provides a compound represented by formula (I), or a stereoisomer, a tautomer, a deuterated compound or a pharmaceutically acceptable salt thereof: 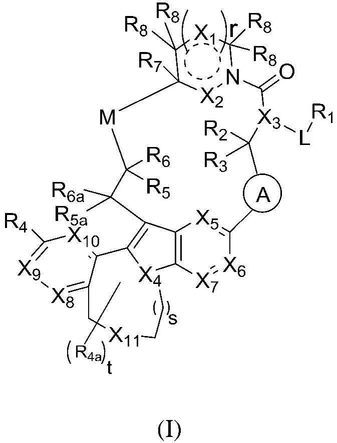 wherein, 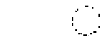 represents that a bond in a ring is a single bond or a double bond;
each X₁ is the same or different, and each X₁ is independently selected from the group consisting of CR₁₀, CR₁₀R₁₀, N, NR₁₀, O, S and S(O)₁₋₂;
X₂ is selected from the group consisting of O, S, S(O)₁₋₂, N and NR₁₀;
X₃ is selected from the group consisting of N and CR₁₀;
X₄ is selected from the group consisting of CR₁₀ and N;
X₅ is selected from the group consisting of CR₁₁ and N;
X₆ is selected from the group consisting of CR₁₂ and N;
X₇ is selected from the group consisting of CR₁₃ and N;
X₈ is selected from the group consisting of CR₁₄ and N;
X₉ is selected from the group consisting of CR₁₅ and N;
X₁₀ is selected from the group consisting of CR₁₆ and N;
X₁₁ is selected from the group consisting of C(R₁₇)₂, O and NR₁₇;
L is selected from the group consisting of 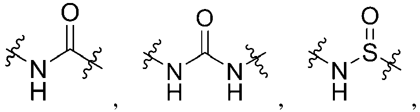 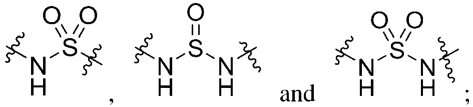
ring A is selected from the group consisting of C₅₋₁₄ cycloalkyl, 5- to 14-membered heterocyclyl, C₆₋₁₈ aryl and 5- to 18-membered heteroaryl; wherein the C₅₋₁₄ cycloalkyl, 5- to 14-membered heterocyclyl, C₆₋₁₈ aryl or 5- to 18-membered heteroaryl is optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₁ is selected from the group consisting of H, hydroxyl, C₁₋₆ hydroxyalkyl, amino, cyano, halogen, C₁₋₆ alkyl, C₁₋₆ haloalkyl, C₁₋₆ alkoxy, C₁₋₆ haloalkoxy, -C₀₋₃ alkylene-N(R_{c})₂, -C₀₋₃ alkylene-C₂₋₆ alkenyl, -C₀₋₃ alkylene-C₂₋₆ alkynyl, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl and -C₀₋₃ alkylene-5- to 18-membered heteroaryl; wherein the hydroxyl, C₁₋₆ hydroxyalkyl, amino, C₁₋₆ alkyl, C₁₋₆ haloalkyl, C₁₋₆ alkoxy, C₁₋₆ haloalkoxy, -C₀₋₃ alkylene-N(R_{c})₂, -C₀₋₃ alkylene-C₂₋₆ alkenyl, -C₀₋₃ alkylene-C₂₋₆ alkynyl, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl or -C₀₋₃ alkylene-5- to 18-membered heteroaryl is optionally further substituted by one or more Rₐ, or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₂ is selected from the group consisting of H, hydroxyl, halogen, C₁₋₆ alkyl, C₁₋₆ haloalkyl, C₁₋₆ alkoxy and C₁₋₆ haloalkoxy;
R₃ is selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; or,
R₂ and R₃ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b}; or two R_{b} on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₄ is selected from the group consisting of H, hydroxyl, C₁₋₆ hydroxyalkyl, amino, cyano, halogen, C₁₋₆ alkyl, C₁₋₆ haloalkyl, C₁₋₆ alkoxy, C₁₋₆ haloalkoxy, C₂₋₆ alkenyl, C₂₋₆ alkynyl, -C₀₋₃ alkylene-N(R_{c})₂, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl and -C₀₋₃ alkylene-5- to 18-membered heteroaryl; wherein the hydroxyl, C₁₋₆ hydroxyalkyl, amino, C₁₋₆ alkyl, C₁₋₆ haloalkyl, C₁₋₆ alkoxy, C₁₋₆ haloalkoxy, C₂₋₆ alkenyl, C₂₋₆ alkynyl, -C₀₋₃ alkylene-N(R_{c})₂, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl or -C₀₋₃ alkylene-5- to 18-membered heteroaryl is optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₄ₐ is selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; wherein the amino, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl or C₁₋₆ hydroxyalkyl is optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b}; or,
two R₄ₐ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b};
R₅ is selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl;
R₆ is selected from the group consisting of H, halogen, hydroxyl, cyano, C₁₋₃ alkoxy, C₁₋₃ alkyl, C₂₋₆ alkenyl, C₂₋₆ alkynyl, C₃₋₈ cycloalkyl, 3- to 14-membered heterocyclyl, 5- to 10-membered heteroaryl and C₆₋₁₀ aryl; wherein the hydroxyl, C₁₋₃ alkoxy, C₁₋₃ alkyl, C₂₋₆ alkenyl, C₂₋₆ alkynyl, C₃₋₈ cycloalkyl, 3- to 14-membered heterocyclyl, 5- to 10-membered heteroaryl or C₆₋₁₀ aryl is optionally further substituted by one or more Rₐ; or,
R₅ and R₆ together with the atom to which they are attached form carbonyl, C₃₋₈ cycloalkyl or 3- to 14-membered heterocyclyl; wherein the C₃₋₈ cycloalkyl or 3- to 14-membered heterocyclyl is optionally further substituted by one or more Rₐ;
R₅ₐ and R₆ₐ are each independently selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; or,
R₅ₐ and R₆ₐ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b};
R₇ is selected from the group consisting of absent, H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl, C₁₋₆ hydroxyalkyl, C₃₋₈ cycloalkyl, 3- to 14-membered heterocyclyl and 5-to 10-membered heteroaryl;
R₈ is selected from the group consisting of absent, H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; or,
R₇ and R₈ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
two R₈ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b};
M is selected from the group consisting of C₁₋₆ alkylene, C₂₋₆ alkenylene, C₂₋₆ alkynylene, C₁₋₆ heteroalkylene, -C(O)O-CH(R₉)-, -C(O)NH-CH(R₉)- and 5- to 8-membered heteroarylene; R₉ is selected from the group consisting of hydrogen and methyl;
when M is -C(O)O-CH(R₉)- or -C(O)NH-CH(R₉)-, CH(R₉) therein is bonded to -C(R₅R₆)-; or,
R₆ and R₉ together with the atom to which they are attached form C₃₋₈ cycloalkyl or 3- to 14-membered heterocyclyl; wherein the C₃₋₈ cycloalkyl or 3- to 14-membered heterocyclyl is optionally further substituted by one or more R_{b};
R₁₀ is selected from the group consisting of H, halogen, cyano, C₁₋₆ alkyl, C₁₋₆ alkoxy, C₁₋₆ haloalkoxy, C₃₋₆ cycloalkyl, 3- to 8-membered heterocyclyl and C₁₋₆ haloalkyl; or,
R₁₀ and R₈ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
two R₁₀ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b};
R₁₁, R₁₂, R₁₃, R₁₄, R₁₅, R₁₆, R₁₇ are each independently selected from the group consisting of H, halogen, C₁₋₆ alkyl, C₁₋₆ alkoxy, C₁₋₆ haloalkoxy and C₁₋₆ haloalkyl; or,
two R₁₇ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
R₁₃ and R₄ₐ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
R₁₆ and R₅ₐ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
R₁₇ and R₄ₐ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
any two of R₁₄, R₁₅, R₁₆, R₄ and R₄ₐ together with the atom to which they are attached form C₃₋₈ cycloalkyl, 3- to 8-membered heterocyclyl, C₆₋₁₀ aryl or 5- to 10-membered heteroaryl; wherein the C₃₋₈ cycloalkyl, 3- to 8-membered heterocyclyl, C₆₋₁₀ aryl or 5- to 10-membered heteroaryl is optionally further substituted by one or more R_{b};
Rₐ is independently selected from the group consisting of absent, H, hydroxyl, oxo, amino, imino, cyano, halogen, C₁₋₆ alkyl, C₁₋₆ haloalkyl, C₁₋₆ alkoxy, C₁₋₆ haloalkoxy, -C₀₋₃ alkylene-OR_{b}, -C₀₋₃ alkylene-SR_{b}, -C₀₋₃ alkylene-N(R_{b})₂, -C₀₋₃ alkylene-S(O)₁₋₂R_{b}, -C₀₋₃ alkylene-S(R_{b})₅, =C(R_{b})₂, C₂₋₆ alkenyl, C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl and -C₀₋₃ alkylene-(5- to 18-membered heteroaryl); wherein the amino, imino, C₁₋₆ alkyl, C₁₋₆ haloalkyl, C₁₋₆ alkoxy, C₁₋₆ haloalkoxy, C₂₋₆ alkenyl, C₂₋₆ alkynyl, -C₀₋₃ alkylene-, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl or -C₀₋₃ alkylene-(5- to 18-membered heteroaryl) is optionally further substituted by one or more R_{b};
each R_{b} is independently selected from the group consisting of **H,** halogen, hydroxyl, oxo, amino, cyano, C₁₋₆ alkyl, C₁₋₆ alkoxy, C₃₋₆ cycloalkyl, 3- to 8-membered heterocyclyl, C₁₋₆ haloalkoxy and C₁₋₆ haloalkyl; wherein the amino, C₁₋₆ alkyl, C₁₋₆ alkoxy, C₃₋₆ cycloalkyl, 3- to 8-membered heterocyclyl, C₁₋₆ haloalkoxy or C₁₋₆ haloalkyl is optionally further substituted by one or more **H,** halogen, hydroxyl, cyano, C₁₋₆ alkyl, C₁₋₆ haloalkyl, C₁₋₆ alkoxy, C₁₋₆ haloalkoxy, amino, -NHC₁₋₃ alkyl or -N(C₁₋₃ alkyl)₂;
each R_{c} is independently selected from the group consisting of **H,** hydroxyl, halogen, C₁₋₆ alkyl, C₃₋₆ cycloalkyl, 3- to 8-membered heterocyclyl and C₁₋₆ haloalkyl;
r is 1 to 3; preferably **1, 2** or 3;
s is 1 to 3, preferably **1, 2** or 3; and
t is 0 to 4, preferably 0, 1, 2, 3 or 4, more preferably 0 to 3.

In some embodiments, formula (I) is formula (I-1): 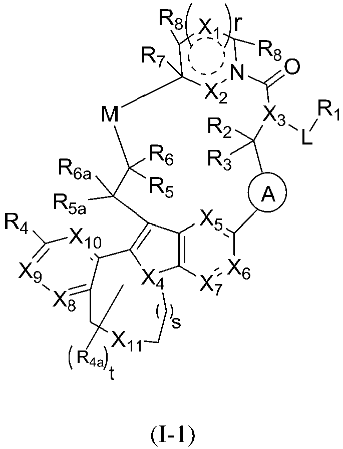 wherein, R₁, R₂, R₃, R₄, R₅, R₆, R₇, R₈, R₄ₐ, R₅ₐ, R₆ₐ, X₁, X₂, X₃, X₄, X₅, X₆, X₇, X₈, X₉, X₁₀, X₁₁, L, M, ring A, r, s, t are as defined in the above-mentioned formula (I).

In some embodiments, formula (I) is selected from the group consisting of formula (II-1) and formula (II-2): 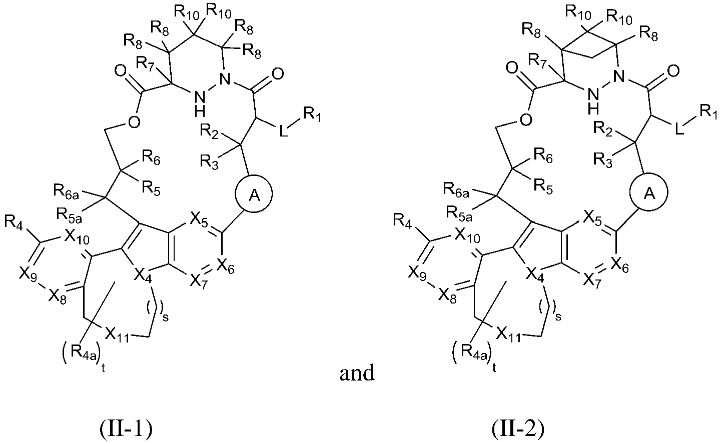 wherein, R₁, R₂, R₃, R₄, R₅, R₆, R₇, R₈, R₁₀, R₄ₐ, R₅ₐ, R₆ₐ, X₄, X₅, X₆, X₇, X₈, X₉, X₁₀, X₁₁, L, ring A, s, t are as defined in the above-mentioned formula (I).

In some embodiments, formula (I) is selected from the group consisting of formula (II-1a) and formula (II-2a): 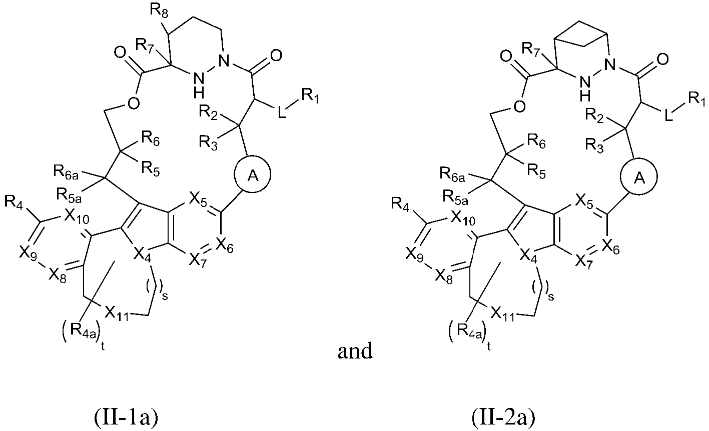 wherein, R₁, R₂, R₃, R₄, R₅, R₆, R₇, R₈, R₄ₐ, R₅ₐ, R₆ₐ, X₄, X₅, X₆, X₇, X₈, X₉, X₁₀, X₁₁, L, ring A, s, t are as defined in the above-mentioned formula (I).

In some embodiments, formula (I) is selected from the group consisting of formulas (IIIa) to (IIIc) and (IIIa') to (IIIc'): 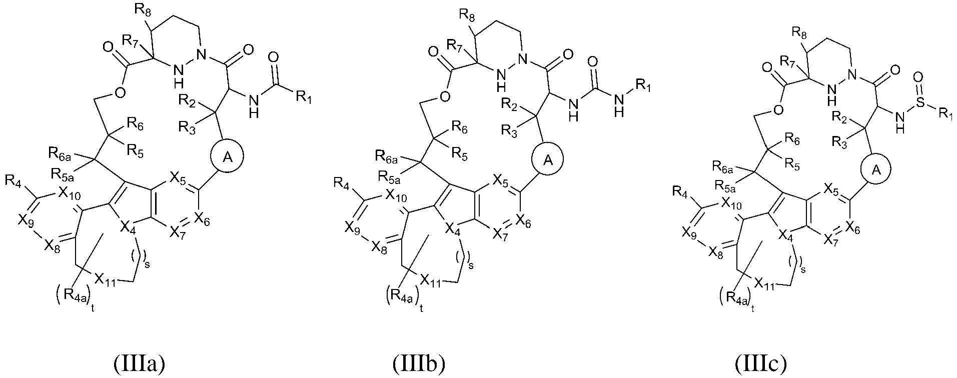 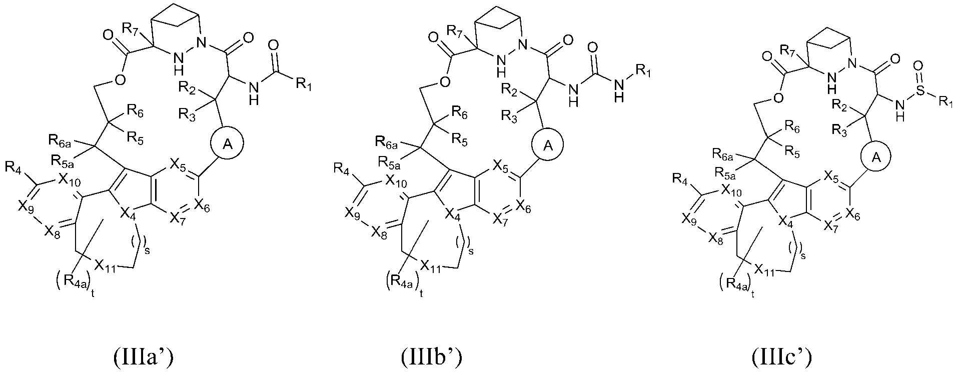 wherein, R₁, R₂, R₃, R₄, R₅, R₆, R₇, R₈, R₄ₐ, R₅ₐ, R₆ₐ, X₄, X₅, X₆, X₇, X₈, X₉, X₁₀, X₁₁, ring A, s, t are as defined in the above-mentioned formula (I).

In some embodiments, ring A in formula (I) is selected from the group consisting of C₆₋₁₈ aryl and 5- to 18-membered heteroaryl; wherein the C₆₋₁₈ aryl or 5- to 18-membered heteroaryl is optionally further substituted by one or more Rₐ, wherein Rₐ is selected from the group consisting of H, hydroxyl, cyano, halogen, C₁₋₆ alkyl, C₁₋₆ haloalkyl, C₁₋₆ alkoxy and C₁₋₆ haloalkoxy, wherein the C₁₋₆ alkyl, C₁₋₆ haloalkyl, C₁₋₆ alkoxy or C₁₋₆ haloalkoxy is optionally further substituted by one or more R_{b}, wherein R_{b} is selected from the group consisting of H, halogen and C₁₋₆ alkyl.

In some embodiments, the C₆₋₁₈ aryl in formula (I) is phenyl.

In some embodiments, the 5- to 18-membered heteroaryl in formula (I) is selected from the group consisting of thiazolyl and oxazolyl.

In some embodiments, X₄ in formula (I) is N.

In some embodiments, X₅ in formula (I) is selected from the group consisting of CH and N.

In some embodiments, X₆ in formula (I) is selected from the group consisting of CH and N.

In some embodiments, X₇ in formula (I) is selected from the group consisting of CH and N.

In some embodiments, X₈ in formula (I) is selected from the group consisting of CH and N.

In some embodiments, X₉ in formula (I) is selected from the group consisting of CH and N.

In some embodiments, X₁₀ in formula (I) is CH.

In some embodiments, X₁₁ in formula (I) is O.

In some embodiments, formula (I) is selected from the group consisting of formulas (Iva) to (IVc) and formulas (IVa') to (IVc'): 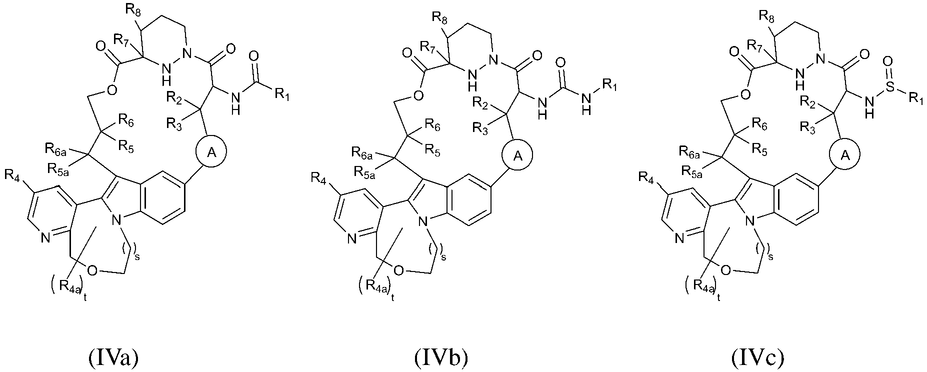 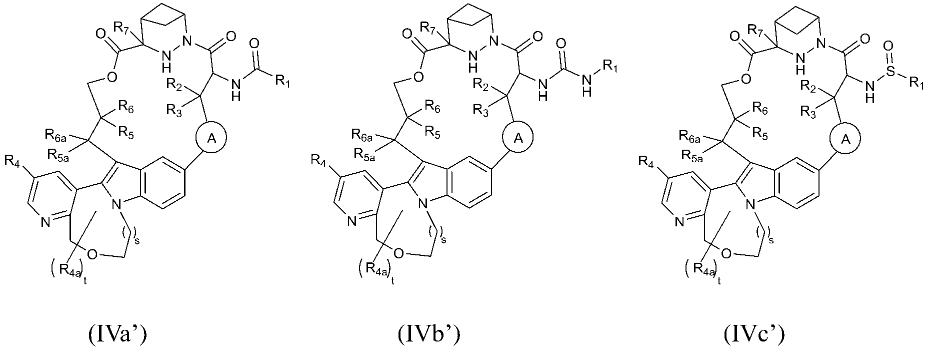 wherein, R₁, R₂, R₃, R₄, R₅, R₆, R₇, R₈, R₄ₐ, R₅ₐ, R₆ₐ, ring A, s, t are as defined in the above-mentioned formula (I).

In some embodiments, formula (I) is selected from the group consisting of formulas (IVa-1) to (IVc-1) and (IVa-1') to (IVc-1'): 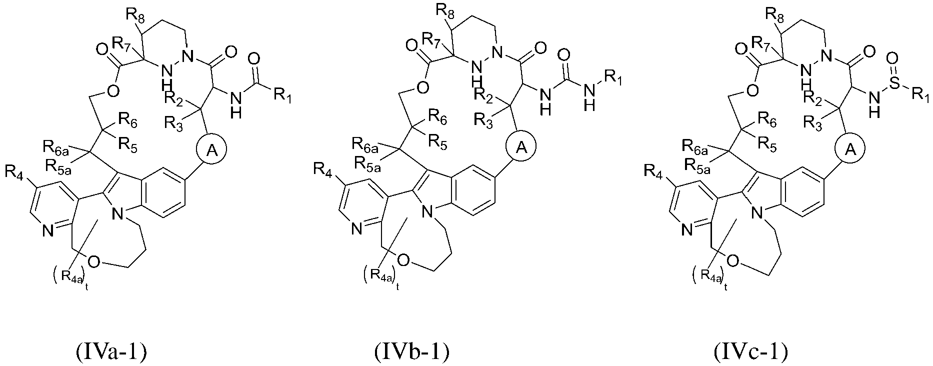 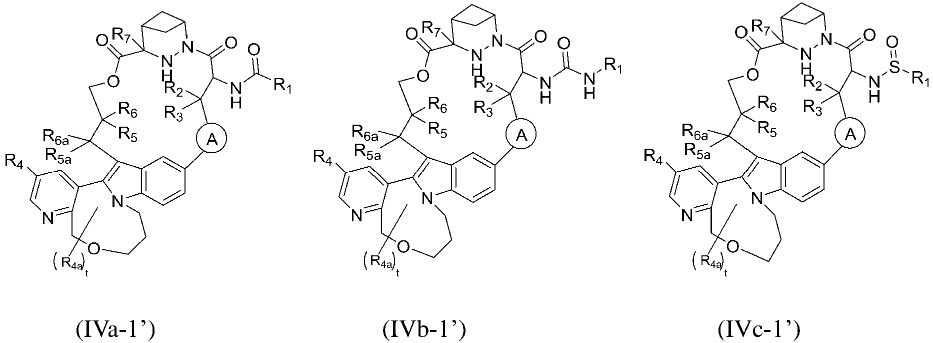 wherein, R₁, R₂, R₃, R₄, R₅, R₆, R₇, R₈, R₄ₐ, R₅ₐ, R₆ₐ, ring A, t are as defined in the above-mentioned formula (I).

In some embodiments, formula (I) is selected from the group consisting of formulas (Va) to (Vc) and formulas (Va') to (Vc'): 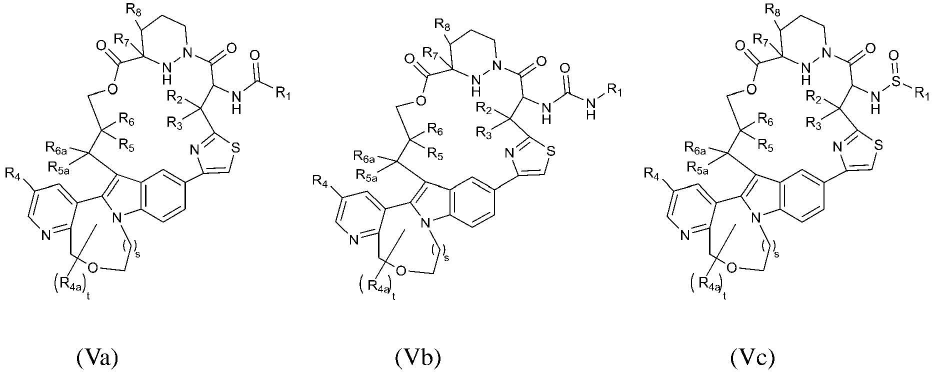 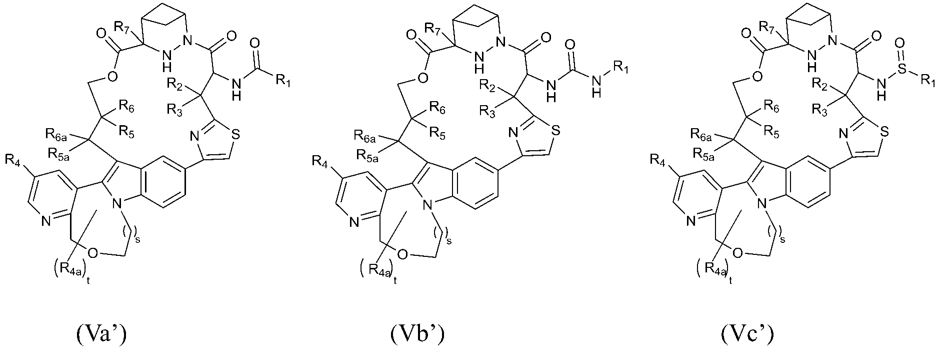 wherein, R₁, R₂, R₃, R₄, R₅, R₆, R₇, R₈, R₄ₐ, R₅ₐ, R₆ₐ, s, t are as defined in the above-mentioned formula (I).

In some embodiments, formula (I) is selected from the group consisting of formulas (Va-1) to (Vc-1) and formulas (Va-1') to (Vc-1'): 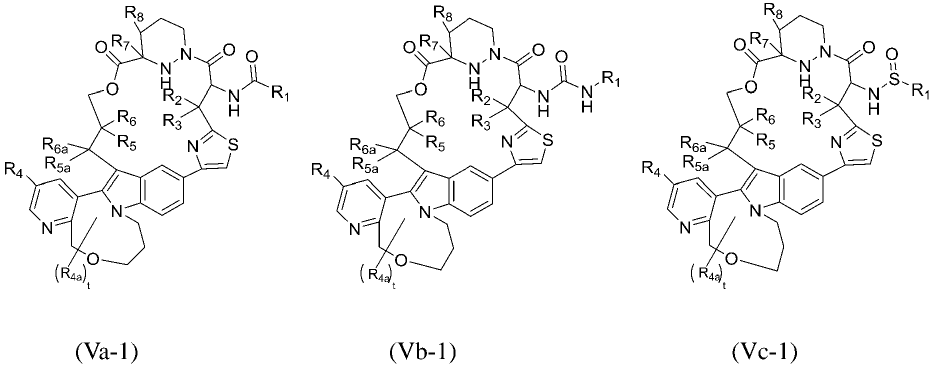 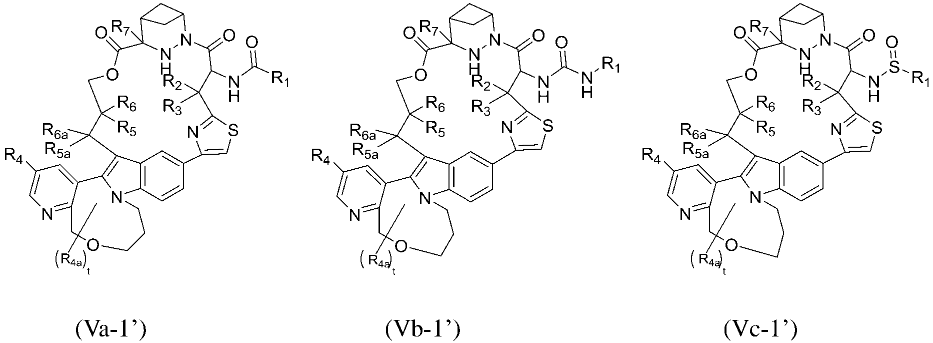 wherein, R₁, R₂, R₃, R₄, R₅, R₆, R₇, R₈, R₄ₐ, R₅ₐ, R₆ₐ, t are as defined in the above-mentioned formula (I).

In some embodiments, formula (I) is selected from the group consisting of formulas (VI) and (VI'): 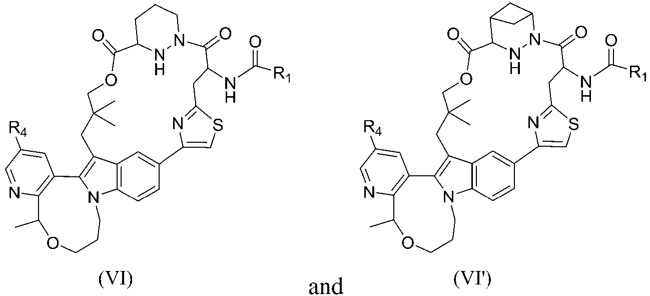 wherein, R₁, R₄ are as defined in the above-mentioned formula (I).

In some embodiments, formula (I) is selected from the group consisting of formulas (VI-1) and (VI'-1): 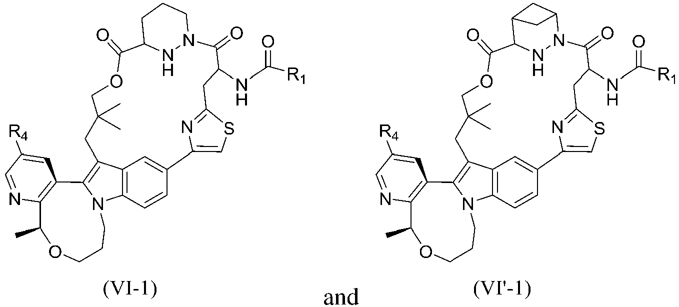 wherein, R₁, R₄ are as defined in the above-mentioned formula (I).

In some embodiments, R₁ in formula (I) is selected from the group consisting of C₁₋₆ alkyl, -C₀₋₃ alkylene-C₃₋₁₄ cycloalkyl, -C₀₋₃ alkylene-3- to 14-membered heterocyclyl, -C₀₋₃ alkylene-N(R_{c})₂, -C₀₋₃ alkylene-C₂₋₆ alkenyl and -C₀₋₃ alkylene-C₂₋₆ alkynyl; wherein the C₁₋₆ alkyl, -C₀₋₃ alkylene-C₃₋₁₄ cycloalkyl, -C₀₋₃ alkylene-3- to 14-membered heterocyclyl, -C₀₋₃ alkylene-N(R_{c})₂, -C₀₋₃ alkylene-C₂₋₆ alkenyl or -C₀₋₃ alkylene-C₂₋₆ alkynyl is optionally further substituted by one or more Rₐ, each R_{c} is independently selected from the group consisting of H, hydroxyl, halogen, C₁₋₆ alkyl, C₃₋₆ cycloalkyl and C₁₋₆ haloalkyl; wherein Rₐ is selected from the group consisting of halogen, C₁₋₆ alkyl, =C(R_{b})₂, C₁₋₆ haloalkyl, C₁₋₆ alkoxy and -C₀₋₃ alkylene-N(R_{b})₂, R_{b} is selected from the group consisting of H, halogen and C₁₋₆ alkyl; or two Rₐ on the same atom together with the atom to which they are attached form C₃₋₈ cycloalkyl or 3- to 8-membered heterocyclyl.

In some embodiments, R₁ in formula (I) is selected from the group consisting of

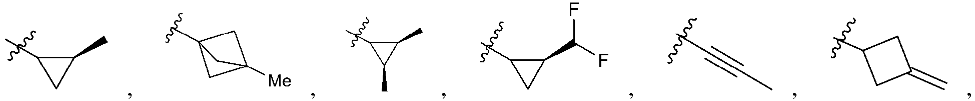

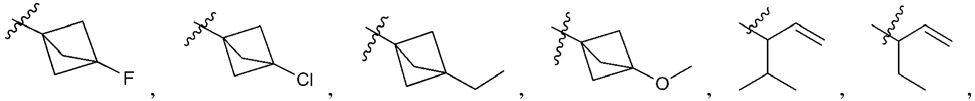

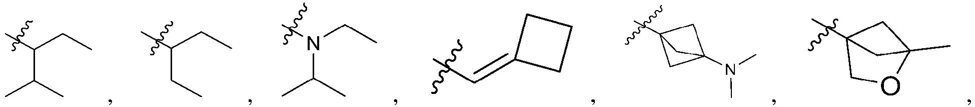

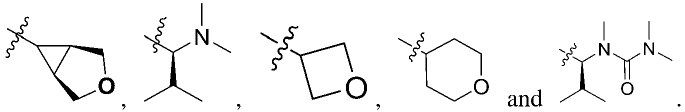

In some embodiments, R₂ in formula (I) is H.

In some embodiments, R₃ in formula (I) is H.

In some embodiments, R₄ in formula (I) is selected from the group consisting of H, C₁₋₆ alkyl, C₁₋₆ alkoxy, -C₀₋₃ alkylene-3- to 14-membered heterocyclyl and -C₀₋₃ alkylene-N(R_{c})₂; wherein the C₁₋₆ alkyl, C₁₋₆ alkoxy, -C₀₋₃ alkylene-3- to 14-membered heterocyclyl or -C₀₋₃ alkylene-N(R_{c})₂ is optionally further substituted by one or more Rₐ, wherein Rₐ is independently selected from the group consisting of absent, H, hydroxyl, oxo, amino, imino, cyano, halogen, C₁₋₆ alkyl, -C₀₋₃ alkylene-N(R_{b})₂, C₁₋₆ haloalkyl, -C₀₋₃ alkylene-C₃₋₁₄ cycloalkyl and -C₀₋₃ alkylene-3- to 14-membered heterocyclyl; wherein the amino, imino, C₁₋₆ alkyl, -C₀₋₃ alkylene-, C₁₋₆ haloalkyl or -C₀₋₃ alkylene-3- to 14-membered heterocyclyl is optionally further substituted by one or more R_{b}, wherein each R_{b} is independently selected from the group consisting of **H,** halogen, hydroxyl, cyano, C₁₋₆ alkyl, C₁₋₆ alkoxy, C₃₋₆ cycloalkyl and C₁₋₆ haloalkyl.

In some embodiments, R₄ in formula (I) is selected from the group consisting of 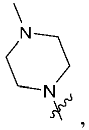 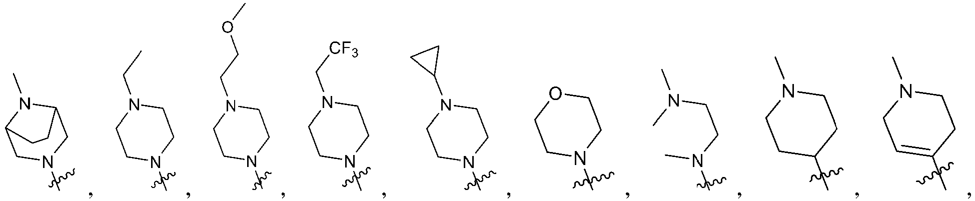 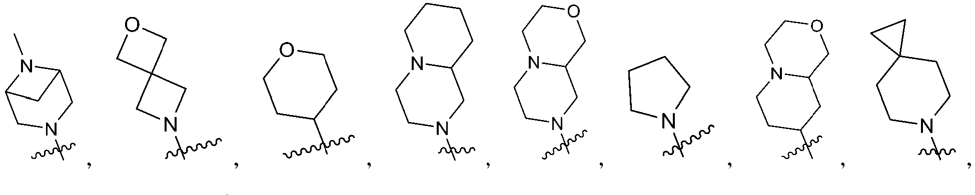 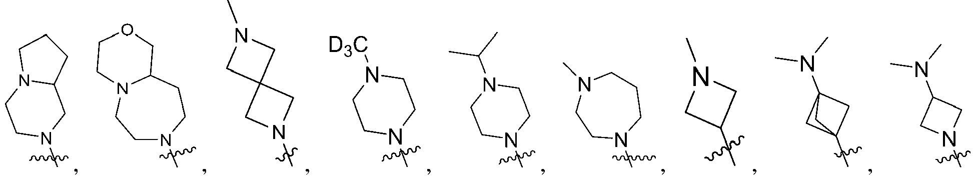 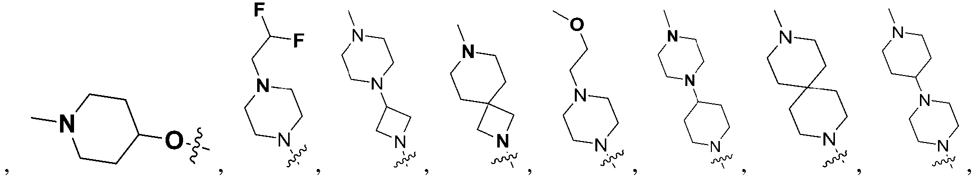 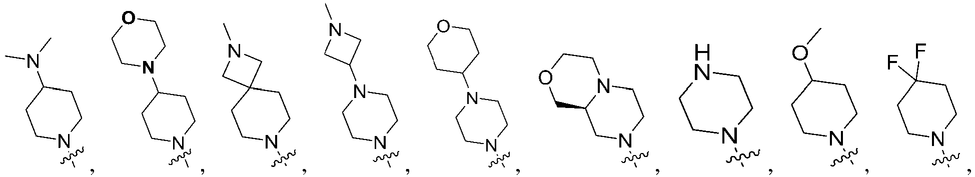 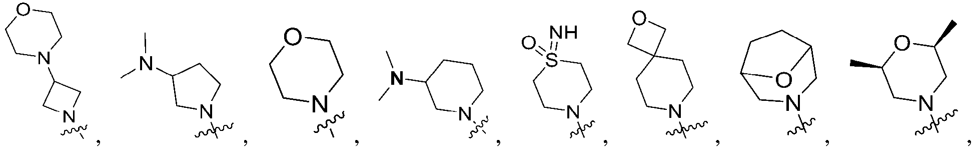 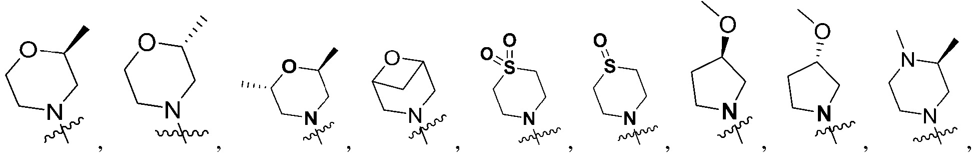 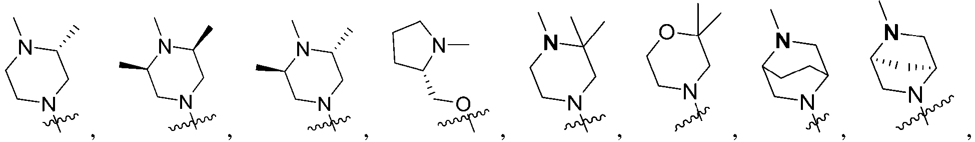 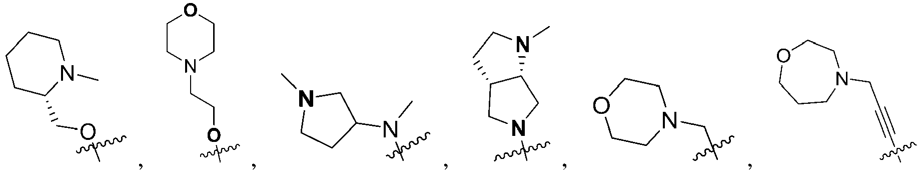 and

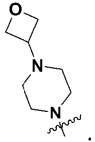

In some embodiments, R₄ₐ in formula (I) is selected from the group consisting of H and C₁₋₆ alkyl.

In some embodiments, R₅ in formula (I) is selected from the group consisting of H and C₁₋₃ alkyl.

In some embodiments, R₆ in formula (I) is selected from the group consisting of H and C₁₋₃ alkyl.

In some embodiments, R₅ₐ in formula (I) is selected from the group consisting of H and C₁₋₃ alkyl.

In some embodiments, R₆ₐ in formula (I) is selected from the group consisting of H and C₁₋₃ alkyl.

In some embodiments, R₇ in formula (I) is selected from the group consisting of H and C₁₋₃ alkyl, preferably H.

In some embodiments, R₈ in formula (I) is selected from the group consisting of H and C₁₋₃ alkyl, preferably H.

In some embodiments, Ring A in formula (I) is selected from the group consisting of 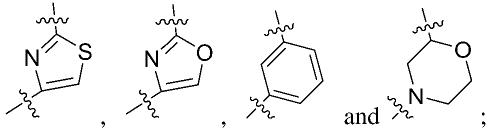 wherein these groups are optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b}.

In some embodiments, the compound represented by formula (I) is selected from the group consisting of:

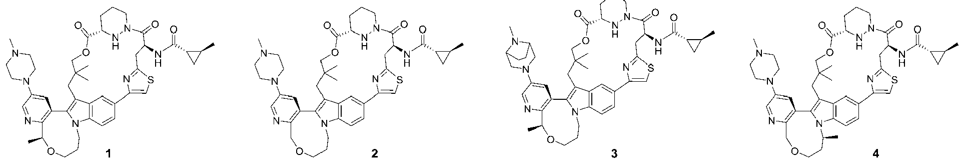

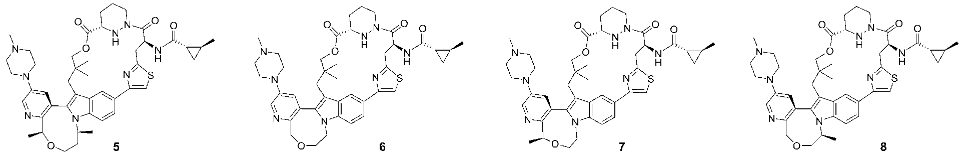

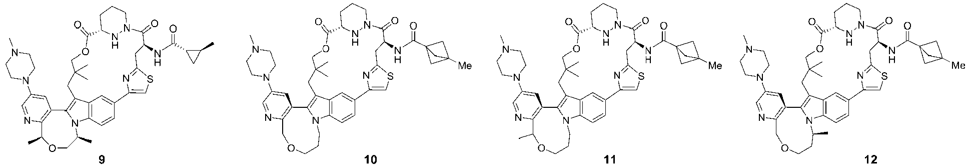

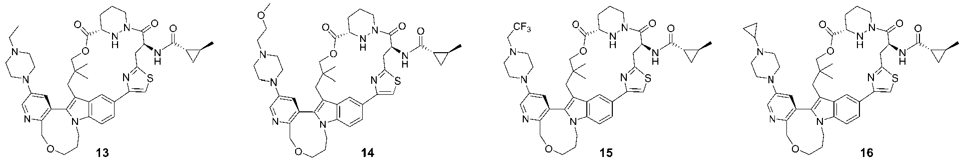

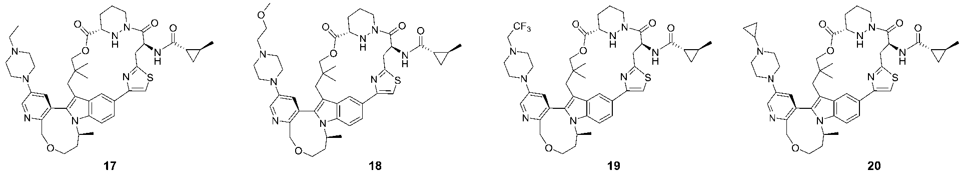

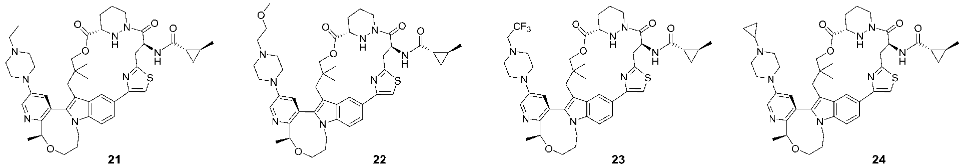

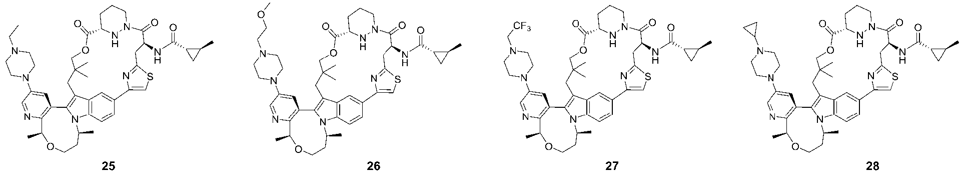

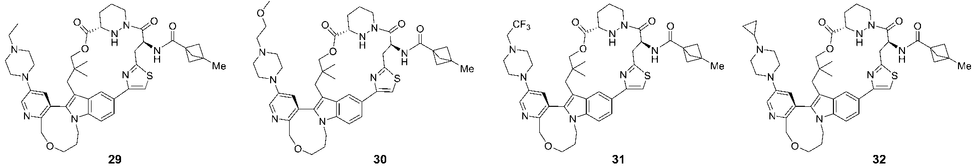

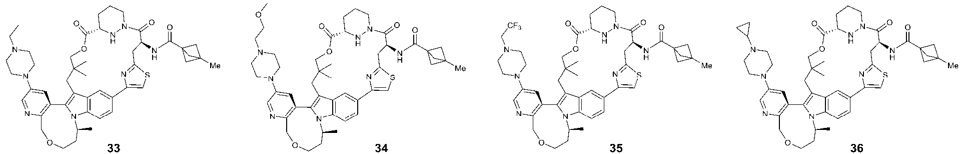

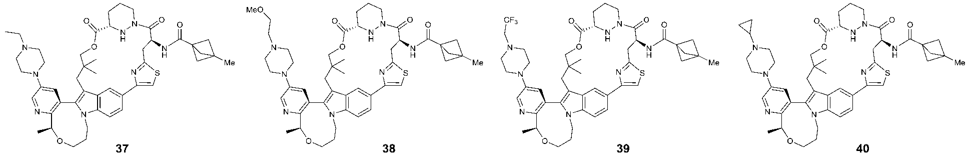

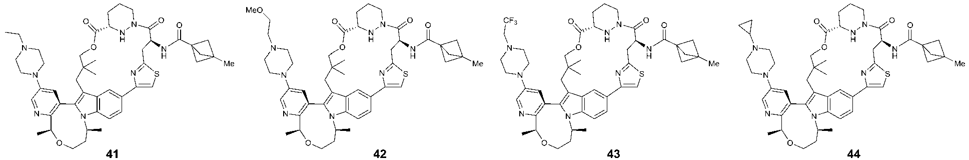

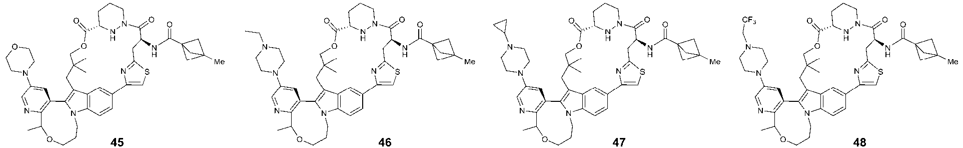

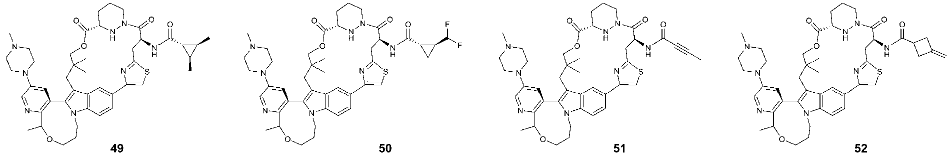

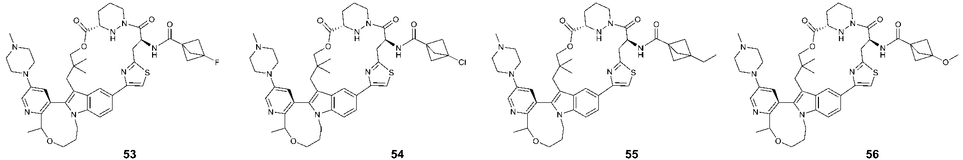

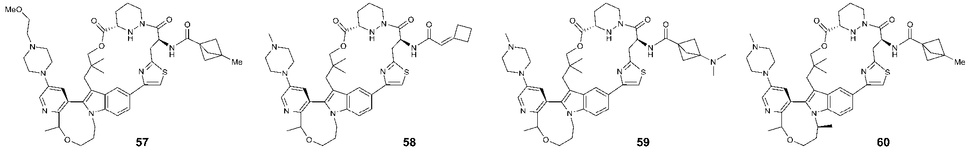

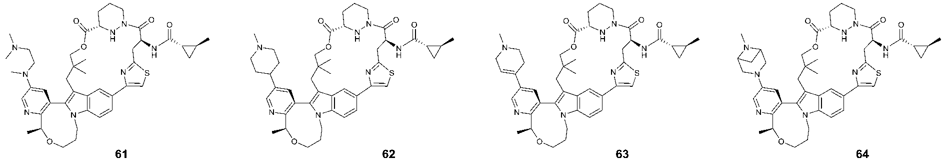

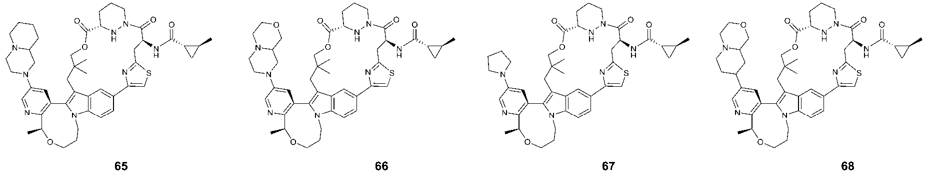

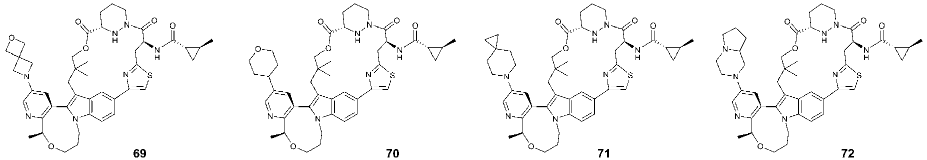

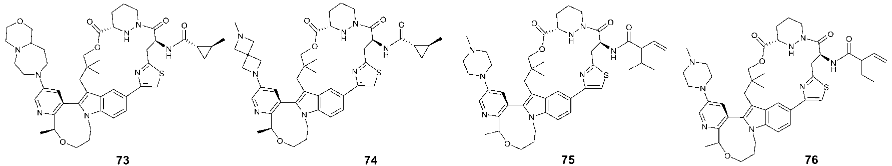

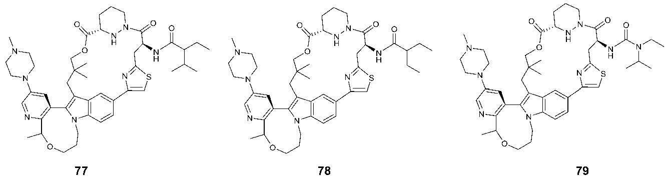

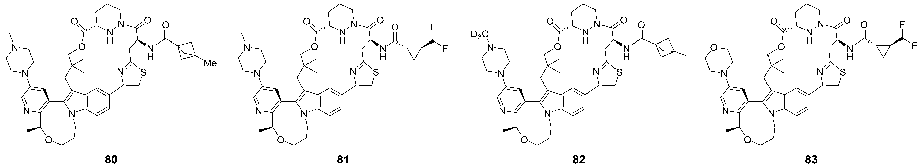

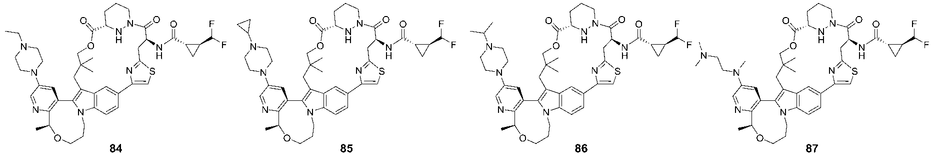

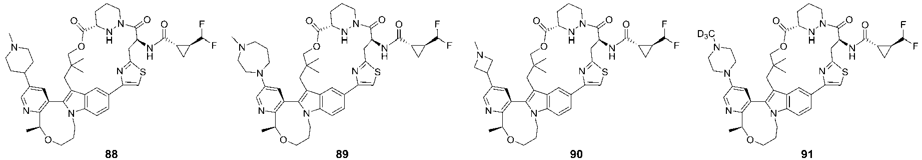

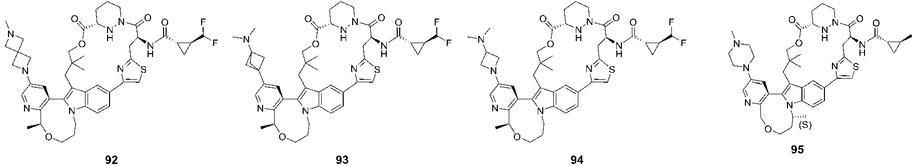

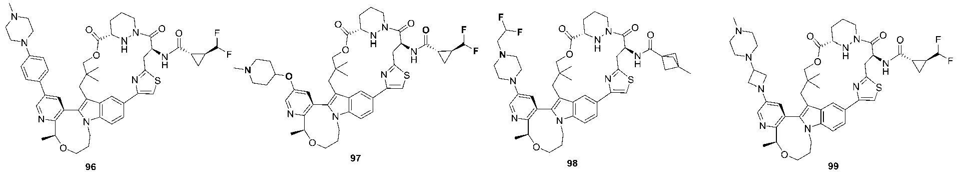

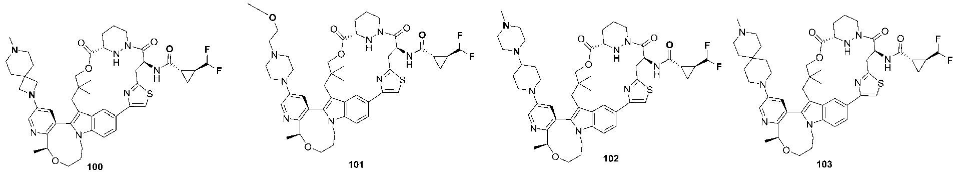

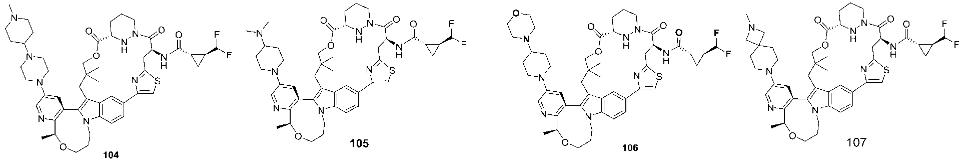

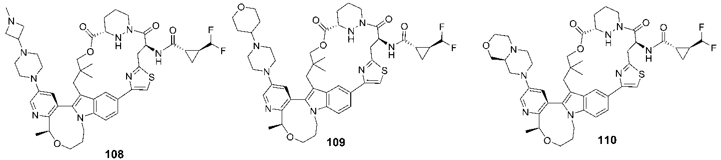

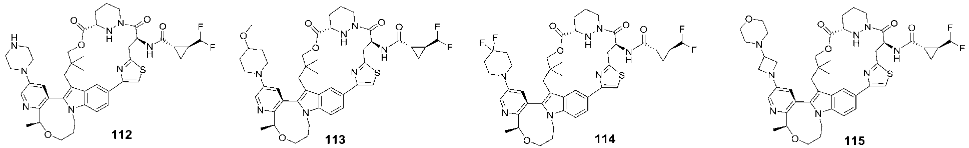

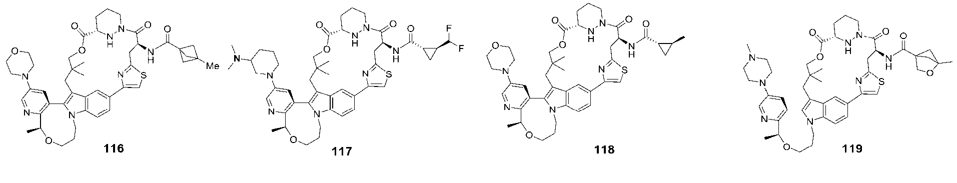

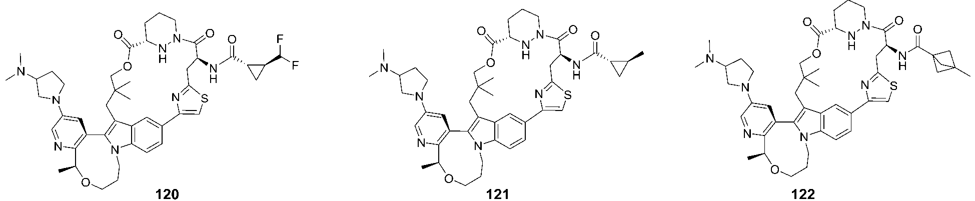

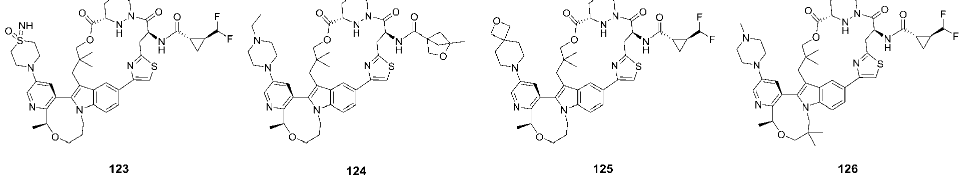

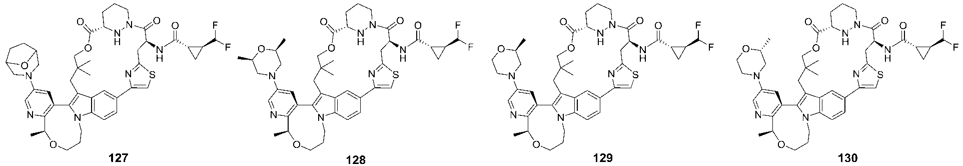

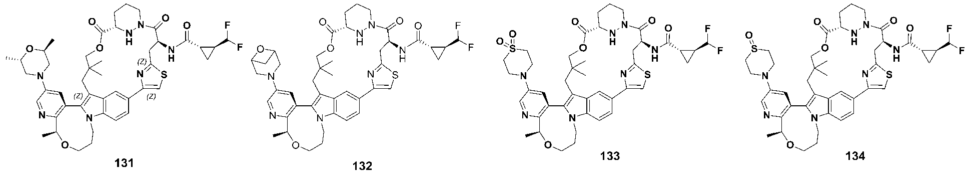

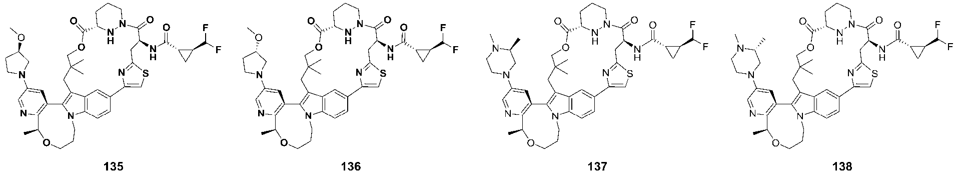

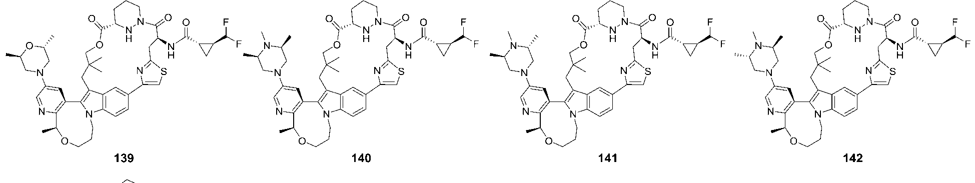

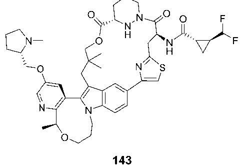

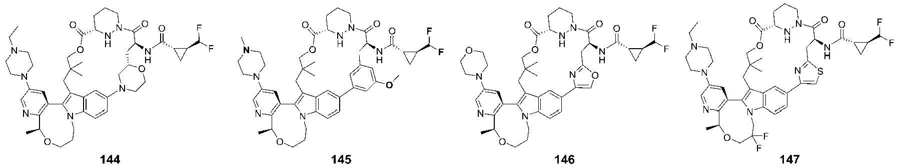

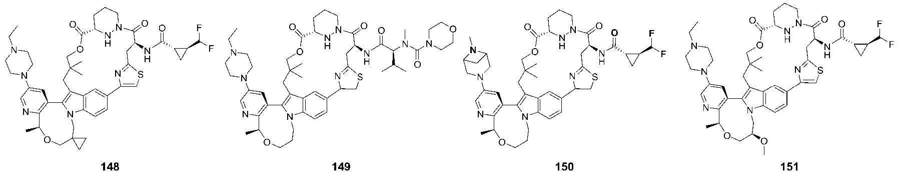

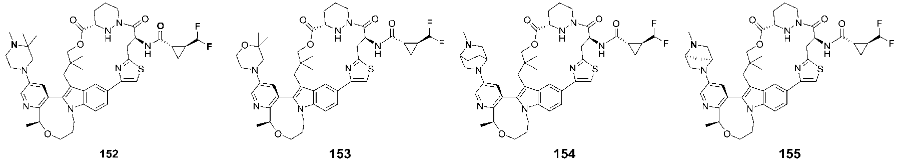

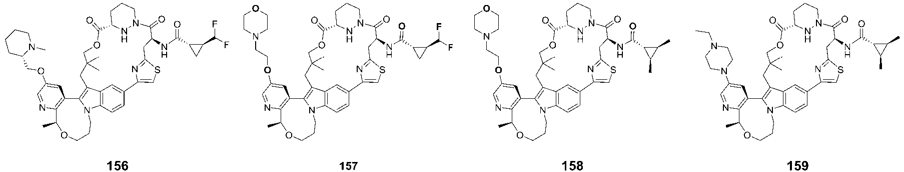

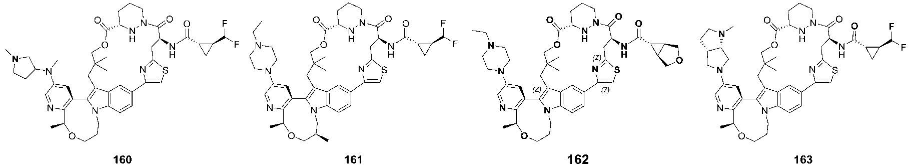

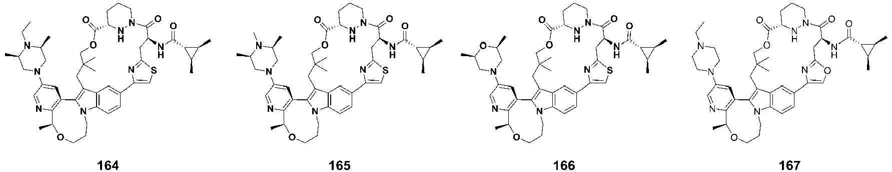

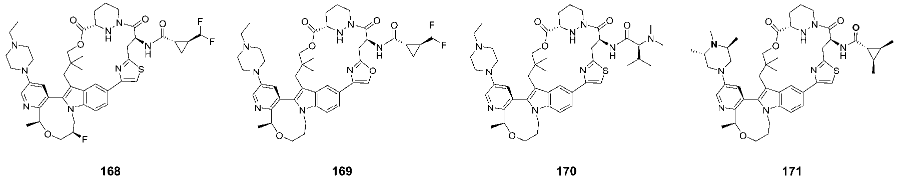

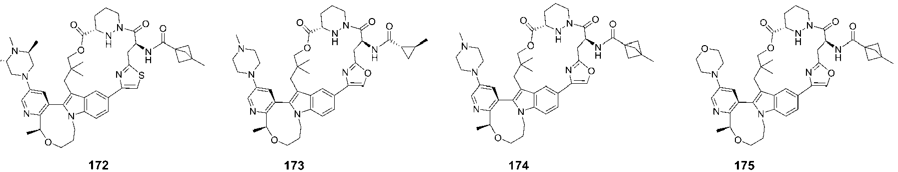

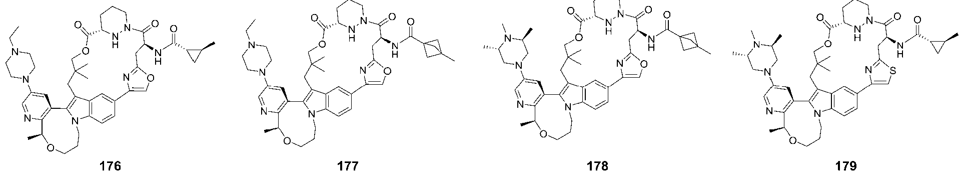

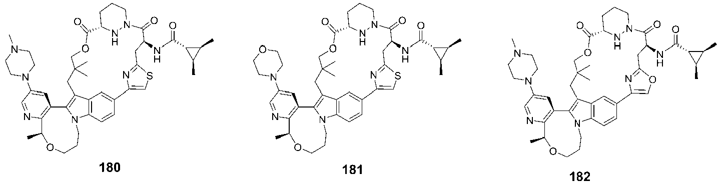

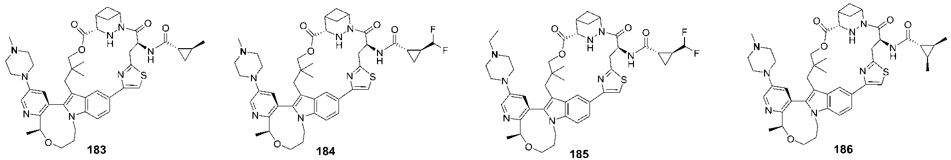

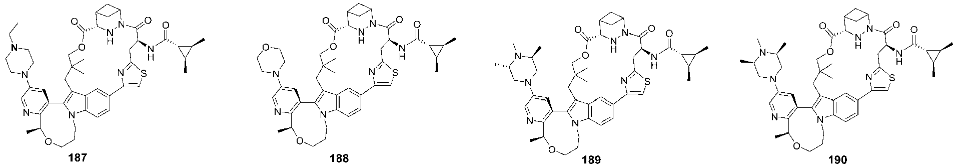

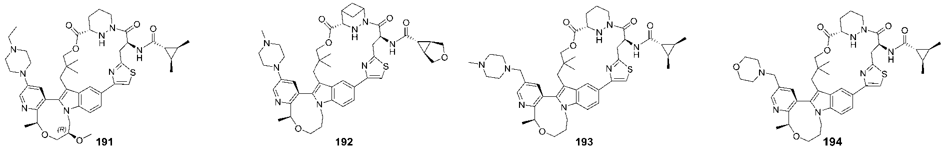

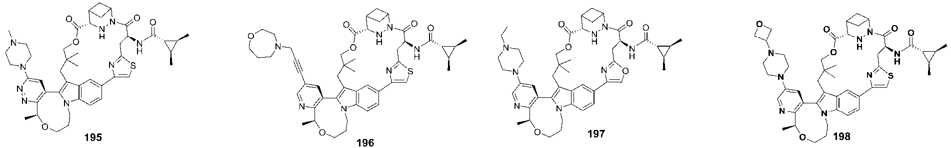

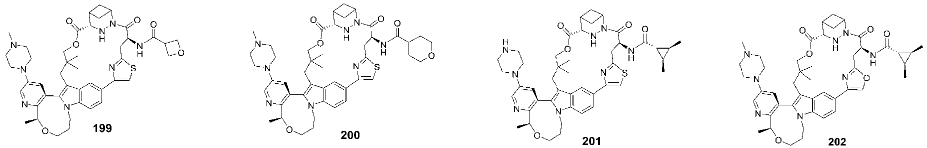

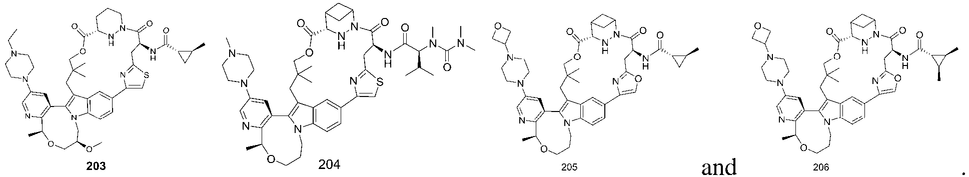

The present application relates to an inhibitor of wild-type KRAS and/or multiple mutant forms of KRAS, such as KRAS G12A, G12C, G12D, G12R, G12S, G12V, G13D and/or Q61H mutations. Specifically, the present application relates to an active compound that inhibits wild-type KRAS and/or KRAS mutations such as G12A, G12C, G12D, G12R, G12S, G12V, G13D and/or Q61H, a pharmaceutical composition thereof, and use thereof, preferably, to an active compound that inhibits G12C, G12D, G12V, a pharmaceutical composition thereof, and use thereof.

The present application also provides a pharmaceutical composition, comprising a therapeutically effective amount of at least one of the above-mentioned compounds represented by formula (I), or the stereoisomer, the tautomer, the deuterated compound or the pharmaceutically acceptable salt thereof.

The present application also provides use of the above-mentioned compound represented by formula (I), or the stereoisomer, the tautomer, the deuterated compound or the pharmaceutically acceptable salt thereof, or the pharmaceutical composition thereof in the manufacture of a medicament.

The present application further provides preferable technical solutions for the use:
Preferably, the use is use in the manufacture of a medicament for treating and/or preventing cancer.

Preferably, the use is use in the manufacture of a medicament for treating a KRAS-mediated disease. Preferably, the disease is cancer.

Preferably, the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumor, intrahepatic cholangiocarcinoma, myelodysplastic syndrome, malignant glioma, prostate cancer, thyroid cancer, schwannoma, lung squamous cell carcinoma, lichenoid keratosis, synovial sarcoma, epidermal cancer, pancreatic cancer, carcinoma of testis and liposarcoma.

The present application also provides a method for treating and/or preventing a disease, comprising administering to a subject in need thereof a therapeutically effective amount of at least any one of the above-mentioned compounds represented by formula (I), or the stereoisomer, the tautomer, the deuterated compound or the pharmaceutically acceptable salt thereof, or the pharmaceutical composition thereof.

The present application also provides a method for treating and/or preventing a KRAS-mediated disease, comprising administering to a subject in need thereof a therapeutically effective amount of at least any one of the above-mentioned compounds represented by formula (I), or the stereoisomer, the tautomer, the deuterated compound or the pharmaceutically acceptable salt thereof, or the pharmaceutical composition thereof.

The present application also provides a method for treating cancer, comprising administering to a subject in need thereof a therapeutically effective amount of at least any one of the above-mentioned compounds represented by formula (I), or the stereoisomer, the tautomer, the deuterated compound or the pharmaceutically acceptable salt thereof, or the pharmaceutical composition thereof.

Preferably, in the above methods, the KRAS-mediated disease is cancer.

Preferably, in the above methods, the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumor, intrahepatic cholangiocarcinoma, myelodysplastic syndrome, malignant glioma, prostate cancer, thyroid cancer, schwannoma, lung squamous cell carcinoma, lichenoid keratosis, synovial sarcoma, pidermal cancer, pancreatic cancer, carcinoma of testis and liposarcoma.

### DETAILED DESCRIPTION

Unless otherwise specified, general chemical terms used in the structural formulae have their usual meanings.

For example, unless otherwise specified, the term "halogen" as used in the present application refers to fluorine, chlorine, bromine or iodine.

In the present application, unless otherwise specified, "alkyl" includes linear or branched chain monovalent saturated hydrocarbon groups. For example, alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl, 2-methylpentyl, and the like. Similarly, "₁₋₆" in "C₁₋₆ alkyl" refers to a group containing 1, 2, 3, 4, 5 or 6 carbon atoms arranged in a linear or branched chain. The term "alkylene" refers to a divalent alkyl linking group. Alkylene formally refers to an alkane with two C-H bonds replaced by points of attachment of the alkylene to the rest of the compound. Similarly, "C₁₋₃" in "C₁₋₃ alkylene" refers to an alkylene group containing 1, 2 or 3 carbon atoms, including but not limited to methylene, 1,2-ethylene, 1,3-propylene or 1,2-isopropylene.

"Alkoxy" refers to an oxygen ether form of the above-mentioned linear or branched alkyl, i.e., -O-alkyl.

The term "haloalkyl" refers to an alkyl in which one or more H atoms have been replaced by halogen atoms.

The term "haloalkoxy" refers to a -O-haloalkyl.

The term "oxo" refers to an oxygen atom in the form of a divalent substituent, which when attached to C forms carbonyl, and when attached to a heteroatom forms sulfinyl or sulfonyl or an N-oxide group.

In the present application, unless otherwise specified, the terms "aromatic ring" or "aromatic heterocycle" refer to a carbocyclic or heterocyclic polyunsaturated ring having aromaticity (having (4n+2) delocalized π electrons, where n is an integer).

The term "aryl", in the present application, unless otherwise specified, refers to an unsubstituted or substituted monocyclic or fused aromatic group containing carbocyclic atoms. Preferably, aryl is C₆₋₁₂ aryl, more preferably, aryl is a C₆₋₁₀ monocyclic or bicyclic aromatic ring group. Preferably, aryl is phenyl or naphthyl. The aryl ring may be fused to heteroaryl, heterocyclyl or cycloalkyl, wherein the ring attached to the parent structure is aryl, non-limiting examples include but are not limited to benzocyclopentyl.

The term "heterocyclyl" refers to a ring system having at least one cycloalkyl or cycloalkenyl containing a heteroatom selected from the group consisting of N, O and S. The heterocyclyl may include monocyclic or polycyclic rings (e.g., having 2, 3 or 4 fused rings, spiro rings, bridged rings, etc.). Heterocyclyl may be attached to the rest of the compound via a carbocyclic atom or a heterocyclic atom. Preferably, heterocyclyl is 3- to 14-membered heterocyclyl, "3- to 14-membered" in "3- to 14-membered heterocyclyl" refers to a heterocyclyl composed of 3 to 14 ring atoms selected from the group consisting of C, N, O and S; more preferably, heterocyclyl is 3- to 8-membered heterocyclyl. Nitrogen or sulfur heteroatoms may optionally be oxidized, and nitrogen heteroatoms may optionally be quaternized. Examples of such heterocyclyls include, but are not limited to, azetidinyl, pyrrolidinyl, piperidinyl, piperazinyl, oxopiperazinyl, oxopiperidinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiomorpholinyl sulfinyl, thiomorpholinyl sulfonyl, and tetrahydrooxadiazolyl. The heterocyclyl may be fused to aryl, heteroaryl or cycloalkyl, wherein the ring attached to the parent structure is heterocyclyl.

The term "heteroaryl", in the present application, unless otherwise specified, refers to a monocyclic or polycyclic (e.g., fused bicyclic) aromatic heterocycle having at least one heteroatom selected from the group consisting of N, O and S, wherein the nitrogen or sulfur heteroatoms may optionally be oxidized, and the nitrogen heteroatoms may optionally be quaternized. Preferably, heteroaryl is 5- to 14-membered heteroaryl, wherein "5- to 14-membered" in "5- to 14-membered heteroaryl" refers to a heteroaryl composed of 5 to 14 ring atoms selected from the group consisting of C, N, O and S. More preferably, heteroaryl is 5- to 10-membered heteroaryl; even more preferably, heteroaryl is 5- to 6-membered heteroaryl. Examples of heteroaryl include, but are not limited to, thienyl, furyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuranyl, benzothienyl, benzoisoxazolyl, benzothiazolyl, benzothiadiazolyl, benzotriazolyl, adeninyl, quinolinyl or isoquinolinyl. The heteroaryl may be fused to aryl, heterocyclyl or cycloalkyl, wherein the ring attached to the parent structure is heteroaryl.

The term "cycloalkyl" refers to a ring system having at least one cyclic alkyl. Preferably, cycloalkyl is C₃₋₁₂ cycloalkyl, wherein "C₃₋₁₂" refers to a cycloalkyl having 3, 4, 5, 6, 7, 8, 9, 10, 11 or 12 ring atoms. More preferably, cycloalkyl is 3- to 10-membered cycloalkyl; even more preferably, cycloalkyl is 3- to 8-membered cycloalkyl or 5- to 6-membered cycloalkyl. Cycloalkyl may include monocyclic and polycyclic rings (e.g., having 2, 3 or 4 fused rings, spiro rings, bridged rings, etc.). In some embodiments, cycloalkyl includes, but is not limited to, cyclopropyl, cyclobutyl, cyclopentyl, etc.; the cycloalkyl may also be fused to aryl, heterocyclyl or heteroaryl, wherein the ring attached to the parent structure is cycloalkyl.

The term "bridged cycloalkyl" refers to a 5- to 20-membered, all-carbon polycyclic group in which any two rings share two carbon atoms that are not directly connected, which may contain one or more double bonds, but none of the rings has a completely conjugated π-electron system. Preferably, bridged cycloalkyl is 5- to 14-membered, more preferably, bridged cycloalkyl is 5- to 10-membered. Depending on the number of constituent rings, it can be classified as bicyclic, tricyclic, tetracyclic or polycyclic bridged cycloalkyl, preferably bicyclic, tricyclic or tetracyclic, more preferably bicyclic or tricyclic. Examples of bridged cycloalkyl include, but are not limited to

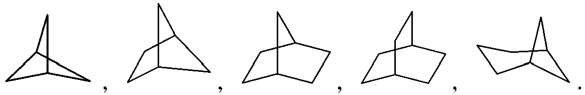

The term "substituted" means that one or more hydrogen atoms in a group are each independently replaced by the same or different substituents. Typical substituents include, but are not limited to, halogen (F, Cl, Br or I), C₁₋₈ alkyl, C₃₋₁₂ cycloalkyl, -OR¹, -SR¹, =O, =S, -C(O)R₁, -C(S)R¹, =NR¹, -C(O)OR¹, -C(S)OR¹, -NR¹R², -C(O)NR¹R², cyano, nitro, -S(O)₂R¹, -O-S(O₂)OR¹, -O-S(O)₂R¹, -OP(O)(OR¹)(OR²); wherein R¹ and R² are independently selected from the group consisting of -H, C₁₋₆ alkyl, C₁₋₆ haloalkyl and C₃₋₆ cycloalkyl. In some embodiments, the substituent is independently selected from the group consisting of -F, -Cl, -Br, -I, -OH, trifluoromethoxy, ethoxy, propoxy, isopropoxy, n-butoxy, isobutoxy, tert-butoxy, -SCH₃, -SC₂H₅, formyl, -C(O)CH₃, cyano, nitro, -CF₃, -OCF₃, amino, dimethylamino, methylthio, sulfonyl and acetyl.

When the number of a linking group is 0, for example -(CH₂)₀- means that the linking group is a bond.

The term "pharmaceutically acceptable salt" refers to a salt prepared from a pharmaceutically acceptable, non-toxic base or acid.

When the compound provided by the present application is an acid, its corresponding salt can be conveniently prepared from pharmaceutically acceptable non-toxic bases, including inorganic bases and organic bases. Salts derived from inorganic bases include salts of aluminum, ammonium, calcium, copper (high valence and low valence), ferric, ferrous, lithium, magnesium, manganese (high valence and low valence), potassium, sodium, zinc and the like. Particularly preferably, salts are salts of ammonium, calcium, magnesium, potassium and sodium. Non-toxic organic bases capable of forming pharmaceutically acceptable salts include primary, secondary and tertiary amines, as well as cyclic amines and substituted amines, such as naturally occurring and synthetic substituted amines. Other pharmaceutically acceptable non-toxic organic bases capable of forming salts include ion exchange resins and arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, reduced glucosamine, glucosamine, histidine, isopropylamine, lysine, methylglucamine, morpholine, piperazine, piperidine, polyamine resins, procaine, chloroprocaine, purines, theobromine, triethylamine, trimethylamine, tripropylamine, tromethamine and the like.

When the compound provided by the present application is a base, its corresponding salt can be conveniently prepared from pharmaceutically acceptable non-toxic acids, including inorganic acids and organic acids. Such acids include, for example, acetic acid, benzenesulfonic acid, benzoic acid, camphorsulfonic acid, citric acid, ethanesulfonic acid, formic acid, fumaric acid, gluconic acid, glutamic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phosphoric acid, succinic acid, sulfuric acid, tartaric acid, oxalic acid, propionic acid, glycolic acid, hydroiodic acid, perchloric acid, cyclamic acid, salicylic acid, 2-naphthalenesulfonic acid, saccharin acid, trifluoroacetic acid and p-toluenesulfonic acid and the like. Preferably, acids are citric acid, hydrobromic acid, formic acid, hydrochloric acid, maleic acid, phosphoric acid, sulfuric acid and tartaric acid. More preferably, acids are formic acid and hydrochloric acid.

A prodrug of the compound of the present application is included within the scope of protection of the present application. Generally, the prodrug is a functional derivative which is readily converted in vivo into the required compound. For example, any pharmaceutically acceptable salt, ester, salt of the ester or other derivative of the compound of the present application, which, upon administration to the subject, is capable of providing, directly or indirectly, the compound of the present application or the pharmaceutically active metabolite or residue thereof.

The compound of the present application may contain one or more asymmetric centers, and may thus give rise to diastereomers and optical isomers. The present application includes all possible diastereomers and their racemic mixtures, their substantially pure resolved enantiomers, all possible geometric isomers, and pharmaceutically acceptable salts thereof.

When the compound represented by formula (I) exists as tautomers, unless specifically stated, the present application includes any possible tautomers and pharmaceutically acceptable salts thereof, and mixtures thereof.

Substitution of the compound represented by formula (I) with heavier isotopes (e.g., deuterium) may afford certain therapeutic advantages resulting from greater metabolic stability, for example, increased in vivo half-life or reduced dosage requirements.

The term "pharmaceutical composition" refers to a mixture consisting of one or more compounds of the present application or the pharmaceutically acceptable salt thereof and a pharmaceutically acceptable excipient. The purpose of the pharmaceutical composition is to facilitate administration of the compound of the present application to an organism.

In the present application, "a", "an", "the", "at least one", and "one or more" are used interchangeably. Thus, for example, a composition comprising "a" pharmaceutically acceptable excipient can be interpreted to mean that the pharmaceutical composition includes "one or more" pharmaceutically acceptable excipients.

The term "pharmaceutically acceptable excipient" refers to those excipients that cause no significant irritation to an organism and do not impair the biological activity and properties of the active compound. Suitable excipients are well known to those skilled in the art, for example, carbohydrates, waxes, water-soluble and/or water-swellable polymers, hydrophilic or hydrophobic materials, gelatin, oils, solvents, water, and the like.

The pharmaceutical composition of the present application can be prepared by combining the compound of the present application with suitable pharmaceutically acceptable excipients, for example, formulated into solid, semi-solid, liquid or gaseous preparations, such as tablets, pills, capsules, powders, granules, ointments, emulsions, suspensions, suppositories, injections, inhalants, gels, microspheres and aerosols.

Typical routes for administering the compound of the present application or the pharmaceutically acceptable salt thereof or the pharmaceutical composition thereof include, but are not limited to, oral, rectal, topical, inhalation, parenteral, sublingual, intravaginal, intranasal, intraocular, intraperitoneal, intramuscular, subcutaneous, intravenous administration.

The term "treating" or "treatment" generally refers to obtaining a desired pharmacological and/or physiological effect. The effect may be therapeutic in terms of partially or completely stabilizing or curing a disease and/or adverse effects caused by the disease. "Treating" or "treatment" as used herein covers any treatment of a disease in a patient, including: (a) inhibiting the symptoms of the disease, i.e., arresting its development; or (b) relieving the symptoms of the disease, i.e., causing regression of the disease or symptoms.

The term "effective amount" refers to an amount of the compound of the present application that (i) treats or prevents a particular disease, condition or disorder, (ii) attenuates, ameliorates or eliminates one or more symptoms of a particular disease, condition or disorder, or (iii) prevents or delays the onset of one or more symptoms of a particular disease, condition or disorder described herein. The amount of a compound of the present application that constitutes a "therapeutically effective amount" varies depending on the compound, the disease state and its severity, the mode of administration, and the age of the mammal to be treated, but can be determined routinely by a person skilled in the art based on their own knowledge and the present disclosure.

### Synthetic scheme

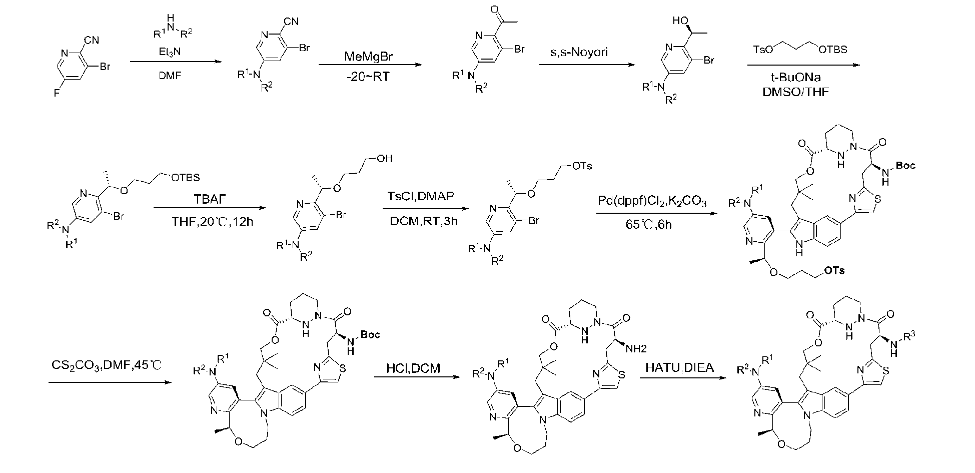

wherein,
R¹ or R² is selected from the group consisting of H, C₁₋₆ alkyl, C₁₋₆ alkoxy, -C₀₋₃ alkylene-(3- to 14-membered heterocyclyl) and -C₀₋₃ alkylene-N(R_{c})₂; wherein the C₁₋₆ alkyl, C₁₋₆ alkoxy, -C₀₋₃ alkylene-(3- to 14-membered heterocyclyl) or -C₀₋₃ alkylene-N(R_{c})₂ is optionally further substituted by one or more Rₐ, wherein Rₐ is independently selected from the group consisting of absent, H, hydroxyl, oxo, amino, imino, cyano, halogen, C₁₋₆ alkyl, -C₀₋₃ alkylene-N(R_{b})₂, C₁₋₆ haloalkyl, -C₀₋₃ alkylene-C₃₋₁₄ cycloalkyl and -C₀₋₃ alkylene-(3- to 14-membered heterocyclyl); wherein the amino, C₁₋₆ alkyl, C₁₋₆ haloalkyl or -C₀₋₃ alkylene-(3- to 14-membered heterocyclyl) is optionally further substituted by one or more R_{b}, wherein each R_{b} is independently selected from the group consisting of H, halogen, hydroxyl, cyano, C₁₋₆ alkyl, C₁₋₆ alkoxy, C₃₋₆ cycloalkyl and C₁₋₆ haloalkyl; or,
R¹ and R² together with the atom to which they are attached form 3- to 14-membered heterocyclyl, wherein the 3- to 14-membered heterocyclyl is optionally further substituted by one or more Rₐ;
R³ is selected from the group consisting of H, hydroxyl, C₁₋₆ hydroxyalkyl, amino, cyano, halogen, C₁₋₆ alkyl, C₁₋₆ haloalkyl, C₁₋₆ alkoxy, C₁₋₆ haloalkoxy, -C₀₋₃ alkylene-N(R_{c})₂, -C₀₋₃ alkylene-C₂₋₆ alkenyl, -C₀₋₃ alkylene-C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl and -C₀₋₃ alkylene-(5- to 18-membered heteroaryl); wherein the C₁₋₆ hydroxyalkyl, amino, C₁₋₆ alkyl, C₁₋₆ haloalkyl, C₁₋₆ alkoxy, C₁₋₆ haloalkoxy, -C₀₋₃ alkylene-N(R_{c})₂, -C₀₋₃ alkylene-C₂₋₆ alkenyl, -C₀₋₃ alkylene-C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl or -C₀₋₃ alkylene-(5- to 18-membered heteroaryl) is optionally further substituted by one or more Rₐ, or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
each R_{b} is independently selected from the group consisting of H, halogen, hydroxyl, oxo, amino, cyano, C₁₋₆ alkyl, C₁₋₆ alkoxy, C₃₋₆ cycloalkyl, C₁₋₆ haloalkoxy and C₁₋₆ haloalkyl; wherein the C₁₋₆ alkyl, C₁₋₆ alkoxy, C₃₋₆ cycloalkyl, C₁₋₆ haloalkoxy or C₁₋₆ haloalkyl is optionally further substituted by one or more H, C₁₋₆ alkoxy, -N(C₁₋₃ alkyl)₂; and
each R_{c} is independently selected from the group consisting of H, hydroxyl, halogen, C₁₋₆ alkyl, C₃₋₆ cycloalkyl and C₁₋₆ haloalkyl.

The compound of formula (I) of the present application can be synthesized according to the general method in the above synthetic scheme.

### BRIEF DESCRIPTION OF THE DRAWINGS

FIG. 1 shows the H-H NOESY spectrum of compound 1-6A.
FIG. 2 shows the absolute configuration diagram of the molecule of compound 83 from single crystal X-ray diffraction.
FIG. 3 shows the three-dimensional structural ellipsoid diagram of the molecule of compound 83 from single crystal X-ray diffraction.
FIG. 4 shows the projection diagram of the unit cell packing of compound 83 along the c-axis from single crystal X-ray diffraction.

### EXAMPLES

To make the above content clearer and more explicit, the present application will further illustrate the technical solutions of the present application with the following examples. The following examples are only used to illustrate specific embodiments of the present application, so that those skilled in the art can understand the present application, but are not intended to limit the scope of protection of the present application. In the specific embodiments of the present application, unless otherwise specified, technical means or methods are conventional technical means or methods in the art.

Unless otherwise specified, all temperatures in the present application refer to degrees Celsius.

The following abbreviations are used in the examples:
EA: ethyl acetate;
PE: petroleum ether;
DCM: dichloromethane;
LiBH₄: lithium borohydride;
THF: tetrahydrofuran;
MeOH: methanol;
EtOH: ethanol;
DMSO: dimethyl sulfoxide;
DMF: N,N-dimethylformamide;
ACN: acetonitrile;
TFA: trifluoroacetic acid;
TEA: triethylamine;
DIEA: N,N-diisopropylethylamine;
[Ir(COD)Cl]₂: bis(1,5-cyclooctadiene)diiridium(I) dichloride;
Dtbpy: 4,4'-di-tert-butyl-2,2'-bipyridine;
Pd₂(dba)₃: tris(dibenzylideneacetone)dipalladium(0);
Xphos: 2-dicyclohexylphosphino-2',4',6'-triisopropyl-1,1'-biphenyl;
Sphos: 2-dicyclohexylphosphino-2',6'-dimethoxybiphenyl;
cataCXium A Pd G3: [(di(1-adamantyl)-n-butylphosphine)-2-(2'-amino-1,1'-biphenyl)] palladium(II) methanesulfonate;
AgOTf: silver trifluoromethanesulfonate;
TBDPSCl: tert-butyldiphenylchlorosilane;
PdCl₂(dppf): [1,1'-bis(diphenylphosphino)ferrocene]dichloropalladium(II);
BINAP: 2,2'-bis(diphenylphosphino)-1,1'-binaphthyl;
NIS: N-iodosuccinimide;
B₂pin₂ or (Bpin)₂: bis(pinacolato)diboron;
HOBt: 1-hydroxybenzotriazole;
EDCI: 1-(3-dimethylaminopropyl)-3-ethylcarbodiimide;
TBAF: tetrabutylammonium fluoride;
DMAP: 4-dimethylaminopyridine;
HATU: N,N,N',N'-tetramethyl-O-(7-azabenzotriazol-1-yl)uranium hexafluorophosphate;
Noyori catalyst (ss): (S,S)-N-(p-toluenesulfonyl)-1,2-diphenylethane-1,2-diamine (p-cymene)ruthenium(II) chloride;
ZnCl₂: zinc chloride;
TsCl: p-toluenesulfonyl chloride;
CuBr₂: copper(II) bromide;
MeMgBr: methylmagnesium bromide.

### Synthesis of intermediate M1:

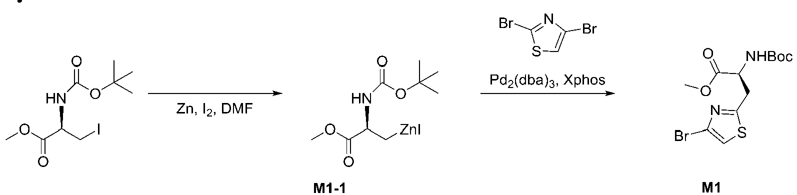

### Step 1: Synthesis of intermediate M1-1

Activated zinc powder (31.79 g), iodine (0.62 g), and one-third of methyl (R)-2-((tert-butoxycarbonyl)amino)-3-iodopropanoate (30.00 g) were added to N,N-dimethylformamide (150.00 mL). Then, under N₂ purge, the reaction mixture was heated to 40 °C and reacted for 1 h. Subsequently, two-thirds of methyl (R)-2-((tert-butoxycarbonyl)amino)-3-iodopropanoate (60.00 g) was added. The internal temperature was rapidly heated to 55 °C, and then the temperature was cooled to 40 °C and the mixture was reacted for 1 h. After standing and cooling, under N₂ protection, the supernatant was filtered through celite, and then the resultant was washed with N,N-dimethylformamide three times, 20 mL each time. The filtrates were combined to obtain 210 mL of a solution of M1-1 in N,N-dimethylformamide, which was used directly in the next reaction step.

### Step 2: Synthesis of intermediate M1

At room temperature, 2,4-dibromothiazole (70.93 g), Pd₂(dba)₃ (6.68 g), and Xphos (3.48 g) were added to a reaction flask. Then, under N₂ protection, 210 mL of the solution of M1-1 in N,N-dimethylformamide was slowly added dropwise. The mixture was purged with N₂ three times, heated to 70 °C and reacted for 2 h. The reaction solution was slowly added to 1 L of saturated ammonium chloride, then extracted with EA three times, 500 mL each time, washed once with 500 mL of saturated sodium chloride, dried over anhydrous sodium sulfate, and the filtrate was concentrated to obtain a crude product. The crude product was purified by column chromatography (PE:EA=95:5 to 80:20) to obtain the target intermediate M1 (47.64 g, yield 53.60%). ESI-MS m/z: 365.20, 367.22 [M+H]⁺.

### Synthesis of intermediate M2:

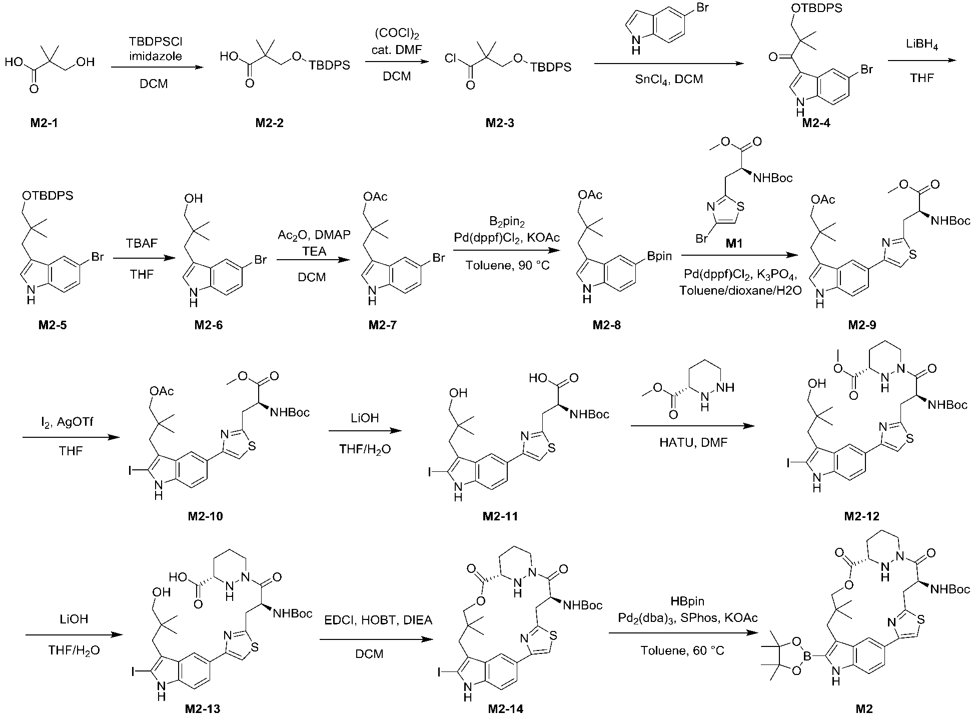

### Step 1: Synthesis of compound M2-2

At 0 °C, TBDPSCl (244 g) was added dropwise to a solution of compound M2-1 (100 g) and imidazole (115 g) in DCM (2.5 L). The system was then stirred for 1 h at room temperature. After the reaction was complete, hydrochloric acid solution (2 N, 2.5 L) was added to the system to neutralize the aqueous phase to acidity. The resultant was then extracted with an appropriate amount of DCM twice. The organic phases were combined and washed three times with sodium hydroxide solution (1 N, 3 L each time), dried over anhydrous sodium sulfate, and concentrated. The concentrate was the target compound M2-2 (309 g, 102% yield, crude product).

### Step 2: Synthesis of compound M2-3

At room temperature, DMF (0.43 mL) was added to a solution of compound M2-2 (200 g) in DCM (600 mL). Then, oxalyl chloride (76 mL) was slowly added dropwise to the stirred system. The reaction mixture was transferred to a 40 °C oil bath and stirred for 2 h. After the reaction was complete, the reaction system was concentrated to obtain compound M2-3 (210 g, 99% yield), which was used directly in the next reaction step.

### Step 3: Synthesis of compound M2-4

Under a nitrogen atmosphere at 0 °C, SnCl₄ (560 mL, 1 M/THF) was added dropwise to a solution of compound M2-3 (210 g) in dichloromethane (800 mL). The reaction solution was transferred to a -10 °C cold bath and stirred for 30 min. Then, a solution of 5-bromoindole (109.8 g) in DCM (560 mL) was added dropwise to the reaction system, and the reaction solution was transferred to a -10 °C cold bath and stirred for an additional 15 min. After the reaction was complete, a saturated sodium bicarbonate solution was added to the system to quench the reaction and neutralize the system to neutrality. The reaction system was stirred for another 10 min, then concentrated to remove dichloromethane. An appropriate amount of ethyl acetate was added for extraction and liquid separation. The aqueous phase was extracted twice more with ethyl acetate. The organic phases were combined and washed once with saturated brine, dried over anhydrous sodium sulfate, and concentrated. The concentrate was slurried with ethyl acetate as a solvent to obtain the target compound M2-4 (200 g, 67% yield).

### Step 4: Synthesis of compound M2-5

Under a nitrogen atmosphere at 0 C, LiBH₄ (23.7 g) was added in batches to a solution of compound M2-4 (194 g) in tetrahydrofuran (2 L). The reaction was then placed in a 66 °C oil bath and stirred for 20 h. After the reaction was complete, methanol was added dropwise to quench the reaction. Then, saturated brine and ethyl acetate were added for extraction and liquid separation. The organic phase was dried over anhydrous sodium sulfate and concentrated. The concentrate was purified by column chromatography (eluting with 0 to 10% EA/PE) to obtain the target compound M2-5 (145 g, 77% yield).

### Step 5: Synthesis of compound M2-6

TBAF (1 M, 500 mL) was added to a solution of compound M2-5 (100 g) in THF (500 mL). The reaction system was then placed in a 50 °C oil bath and stirred for 12 h. After the reaction was complete, the reaction system was concentrated, then EA and water were added for extraction and liquid separation. The organic phase was washed twice more with water. The organic phases were combined and dried over anhydrous sodium sulfate and concentrated. The concentrate was purified by column chromatography (eluting with 0 to 35% EA/PE) to obtain the target compound M2-6 (54 g, 100% yield).

### Step 6: Synthesis of compound M2-7

At 0 °C, acetic anhydride (18 mL) was added dropwise to a solution of compound M2-6 (54 g), triethylamine (80 mL), and DMAP (1.2 g) in dichloromethane (300 mL). The mixture was stirred in a 0 °C ice bath for 10 min. After the reaction was complete, an appropriate amount of water was added to the reaction system. After extraction and liquid separation, the organic phase was washed twice with water, dried over anhydrous sodium sulfate, and concentrated to obtain compound M2-7 (62 g, 100% yield), which was used directly in the next reaction step.

### Step 7: Synthesis of compound M2-8

Compound M2-7 (40 g), (Bpin)₂ (78.3 g), potassium acetate (30.3 g), PdCl₂(dppf) (9.0 g), and 400 mL of toluene were added to a 1 L three-necked flask. The flask was purged with nitrogen three times and then transferred to a 90 °C oil bath and stirred for 2 h. After the reaction was complete, the reaction system was concentrated. The concentrate was purified by column chromatography (eluting with 0 to 25% EA/PE) to obtain the target compound M2-8 (36 g, 78% yield). ESI-MS m/z: 372.3 [M+H]⁺.

### Step 8: Synthesis of compound M2-9

Compound M2-8 (36 g), intermediate M1 (47 g), potassium phosphate (51.5 g), PdCl₂(dppf) (7.1 g), and 210 mL of toluene, 70 mL of dioxane, and 70 mL of water were added to a 1 L three-necked flask. The flask was purged with nitrogen three times and then transferred to a 70 C oil bath and stirred for 20 h. After the reaction was complete, an appropriate amount of water and EA were added to the reaction system. After extraction and liquid separation, the organic phase was washed once with saturated brine, dried over anhydrous sodium sulfate, and concentrated. The concentrate was purified by column chromatography (eluting with 20 to 35% EA/PE) to obtain the target compound M2-9 (40 g, 78% yield). ESI-MS m/z: 530.3 [M+H]⁺.

### Step 9: Synthesis of compound M2-10

At 0 °C, AgOTf (23.3 g) was added to a solution of compound M2-9 (40 g) in tetrahydrofuran (400 mL). Then, a solution of iodine (19.2 g) in tetrahydrofuran (80 mL) was added dropwise. The mixture was stirred in a 0 °C ice bath for 10 min. After the reaction was complete, an appropriate amount of sodium thiosulfate solution was added to quench the reaction. An appropriate amount of ethyl acetate was added to the system for extraction and liquid separation. The organic phase was washed once each with water and saturated brine, dried over anhydrous sodium sulfate, and concentrated. The concentrate was purified by column chromatography (eluting with 0 to 30% EA/PE) to obtain the target compound M2-10 (25 g, 51% yield). ESI-MS m/z: 656.3 [M+H]⁺.

### Step 10: Synthesis of compound M2-11

At 0 °C, lithium hydroxide (2.7 g) was added to a solution of compound M2-10 (25 g) in tetrahydrofuran (180 mL)/water (60 mL). The mixture was transferred to room temperature and stirred for 12 h. After the reaction was complete, an appropriate amount of concentrated hydrochloric acid was added to adjust the pH of the reaction system to about 5. Then, an appropriate amount of ethyl acetate was added for extraction and liquid separation. The organic phase was washed once each with water and saturated brine, dried over anhydrous sodium sulfate, and concentrated to obtain the target compound M2-11 (23 g, 100% yield). ESI-MS m/z: 600.3 [M+H]⁺.

### Step 11: Synthesis of compound M2-12

At 0 °C, DIEA (25 mL) was added to a solution of compound M2-11 (26 g), methyl (S)-hexahydropyridazine-3-carboxylate(10.2 g), and HATU (19.8 g) in DMF (130 mL). The reaction solution was transferred to room temperature and stirred for 2 h. After the reaction was complete, an appropriate amount of EA and water were added to the system for extraction and liquid separation. The aqueous phase was extracted twice more with an appropriate amount of EA. The organic phases were combined and washed once with saturated brine, dried over anhydrous sodium sulfate, and concentrated. The concentrate was purified by column chromatography (eluting with 60% EA/PE) to obtain the target compound M2-12 (25 g, 79% yield). ESI-MS m/z: 726.4 [M+H]⁺.

### Step 12: Synthesis of compound M2-13

At 0 °C, an aqueous solution (80 mL) of lithium hydroxide (2.1 g) was added dropwise to a solution of compound M2-12 (26 g) in THF (240 mL). The reaction solution was transferred to room temperature and stirred for 30 min. After the reaction was complete, an appropriate amount of concentrated hydrochloric acid was added to adjust the pH of the reaction system to about 6. Then, an appropriate amount of ethyl acetate was added for extraction and liquid separation. The organic phase was washed once each with water and saturated brine, dried over anhydrous sodium sulfate, and concentrated to obtain the target compound M2-13 (25 g, 99% yield). ESI-MS m/z: 712.5 [M+H]⁺.

### Step 13: Synthesis of compound M2-14

At 0 °C, EDCI (27.2 g) was added to a solution of compound M2-13 (25 g), HOBt (14.2 g), and DIEA (35 mL) in dichloromethane (2 L). The reaction solution was transferred to room temperature and stirred for 4 h. After the reaction was complete, water was added to the system for extraction and liquid separation. The organic phase was washed once each with water and saturated brine, dried over anhydrous sodium sulfate, and concentrated. The concentrate was purified by column chromatography (eluting with 0 to 50% EA/PE) to obtain the target compound M2-14 (18.3 g, 75% yield). ESI-MS m/z: 694.4 [M+H]⁺.

### Step 14: Synthesis of compound M2

Compound M2-14 (10 g), Sphos (1.8 g), Pd₂(dba)₃ (1.6 g), potassium acetate (5.0 g), and 100 mL of toluene were added to a 250 mL three-necked flask. The flask was then purged with nitrogen three times and transferred to a 0 °C ice bath for stirring. Subsequently, pinacolborane (15.7 mL) was added to the reaction system, and the reaction system was then placed in a 60 °C oil bath and stirred for 4 h. After the reaction was complete, an appropriate amount of saturated ammonium chloride was added to the reaction system to quench the reaction. Then, EA was added for extraction and liquid separation. The organic phase was washed twice with water and once with saturated brine, dried over anhydrous sodium sulfate, and concentrated. The concentrate was purified by column chromatography (eluting with 0 to 40% EA/PE) to obtain the target compound M2 (9.7 g, 97% yield). ESI-MS m/z: 694.5 [M+H]⁺.

### Synthesis of intermediate M3:

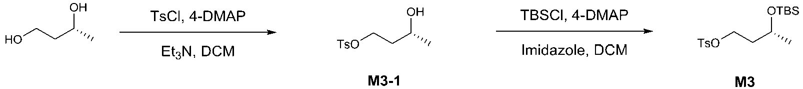

### Step 1: Synthesis of compound M3-1

Compound (S)-(+)-1,3-butanediol (5.00 g), triethylamine (11.60 mL), and 4-DMAP (0.14 g) were sequentially added to a solution of DCM (60.00 mL). Under N₂ protection, the temperature was cooled to -20 °C. A solution of TsCl (11.63 g) dissolved in DCM (60.00 mL) was slowly added dropwise over 2 h. The mixture was then maintained at -25 to -20 °C and reacted for 3 h, then heated to room temperature and reacted for 18 h. After the reaction was complete, 100 mL of water was added to the reaction solution to quench the reaction. The mixture was then extracted three times with 50 mL of DCM and washed twice with 50 mL of saturated sodium chloride. The organic phases were combined and concentrated to dryness to obtain a crude product. The crude product was separated by column chromatography (PE:EA=95:5 to 80:20) to obtain a colorless liquid, which was the target compound M3-1 (9.50 g, 70.09% yield). ESI-MS m/z: 245.32 [M+H]⁺.

### Step 2: Synthesis of compound M3

At room temperature, compound M3-1 (9.50 g), 4-DMAP (0.24 g), and imidazole (6.62 g) were added to DCM (80.00 mL). Then, under N₂ protection, tert-butyldimethylchlorosilane (8.79 g) was slowly added, and the mixture was stirred and reacted at room temperature for 2 h. After the reaction was complete, 100 mL of water was added to the reaction solution to quench the reaction. The mixture was then extracted with DCM (50 mL * 3), washed with saturated sodium chloride (50 mL * 2), and the organic phases were combined and concentrated to obtain a crude product. The crude product was separated by column chromatography (PE:EA=95:5 to 80:20) to obtain a colorless liquid, which was the target compound M3 (13.80 g, 98.97% yield). ESI-MS m/z: 359.43 [M+H]⁺.

### Synthesis of intermediate M4:

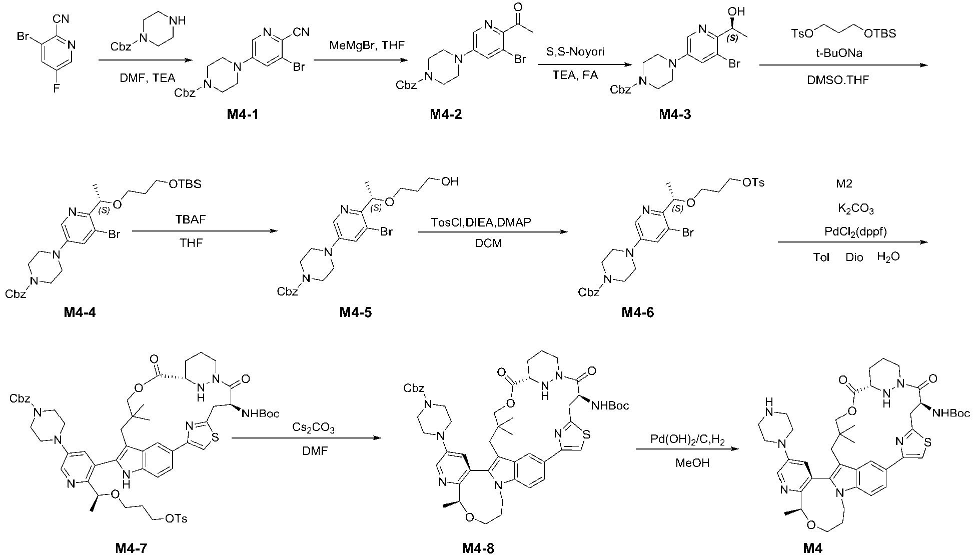

### Step 1: Synthesis of compound M4-1

3-Bromo-5-fluoropicolinonitrile (25.00 g, 124.38 mmol), benzyl piperazine-1-carboxylate (30.14 g, 136.82 mmol), and DMF (150.00 mL) were added to a 500 mL three-necked flask. Under nitrogen protection, triethylamine (25.93 mL, 186.57 mmol) was added dropwise at 0 °C. The reaction was carried out at room temperature for 2 h, then poured into ice water, stirred for 30 min, filtered with suction, washed with water, and suction dried. The solid was slurried with PE to obtain compound M4-1 (46.50 g, 93.17%). ESI-MS m/z: 402.23 [M+H]⁺.

### Step 2: Synthesis of compound M4-2

Compound M4-1 (46.50 g, 115.89 mmol) and tetrahydrofuran (465.00 mL) were added to a 1 L three-necked flask. Under nitrogen protection, methylmagnesium bromide (108.16 mL, 3.00 mol/L, 324.48 mmol) was added dropwise at -25 °C. After the addition was complete, the reaction was carried out at -20 °C for 2 h, then poured into ice-cold aqueous ammonium chloride solution. The pH was adjusted to 3 with 1 N HCl, stirred for 10 min, adjusted to pH 8 with sodium carbonate. The mixture was extracted with EA, washed with brine, dried, dried by rotary evaporation, and separated by column chromatography (PE:EA=4:1) to obtain compound M4-2 (44.00 g, 90.77%). ESI-MS m/z: 419.26 [M+H]⁺.

### Step 3: Synthesis of compound M4-3

Triethylamine (23.26 mL, 167.35 mmol) was added to a 100 mL three-necked flask. Under nitrogen protection, formic acid (7.70 g, 167.35 mmol) was added dropwise at 0 °C. After the addition was complete, Noyori catalyst (ss) (1.06 g, 1.67 mmol) was added. The mixture was stirred at 0 °C for 10 min, then compound M4-2 (14.00 g, 33.47 mmol) was added. The reaction was carried out at room temperature for 48 h, then dried by rotary evaporation, added EA, washed with brine, dried, and dried by rotary evaporation. The residue was separated by column chromatography (PE:EA=2:1) to obtain compound M4-3 (14.00 g, 75.00%). ESI-MS m/z: 421.32 [M+H]⁺.

### Step 4: Synthesis of compound M4-4

Compound M4-3 (4000.00 mg, 9.52 mmol), 3-((tert-butyldimethylsilyl)oxy)propyl 4-methylbenzenesulfonate (9837.02 mg, 28.55 mmol), DMSO (100.00 mL), and THF (20.00 mL) were added sequentially to a 250 mL three-necked flask. The mixture was cooled in an ice-water bath, and sodium tert-butoxide (2286.46 mg, 23.79 mmol) was added in batches. The ice-water bath was removed, and the mixture was stirred and reacted at room temperature. After the reaction was complete as monitored by LC-MS, the reaction solution was added to a mixture of ethyl acetate and cold saturated aqueous ammonium chloride solution to quench. The mixture was then extracted with ethyl acetate. The organic phases were combined and washed with brine, separated, and concentrated to obtain a crude product. The crude product was purified by silica gel column chromatography (PE to PE/EA=75/25) to obtain the target compound M4-4 (2630 mg, slightly yellow oil). ESI-MS m/z: 592.27, 594.27 [M+H]⁺.

### Step 5: Synthesis of compound M4-5

Compound M4-4 (6159.00 mg, 10.39 mmol) and tetrabutylammonium fluoride (50.00 mL, 1.0 mol/L in THF) were added to a 250 mL single-necked flask. Under nitrogen protection, the reaction was carried out at room temperature overnight. After the reaction was complete as monitored by LC-MS, the reaction solution was diluted with ethyl acetate, washed sequentially with saturated aqueous sodium bicarbonate solution, saturated aqueous ammonium chloride solution and brine, then separated. The organic phase was concentrated to obtain a crude product. The crude product was purified by silica gel column chromatography (PE/EA=90/10 to 75/25) to obtain the target product M4-5 (4820 mg, light brown-yellow viscous substance). ESI-MS m/z: 478.09, 480.06 [M+H]⁺.

### Step 6: Synthesis of compound M4-6

Compound M4-5 (4820.00 mg, 10.08 mmol), DCM (75.00 mL), DIEA (4.16 mL, 25.19 mmol), and DMAP (123.10 mg, 1.01 mmol) were added sequentially in a 250 mL single-necked flask. TosCL (2305.07 mg, 12.09 mmol) was added in batches, and the mixture was stirred and reacted at room temperature. After 1 h of reaction, additional DMAP (1354.05 mg, 11.08 mmol) and TosCL (2305.07 mg, 12.09 mmol) were added, and the mixture was stirred and reacted. After the reaction was complete as monitored by LC-MS, the reaction solution was diluted with dichloromethane, washed sequentially with saturated aqueous sodium bicarbonate solution, saturated aqueous ammonium chloride solution and brine, then separated. The organic phase was concentrated to obtain a crude product. The crude product was purified by silica gel column chromatography (PE/EA=90/10 to 75/25) to obtain the target compound M4-6 (4650 mg, slightly yellow-brown viscous substance). ESI-MS m/z: 632.16, 634.14 [M+H]⁺.

### Step 7: Synthesis of compound M4-7

Compound M4-6 (4650.00 mg, 7.35 mmol), M2 (5099.10 mg, 7.35 mmol), toluene (75.00 mL), dioxane (25.00 mL), water (25.00 mL), K₂CO₃ (2539.95 mg, 18.38 mmol), and [PdCl₂(dppf)] CH₂Cl₂ (600.31 mg, 0.74 mmol) were added sequentially in a 250 mL three-necked flask. Under nitrogen protection, the mixture was heated in an oil bath and reacted at 60 °C to 65 °C. After the reaction was complete as monitored by LC-MS, the reaction solution was diluted with ethyl acetate, washed with saturated aqueous sodium bicarbonate solution and saturated aqueous ammonium chloride solution, then separated. The organic phase was concentrated to obtain a crude product. The crude product was purified by silica gel column chromatography (DCM/MeOH=500/15) to obtain the target compound M4-7 (8320 mg, slightly yellow foamy solid).

### Step 8: Synthesis of compound M4-8

Compound M4-7 (8228.00 mg, 7.35 mmol), DMF (100.00 mL), and Cs₂CO₃ (4790.01 mg, 14.70 mmol) were added sequentially in a 500 mL single-necked flask. Under nitrogen protection, the mixture was heated in an oil bath and reacted at 60-65 °C. After the reaction was complete as monitored by LC-MS, the reaction mixture was filtered, and the filtrate was collected. The collected filtrate was cooled in an ice-water bath, and ice water was added dropwise, an off-white solid powder precipitated. Filtration was performed, and the filter cake was collected, dissolved in ethyl acetate, washed sequentially with water and brine, then separated. The organic phase was concentrated to obtain a crude product. The crude product was purified by silica gel column chromatography (DCM/MeOH=50/1) to obtain the target compound M4-8 (6760 mg, slightly yellow foamy solid). ESI-MS m/z: 947.61 [M+H]⁺.

### Step 9: Synthesis of compound M4

Compound M4-8 (6760.00 mg, 7.14 mmol) and MeOH (110.00 mL) were added sequentially in a 500 mL single-necked flask. The flask was purged with nitrogen, then Pd(OH)₂/C (4009.14 mg, 10%, 2.85 mmol) was added. After purging with nitrogen again, the atmosphere was purged with hydrogen using a hydrogen balloon, and the reaction was carried out at room temperature under a hydrogen atmosphere. After the reaction was complete as monitored by LC-MS, the reaction mixture was filtered. The filtrate was collected and concentrated to dryness, and purified by silica gel column chromatography (DCM to DCM/MeOH=85/15) to obtain the target compound M4 (4270 mg, beige foamy solid). ESI-MS m/z: 813.54 [M+H]⁺.

### Synthesis of intermediate M5:

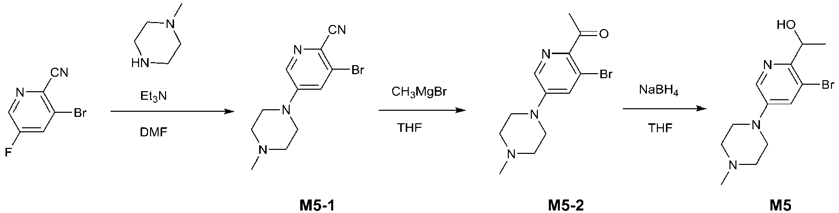

### Step 1: Synthesis of compound M5-1

3-Bromo-5-fluoropicolinonitrile (25.00 g) and N-methylpiperazine (13.70 g) were sequentially added to DMF (125.00 mL). Then, under N₂ protection and ice-water bath conditions, triethylamine (22.50 mL) was slowly added in batches. The reaction was carried out at room temperature for 2 h. After the reaction was complete, the reaction solution was slowly added to 500 mL of ice water in batches, a white solid precipitated. The solid was then slurried with water (500 mL * 3), filtered, and dried under vacuum to obtain the target compound M5-1 (30.60 g, 87.50% yield). ESI-MS m/z: 281.2, 283.2 [M+H]⁺.

### Step 2: Synthesis of compound M5-2

Compound M5-1 (30.60 g) was added to THF (300.00 mL). Then, under N₂ protection, the temperature was maintained at -25 to -15 °C. Methylmagnesium bromide (108.90 mL, 3.00 mol/L) was slowly added in batches, and the mixture was maintained at -20 °C to -10 °C and reacted for 2 h. After the reaction was complete, the reaction solution was slowly added to 800 mL of 1M HCl to quench the reaction. Then, saturated sodium bicarbonate was added to adjust the pH to 8 to 9. The mixture was extracted with DCM (200 mL * 3). The organic phases were combined and washed with saturated sodium chloride and concentrated to obtain a crude product. The crude product was purified by column chromatography (DCM:MeOH=97:3 to 88:12) to obtain the target compound M5-2 (17.90 g, 55.16% yield). ESI-MS m/z: 298.2, 300.2 [M+H]⁺.

### Step 3: Synthesis of compound M5

Compound M5-2 (7.80 g) was added to THF (80.00 mL) and MeOH (20.00 mL). Then, under N₂ protection and ice-water bath conditions, NaBH₄ (1.29 g) was slowly added in batches. The mixture was reacted for 1 h in the ice-water bath while maintaining the temperature at 0 °C to 10 °C. After the reaction was complete, 100 mL of 1M HCl was added to the reaction solution to quench the reaction (approximately 25% of the complex was quenched). The mixture was then heated to reflux to dissociate the target product. Saturated sodium carbonate solution was added to adjust the pH to 9 to 10. The mixture was then extracted with EA (60 mL * 4). The organic phases were combined and washed with saturated sodium chloride and concentrated to obtain a crude product. The crude product was purified by column chromatography (DCM:MeOH=97:3 to 88:12) to obtain the target compound M5 (7.00 g, 89.14% yield). ESI-MS m/z: 300.2, 302.2 [M+H]⁺.

### Synthesis of intermediate M6:

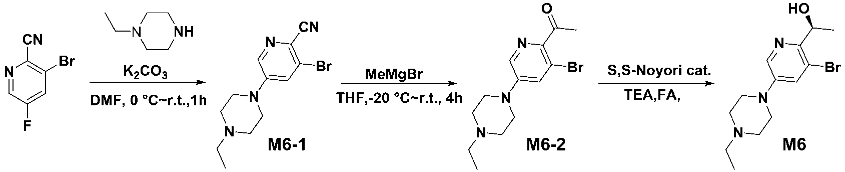

### Step 1: Synthesis of compound M6-1

Compound 3-bromo-5-fluoropicolinonitrile (99.0 g, 492.5 mmol) and potassium carbonate (102.1 g, 738.8 mmol) were added to 600 mL of DMF. The mixture was cooled to 0 °C, then ethylpiperazine (67.5 g, 591.1 mmol) was slowly added. The reaction was carried out at room temperature for 1 h. After the reaction was complete as monitored by LC-MS, the reaction solution was poured into 3 L of crushed ice. A solid precipitated out under stirring. The mixture was filtered, and the solid was washed with water (500 mL * 3). The solid was then dissolved in 1000 mL of DCM, washed three times with brine, dried over anhydrous sodium sulfate, and concentrated. The concentrate was used directly in the next reaction step. The target compound M6-1 was obtained (132.9 g). ESI-MS m/z: 295.05, 297.05 [M+H]⁺.

### Step 2: Synthesis of compound M6-2

Compound M6-1 (134.0 g, 453.9 mmol) was dissolved in 2100 mL of tetrahydrofuran and cooled to -30 °C. Then, MeMgBr (453 mL, 1362 mmol) was added dropwise. After the addition was complete, the mixture was maintained at 0 °C to 5 °C and reacted for 4 h. After the reaction was complete as monitored by LC-MS, the reaction solution was added to 2.5 L of 1N dilute hydrochloric acid to quench, and stirred for half an hour. Solid sodium bicarbonate was then added to adjust the pH of the system to 7-8. The mixture was extracted with ethyl acetate (1000 mL * 2). The organic phases were combined and washed with saturated brine, dried over anhydrous sodium sulfate, and concentrated to obtain a crude product, which was the target compound M6-2 (132.0 g). This was used directly in the next reaction step. ESI-MS m/z: 312.02, 314.11 [M+H]⁺.

### Step 3: Synthesis of compound M6

Triethylamine (868 mL, 6246 mmol) was added to a 2000 mL three-necked flask under nitrogen protection and cooled to 0 °C. Formic acid (31.4 g, 832 mmol) was then slowly added. Then, (S,S)-N-(p-toluenesulfonyl)-1,2-diphenylethane-1,2-diamine (p-cymene)ruthenium(II) chloride (10.5 g, 16.6 mmol) was added. The mixture was reacted at 40 °C for 15 min, then cooled to room temperature. Compound M6-2 (130 g, 416 mmol) was added, and the reaction was carried out at room temperature for 64 h. After the reaction was complete as monitored by LC-MS, the reaction solution was diluted with DCM, washed sequentially with water and brine, separated, and concentrated to obtain a crude product. The crude product was purified by silica gel column chromatography to obtain the target compound M6 (109 g). ESI-MS m/z: 314.03, 316.07 [M+H]⁺.

### Synthesis of intermediate M7:

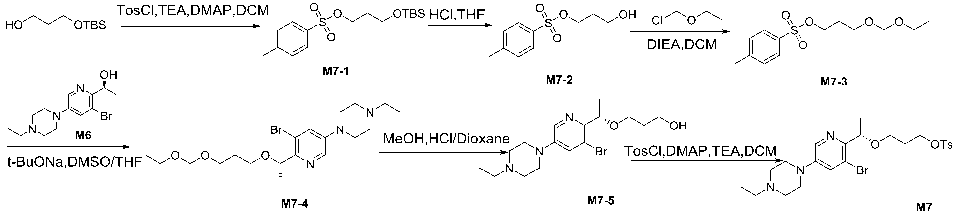

### Step 1: Synthesis of compound M7-1

Compound 3-((tert-butyldimethylsilyl)oxy)propan-1-ol (200.00 g, 1050.67 mmol), dichloromethane (1000.00 mL), triethylamine (292.08 mL, 2101.34 mmol), and 4-dimethylaminopyridine (6.42 g, 52.53 mmol) were added sequentially to a 3 L three-necked flask. p-Toluenesulfonyl chloride (210.32 g, 1103.20 mmol) was added in batches at 0 °C, and the mixture was stirred at room temperature for 5 h. After the reaction was complete as monitored by TLC, the reaction solution was diluted with dichloromethane and poured into saturated NH₄Cl solution to quench. The organic layer was washed with water and saturated brine, and finally dried over anhydrous sodium sulfate. The resultant was concentrated under reduced pressure, cooled, and solid impurities precipitated. Petroleum ether was added, and the mixture was filtered. The filter cake was rinsed with petroleum ether. The filtrate was concentrated under reduced pressure to obtain the target product M7-1 (327.00 g).

### Step 2: Synthesis of compound M7-2

Compound M7-1 (367.00 g, 1065.18 mmol) and tetrahydrofuran (1000.00 mL) were added sequentially to a 3 L three-necked flask. Concentrated hydrochloric acid (44.38 mL, 12.00 mol/L, 532.59 mmol) was added dropwise at 0 °C. The mixture was stirred at room temperature for 1 h. After the reaction was complete as monitored by LC-MS, the reaction solution was poured into ice water to quench, and extracted with ethyl acetate. The organic layer was washed with saturated NaHCO₃ solution and saturated brine, dried over anhydrous sodium sulfate, concentrated under reduced pressure, and purified by column chromatography to obtain the target product M7-2 (180.00 g).

### Step 3: Synthesis of compound M7-3

Compound M7-2 (151.20 g, 656.59 mmol), dichloromethane (800.00 mL), and N,N-diisopropylethylamine (173.62 mL, 1050.54 mmol) were added sequentially to a 3 L three-necked flask. Under nitrogen protection, the temperature was cooled to 0 °C, and chloromethyl ethyl ether (97.47 mL, 1050.54 mmol) was added dropwise. The mixture was stirred at room temperature for 2 h. LC-MS monitored the starting material remained. Additional N,N-diisopropylethylamine (32.55 mL, 196.98 mmol) and chloromethyl ethyl ether (18.27 mL, 196.98 mmol) were added. The mixture was stirred at room temperature for 1 h. After the reaction was complete as monitored by LC-MS, the reaction solution was diluted with dichloromethane and poured into water to quench. The organic layer was washed with water and twice with saturated brine, then concentrated under reduced pressure. The residue was dissolved in ethyl acetate, washed three times with saturated brine, and dried over anhydrous sodium sulfate. Activated carbon (40 g) was added to the organic layer, and the mixture was stirred for 1 h. The mixture was filtered, and the filter cake was rinsed with ethyl acetate. The filtrate was concentrated under reduced pressure to obtain the target product M7-3 (169.1 g).

### Step 4: Synthesis of compound M7-4

Intermediate M6 (17.70 g, 56.33 mmol), dimethyl sulfoxide (130.00 mL), tetrahydrofuran (26.00 mL), and M7-3 (16.24 g, 56.33 mmol) were added sequentially to a 500 mL three-necked flask. At 10 °C, sodium tert-butoxide (8.12 g, 84.49 mmol) was added in batches, and the mixture was stirred at room temperature for 5 h. LC-MS monitoring showed remaining starting material. Additional M7-3 (21.12 g, 73.23 mmol) and sodium tert-butoxide (7.04 g, 73.23 mmol) were added. The mixture was stirred at room temperature for 12 h. After the reaction was complete as monitored by LC-MS, the reaction solution was poured into ice water to quench. The mixture was extracted twice with ethyl acetate. The organic layers were combined and washed three times with saturated brine, dried over anhydrous sodium sulfate, and concentrated under reduced pressure to obtain the target product M7-4 (30.0 g). ESI-MS m/z: 430.2 [M+H]⁺.

### Step 5: Synthesis of compound M7-5

Compound M7-4 (30.00 g, 69.71 mmol) and methanol (150.00 mL) were added sequentially to a 500 mL single-necked flask. At 0 °C, hydrochloric acid (4M in dioxane) (90.00 mL) was added, and the mixture was stirred at room temperature for 4 h. After the reaction was complete as monitored by LC-MS, the mixture was concentrated under reduced pressure. Water and dichloromethane were added for back-extraction. The aqueous layer was adjusted to pH 8 to 9 with saturated NaHCO₃ and extracted with dichloromethane. The organic layer was washed with saturated brine, dried over anhydrous sodium sulfate, and concentrated under reduced pressure to obtain the target product M7-5 (21.7 g). ESI-MS m/z: 372.2 [M+H]⁺.

### Step 6: Synthesis of compound M7

Compound M7-5 (20.70 g, 55.60 mmol), dichloromethane (100.00 mL), 4-dimethylaminopyridine (0.34 g, 2.78 mmol), and triethylamine (15.46 mL, 111.20 mmol) were added sequentially to a 500 mL three-necked flask. At 0 °C, p-toluenesulfonyl chloride (13.78 g, 72.28 mmol) was added in batches, and the mixture was stirred at room temperature for 4 h. After the reaction was complete as monitored by LC-MS, the reaction solution was diluted with dichloromethane, washed with water, and the organic layer was washed with saturated brine, dried over anhydrous sodium sulfate, concentrated under reduced pressure, and purified by column chromatography to obtain the target product M7 (21.9 g). ESI-MS m/z: 526.2 [M+H]⁺.

### Synthesis of intermediate M8:

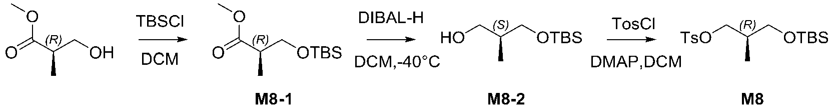

### Step 1: Synthesis of compound M8-1

Compound (-)-methyl D-β-hydroxyisobutyrate (5.00 g) and imidazole (5.76 g) were sequentially added to a solution of DCM (50.00 mL). Under N₂ protection, the temperature was cooled to 0 °C, and TBSCl (7.66 g) was slowly added dropwise. The reaction was then stirred at room temperature for 4 h. After the reaction was complete, 100 mL of ammonium chloride was added to the reaction solution to quench the reaction. The mixture was then extracted with ethyl acetate, washed with 50 mL of saturated sodium chloride, and dried by rotary evaporation. The crude product was purified by column chromatography to obtain a colorless liquid, which was the target compound M8-1 (9.70 g, 98.62% yield). ESI-MS m/z: 233.15 [M+H]⁺.

### Step 2: Synthesis of compound M8-2

Compound M8-1 (4.0 g) was dissolved in THF (40 mL). The temperature was cooled to -40 °C, and DIBAL-H (51.64 mL, 1M in n-hexane) was added dropwise. After the addition was complete, the mixture was stirred at -40 °C for 3 h. Water (20 mL) was added to quench the reaction. The aqueous phase was extracted with DCM. The organic phases were combined and dried over anhydrous sodium sulfate and purified by column chromatography to obtain the target compound M8-2 (2.28 g, 64.81% yield). ESI-MS m/z: 205.38 [M+H]⁺.

### Step 3: Synthesis of compound M8

At room temperature, compound M8-2 (2.28 g) and 4-DMAP (2.18 g) were added to DCM (80.00 mL). Then, under N₂ protection, tert-butyldimethylchlorosilane (2.76 g) was slowly added, and the reaction was carried out at room temperature overnight. After the reaction was complete, 100 mL of water was added to the reaction solution to quench the reaction. The mixture was then extracted three times with 50 mL of DCM and washed twice with 50 mL of saturated sodium chloride. The organic phases were combined and concentrated to obtain a crude product. The crude product was purified by column chromatography to obtain the target compound M8 (3.26 g, 81.50% yield). ESI-MS m/z: 359.57 [M+H]⁺.

### Synthesis of intermediate M9:

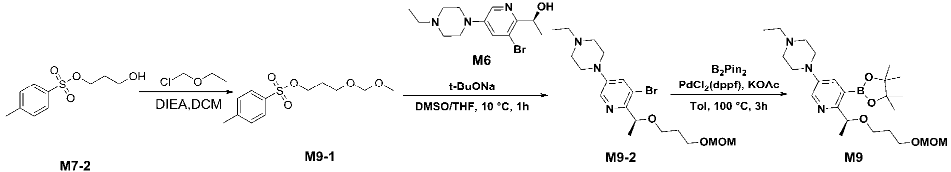

### Step 1: Synthesis of compound M9-1

Compound M7-2 (5.70 g, 24.75 mmol), dichloromethane (80.00 mL), and N,N-diisopropylethylamine (8.62 mL, 49.50 mmol) were added sequentially to a 3 L three-necked flask. Under nitrogen protection, the temperature was cooled to 0 °C, and chloromethyl methyl ether (2.23 mL, 29.70 mmol) was added dropwise. The mixture was stirred at room temperature for 5 h. After the reaction was complete as monitored by LC-MS, the reaction solution was diluted with dichloromethane, poured into water to quench, washed twice with saturated brine, and the organic layer was concentrated under reduced pressure. The residue was separated by column chromatography to obtain the target product M9-1 (3.6 g).

### Step 2: Synthesis of compound M9-2

Compound M6 (8.00 g, 26.65 mmol) and compound M9-1 (14.62 g, 53.30 mmol) were dissolved in DMSO (50.00 mL) and THF (10.00 mL). Under nitrogen protection, the temperature was cooled to 10 °C, then t-BuONa (6.40 g, 66.62 mmol) was added. The reaction was carried out at 10 °C for 2 h. After the reaction was complete as monitored by LC-MS, the reaction solution was diluted with ethyl acetate, washed sequentially with water and brine, separated, and concentrated to obtain a crude product. The crude product was purified by silica gel column chromatography to obtain the target compound M9-2 (14.1 g). ESI-MS m/z: 401.13, 402.22 [M+H]⁺.

### Step 3: Synthesis of compound M9

Compound M9-2 (6.00 g, 14.91 mmol), B₂Pin₂ (5.68 g, 22.37 mmol), [PdCl₂(dppf)] CH₂Cl₂ (1.22 g, 1.49 mmol), and potassium acetate (4.39 g, 44.74 mmol) were dissolved in toluene (60.00 mL). Under nitrogen protection, the mixture was reacted at 100 °C for 3 h. After the reaction was complete as monitored by LC-MS, the reaction solution was diluted with ethyl acetate, washed sequentially with water and brine, separated, and concentrated to obtain a crude product of the target compound M9 (13 g). ESI-MS m/z: 463.32, 464.33 [M+H]⁺.

### Synthesis of intermediate M10:

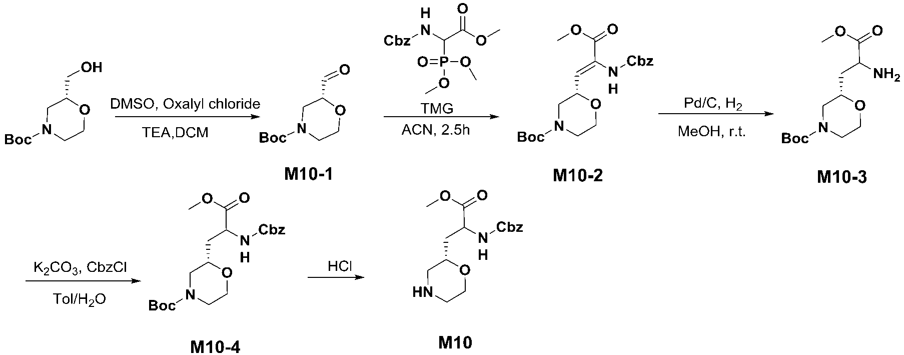

### Step 1: Synthesis of compound M10-1

DMSO (14.69 mL, 207.12 mmol) was dissolved in DCM (350.00 mL) and cooled to -78 °C. Then, a solution of oxalyl chloride (8.76 mL, 103.56 mmol) in DCM (20.00 mL) was slowly added (ensure temperature below -60 °C during addition). After the addition was complete, the mixture was reacted at -78 °C for 30 min. Then, a solution of (R)-N-Boc-2-hydroxymethylmorpholine in DCM (50.00 mL) was slowly added, and the reaction was continued at -78 °C for 2 h. Then, TEA (38.39 mL, 276.16 mmol) was slowly added (ensure temperature below -60 °C during addition), and the mixture was reacted at -78 °C for 30 min, then slowly heated to room temperature. The mixture was extracted with DCM and saturated aqueous sodium bicarbonate solution, and then extracted again with DCM and saturated aqueous sodium chloride solution. The organic layer was dried over anhydrous sodium sulfate and concentrated to obtain a crude product of the target compound M10-1 (16 g), which was used directly in the next reaction step.

### Step 2: Synthesis of compound M10-2

Compound M10-1 (14.50 g, 67.36 mmol) and compound (±)-trimethyl Z-α-phosphonoglycinate (26.78 g, 80.84 mmol) were dissolved in ACN (300.00 mL) and cooled to 10 °C. Then, TMG (16.94 mL, 134.73 mmol) was added. The mixture was reacted at 10 °C for 30 min, then heated to room temperature and reacted for 2 h. The mixture was directly concentrated and purified by wet-loading column chromatography to obtain the target compound M10-2 (26 g).

### Step 3: Synthesis of compound M10-3

Compound M10-2 (26.00 g, 61.84 mmol) was dissolved in MeOH (300.00 mL). Pd/C (6.58 g, 61.84 mmol) was added, followed by a few drops of HOAc (0.18 mL, 3.09 mmol) (to accelerate the reaction). The mixture was purged with hydrogen 5 times and reacted at 65 °C overnight. After the reaction was complete as monitored by LC-MS, the mixture was filtered and directly concentrated to obtain a crude product of the target compound M10-3 (24 g). ESI-MS m/z: 288.17, 289.29 [M+H]⁺.

### Step 4: Synthesis of compound M10-4

Compound M10-3 (24.00 g, 83.24 mmol) was dissolved in 1,4-dioxane (150.00 mL) and H₂O (150.00 mL) and cooled to 0 °C. Then, K₂CO₃ (34.51 g, 249.71 mmol) and CbzCl (17.57 mL, 124.85 mmol) were added. The mixture was reacted at room temperature for 3 h. After the reaction was complete as monitored by LC-MS, saturated ammonium chloride was added to quench, followed by extraction with EA and water. The organic layer was dried over anhydrous sodium sulfate and concentrated to obtain a crude product. The crude product was purified by silica gel column chromatography to obtain the target compound M10-4 (28 g). ESI-MS m/z: 422.21, 423.19 [M+H]⁺.

### Step 5: Synthesis of compound M10

Compound M10-4 (26.00 g, 61.54 mmol) was dissolved in DCM (80.00 mL). Then, HCl (4M /dioxane) (80.00 mL) was added, and the mixture was reacted at room temperature for 30 min. After the reaction was complete as monitored by LC-MS, the mixture was concentrated under reduced pressure. Sodium bicarbonate and DCM were added for extraction. The organic layer was dried over anhydrous sodium sulfate and concentrated to obtain a crude product. The crude product was purified by silica gel column chromatography to obtain the target compound M10 (12.8 g). ESI-MS m/z: 322.15, 323.18 [M+H]⁺.

### Synthesis of intermediate M11:

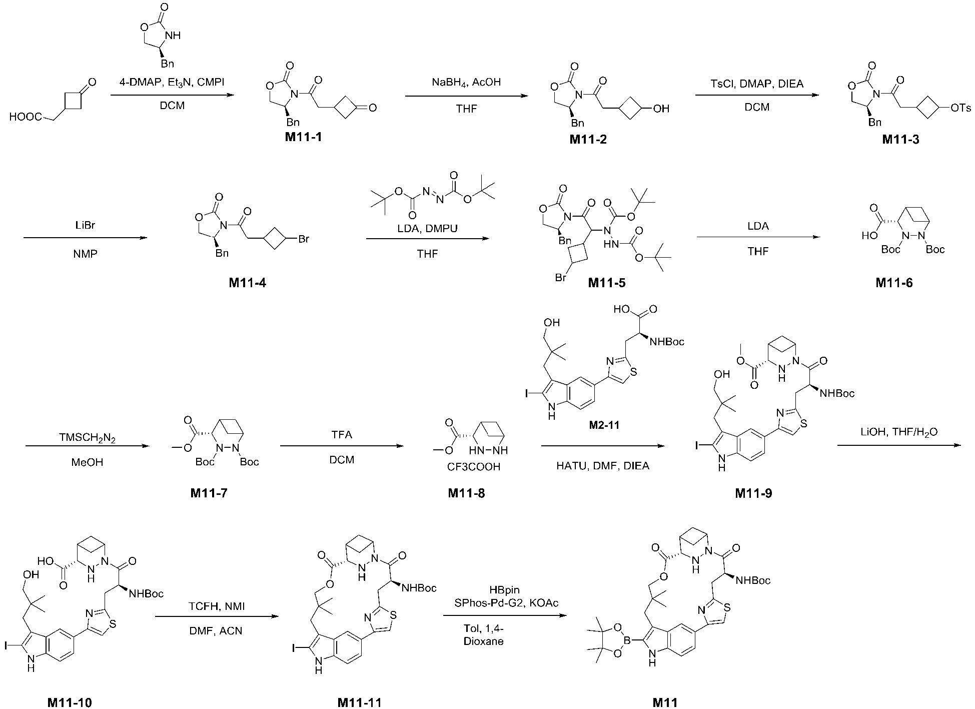

### Step 1: Synthesis of compound M11-1

2-(3-Oxocyclobutyl)acetic acid (4.5 g, 35.12 mmol), (S)-4-benzyl-2-oxazolidinone (7.47 g, 42.15 mmol), 4-dimethylaminopyridine (0.43 g, 3.51 mmol), triethylamine (14.65 mL, 105.36 mmol), and DCM (90.00 mL) were added sequentially to a 250 mL three-necked flask. Under nitrogen protection and ice-water bath cooling, 2-chloro-1-methylpyridinium iodide (15.25 g, 59.71 mmol) was slowly added in batches. The reaction was carried out at room temperature for 2 h. After the reaction was complete as monitored by LC-MS, the reaction solution was slowly added to 100 mL of water to quench. The mixture was then extracted with DCM (60 mL * 3). The organic phases were combined and washed with saturated sodium chloride, dried over anhydrous sodium sulfate, and concentrated to dryness to obtain a crude product of compound M11-1. The crude product was purified by silica gel column chromatography to obtain the target product M11-1 (10.00 g, pale yellow liquid). ESI-MS m/z: 288.42 [M+H]⁺.

### Step 2: Synthesis of compound M11-2

Compound M11-1 (10.00 g, 34.81 mmol), glacial acetic acid (3.98 mL, 69.61 mmol), and THF (90.00 mL) were added sequentially to a 250 mL three-necked flask. Under nitrogen protection and ice-water bath cooling, sodium borohydride (1.05 g, 27.84 mmol) was slowly added in batches. The reaction was carried out in the ice-water bath for 2.5 h. After the reaction was complete as monitored by LC-MS, saturated ammonium chloride (20 mL) was added dropwise to quench. The mixture was then extracted with EA (60 mL * 3), washed with saturated sodium bicarbonate. The organic phases were combined and concentrated to dryness to obtain a crude product. The crude product was purified by column chromatography to obtain the target product M11-2 (10.00 g, pale yellow liquid). ESI-MS m/z: 290.41 [M+H]⁺.

### Step 3: Synthesis of compound M11-3

Compound M11-2 (10.00 g, 34.56 mmol), DIEA (8.57 mL, 51.84 mmol), and DMAP (3.38 g, 27.65 mmol) were sequentially added to an anhydrous DCM (90.00 mL) solution. Under N₂ protection, the temperature was cooled to 0 °C, and TsCl (7.91 g, 41.48 mmol) was slowly added. The mixture was then heated to room temperature and reacted for 3 h. After the reaction was complete, 100 mL of water was added to the reaction solution to quench. The mixture was then extracted with DCM (50 mL * 3) and washed with saturated sodium chloride (50 mL * 2). The organic phases were combined and concentrated to obtain a crude product. The crude product was purified by silica gel column chromatography to obtain the target product M11-3 (14.10 g, pale yellow liquid). ESI-MS m/z: 444.52 [M+H]⁺.

### Step 4: Synthesis of compound M11-4

Compound M11-3 (14.00 g, 31.56 mmol), lithium bromide (5.47 g, 63.12 mmol), and NMP (140.00 mL) were added sequentially to a 250 mL single-necked flask. Under nitrogen protection, the mixture was reacted at 90 °C for 3 h. After the reaction was complete as monitored by LC-MS, the reaction solution was slowly added to 60 mL of ice water to quench. The mixture was then extracted with EA (50 mL * 3). The organic phases were combined and washed with saturated sodium chloride, dried over anhydrous sodium sulfate, and concentrated to dryness to obtain a crude product of compound M11-4. The crude product was purified by silica gel column chromatography to obtain the target product M11-4 (11.00 g, pale yellow liquid). ESI-MS m/z: 353.24, 355.21 [M+H]⁺.

### Step 5: Synthesis of compound M11-5

Compound M11-4 (7.50 g, 21.29 mmol), N,N-dimethylpropyleneurea (5.15 mL, 42.59 mmol), and THF (120.00 mL) were added sequentially to a 250 mL three-necked flask. Under nitrogen protection at -78 °C, LDA (2M in THF/hexane) (13.84 mL, 27.68 mmol) was slowly added dropwise. The temperature was maintained and reacted for 1 h. Then, a solution of di-tert-butyl azodicarboxylate (5.88 g, 25.55 mmol) in THF (10.00 mL) was added. The reaction was continued at -78 to -65 °C for 2 h. After the reaction was complete as monitored by LC-MS, the reaction solution was slowly added to 200 mL of ice water to quench. The mixture was then extracted with EA (100 mL * 3). The organic phases were combined and washed with saturated sodium chloride, dried over anhydrous sodium sulfate, and concentrated to dryness to obtain a crude product of compound M11-5. The crude product was purified by silica gel column chromatography to obtain the target product M11-5 (12.00 g, white solid). ESI-MS m/z: 583.52 [M+H]⁺.

### Step 6: Synthesis of compound M11-6

Compound M11-5 (11.50 g, 19.74 mmol) and THF (200.00 mL) were added sequentially to a 500 mL three-necked flask. Under nitrogen protection, LDA (2M in THF/hexane) (13.82 mL, 27.64 mmol) was slowly added dropwise. The mixture was heated to room temperature and reacted for 4 h. After the reaction was complete as monitored by LC-MS, the reaction solution was slowly added to 100 mL of water to quench. Then, lithium hydroxide monohydrate (2.48 g, 59.22 mmol) was added, and the mixture was stirred and reacted at room temperature for 1 h. Then, 1M HCl was added to adjust the pH to 5 to 6. The mixture was then extracted with EA (50 mL * 3). The organic phases were combined and washed with saturated sodium chloride, dried over anhydrous sodium sulfate, and concentrated to dryness to obtain a crude product of compound M11-6. The crude product was purified by silica gel column chromatography to obtain the target product M11-6 (2.20 g). ESI-MS m/z: 343.27 [M+H]⁺.

### Step 7: Synthesis of compound M11-7

Compound M11-6 (2.20 g, 6.43 mmol) and MeOH (30.00 mL) were added sequentially to a 100 mL three-necked flask. Under nitrogen protection and ice-water bath conditions, trimethylsilyldiazomethane (16.06 mL, 2.00 mol/L, 32.13 mmol) was slowly added. The mixture was then heated to room temperature and reacted for 1 h. After the reaction was complete as monitored by LC-MS, the reaction solution was slowly added to 0.3 mL of acetic acid to quench. Saturated sodium bicarbonate was then added to adjust the pH to 8 to 9. The mixture was extracted with EA (50 mL * 3). The organic phases were combined and washed with saturated sodium chloride, dried over anhydrous sodium sulfate, and concentrated to dryness to obtain a crude product of compound M11-7. The crude product was purified by silica gel column chromatography to obtain the target product M11-7 (0.78 g, pale yellow liquid). ESI-MS m/z: 357.43 [M+H]⁺.

### Step 8: Synthesis of compound M11-8

Compound M11-7 (0.78 g, 2.28 mmol) and DCM (9.00 mL) were added sequentially to a 100 mL single-necked flask. Under nitrogen protection and ice-water bath conditions, trifluoroacetic acid (3.00 mL) was slowly added. The mixture was then heated to room temperature and stirred for 8 h. After the reaction was complete as monitored by LC-MS, the reaction solution was concentrated to remove the solvent. Then, 10 mL of methyl tert-butyl ether was slowly added to the reaction solution, and a white solid precipitated upon ultrasonication. Subsequently, 10 mL of n-hexane was added for sufficient crystallization. The mixture was then filtered and dried to obtain the target product M11-8 (0.41 g, white solid). ESI-MS m/z: 157.24 [M+H]⁺.

### Step 9: Synthesis of compound M11-9

Compound M2-11 (600.00 mg, 1.00 mmol), N,N-diisopropylethylamine (0.83 mL, 5.00 mmol), and DMF (15.00 mL) were added sequentially to a 100 mL single-necked flask. The mixture was stirred at room temperature for 10 min. Under nitrogen protection at 0 °C, HATU (951.45 mg, 2.50 mmol) was added, and mixture was reacted for 20 min. Then, M11-8 (384.6 mg, 1.00 mmol) was added. The mixture was heated to 25 °C and reacted for 1 h. After the reaction was complete as monitored by LC-MS, the reaction solution was slowly added to 20 mL of ice water to quench. The mixture was then extracted with EA (20 mL * 3). The organic phases were combined and washed with saturated sodium chloride, dried over anhydrous sodium sulfate, and concentrated to dryness to obtain a crude product of compound M11-9. The crude product was purified by silica gel column chromatography to obtain the target product M11-9 (704.00 mg, pale yellow solid). ESI-MS m/z: 738.21 [M+H]⁺.

### Step 10: Synthesis of compound M11-10

Compound M11-9 (0.88 g, 2.98 mmol) and THF (12.00 mL) were added sequentially to a 100 mL single-necked flask. Under nitrogen protection and ice-water bath conditions, an aqueous solution (4.00 mL) of lithium hydroxide monohydrate (0.13 g, 2.98 mmol) dissolved in water was slowly added. The reaction was carried out in the ice-water bath for 1 h. After the reaction was complete as monitored by LC-MS, 1M HCl was added to adjust the pH to 5 to 6. The mixture was then extracted with EA (50 mL * 3). The organic phases were combined and washed with saturated sodium chloride, dried over anhydrous sodium sulfate, and concentrated to dryness to obtain a crude product of compound M11-10. The crude product was purified by silica gel column chromatography to obtain the target product M11-10 (0.5 g, white foamy solid). ESI-MS m/z: 724.65 [M+H]⁺.

### Step 11: Synthesis of compound M11-11

Compound M11-10 (470.00 mg, 0.64 mmol), N-methylimidazole (159.97 mg, 1.95 mmol), DMF (5.00 mL), and acetonitrile (3.00 mL) were added sequentially to a 100 mL single-necked flask. Under nitrogen protection at room temperature, N,N,N',N'-tetramethylchloroformamidinium hexafluorophosphate (23.29 mg, 0.08 mmol) was slowly added in batches. The mixture was reacted for 0.5 h with temperature maintained. After the reaction was complete as monitored by LC-MS, the reaction solution was slowly added to 20 mL of ice water to quench. The mixture was then extracted with EA (20 mL * 3). The organic phases were combined and washed with saturated sodium chloride and saturated sodium bicarbonate, dried over anhydrous sodium sulfate, and concentrated to dryness to obtain a crude product of compound M11-11. The crude product was purified by silica gel column chromatography to obtain the target product M11-11 (200.00 mg, white solid). ESI-MS m/z: 706.65 [M+H]⁺.

### Step 12: Synthesis of compound M11

Compound M11-11 (200.00 mg, 0.28 mmol), Sphos-Pd-G2 (10.22 mg, 0.01 mmol), potassium acetate (97.35 mg, 0.99 mmol), toluene (4.50 mL), and 1,4-dioxane (1.50 mL) were added sequentially to a 50 mL three-necked flask. Then, under N₂ protection and ice-water bath conditions, 4,4,5,5-tetramethyl-1,3,2-dioxaborolane (0.33 mL, 2.27 mmol) was added. The mixture was heated to room temperature and stirred for 3 h. After the reaction was complete as monitored by LC-MS, the reaction solution was slowly added to 10 mL of saturated ammonium chloride to quench. The mixture was then extracted with EA (20 mL * 3). The organic phases were combined and washed with saturated sodium chloride and saturated sodium bicarbonate, dried over anhydrous sodium sulfate, and concentrated to dryness to obtain a crude product of compound M11. The crude product was purified by silica gel column chromatography to obtain the target product M11 (80.00 mg, white solid). ESI-MS m/z: 706.69 [M+H]⁺.

### Synthesis of intermediate M11-8:

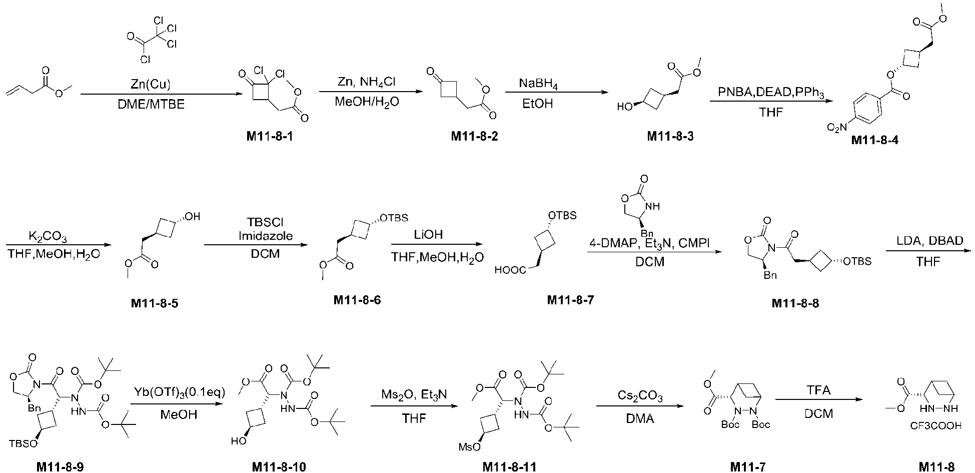

### Step 1: Synthesis of compound M11-8-1

Methyl 3-butenoate (98.00 g, 978.87 mmol), ethylene glycol dimethyl ether (500.00 mL), methyl tert-butyl ether (2500.00 mL), and Zn(Cu) (189.32 g, 1468.30 mmol) were added sequentially to a 5000 mL four-necked flask. The flask was purged and protected with nitrogen. The mixture was cooled with a cold water bath, and trichloroacetyl chloride (373.78 g, 2055.62 mmol) was added dropwise while the temperature was maintained at 20 °C to 25 °C. After the addition was complete, the mixture was stirred and reacted overnight. The reaction solution was filtered and washed with MTBE (400 mL * 3). The filtrate was concentrated to dryness. The concentrate was purified by column chromatography (PE to PE/EA=16/1) to obtain 297.5 g of a light yellow-brown liquid, which was the crude product of compound M11-8-1, used directly in the next reaction step.

### Step 2: Synthesis of compound M11-8-2

Compound M11-8-1 (295.50 g, content 69.9%, 978.73 mmol), methanol (1500.00 mL), water (1500.00 mL), and ammonium chloride (261.76 g, 4893.66 mmol) were added sequentially to a 5000 mL three-necked flask. The mixture was cooled in an ice-water bath, and Zn/zinc powder (192.00 g, 2936.20 mmol) was added in batches while maintaining the temperature at 10 °C to 25 °C. After the addition was complete, the mixture was stirred and reacted for 5 h, then the mixture was stand overnight. After the reaction was complete, the reaction solution was filtered, and the filtrate was concentrated to remove methanol. The concentrate was dissolved by adding 250 mL of water. The mixture was extracted with ethyl acetate (200 mL * 3). The organic phases were combined and washed with 300 mL of saturated brine, dried over anhydrous magnesium sulfate, filtered, and concentrated to obtain 109.5 g of a pale brown liquid, which was the crude product of compound M11-8-2, used directly in the next reaction step.

### Step 3: Synthesis of compound M11-8-3

Compound M11-8-2 (108.00 g, 759.75 mmol) and ethanol (800.00 mL) were added sequentially to a 2000 mL three-necked flask. Under nitrogen protection and ice-water bath cooling, sodium borohydride (12.93 g, 341.89 mmol) was added in batches while the temperature was maintained at 0 °C to 5 °C. After the addition was complete, the reaction was maintained at 0 °C to 5 °C. After the reaction was monitored to complete, saturated aqueous ammonium chloride solution (100 mL) was slowly added to quench the reaction. The reaction solution was then concentrated to remove the organic solvent. Water (300 mL) was added to the concentrated residue, followed by extraction with ethyl acetate (250 mL * 3) and liquid separation. The organic phases were combined and washed with 250 mL of saturated aqueous sodium bicarbonate solution, separated, dried over anhydrous sodium sulfate, and dried by rotary evaporation to obtain 89.3 g of a liquid, which was compound M11-8-3.

### Step 4: Synthesis of compound M11-8-4

Compound M11-8-3 (30.20 g, 209.48 mmol), tetrahydrofuran (500.00 mL), triphenylphosphine (65.94 g, 251.37 mmol), and p-nitrobenzoic acid (42.01 g, 251.37 mmol) were added sequentially to a 1000 mL three-necked flask. Then, diethyl azodicarboxylate (43.78 g, 251.37 mmol) was added dropwise. After the addition was complete, the mixture was stirred and reacted. After the reaction was complete, the reaction solution was concentrated. When a solid precipitated, MTBE was added, and concentration was continued (100 mL * 2). Then, MTBE (300 mL) was added, and the mixture was stirred and slurried for 10 min. PE (150 mL) was added, and the mixture was stirred and slurried for 1 h. The mixture was filtered and suction dried. The filter cake was washed with a mixture of MTBE/PE=1/1, and the filtrate was collected. The filtrate was dried by rotary evaporation and purified by column chromatography to obtain 49.5 g of a slightly yellow solid, which was compound M11-8-4. ESI-MS m/z: 294.09 [M+H]⁺.

### Step 5: Synthesis of compound M11-8-5

Compound M11-8-4 (50.50 g, 172.19 mmol), methanol (500.00 mL), tetrahydrofuran (250.00 mL), water (60.00 mL), and potassium carbonate (23.80 g, 172.19 mmol) were added sequentially to a 500 mL three-necked flask. After the addition was complete, the mixture was stirred and reacted. The reaction solution was filtered, and the filtrate was concentrated to obtain a concentrated residue. Cold water (400 mL) was added to the concentrated residue, followed by extraction with dichloromethane and liquid separation (250 mL * 3). The organic phases were combined and dried over anhydrous sodium sulfate, concentrated, and purified by column chromatography to obtain 21.8 g of a colorless liquid, which was compound M11-8-5.

### Step 6: Synthesis of compound M11-8-6

Compound M11-8-5 (43.00 g, 298.26 mmol) and DCM/dichloromethane (450.00 mL) were added sequentially to a 1000 mL three-necked flask. The mixture was cooled in an ice-water bath to below 5 °C. DMAP/4-dimethylaminopyridine (3.64 g, 29.83 mmol) and imidazole (22.34 g, 328.09 mmol) were added. Then, TBSCl/tert-butyldimethylchlorosilane (47.20 g, 313.18 mmol) was added. After the addition was complete, the reaction was maintained at 0 °C to 5 °C and stirred for 3 h. After the reaction was monitored to complete, the mixture was washed sequentially with 300 mL of water and 200 mL of saturated aqueous sodium chloride solution, and separated. The organic phase was dried over anhydrous sodium sulfate, filtered to remove the drying agent. The filtrate was concentrated to dryness to obtain 77 g of a slightly yellow liquid, which was compound M11-8-6.

### Step 7: Synthesis of compound M11-8-7

Compound M11-8-6 (2.60 g, 10.06 mmol), tetrahydrofuran (10.00 mL), methanol (10.00 mL), and water (10.00 mL) were added sequentially to a 100 mL three-necked flask. The mixture was cooled in an ice-water bath, and lithium hydroxide monohydrate (0.63 g, 15.09 mmol) was added. The mixture was stirred and reacted. After the reaction was complete, 20 mL of water was added to the reaction solution. The reaction mixture was then concentrated in a 35to 40 °C water bath to remove THF and methanol. Another 20 mL of water was added, followed by extraction with 30 mL of ethyl acetate and liquid separation; the organic phase was discarded. The aqueous phase was collected, and 1 N dilute hydrochloric acid was added dropwise to adjust the pH to about 3 to 4. The mixture was extracted with ethyl acetate (30 mL * 2) and separated. The organic phases were combined and dried over anhydrous sodium sulfate. After filtration, the filtrate was concentrated to obtain 2.2 g of a colorless liquid, which was compound M11-8-7.

### Step 8: Synthesis of compound M11-8-8

Compound M11-8-7 (57.30 g, 234.45 mmol), (S)-4-benzyl-2-oxazolidinone (45.70 g, 257.89 mmol), 4-dimethylaminopyridine (2.86 g, 23.44 mmol), triethylamine (97.76 mL, 703.35 mmol), and dichloromethane (860.00 mL) were added sequentially to a 2000 mL three-necked flask. The mixture was cooled with a cold water bath, and CMPI/2-chloro-1-methylpyridinium iodide (71.88 g, 281.34 mmol) was added in batches while the temperature was maintained at 20 °C to 25 °C. After the addition was complete, the mixture was reacted at 20 °C to 25 °C for 2 h. TLC monitoring showed complete conversion of the starting material. The reaction was quenched with 500 mL of water, extracted with DCM (400 mL * 2), and washed with saturated sodium chloride (500 mL * 2). The mixture was dried over anhydrous sodium sulfate, filtered, and the filtrate was concentrated. The residue was separated by column chromatography to obtain 79.2 g of a slightly yellow viscous substance, which was compound M11-8-8. ESI-MS m/z: 404.2 [M+H]⁺.

### Step 9: Synthesis of compound M11-8-9

Compound M11-8-8 (76.20 g, 188.81 mmol) and tetrahydrofuran (750.00 mL) were added sequentially to a 2 L three-necked flask. Under nitrogen protection, the mixture was cooled in a dry ice-ethanol bath to below -78 °C. Lithium diisopropylamide (2M in THF/hexane) (122.72 mL, 2.00 mol/L, 245.45 mmol) was added dropwise. After the addition was complete, the mixture was stirred and reacted at that temperature. After 1 h of reaction, a solution of di-tert-butyl azodicarboxylate (52.17 g, 226.57 mmol) in tetrahydrofuran (100.00 mL) was added. The mixture was reacted at -78 °C for 1 h until the reaction was complete. The reaction solution was added to 1000 mL of cold aqueous ammonium chloride solution and stirred for 10 min. The mixture was extracted with ethyl acetate (500 mL * 3) and separated. The organic phases were combined and washed with saturated brine (500 mL * 2) and separated. The organic phase was dried over anhydrous sodium sulfate, filtered to remove the drying agent. The filtrate was concentrated. Silica gel was added for sample preparation and sand making. The residue was separated by column chromatography (PE to PE/EA=88:12) to obtain, after concentration, 105.8 g of a white foamy solid, which was compound M11-8-9. ESI-MS m/z: 634.3 [M+H]⁺.

### Step 10: Synthesis of compound M11-8-10

Compound M11-8-9 (95.00 g, 149.88 mmol) and methanol (400.00 mL) were added sequentially to a 1 L three-necked flask. Under nitrogen protection at room temperature, ytterbium trifluoromethanesulfonate (9.30 g, 14.99 mmol) was added. The mixture was stirred and reacted at that temperature for 50 h. The reaction was complete. The reaction solution was concentrated to dryness and purified by column chromatography to obtain 52.00 g of a white foamy solid, which was compound M11-8-10. ESI-MS m/z: 175.2 [M+H]⁺.

### Step 11: Synthesis of compound M11-8-11

Compound M11-8-10 (42.00 g, 112.17 mmol), tetrahydrofuran (630.00 mL), and triethylamine (46.77 mL, 336.51 mmol) were added sequentially to a 2000 mL three-necked flask. The mixture was cooled with a water bath, and Ms₂O/methanesulfonic anhydride (25.40 g, 145.82 mmol) was added. The reaction was heated to room temperature and stirred for 0.5 h. TLC monitoring showed complete conversion of the starting material. The reaction solution was washed sequentially with 400 mL of water and 400 mL of saturated brine, and separated. The organic phase was dried over anhydrous sodium sulfate and concentrated to obtain 50.0 g of a pale yellow oil, which was compound M11-8-11. ESI-MS m/z: 453.2 [M+H]⁺.

### Step 12: Synthesis of compound M11-7

Compound M11-8-11 (50.00 g, 110.49 mmol), N,N-dimethylacetamide (500.00 mL), and cesium carbonate (90.00 g, 276.23 mmol) were added sequentially to a 1000 mL single-necked flask. Under nitrogen protection, the mixture was heated in an oil bath and reacted at 60 °C to 65 °C for 1 to 1.5 h. After the reaction was complete, the reaction solution was added to 500 g of ice water, stirred, and then extracted with ethyl acetate (400 mL * 3) and separated. The organic phases were combined and washed with saturated sodium chloride (300 mL * 5), separated, dried over anhydrous sodium sulfate, concentrated, and purified by column chromatography to obtain 27.5 g of a light yellow-brown viscous substance, which was compound M11-7. ESI-MS m/z: 357.43 [M+H]⁺.

### Step 13: Synthesis of compound M11-8

Compound M11-7 (27.50 g, 77.16 mmol) was added to dichloromethane (82.50 mL). Then, under N₂ protection and ice-water bath conditions, trifluoroacetic acid (82.50 mL) was slowly added. The reaction was carried out at 25 °C for 4 h until the starting material was consumed. Most of the solvent was evaporated from the reaction solution at 30 °C, and a large amount of trifluoroacetic acid was removed with an oil pump. Then, 100 mL of MTBE was added, and a solid precipitated upon ultrasonication. Subsequently, 100 mL of n-hexane was added for slurrying. The mixture was then filtered, and the solid was suction dried to obtain 18.4 g, which was compound M11-8. ESI-MS m/z: 157.24 [M+H]⁺.

### Synthesis of intermediate M12

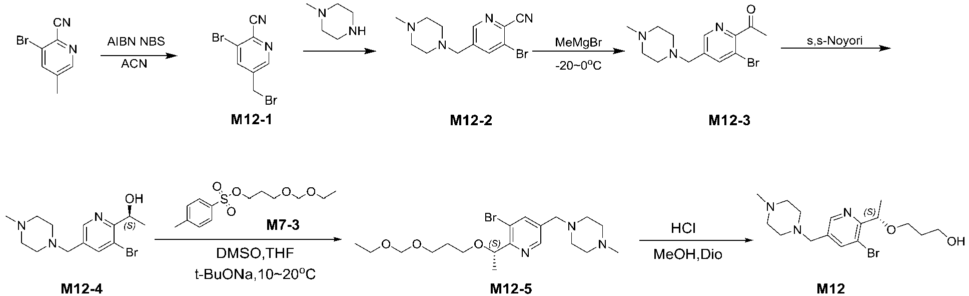

### Step 1: Synthesis of compound M12-1

Compound 3-bromo-5-methyl-pyridine-2-carbonitrile (5 g, 25.38 mmol) was dissolved in carbon tetrachloride (100 mL). N-Bromosuccinimide (4.52 g, 25.38 mmol) and azobisisobutyronitrile (0.42 g, 2.54 mmol) were added. The mixture was heated to 80 °C and reacted. After the reaction was complete, the reaction solution was diluted with EA and water, extracted twice with EA, and purified by column chromatography to obtain compound M12-1 (5.94 g). ESI-MS m/z: 274.97 [M+H]⁺.

### Step 2: Synthesis of compound M12-2

Compound M12-1 (5.94 g, 21.53 mmol) and methylpiperazine (3.23 g, 32.29 mmol) were dissolved in acetonitrile (60 mL). N,N-Diisopropylethylamine (10.67 mL, 64.58 mmol) was added, and the reaction was carried out at room temperature for 2 h. After the reaction was complete, the mixture was directly combined with silica gel and purified by column chromatography to obtain compound M12-2 (2.82 g). ESI-MS m/z: 295.15 [M+H]⁺.

### Step 3: Synthesis of compound M12-3

Compound M12-2 (2.82 g, 9.55 mmol) was dissolved in tetrahydrofuran (30 mL). The temperature was cooled to -20 °C, and methylmagnesium bromide (9.55 mL, 3.00 mol/L, 28.66 mmol) was slowly added dropwise. After the addition was complete, the mixture was heated to room temperature. After the reaction was complete, the reaction solution was added to ice-cold dilute hydrochloric acid (pH=3 to 4) and stirred for ten min until all the imine intermediate was converted to the ketone. The pH was adjusted to 9 with saturated sodium carbonate. The mixture was extracted twice with EA, dried, filtered, and dried by rotary evaporation to obtain compound M12-3 (2.8 g). The crude product was used directly in the next step. ESI-MS m/z: 312.16 [M+H]⁺.

### Step 4: Synthesis of compound M12-4

Compound M12-3 (2.80 g, 8.97 mmol) was dissolved in triethylamine (30 mL). (S,S)-N-(p-toluenesulfonyl)-1,2-diphenylethane-1,2-diamine (p-cymene)ruthenium(II) chloride (0.23 g, 0.36 mmol) and formic acid (1.65 g, 35.87 mmol) were added. The reaction was carried out at room temperature for 6 h. After the reaction was complete, the mixture was directly combined with silica gel and purified by column chromatography to obtain compound M12-4 (2.7 g). ESI-MS m/z: 314.18 [M+H]⁺.

### Step 5: Synthesis of compound M12-5

Compound M12-4 (2.6 g, 8.27 mmol) and compound M7-3 (4.77 g, 16.55 mmol) were dissolved in dimethyl sulfoxide (20 mL) and tetrahydrofuran (4 mL). The temperature was cooled to 0 °C, and sodium tert-butoxide (1.59 g, 16.55 mmol) was added. The mixture was then heated to room temperature and stirred for 3 h. After the reaction was complete, the temperature was cooled to 0 °C, and the reaction was quenched with saturated ammonium chloride. The mixture was extracted twice with EA, washed three times with saturated brine, dried, filtered, and dried by rotary evaporation to obtain compound M12-5 (3.6 g). The crude product was used directly in the next step. ESI-MS m/z: 430.26 [M+H]⁺.

### Step 6: Synthesis of compound M12

Compound M12-5 (3.60 g, 8.36 mmol) was dissolved in methanol (10 mL). Hydrochloric acid (4M in dioxane) (10 mL) was added, and the mixture was reacted at room temperature for 2 h. After the reaction was complete, the reaction solution was dried by rotary evaporation. The residue was dissolved in DCM and water. The mixture was extracted twice with DCM the discarded. The aqueous phase was treated with saturated sodium carbonate to liberate the compound, then extracted three times with EA, dried, filtered, and dried by rotary evaporation to obtain compound M12 (2.6 g). ESI-MS m/z: 373.39 [M+H]⁺.

### Synthesis of intermediate M13:

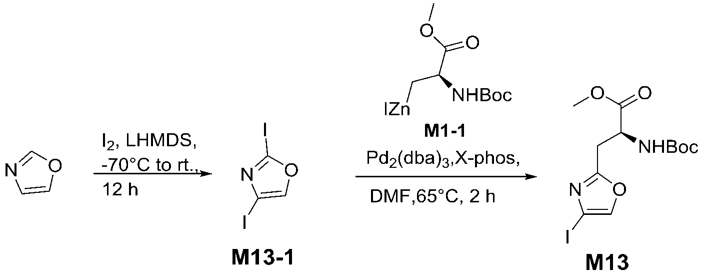

### Step 1: Synthesis of compound M13-1:

Intermediate oxazole (2.1 g) was added to a 100 mL three-necked flask, followed by anhydrous THF (13 mL) and DMPU (10.4 mL). Under N₂ protection, the temperature was cooled to -78 °C, and LiHMDS (67 mL, 1 mol/L solution in THF) was slowly added dropwise. The reaction was maintained at -75 °C for 1.0 h. Iodine (15.4 g) was added in batches. After maintaining at -75 °C for 30 min, the mixture was heated to room temperature and reacted for 8.0 h. After the reaction was complete, the mixture was diluted with saturated aqueous sodium thiosulfate solution (20 mL), extracted with Et₂O. The organic phase was dried over anhydrous sodium sulfate, and purified by column chromatography (PE:EA = 9:1) to obtain M13-1 (6.54 g, 67.3% yield). ESI-MS m/z: 321.1 [M+H]⁺.

### Step 2: Synthesis of compound M13:

Compound M13-1 (4.0 g), Pd₂(dba)₃ (626 mg), X-Phos (978 mg), and anhydrous DMF (20 mL) were added to a 250 mL three-necked flask. The flask was purged with N₂ three times. Finally, M1-1 (61.56 mL, 0.33 mol/L solution in DMF) was added. The temperature was heated to 70 °C and the reaction was carried out for 2.5 h. After the reaction was complete, the mixture was cooled to room temperature. EA and water were added to the reaction solution for extraction. The aqueous phase was extracted twice more with EA. The organic phases were combined and collected, dried over anhydrous sodium sulfate, and concentrated. The concentrate was purified by column chromatography (PE:EA = 3:1) to obtain the target compound M13 (1.52 g, 31% yield). ESI-MS m/z: 396.3 [M+H]⁺.

### Example 1: Synthesis of compound (1S,2S)-2-methyl-N-((1⁵S,6³S,4S,Z)-1⁵,10,10-trimethyl-1²-(4-methylpiperazin-1-yl)-5,7-dioxo-1 ⁸,1⁹,6¹,6²,6³,6⁴,6⁵,6⁶-octahydro-1⁵H,1⁷-oxa-1(13,15)-pyrido[3',2':7,8][1,5]oxazonino[5,6-a]in dola-2(4,2)-thiazola-6(1,3)-pyridazinacycloundecaphane-4-yl)cyclopropane-1-carboxamide

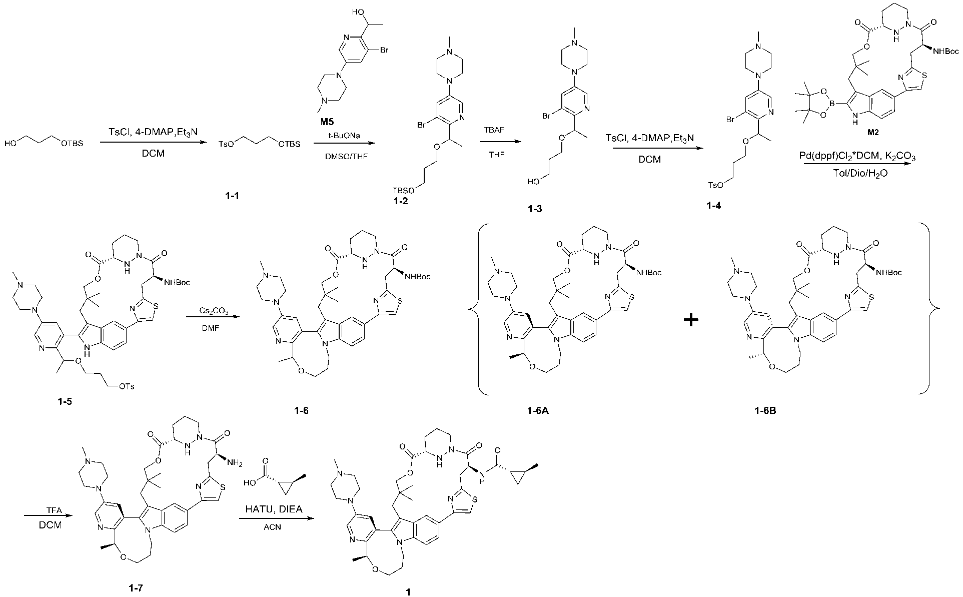

### Step 1: Synthesis of compound 1-1

Compound 3-((tert-butyldimethylsilyl)oxy)-propanol (3.00 g), triethylamine (3.29 mL), and 4-DMAP (0.19 g) were sequentially added to a DCM (40.00 mL) solution. Under nitrogen protection, the mixture was cooled to 0 °C. A solution of TsCl (3.91 g) was slowly added. Then, the mixture was heated to room temperature and reacted for 13 h. After the reaction was complete, 100 mL of water was added to the reaction mixture to quench the reaction. The mixture was then extracted three times with 50 mL of DCM and washed twice with 50 mL of saturated sodium chloride. The organic phases were combined and concentrated to obtain a crude product. The crude product was purified by column chromatography (PE:EA = 95:5 to 80:20) to obtain a colorless liquid, which was the target compound 1-1 (4.00 g, 73.66% yield). ESI-MS m/z: 345.4 [M+H]⁺.

### Step 2: Synthesis of compound 1-2

Compound 1-1 (1.72 g) and compound M5 (0.75 g) were added to DMSO (10.00 mL) and THF (2.00 mL). Then, under nitrogen protection at room temperature, a solution of sodium tert-butoxide (0.60 g) dissolved in THF (2.00 mL) was slowly added. The mixture was reacted at room temperature for 5 h. After the reaction was complete, 30 mL of water was added to the reaction mixture to quench the reaction. The mixture was then extracted three times with 30 mL of EA. The organic phases were combined, washed with saturated sodium chloride, and concentrated to obtain a crude product. The crude product was purified by column chromatography (DCM:EA=90:10 to 10:90) to obtain the target compound 1-2 (0.80 g, 67.77% yield). ESI-MS m/z: 472.5, 474.5 [M+H]⁺.

### Step 3: Synthesis of compound 1-3

Compound 1-2 (0.80 g) and TBAF (1.86 mL, 1.00 mol/L) were added to THF (10.00 mL). Then, under nitrogen protection, the mixture was reacted at 50 °C for 1 h. After the reaction was complete, 30 mL of water was added to the reaction mixture to quench the reaction. The mixture was then extracted five times with 30 mL of DCM. The organic phases were combined, washed with saturated sodium chloride, and concentrated to obtain a crude product. The crude product was purified by column chromatography (DCM:MeOH=97:3 to 90:10) to obtain the target compound 1-3 (0.60 g, 98.92% yield). ESI-MS m/z: 358.3, 360.3 [M+H]⁺.

### Step 4: Synthesis of compound 1-4

Compound 1-3 (600.00 mg), triethylamine (0.35 mL), and 4-DMAP (20.50 mg) were sequentially added to a DCM (10.00 mL) solution. Under nitrogen protection, the mixture was cooled to 0 °C. TsCL (415.1 mg) was slowly added. Then, the mixture was heated to room temperature and reacted for 12 h. After the reaction was complete, 100 mL of water was added to the reaction mixture to quench the reaction. The mixture was then extracted three times with 50 mL of DCM and washed twice with 50 mL of saturated sodium chloride. The organic phases were combined and concentrated to obtain a crude product. The crude product was purified by column chromatography (DCM:MeOH=95:5 to 90:10) to obtain the target compound 1-4 (450.00 mg, 52.43% yield). ESI-MS m/z: 512.4, 514.4 [M+H]⁺.

### Step 5: Synthesis of compound 1-5

Compound 1-4 (265.97 mg), intermediate M2 (300.00 mg), PdCl₂(dppf) (31.61 mg), and K₂CO₃ (149.43 mg) were added to toluene (4.50 mL), 1,4-dioxane (1.50 mL), and water (1.00 mL). Then, under nitrogen protection, the mixture was reacted at 65 °C for 2 h. After the reaction was complete, 30 mL of water was added to the reaction mixture to quench the reaction. The mixture was then extracted three times with 30 mL of DCM and washed twice with 30 mL of saturated sodium chloride. The organic phases were combined and concentrated to obtain a crude product. The crude product was purified by column chromatography (DCM:MeOH=95:5 to 90:10) to obtain the target compound 1-5 (270.00 mg, 62.48% yield). ESI-MS m/z: 1000.2 [M+H]⁺.

### Step 6: Synthesis of compounds 1-6A and 1-6B

Compound 1-5 (270.00 mg) and Cs₂CO₃ (176.04 mg) were added to DMF (30.00 mL). Then, under nitrogen protection, the mixture was reacted at 90 °C for 0.5 h. After the reaction was complete, 50 mL of water was added to the reaction mixture to quench the reaction. The mixture was then extracted three times with 40 mL of DCM and washed four times with 30 mL of saturated sodium chloride. The organic phases were combined and concentrated to obtain a crude product. The crude product was purified by column chromatography (DCM:MeOH=95:5 to 90:10) to obtain the target compound 1-6 (170.00 mg, 68.48% yield). ESI-MS m/z: 828.1 [M+H]⁺.

The preparative separation conditions were as follows: column: Phenomenex (21.2×150 mm, 5 µm); mobile phase: A: 0.05% TFA/ H2O, B: ACN; gradient: 39% to 42%; flow rate: 20 ml/min.

Compound 1-6A: retention time: 5.45 to 5.95 min (55.00 mg, 32.34% yield). ESI-MS m/z: 828.1 [M+H]+; 1H NMR (500 MHz, CDCl₃)δ 8.60 (d, J = 1.6 Hz, 1H), 8.42 (d, J = 3.0 Hz, 1H), 7.58 (dd, J = 8.5, 1.7 Hz, 1H), 7.32-7.27 (m, 2H), 7.09 (d, J = 2.9 Hz, 1H), 5.58 (t, J = 9.4 Hz, 1H), 5.48 (d, J = 10.4 Hz, 1H), 4.61-4.56 (m, 1H), 4.38-4.32 (m, 1H), 4.26-4.17 (m, 2H), 4.09 - 3.95 (m, 2H), 3.87 - 3.73 (m, 3H), 3.41 (d, J = 14.8 Hz, 1H), 3.35-3.31 (m, 5H), 3.20 (d, J = 14.4 Hz, 1H), 3.05 (dd, J = 14.9, 8.8 Hz, 1H), 2.68 (td, J = 12.9, 3.0 Hz, 1H), 2.62-3.58 (m, 4H), 2.43 (d, J = 14.4 Hz, 1H), 2.36 (s, 3H), 2.32 - 2.29 (m,1H), 2.22 - 2.18 (m,1H), 1.99-1.92 (m, 2H), 1.84-1.76 (m, 1H), 1.62-1.56 (m, 1H), 1.51 (d, J = 6.5 Hz, 3H), 1.51 - 1.41 (m, 9H), 0.97 (s, 3H), 0.41 (s, 3H).

Compound 1-6B: retention time: 6.15 to 6.9 min (30.00 mg, 17.64% yield). 1H NMR (500 MHz, CDCl₃)δ 8.61 (s, 1H), 8.40 (d, J = 2.9 Hz, 1H), 7.56 (d, J = 8.6 Hz, 1H), 7.32 - 7.23 (m, 3H), 5.63 (t, J = 9.3 Hz, 1H), 5.44 (d, J = 10.2 Hz, 1H), 4.61 - 4.54 (m, 1H), 4.35 - 4.19 (m, 3H), 4.03 (d, J = 11.9 Hz, 1H), 3.94 (d, J = 11.0 Hz, 1H), 3.83 (d, J = 11.0 Hz, 1H), 3.67-3.61 (m, 1H), 3.48-3.44 (m, 1H), 3.38 (d, J = 14.9 Hz, 1H), 3.31-3.25 (m, 4H), 3.15 (d, J = 14.3 Hz, 1H), 3.05 (dd, J = 14.9, 8.7 Hz, 1H), 2.83-2.78 (m, 1H), 2.70-2.64 (m, 1H), 2.60 (t, J = 5.3 Hz, 3H), 2.36 (s, 3H), 2.21-2.03 (m, 3H), 1.97 - 1.90 (m, 1H), 1.82 - 1.70 (m, 1H), 1.63-1.54 (m, 3H), 1.51 (d, J = 6.6 Hz, 3H), 1.46 (s, 9H), 0.93 (s, 3H), 0.74 (s, 3H).

### Step 8: Synthesis of compound 1-7

Compound 1-6A (55.00 mg) was added to DCM (2.00 mL). Then, under N₂ protection and ice-water bath conditions, HCl/dioxane (0.83 mL, 4M) was slowly added. The mixture was heated to room temperature and stirred for 3 h. After the reaction was complete, the reaction solution was concentrated to obtain a crude product. Toluene (10 mL) was added to the crude product, and the mixture was concentrated to obtain the target compound 1-7 (48.00 mg, 99.30% yield). ESI-MS m/z: 728.0 [M+H]⁺.

### Step 9: Synthesis of compound 1

(1S,2S)-2-methylcyclopropanecarboxylic acid (17.20 mg), HATU (50.20 mg), DIEA (50 µL), and ACN (5.00 mL) were added sequentially to a reaction flask. After stirring at 0°C for 15 min, the starting material 1-7 (48.00 mg) was added. The mixture was then transferred to room temperature and stirred for 1 h. After the reaction was complete, the solvent was dried by rotary evaporation under reduced pressure. The concentrate was purified by preparative chromatography to obtain the target compound 1 (39.8 mg, 74.54% yield). ESI-MS m/z: 809.61 [M+H]⁺. ¹H NMR (500 MHz, CDCl₃) δ 8.58 (d, J = 1.7 Hz, 1H), 8.42 (d, J = 2.9 Hz, 1H), 7.57 (dd, J = 8.6, 1.6 Hz, 1H), 7.31 (d, J = 8.6 Hz, 1H), 7.27 (s, 1H), 7.09 (d, J = 3.0 Hz, 1H), 6.42 (d, J = 9.7 Hz, 1H), 5.89 (t, J = 9.0 Hz, 1H), 4.60 (d, J = 13.0 Hz, 1H), 4.35 (td, J = 12.1, 3.3 Hz, 1H), 4.24-4.16 (m, 2H), 4.04 - 3.96 (m, 2H), 3.86 - 3.74 (m, 3H), 3.45 (d, J = 14.9 Hz, 1H), 3.32-3.26 (m, 5H), 3.23 - 3.16 (m, 1H), 3.11 (dd, J = 14.9, 8.6 Hz, 1H), 2.69-2.60 (m, 5H), 2.42 (d, J = 14.4 Hz, 1H), 2.38 (s, 3H), 2.32-2.21 (m, 1H), 2.01-1.94 (m, 2H), 1.85-1.80 (m, 1H), 1.51 (d, J = 6.5 Hz, 3H), 1.30 - 1.19 (m, 4H), 1.10 (d, J = 6.0 Hz, 3H), 0.96 (s, 3H), 0.91 - 0.86 (m, 1H), 0.66-0.60 (m, 1H), 0.40 (s, 3H).

### Example 2: Synthesis of compound (1S,2S)-N-((6³S,4S,Z)-10,10-dimethyl-1²-(4-methylpiperazin-1-yl)-5,7-dioxo-1⁸,1⁹,6¹,6²,6^{3,}6⁴,6⁵ ,6⁶-octahydro-1⁵H,1⁷H-8-oxa-1(13,15)-pyrido[3',2':7,8][1,5]oxazonino[5,6-a]indola-2(4,2)-thiaz ola-6(1,3)-pyridazinacycloundecaphane-4-yl)-2-methylcyclopropane-1-carboxamide

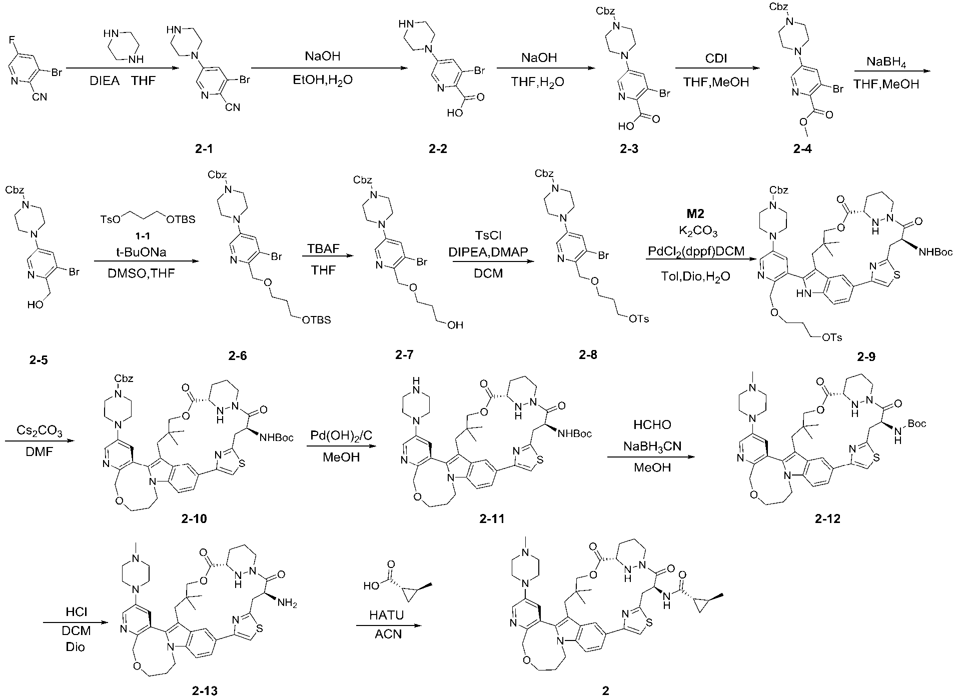

### Step 1: Synthesis of compound 2-1

3-Bromo-5-fluoroacrylonitrile (13.60 g, 67.66 mmol), THF (180.00 mL), DIEA (16.77 mL, 101.49 mmol), and piperazine (6.41 g, 74.43 mmol) were added sequentially to a 500 mL three-necked flask. The reaction was carried out at room temperature. After the reaction was complete as monitored by LC-MS, the reaction solution was diluted with ethyl acetate, washed sequentially with water and brine, separated, and the organic phase was concentrated to obtain a crude product of compound 2-1 (18.1 g), which was used directly in the next reaction step. ESI-MS m/z: 264.10, 269.09 [M+H]⁺.

### Step 2: Synthesis of compound 2-2

Compound 2-1 (18.00 g, 67.38 mmol), EtOH (200.00 mL), H₂O (40.00 mL), and NaOH (13.48 g, 336.92 mmol) were added sequentially to a 500 mL three-necked flask. The mixture was heated in an oil bath, maintained at 80-90 °C and reacted. After the reaction was complete as monitored by LC-MS, the reaction solution was concentrated to dryness. Water (200 mL) and THF (100 mL) were added again, and the mixture was concentrated to remove absolute ethanol, obtaining a mixture containing compound 2-2, which was used directly in the next reaction step. ESI-MS m/z: 286.08, 288.05 [M+H]⁺.

### Step 3: Synthesis of compound 2-3

THF (200.00 mL) and H₂O (150.00 mL) were added to the work-up mixture containing compound 2-2 (19.28 g, 67.38 mmol). The mixture was transferred to a 500 mL three-necked flask. NaOH (8.09 g, 202.15 mmol) was added, and the mixture was stirred at room temperature. Then, CbzCl (18.97 mL, 134.77 mmol) was added dropwise. After the addition was complete, the mixture was stirred and reacted. After the reaction was complete as monitored by LC-MS, dilute hydrochloric acid was added dropwise to adjust the pH to about 3-4. The mixture was extracted three times with ethyl acetate. The organic phases were combined and washed sequentially with water and brine, separated, and concentrated to obtain a crude product. The crude product was purified by silica gel column chromatography (DCM to DCM/MeOH=95/5) to obtain the target compound 2-3 (19.1 g). ESI-MS m/z: 420.24, 422.26 [M+H]⁺.

### Step 4: Synthesis of compound 2-4

Compound 2-3 (19.10 g, 45.45 mmol), THF (200.00 mL), and CDI (11.05 g, 68.17 mmol) were added sequentially to a 500 mL single-necked flask. The mixture was heated in an oil bath and stirred at reflux overnight (about 15 h). After cooling to room temperature, MeOH (200.00 mL) was added, the mixture was stirred and reacted. After the reaction was complete as monitored by LC-MS, the mixture was combined with another reaction batch (starting material: 2-3: 7.4 g) and poured into saturated aqueous ammonium chloride solution to quench. The mixture was extracted with ethyl acetate, washed with brine, separated, and the organic phase was concentrated to obtain a crude product. The crude product was purified by silica gel column chromatography (PE to PE/EA=50/50) to obtain the target compound 2-4 (20.5 g). ESI-MS m/z: 434.23, 436.24 [M+H]⁺.

### Step 5: Synthesis of compound 2-5

Compound 2-4 (7500.00 mg, 17.27 mmol), THF (150.00 mL), and MeOH (30.00 mL) were added sequentially to a 500 mL three-necked flask. With water bath cooling, NaBH₄ (3266.58 mg, 86.35 mmol) was added in batches. After the addition was complete, the mixture was stirred and reacted. After the reaction was complete as monitored by LC-MS, the reaction mixture was poured into saturated aqueous ammonium chloride solution to quench. The mixture was extracted with ethyl acetate, washed with brine, separated, and the organic phase was concentrated to obtain a crude product. The crude product was purified by silica gel column chromatography (PE/EA=95/5 to 50/50) to obtain the target compound 2-5 (5.43 g). ESI-MS m/z: 406.29, 408.29 [M+H]⁺.

### Step 6: Synthesis of compound 2-6

Compound 2-5 (630.00 mg, 1.55 mmol), compound 1-1 (1068.56 mg, 3.10 mmol), DMSO (6.00 mL), and THF (1.20 mL) were added sequentially to a 50 mL single-necked flask. Sodium tert-butoxide (372.56 mg, 3.88 mmol) was added, and the reaction was carried out at room temperature. After the reaction was complete as monitored by TLC, the reaction mixture was poured into saturated aqueous ammonium chloride solution to quench. The mixture was extracted with ethyl acetate, washed with brine, separated, and the organic phase was concentrated to obtain a crude product. The crude product was purified by silica gel column chromatography (PE to PE/EA=50/50) to obtain the target compound 2-6 (528 mg).

### Step 7: Synthesis of compound 2-7

Compound 2-6 (528.00 mg, 0.91 mmol) and TBAF (5.00 mL, 1M solution in THF) were added to a 50 mL single-necked flask. The reaction was carried out at room temperature overnight. After the reaction was complete as monitored by TLC, the reaction solution was diluted with ethyl acetate, washed sequentially with saturated aqueous ammonium chloride solution and brine, separated, and the organic phase was concentrated to obtain a crude product. The crude product was purified by silica gel column chromatography (PE to PE/EA=50/50) to obtain the target compound 2-7 (308 mg). ESI-MS m/z: 464.27, 466.28 [M+H]⁺.

### Step 8: Synthesis of compound 2-8

Compound 2-7 (308.00 mg, 0.66 mmol), DCM (10.00 mL), DIEA (0.27 mL, 1.66 mmol), and DMAP (8.10 mg, 0.07 mmol) were added sequentially to a 50 mL single-necked flask. Then, TosCl (189.69 mg, 0.99 mmol) was added in batches. After the addition was complete, the reaction was carried out at room temperature. After the reaction was complete as monitored by LC-MS, the reaction solution was combined with silica gel for sample preparation and sand making, and purified by silica gel column chromatography (PE/EA=90/10 to 50/50) to obtain compound 2-8 (251 mg). ESI-MS m/z: 618.36, 620.37 [M+H]⁺.

### Step 9: Synthesis of compound 2-9

Compound 2-8 (196.14 mg, 0.32 mmol), M2 (200.00 mg, 0.29 mmol), K₂CO₃ (99.62 mg, 0.72 mmol), [PdCl₂(dppf)] CH₂Cl₂ (23.52 mg, 0.03 mmol), toluene (3.00 mL), dioxane (1.00 mL), and water (1.00 mL) were added sequentially to a 10 mL single-necked flask. The flask was purged and protected with nitrogen. The mixture was heated in an oil bath and reacted at 60 °C to 65 °C. After the reaction was complete as monitored by LC-MS, the reaction solution was diluted with ethyl acetate, washed sequentially with water and brine, and separated. The aqueous phase was extracted again with DCM. The organic phases were combined and concentrated to obtain a crude product. The crude product was purified by Prep-TLC (DCM/MeOH=20/1) to obtain compound 2-9 (220 mg), which was used directly in the next reaction step.

### Step 10: Synthesis of compound 2-10

Compound 2-9 (220.00 mg, 0.20 mmol), DMF (2.00 mL), and Cs₂CO₃ (129.68 mg, 0.40 mmol) were added sequentially to a 10 mL single-necked flask. Under nitrogen protection, the mixture was heated in an oil bath and reacted at 60 °C to 65 °C. After the reaction was complete as monitored by LC-MS, the reaction solution was diluted with ethyl acetate, washed sequentially with water and brine, separated, and concentrated to obtain a crude product of compound 2-10. The crude product was purified by silica gel column chromatography (DCM to DCM/MeOH=20/1) to obtain the target compound 2-10 (59 mg). ESI-MS m/z: 933.53 [M+H]⁺.

¹H NMR (500 MHz, CDCl₃) δ 8.60 (d, J = 1.6 Hz, 1H), 8.37 (d, J = 2.9 Hz, 1H), 7.59 (dd, J = 8.5, 1.7 Hz, 1H), 7.30 (d, J = 9.9 Hz, 2H), 7.08 (d, J = 2.9 Hz, 1H), 5.58 (t, J = 9.4 Hz, 1H), 5.43 (d, J = 10.3 Hz, 1H), 4.88 (d, J = 12.6 Hz, 1H), 4.59 (d, J = 13.2 Hz, 1H), 4.34 (td, J = 12.1, 3.3 Hz, 1H), 4.23 (d, J = 12.6 Hz, 1H), 4.14 - 4.05 (m, 2H), 4.02 (d, J = 12.2 Hz, 1H), 3.87 - 3.75 (m, 3H), 3.41 (d, J = 14.8 Hz, 1H), 3.38 - 3.31 (m, 1H), 3.30 (t, J = 5.1 Hz, 4H), 3.20 (d, J = 14.4 Hz, 1H), 3.05 (dd, J = 14.9, 8.8 Hz, 1H), 2.68 (td, J = 12.9, 3.0 Hz, 1H), 2.64 - 2.56 (m, 4H), 2.39 (s, 1H), 2.36 (s, 3H), 2.35 - 2.19 (m, 2H), 2.02-1.95 (m, 5H), 1.93 - 1.87 (m, 1H), 1.85-1.76 (m, 1H), 1.62-1.56 (m, 1H), 1.47 (s, 9H), 0.96 (s, 3H), 0.45 (s, 3H).

### Step 11: Synthesis of compound 2-11

Compound 2-10 (59.00 mg, 0.06 mmol), MeOH (3.00 mL), and Pd(OH)₂/C (71.00 mg, 0.51 mmol, 10% Pd) were added sequentially to a 10 mL single-necked flask. The flask was purged with hydrogen, and the reaction was carried out at room temperature under a hydrogen atmosphere. After the reaction was complete as monitored by LC-MS, the mixture was filtered to remove the catalyst. The filtrate was concentrated to obtain a crude product of compound 2-11 (42 mg), which was used directly in the next reaction step. ESI-MS m/z: 799.53 [M+H]⁺.

### Step 12: Synthesis of compound 2-12

Compound 2-11 (42.00 mg, 0.05 mmol), MeOH (2.00 mL), 37% aqueous formaldehyde solution (2.00 mL), and HOAc (0.10 mL) were added sequentially to a 10 mL single-necked flask. The mixture was stirred at room temperature for 20 min. NaBH₃CN (16.53 mg, 0.26 mmol) was added, and the reaction was carried out at room temperature. After the reaction was complete as monitored by LC-MS, ethylenediamine was added to quench excess formaldehyde in the reaction solution. The mixture was then poured into saturated aqueous sodium bicarbonate solution to quench, extracted with DCM, and separated. The organic phase was concentrated to obtain a crude product (45 mg, white foamy solid). The crude product was purified by Prep-HPLC (basic method) and lyophilization of the eluent to obtain the target compound 2-12 (22 mg). ESI-MS m/z: 813.50 [M+H]⁺.

### Step 13: Synthesis of compound 2-13

Compound 2-12 (22.00 mg, 0.03 mmol), DCM (2.00 mL), and HCl (4M in dioxane) (1.00 mL) were added sequentially to a 10 mL single-necked flask. The reaction was carried out at room temperature. After the reaction was complete as monitored by LC-MS, the reaction solution was concentrated to dryness. DCM was added, and the mixture was concentrated to dryness again to obtain a crude product of compound 2-13 (19.1 mg, hydrochloride salt), which was used directly in the next reaction step. ESI-MS m/z: 713.54 [M+H]⁺.

### Step 14: Synthesis of compound 2

(1S,2S)-2-methylcyclopropane-1-carboxylic acid (8.02 mg, 0.08 mmol), HATU (45.70 mg, 0.12 mmol), and DIEA (0.04 mL) were added sequentially to a 10 mL single-necked flask. The mixture was stirred at room temperature for 30 min. After cooling in an ice-water bath, a solution of compound 2-13 (19.00 mg, 0.03 mmol) in acetonitrile was added. After the reaction was complete as monitored by LC-MS, the reaction solution was directly purified by Prep-HPLC (acidic method). The eluent was lyophilized to obtain the target compound 2 (11.9 mg). ESI-MS m/z: 795.59 [M+H]⁺. ¹H NMR (500 MHz, DMSO) δ 9.82 (s, 1H), 8.53 (d, J = 9.0 Hz, 1H), 8.49 (d, J = 1.7 Hz, 1H), 8.44 (d, J = 2.8 Hz, 1H), 7.81 (s, 1H), 7.72 (dd, J = 8.6, 1.7 Hz, 1H), 7.50 (d, J = 8.6 Hz, 1H), 7.38 (d, J = 2.9 Hz, 1H), 5.53 (t, J = 9.2 Hz, 1H), 5.08 (d, J = 12.2 Hz, 1H), 4.71 (d, J = 12.6 Hz, 1H), 4.28 - 4.11 (m, 4H), 4.03 (t, J = 13.4 Hz, 2H), 3.95-3.91 (m, 1H), 3.69 (d, J = 11.0 Hz, 1H), 3.68 - 3.57 (m, 2H), 3.53 (d, J = 11.9 Hz, 2H), 3.33 (d, J = 14.5 Hz, 1H), 3.27-3.03 (m, 6H), 3.00 (d, J = 14.4 Hz, 1H), 2.79-2.72 (m, 1H), 2.36 (d, J = 14.3 Hz, 1H), 2.31 - 2.21 (m, 1H), 2.10 (d, J = 12.1 Hz, 1H), 1.83 - 1.78 (m, 3H), 1.53 - 1.47 (m, 2H), 1.08-1.04 (m, 4H), 1.06 (s, 3H), 0.95 (s, 3H), 0.89-0.85 (m, 1H), 0.57-0.53 (m, 1H), 0.36 (s, 3H).

### Example 4: Synthesis of compound (1S,2S)-2-methyl-N-((1⁹S,6³S,4S,Z)-1⁹,10,10-trimethyl-1²-(4-methylpiperazin-1-yl)-5,7-dioxo-1 ⁸,1⁹,6¹,6²,6³,6⁴,6⁵,6⁶-octahydro-1⁵H,1⁷H-8-oxa-1(13,15)-pyrido[3',2':7,8][1,5]oxazonino[5,6-a]in dola-2(4,2)-thiazola-6(1,3)-pyridazinacycloundecaphane-4-yl)cyclopropane-1-carboxamide

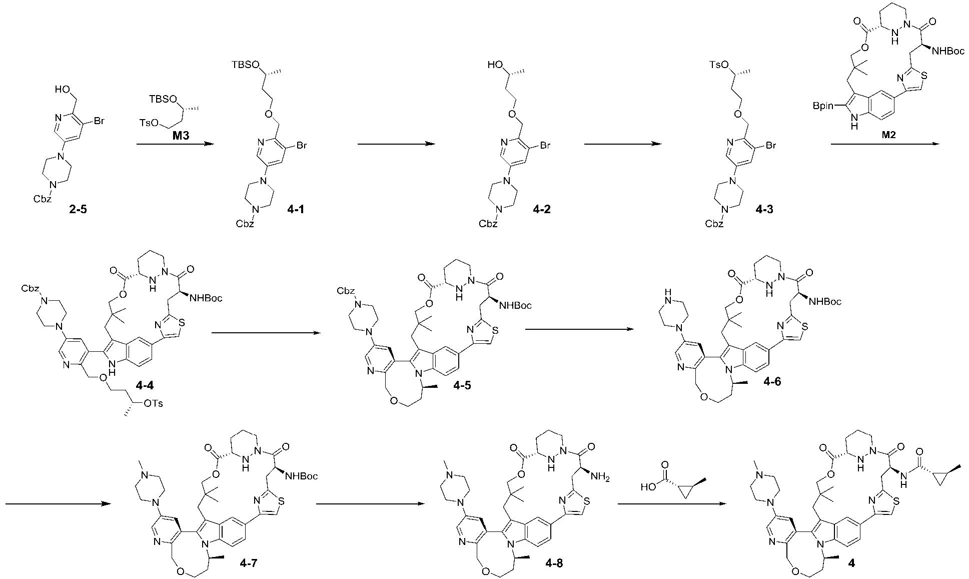

### Step 1: Synthesis of compound 4-1

Compound 2-5 (1.00 g, 2.46 mmol), M3 (1.32 g, 3.69 mmol), DMSO (10.00 mL), and THF (2 mL) were added sequentially to a 50 mL single-necked flask. Sodium tert-butoxide (0.59 g, 6.15 mmol) was added, and the reaction was carried out at room temperature. After the reaction was complete as monitored by TLC, the reaction mixture was poured into saturated aqueous ammonium chloride solution to quench. The mixture was extracted with ethyl acetate, washed with brine, separated, and the organic phase was concentrated to obtain a crude product. The crude product was purified by silica gel column chromatography to obtain the target compound 4-1 (0.5 g). ESI-MS m/z: 592.41 [M+H]⁺.

### Step 2: Synthesis of compound 4-2

Compound 4-1 (0.5 g, 0.84 mmol) and TBAF (5 mL, 1M solution in THF) were added to a 50 mL single-necked flask. The reaction was carried out at room temperature overnight. After the reaction was complete as monitored by TLC, the reaction solution was diluted with ethyl acetate, washed sequentially with saturated aqueous ammonium chloride solution and brine, separated, and the organic phase was concentrated to obtain a crude product. The crude product was purified by silica gel column chromatography to obtain the target compound 4-2 (0.43 g). ESI-MS m/z: 478.33 [M+H]⁺.

### Step 3: Synthesis of compound 4-3

Compound 4-2 (0.43 g, 0.9 mmol), DCM (5.00 mL), DIEA (0.37 mL, 2.25 mmol), and DMAP (10 mg, 0.09 mmol) were added sequentially to a 50 mL single-necked flask. Then, TosCl (0.26 g, 1.35 mmol) was added in batches. After the addition was complete, the reaction was carried out at room temperature. After the reaction was complete as monitored by LC-MS, the reaction solution was combined with silica gel for sample preparation and sand making, and purified by silica gel column chromatography to obtain compound 4-3 (185 mg). ESI-MS m/z: 632.34 [M+H]⁺.

### Step 4: Synthesis of compound 4-4

Compound 4-3 (164 mg, 0.26 mmol), M2 (150 mg, 0.22 mmol), K₂CO₃ (75 mg, 0.54 mmol), [PdCl₂(dppf)] (16 mg, 0.02 mmol), toluene (3.00 mL), dioxane (1.00 mL), and water (1.00 mL) were added sequentially to a 10 mL single-necked flask. The flask was purged and protected with nitrogen. The mixture was heated in an oil bath and reacted at 60 °C to 65 °C. After the reaction was complete as monitored by LC-MS, the reaction solution was diluted with ethyl acetate, washed sequentially with water and brine, and separated. The aqueous phase was extracted again with DCM. The organic phases were combined and concentrated to obtain a crude product. The crude product was purified by Prep-TLC to obtain compound 4-4 (276 mg). ESI-MS m/z: 1119.66 [M+H]⁺.

### Step 5: Synthesis of compound 4-5

Compound 4-4 (226 mg, 0.20 mmol), DMF (25.00 mL), and Cs₂CO₃ (197 mg, 0.61 mmol) were added sequentially to a 10 mL single-necked flask. Under nitrogen protection, the mixture was heated in an oil bath and reacted at 60-65 °C. After the reaction was complete as monitored by LC-MS, the reaction solution was diluted with ethyl acetate, washed sequentially with water and brine, separated, and concentrated to obtain a crude product of compound 4-5. The crude product was purified by silica gel column chromatography to obtain the target compound 4-5 (15 mg). ESI-MS m/z: 947.64 [M+H]⁺.

¹H NMR (500 MHz, CDCl₃) δ 8.56 (d, J = 1.7 Hz, 1H), 8.32 (d, J = 2.8 Hz, 1H), 7.58 (dd, J = 8.6, 1.7 Hz, 1H), 7.37 (d, J = 4.4 Hz, 4H), 7.37 - 7.29 (m, 2H), 7.28 (s, 1H), 7.07 (d, J = 2.8 Hz, 1H), 5.56 (dd, J = 10.2, 8.5 Hz, 1H), 5.43-5.39 (m, 1H), 5.16 (s, 2H), 4.89 (d, J = 15.9 Hz, 1H), 4.82-4.76 (m, 1H), 4.58 (d, J = 13.0 Hz, 1H), 4.52 (d, J = 16.0 Hz, 1H), 4.33 (td, J = 12.1, 3.3 Hz, 1H), 4.03 - 3.94 (m, 2H), 3.91 - 3.80 (m, 2H), 3.76 (d, J = 11.2 Hz, 1H), 3.72-3.69 (m, 4H), 3.40 (d, J = 14.9 Hz, 1H), 3.22 (s, 4H), 3.13 (d, J = 14.4 Hz, 1H), 3.04 (dd, J = 15.0, 8.7 Hz, 1H), 2.69-2.63 (m, 1H), 2.26 - 2.16 (m, 2H), 2.09 - 1.98 (m, 1H), 1.97-1.93 (m, 1H), 1.87 - 1.79 (m, 2H), 1.60-1.54 (m, 1H), 1.47 (s, 9H), 1.06 (d, J = 6.7 Hz, 3H), 0.92 (s, 3H), 0.50 (s, 3H).

### Step 6: Synthesis of compound 4-6

Compound 4-5 (15 mg, 0.02 mmol), MeOH (2.00 mL), and Pd(OH)₂/C (18 mg, 0.13 mmol, 10% Pd) were added sequentially to a 10 mL single-necked flask. The flask was purged with hydrogen, and the reaction was carried out at room temperature under a hydrogen atmosphere. After the reaction was complete as monitored by LC-MS, the mixture was filtered to remove the catalyst. The filtrate was concentrated to obtain a crude product of compound 4-6 (15 mg), which was used directly in the next reaction step. ESI-MS m/z: 813.60 [M+H]⁺.

### Step 7: Synthesis of compound 4-7

Compound 4-6 (15 mg, 0.02 mmol), MeOH (2.00 mL), 37% aqueous formaldehyde solution (15 mg), and HOAc (0.10 mL) were added sequentially to a 10 mL single-necked flask. The mixture was stirred at room temperature for 20 min. NaBH₃CN (2.3 mg, 0.04 mmol) was added, and the reaction was carried out at room temperature. After the reaction was complete as monitored by LC-MS, ethylenediamine was added to quench excess formaldehyde in the reaction solution. The mixture was then poured into saturated aqueous sodium bicarbonate solution to quench, extracted with DCM, and separated. The organic phase was concentrated to obtain a crude product of compound 4-7 (15 mg, white foamy solid). ESI-MS m/z: 827.62 [M+H]⁺.

### Step 8: Synthesis of compound 4-8

Compound 4-7 (15 mg, 0.02 mmol), DCM (1.00 mL), and HCl (4M solution in dioxane) (1.00 mL) were added sequentially to a 10 mL single-necked flask. The reaction was carried out at room temperature. After the reaction was complete as monitored by LC-MS, the reaction solution was concentrated to dryness. DCM was added, and the mixture was concentrated to dryness again to obtain a crude product of compound 4-8 (slightly yellow solid powder, 15 mg, hydrochloride salt), which was used directly in the next reaction step. ESI-MS m/z: 727.57 [M+H]⁺.

### Step 9: Synthesis of compound 4

(1S,2S)-2-methylcyclopropanecarboxylic acid (2.5 mg, 0.025 mmol), HATU (16 mg, 0.041 mmol), and DIEA (0.02 mL, 0.124 mmol) were added sequentially to a 10 mL single-necked flask. The mixture was stirred at room temperature for 30 min. After cooling in an ice-water bath, a solution of compound 4-8 (15.00 mg, 0.021 mmol) in acetonitrile was added. After the reaction was complete as monitored by LC-MS, the reaction solution was directly purified by Prep-HPLC (acidic method). The eluent was lyophilized to obtain the target compound 4 (7.9 mg). ESI-MS m/z: 809.61 [M+H]⁺.

¹H NMR (500 MHz, DMSO) δ 9.72 (s, 1H), 8.52 (d, J = 9.0 Hz, 1H), 8.44 - 8.34 (m, 2H), 7.78 (s, 1H), 7.70 (d, J = 7.5 Hz, 1H), 7.54 (d, J = 8.9 Hz, 1H), 7.33 (d, J = 2.5 Hz, 1H), 5.51 (t, J = 9.2 Hz, 1H), 5.06 (d, J = 12.1 Hz, 1H), 5.00 - 4.92 (m, 1H), 4.78 (d, J = 16.2 Hz, 1H), 4.45 (d, J = 16.1 Hz, 2H), 4.21 (dd, J = 30.6, 11.9 Hz, 3H), 4.01 - 3.83 (m, 4H), 3.76 (d, J = 12.8 Hz, 2H), 3.56 (d, J = 5.1 Hz, 2H), 3.31 (d, J = 14.3 Hz, 1H), 3.14 (dd, J = 14.7, 9.4 Hz, 2H), 3.07 - 2.99 (m, 2H), 2.95 (d, J = 14.3 Hz, 1H), 2.86 (s, 3H), 2.76 (s, 1H), 2.19 (d, J = 14.4 Hz, 1H), 2.09 (d, J = 10.4 Hz, 1H), 1.91 - 1.72 (m, 4H), 1.51 (d, J = 3.8 Hz, 2H), 1.24 (s, 1H), 1.07 (s, 3H), 0.98 (d, J = 6.6 Hz, 3H), 0.91 (s, 3H), 0.88 (s, 1H), 0.55 (d, J = 7.4 Hz, 1H), 0.42 (s, 3H).

### Example 5: Synthesis of compound (1S,2S)-2-methyl-N-((1⁵S,1⁹S,6³S,4S,Z)-1⁵,1⁹,10,10-tetramethyl-1²-(4-methylpiperazin-1-yl)-5,7 -dioxo-1⁸,1⁹,6¹,6²,6³,6⁴,6⁵,6⁶-octahydro-1⁵H,1⁷H-8-oxa-1(13,15)-pyrido[3',2':7,8][1,5]oxazonino[ 5,6-a]indola-2(4,2)-thiazola-6(1,3)-pyridazinacycloundecaphane-4-yl)cyclopropane-1-carboxami de

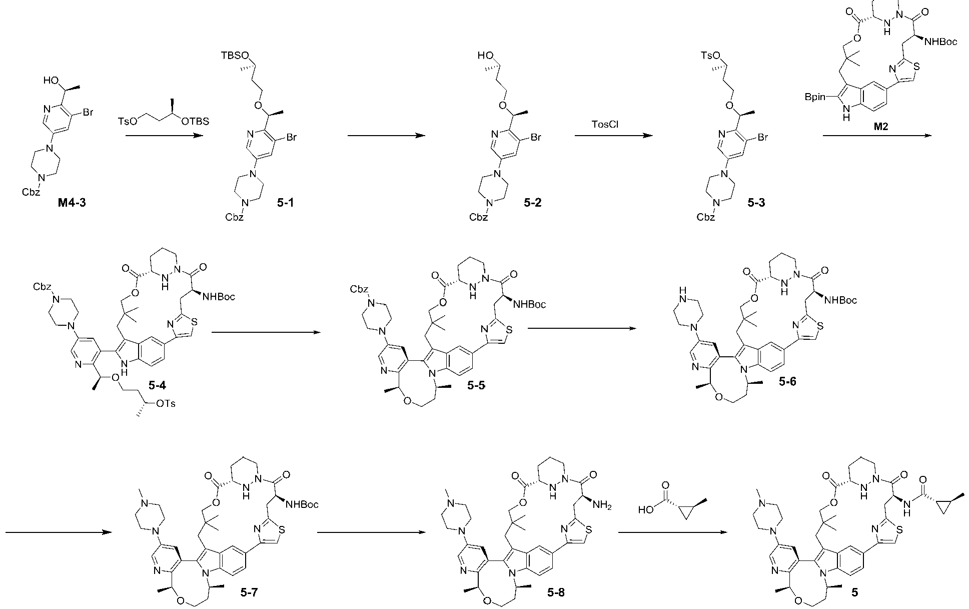

### Step 1: Synthesis of compound 5-1

Compound M4-3 (1.00 g, 2.38 mmol), (R)-3-((tert-butyldimethylsilyl)oxy)butyl 4-methylbenzenesulfonate (2.13 g, 5.95 mmol), DMSO (10.00 mL), and THF (2 mL) were added sequentially to a 50 mL single-necked flask. Sodium tert-butoxide (0.57 g, 5.95 mmol) was added, and the mixture was stirred and reacted at room temperature. After TLC monitoring indicated the reaction was complete, the reaction mixture was added to saturated aqueous ammonium chloride solution to quench. The mixture was extracted with ethyl acetate, washed with brine, separated, and the organic phase was concentrated to obtain a crude product, which was separated and purified by silica gel column chromatography to obtain the target compound 5-1 (0.77 g). ESI-MS m/z: 606.43 [M+H]⁺.

### Step 2: Synthesis of compound 5-2

Compound 5-1 (0.77 g, 1.26 mmol) and TBAF (7 mL, 1M solution in THF) were added to a 50 mL single-necked flask. The mixture was stirred and reacted at room temperature overnight. After TLC monitoring indicated the reaction was complete, the reaction solution was diluted with ethyl acetate, washed sequentially with saturated aqueous ammonium chloride solution and brine, separated, and the organic phase was concentrated to obtain a crude product, which was separated and purified by silica gel column chromatography (PE to PE/EA=50/50) to obtain the target compound 5-2 (0.55 g). ESI-MS m/z: 492.34 [M+H]⁺.

### Step 3: Synthesis of compound 5-3

Compound 5-2 (0.36 g, 0.731 mmol), DCM (5.00 mL), DIEA (0.36 mL, 2.19 mmol), and DMAP (0.179 g, 1.462 mmol) were added sequentially to a 50 mL single-necked flask. Then, TosCl (0.279 g, 1.46 mmol) was added in batches. After the addition was complete, the mixture was stirred and reacted at room temperature. After LC-MS monitoring indicated the reaction was complete, the reaction solution was combined with silica gel for sample preparation and sand making, and separated and purified by silica gel column chromatography to obtain compound 5-3 (0.23 g). ESI-MS m/z: 646.34 [M+H]⁺.

### Step 4: Synthesis of compound 5-4

Compound 5-3 (224 mg, 0.35 mmol), M2 (200 mg, 0.29 mmol), K₂CO₃ (100 mg, 0.72 mmol), [PdCl₂(dppf)] (21 mg, 0.03 mmol), toluene (3.00 mL), dioxane (1.00 mL), and water (1.00 mL) were added sequentially to a 10 mL single-necked flask. The flask was purged and protected with nitrogen. The mixture was heated in an oil bath and reacted at 60 °C to 65 °C. After LC-MS monitoring indicated the reaction was complete, the reaction solution was diluted with ethyl acetate, washed sequentially with water and brine, and separated. The aqueous phase was extracted again with DCM. The combined organic phases were concentrated to obtain a crude product, which was separated and purified by Prep-TLC to obtain compound 5-4 (246 mg). ESI-MS m/z: 1133.68 [M+H]⁺.

### Step 5: Synthesis of compound 5-5

Compound 5-4 (200 mg, 0.20 mmol), DMF (20.00 mL), and Cs₂CO₃ (291 mg, 0.89 mmol) were added sequentially to a 10 mL single-necked flask. Under nitrogen protection, the reaction was heated in an oil bath at 60-65 °C. After LC-MS monitoring indicated the reaction was complete, the reaction solution was diluted with ethyl acetate, washed sequentially with water and brine, separated, and concentrated to obtain a crude product of compound 5-5, which was separated and purified by silica gel column chromatography to obtain the target compound 5-5 (12 mg). ESI-MS m/z: 961.64 [M+H]⁺.

### Step 6: Synthesis of compound 5-6

Compound 5-5 (12 mg, 0.02 mmol), MeOH (2.00 mL), and Pd(OH)₂/C (18 mg, 0.13 mmol, 10% Pd) were added sequentially to a 10 mL single-necked flask. The flask was purged with hydrogen, and the mixture was stirred and reacted at room temperature under a hydrogen atmosphere. After LC-MS monitoring indicated the reaction was complete, the mixture was filtered to remove the catalyst. The filtrate was concentrated to obtain a crude product of compound 5-6 (12 mg), which was used directly in the next reaction step. ESI-MS m/z: 817.62 [M+H]⁺.

### Step 7: Synthesis of compound 5-7

Compound 5-6 (12 mg, 0.02 mmol), MeOH (2.00 mL), 37% aqueous formaldehyde solution (12 mg), and HOAc (0.10 mL) were added sequentially to a 10 mL single-necked flask. The mixture was stirred at room temperature for 20 min. NaBH₃CN (2 mg, 0.03 mmol) was added, and the mixture was stirred and reacted at room temperature. After LC-MS monitoring indicated the reaction was complete, ethylenediamine was added to quench excess formaldehyde in the reaction solution. The mixture was then added to saturated aqueous sodium bicarbonate solution to quench, extracted with DCM, and separated. The organic phase was concentrated to obtain a crude product of compound 5-7 (12 mg, white foamy solid). ESI-MS m/z: 841.64 [M+H]⁺.

### Step 8: Synthesis of compound 5-8

Compound 5-7 (12 mg, 0.02 mmol), DCM (1.00 mL), and HCl (4M solution in dioxane) (1.00 mL) were added sequentially to a 10 mL single-necked flask. The mixture was stirred and reacted at room temperature. After LC-MS monitoring indicated the reaction was complete, the reaction solution was concentrated to dryness. DCM was added, and the mixture was concentrated to dryness again to obtain a crude product of compound 5-8 (slightly yellow solid powder, 13 mg, hydrochloride salt), which was used directly in the next reaction step. ESI-MS m/z: 727.57 [M+H]⁺.

### Step 9: Synthesis of compound 5

(1S,2S)-2-methylcyclopropanecarboxylic acid (2 mg, 0.025 mmol), HATU (12 mg, 0.032 mmol), and DIEA (0.016 mL, 0.097 mmol) were added sequentially to a 10 mL single-necked flask. The mixture was stirred at room temperature for 30 min. After cooling in an ice-water bath, a solution of compound 5-8 (13 mg, 0.021 mmol) in acetonitrile was added. After LC-MS monitoring indicated the reaction was complete, the reaction solution was directly purified by Prep-HPLC (acidic method). The eluent was lyophilized to obtain the target compound 5 (2.1 mg). ESI-MS m/z: 823.63 [M+H]⁺.

### Example 21: Synthesis of compound (1S,2S)-N-((1⁵S,6³S,4S,Z)-12-(4-ethylpiperazin-1-yl)-1⁵,10,10-trimethyl-5,7-dioxo-1⁸,1⁹,6¹,6²,6³ ,6⁴,6⁵,6⁶-octahydro-1⁵H,1⁷H-8-oxa-1(13,15)-pyrido[3',2':7,8][1,5]oxazonino[5,6-a]indola-2(4,2)-thiazola-6(1,3)-pyridazinacycloundecaphane-4-yl)-2-methylcyclopropane-1-carboxamide

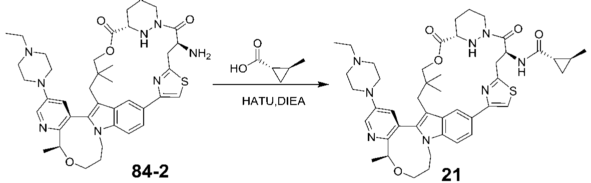

### Step 1: Synthesis of compound 21

(1S,2S)-2-methylcyclopropanecarboxylic acid (8.8 mg, 0.09 mmol), HATU (38.5 mg, 0.10 mmol), and DIEA (0.04 mL, 0.27 mmol) were added sequentially to a 10 mL single-necked flask. The mixture was stirred at room temperature for 10 min. After cooling in an ice-water bath, a solution of compound 84-2 (50.00 mg, 0.07 mmol) in acetonitrile was added. After LC-MS monitoring indicated the reaction was complete, the reaction solution was directly purified by Prep-HPLC (acidic method). The eluent was lyophilized to obtain the target compound 21 (10.0 mg). ESI-MS m/z: 823.63 [M+H]⁺.

¹H NMR (500 MHz, CDCl₃) δ 8.59 (s, 1H), 8.43 (s, 1H), 7.58 (d, J = 8.1 Hz, 1H), 7.36 - 7.27 (m, 2H), 7.09 (s, 1H), 6.46 (d, J = 9.0 Hz, 1H), 5.89 (t, J = 11.0 Hz, 1H), 4.61 (d, J = 12.1 Hz, 1H), 4.36 (t, J =11.4 Hz, 1H), 4.28 - 4.17 (m, 2H), 4.06 - 3.96 (m, 2H), 3.86 - 3.75 (m, 3H), 3.45 (d, J = 14.7 Hz, 1H), 3.40 - 3.28 (m, 5H), 3.19 (d, J = 14.0 Hz, 1H), 3.15 - 3.08 (m, 1H), 2.80 - 2.71 (m, 4H), 2.60 (d, J =6.4 Hz, 2H), 2.41 (d, J = 14.4 Hz, 1H), 2.37 - 2.20 (m, 3H), 2.01 - 1.92 (m, 2H), 1.87 - 1.77 (m, 1H), 1.65 - 1.57 (m, 1H), 1.55 - 1.46 (m, 3H), 1.38 - 1.32 (m, 1H), 1.29 - 1.23 (m, 2H), 1.22 - 1.15 (m, 4H), 1.13 - 1.07 (m, 3H), 0.96 (s, 3H), 0.40 (s, 3H).

### Example 22: Synthesis of compound (1S,2S)-N-((1⁵S,6³S,4S,Z)-1²-(4-(2-methoxyethyl)piperazin-1-yl)-1⁵,10,10-trimethyl-5,7-dioxo-1 ⁸,1⁹,6¹,6²,6³,6⁴,6⁵,6⁶-octahydro-1⁵H,1⁷H-8-oxa-1(13,15)-pyrido[3',2':7,8][1,5]oxazonino[5,6-a]in dola-2(4,2)-thiazola-6(1,3)-pyridazinacycloundecaphane-4-yl)-2-methylcyclopropane-1-carboxa mide

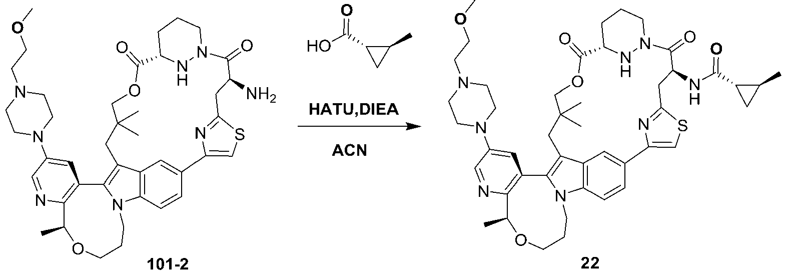

Compound (1S,2S)-2-methylcyclopropanecarboxylic acid (5.85 mg, 0.06 mmol) was dissolved in acetonitrile (2.00 mL). DIEA (0.06 mL, 0.39 mmol) and HATU (22.21 mg, 0.06 mmol) were added sequentially under ice-water bath cooling. After reacting in the ice-water bath for 10 min, a solution of compound 101-2 (30.00 mg, 0.04 mmol) in acetonitrile (2.00 mL) and DIEA (0.06 mL, 0.39 mmol) was added and reacted at room temperature for 1 h. The mixture was concentrated to dryness and purified by preparative separation to obtain compound 22 (19.00 mg, 56.87%). ESI-MS m/z: 853.79 [M+H]⁺.

¹H NMR (500 MHz, DMSO) δ 8.56 (d, J = 9.0 Hz, 1H), 8.49 (s, 1H), 8.40 (d, J = 2.7 Hz, 1H), 7.81 (s, 1H), 7.75 - 7.68 (m, 1H), 7.49 (d, J = 8.6 Hz, 1H), 7.21 (d, J = 2.7 Hz, 1H), 5.52 (t, J = 9.3 Hz, 1H), 5.08 (d, J = 12.2 Hz, 1H), 4.23 (dd, J = 11.9, 9.4 Hz, 2H), 4.15 (q, J = 6.2 Hz, 1H), 4.08 - 3.97 (m, 2H), 3.67 (d, J = 11.0 Hz, 1H), 3.60 (t, J = 13.7 Hz, 2H), 3.46 (t, J = 5.7 Hz, 2H), 3.24 (s, 7H), 3.15 (dt, J = 14.6, 9.8 Hz, 2H), 3.00 (d, J = 14.4 Hz, 1H), 2.75 (dd, J = 16.2, 12.0 Hz, 1H), 2.56 (s, 3H), 2.41 (d, J = 14.4 Hz, 1H), 2.30 (s, 1H), 2.11 (d, J = 10.3 Hz, 1H), 1.80 (s, 3H), 1.56 - 1.45 (m, 3H), 1.37 (d, J = 6.3 Hz, 4H), 1.07 (s, 5H), 0.95 (s, 3H), 0.86 (dd, J = 7.6, 4.1 Hz, 3H), 0.56 (dd, J = 7.4, 5.6 Hz, 1H), 0.32 (s, 3H).

### Example 37: Synthesis of compound N-((1⁵S,6³S,4S,Z)-1²-(4-ethylpiperazin-1-yl)-1⁵,10,10-trimethyl-5,7-dioxo-1⁸,1⁹,6¹,6²,6³,6⁴,6⁵6⁶-octahydro-1⁵H,1⁷H-8-oxa-1(13,15)-pyrido[3',2':7,8][1,5]oxazonino[5,6-a]indola-2(4,2)-thiazola-6(1,3)-pyridazinacycloundecaphane-4-yl)-3-methylbicyclo[1.1.1]pentane-1-carboxamide

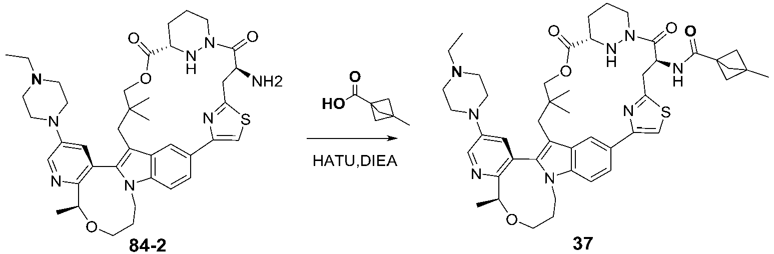

### Step 1: Synthesis of compound 37

1-Methylbicyclo[1.1.1]pentane-3-carboxylic acid (11.76 mg, 0.09 mmol) and N,N-dimethylformamide (1.00 mL) were added sequentially to a 10 mL single-necked flask. At 0 °C, N,N,N',N'-tetramethyl-O-(7-azabenzotriazol-1-yl)uronium hexafluorophosphate (35.44 mg, 0.09 mmol) and N,N-diisopropylethylamine (0.10 mL, 0.62 mmol) were added. The mixture was stirred at 0 °C for 10 min. Finally, compound 84-2 (46.00 mg, 0.06 mmol) was added, and the mixture was stirred and reacted at 0 °C for 10 min. After LC-MS monitoring indicated the reaction was complete, the reaction solution was directly purified by prep-HPLC to obtain the target compound 37 (16.70 mg). ESI-MS m/z: 849.8 [M+H]⁺.

¹H NMR (500 MHz, Methanol-d₄) δ 8.56 (d, J = 1.5 Hz, 1H), 8.38 (d, J = 2.9 Hz, 1H), 7.68 (dd, J = 8.6, 1.6 Hz, 1H), 7.54 (s, 1H), 7.44 (d, J = 8.6 Hz, 1H), 7.31 (d, J = 3.0 Hz, 1H), 5.69 - 5.63 (m, 1H), 4.60 (s, 2H), 4.42 (d, J = 12.8 Hz, 1H), 4.33 - 4.26 (m, 2H), 4.15 (dd, J = 12.4, 5.7 Hz, 1H), 4.06 (dd, J = 14.5, 5.0 Hz, 1H), 3.82 - 3.68 (m, 3H), 3.43 - 3.39 (m, 5H), 3.29 - 3.25 (m, 1H), 3.20 (d, J = 14.5 Hz, 1H), 2.87 - 2.74 (m, 5H), 2.72 - 2.63 (m, 2H), 2.49 (d, J = 14.8 Hz, 1H), 2.38 (t, J = 13.6 Hz, 1H), 2.27 - 2.20 (m, 1H), 1.98 (s, 6H), 1.83 (d, J = 12.9 Hz, 1H), 1.62-1.57 (m, 1H), 1.47 (d, J = 6.5 Hz, 3H), 1.24 - 1.17 (m, 6H), 1.00 (s, 3H), 0.41 (s, 3H).

### Example 38: Synthesis of compound N-((1⁵S,6³S,4S,Z)-1²-(4-(2-methoxyethyl)piperazin-1-yl)-1⁵,10,10-trimethyl-5,7-dioxo-1⁸,1⁹,6¹,6 ²,6³,6⁴,6⁵,6⁶ -octahydro-1⁵H,1⁷H-8-oxa-1(13,15)-pyrido[3',2':7,8][1,5]oxazonino[5,6-a]indola-2(4 ,2)-thiazola-6(1,3)-pyridazinacycloundecaphane-4-yl)-3-methylbicyclo[1.1.1]pentane-1-carboxa mide

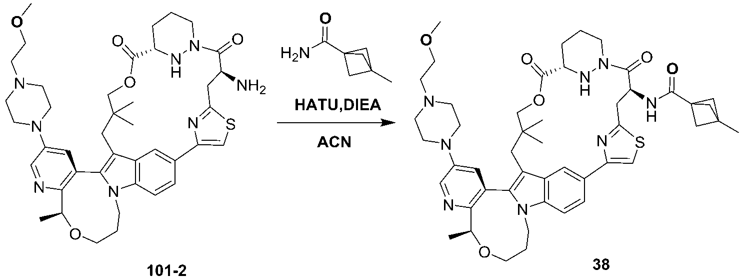

3-Methylbicyclo[1.1.1]pentane-1-carboxamide (5.89 mg, 0.05 mmol) was dissolved in acetonitrile (2.00 mL). DIEA (0.06 mL, 0.39 mmol) and HATU (22.21 mg, 0.06 mmol) were added sequentially under ice-water bath cooling. After reacting in the ice-water bath for 10 min, a solution of compound 101-2 (30.00 mg, 0.04 mmol) in acetonitrile (2.00 mL) and DIEA (0.06 mL, 0.39 mmol) was added, and reacted at room temperature for 1 h. The mixture was concentrated to dryness and purified by preparative separation to obtain compound 38 (9.00 mg, 25.22%). ESI-MS m/z: 879.91 [M+H]⁺.

### Example 39: Synthesis of compound 3-methyl-N-((1⁵S,6³S,4S,Z)-1⁵,10,10-trimethyl-5,7-dioxo-1²-(4-(2,2,2-trifluoroethyl)piperazin-1-yl)-1⁸,1⁹,6¹,6²,6³,6⁴,6⁵,6⁶-octahydro-1⁵H,1⁷H-8-oxa-1(13,15)-pyrido[3',2':7,8][1,5]oxazonino[5,6 -a]indola-2(4,2)-thiazola-6(1,3)-pyridazinacycloundecaphane-4-yl)bicyclo[1.1.1]pentane-1-carbo xamide

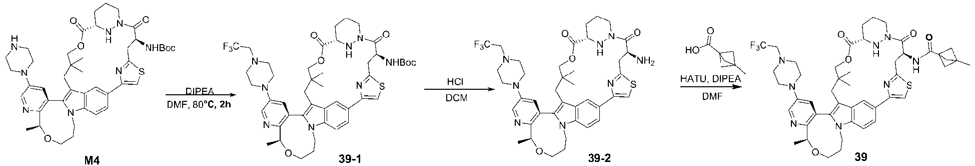

### Step 1: Synthesis of compound 39-1

Compound M4 (90.00 mg, 0.11 mmol), DIPEA (0.19 mL, 1.11 mmol), and 2,2,2-trifluoroethyl p-toluenesulfonate (56.29 mg, 0.22 mmol) were dissolved in DMF (2.00 mL) and reacted at 80 °C for 3 h. After LC-MS monitoring indicated the reaction was complete, the reaction solution was diluted with ethyl acetate, washed sequentially with water and brine, separated, and the organic phase was concentrated to obtain a crude product. Purification by silica gel column chromatography yielded the target compound 39-1 (75 mg). ESI-MS m/z: 894.41, 895.42 [M+H]⁺.

### Step 2: Synthesis of compound 39-2

Compound 39-1 (65.00 mg, 0.07 mmol) was dissolved in DCM (1.00 mL). Then, HCl (4M solution in dioxane) (1.50 mL) was added, and the mixture was reacted at room temperature for 30 min. The reaction solution was concentrated to obtain a crude product of compound 39-2 (95 mg), which was used directly in the next reaction step. ESI-MS m/z: 794.35, 795.34 [M+H]⁺.

### Step 3: Synthesis of compound 39

1-Methylbicyclo[1.1.1]pentane-3-carboxylic acid (20.23 mg, 0.16 mmol) was dissolved in DMF (1.00 mL) and cooled to 0 °C. Then, DIPEA (0.19 mL, 1.07 mmol) and HATU (81.29 mg, 0.21 mmol) were added. After reacting at 0 °C for 5 min, compound 39-2 (85.00 mg, 0.11 mmol) was added, and the reaction was continued at 0 °C for 5 min. After LC-MS monitoring indicated the reaction was complete, purification by preparative chromatography (acetonitrile:water (containing 0.05% diethylamine) = 5:1) was performed to obtain the target compound 39 (51 mg). ESI-MS m/z: 902.41, 903.42 [M+H]⁺.

¹H NMR (500 MHz, CDCl₃) δ 8.59 (d, J = 1.7 Hz, 1H), 8.42 (d, J = 2.9 Hz, 1H), 7.58 (dd, J = 8.6, 1.6 Hz, 1H), 7.31 (d, J = 8.6 Hz, 1H), 7.28 (s, 1H), 7.08 (d, J = 3.0 Hz, 1H), 6.36 (d, J = 9.7 Hz, 1H), 5.87 - 5.78 (m, 1H), 4.58 (d, J = 12.8 Hz, 1H), 4.34 (td, J = 12.1, 3.3 Hz, 1H), 4.22 (dq, J = 17.7, 6.1 Hz, 2H), 4.05 - 3.96 (m, 2H), 3.86 - 3.74 (m, 3H), 3.46 (d, J = 14.9 Hz, 1H), 3.35 - 3.27 (m, 5H), 3.19 (d, J = 14.4 Hz, 1H), 3.15 - 3.02 (m, 3H), 2.87 (q, J = 4.2 Hz, 4H), 2.72 - 2.63 (m, 1H), 2.41 (d, J = 14.3 Hz, 1H), 2.32 (t, J = 14.0 Hz, 1H), 2.28 - 2.19 (m, 1H), 1.97 (s, 6H), 1.88 - 1.76 (m, 1H), 1.71 - 1.60 (m, 6H), 1.51 (d, J = 6.4 Hz, 3H), 1.25 (s, 1H), 1.22 (s, 3H), 0.96 (s, 3H), 0.40 (s, 3H).

### Example 40: Synthesis of compound N-((1⁵S,6³S,4S,Z)-1²-(4-cyclopropylpiperazin-1-yl)-1⁵,10,10-trimethyl-5,7-dioxo-1⁸,1⁹,6¹,6²,6³,6 ⁴,6⁵,6⁶-octahydro-1⁵H,1⁷H-8-oxa-1(13,15)-pyrido[3',2':7,8][1,5]oxazonino[5,6-a]indola-2(4,2)-th iazola-6(1,3)-pyridazinacycloundecaphane-4-yl)-3-methylbicyclo[1.1.1]pentane-1-carboxamide

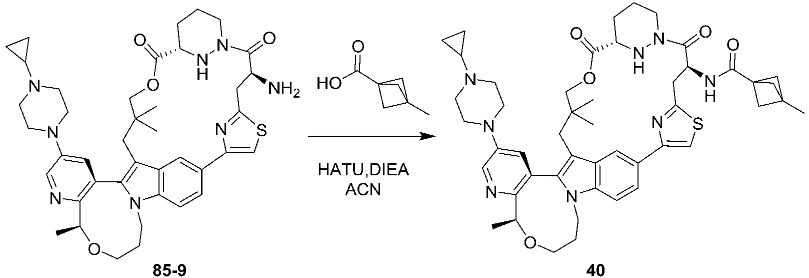

### Step 1: Synthesis of compound 40

1-Methylbicyclo[1.1.1]pentane-3-carboxylic acid (34.19 mg, 0.27 mmol) was dissolved in acetonitrile (4.00 mL). DIEA (0.37 mL, 2.26 mmol) and HATU (128.78 mg, 0.34 mmol) were added sequentially under ice-water bath cooling. After reacting in the ice-water bath for 10 min, a solution of compound 85-9 (170.00 mg, 0.23 mmol) in acetonitrile (4.00 mL) and DIEA (0.37 mL, 2.26 mmol) was added, and reacted at room temperature for 1 h. The mixture was concentrated to dryness and purified by preparative separation to obtain compound 40 (67.00 mg, 33.84%). ESI-MS m/z: 861.48 [M+H]⁺.

¹H NMR (500 MHz, DMSO) δ 8.51 (d, J = 1.0 Hz, 1H), 8.40 (d, J = 2.8 Hz, 1H), 8.00 (d, J = 9.0 Hz, 1H), 7.79 (s, 1H), 7.70 (dd, J = 8.6, 1.4 Hz, 1H), 7.48 (d, J = 8.6 Hz, 1H), 7.21 (d, J = 2.8 Hz, 1H), 5.48 (t, J = 9.4 Hz, 1H), 5.09 (d, J = 12.2 Hz, 1H), 4.29 - 4.19 (m, 2H), 4.15 (q, J = 6.3 Hz, 1H), 4.03 (dd, J = 12.6, 4.9 Hz, 2H), 3.69 (d, J = 11.0 Hz, 1H), 3.64 - 3.55 (m, 2H), 3.38 (dd, J = 14.6, 9.3 Hz, 1H), 3.29 (d, J = 14.3 Hz, 1H), 3.24 - 3.13 (m, 5H), 3.01 (d, J = 14.4 Hz, 1H), 2.75 (td, J = 12.6, 50 Hz, 1H), 2.69 (d, J = 17.2 Hz, 4H), 2.41 (d, J = 14.3 Hz, 1H), 2.29 (t, J = 10.3 Hz, 1H), 2.11 (d, J = 10.0 Hz, 1H), 1.93 - 1.85 (m, 6H), 1.80 (s, 3H), 1.65 (ddd, J = 10.1, 6.6, 3.6 Hz, 1H), 1.52 (dt, J = 18.1, 12.5 Hz, 1H), 1.37 (d, J = 6.4 Hz, 3H), 1.19 (s, 3H), 0.95 (s, 3H), 0.50 - 0.40 (m, 2H), 0.33 (dd, J = 7.3, 4.8 Hz, 5H).

### Example 80: Synthesis of compound 3-methyl-N-((1⁵3 ³S,4S,Z)-1⁵,10,10-trimethyl-1²-(4-methylpiperazin-1-yl)-5,7-dioxo-1⁸,1⁹,6¹,6² ,6³,6⁴,6⁵,⁶-octahydro-1⁵H,1⁷H-8-oxa-1(13,15)-pyrido[3',2':7,8][1,5]oxazonino[5,6-a]indola-2(4, 2)-thiazola-6(1,3)-pyridazinacycloundecaphane-4-yl)bicyclo[1.1.1]pentane-1-carboxamide

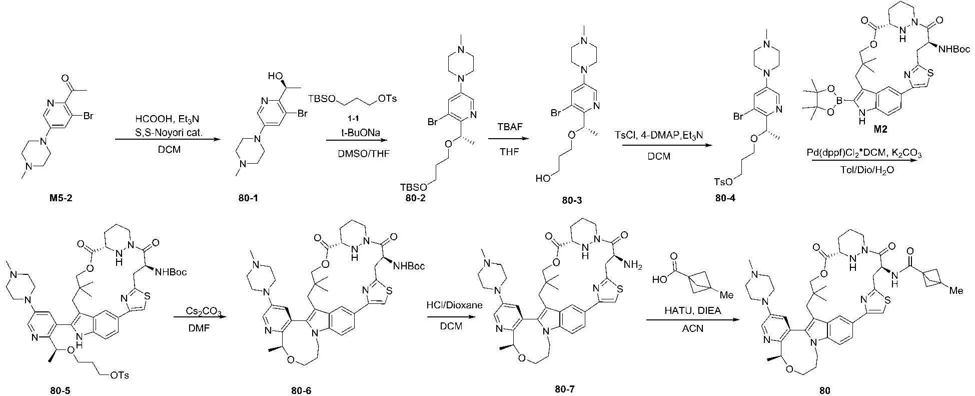

### Step 1: Synthesis of compound 80-1

Compound M5-2 (2.00 g), triethylamine (4.66 mL), and (S,S)-N-(p-toluenesulfonyl)-1,2-diphenylethane-1,2-diamine (p-cymene)ruthenium(II) chloride (0.21 g) were added to DCM (10.00 mL). Then, under N₂ protection and ice-water bath conditions, formic acid (1.27 mL) was slowly added in batches. The mixture was allowed to warm to room temperature and reacted for 72 h. After the reaction was complete, 100 mL of water was added to the reaction solution to quench. The mixture was then extracted four times with EA (60 mL). The combined organic phases were washed with saturated sodium chloride and concentrated to obtain a crude product. Purification by column chromatography yielded the target compound 80-1 (1.80 g, 89.40% yield). ESI-MS m/z: 300.2, 302.2 [M+H]⁺.

### Step 2: Synthesis of compound 80-2

Compound 80-1 (1.00 g) and compound 1-1 (2.30 g) were added to DMSO (15.00 mL) and THF (2.00 mL). Then, under N₂ protection at room temperature, a solution of sodium tert-butoxide (0.80 g) in THF (2.00 mL) was slowly added. The mixture was stirred and reacted at 40 °C for 5 h. After the reaction was complete, 30 mL of water was added to quench. The mixture was extracted three times with EA (30 mL). The combined organic phases were washed with saturated sodium chloride and concentrated to obtain a crude product. Purification by column chromatography yielded the target compound 80-2 (1.00 g, 63.53% yield). ESI-MS m/z: 472.5, 474.5 [M+H]⁺.

### Step 3: Synthesis of compound 80-3

Compound 80-2 (1.00 g) and TBAF (4.23 mL, 1.00 mol/L) were added to THF (2.00 mL). Then, under N₂ protection, the mixture was stirred at 50 °C for 1 h. After the reaction was complete, 30 mL of water was added to quench. The mixture was extracted five times with DCM (30 mL). The combined organic phases were washed with saturated sodium chloride and concentrated to obtain a crude product. Purification by column chromatography (DCM:MeOH=97:3 to 90:10) yielded the target compound 80-3 (0.78 g, 100% yield). ESI-MS m/z: 358.3, 360.3 [M+H]⁺.

### Step 4: Synthesis of compound 80-4

Compound 80-3 (780.00 mg), triethylamine (0.76 mL), and DMAP (26.60 mg) were sequentially added to a DCM (10.00 mL) solution. Under N₂ protection, the temperature was lowered to 0 °C, and TsCl (830.12 mg) was slowly added. The mixture was then allowed to warm to room temperature and reacted for 12 h. After the reaction was complete, 100 mL of water was added to the reaction solution to quench. The mixture was then extracted three times with DCM (50 mL) and washed twice with saturated sodium chloride (50 mL). The combined organic phases were concentrated to obtain a crude product. Purification by column chromatography yielded the target compound 80-4 (1300.00 mg, 100% yield). ESI-MS m/z: 512.4, 514.4 [M+H]⁺.

### Step 5: Synthesis of compound 80-5

Compound 80-4 (265.97 mg), intermediate M2 (300.00 mg), PdCl₂(dppf) (31.61 mg), and K₂CO₃ (149.43 mg) were added to toluene (4.50 mL), 1,4-dioxane (1.50 mL), and water (1.00 mL). Then, under N₂ protection, the mixture was stirred at 65 °C for 2 h. After the reaction was complete, 30 mL of water was added to quench. The mixture was extracted three times with DCM (30 mL) and washed twice with saturated sodium chloride (30 mL). The combined organic phases were concentrated to obtain a crude product. Purification by column chromatography yielded the target compound 80-5 (280.00 mg, 64.79% yield). ESI-MS m/z: 1000.2 [M+H]⁺.

### Step 6: Synthesis of compound 80-6

Compound 80-5 (280.00 mg) and Cs₂CO₃ (183.00 mg) were added to DMF (3.00 mL). Then, under N₂ protection, the mixture was stirred at 90 °C for 0.5 h. After the reaction was complete, 50 mL of water was added to quench. The mixture was extracted three times with DCM (40 mL) and washed four times with saturated sodium chloride (30 mL). The combined organic phases were concentrated to obtain a crude product. Purification by column chromatography yielded compound 80-6 (170.00 mg, 75.52% yield). ESI-MS m/z: 828.1 [M+H]⁺.

¹H NMR (500 MHz, CDCl₃) δ 8.60 (d, J = 1.6 Hz, 1H), 8.42 (d, J = 3.0 Hz, 1H), 7.58 (dd, J = 8.5, 1.7 Hz, 1H), 7.32-7.27 (m, 2H), 7.09 (d, J = 2.9 Hz, 1H), 5.58 (t, J = 9.4 Hz, 1H), 5.48 (d, J = 10.4 Hz, 1H), 4.61-4.56 (m, 1H), 4.38-4.32 (m, 1H), 4.26-4.17 (m, 2H), 4.09 - 3.95 (m, 2H), 3.87 - 3.73 (m, 3H), 3.41 (d, J = 14.8 Hz, 1H), 3.35-3.31 (m, 5H), 3.20 (d, J = 14.4 Hz, 1H), 3.05 (dd, J = 14.9, 8.8 Hz, 1H), 2.68 (td, J = 12.9, 3.0 Hz, 1H), 2.62-3.58 (m, 4H), 2.43 (d, J = 14.4 Hz, 1H), 2.36 (s, 3H), 2.32 - 2.29 (m,1H), 2.22 - 2.18 (m,1H), 1.99-1.92 (m, 2H), 1.84-1.76 (m, 1H), 1.62-1.56 (m, 1H), 1.51 (d, J = 6.5 Hz, 3H), 1.51 - 1.41 (m, 9H), 0.97 (s, 3H), 0.41 (s, 3H).

### Step 7: Synthesis of compound 80-7

Compound 80-6 (175.00 mg) was added to DCM (3.00 mL). Then, under N₂ protection and ice-water bath conditions, HCl/dioxane (2 mL, 4M) was slowly added. The mixture was allowed to warm to room temperature and reacted for 3 h. After the reaction was complete, the reaction solution was concentrated to obtain a crude product. Toluene (10 mL) was added to the crude product, and the mixture was concentrated to obtain the target compound 80-7 (133.00 mg, 86.47% yield). ESI-MS m/z: 728.0 [M+H]⁺.

### Step 8: Synthesis of compound 80

1-Methylbicyclo[1.1.1]pentane-3-carboxylic acid (19.78 mg, 0.16 mmol), HATU (91.41 mg, 0.24 mmol), acetonitrile (3.00 mL), and DIEA (0.14 mL, 0.84 mmol) were added sequentially to a 10 mL single-necked flask. The mixture was stirred at room temperature for 1 h. After cooling in an ice-water bath, a solution of compound 80-7 (76.00 mg, 0.10 mmol) in acetonitrile was added dropwise, and the mixture was stirred and reacted. After LC-MS monitoring indicated the reaction was complete, the reaction solution was diluted with ethyl acetate, washed with saturated aqueous ammonium chloride solution and brine, and concentrated to obtain a crude product. The crude product was purified by preparative HPLC (acidic method). The eluent was adjusted to pH=8 with saturated aqueous sodium bicarbonate solution, extracted with DCM, and concentrated to remove DCM. The obtained compound was dissolved in acetonitrile, purified water was added, and the mixture was lyophilized to obtain the target compound 80 (48.4 mg). ESI-MS m/z: 835.78 [M+H]⁺.

¹H NMR (500 MHz, CDCl₃) δ 8.58 (d, J = 1.6 Hz, 1H), 8.42 (d, J = 3.0 Hz, 1H), 7.57 (dd, J = 8.6, 1.7 Hz, 1H), 7.31 (d, J = 8.6 Hz, 1H), 7.28 (s, 1H), 7.09 (d, J = 3.0 Hz, 1H), 6.37 (d, J = 9.7 Hz, 1H), 5.85-5.81 (m, 1H), 4.57 (d, J = 12.7 Hz, 1H), 4.36-4.30 (m, 1H), 4.25-4.18 (m, 2H), 4.05 - 3.96 (m, 2H), 3.85 - 3.74 (m, 3H), 3.46 (d, J = 14.9 Hz, 1H), 3.31-3.27 (m, 5H), 3.19 (d, J = 14.4 Hz, 1H), 3.11 (dd, J = 15.0, 8.5 Hz, 1H), 2.70-2.64 (m, 1H), 2.64-2.61 (m, 4H), 2.42 (d, J = 14.5 Hz, 1H), 2.38 (s, 3H), 2.36 - 2.21 (m, 2H), 2.00 - 1.93 (m, 8H), 1.82-1.79 (m, 1H), 1.64-1.57 (m, 1H), 1.51 (d, J = 6.4 Hz, 3H), 1.22 (s, 3H), 0.97 (s, 3H), 0.41 (s, 3H).

### Example 81: Synthesis of compound (1S,2S)-2-(difluoromethyl)-N-((1⁵S,6³S,4S,Z)-1⁵,10,10-trimethyl-1²-(4-methylpiperazin-1-yl)-5, 7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxazonin o[5,6-a]indola-2(4,2)-thiazola-6(1,3)-pyridazinacycloundecaphane-4-yl)cyclopropane-1-carboxa mide

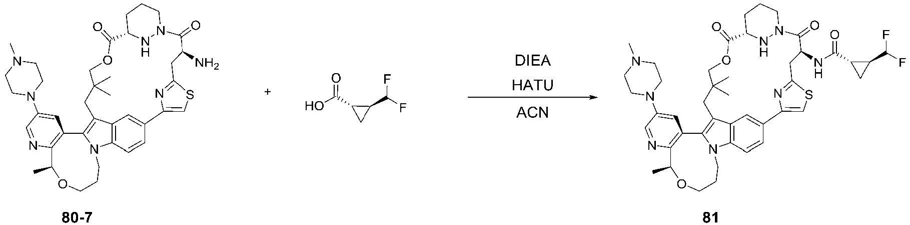

(1S,2S)-2-(difluoromethyl)cyclopropanecarboxylic acid (21.34 mg, 0.16 mmol), acetonitrile (3.00 mL), HATU (99.35 mg, 0.26 mmol), and DIEA (0.14 mL, 0.84 mmol) were added sequentially to a 10 mL single-necked flask. The mixture was stirred at room temperature for 1 h. After cooling in an ice-water bath, a solution of compound 80-7 (76.00 mg, 0.10 mmol) in acetonitrile was added, and the mixture was stirred and reacted. After LC-MS monitoring indicated the reaction was complete, the reaction solution was diluted with ethyl acetate, washed with saturated aqueous ammonium chloride solution and brine, and concentrated to obtain a crude product. The crude product was purified by preparative HPLC (acidic method). The eluent was adjusted to pH about 8 with saturated aqueous sodium bicarbonate solution, extracted with DCM, and concentrated to remove DCM. The obtained compound was dissolved in acetonitrile, purified water was added, and the mixture was lyophilized to obtain the target compound 81 (48.9 mg). ESI-MS m/z: 845.64 [M+H]⁺.

¹H NMR (500 MHz, CDCl₃) δ 8.58 (d, J = 1.6 Hz, 1H), 8.42 (d, J = 2.9 Hz, 1H), 7.57 (dd, J = 8.5, 1.7 Hz, 1H), 7.31 (d, J = 8.5 Hz, 1H), 7.28 (s, 1H), 7.09 (d, J = 2.9 Hz, 1H), 6.68 (d, J = 9.5 Hz, 1H), 5.96 - 5.71 (m, 2H), 4.60 (d, J = 13.1 Hz, 1H), 4.35 (td, J = 12.2, 3.4 Hz, 1H), 4.24-4.18 (m, 2H), 4.06 - 3.96 (m, 2H), 3.82 (s, 2H), 3.83 - 3.74 (m, 1H), 3.47 (d, J = 14.9 Hz, 1H), 3.33-3.28 (m, 5H), 3.19 (d, J = 14.4 Hz, 1H), 3.14 (dd, J = 15.0, 8.5 Hz, 1H), 2.69 (td, J = 12.9, 3.0 Hz, 1H), 2.64-2.61 (m, 4H), 2.42 (d, J = 14.4 Hz, 1H), 2.38 (s, 3H), 2.33-2.23 (m, 2H), 1.98-1.87 (m, 2H), 1.85-1.81 (m, 1H), 1.75 - 1.66 (m, 2H), 1.67 - 1.55 (m, 1H), 1.51 (d, J = 6.5 Hz, 3H), 1.32 (dt, J = 9.1, 4.3 Hz, 1H), 1.19 - 1.11 (m, 1H), 0.97 (s, 3H), 0.41 (s, 3H).

### Example 83: Synthesis of compound (1S,2S)-2-(difluoromethyl)-N-((1⁵S,6³S,4S,Z)-1⁵,10,10-trimethyl-1²-morpholino-5,7-dioxo-1⁸,1⁹, 6¹,6²,6³,6⁴,6⁵⁶-octahydro-1⁵H,1⁷H-8-oxa-1(13,15)-pyrido[3',2':7,8][1,5]oxazonino[5,6-a]indola -2(4,2)-thiazola-6(1,3)-pyridazinacycloundecaphane-4-yl)cyclopropane-1-carboxamide

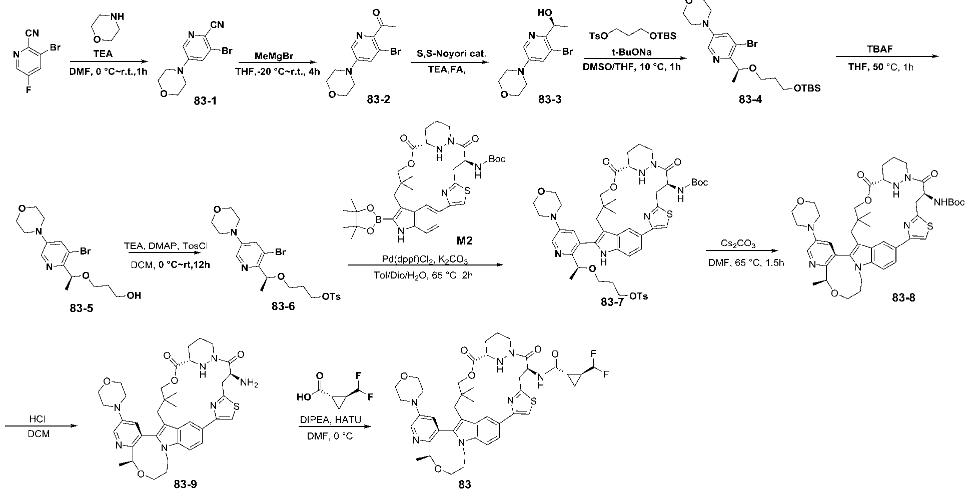

### Step 1: Synthesis of compound 83-1

Compound 3-bromo-5-fluoro-pyridine-2-carbonitrile (3.00 g, 14.93 mmol) was dissolved in 30 mL of DMF and cooled to 0 °C. Then, morpholine (1.43 g, 16.42 mmol) and triethylamine (4.15 mL, 29.85 mmol) were added sequentially. The mixture was stirred and reacted at room temperature for 1 h. After LC-MS monitoring indicated the reaction was complete, the reaction solution was diluted with ethyl acetate, washed sequentially with water and brine, separated, and concentrated. The concentrate was slurried with DCM/PE (1:10), filtered, and dried to obtain the target compound 83-1 (4.17 g). ESI-MS m/z: 267.00, 268.12 [M+H]⁺.

### Step 2: Synthesis of compound 83-2

Compound 83-1 (4.00 g, 14.92 mmol) was dissolved in 40.00 mL of tetrahydrofuran and cooled to -20 °C. Then, MeMgBr (4.45 g, 37.30 mmol) was added, and the mixture was allowed to warm to room temperature and reacted for 4 h. After LC-MS monitoring indicated the reaction was complete, 6M HCl was added dropwise to quench. Saturated sodium bicarbonate was then added to adjust the pH of the system to 7 to 8. The reaction solution was diluted with ethyl acetate, washed sequentially with water and brine, separated, and the organic phase was concentrated to obtain a crude product, which was separated and purified by silica gel column chromatography yielded the target compound 83-2 (2.75 g). ESI-MS m/z: 284.02, 285.11 [M+H]⁺.

### Step 3: Synthesis of compound 83-3

Triethylamine (29.25 mL, 210.43 mmol) was dissolved in a 50 mL three-necked flask under nitrogen protection and cooled to 0 °C. Formic acid (2.02 g, 43.84 mmol) was then slowly added. Then, (S,S)-N-(p-toluenesulfonyl)-1,2-diphenylethane-1,2-diamine (p-cymene)ruthenium(II) chloride (0.28 g, 0.44 mmol) was added. The mixture was reacted at 40 °C for 15 min, then cooled to room temperature. Compound 83-2 (2.50 g, 8.77 mmol) was added and the mixture was reacted at room temperature for 12 h. After LC-MS monitoring indicated the reaction was complete, the reaction solution was diluted with DCM, washed sequentially with water and brine, separated, and the organic phase was concentrated to obtain a crude product. Purification by silica gel column chromatography yielded the target compound 83-3 (2.37 g). ESI-MS m/z: 286.03, 287.07 [M+H]⁺.

### Step 4: Synthesis of compound 83-4

Compound 83-3 (2.27 g, 7.91 mmol) and compound 3-((tert-butyldimethylsilyl)oxy)propyl 4-methylbenzenesulfonate (9.53 g, 27.67 mmol) were dissolved in DMSO (20.00 mL) and THF (4.00 mL) under nitrogen protection and cooled to 10 °C. Then, sodium tert-butoxide (1.90 g, 19.76 mmol) was added and the mixture was reacted at 10 °C for 1 h. After LC-MS monitoring indicated the reaction was complete, the reaction solution was diluted with ethyl acetate, washed sequentially with water and brine, separated, and the organic phase was concentrated to obtain a crude product. Purification by silica gel column chromatography yielded the target compound 83-4 (2.72 g). ESI-MS m/z: 458.16, 458.18 [M+H]⁺.

### Step 5: Synthesis of compound 83-5

Compound 83-4 (2.60 g, 5.66 mmol) was dissolved in THF (20.00 mL). Then, TBAF (11.32 mL, 1.00 M, 11.32 mmol) was added, and the mixture was reacted at 50 °C for 1 h. After LC-MS monitoring indicated the reaction was complete, the reaction solution was diluted with ethyl acetate, washed sequentially with water and brine, separated, and the organic phase was concentrated to obtain a crude product. Purification by silica gel column chromatography yielded the target compound 83-5 (1.93 g). ESI-MS m/z: 344.07, 344.11 [M+H]⁺.

### Step 6: Synthesis of compound 83-6

Compound 83-5 (2.10 g, 6.08 mmol), TEA (2.54 mL, 18.25 mmol), and DMAP (0.37 g, 3.04 mmol) were dissolved in DCM (20.00 mL) under nitrogen protection and cooled to 0 °C. Then, TosCl (2.32 g, 12.17 mmol) was added, and the mixture was reacted at room temperature for 12 h. After LC-MS monitoring indicated the reaction was complete, saturated ammonium chloride was added to quench. The reaction solution was diluted with DCM, washed sequentially with water and brine, separated, and the organic phase was concentrated to obtain a crude product. Purification by silica gel column chromatography yielded the target compound 83-6 (2.24 g). ESI-MS m/z: 498.08, 399.09 [M+H]⁺.

### Step 7: Synthesis of compound 83-7

Compound 83-6 (158.62 mg, 0.32 mmol), compound M2 (150.00 mg, 0.26 mmol), [PdCl₂(dppf)] CH₂Cl₂ (21.64 mg, 0.03 mmol), and K₂CO₃ (109.75 mg, 0.79 mmol) were dissolved in toluene (3.00 mL), dioxane (1.00 mL), and water (1.00 mL) under nitrogen protection and reacted at 65 °C for 2 h. After LC-MS monitoring indicated the reaction was complete, the reaction solution was diluted with ethyl acetate, washed sequentially with water and brine, separated, and the organic phase was concentrated to obtain a crude product. Purification by silica gel column chromatography yielded the target compound 83-7 (180 mg). ESI-MS m/z: 985.41, 986.42 [M+H]⁺.

### Step 8: Synthesis of compound 83-8

Compound 83-7 (160.00 mg, 0.16 mmol) was dissolved in DMF (2.00 mL). Then, Cs₂CO₃ (158.54 mg, 0.49 mmol) was added, and the mixture was reacted at 65 °C for 1 h. After LC-MS monitoring indicated the reaction was complete, the reaction solution was diluted with ethyl acetate, washed sequentially with water and brine, separated, and the organic phase was concentrated to obtain a crude product. Purification by silica gel column chromatography yielded the target compound 83-8 (114 mg). ESI-MS m/z: 813.39, 814.40 [M+H]⁺.

### Step 9: Synthesis of compound 83-9

Compound 83-8 (100.00 mg, 0.12 mmol) was dissolved in DCM (1.00 mL). Then, HCl (4M solution in dioxane) (1.50 mL) was added, and the mixture was reacted at room temperature for 30 min. The reaction solution was concentrated to obtain a crude product of compound 83-9 (120 mg), which was used directly in the next reaction step. ESI-MS m/z: 713.39, 714.40 [M+H]⁺.

### Step 10: Synthesis of compound 83

Compound (1S,2S)-2-(difluoromethyl)cyclopropanecarboxylic acid (94.79 mg, 0.70 mmol) was dissolved in DMF (1.00 mL) and cooled to 0 °C. Then, DIPEA (0.81 mL, 4.64 mmol) and HATU (353.08 mg, 0.93 mmol) were added. After reacting at 0 °C for 5 min, compound 83-9 (60 mg, 0.08 mmol) was added, and the reaction was continued at 0 °C for 5 min. After LC-MS monitoring indicated the reaction was complete, purification by preparative chromatography (acetonitrile:water (containing 0.05% diethylamine) = 5:1) was performed to obtain the target compound 83 (25.9 mg). ESI-MS m/z: 832.49 [M+H]⁺.

¹H NMR (500 MHz, CDCl₃) δ 8.58 (d, J = 1.6 Hz, 1H), 8.42 (d, J = 2.9 Hz, 1H), 7.58 (dd, J = 8.5, 1.7 Hz, 1H), 7.34 - 7.27 (m, 2H), 7.08 (d, J = 3.0 Hz, 1H), 6.67 (d, J = 9.5 Hz, 1H), 5.96 - 5.68 (m, 2H), 4.59 (d, J = 13.0 Hz, 1H), 4.36 (td, J = 12.1, 3.3 Hz, 1H), 4.26-4.18 (m, 2H), 4.05 - 3.96 (m, 2H), 3.93 - 3.86 (m, 4H), 3.83 (s, 2H), 3.82 - 3.73 (m, 1H), 3.47 (d, J = 14.9 Hz, 1H), 3.35-3.29 (m, 1H), 3.24 (t, J = 4.9 Hz, 4H), 3.22 - 3.10 (m, 2H), 2.70-3.64 (m, 1H), 2.42 (d, J = 14.4 Hz, 1H), 2.34-3.23 (m, 2H), 2.0-1.96 (m, 1H), 1.85-1.81 (m, 1H), 1.64-1.57 (m, 4H), 1.52 (d, J = 6.5 Hz, 3H), 1.33-1.29 (m, 1H), 1.17-1.13 (m, 1H), 0.98 (s, 3H), 0.41 (s, 3H).

### Single crystal cultivation of compound 83

10 mg of compound 83 was added to a 3 mL sample vial, and 1 mL of a mixed solvent of ethanol/water (19/1, v/v) was added at room temperature to prepare a solution. The solution was filtered through a 0.22 µm PTFE membrane, sealed with parafilm, and punctured with a needle. The vial was then placed in a 20 mL sample vial containing purified water. Single crystals were obtained by slow vapor diffusion crystallization at room temperature in a water atmosphere.

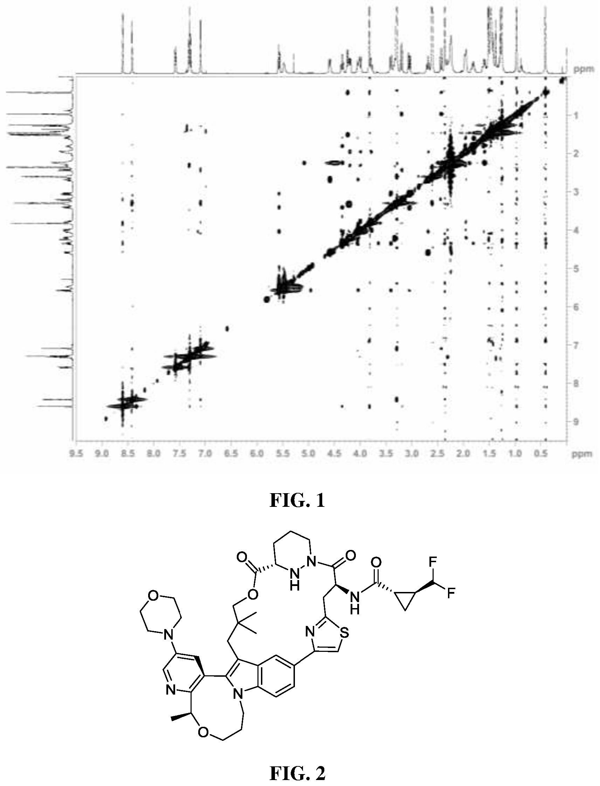

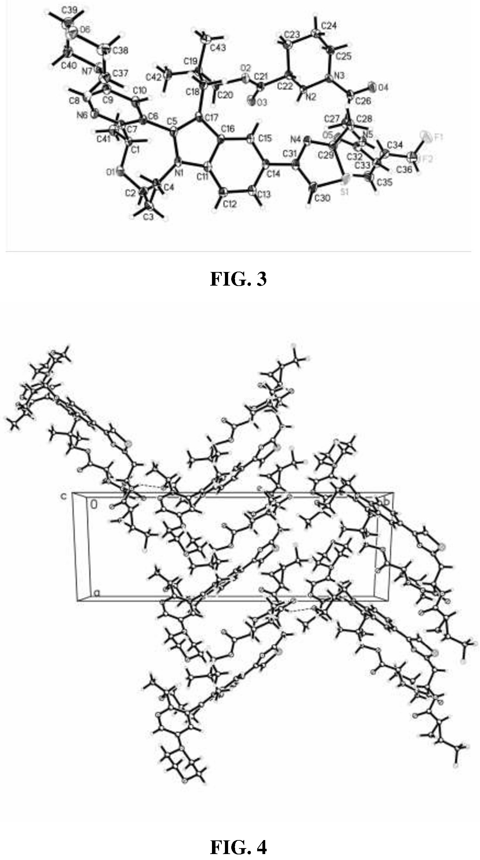

Single crystal X-ray diffraction data for compound 83 were collected using a Bruker D8 VENTURE single crystal diffractometer. The crystal structure was solved by direct methods (Shelxs97). The crystal belonged to the monoclinic system, space group P2(1), with unit cell parameters: a = 9.3519(4) Å, b = 25.2142(12) Å, c = 10.2423(4) Å, α = 90°, β = 111.4680(10)°, y = 90°, unit cell volume V = 2247.58(17) Å3. The structural parameters were refined and atomic types were determined using the least-squares method. The positions of all hydrogen atoms were obtained by geometric calculation and difference Fourier methods. After refinement, R₁ = 0.0873, wR₂ = 0.1319 (All data), S = 1.023; the refined Flack x (µ) was 0.01 (10). The absolute configuration diagram of the molecule of compound 83 in the crystal is shown in FIG. 2, the three-dimensional structural ellipsoid diagram of the molecule is shown in FIG. 3, and FIG. 4 is a projection diagram of the unit cell packing along the c-axis.

### Example 84: Synthesis of compound (1S,2S)-2-(difluoromethyl)-N-((1⁵S,6³S,4S,Z)-1²-(4-ethylpiperazin-1-yl)-1⁵,10,10-trimethyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxazonino[ 5,6-a]indola-2(4,2)-thiazola-6(1,3)-pyridazinacycloundecaphane-4-yl)cyclopropane-1-carboxami de

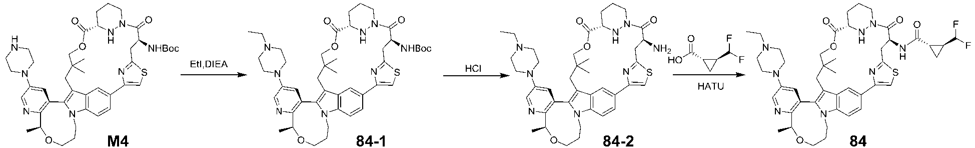

### Step 1: Synthesis of compound 84-1

Compound M4 (200.0 mg, 0.25 mmol), dichloromethane (5.00 mL), N,N-diisopropylethylamine (0.12 mL, 0.74 mmol), and iodoethane (39 µL, 0.49 mmol) were added sequentially to a 25 mL single-necked flask. The mixture was stirred and reacted at room temperature. After LC-MS monitoring indicated the reaction was complete, water was added to quench. The mixture was extracted twice with dichloromethane. The combined organic phases were washed with saturated brine and concentrated to obtain a crude product of compound 84-1 (white solid, 200.0 mg). ESI-MS m/z: 841.52 [M+H]⁺.

### Step 2: Synthesis of compound 84-2

Compound 84-1 (200.0 mg, 0.24 mmol), DCM (4.00 mL), and HCl (4M solution in dioxane) (2.00 mL) were added sequentially to a 10 mL vial. The mixture was stirred and reacted at room temperature. After LC-MS monitoring indicated the reaction was complete, the reaction solution was neutralized with saturated sodium bicarbonate solution, extracted with DCM, washed with saturated brine, and concentrated to obtain a crude product of compound 84-2 (yellow solid, 200.0 mg). ESI-MS m/z: 740.98 [M+H]⁺.

### Step 3: Synthesis of compound 84

(1S,2S)-2-(difluoromethyl)cyclopropanecarboxylic acid (37 µL, 0.36 mmol), HATU (140.0 mg, 0.36 mmol), and DIEA (0.16 mL, 0.95 mmol) were added sequentially to a 10 mL single-necked flask. The mixture was stirred at room temperature for 10 min. After cooling in an ice-water bath, a solution of 84-2 (200.0 mg, 0.26 mmol) in acetonitrile was added. After LC-MS monitoring indicated the reaction was complete, the reaction solution was directly purified by Prep-HPLC (basic method). The eluent was lyophilized to obtain the target compound 84 (98.8 mg). ESI-MS m/z: 859.48 [M+H]⁺.

¹H NMR (500 MHz, CDCl₃) δ 8.58 (d, J = 1.6 Hz, 1H), 8.43 (d, J = 2.9 Hz, 1H), 7.57 (dd, J = 8.6, 1.7 Hz, 1H), 7.34 - 7.27 (m, 2H), 7.09 (d, J = 2.9 Hz, 1H), 6.63 (d, J = 9.6 Hz, 1H), 5.97 - 5.71 (m, 2H), 4.60 (d, J = 12.7 Hz, 1H), 4.38-4.32 (m, 1H), 4.24-4.18 (m, 2H), 4.05 - 3.96 (m, 2H), 3.84 - 3.75 (m, 3H), 3.51 - 3.44 (m, 1H), 3.36 - 3.28 (m, 4H), 3.19 (d, J = 14.4 Hz, 1H), 3.14 (dd, J = 15.0, 8.5 Hz, 1H), 2.72-3.62 (m, 5H), 2.65 (s, 4H), 2.51 (d, J = 11.7 Hz, 2H), 2.42 (d, J = 14.4 Hz, 1H), 2.33 - 2.19 (m, 2H), 1.84 - 1.80 (m, 1H), 1.51 (d, J = 6.5 Hz, 3H), 1.36 - 1.23 (m, 3H), 1.16 - 1.12 (m, 4H), 0.97 (s, 3H), 0.40 (s, 3H).

### Example 85: Synthesis of compound (1S,2S)-N-((1⁵S,6³S,4S,Z)-1²-(4-cyclopropylpiperazin-1-yl)-15,10,10-trimethyl-5,7-dioxo-1⁸,1⁹,6 ,6²,6³,6⁴,6⁵,6⁶-octahydro-1⁵H,1⁷H-8-oxa-1(13,15)-pyrido[3',2':7,8][1,5]oxazonino[5,6-a]indola-2(4,2)-thiazola-6(1,3)-pyridazinacycloundecaphane-4-yl)-2-(difluoromethyl)cyclopropane-1-car boxamide

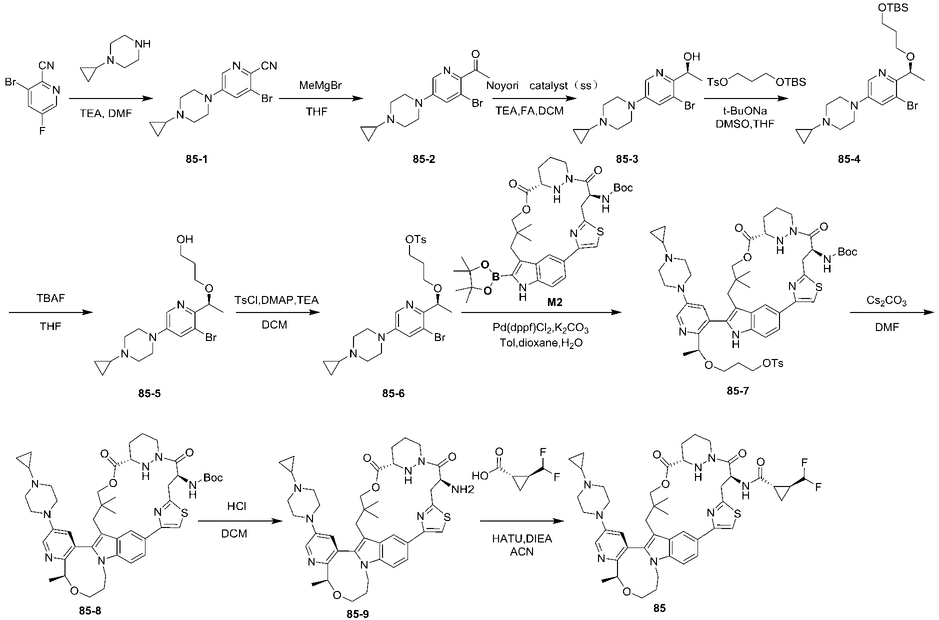

### Step 1: Synthesis of compound 85-1

Under ice-water bath conditions, 3-bromo-5-fluoro-pyridine-2-carbonitrile (2.00 g, 9.95 mmol), triethylamine (4.15 mL, 29.85 mmol), and compound 1-cyclopropylpiperazine (1.26 g, 9.95 mmol) were added sequentially to DMF (20.00 mL). The mixture was reacted at room temperature for 1 h. Water (50 mL) was added to the reaction solution. The mixture was extracted with EA (40 mL * 2), washed with saturated brine (40 mL * 2), dried over anhydrous sodium sulfate, and concentrated to dryness to obtain a crude product of compound 85-1 (3.00 g, 98.85%), which was used directly in the next reaction step. ESI-MS m/z: 307.12 [M+H]⁺.

### Step 2: Synthesis of compound 85-2

Compound 85-1 (3.00 g, 9.17 mmol) was dissolved in tetrahydrofuran (60.00 mL), purged with nitrogen. Methylmagnesium bromide (9.11 mL, 3.00 mol/L, 27.34 mmol) was added dropwise at -20 °C. The mixture was allowed to warm to room temperature and reacted for 2 h. The reaction solution was added dropwise to 40 mL of ice-cold saturated aqueous ammonium chloride solution. The pH was adjusted to 3 with 1N aqueous hydrochloric acid solution. After stirring for 10 min, the pH was adjusted to 9 with saturated aqueous sodium bicarbonate solution. The mixture was extracted with EA (40 mL * 2), washed with saturated brine (40 mL * 2), dried over anhydrous sodium sulfate, and purified by column chromatography (DCM:EA=75%:25%) to obtain compound 85-2 (2.24 g, 70.75%). ESI-MS m/z: 324.10 [M+H]⁺.

### Step 3: Synthesis of compound 85-3

Compound 85-2 (2.24 g, 6.91 mmol), (S,S)-N-(p-toluenesulfonyl)-1,2-diphenylethane-1,2-diamine (p-cymene)ruthenium(II) chloride (0.22 g, 0.35 mmol), and TEA (4.80 mL, 34.55 mmol) were added to DCM (10.00 mL). Formic acid (1.33 g, 34.55 mmol) was added under ice-water bath cooling, and the mixture was reacted at room temperature for 72 h. Water (50 mL) was added to the reaction solution. The mixture was extracted with DCM (40 mL * 2), washed with saturated brine (40 mL * 2), dried over anhydrous sodium sulfate, and purified by column chromatography to obtain compound 85-3 (1.90 g, 84.30%). ESI-MS m/z: 326.17 [M+H]⁺.

### Step 4: Synthesis of compound 85-4

Compound 85-3 (1.90 g, 5.82 mmol) and compound 3-((tert-butyldimethylsilyl)oxy)propyl 4-methylbenzenesulfonate (4.01 g, 11.65 mmol) were added to DMSO (30.00 mL). Then, a solution of sodium tert-butoxide (1.40 g, 14.56 mmol) in tetrahydrofuran (6.00 mL) was added, and the mixture was reacted at 40 °C for 2 h. Water (50 mL) was added to the reaction solution. The mixture was extracted with EA (40 mL * 2), washed with saturated brine (40 mL * 2), dried over anhydrous sodium sulfate, and purified by column chromatography (DCM:EA=80%:20%) to obtain compound 85-4 (1.44 g, 49.59%). ESI-MS m/z: 498.24 [M+H]⁺.

### Step 5: Synthesis of compound 85-5

Compound 85-4 (1.44 g, 2.89 mmol) and tetrabutylammonium fluoride (4.33 mL, 1.00 mol/L, 4.33 mmol) were added to tetrahydrofuran (15.00 mL) and reacted at 50 °C for 1 h. The mixture was concentrated to dryness and purified by column chromatography to obtain compound 85-5 (0.97 g, 87.39%). ESI-MS m/z: 384.10 [M+H]⁺.

### Step 6: Synthesis of compound 85-6

Compound 85-5 (0.97 g, 2.52 mmol), DMAP (0.03 g, 0.25 mmol), and TEA (0.88 mL, 6.31 mmol) were added to DCM (10.00 mL). p-Toluenesulfonyl chloride (0.96 g, 5.05 mmol) was added in batches under ice-water bath cooling, and the mixture was reacted at room temperature for 2 h. Water (50 mL) was added to the reaction solution. The mixture was extracted with DCM (40 mL * 2), washed with saturated brine (40 mL * 2), dried over anhydrous sodium sulfate, and purified by column chromatography to obtain compound 85-6 (1.00 g, 73.57%). ESI-MS m/z: 538.18 [M+H]⁺.

### Step 7: Synthesis of compound 85-7

Compound 85-6 (326.06 mg, 0.61 mmol), compound M2 (350.00 mg, 0.50 mmol), [1,1'-bis(diphenylphosphino)ferrocene]dichloropalladium(II) dichloromethane complex (41.24 mg, 0.05 mmol), potassium carbonate (174.35 mg, 1.26 mmol), water (0.50 mL), and 1,4-dioxane (2.00 mL) were added to toluene (6.00 mL). Under a nitrogen atmosphere, the mixture was reacted at 65 °C for 2 h. The mixture was concentrated and purified by column chromatography (DCM:EA=70%:30%) to obtain compound 85-7 (400 mg, 77.32%). ESI-MS m/z: 485.47 [M+H]⁺.

### Step 8: Synthesis of compound 85-8

Compound 85-7 (350.00 mg, 0.34 mmol) and cesium carbonate (222.47 mg, 0.68 mmol) were added to DMF (8.00 mL) and reacted at 80 °C for 1 h. Water (50 mL) was added to the reaction solution. The mixture was extracted with EA (40 mL * 2), washed with saturated brine (40 mL * 2), dried over anhydrous sodium sulfate, and purified by column chromatography (DCM:EA=70%:30%) to obtain compound 85-8 (290 mg, 99.57%). ESI-MS m/z: 853.69 [M+H]⁺.

### Step 9: Synthesis of compound 85-9

Compound 85-8 (290.00 mg, 0.34 mmol) was dissolved in DCM (1.00 mL). Then, hydrochloric acid (4M solution in dioxane) (2.00 mL) was added, and the mixture was reacted at room temperature for 1 h. The reaction solution was concentrated to dryness, diluted with 5 mL of toluene, and concentrated to dryness again to obtain a crude product of compound 85-9 (250 mg), which was used directly in the next reaction step. ESI-MS m/z: 753.02 [M+H]⁺.

### Step 10: Synthesis of compound 85

Compound (1S,2S)-2-(difluoromethyl)cyclopropanecarboxylic acid (36.88 mg, 0.27 mmol) was dissolved in acetonitrile (4.00 mL). DIEA (0.37 mL, 2.26 mmol) and HATU (128.78 mg, 0.34 mmol) were added sequentially under ice-water bath cooling. After reacting in the ice-water bath for 10 min, a solution of compound 85-9 (170.00 mg, 0.23 mmol) in acetonitrile (4.00 mL) and DIEA (0.37 mL, 2.26 mmol) was added. The mixture was reacted at room temperature for 1 h. The mixture was concentrated to dryness and purified by preparative separation to obtain compound 85 (66.00 mg, 33.36%). ESI-MS m/z: 871.42 [M+H]⁺.

¹H NMR (500 MHz, DMSO) δ 8.89 (d, J = 9.0 Hz, 1H), 8.50 (d, J = 1.1 Hz, 1H), 8.40 (d, J = 2.8 Hz, 1H), 7.81 (s, 1H), 7.71 (dd, J = 8.6, 1.5 Hz, 1H), 7.49 (d, J = 8.6 Hz, 1H), 7.20 (d, J = 2.8 Hz, 1H), 5.95 (td, J = 56.7, 5.1 Hz, 1H), 5.56 (t, J = 9.4 Hz, 1H), 5.11 (d, J = 12.2 Hz, 1H), 4.28 - 4.19 (m, 2H), 4.15 (q, J = 6.3 Hz, 1H), 4.03 (dd, J = 12.5, 4.4 Hz, 2H), 3.68 (d, J = 10.9 Hz, 1H), 3.60 (dd, J = 14.9, 9.7 Hz, 2H), 3.38 (d, J = 14.5 Hz, 1H), 3.24 - 3.11 (m, 6H), 3.01 (d, J = 14.4 Hz, 1H), 2.76 (td, J = 12.7, 8.2 Hz, 1H), 2.70 - 2.63 (m, 4H), 2.41 (d, J = 14.3 Hz, 1H), 2.29 (t, J = 10.4 Hz, 1H), 2.10 (dt, J = 9.1, 5.5 Hz, 2H), 1.81 (s, 3H), 1.69 - 1.61 (m, 2H), 1.58 - 1.47 (m, 1H), 1.37 (d, J = 6.4 Hz, 3H), 1.10 - 0.99 (m, 2H), 0.95 (s, 3H), 0.48 - 0.40 (m, 2H), 0.37 - 0.28 (m, 5H).

### Example 86: Synthesis of compound (1S,2S)-2-(difluoromethyl)-N-((1⁵S,6³S,4S,Z)-1²-(4-isopropylpiperazin-1-yl)-1⁵,10,10-trimethyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxazoni no[5,6-a]indola-2(4,2)-thiazola-6(1,3)-pyridazinacycloundecaphane-4-yl)cyclopropane-1-carbox amide

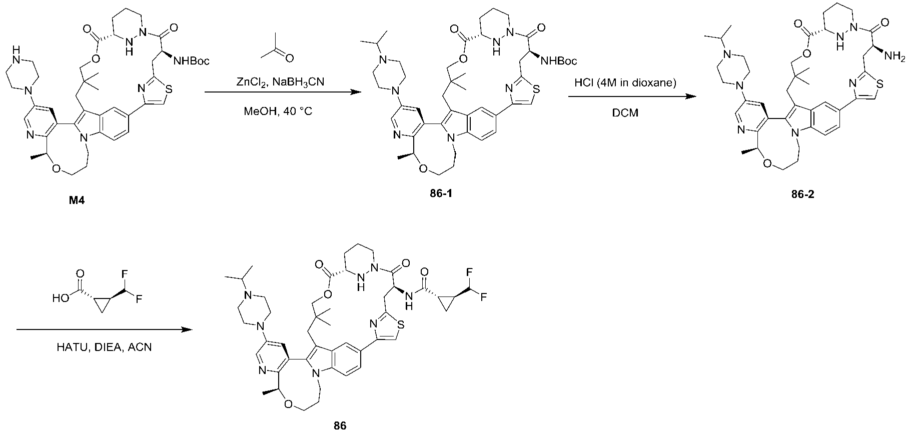

### Step 1: Synthesis of compound 86-1

Under nitrogen protection, acetone (7 µL) and ZnCl₂ (100 µL, 1M in THF) were added sequentially to a solution of M4 (50.0 mg) in MeOH (2.0 mL). The mixture was then stirred in a 40 °C oil bath for 1 h. Then, sodium cyanoborohydride (19.3 mg) was added, and the mixture was stirred and reacted in the 40 °C oil bath for an additional 2 h. Additional acetone (7 µL) and ZnCl₂ (100 µL, 1M in THF) were added, and the mixture was stirred in the 40°C oil bath for 1 h. Sodium cyanoborohydride (19.3 mg) was added again, and the mixture was stirred and reacted in the 40 °C oil bath for another 2 h. After LC-MS monitoring indicated the reaction was complete, the reaction was quenched with saturated sodium bicarbonate solution, and DCM was added for extraction and liquid separation. The aqueous phase was extracted twice more with DCM. The combined organic phases were dried over anhydrous sodium sulfate and concentrated. The concentrate was purified by column chromatography (0% to 5% MeOH/DCM) to obtain compound 86-1 (40 mg). ESI-MS m/z: 855.5 [M+H]⁺.

### Step 2: Synthesis of compound 86-2

HCl (4M solution in dioxane) (2.0 mL) was added to a solution of compound 86-1 (40 mg) in DCM (2.0 mL). The mixture was stirred and reacted at room temperature for 1 h. After LC-MS monitoring indicated the reaction was complete, the reaction solution was concentrated to dryness to obtain a crude product of compound 86-2 (slightly yellow solid powder, 40 mg, hydrochloride salt), which was used directly in the next reaction step. ESI-MS m/z: 755.5 [M+H]⁺.

### Step 3: Synthesis of compound 86

At room temperature, HATU (40.3 mg) and DIEA (44 µL) were added to a solution of (1S,2S)-2-(difluoromethyl)cyclopropanecarboxylic acid (14.4 mg) in acetonitrile (1.0 mL). The mixture was stirred at room temperature for 10 min. Then, a solution of 86-2 and DIEA (44 µL) in acetonitrile (1.0 mL) was added to the above system. The mixture was stirred and reacted at room temperature for 30 min. After the reaction was complete, the system was concentrated. The concentrate was purified by preparative chromatography (acetonitrile:water (containing 0.1% formic acid) = 1:1) to obtain the target compound 86 (19.2 mg, 41% yield). ESI-MS m/z: 873.9 [M+H]⁺.

¹H NMR (500 MHz, DMSO) δ 8.89 (d, J = 9.0 Hz, 1H), 8.50 (d, J = 1.2 Hz, 1H), 8.40 (d, J = 2.8 Hz, 1H), 7.81 (s, 1H), 7.71 (dd, J = 8.6, 1.5 Hz, 1H), 7.49 (d, J = 8.6 Hz, 1H), 7.20 (d, J = 2.9 Hz, 1H), 5.95 (td, J = 56.7, 5.1 Hz, 1H), 5.56 (t, J = 9.4 Hz, 1H), 5.11 (d, J = 12.2 Hz, 1H), 4.28 - 4.19 (m, 2H), 4.14 (q, J = 6.2 Hz, 1H), 4.07 - 3.99 (m, 2H), 3.70 - 3.56 (m, 3H), 3.38 (d, J = 14.6 Hz, 1H), 3.25 (s, 4H), 3.20 - 3.10 (m, 2H), 3.01 (d, J = 14.3 Hz, 1H), 2.76 (t, J = 14.1 Hz, 1H), 2.68 (dt, J = 13.0, 6.5 Hz, 1H), 2.62 - 2.54 (m, 4H), 2.41 (d, J = 14.3 Hz, 1H), 2.29 (s, 1H), 2.13 - 2.06 (m, 2H), 1.87 - 1.75 (m, 3H), 1.65 (dd, J = 9.5, 4.6 Hz, 1H), 1.37 (d, J = 6.4 Hz, 3H), 1.08 - 0.97 (m, 8H), 0.95 (s, 3H), 0.32 (s, 3H).

### Example 87: Synthesis of compound (1S,2S)-2-(difluoromethyl)-N-((1⁵S,6³S,4S,Z)-1²-((2-(dimethylamino)ethyl)(methyl)amino)-1⁵,1 0,10-trimethyl-5,7-dioxo-1⁸,1⁹,6¹,6²,6³,6⁴,6⁵,6⁶-octahydro-1⁵H,1⁷H-8-oxa-l(13,15)-pyrido[3',2':7 ,8][1,5]oxazonino[5,6-a]indola-2(4,2)-thiazola-6(1,3)-pyridazinacycloundecaphane-4-yl)cyclopr opane-1-carboxamide

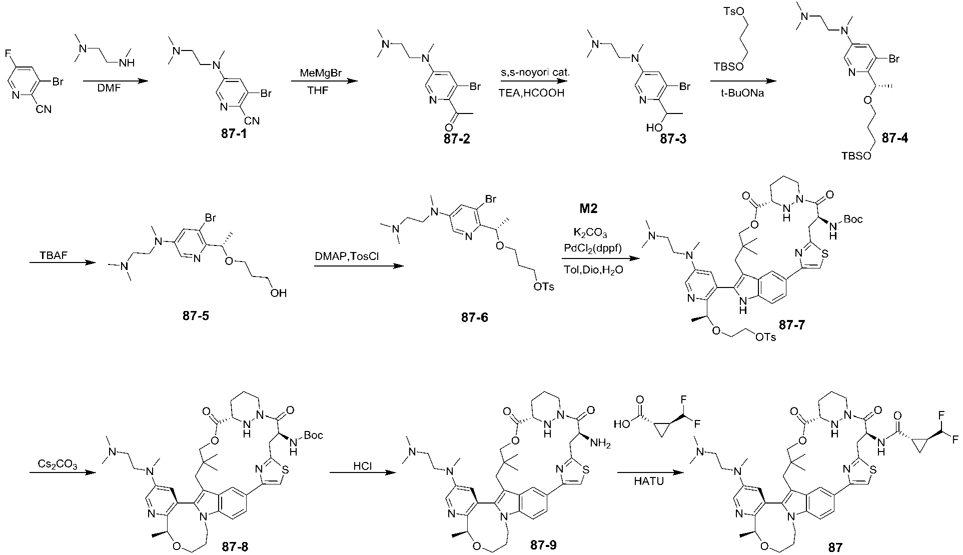

### Step 1: Synthesis of compound 87-1

3-Bromo-5-fluoro-pyridine-2-carbonitrile (3.00 g, 14.93 mmol), N,N-dimethylformamide (30.00 mL), and N,N,N'-trimethylethylenediamine (2.13 mL, 16.42 mmol) were added sequentially to a 100 mL single-necked flask. The mixture was stirred and reacted at room temperature. After LC-MS monitoring indicated the reaction was complete, the mixture was concentrated to remove DMF, obtaining a crude product of compound 87-1 (white solid powder, 4.50 g), which was used directly in the next reaction step. ESI-MS m/z: 283.15 [M+H]⁺.

### Step 2: Synthesis of compound 87-2

Compound 87-1 (4.20 g, 14.83 mmol) and THF (50.00 mL) were added sequentially to a 100 mL three-necked flask. Under a nitrogen atmosphere and ice bath cooling, methylmagnesium bromide (14.83 mL, 3 mol/L in THF, 44.49 mmol) was slowly added dropwise. The mixture was slowly allowed to warm to room temperature, stirred and reacted. After LC-MS monitoring indicated the reaction was complete, saturated ammonium chloride solution was added under ice bath cooling to quench the reaction. The mixture was extracted twice with dichloromethane. The combined organic phases were washed with saturated brine and concentrated to obtain a crude product. Purification by silica gel column chromatography yielded compound 87-2 (white solid powder, 3.30 g). ESI-MS m/z: 300.20 [M+H]⁺.

### Step 3: Synthesis of compound 87-3

Compound 87-2 (1.00 g, 3.33 mmol), dichloromethane (10.00 mL), (S,S)-Noyori catalyst (0.11 g, 0.17 mmol), and triethylamine (2.32 mL, 16.66 mmol) were added sequentially to a 50 mL three-necked flask. Under a nitrogen atmosphere and ice bath cooling, formic acid (0.63 mL, 16.66 mmol) was slowly added dropwise. The mixture was slowly allowed to warm to room temperature, stirred and reacted. After LC-MS monitoring indicated the reaction was complete, purification by silica gel column chromatography was performed to obtain compound 87-3 (yellow oil, 0.80 g). ESI-MS m/z: 302.32 [M+H]⁺.

### Step 4: Synthesis of compound 87-4

Compound 87-3 (0.70 g, 2.32 mmol), dimethyl sulfoxide (10.00 mL), tetrahydrofuran (2.00 mL), and 3-((tert-butyldimethylsilyl)oxy)propyl 4-methylbenzenesulfonate (3.59 g, 10.42 mmol) were added sequentially to a 50 mL single-necked flask. Under a nitrogen atmosphere and ice bath cooling, sodium tert-butoxide (0.56 g, 5.79 mmol) was slowly added. The mixture was slowly allowed to warm to room temperature, stirred and reacted. After LC-MS monitoring indicated the reaction was complete, saturated ammonium chloride solution was added under ice bath cooling to quench the reaction. The mixture was extracted twice with ethyl acetate and twice with dichloromethane. The combined organic phases were washed with saturated brine and concentrated to obtain a crude product. Purification by silica gel column chromatography yielded compound 87-4 (yellow oil, 0.85 g). ESI-MS m/z: 474.56 [M+H]⁺.

### Step 5: Synthesis of compound 87-5

Compound 87-4 (0.85 g, 1.79 mmol), THF (2.00 mL), and tetrabutylammonium fluoride (3.58 mL, 1 mol/L in THF, 3.58 mmol) were added sequentially to a 25 mL single-necked flask. The mixture was stirred and reacted at 50 °C. After LC-MS monitoring indicated the reaction was complete, water was added under ice bath cooling to quench the reaction. The mixture was extracted twice with dichloromethane. The combined organic phases were washed with saturated brine and concentrated to obtain a crude product. Purification by silica gel column chromatography yielded compound 87-5 (yellow oil, 0.66 g). ESI-MS m/z: 360.12 [M+H]⁺.

### Step 6: Synthesis of compound 87-6

Compound 87-5 (0.60 g, 1.67 mmol), dichloromethane (10.00 mL), 4-dimethylaminopyridine (0.51 g, 4.16 mmol), and p-toluenesulfonyl chloride (0.64 g, 3.33 mmol) were added sequentially to a 25 mL single-necked flask. The mixture was stirred and reacted at room temperature. After LC-MS monitoring indicated the reaction was complete, purification by silica gel column chromatography was performed to obtain compound 87-6 (yellow solid, 0.44 g). ESI-MS m/z: 514.36 [M+H]⁺.

### Step 7: Synthesis of compound 87-7

Compound 87-6 (89.0 mg, 0.17 mmol), M2 (89.0 mg, 0.12 mmol), potassium carbonate (40.0 mg, 0.29 mmol), toluene (1.50 mL), 1,4-dioxane (0.50 mL), water (0.50 mL), and PdCl₂(dppf) (8.4 mg, 0.01 mmol) were added sequentially to a 5 mL microwave tube. After nitrogen purging, the mixture was stirred at 65 °C. After LC-MS monitoring indicated the reaction was complete, purification by silica gel column chromatography was performed to obtain compound 87-7 (yellow solid, 60.0 mg). ESI-MS m/z: 987.51 [M+H]⁺.

### Step 8: Synthesis of compound 87-8

Compound 87-7 (60.0 mg, 0.06 mmol), cesium carbonate (39.0 mg, 0.12 mmol), and N,N-dimethylformamide (30.00 mL) were added sequentially to a 10 mL vial. The mixture was stirred and reacted at 80 °C. After LC-MS monitoring indicated the reaction was complete, the mixture was concentrated to remove DMF. The residue was dissolved in DCM, filtered, and the filtrate was concentrated to obtain a crude product of 87-8 (yellow solid, 35.0 mg). ESI-MS m/z: 829.37 [M+H]⁺.

### Step 9: Synthesis of compound 87-9

Compound 87-8 (35.0 mg, 0.04 mmol), DCM (2.00 mL), and HCl (4M solution in dioxane) (1.00 mL) were added sequentially to a 10 mL vial. The mixture was stirred and reacted at room temperature. After LC-MS monitoring indicated the reaction was complete, the reaction solution was neutralized with saturated sodium bicarbonate solution, extracted with DCM, washed with saturated brine, and concentrated to obtain a crude product of compound 87-9 (yellow solid, 30.0 mg). ESI-MS m/z: 729.35 [M+H]⁺.

### Step 10: Synthesis of compound 87

(1S,2S)-2-(difluoromethyl)cyclopropanecarboxylic acid (6 µL, 0.06 mmol), HATU (47 mg, 0.12 mmol), and DIEA (27 µL, 0.16 mmol) were added sequentially to a 10 mL single-necked flask. The mixture was stirred at room temperature for 10 min. After cooling in an ice-water bath, a solution of compound 87-9 (30.0 mg, 0.04 mmol) in acetonitrile was added. After LC-MS monitoring indicated the reaction was complete, the reaction solution was directly purified by Prep-HPLC (basic method). The eluent was lyophilized to obtain the target compound 87 (1.6 mg). ESI-MS m/z: 847.48 [M+H]⁺.

¹H NMR (500 MHz, CDCl₃) δ 8.52 (d, J =1.5 Hz, 1H), 8.16 (d, J = 2.9Hz,1H), 7.50 (dd, J=8.6,1.5Hz 1H), 7.24 (t, J=10.3Hz, 2H), 6.80 (d, J=2.9Hz,1H), 6.60 (d, J=9.7Hz,1H), 5.89 - 5.64 (m, 2H), 5.29 - 5.26 (m, 6H), 4.56 - 4.49 (m, 1H), 4.34 - 4.24 (m, 1H), 4.18 - 4.02 (m, 4H), 3.97 - 3.89 (m, 3H), 3.76 (s, 3H), 3.44 - 3.38 (m, 2H), 3.29 - 3.21 (m, 1H), 3.17 - 3.02 (m, 3H), 2.73 - 2.58 (m, 3H), 2.42 - 2.35 (m, 3H), 2.31 - 2.23 (m, 3H), 2.17 - 2.12 (m, 7H), 1.79 - 1.72 (m, 3H), 1.44 (d, J=6.8Hz,7 H), 0.91 (s, 4H), 0.33 (s, 3H).

### Example 89: Synthesis of compound (1S,2S)-2-(difluoromethyl)-N-((1⁵S,6³S.4S,Z)-1⁵,10,10-trimethyl-1²-(4-methyl-1,4-diazepan-1-yl )-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxazo nino[5,6-a]indola-2(4,2)-thiazola-6(1,3)-pyridazinacycloundecaphane-4-yl)cyclopropane-1-carbo xamide

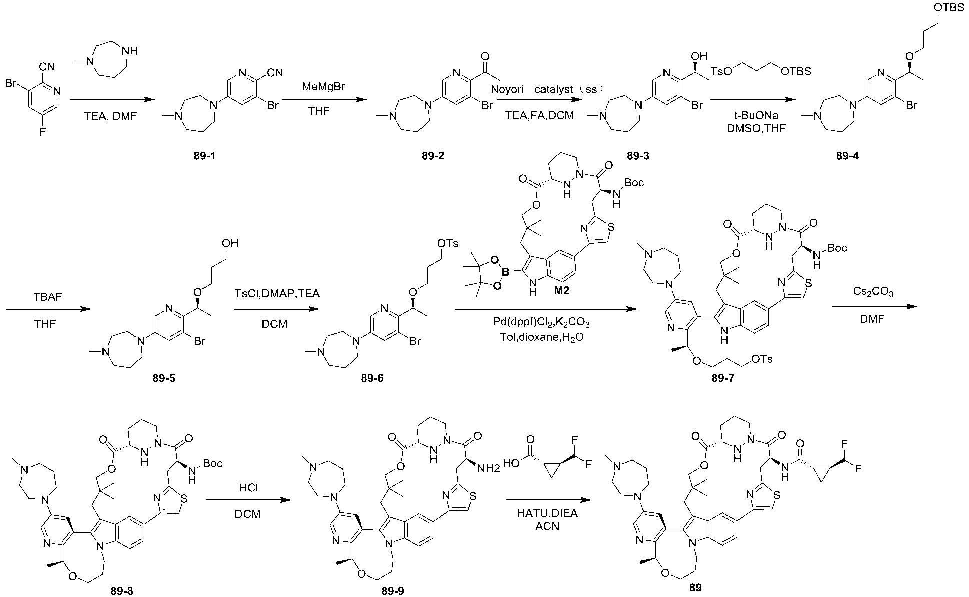

### Step 1: Synthesis of compound 89-1

Under ice-water bath conditions, 3-bromo-5-fluoro-pyridine-2-carbonitrile (2.00 g, 9.95 mmol), triethylamine (4.15 mL, 29.85 mmol), and compound N-methylhomopiperazine (1.25 g, 10.95 mmol) were added sequentially to DMF (20.00 mL). The mixture was reacted at room temperature for 1 h. Water (50 mL) was added to the reaction solution. The mixture was extracted with EA (40 mL * 2), washed with saturated brine (40 mL * 2), dried over anhydrous sodium sulfate, and concentrated to dryness to obtain a crude product of compound 89-1 (2.90 g, 98.74%), which was used directly in the next reaction step. ESI-MS m/z: 295.07 [M+H]⁺.

### Step 2: Synthesis of compound 89-2

Compound 89-1 (2.90 g, 9.82 mmol) was dissolved in tetrahydrofuran (60.00 mL), purged with nitrogen, and cooled to -20 °C. Methylmagnesium bromide (9.17 mL, 3.00 mol/L, 27.51 mmol) was added dropwise. The mixture was allowed to warm to room temperature and reacted for 2 h. The reaction solution was added dropwise to 40 mL of ice-cold saturated aqueous ammonium chloride solution. The pH was adjusted to 3 with 1N aqueous hydrochloric acid solution. After stirring for 10 min, the pH was adjusted to 9 with saturated aqueous sodium bicarbonate solution. The mixture was extracted with EA (40 mL * 2), washed with saturated brine (40 mL * 2), dried over anhydrous sodium sulfate, and purified by column chromatography to obtain compound 89-2 (1.17 g, 38.14%). ESI-MS m/z: 312.07 [M+H]⁺.

### Step 3: Synthesis of compound 89-3

Compound 89-2 (1.17 g, 3.75 mmol), (S,S)-N-(p-toluenesulfonyl)-1,2-diphenylethane-1,2-diamine (p-cymene)ruthenium(II) chloride (0.12 g, 0.19 mmol), and TEA (2.60 mL, 18.74 mmol) were added to DCM (10.00 mL). Formic acid (0.72 g, 18.74 mmol) was added under ice-water bath cooling, and the mixture was reacted at room temperature for 72 h. Water (50 mL) was added to the reaction solution. The mixture was extracted with DCM (40 mL * 2), washed with saturated brine (40 mL * 2), dried over anhydrous sodium sulfate, and purified by column chromatography to obtain compound 89-3 (0.61 g, 51.80%). ESI-MS m/z: 314.22 [M+H]⁺.

### Step 4: Synthesis of compound 89-4

Compound 89-3 (610.00 mg, 1.94 mmol) and compound 3-((tert-butyldimethylsilyl)oxy)propyl 4-methylbenzenesulfonate (2006.58 mg, 5.82 mmol) were added to DMSO (10.00 mL). Then, a solution of sodium tert-butoxide (466.40 mg, 4.85 mmol) in tetrahydrofuran (2.00 mL) was added, and the mixture was reacted at 40 °C for 1 h. Water (50 mL) was added to the reaction solution. The mixture was extracted with EA (40 mL * 2), washed with saturated brine (40 mL * 2), dried over anhydrous sodium sulfate, and purified by column chromatography to obtain compound 89-4 (690.00 mg, 73.05%). ESI-MS m/z: 486.28 [M+H]⁺.

### Step 5: Synthesis of compound 89-5

Compound 89-4 (680.00 mg, 1.40 mmol) and tetrabutylammonium fluoride (2.10 mL, 1.00 mol/L, 2.10 mmol) were added to tetrahydrofuran (7.00 mL), and the mixture was reacted at 50 °C for 1 h. The mixture was concentrated to dryness and purified by column chromatography to obtain compound 89-5 (500.00 mg, 96.09%). ESI-MS m/z: 372.13 [M+H]⁺.

### Step 6: Synthesis of compound 89-6

Compound 89-5 (500.00 mg, 1.34 mmol), DMAP (328.15 mg, 2.69 mmol), and TEA (1.12 mL, 8.06 mmol) were added to DCM (10.00 mL). p-Toluenesulfonyl chloride (1280.19 mg, 6.71 mmol) was added in batches under ice-water bath cooling, and the mixture was reacted at room temperature for 2 h. Water (50 mL) was added to the reaction solution. The mixture was extracted with DCM (40 mL * 2), washed with saturated brine (40 mL * 2), dried over anhydrous sodium sulfate, and purified by column chromatography to obtain compound 89-6 (700.00 mg, 99.00%). ESI-MS m/z: 526.22 [M+H]⁺.

### Step 7: Synthesis of compound 89-7

Compound 89-6 (303.57 mg, 0.58 mmol), compound M2 (200.00 mg, 0.29 mmol), [1,1'-bis(diphenylphosphino)ferrocene]dichloropalladium(II) dichloromethane complex (23.52 mg, 0.03 mmol), potassium carbonate (99.62 mg, 0.72 mmol), water (0.50 mL), and 1,4-dioxane (2.00 mL) were added to toluene (6.00 mL). Under a nitrogen atmosphere, the mixture was reacted at 65 °C for 2 h. The mixture was concentrated and purified by column chromatography to obtain compound 89-7 (200 mg, 68.46%). ESI-MS m/z: 479.42 [M+H]⁺.

### Step 8: Synthesis of compound 89-8

Compound 89-7 (200.00 mg, 0.20 mmol) and cesium carbonate (128.63 mg, 0.39 mmol) were added to DMF (5.00 mL), and the mixture was reacted at 85 °C for 1 h. Water (50 mL) was added to the reaction solution. The mixture was extracted with EA (40 mL * 2), washed with saturated brine (40 mL * 2), dried over anhydrous sodium sulfate, and purified by column chromatography to obtain compound 89-8 (130 mg, 78.30%). ESI-MS m/z: 841.66 [M+H]⁺.

### Step 9: Synthesis of compound 89-9

Compound 89-8 (130.00 mg, 0.15 mmol) was dissolved in DCM (1.00 mL). Then, hydrochloric acid (4M solution in dioxane) (2.00 mL) was added, and the mixture was reacted at room temperature for 1 h. The reaction solution was concentrated to dryness, diluted with 5 mL of toluene, and concentrated to dryness again to obtain a crude product of compound 89-9 (90 mg, 78.57%), which was used directly in the next reaction step. ESI-MS m/z: 741.66 [M+H]⁺.

### Step 10: Synthesis of compound 89

Compound (1S,2S)-2-(difluoromethyl)cyclopropanecarboxylic acid (11.02 mg, 0.08 mmol) was dissolved in acetonitrile (3.00 mL). DIEA (0.11 mL, 0.68 mmol) and HATU (38.35 mg, 0.10 mmol) were added sequentially under ice-water bath cooling. After reacting in the ice-water bath for 10 min, a solution of compound 89-9 (50.00 mg, 0.07 mmol) in acetonitrile (3.00 mL) and DIEA (0.11 mL, 0.68 mmol) was added. The mixture was reacted at room temperature for 1 h. The mixture was concentrated to dryness and purified by preparative separation to obtain compound 89 (7 mg, 11.49%). ESI-MS m/z: 859.49 [M+H]⁺.

### Example 91: Synthesis of compound (1S,2S)-2-(difluoromethyl)-N-((1⁵S,6³S,4S,Z)-1⁵,10,10-trimethyl-1²-(4-(methyl-d3)piperazin-1-y l)-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxaz onino[5,6-a]indola-2(4,2)-thiazola-6(1,3)-pyridazinacycloundecaphane-4-yl)cyclopropane-1-carb oxamide

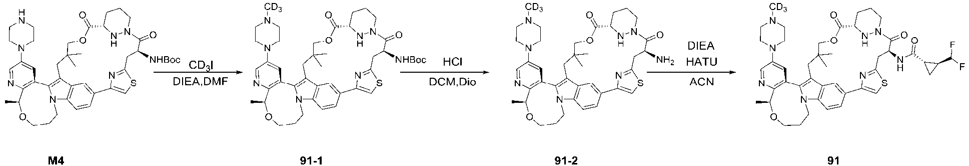

### Step 1: Synthesis of compound 91-1

Compound M4 (50.00 mg, 0.06 mmol), DMF (1.00 mL), and DIEA (0.03 mL, 0.15 mmol) were added sequentially to a 10 mL single-necked flask. A solution of CD₃I (8.91 mg, 0.06 mmol) in N,N-dimethylformamide (1.00 mL) was added dropwise. The mixture was stirred and reacted at room temperature. After LC-MS monitoring indicated the reaction was complete, the reaction solution was added to saturated aqueous ammonium chloride solution, then extracted with ethyl acetate. The combined organic phases were washed with brine, separated, and concentrated to obtain a crude product. Purification by Prep-TLC yielded the target compound 91-1 (35 mg, beige foamy solid). ESI-MS m/z: 830.65 [M+H]⁺.

### Step 2: Synthesis of compound 91-2

Compound 91-1 (62.00 mg, 0.07 mmol), DCM (2.00 mL), and HCl (4M solution in dioxane) (1.50 mL) were added sequentially to a 10 mL single-necked flask. The mixture was stirred and reacted at room temperature. After LC-MS monitoring indicated the reaction was complete, the reaction solution was concentrated to dryness. Acetonitrile was added, and the mixture was concentrated to dryness again (3 mL * 2) to obtain the hydrochloride salt of the target compound 91-2 (54.5 mg, pale yellow powder), which was used directly in the next reaction step. ESI-MS m/z: 730.25 [M+H]⁺.

### Step 3: Synthesis of compound 91

(1S,2S)-2-(difluoromethyl)cyclopropanecarboxylic acid (20.33 mg, 0.15 mmol), ACN (3.00 mL), HATU (85.21 mg, 0.22 mmol), and DIEA (0.10 mL, 0.60 mmol) were added sequentially to a 10 mL single-necked flask. The mixture was stirred at room temperature for 30 min. After cooling in an ice-water bath, a solution of compound 91-2 (hydrochloride salt: 54.50 mg, 0.07 mmol, dissolved in 1 mL acetonitrile with a few drops of DIEA until completely dissolved) in acetonitrile was added dropwise. After LC-MS monitoring indicated the reaction was complete, the reaction solution was diluted with ethyl acetate, washed sequentially with water and brine, separated, and the organic phase was concentrated to obtain a crude product. Purification by Prep-HPLC and lyophilization of the eluent yielded the target compound 91 (22.5 mg). ESI-MS m/z: 848.63 [M+H]⁺.

¹H NMR (500 MHz, CDCl₃) δ 8.51 (d, J = 1.7 Hz, 1H), 8.36 (d, J = 2.9 Hz, 1H), 7.51 (dd, J = 8.6, 1.6 Hz, 1H), 7.27 - 7.20 (m, 2H), 7.02 (d, J = 3.0 Hz, 1H), 6.68 (d, J = 9.5 Hz, 1H), 5.90 - 5.64 (m, 2H), 4.53 (d, J = 13.0 Hz, 1H), 4.29 (td, J = 12.1, 3.3 Hz, 1H), 4.16 (dq, J = 18.1, 6.2 Hz, 2H), 3.99 - 3.89 (m, 2H), 3.75 (s, 2H), 3.75 - 3.65 (m, 1H), 3.40 (d, J = 14.8 Hz, 1H), 3.34 (s, 4H), 3.25 (td, J = 12.4, 3.2 Hz, 1H), 3.16 - 3.04 (m, 2H), 2.75 (s, 4H), 2.62 (td, J = 12.9, 3.0 Hz, 1H), 2.34 (d, J = 14.4 Hz, 1H), 2.25-2.13 (m, 2H), 1.88 - 1.70 (m, 2H), 1.63 - 1.48 (m, 2H), 1.44 (d, J = 6.5 Hz, 3H), 1.41 - 1.28 (m, 1H), 1.25-1.21 (m, 1H), 1.12 - 0.98 (m, 1H), 0.82 - 0.77 (m, 1H), 0.91 (s, 3H), 0.34 (s, 3H).

### Example 94: Synthesis of compound (1S,2S)-2-(difluoromethyl)-N-((1⁵S,6³S,4S,Z)-1²-(3-(dimethylamino)azetidin-1-yl)-1⁵,10,10-trim ethyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]o xazonino[5,6-a]indola-2(4,2)-thiazola-6(1,3)-pyridazinacycloundecaphane-4-yl)cyclopropane-1-carboxamide

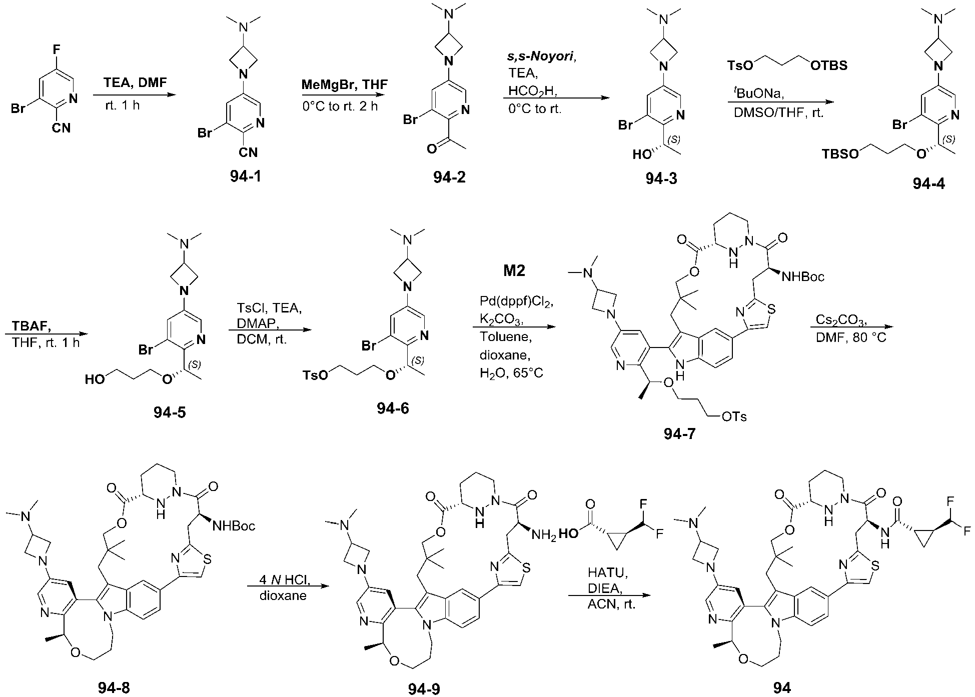

### Step 1: Synthesis of compound 94-1

Compound 3-bromo-5-fluoro-pyridine-2-carbonitrile (3.0 g) was added to a 50 mL single-necked flask, anhydrous DMF (30 mL) was added, TEA (9 mL) and 3-(dimethylamino)azetidine dihydrochloride (2.9 g) were weighed and added respectively, and reacted at room temperature for 0.5 h. After the reaction was complete, saturated brine (25 mL) was added, extracted with EA, and the organic phase was concentrated to dryness to obtain the target compound 94-1 (3.5 g, 83.5% yield). ESI-MS m/z: 281.2 [M+H]⁺.

### Step 2: Synthesis of compound 94-2

Compound 94-1 (3.5 g) and anhydrous THF (70 mL) were added to a 250 mL three-necked flask, the temperature was lowered to 0 °C under N₂ protection, MeMgBr (3 M in THF, 12 mL) was slowly added dropwise, and the mixture was warmed to room temperature and reacted for 2 h. After the reaction was complete, HCl (1N, 30 mL) was added to the reaction solution, extracted with EA, the organic phase was collected, the aqueous phase was further extracted twice with EA (30 mL), the organic phases were collected and combined, dried over anhydrous sodium sulfate, and the concentrate was purified by column chromatography to obtain the target compound 94-2 (3.34 g, 90% yield). ESI-MS m/z: 298.4 [M+H]⁺.

### Step 3: Synthesis of compound 94-3

Compound 94-2 (3.34 g) was added to a 25 mL single-necked flask, TEA (20 mL) and s,s-Noyori catalyst (0.356 g) were added, the temperature was lowered to 0 C under N₂ protection, formic acid (2 mL) was slowly added dropwise and reacted at room temperature for 12 h. After the reaction was complete, ethyl acetate (50 mL) and water (50 mL) were added to the reaction solution, extracted, the organic phase was collected, and the aqueous phase was further extracted twice with ethyl acetate (25 mL). The organic phases were collected and combined, dried over anhydrous sodium sulfate, concentrated, and the concentrate was purified by column chromatography to obtain the target axially chiral compound 94-3 (4.3 g, crude product). ESI-MS m/z: 300.3 [M+H]⁺.

### Step 4: Synthesis of compound 94-4

Compound 94-3 (1.05 g), 3-((tert-butyldimethylsilyl)oxy)propyl-4-methylbenzenesulfonate (2.25 g) and DMSO/ACN (12 mL/3 mL) were weighed and added to the reaction flask respectively, and the temperature was lowered to 10 °C under N₂ protection. Sodium tert-butoxide (1.0 g) was added, and the mixture was reacted at room temperature for 1.0 h. After the reaction was complete, the temperature was lowered to 0 °C, tap water (50 mL) was added for dilution, extracted with EA, the organic phase was dried over anhydrous sodium sulfate, concentrated, and the concentrate was purified by column chromatography to obtain 94-4 (1.99 g, crude product). ESI-MS m/z: 472.6 [M+H]⁺.

### Step 5: Synthesis of compound 94-5

Compound 94-4 (1.99 g) was added to a 50 mL single-necked flask, anhydrous TBAF (1N in THF, 7 mL) was added, and the mixture was reacted at room temperature for 1.0 h. After the reaction was complete, calcium carbonate (5 g), DOWEX(R)50WX8 resin (8 g) and methanol (20 mL) were added respectively, and the mixture was reacted at room temperature for 1.0 h. The mixture was filtered with suction, and the filtrate was concentrated to dryness to obtain the target compound 94-5 (2.37 g, crude product). ESI-MS m/z: 358.3 [M+H]⁺.

### Step 6: Synthesis of compound 94-6

Compound 94-5 (2.37 g) was added to a 50 mL single-necked flask, anhydrous DCM (20 mL) was added, TEA (3 mL) and DMAP (0.9 g) were weighed and added respectively, the temperature was lowered to 0 °C under N₂ protection, TsCl (2.53 g) was added, and the mixture was reacted at room temperature for 1.0 h. After the reaction was complete, saturated brine (25 mL) was added, extracted with DCM, the organic phase was concentrated to dryness, and the concentrate was purified by column chromatography to obtain the target compound 94-6 (1.10 g, 30% yield). ESI-MS m/z: 512.6 [M+H]⁺.

### Step 7: Synthesis of compound 94-7

Compound 94-6 (202 mg), M2 (110 mg), Pd(dppf)Cl₂ (30 mg), and potassium carbonate (63 mg) were respectively weighed to a 100 mL single-necked flask. Toluene (6 mL), 1,4-dioxane (2 mL) and 2 mL of water were added. N₂ was replaced 3 times. The mixture was warmed to 65 °C and reacted for 5 h. After the reaction was complete, EA and water (20 mL each) were added to the reaction solution, extracted, the aqueous phase was further extracted twice with EA. The organic phases were collected and combined, dried over anhydrous sodium sulfate, concentrated, and the concentrate was purified by column chromatography to obtain the target compound 94-7 (200 mg, 88% yield). ESI-MS m/z: 999.4 [M+H]⁺.

### Step 8: Synthesis of compound 94-8

Compound 94-7 (200 mg) was added to a 25 mL single-necked flask, Cs₂CO₃ (250 mg) and anhydrous DMF (12 mL) were weighed in, and the mixture was reacted at 80 °C for 1 h under N₂ protection. After the reaction was complete, tap water (50 mL) was added for dilution, extracted with EA. The organic phase was dried over anhydrous sodium sulfate, concentrated, and the concentrate was purified by column chromatography to obtain the target compound 94-8 (125 mg, 75.5% yield). ESI-MS m/z: 827.2 [M+H]⁺.

### Step 9: Synthesis of compound 94-9

Compound 94-8 (125 mg) was added to a 25 mL single-necked flask, 15 mL of hydrochloric acid (4 M solution in dioxane) was added, and the mixture was reacted at room temperature for 1 h. After the reaction was complete, it was concentrated under reduced pressure to dryness to obtain the target compound 94-9 hydrochloride (140 mg, crude product). ESI-MS m/z: 727.1 [M+H]⁺.

### Step 10: Synthesis of compound 94

Compound (1S,2S)-2-(difluoromethyl)cyclopropane-1-carboxylic acid (70 mg) was added to a 25 mL single-necked flask, HATU (131 mg), N,N-diisopropylethylamine (0.28 mL) and anhydrous acetonitrile (10 mL) were weighed in, and the mixture was reacted at room temperature for 10 min. A pre-liberated acetonitrile solution (5 mL) of compound 94-9 (140 mg) was added dropwise at room temperature, and the mixture was reacted at room temperature for 15 min. After the reaction was complete, it was directly concentrated, and the concentrate was purified by preparation (60% acetonitrile/water) to obtain the target compound 94 (19.7 mg, 13.44% yield). ESI-MS m/z: 845.3 [M+H]⁺.

¹H NMR (500 MHz, CDCl₃)δ 8.57 (s, 1H), 7.60 (d, J = 8.6 Hz, 1H), 7.31 (d, J = 10.8 Hz, 1H), 6.96 (s, 1H), 6.73 (d, J = 9.2 Hz, 1H), 5.97 - 5.81 (m, 2H)., 4.60 (d, J = 11.8 Hz, 1H), 4.36 (td, J = 12.1, 3.2 Hz, 1H), 4.27 - 4.17 (m, 2H), 4.10 (dd, J = 23.7, 9.7 Hz, 1H), 4.00 (dd, J = 14.5, 4.4 Hz, 1H), 3.85 - 3.75 (m, 5H), 3.50 - 3.43 (m, 1H), 3.32 (td, J = 12.3, 2.8 Hz, 1H), 3.22 - 3.12 (m, 2H), 2.85 (td, J = 14.0, 2.1 Hz, 2H), 2.70 (td, J = 12.9, 2.6 Hz, 1H), 2.47 - 2.39 (m, 1H), 2.36 (d, J = 13.4 Hz, 7H), 2.31 - 2.22 (m, 2H), 2.03 - 1.94 (m, 4H), 1.91 - 1.79 (m, 3H), 1.74 - 1.56 (m, 3H), 1.52 (d, J = 6.4 Hz, 3H), 1.28 (ddd, J = 14.3, 8.1, 4.9 Hz, 2H), 1.14 (dt, J = 8.6, 5.4 Hz, 1H), 0.97 (s, 3H).

### Example 95: Synthesis of compound (1S,2S)-2-methyl-N-((1⁹R,6³S,4S,Z)-1⁹,10,10-trimethyl-1²-(4-methylpiperazin-1-yl)-5,7-dioxo-1 ⁸,1⁹,6¹,6²,6³,6⁴,6⁵,6⁶-octahydro-1⁵H,1⁷H-8-oxa-1(13,15)-pyrido[3',2':7,8][1,5]oxazonino[5,6-a]in dola-2(4,2)-thiazola-6(1,3)-pyridazinacycloundecaphane-4-yl)cyclopropane-1-carboxamide

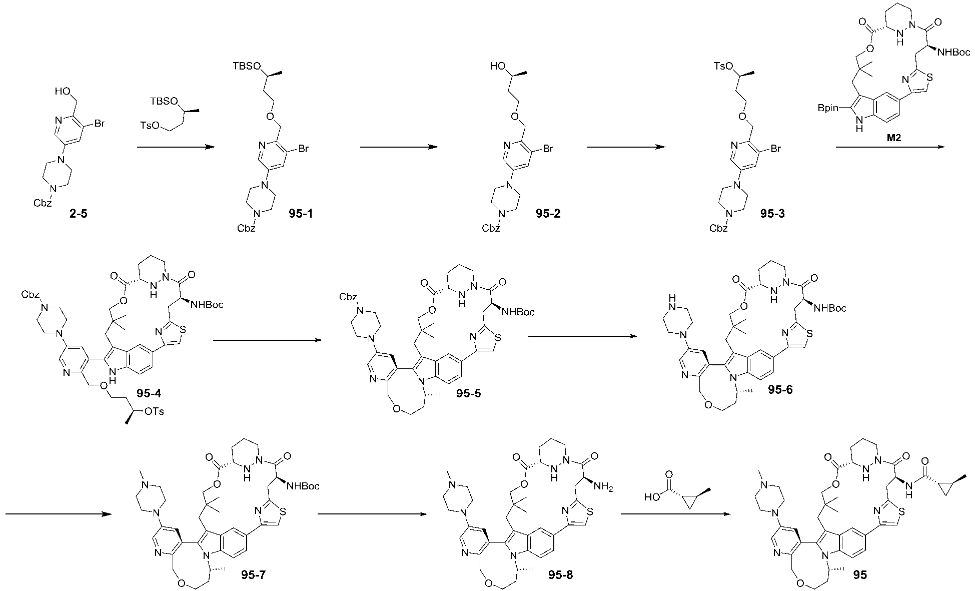

### Step 1: Synthesis of compound 95-1

Compound 2-5 (1.50 g, 3.69 mmol), (S)-3-((tert-butyldimethylsilyl)oxy)butyl 4-methylbenzenesulfonate (3.97 g, 11.08 mmol), DMSO (15 mL), THF (3 mL) were added sequentially to a 50 mL single-necked flask, sodium tert-butoxide (0.89 g, 9.23 mmol) was added, and the mixture was stirred and reacted at room temperature. After TLC monitoring indicated the reaction was complete, the reaction mixture was added to a saturated aqueous ammonium chloride solution for quenching. The mixture was extracted with ethyl acetate, washed with brine, separated, and the organic phase was concentrated to obtain a crude product. Purification by silica gel column chromatography (PE to PE/EA = 50/50) finally obtained the target compound 95-1 (1.59 g). ESI-MS m/z: 592.41, [M+H]⁺.

### Step 2: Synthesis of compound 95-2

Compound 95-1 (1.59 g, 0.84 mmol) and TBAF (15 mL, 1M solution in THF) were added to a 50 mL single-necked flask, and the mixture was stirred and reacted at room temperature overnight. After TLC monitoring indicated the reaction was complete, the reaction mixture was diluted with ethyl acetate, washed sequentially with saturated aqueous ammonium chloride solution and brine, separated, and the organic phase was concentrated to obtain a crude product. Purification by silica gel column chromatography (PE to PE/EA = 50/50) finally obtained the target compound 95-2 (1.28 g). ESI-MS m/z: 478.33, [M+H]⁺.

### Step 3: Synthesis of compound 95-3

Compound 95-2 (1.28 g, 2.676 mmol), DCM (13.00 mL), DIEA (1.33 mL, 8.03 mmol), DMAP (0.98 g, 8.03 mmol) were added sequentially to a 50 mL single-necked flask, then TosCl (1.02 g, 5.35 mmol) was added in batches. After the addition was complete, the mixture was stirred and reacted at room temperature. After LC-MS monitoring indicated the reaction was complete, silica gel was added to the reaction solution for sample preparation and sand preparation. Purification by silica gel column chromatography (PE/EA = 90/10 to 50/50) obtained compound 95-3 (1.2 g). ESI-MS m/z: 632.34, [M+H]⁺.

### Step 4: Synthesis of compound 95-4

Compound 95-3 (274 mg, 0.43 mmol), M2 (250 mg, 0.36 mmol), K₂CO₃ (125 mg, 0.90 mmol), [PdCl₂(dppf)] (26 mg, 0.04 mmol), toluene (3.00 mL), dioxane (1.00 mL), and water (1.00 mL) were added sequentially to a 10 mL single-necked flask. The mixture was purged and protected with nitrogen, heated in an oil bath, the temperature was maintained at 60 °C: to 65 °C and the mixture was reacted. After LC-MS monitoring indicated the reaction was complete, the reaction mixture was diluted with ethyl acetate, washed sequentially with water and brine, separated, and the aqueous phase was further extracted with DCM. The organic phases were combined and concentrated to obtain a crude product. Purification by Prep-TLC obtained compound 95-4 (230 mg). ESI-MS m/z: 1119.66, [M+H]⁺.

### Step 5: Synthesis of compound 95-5

Compound 95-4 (210 mg, 0.19 mmol), DMF (20.00 mL), and Cs₂CO₃ (183 mg, 0.56 mmol) were added sequentially to a 10 mL single-necked flask. Under nitrogen protection, the mixture was heated in an oil bath, the temperature was maintained at 60 °C to 65 °C and the mixture was reacted. After LC-MS monitoring indicated the reaction was complete, the reaction mixture was diluted with ethyl acetate, washed sequentially with water and brine, separated, and the organic phase was concentrated to obtain a crude product of compound 95-5. Purification by silica gel column chromatography finally obtained the target compound 95-5 (17 mg). ESI-MS m/z: 947.64, [M+H]⁺.

### Step 6: Synthesis of compound 95-6

Compound 95-5 (17 mg, 0.02 mmol), MeOH (2.00 mL), and Pd(OH)₂/C (20 mg, 0.14 mmol, 10% Pd) were added sequentially to a 10 mL single-necked flask. The mixture was purged with hydrogen, maintained under a hydrogen atmosphere, stirred and reacted at room temperature. After LC-MS monitoring indicated the reaction was complete, the mixture was filtered to remove the catalyst, and the filtrate was concentrated to obtain a crude product of compound 95-6 (17 mg), which was directly used in the next reaction. ESI-MS m/z: 813.60, [M+H]⁺.

### Step 7: Synthesis of compound 95-7

Compound 95-6 (15 mg, 0.02 mmol), MeOH (2.00 mL), 37% aqueous formaldehyde solution (17 mg), and HOAc (0.10 mL) were added sequentially to a 10 mL single-necked flask, and the mixture was stirred at room temperature for 20 min. NaBH₃CN (2.6 mg, 0.04 mmol) was added, and the mixture was stirred and reacted at room temperature. After LC-MS monitoring indicated the reaction was complete, ethylenediamine was added to quench the excess formaldehyde in the reaction mixture, and then the mixture was added to a saturated aqueous sodium bicarbonate solution for quenching, then extracted with DCM and separated; the organic phase was concentrated to obtain a crude product of compound 95-7 (17 mg). ESI-MS m/z: 827.62, [M+H]⁺.

### Step 8: Synthesis of compound 95-8

Compound 95-7 (17 mg, 0.02 mmol), DCM (1.00 mL), and HCl (4M solution in dioxane) (1.00 mL) were added sequentially to a 10 mL single-necked flask, and the mixture was stirred and reacted at room temperature. After LC-MS monitoring indicated the reaction was complete, the reaction mixture was concentrated to dryness, DCM was added again, and the mixture was concentrated to dryness again. A crude product of compound 95-8 (15 mg, hydrochloride salt) was obtained as a slightly yellow solid powder, which was directly used in the next reaction. ESI-MS m/z: 727.57, [M+H]⁺.

### Step 9: Synthesis of compound 95

(1S,2S)-2-methylcyclopropanecarboxylic acid (2.5 mg, 0.025 mmol), HATU (16 mg, 0.041 mmol), and DIEA (0.02 mL, 0.124 mmol) were added sequentially to a 10 mL single-necked flask, and the mixture was stirred at room temperature for 30 min. The mixture was cooled in an ice-water bath, and then a solution of compound 95-8 (15.00 mg, 0.021 mmol) in acetonitrile was added. After LC-MS monitoring indicated the reaction was complete, the reaction mixture was directly purified by Prep-HPLC acidic method, and the eluate was lyophilized to finally obtain the target compound 95 (2.8 mg). ESI-MS m/z: 809.61, [M+H]⁺.

### Example 96: Synthesis of compound (1S,2S)-2-(difluoromethyl)-N-((1⁵S,6³S,4S,Z)-1⁵,10,10-trimethyl-1²-(4-(4-methylpiperazin-1-yl) phenyl)-5,7-dioxo-1⁸,1⁹,6¹,6²,6³,6⁴,6⁵,6⁶-octahydro-15H,17H-8-oxa-1(13,15)-pyrido[3',2':7,8][1, 5]oxazonino[5,6-a]indola-2(4,2)-thiazola-6(1,3)-pyridazinacycloundecaphane-4-yl)cyclopropane -1-carboxamide

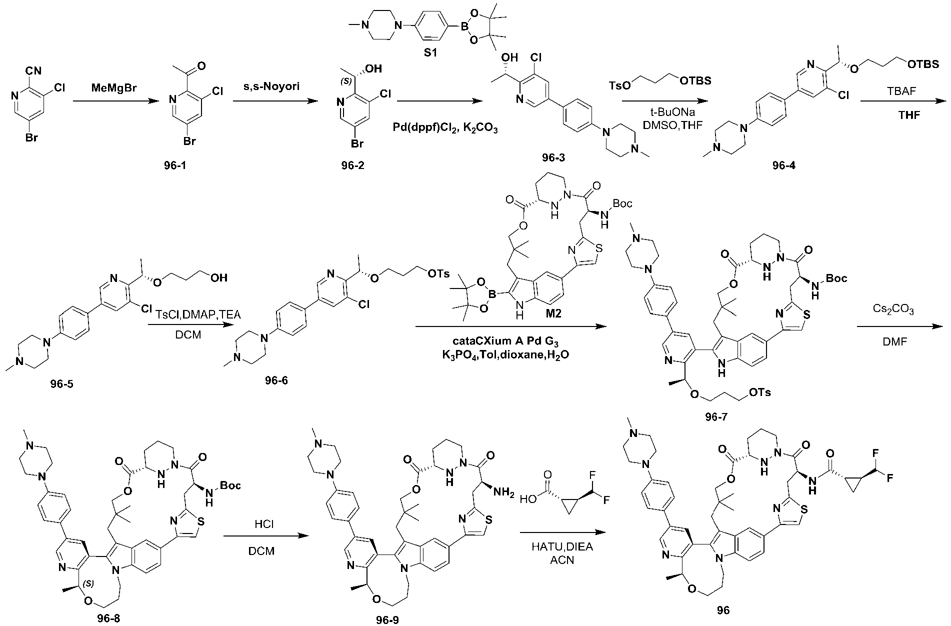

### Step 1: Synthesis of compound 96-1

Compound 5-bromo-3-chloroacrylonitrile (2.0 g, 9.2 mmol) was added to anhydrous THF (20.00 mL) under nitrogen protection, and methylmagnesium bromide (8.58 mL, 25.75 mmol) was added dropwise at -5 °C. After the addition was complete, the mixture was reacted at -5 °C for 2 h, poured into a saturated ammonium chloride solution. The pH was adjusted to 2 with 1N HCl. The mixture was stirred at room temperature for 5 min. The pH was adjusted to 8 with sodium carbonate. The mixture was extracted with EA, washed with brine, dried, and separated by column chromatography to obtain compound 96-1 (2.0 g, 92.74%). ESI-MS m/z: 235.28, [M+H]⁺.

### Step 2: Synthesis of compound 96-2

Triethylamine (14.23 mL, 102.36 mmol) was added to a 50 mL three-necked flask under nitrogen protection, formic acid (1.96 g, 42.65 mmol) was added dropwise at 0 °C. After the addition was complete, (s,s)-Noyori catalyst (0.27 g, 0.43 mmol) was added at 0 °C, stirred for 10 min, compound 96-1 (2.00 g, 8.53 mmol) was added, and the mixture was warmed to room temperature and reacted for 24 h. EA was added for dilution. The mixture was washed with brine, dried over anhydrous sodium sulfate, and separated by column chromatography (PE:EA=4:1) to obtain compound 96-2 (1.80 g, 89.23%). ESI-MS m/z: 237.26, [M+H]⁺.

### Step 3: Synthesis of compound 96-3

Compound 96-2 (1.00 g, 4.23 mmol), S1 (1.41 g, 4.65 mmol), PdCl₂(dppf) (0.31 g, 0.42 mmol), potassium carbonate (1.75 g, 12.69 mmol), 1,4-dioxane (15.00 mL), and water (3.00 mL) were added to a 20 mL microwave tube, bubbled with nitrogen, and reacted at 70 °C for 3 h. Water was added. The mixture was extracted with EA, washed with brine, dried and rotary evaporated, and separated by column chromatography to obtain compound 96-3 (1.32 g, 94.07%). ESI-MS m/z: 332.41, [M+H]⁺.

### Step 4: Synthesis of compound 96-4

Compound 96-3 (1.50 g, 4.52 mmol) and compound 3-((tert-butyldimethylsilyl)oxy)propyl-4-methylbenzenesulfonate (4.67 g, 13.56 mmol) were added to DMSO (15.00 mL), and then a solution of sodium tert-butoxide (1.09 g, 11.30 mmol) in tetrahydrofuran (3.00 mL) was added, and the mixture was reacted at 40 °C for 1 h. 50 mL of water was added to the reaction solution, extracted twice with 40 mL of EA, washed twice with 40 mL of saturated brine, dried over anhydrous sodium sulfate, and separated by column chromatography to obtain compound 96-4 (1.20 g, 52.65%). ESI-MS m/z: 504.28, [M+H]⁺.

### Step 5: Synthesis of compound 96-5

Compound 96-4 (1.2 g, 2.38 mmol) and tetrabutylammonium fluoride (3.57 mL, 1.00 mol/L, 3.57 mmol) were added to tetrahydrofuran (10.00 mL), and the mixture was reacted at 50 °C for 1 h. The mixture was concentrated to dryness and separated by column chromatography to obtain compound 96-5 (0.52 mg, 56.03%). ESI-MS m/z: 390.35, [M+H]⁺.

### Step 6: Synthesis of compound 96-6

Compound 96-5 (0.50 g, 1.28 mmol), DMAP (0.16 g, 1.28 mmol), and TEA (0.36 mL, 2.56 mmol) were added to DCM (10.00 mL), p-toluenesulfonyl chloride (0.37 g, 1.92 mmol) was added in batches in an ice-water bath, and the mixture was reacted at room temperature for 2 h. 50 mL of water was added to the reaction solution, extracted twice with 40 mL of DCM, washed twice with 40 mL of saturated brine, dried over anhydrous sodium sulfate, and separated by column chromatography to obtain compound 96-6 (0.52 g, 74.53%). ESI-MS m/z: 544.26, [M+H]⁺.

### Step 7: Synthesis of compound 96-7

Compound 96-6 (258.88 mg, 0.48 mmol), compound M2 (300.00 mg, 0.43 mmol), (2-dicyclohexylphosphino-2',6'-diisopropoxy-1,1'-biphenyl)[2-(2'-amino-1,1'-biphenyl)]palladiu m(II) methanesulfonate (31.53 mg, 0.04 mmol), potassium phosphate (183.61 mg, 0.86 mmol), water (0.50 mL), and 1,4-dioxane (2.00 mL) were added to toluene (6.00 mL), and the mixture was reacted at 70 °C for 3 h under a nitrogen atmosphere. 50 mL of water was added to the reaction solution, extracted twice with 40 mL of EA, washed twice with 40 mL of saturated brine, dried over anhydrous sodium sulfate, and separated by column chromatography to obtain compound 96-7 (270.00 mg, 58.05%). ESI-MS m/z: 538.62, [M+H]⁺.

### Step 8: Synthesis of compound 96-8

Compound 96-7 (270.00 mg, 0.25 mmol) and cesium carbonate (163.63 mg, 0.50 mmol) were added to DMF (15.00 mL), and the mixture was reacted at 70 °C for 2 h. 50 mL of water was added to the reaction solution, extracted twice with 40 mL of EA, washed twice with 40 mL of saturated brine, dried over anhydrous sodium sulfate, and separated by column chromatography to obtain compound 96-8 (170 mg, 74.96%). ESI-MS m/z: 903.63, [M+H]⁺.

### Step 9: Synthesis of compound 96-9

Compound 96-8 (170.00 mg, 0.19 mmol) was dissolved in DCM (1.00 mL), then hydrochloric acid (4M solution in dioxane) (2.00 mL) was added, and the mixture was reacted at room temperature for 1 h. The reaction solution was concentrated to dryness, 5 mL of toluene was added for dilution, and concentrated to dryness again to obtain a crude product of compound 96-9 (150 mg, 99.25%), which was directly used in the next reaction. ESI-MS m/z: 803.57, [M+H]⁺.

### Step 10: Synthesis of compound 96

Compound (1S,2S)-2-(difluoromethyl)cyclopropane-1-carboxylic acid (16.26 mg, 0.12 mmol) was dissolved in acetonitrile (3.00 mL), DIEA (0.16 mL, 1.00 mmol) and HATU (58.81 mg, 0.15 mmol) were added sequentially in an ice-water bath. After reacting in the ice-water bath for 10 min, a solution of compound 96-9 (80.00 mg, 0.10 mmol) in acetonitrile (3.00 mL) and DIEA (0.16 mL, 1.00 mmol) was added and reacted at room temperature for 1 h. The mixture was concentrated to dryness and purified by preparative separation to obtain compound 96 (23.00 mg, 24.59%). ESI-MS m/z: 921.57, [M+H]⁺.

¹H NMR (500 MHz, DMSO)δ 9.97 (s, 1H), 9.02 (d, J = 2.2 Hz, 1H), 8.92 (d, J = 9.0 Hz, 1H), 8.53 (s, 1H), 7.99 (d, J = 2.2 Hz, 1H), 7.84 (s, 1H), 7.75 (dd, J = 15.3, 5.1 Hz, 3H), 7.54 (d, J = 8.6 Hz, 1H), 7.14 (d, J = 8.9 Hz, 2H), 5.95 (td, J = 56.7, 5.1 Hz, 1H), 5.56 (t, J = 9.4 Hz, 1H), 5.14 (d, J = 12.2 Hz, 1H), 4.31 (t, J = 6.3 Hz, 1H), 4.23 (dd, J = 15.9, 7.4 Hz, 2H), 4.14 - 4.03 (m, 2H), 3.97 (s, 2H), 3.71 (d, J = 10.9 Hz, 1H), 3.62 (dd, J = 25.9, 11.6 Hz, 2H), 3.53 (d, J = 11.8 Hz, 2H), 3.38 (d, J = 14.4 Hz, 1H), 3.26 (t, J = 11.5 Hz, 1H), 3.14 (dd, J = 14.7, 8.9 Hz, 3H), 3.03 (t, J = 12.9 Hz, 3H), 2.87 (d, J = 3.7 Hz, 3H), 2.76 (d, J = 6.6 Hz, 1H), 2.38 (dd, J = 23.8, 13.4 Hz, 2H), 2.13 - 2.07 (m, 2H), 1.81 (s, 3H), 1.70 - 1.61 (m, 1H), 1.57 - 1.48 (m, 1H), 1.44 (d, J = 6.3 Hz, 3H), 1.09 - 0.98 (m, 2H), 0.92 (s, 3H), 0.34 (s, 3H).

### Example 97: Synthesis of compound (1S,2S)-2-(difluoromethyl)-N-((1⁵S,6³S,4S,Z)-1⁵,10,10-trimethyl-1²-((1-methylpiperidin-4-yl)ox y)-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxaz onino[5,6-a]indola-2(4,2)-thiazola-6(1,3)-pyridazinacycloundecaphane-4-yl)cyclopropane-1-carb oxamide

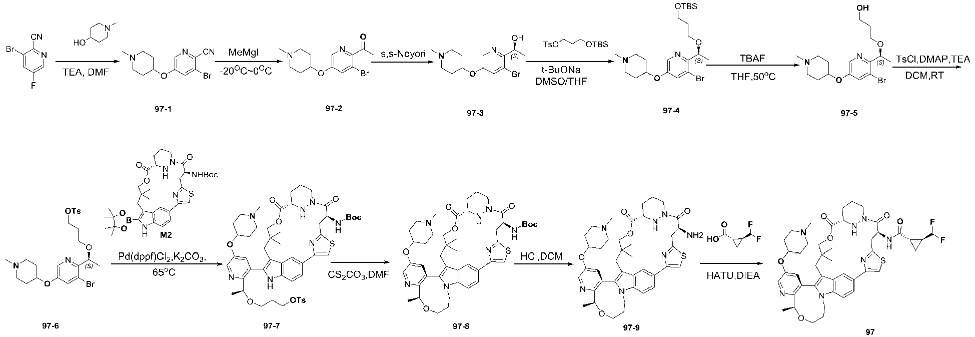

### Step 1: Synthesis of compound 97-1

1-Methylpiperidin-4-ol (0.77 g, 6.72 mmol) and N,N-dimethylformamide (10.00 mL) were added sequentially to a 25 mL single-necked flask. Sodium hydride (0.40 g, 60%, 9.95 mmol) was added in batches at 0 °C, and the mixture was stirred at 0 °C for 10 min. Finally, 3-bromo-5-fluoro-pyridine-2-carbonitrile (1.00 g, 4.98 mmol) was added. The mixture was stirred at room temperature for 1 h. After LC-MS monitoring indicated the reaction was complete, the reaction solution was poured into water. A solid precipitated, filtered, and the filter cake was washed with water. The filter cake was dried to obtain the target compound 97-1 (1.14 g). ESI-MS m/z: 296.0 [M+H]⁺.

### Step 2: Synthesis of compound 97-2

Compound 97-1 (1.14 g, 3.85 mmol) and tetrahydrofuran (10.00 mL) were added sequentially to a 50 mL three-necked flask, the temperature was lowered to -20 °C under nitrogen protection, and then methylmagnesium iodide (3.85 mL, 3.00 mol/L, 11.55 mmol) was added dropwise. The mixture was stirred at 0 °C for 1 h. After LC-MS monitoring indicated the reaction was complete, the mixture was quenched with saturated NH₄Cl solution, adjusted to pH=4 with dilute hydrochloric acid, and stirred for 30 min. The mixture was then adjusted to pH=8 with saturated NaHCO3 solution, extracted twice with ethyl acetate, and the organic layer was washed with saturated brine and dried over anhydrous sodium sulfate. The mixture was concentrated under reduced pressure and purified by column chromatography to obtain the target compound 97-2 (0.39 g). ESI-MS m/z: 313.0 [M+H]⁺.

### Step 3: Synthesis of compound 97-3

Triethylamine (4.34 mL, 31.21 mmol) was added to a 25 mL three-necked flask, the temperature was lowered to 0 C under nitrogen protection, formic acid (0.24 mL, 6.24 mmol) was added dropwise, and then (S,S)-N-(p-toluenesulfonyl)-1,2-diphenylethane-1,2-diamine (p-cymene)ruthenium(II) chloride (15.78 mg, 0.03 mmol) was added. The reaction was warmed to 40 °C and stirred for 15 min. After cooling to room temperature, compound 97-2 (391.00 mg, 1.25 mmol) and dichloromethane (1.00 mL) were finally added, and the mixture was stirred at room temperature for 24 h. After LC-MS monitoring indicated the reaction was complete, the mixture was extracted twice with dichloromethane, and the organic layer was washed with saturated brine and dried over anhydrous sodium sulfate. The mixture was concentrated under reduced pressure and purified by column chromatography to obtain the target compound 97-3 (279.00 mg). ESI-MS m/z: 315.1 [M+H]⁺.

### Step 4: Synthesis of compound 97-4

Compound 97-3 (259.00 mg, 0.82 mmol), dimethyl sulfoxide (4.00 mL), tetrahydrofuran (1.00 mL), and 3-((tert-butyldimethylsilyl)oxy)propyl-4-methylbenzenesulfonate (990.90 mg, 2.88 mmol) were added sequentially to a 25 mL single-necked flask. Sodium tert-butoxide (197.42 mg, 2.05 mmol) was added in batches at 10 °C, and the mixture was stirred at room temperature for 1 h. After LC-MS monitoring indicated the reaction was complete, and the reaction mixture was added to a saturated aqueous ammonium chloride solution for quenching. The mixture was extracted twice with ethyl acetate, washed three times with saturated brine, concentrated under reduced pressure, and purified by column chromatography to obtain the target compound 97-4 (293.00 mg). ESI-MS m/z: 487.2 [M+H]⁺.

### Step 5: Synthesis of compound 97-5

Compound 97-4 (293.00 mg, 0.60 mmol) and tetrahydrofuran (5.00 mL) were added sequentially to a 25 mL single-necked flask. Tetrabutylammonium fluoride (0.90 mL, 1.00 mol/L, 0.90 mmol) was added dropwise at 0 °C, and the reaction was warmed to 50 °C and stirred for 1 h. After LC-MS monitoring indicated the reaction was complete, concentrated under reduced pressure, and purified by column chromatography to obtain the target compound 97-5 (183.00 mg). ESI-MS m/z: 373.1 [M+H]⁺.

### Step 6: Synthesis of compound 97-6

Compound 97-5 (173.00 mg, 0.46 mmol), dichloromethane (3.00 mL), and 4-dimethylaminopyridine (5.67 mg, 0.05 mmol) were added sequentially to a 25 mL single-necked flask. Then p-toluenesulfonyl chloride (176.73 mg, 0.93 mmol) and triethylamine (0.16 mL, 1.16 mmol) were added in batches, and the mixture was stirred at room temperature for 4 h. After LC-MS monitoring indicated the reaction was complete, concentrated under reduced pressure, and purified by column chromatography to obtain the target compound 97-6 (254.00 mg). ESI-MS m/z: 527.1 [M+H]⁺.

### Step 7: Synthesis of compound 97-7

Compound 97-6 (78.86 mg, 0.15 mmol), M2 (80.00 mg, 0.12 mmol), toluene (1.50 mL), 1,4-dioxane (0.50 mL), water (0.50 mL), potassium carbonate (39.74 mg, 0.29 mmol), and [1,1'-bis(diphenylphosphino)ferrocene]dichloropalladium(II) dichloromethane complex (9.39 mg, 0.01 mmol) were added sequentially to a 10 mL single-necked flask. Nitrogen was replaced three times, and under nitrogen protection, the reaction was warmed to 65 °C and stirred for 3 h. After LC-MS monitoring indicated the reaction was complete, the mixture was extracted twice with ethyl acetate, washed with saturated brine, concentrated under reduced pressure, and purified by column chromatography to obtain the target compound 97-7 (42.00 mg). ESI-MS m/z: 507.7 1/2[M+2H]+.

### Step 8: Synthesis of compound 97-8

Compound 97-7 (42.00 mg, 0.04 mmol), N,N-dimethylformamide (1.50 mL), and cesium carbonate (40.47 mg, 0.12 mmol) were added sequentially to a 10 mL single-necked flask, and the reaction was warmed to 80 °C and stirred for 1 h. After LC-MS monitoring indicated the reaction was complete, the reaction solution was diluted with ethyl acetate, washed sequentially with water and brine three times, concentrated under reduced pressure, and purified by column chromatography to obtain the target compound 97-8 (32.00 mg). ESI-MS m/z: 842.4 [M+H]⁺.

### Step 9: Synthesis of compound 97-9

Compound 97-8 (32.00 mg, 0.04 mmol), dichloromethane (1.00 mL), and hydrochloric acid (4M solution in dioxane) (1.00 mL) were added sequentially to a 10 mL single-necked flask, and the mixture was stirred at room temperature for 0.5 h. After LC-MS monitoring indicated the reaction was complete, the mixture was concentrated under reduced pressure to obtain the crude product compound 97-9 (28.00 mg), which was directly used in the next step. ESI-MS m/z: 742.4 [M+H]⁺.

### Step 10: Synthesis of compound 97

(1S,2S)-2-(difluoromethyl)cyclopropane-1-carboxylic acid (7.70 mg, 0.06 mmol) and N,N-dimethylformamide (1.00 mL) were added sequentially to a 10 mL single-necked flask. N,N,N',N'-tetramethyl-O-(7-azabenzotriazol-1-yl)uranium hexafluorophosphate (21.52 mg, 0.06 mmol) and N,N-diisopropylethylamine (0.06 mL, 0.38 mmol) were added at 0 °C, and the mixture was stirred at 0 °C for 10 min. Finally, 97-9 (28.00 mg, 0.04 mmol) was added, and the mixture was stirred at 0 °C for 10 min. After LC-MS monitoring indicated the reaction was complete, and the reaction solution was directly purified by pre-HPLC to obtain the target compound 97 (10.70 mg). ESI-MS m/z: 860.4 [M+H]⁺.

¹H NMR (500 MHz, Methanol-d4)δ 8.56 (d, J = 1.6 Hz, 1H), 8.43 (d, J = 2.9 Hz, 1H), 7.70 (dd, J = 8.7, 1.6 Hz, 1H), 7.56 (s, 1H), 7.48 - 7.42 (m, 2H), 5.80 (dd, J = 16.0, 4.4 Hz, 1H), 5.71 - 5.65 (m, 2H), 4.43 (d, J = 12.0 Hz, 1H), 4.35 - 4.27 (m, 2H), 4.17 (dd, J = 12.4, 5.8 Hz, 1H), 4.07 (dd, J = 14.6, 5.0 Hz, 1H), 3.81 - 3.66 (m, 3H), 3.46 (d, J = 14.9 Hz, 1H), 3.26 - 3.19 (m, 2H), 2.77 (s, 3H), 2.49 - 2.44 (m, 1H), 2.39 (t, J = 16.7 Hz, 1H), 2.27 - 2.15 (m, 3H), 2.07 (dt, J = 9.1, 4.8 Hz, 3H), 1.95 (t, J = 15.2 Hz, 2H), 1.86 - 1.71 (m, 6H), 1.61 (dt, J = 13.1, 6.5 Hz, 1H), 1.48 (d, J = 6.5 Hz, 3H), 1.15 (dtt, J = 14.7, 10.3, 4.9 Hz, 4H), 1.01 (d, J = 11.9 Hz, 4H), 0.40 (s, 3H).

### Example 98: Synthesis of compound N-((1⁵S,6³S,4S,Z)-1²-(4-(2,2-difluoroethyl)piperazin-1-yl)-1⁵,10,10-trimethyl-5,7-dioxo-1⁸,1⁹,6¹, 6²,6³,6⁴,6⁵,6⁶-octahydro-1⁵H,1⁷H-8-oxa-1(13,15)-pyrido[3',2':7,8][1,5]oxazonino[5,6-a]indola-2( 4,2)-thiazola-6(1,3)-pyridazinacycloundecaphane-4-yl)-3-methylbicyclo[1.1.1]pentane-1-carbox amide

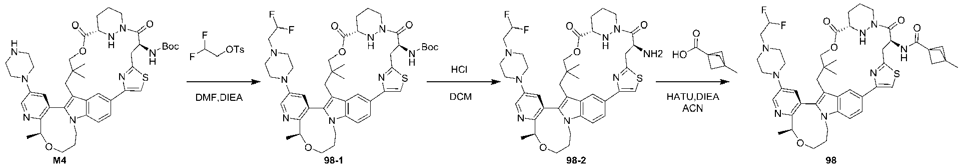

### Step 1: Synthesis of compound 98-1

Compound M4 (100.00 mg, 0.12 mmol), compound 2,2-difluoroethyl p-toluenesulfonate (28.97 mg, 0.14 mmol), and DIEA (0.06 mL, 0.37 mmol) were added to DMF (3.00 mL), and the mixture was reacted at 40 °C for 2 h. Water (50 mL) was added to the reaction solution, extracted twice with 40 mL of EA, washed twice with 40 mL of saturated brine, dried over anhydrous sodium sulfate, and separated by column chromatography to obtain a crude product of compound 98-1 (90.00 mg, 83.43%). ESI-MS m/z: 877.65 [M+H]⁺.

### Step 2: Synthesis of compound 98-2

Compound 98-1 (90.00 mg, 0.10 mmol) was dissolved in DCM (1.00 mL), then hydrochloric acid (4M solution in dioxane) (2.00 mL) was added, and the mixture was reacted at room temperature for 1 h. The reaction solution was concentrated to dryness, 5 mL of toluene was added for dilution, and concentrated to dryness again to obtain a crude product of compound 98-2 (70 mg, 87.81%), which was directly used in the next reaction. ESI-MS m/z: 777.54, [M+H]⁺.

### Step 3: Synthesis of compound 98

Compound 1-methylbicyclo[1.1.1]pentane-3-carboxylic acid (13.64 mg, 0.11 mmol) was dissolved in acetonitrile (2.00 mL). DIEA (0.15 mL, 0.90 mmol) and HATU (51.41 mg, 0.14 mmol) were added sequentially in an ice-water bath. After reacting in the ice-water bath for 10 min, a solution of compound 98-2 (70.00 mg, 0.09 mmol) in acetonitrile (2.00 mL) and DIEA (0.15 mL, 0.90 mmol) was added, and the mixture was reacted at room temperature for 1 h. The mixture was concentrated to dryness and purified by preparative separation to obtain compound 98 (32.00 mg, 39.64%). ESI-MS m/z: 885.43, [M+H]⁺.

¹H NMR (500 MHz, DMSO)δ 8.50 (s, 1H), 8.41 (d, J = 2.8 Hz, 1H), 8.00 (d, J = 9.0 Hz, 1H), 7.80 (s, 1H), 7.71 (dd, J = 8.6, 1.4 Hz, 1H), 7.49 (d, J = 8.6 Hz, 1H), 7.23 (d, J = 2.8 Hz, 1H), 6.18 (tt, J = 55.7, 4.3 Hz, 1H), 5.48 (t, J = 9.4 Hz, 1H), 5.09 (d, J = 12.2 Hz, 1H), 4.28 - 4.19 (m, 2H), 4.15 (q, J = 6.3 Hz, 1H), 4.03 (dd, J = 12.7, 4.9 Hz, 2H), 3.68 (d, J = 10.9 Hz, 1H), 3.60 (t, J = 14.0 Hz, 2H), 3.38 (dd, J = 14.7, 9.3 Hz, 1H), 3.30 (s, 1H), 3.27 (s, 4H), 3.16 (t, J = 11.4 Hz, 1H), 3.00 (d, J = 14.5 Hz, 1H), 2.78 (tt, J = 22.3, 11.1 Hz, 3H), 2.68 (s, 4H), 2.41 (d, J = 14.2 Hz, 1H), 2.29 (s, 1H), 2.10 (d, J = 11.1 Hz, 1H), 1.90 - 1.76 (m, 9H), 1.56 - 1.47 (m, 1H), 1.37 (d, J = 6.3 Hz, 3H), 1.19 (s, 3H), 0.96 (s, 3H), 0.32 (s, 3H).

### Example 99: Synthesis of compound (1S,2S)-2-(difluoromethyl)-N-((1⁵S,6³S,4S,Z)-1⁵,10,10-trimethyl-1²-(3-(4-methylpiperazin-1-yl) azetidin-1-yl)-5,7-dioxo-1⁸,1⁹,6¹,6²,6³,6⁴,6⁵,6⁶-octahydro-1⁵H,1⁷H-8-oxa-1(13,15)-pyrido[3',2':7, 8][1,5]oxazonino[5,6-a]indola-2(4,2)-thiazola-6(1,3)-pyridazinacycloundecaphane-4-yl)cyclopro pane-1-carboxamide

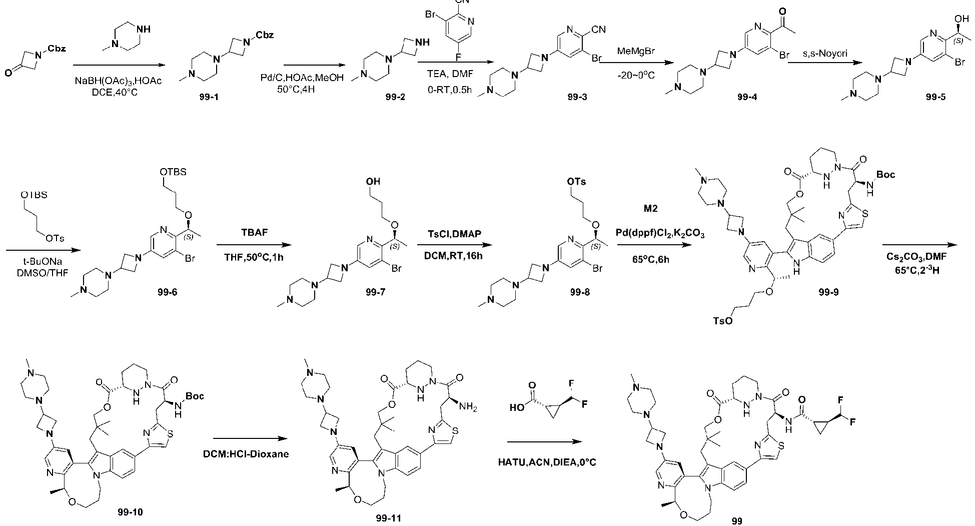

### Step 1: Synthesis of compound 99-1

Benzyl 3-oxoazetidine-1-carboxylate (5 g, 24.37 mmol), 1,2-dichloroethane (25.00 mL), 1-methylpiperazine (4.07 mL, 36.55 mmol), and glacial acetic acid (2.09 mL, 36.55 mmol) were added sequentially to a 100 mL single-necked flask, and the mixture was stirred at 40 °C for 3 h. Then sodium triacetoxyborohydride (10.33 g, 48.73 mmol) was added, and the mixture was stirred at 40 °C for 2 h. After the reaction was complete as monitored by LC-MS, the reaction solution was diluted with dichloromethane, washed sequentially with water and brine, separated, and the organic phase was concentrated to obtain a crude product, which was purified by silica gel column chromatography to finally obtain the target compound 99-1 (6.34 g, off-white solid powder). ESI-MS m/z: 290 [M+H]⁺.

### Step 2: Synthesis of compound 99-2

Compound 99-1 (3 g, 10.37 mmol), palladium on carbon (1.54 g, 10%, 1.45 mmol), methanol (30.00 mL), and acetic acid (10 drops) were added sequentially to a 100 mL single-necked flask. After purging with H₂, the mixture was heated in an oil bath and kept at 50 °C for reaction for 4 h. After the reaction was complete as monitored by LC-MS, the reaction solution was filtered through celite, and the filtrate was concentrated to dryness. The resultant was purified by silica gel column chromatography to finally obtain the target compound 99-2 (1.6 g, off-white solid). ESI-MS m/z: 156 [M+H]⁺.

### Step 3: Synthesis of compound 99-3

3-Bromo-5-fluoro-pyridine-2-carbonitrile (2 g, 9.95 mmol) and N,N-dimethylformamide (20.00 mL) were added sequentially to a 100 mL single-necked flask. At 0 °C, compound 99-2 (1.54 g, 9.95 mmol) and triethylamine (4.15 mL, 29.85 mmol) were added dropwise. After the addition was complete, the mixture was transferred to room temperature and stirred for 0.5 h. After the reaction was complete as monitored by LC-MS, the mixture was extracted 3 times with water and ethyl acetate. The organic phases were combined, washed sequentially with water and brine, separated, and concentrated to obtain a crude product, which was purified by silica gel column chromatography to finally obtain the target compound 99-3 (1.9 g, off-white solid). ESI-MS m/z: 336, 338 [M+H]⁺.

### Step 4: Synthesis of compound 99-4

Compound 99-3 (1.9 g, 4.16 mmol) and THF (15 mL) were added sequentially to a 50 mL three-necked flask. Nitrogen was purged three times, the temperature was lowered to -20 °C, methylmagnesium bromide (3.89 mL, 3.00 mol/L, 11.66 mmol) was added dropwise. After the addition was complete, the mixture was transferred to 0 °C and stirring was continued for reaction for 2 h. After the reaction was complete as monitored by LC-MS, 1M dilute hydrochloric acid was added dropwise to adjust the pH to about 3 to 4; the mixture was stirred at room temperature for 15 min, basified with saturated aqueous sodium bicarbonate solution, extracted with ethyl acetate, washed with brine, separated, and the organic phase was concentrated to obtain a crude product, which was purified by silica gel column chromatography to finally obtain the target compound 99-4 (1.78 g, reddish-brown mucus). ESI-MS m/z: 354, 356 [M+H]⁺.

### Step 5: Synthesis of compound 99-5

Triethylamine (8.40 mL, 60.47 mmol) was added sequentially to a 100 mL three-necked flask. At 0 °C, formic acid (0.48 mL, 12.60 mmol) was added, then (S,S)-N-(p-toluenesulfonyl)-1,2-diphenylethane-1,2-diamine (p-cymene)ruthenium(II) chloride (0.03 g, 0.05 mmol) was added, and the mixture was stirred at 40 °C for 15 min. After cooling to room temperature, compound 99-4 (1.78 g, 5.04 mmol) was added, and the reaction was carried out at room temperature (oil temperature 28 °C) for 20 h. After the reaction was complete as monitored by LC-MS, the reaction mixture was added to an aqueous solution, extracted with ethyl acetate, washed with brine, separated, and the organic phase was concentrated to obtain a crude product, which was purified by silica gel column chromatography to finally obtain the target compound 99-5 (1.6 g, reddish-brown mucus). ESI-MS m/z: 356, 358 [M+H]⁺.

### Step 6: Synthesis of compound 99-6

Compound 99-5 (600.00 mg, 1.69 mmol) and 3-((tert-butyldimethylsilyl)oxy)propyl-4-methylbenzenesulfonate (1163.72 mg, 3.38 mmol) were added sequentially to a 50 mL single-necked flask, dissolved in dimethyl sulfoxide (6.00 mL). Under ice-water cooling, a solution of sodium tert-butoxide (405.73 mg, 4.22 mmol) in THF/tetrahydrofuran (1.20 mL) was added, and the mixture was transferred to room temperature and stirred for 0.5 h. After the reaction was complete as monitored by LC-MS, the reaction mixture was added to an aqueous solution, extracted with dichloromethane, washed with brine, separated, and the organic phase was concentrated to obtain a crude product, which was purified by silica gel column chromatography to finally obtain the target compound 99-6 (570 mg, reddish-brown mucus). ESI-MS m/z: 527, 529 [M+H]⁺.

### Step 7: Synthesis of compound 99-7

Compound 99-6 (570 mg, 0.89 mmol), tetrahydrofuran (5.00 mL), and TBAF (1.34 mL, 1M solution in THF) were added to a 50 mL single-necked flask, and the mixture was stirred at 50 °C for 1 h. After the reaction was complete as monitored by LC-MS, silica gel was added for sample preparation, followed by purification by silica gel column chromatography to finally obtain the target compound 99-7 (300 mg, reddish-brown mucus). ESI-MS m/z: 414, 416 [M+H]⁺.

### Step 8: Synthesis of compound 99-8

Compound 99-7 (200 mg, 0.48 mmol), 4-dimethylaminopyridine (5.91 mg, 0.05 mmol), dichloromethane (3.00 mL), and triethylamine (0.17 mL, 1.21 mmol) were added sequentially to a 50 mL single-necked flask. At 0 °C under nitrogen protection, p-toluenesulfonyl chloride (184.47 mg, 0.97 mmol) was added. After the addition was complete, the mixture was transferred to room temperature and stirred overnight. After the reaction was complete as monitored by LC-MS, the reaction mixture was added to an aqueous solution, extracted with dichloromethane, washed with brine, separated, and the organic phase was concentrated to obtain a crude product, which was purified by silica gel column chromatography to finally obtain the target compound 99-8 (270 mg, reddish-brown mucus). ESI-MS m/z: 568, 570 [M+H]⁺.

### Step 9: Synthesis of compound 99-9

Compound 99-8 (100 mg, 0.18 mmol), M2 (122.22 mg, 0.18 mmol), potassium carbonate (60.88 mg, 0.44 mmol), [1,1'-bis(diphenylphosphino)ferrocene]dichloropalladium(II) dichloromethane complex (14.37 mg, 0.02 mmol), toluene (3.00 mL), 1,4-dioxane (1.00 mL), and water (1.00 mL) were added sequentially to a 10 mL single-necked flask. Nitrogen was purged three times, and the mixture was stirred at 60 °C to 65 °C for 1.5 h. After the reaction was complete as monitored by LC-MS, the reaction solution was diluted with ethyl acetate, washed sequentially with water and brine, separated, the organic phases were combined, and concentrated to obtain a crude product, which was purified by silica gel column chromatography to finally obtain the target compound 99-9 (85 mg, reddish-brown mucus). ESI-MS m/z: 528, [M+2H]²⁺/2.

### Step 10: Synthesis of compound 99-10

Compound 99-9 (85 mg, 0.08 mmol), DMF (3 mL), and Cs₂CO₃ (52.52 mg, 0.16 mmol) were added sequentially to a 10 mL single-necked flask. Under nitrogen protection, the mixture was heated in an oil bath and kept at 60 °C to 65 °C for reaction for 2 h. After the reaction was complete as monitored by LC-MS, the reaction solution was diluted with ethyl acetate, washed sequentially with water and brine, separated, and the organic phase was concentrated to obtain a crude product of compound 99-10, which was purified by silica gel column chromatography to finally obtain the target compound 99-10 (71 mg, reddish-brown mucus). ESI-MS m/z: 442, [M+2H]²⁺/2.

### Step 11: Synthesis of compound 99-11

Compound 99-10 (71 mg, 0.08 mmol), DCM (2.00 mL), and HCl (4M solution in dioxane) (1.00 mL) were added sequentially to a 10 mL single-necked flask, and the reaction was stirred at room temperature for 0.5 h. After the reaction was complete as monitored by LC-MS, the reaction solution was concentrated to dryness, DCM was added again, and the mixture was concentrated to dryness again. A crude product of compound 99-11 (slightly yellow solid powder, 62 mg, hydrochloride salt) was obtained, which was directly used in the next reaction. ESI-MS m/z: 783 [M+H]⁺.

### Step 12: Synthesis of compound 99

(1S,2S)-2-(difluoromethyl)cyclopropane-1-carboxylic acid (54.85 mg, 0.40 mmol), HATU/N,N,N',N'-tetramethyl-O-(7-azabenzotriazol-1-yl)uranium hexafluorophosphate (229.85 mg, 0.60 mmol), acetonitrile (3.00 mL), and N,N-diisopropylethylamine (0.13 mL, 0.81 mmol) were added sequentially to a 10 mL single-necked flask. After stirring at 0 °C for 15 min, a solution of compound 99-11 (63.00 mg, 0.08 mmol) in acetonitrile (3.00 mL) (DIEA was added dropwise until dissolved) was added, and resultant was stirred at 0 °C for 15 min. After the reaction was complete as monitored by LC-MS, the reaction solution was directly purified by Prep-HPLC acidic method, and the eluate was lyophilized to finally obtain the target compound 99 (11.6 mg). ESI-MS m/z: 900 [M+H]⁺.

¹H NMR (500 MHz, MeOD)δ 8.91 (d, J = 8.5 Hz, 1H), 8.58 (s, 1H), 7.91 (d, J = 2.6 Hz, 1H), 7.71 (d, J = 8.6 Hz, 1H), 7.57 (s, 1H), 7.47 (d, J = 8.6 Hz, 1H), 7.15 (d, J = 2.5 Hz, 1H), 5.93 (d, J = 3.9 Hz, 0.2H), 5.81 (d, J = 4.0 Hz, 0.5H), 5.71-5.68 (m, 1.3H), 4.48 - 4.25 (m, 4H), 4.21-4.18 (m, 2H), 3.95-3.93 (m, 2H), 3.79-3.76 (m, 2H), 3.70-3.64 (m, 2H), 3.60 - 3.48 (m, 4H), 3.27-3.14 (m, 4H), 2.91 (s, 3H), 2.81-2.77 (m, 1H), 2.52 (d, J = 14.6 Hz, 1H), 2.39 (s, 3H), 2.25 - 2.17 (m, 2H), 2.10 - 1.89 (m, 4H), 1.86 - 1.73 (m, 2H), 1.68 - 1.56 (m, 2H), 1.51 (d, J = 6.6 Hz, 3H), 1.33-1.28 (m, 3H), 1.19-1.11 (m, 3H), 1.03 (s, 3H), 0.44 (s, 3H).

### Example 100: Synthesis of compound (1S,2S)-2-(difluoromethyl)-N-((1⁵S,6³S,4S,Z)-1⁵,10,10-trimethyl-1²-(7-methyl-2,7-diazaspiro[3. 5]nonan-2-yl)-5,7-dioxo-1⁸,1⁹,6¹,6²,6³,6⁴,6⁵,6⁶-octahydro-1⁵H,1⁷H-8-oxa-1(13,15)-pyrido[3',2':7, 8][1,5]oxazonino[5,6-a]indola-2(4,2)-thiazola-6(1,3)-pyridazinacycloundecaphane-4-yl)cyclopro pane-1-carboxamide

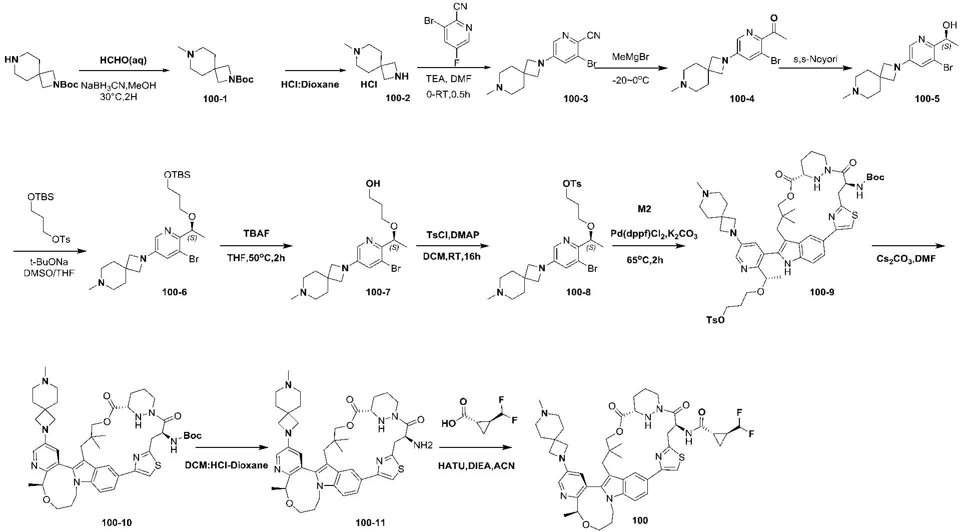

### Step 1: Synthesis of compound 100-1

Tert-butyl 2,7-diazaspiro[3.5]nonane-2-carboxylate (5 g, 22.09 mmol), 37% aqueous formaldehyde solution (17.91 g, 220.93 mmol), and methanol (50.00 mL) were added sequentially to a 100 mL single-necked flask. Sodium cyanoborohydride (2.78 g, 44.19 mmol) was added in batches, and the mixture was stirred at room temperature for 2 h. After the reaction was complete as monitored by LC-MS, the reaction solution was diluted with ethyl acetate, washed sequentially with water and brine, separated, and the organic phase was concentrated to obtain a crude product, which was purified by silica gel column chromatography to finally obtain the target compound 100-1 (5.31 g, off-white solid powder). ESI-MS m/z: 241 [M+H]⁺.

### Step 2: Synthesis of compound 100-2

Compound 100-1 (5.31 g, 22.09 mmol), dichloromethane (20.00 mL), and hydrochloric acid (4M solution in dioxane) (40.00 mL) were added sequentially to a 100 mL single-necked flask, and the reaction was carried out at 25 °C for 2 h. After the reaction was complete as monitored by LC-MS, the reaction solution was concentrated to dryness, and evaporated twice with dichloromethane to obtain a crude product of the target compound 100-2 (3.1 g, off-white solid). It was directly used in the next step without purification. ESI-MS m/z: 141 [M+H]⁺.

### Step 3: Synthesis of compound 100-3

3-Bromo-5-fluoro-pyridine-2-carbonitrile (2 g, 9.95 mmol) and DMF (20.00 mL) were added sequentially to a 100 mL single-necked flask. At 0 °C, compound 100-2 (1.75 g, 9.95 mmol) and triethylamine (4.15 mL, 29.85 mmol) were added dropwise. After the addition was complete, the mixture was transferred to room temperature and stirred for 0.5 h. After the reaction was complete as monitored by LC-MS, the mixture was extracted 3 times with water and ethyl acetate. The organic phases were combined, washed sequentially with water and brine, separated, and concentrated to obtain a crude product, which was purified by silica gel column chromatography to finally obtain the target product 100-3 (1.34 g, off-white solid). ESI-MS m/z: 322, 324 [M+H]⁺.

### Step 4: Synthesis of compound 100-4

Compound 100-3 (1.34 g, 4.17 mmol) and THF (15 mL) were added sequentially to a 50 mL three-necked flask. Nitrogen was purged three times, the temperature was lowered to -20 °C, methylmagnesium bromide (3.89 mL, 3.00 mol/L, 11.66 mmol) was added dropwise. After the addition was complete, the mixture was transferred to 0 °C and stirring was continued for reaction for 2 h. After the reaction was complete as monitored by LC-MS, 1M dilute hydrochloric acid was added dropwise to adjust the pH to about 3 to 4; the mixture was stirred at room temperature for 15 min, basified with saturated aqueous sodium bicarbonate solution, extracted with ethyl acetate, washed with brine, separated, and the organic phase was concentrated to obtain a crude product, which was purified by silica gel column chromatography to finally obtain the target compound 100-4 (1.00 g, reddish-brown mucus). ESI-MS m/z: 339, 341 [M+H]⁺.

### Step 5: Synthesis of compound 100-5

Triethylamine (4.93 mL, 35.48 mmol) was added sequentially to a 500 mL single-necked flask. At 0 °C, formic acid (0.28 mL, 7.39 mmol) was added, then (S,S)-N-(p-toluenesulfonyl)-1,2-diphenylethane-1,2-diamine (p-cymene)ruthenium(II) chloride (0.02 g, 0.03 mmol) was added, and the mixture was stirred at 40 °C for 15 min. After cooling to room temperature, compound 100-4 (1.00 g, 2.96 mmol) was added, and the reaction was carried out at room temperature (oil temperature 28 °C) for 20 h. After the reaction was complete as monitored by LC-MS, the reaction mixture was added to an aqueous solution, extracted with ethyl acetate, washed with brine, separated, and the organic phase was concentrated to obtain a crude product, which was purified by silica gel column chromatography to finally obtain the target compound 100-5 (0.47 g, reddish-brown mucus). ESI-MS m/z: 341, 343 [M+H]⁺.

### Step 6: Synthesis of compound 100-6

Compound 100-5 (420.00 mg, 1.23 mmol) and 3-((tert-butyldimethylsilyl)oxy)propyl-4-methylbenzenesulfonate (850.61 mg, 2.47 mmol) were added sequentially to a 50 mL single-necked flask, dissolved in dimethyl sulfoxide (5.00 mL). Under ice-water cooling, a solution of sodium tert-butoxide (296.56 mg, 3.09 mmol) in tetrahydrofuran (1.00 mL) was added, and the mixture was transferred to room temperature and stirred for 0.5 h. After the reaction was complete as monitored by LC-MS, the reaction mixture was added to an aqueous solution, extracted with dichloromethane, washed with brine, separated, and the organic phase was concentrated to obtain a crude product, which was purified by silica gel column chromatography to finally obtain the target compound 100-6 (330 mg, reddish-brown mucus). ESI-MS m/z: 512, 514 [M+H]⁺.

### Step 7: Synthesis of compound 100-7

Compound 100-6 (330 mg, 0.97 mmol), tetrahydrofuran (5.00 mL), and TBAF (0.97 mL, 1M solution in THF) were added to a 50 mL single-necked flask, and the mixture was stirred at 50 °C for 1 h. After the reaction was complete as monitored by LC-MS, silica gel was added for sample preparation, followed by purification by silica gel column chromatography to finally obtain the target compound 100-7 (280 mg, reddish-brown mucus). ESI-MS m/z: 399, 401 [M+H]⁺.

### Step 8: Synthesis of compound 100-8

Compound 100-7 (280 mg, 0.7 mmol), 4-dimethylaminopyridine (8.59 mg, 0.07 mmol), dichloromethane (3.00 mL), and triethylamine (0.24 mL, 1.76 mmol) were added sequentially to a 50 mL single-necked flask. At 0 °C under nitrogen protection, p-toluenesulfonyl chloride (268.01 mg, 1.41 mmol) was added. After the addition was complete, the mixture was transferred to room temperature and stirred overnight. After the reaction was complete as monitored by LC-MS, the reaction mixture was added to an aqueous solution, extracted with dichloromethane, washed with brine, separated, and the organic phase was concentrated to obtain a crude product, which was purified by silica gel column chromatography to finally obtain the target compound 100-8 (320 mg, reddish-brown mucus). ESI-MS m/z: 552, 554 [M+H]⁺.

### Step 9: Synthesis of compound 100-9

Compound 100-8 (100 mg, 0.18 mmol), M2 (125.55 mg, 0.18 mmol), K₂CO₃/potassium carbonate (62.54 mg, 0.45 mmol), [1,1'-bis(diphenylphosphino)ferrocene]dichloropalladium(II) dichloromethane complex (14.78 mg, 0.02 mmol), toluene (3.00 mL), 1,4-dioxane (1.00 mL), and water (1.00 mL) were added sequentially to a 10 mL single-necked flask. Nitrogen was purged three times, and the mixture was stirred at 60 °C to 65 °C for 1.5 h. After the reaction was complete as monitored by LC-MS, the reaction solution was diluted with ethyl acetate, washed sequentially with water and brine, separated, the organic phases were combined, and concentrated to obtain a crude product, which was purified by silica gel column chromatography to finally obtain the target compound 100-9 (113 mg, reddish-brown mucus). ESI-MS m/z: 520, [M+2H]²⁺/2.

### Step 10: Synthesis of compound 100-10

Compound 100-9 (113 mg, 0.11 mmol), DMF (3 mL), and Cs₂CO₃ (70.83 mg, 0.22 mmol) were added sequentially to a 10 mL single-necked flask. Under nitrogen protection, the mixture was heated in an oil bath and kept at 60 °C to 65 °C for reaction for 2 h. After the reaction was complete as monitored by LC-MS, the reaction solution was diluted with ethyl acetate, washed sequentially with water and brine, separated, and the organic phase was concentrated to obtain a crude product of compound 100-10, which was purified by silica gel column chromatography to finally obtain the target compound 100-10 (94 mg, reddish-brown mucus). ESI-MS m/z: 434 1/2[M+2]⁺.

### Step 11: Synthesis of compound 100-11

Compound 100-10 (94 mg, 0.08 mmol), DCM (2.00 mL), and HCl (4M solution in dioxane) (1.00 mL) were added sequentially to a 10 mL single-necked flask, and the reaction was stirred at room temperature for 0.5 h. After the reaction was complete as monitored by LC-MS, the reaction solution was concentrated to dryness, DCM was added again, and the mixture was concentrated to dryness again. A crude product of compound 100-11 (slightly yellow solid powder, 83 mg, hydrochloride salt) was obtained, which was directly used in the next reaction. ESI-MS m/z: 768 [M+H]⁺.

### Step 12: Synthesis of compound 100

(1S,2S)-2-(difluoromethyl)cyclopropane-1-carboxylic acid (84 mg, 0.55 mmol), N,N,N',N'-tetramethyl-O-(7-azabenzotriazol-1-yl)uranium hexafluorophosphate (312.28 mg, 0.82 mmol), acetonitrile (3.00 mL), and N,N-diisopropylethylamine (0.18 mL, 1.09 mmol) were added sequentially to a 10 mL single-necked flask. After stirring at 0 C for 15 min, a solution of compound 100-11 (84.00 mg, 0.11 mmol) in acetonitrile (3.00 mL) (DIEA was added dropwise until dissolved) was added, and the resultant was stirred at 0 °C for 15 min. After the reaction was complete as monitored by LC-MS, the reaction solution was directly purified by Prep-HPLC acidic method, and the eluate was lyophilized to finally obtain the target compound 100 (7.6 mg). ESI-MS m/z: 885 [M+H]⁺.

### Example 101: Synthesis of compound (1S,2S)-2-(difluoromethyl)-N-((1⁵S,6³S,4S,Z)-1²-(4-(2-methoxyethyl)piperazin-1-yl)-1⁵,10,10-tr imethyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5 ]oxazonino[5,6-a]indola-2(4,2)-thiazola-6(1,3)-pyridazinacycloundecaphane-4-yl)cyclopropane-1-carboxamide

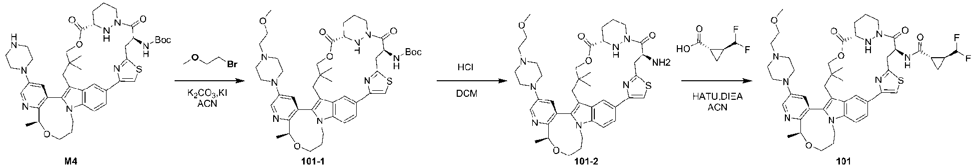

### Step 1: Synthesis of compound 101-1

Compound M4 (100.00 mg, 0.12 mmol), compound 2-bromoethyl methyl ether (20.52 mg, 0.15 mmol), potassium carbonate (17.00 mg, 0.12 mmol), and potassium iodide (20.42 mg, 0.12 mmol) were added to acetonitrile (2.00 mL), and the reaction was carried out at 70 °C for 8 h. 50 mL of water was added to the reaction solution, extracted with 40 mL*2 of DCM, washed with 40 mL*2 of saturated brine, dried over anhydrous sodium sulfate, and separated by column chromatography to obtain a crude product of compound 101-1 (107.00 mg, 99.87%). ESI-MS m/z: 871.71 [M+H]⁺.

### Step 2: Synthesis of compound 101-2

Compound 101-1 (107.00 mg, 0.12 mmol) was dissolved in DCM (1.00 mL), then hydrochloric acid (4M solution in dioxane) (2.00 mL) was added, and the reaction was carried out at room temperature for 1 h. The reaction solution was concentrated to dryness, 5 mL of toluene was added for dilution, and concentrated to dryness again to obtain a crude product of compound 101-2 (90 mg, 95.04%), which was directly used in the next reaction. ESI-MS m/z: 771.49, [M+H]⁺.

### Step 3: Synthesis of compound 101

Compound (1S,2S)-2-(difluoromethyl)cyclopropane-1-carboxylic acid (25.41 mg, 0.19 mmol) was dissolved in acetonitrile (5.00 mL). DIEA (0.26 mL, 1.56 mmol) and HATU (88.75 mg, 0.23 mmol) were added sequentially in an ice-water bath. After reacting in the ice-water bath for 10 min, a solution of compound 101-2 (90.00 mg, 0.12 mmol) in acetonitrile (5.00 mL) and DIEA (0.26 mL, 1.56 mmol) was added, and the reaction was carried out at room temperature for 1 h. The mixture was concentrated to dryness and purified by preparative separation to obtain compound 101 (73.00 mg, 70.33%). ESI-MS m/z: 889.44, [M+H]⁺.

¹H NMR (500 MHz, DMSO)δ 8.90 (d, J = 9.0 Hz, 1H), 8.50 (s, 1H), 8.40 (d, J = 2.8 Hz, 1H), 7.81 (s, 1H), 7.71 (dd, J = 8.6, 1.3 Hz, 1H), 7.49 (d, J = 8.6 Hz, 1H), 7.21 (d, J = 2.8 Hz, 1H), 5.95 (td, J = 56.7, 5.1 Hz, 1H), 5.76 (s, 1H), 5.56 (t, J = 9.4 Hz, 1H), 5.12 (d, J = 12.2 Hz, 1H), 4.29 - 4.19 (m, 2H), 4.15 (q, J = 6.3 Hz, 1H), 4.03 (dd, J = 12.4, 4.5 Hz, 2H), 3.68 (d, J = 11.0 Hz, 1H), 3.61 (dd, J = 21.5, 7.5 Hz, 2H), 3.46 (t, J = 5.7 Hz, 2H), 3.38 (d, J = 14.4 Hz, 1H), 3.24 (s, 7H), 3.19 - 3.11 (m, 2H), 3.01 (d, J = 14.4 Hz, 1H), 2.76 (dt, J = 12.6, 8.2 Hz, 1H), 2.58 (d, J = 13.3 Hz, 4H), 2.52 (d, J = 5.6 Hz, 1H), 2.42 (d, J = 14.3 Hz, 1H), 2.29 (t, J = 10.2 Hz, 1H), 2.10 (dt, J = 9.2, 5.1 Hz, 2H), 1.81 (s, 3H), 1.66 (td, J = 9.7, 5.1 Hz, 1H), 1.58 - 1.48 (m, 1H), 1.37 (d, J = 6.3 Hz, 3H), 1.09 - 0.99 (m, 2H), 0.95 (s, 3H), 0.32 (s, 3H).

### Example 102: Synthesis of compound (1S,2S)-2-(difluoromethyl)-N-((1⁵S,6³S,4S,Z)-1⁵,10,10-trimethyl-1²-(4-(4-methylpiperazin-1-yl) piperidin-1-yl)-5,7-dioxo-1⁸,1⁹,6¹,6²,6³,6⁴,6⁵,6⁶-octahydro-1⁵H,1⁷H-8-oxa-1(13,15)-pyrido[3',2':7 ,8][1,5]oxazonino[5,6-a]indola-2(4,2)-thiazola-6(1,3)-pyridazinacycloundecaphane-4-yl)cyclopr opane-1-carboxamide

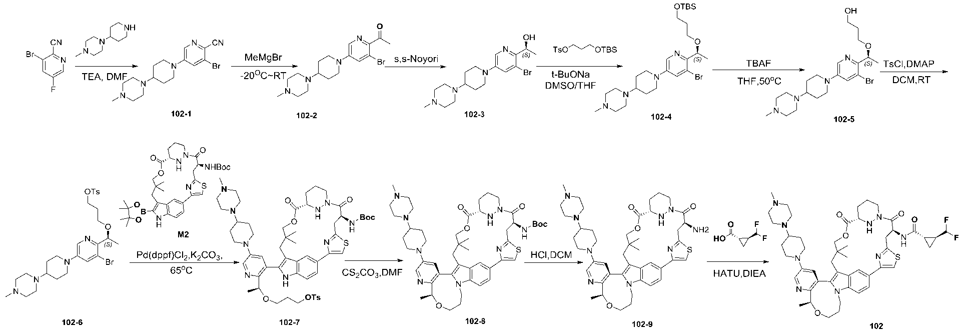

### Step 1: Synthesis of compound 102-1

3-Bromo-5-fluoro-pyridine-2-carbonitrile (2.00 g, 9.95 mmol), N,N-dimethylformamide (20.00 mL), and 1-methyl-4-(piperidin-4-yl)piperazine (2.19 g, 11.94 mmol) were added sequentially to a 50 mL single-necked flask. Triethylamine (3.46 mL, 24.88 mmol) was added dropwise at 0 °C, and the mixture was stirred at room temperature for 1 h. The reaction was complete as monitored by LC-MS, the reaction solution was poured into water, a solid precipitated, filtered, and the filter cake was washed with water. The filter cake was dried to obtain the target compound 102-1 (3.19 g). ESI-MS m/z: 364.1 [M+H]⁺.

### Step 2: Synthesis of compound 102-2

Compound 102-1 (3.19 g, 8.76 mmol) and tetrahydrofuran (30.00 mL) were added sequentially to a 100 mL three-necked flask. Under nitrogen protection, the temperature was lowered to -20 °C, and then methylmagnesium bromide (8.76 mL, 3.00 mol/L, 26.27 mmol) was added dropwise. The mixture was stirred at room temperature for 1 h. The reaction was complete as monitored by LC-MS, quenched with saturated NH₄Cl solution, adjusted to pH = 4 with dilute hydrochloric acid, and stirred for 10 min. The mixture was then adjusted to pH = 8 with saturated NaHCO₃ solution, extracted twice with ethyl acetate, and the organic layer was washed with saturated brine and dried over anhydrous sodium sulfate. The mixture was concentrated under reduced pressure and purified by column chromatography to obtain the target compound 102-2 (2.06 g). ESI-MS m/z: 381.1 [M+H]⁺.

### Step 3: Synthesis of compound 102-3

Triethylamine (15.02 mL, 108.05 mmol) was added to a 100 mL three-necked flask. Under nitrogen protection, the temperature was lowered to 0 °C, formic acid (1.02 mL, 27.01 mmol) was added dropwise, and then (S,S)-N-(p-toluenesulfonyl)-1,2-diphenylethane-1,2-diamine (p-cymene)ruthenium(II) chloride (0.07 g, 0.11 mmol) was added. The reaction was heated to 40 °C and stirred for 15 min. After cooling to room temperature, compound 102-2 (2.06 g, 5.40 mmol) and dichloromethane (5.00 mL) were finally added, and the mixture was stirred at room temperature for 24 h. The reaction was complete as monitored by LC-MS, extracted twice with dichloromethane, and the organic layer was washed with saturated brine and dried over anhydrous sodium sulfate. The mixture was concentrated under reduced pressure and purified by column chromatography to obtain the target compound 102-3 (1.53 g). ESI-MS m/z: 383.1 [M+H]⁺.

### Step 4: Synthesis of compound 102-4

Compound 102-3 (800.00 mg, 2.09 mmol), dimethyl sulfoxide (10.00 mL), tetrahydrofuran (2.00 mL), and 3-((tert-butyldimethylsilyl)oxy)propyl-4-methylbenzenesulfonate (2.52 g, 7.30 mmol) were added sequentially to a 50 mL single-necked flask. Sodium tert-butoxide (501.40 mg, 5.22 mmol) was added in batches at 10 °C, and the mixture was stirred at room temperature for 1 h. The reaction was complete as monitored by LC-MS, and the reaction mixture was added to a saturated aqueous ammonium chloride solution for quenching. The mixture was extracted twice with ethyl acetate, washed three times with saturated brine, concentrated under reduced pressure, and purified by column chromatography to obtain the target compound 102-4 (972.00 mg). ESI-MS m/z: 555.3 [M+H]⁺.

### Step 5: Synthesis of compound 102-5

Compound 102-4 (970.00 mg, 1.75 mmol) and tetrahydrofuran (10.00 mL) were added sequentially to a 50 mL single-necked flask. Tetrabutylammonium fluoride (2.27 mL, 1.00 mol/L, 2.27 mmol) was added dropwise at 0 °C, and the reaction was heated to 50 °C and stirred for 1 h. The reaction was complete as monitored by LC-MS, concentrated under reduced pressure, and purified by column chromatography to obtain the target compound 102-5 (723.00 mg). ESI-MS m/z: 441.2 [M+H]⁺.

### Step 6: Synthesis of compound 102-6

Compound 102-5 (723.00 mg, 1.64 mmol), dichloromethane (10.00 mL), and 4-dimethylaminopyridine (20.04 mg, 0.16 mmol) were added sequentially to a 50 mL single-necked flask. Then p-toluenesulfonyl chloride (624.52 mg, 3.28 mmol) and triethylamine (0.57 mL, 4.09 mmol) were added in batches, and the mixture was stirred at room temperature for 4 h. The reaction was complete as monitored by LC-MS, concentrated under reduced pressure, and purified by column chromatography to obtain the target compound 102-6 (1.14 g). ESI-MS m/z: 595.2 [M+H]⁺.

### Step 7: Synthesis of compound 102-7

Compound 102-6 (154.02 mg, 0.26 mmol), M2 (120.00 mg, 0.17 mmol), toluene (1.50 mL), 1,4-dioxane (0.50 mL), water (0.50 mL), potassium carbonate (59.57 mg, 0.43 mmol), and [1,1'-bis(diphenylphosphino)ferrocene]dichloropalladium(II) dichloromethane complex (14.05 mg, 0.02 mmol) were added sequentially to a 10 mL single-necked flask. Nitrogen was purged three times, and under nitrogen protection, the reaction was heated to 65 °C and stirred for 3 h. The reaction was complete as monitored by LC-MS, extracted twice with ethyl acetate, washed with saturated brine, concentrated under reduced pressure, and purified by column chromatography to obtain the target compound 102-7 (67.00 mg). ESI-MS m/z: 541.8 1/2[M+2H]⁺.

### Step 8: Synthesis of compound 102-8

Compound 102-7 (61.00 mg, 0.06 mmol), N,N-dimethylformamide (5.00 mL), and cesium carbonate (55.13 mg, 0.17 mmol) were added sequentially to a 10 mL single-necked flask, and the reaction was heated to 45 °C and stirred for 1 h. The reaction was complete as monitored by LC-MS, the reaction solution was diluted with ethyl acetate, washed sequentially with water and brine three times, concentrated under reduced pressure, and purified by column chromatography to obtain the target compound 102-8 (64.00 mg). ESI-MS m/z: 910.5 [M+H]⁺.

### Step 9: Synthesis of compound 102-9

Compound 102-8 (64.00 mg, 0.07 mmol), dichloromethane (2.00 mL), and hydrochloric acid (4M solution in dioxane) (2.00 mL) were added sequentially to a 10 mL single-necked flask, and the mixture was stirred at room temperature for 1 h. The reaction was complete as monitored by LC-MS, and the mixture was concentrated under reduced pressure. The crude product was directly used in the next step to obtain the crude product compound 102-9 (57.00 mg). ESI-MS m/z: 810.4 [M+H]⁺.

### Step 10: Synthesis of compound 102

(1S,2S)-2-(difluoromethyl)cyclopropane-1-carboxylic acid (14.37 mg, 0.11 mmol) and N,N-dimethylformamide (1.00 mL) were added sequentially to a 10 mL single-necked flask. At 0 °C, N,N,N',N'-tetramethyl-O-(7-azabenzotriazol-1-yl)uranium hexafluorophosphate (40.15 mg, 0.11 mmol) and N,N-diisopropylethylamine (0.12 mL, 0.70 mmol) were added, and the mixture was stirred at 0 °C for 10 min. Finally, compound 102-9 (57.00 mg, 0.07 mmol) was added, and the mixture was stirred at 0 °C for 10 min. The reaction was complete as monitored by LC-MS, and the reaction solution was directly purified by pre-HPLC to obtain the target compound 102 (9.30 mg). ESI-MS m/z: 928.5 [M+H]⁺.

¹H NMR (500 MHz, Methanol-d₄)δ 8.93 (d, J = 8.5 Hz, 1H), 8.59 (d, J = 1.6 Hz, 1H), 8.35 (d, J = 2.9 Hz, 1H), 7.74 - 7.70 (m, 2H), 7.58 (s, 1H), 7.48 (d, J = 8.6 Hz, 1H), 5.87 (dd, J = 57.3, 4.1 Hz, 1H), 5.69 (td, J = 8.1, 7.5, 2.5 Hz, 1H), 4.41 (dd, J = 21.2, 10.2 Hz, 2H), 4.30 (dd, J = 12.1, 3.2 Hz, 1H), 4.19 - 4.02 (m, 5H), 3.80 - 3.64 (m, 4H), 3.46 (d, J = 15.1 Hz, 2H), 3.39 - 3.34 (m, 3H), 3.28 - 3.23 (m, 3H), 3.01 (d, J = 12.0 Hz, 3H), 2.88 (s, 3H), 2.82 - 2.76 (m, 1H), 2.50 (d, J = 14.5 Hz, 1H), 2.39 (d, J = 14.7 Hz, 1H), 2.24 (d, J = 12.7 Hz, 1H), 2.15 - 2.04 (m, 4H), 1.97 (d, J = 12.7 Hz, 2H), 1.84 (d, J = 13.5 Hz, 1H), 1.81 - 1.73 (m, 3H), 1.63 (tt, J = 13.1, 6.4 Hz, 2H), 1.53 (d, J = 6.6 Hz, 3H), 1.18 (dd, J = 9.3, 4.6 Hz, 1H), 1.03 (s, 3H), 0.44 (s, 3H).

### Example 103: Synthesis of compound (1S,2S)-2-(difluoromethyl)-N-((1⁵S,6³S,4S,Z)-1⁵,10,10-trimethyl-1²-(9-methyl-3,9-diazaspiro[5. 5]undecan-3-yl)-5,7-dioxo-1⁸,1⁹,6¹,6²,6³,6⁴,6⁵,6⁶-octahydro-1⁵H,1⁷H-8-oxa-1(13,15)-pyrido[3',2': 7,8][1,5]oxazonino[5,6-a]indola-2(4,2)-thiazola-6(1,3)-pyridazinacycloundecaphane-4-yl)cyclop ropane-1-carboxamide

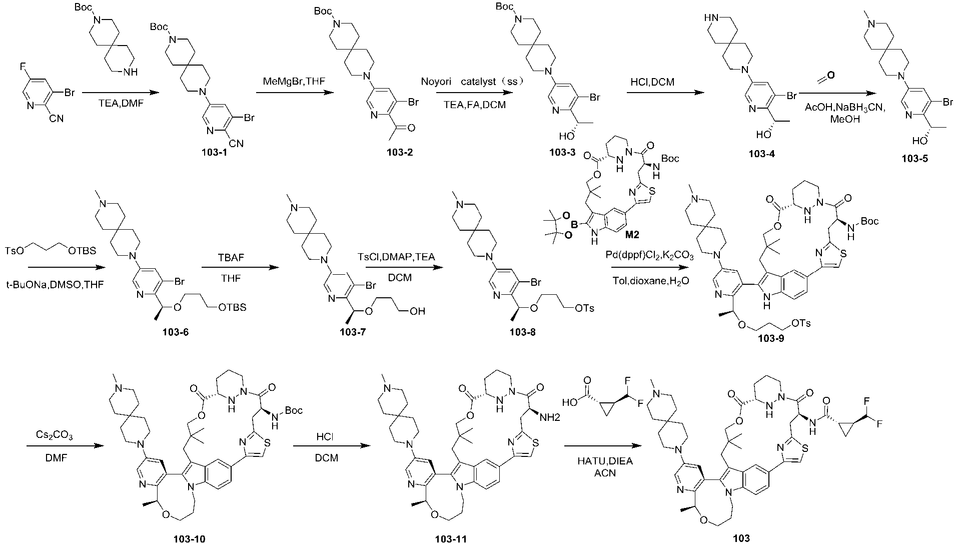

### Step 1: Synthesis of compound 103-1

Under ice-water bath conditions, 3-bromo-5-fluoro-pyridine-2-carbonitrile (3.00 g, 14.93 mmol), triethylamine (6.22 mL, 44.78 mmol), and compound tert-butyl 3,9-diazaspiro[5.5]undecane-3-carboxylate (4.18 g, 16.42 mmol) were added sequentially to DMF (30.00 mL), and the reaction was carried out at room temperature for 1 h. 50 mL of water was added to the reaction solution, the product precipitated, and filtered. The filter cake was washed with 30 mL of water, and dried to obtain a crude product of compound 103-1 (white solid powder, 6.10 g), which was directly used in the next reaction. ESI-MS m/z: 435.11 [M+H]⁺.

### Step 2: Synthesis of compound 103-2

Compound 103-1 (6.00 g, 13.78 mmol) was dissolved in tetrahydrofuran (60.00 mL), the atmosphere was purged with nitrogen, methylmagnesium bromide (13.78 mL, 3.00 mol/L, 41.35 mmol) was added dropwise at -20 °C, and the mixture was heated to room temperature and reacted for 2 h. The reaction solution was added dropwise to 40 mL of ice-cold saturated aqueous ammonium chloride solution, adjusted to pH = 3 with 1N aqueous hydrochloric acid solution, and stirred for 10 min. Then resultant was adjusted to pH = 9 with saturated aqueous sodium bicarbonate solution, extracted with 40 mL*2 of EA, washed with 40 mL*2 of saturated brine, dried over anhydrous sodium sulfate, and separated by column chromatography to obtain compound 103-2 (2.10 g, 33.68%). ESI-MS m/z: 452.18, [M+H]⁺.

### Step 3: Synthesis of compound 103-3

Compound 103-2 (2.10 g, 4.64 mmol), (S,S)-N-(p-toluenesulfonyl)-1,2-diphenylethane-1,2-diamine (p-cymene)ruthenium(II) chloride (0.15 g, 0.23 mmol), and TEA (6.45 mL, 46.42 mmol) were added to DCM (20.00 mL). Formic acid (1.07 g, 23.21 mmol) was added in an ice-water bath, and the reaction was carried out at room temperature for 72 h. 50 mL of water was added to the reaction solution, extracted with 40 mL*2 of DCM, washed with 40 mL*2 of saturated brine, dried over anhydrous sodium sulfate, and separated by column chromatography to obtain compound 103-3 (1.12 g, 53.10%). ESI-MS m/z: 454.18 [M+H]⁺.

### Step 4: Synthesis of compound 103-4

Compound 103-3 (1.12 g, 2.46 mmol) was dissolved in DCM (2.00 mL), then hydrochloric acid (4M solution in dioxane) (4.00 mL) was added, and the reaction was carried out at room temperature for 1 h. The reaction solution was concentrated to dryness, diluted with 40 mL of toluene, and concentrated to dryness again to obtain a crude product of compound 103-4 (0.90 g, reddish-brown solid powder). ESI-MS m/z: 354.13, [M+H]⁺.

### Step 5: Synthesis of compound 103-5

Compound 103-4 (0.90 g, 2.54 mmol) was added to methanol (10.00 mL). In an ice-water bath, acetic acid (0.29 mL, 5.08 mmol) and 37% aqueous formaldehyde solution (0.41 g, 37%, 5.08 mmol) were added, and the mixture was kept for reaction for 10 min. Then sodium cyanoborohydride (0.24 g, 3.81 mmol) was added, and the reaction was carried out at room temperature for 1 h. The reaction solution was concentrated to dryness, adjusted to basic with 7M ammonia in methanol solution, concentrated to dryness, and separated by column chromatography to obtain compound 103-5 (1.10 g, 117.57%). ESI-MS m/z: 368.16, [M+H]⁺.

### Step 6: Synthesis of compound 103-6

Compound 103-5 (1.10 g, 2.99 mmol) and compound 3-((tert-butyldimethylsilyl)oxy)propyl-4-methylbenzenesulfonate (3.09 g, 8.96 mmol) were added to DMSO (10.00 mL), and then a solution of sodium tert-butoxide (0.72 g, 7.47 mmol) in tetrahydrofuran (2.00 mL) was added, and the reaction was carried out at 40 °C for 3 h. 50 mL of water was added to the reaction solution, extracted with 40 mL*2 of EA, washed with 40 mL*2 of saturated brine, dried over anhydrous sodium sulfate, and separated by column chromatography to obtain compound 103-6 (0.70 g, 43.48%). ESI-MS m/z: 540.37, [M+H]⁺.

### Step 7: Synthesis of compound 103-7

Compound 103-6 (700.00 mg, 1.29 mmol) and tetrabutylammonium fluoride (1.94 mL, 1.00 mol/L, 1.94 mmol) were added to THF/tetrahydrofuran (7.00 mL), and the reaction was carried out at 50 °C for 1 h. The mixture was concentrated to dryness and separated by column chromatography to obtain compound 103-7 (0.52 g, 94.19%). ESI-MS m/z: 426.27, [M+H]⁺.

### Step 8: Synthesis of compound 103-8

Compound 103-7 (520.00 mg, 1.22 mmol), DMAP (297.97 mg, 2.44 mmol), and TEA (0.85 mL, 6.10 mmol) were added to DCM (8.00 mL). p-Toluenesulfonyl chloride (813.74 mg, 4.27 mmol) was added in batches in an ice-water bath, and the reaction was carried out at room temperature for 2 h. 50 mL of water was added to the reaction solution, extracted with 40 mL*2 of DCM, washed with 40 mL*2 of saturated brine, dried over anhydrous sodium sulfate, and separated by column chromatography to obtain compound 103-8 (515 mg, 72.74%). ESI-MS m/z: 580.35, [M+H]⁺.

### Step 9: Synthesis of compound 103-9

Compound 103-8 (167.44 mg, 0.29 mmol), compound M2 (100.00 mg, 0.14 mmol), [1,1'-bis(diphenylphosphino)ferrocene]dichloropalladium(II) dichloromethane complex (11.76 mg, 0.01 mmol), potassium carbonate (49.82 mg, 0.36 mmol), water (0.50 mL), and 1,4-dioxane (1.00 mL) were added to toluene (3.00 mL), and the reaction was carried out at 65 °C for 2 h under a nitrogen atmosphere. The mixture was concentrated and separated by column chromatography to obtain compound 103-9 (140 mg, 90.96%). ESI-MS m/z: 534.54, [M+H]⁺.

### Step 10: Synthesis of compound 103-10

Compound 103-9 (140.00 mg, 0.13 mmol) and cesium carbonate (85.50 mg, 0.26 mmol) were added to DMF (14.00 mL), and the reaction was carried out at 85 °C for 1 h. 50 mL of water was added to the reaction solution, extracted with 40 mL*2 of EA, washed with 40 mL*2 of saturated brine, dried over anhydrous sodium sulfate, and separated by column chromatography to obtain compound 103-10 (80 mg, 68.12%). ESI-MS m/z: 895.71, [M+H]⁺.

### Step 11: Synthesis of compound 103-11

Compound 103-10 (80.00 mg, 0.09 mmol) was dissolved in DCM (1.00 mL), then hydrochloric acid (4M solution in dioxane) (2.00 mL) was added, and the reaction was carried out at room temperature for 1 h. The reaction solution was concentrated to dryness, 5 mL of toluene was added for dilution, and concentrated to dryness again to obtain a crude product of compound 103-11 (50 mg, 70.35%), which was directly used in the next reaction. ESI-MS m/z: 795.71, [M+H]⁺.

### Step 12: Synthesis of compound 103

Compound (1S,2S)-2-(difluoromethyl)cyclopropane-1-carboxylic acid (10.28 mg, 0.08 mmol) was dissolved in acetonitrile (3.00 mL). DIEA (0.10 mL, 0.63 mmol) and HATU (35.89 mg, 0.09 mmol) were added sequentially in an ice-water bath. After reacting in the ice-water bath for 10 min, a solution of compound 103-11 (50.00 mg, 0.06 mmol) in acetonitrile (3.00 mL) and DIEA (0.10 mL, 0.63 mmol) was added, and the reaction was carried out at room temperature for 1 h. The mixture was concentrated to dryness and purified by preparative separation to obtain compound 103 (10 mg, 16.02%). ESI-MS m/z: 913.52, [M+H]⁺.

### Example 104: Synthesis of compound (1S,2S)-2-(difluoromethyl)-N-((1⁵S,6³S,4S,Z)-1⁵,10,10-trimethyl-1²-(4-(1-methylpiperidin-4-yl) piperazin-1-yl)-5,7-dioxo-1⁸,1⁹,6¹,6²,6³,6⁴,6⁵,6⁶-octahydro-1⁵H,1⁷H-8-oxa-1(13,15)-pyrido[3',2': 7,8][1,5]oxazonino[5,6-a]indola-2(4,2)-thiazola-6(1,3)-pyridazinacycloundecaphane-4-yl)cyclop ropane-1-carboxamide

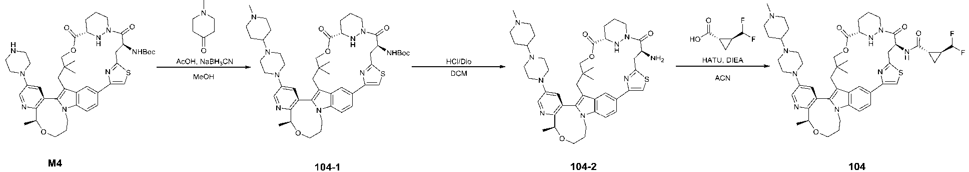

### Step 1: Synthesis of compound 104-1

Compound M4 (200.0 mg, 0.12 mmol), acetic acid (29.5 mg, 0.50 mmol), and methanol (4.00 mL) were added sequentially to a 50 mL single-necked flask. Under nitrogen protection, the mixture was cooled in an ice-water bath, N-methyl-4-piperidone (55.7 mg, 0.50 mmol) was slowly added, and the reaction was kept for 15 min. Then sodium cyanoborohydride (34.8 mg, 0.36 mmol) was added, the temperature was raised to 50 °C, and the reaction was stirred with heating for 3 h. After the reaction was complete as monitored by LC-MS, saturated sodium bicarbonate was slowly added to the reaction solution to adjust the pH to 8 to 9, concentrated to remove methanol. Then the resultant was extracted with EA (20 mL*3), the organic phases were combined, washed with saturated sodium chloride, dried over anhydrous sodium sulfate, and the organic phase was concentrated to dryness to obtain a crude product of compound 104-1, which was purified by silica gel column chromatography to finally obtain the target product 104-1 (140.0 mg, light yellow solid). ESI-MS m/z: 910.21 [M+H]⁺.

### Step 2: Synthesis of compound 104-2

Compound 104-1 (140.00 mg, 0.15 mmol), DCM (3.00 mL), and HCl (4M solution in dioxane) (1.54 mL) were added sequentially to a 10 mL single-necked flask, and the reaction was stirred at room temperature. After the reaction was complete as monitored by LC-MS, the reaction solution was concentrated to dryness, DCM was added again, and the mixture was concentrated to dryness again. A crude product of compound 104-2 (slightly yellow solid powder, 120.0 mg, hydrochloride salt) was obtained, which was directly used in the next reaction. ESI-MS m/z: 810.23, [M+H]⁺.

### Step 3: Synthesis of compound 104

(1S,2S)-2-(difluoromethyl)cyclopropane-1-carboxylic acid (24.18 mg, 0.18 mmol), HATU (112.62 mg, 0.30 mmol), and DIEA (0.12 mL, 0.74 mmol) were added sequentially to a 10 mL single-necked flask, and the mixture was stirred at room temperature for 15 min. The mixture was cooled in an ice-water bath, and then a solution of compound 104-2 (120.00 mg, 0.15 mmol) in acetonitrile was added. After the reaction was complete as monitored by LC-MS, the reaction solution was directly purified by Prep-HPLC basic method, and the eluate was lyophilized to finally obtain the target compound 104 (53.70 mg). ESI-MS m/z: 928.46, [M+H]⁺.

¹H NMR (500 MHz, DMSO)δ 8.88 (d, J = 9.1 Hz, 1H), 8.49 (s, 1H), 8.39 (s, 1H), 7.80 (s, 1H), 7.71 (d, J = 8.4 Hz, 1H), 7.49 (d, J = 8.7 Hz, 1H), 7.19 (s, 1H), 5.95 (s, 1H), 5.57 (d, J = 9.0 Hz, 1H), 5.10 (d, J = 12.2 Hz, 1H), 4.26-4.22 (m, 2H), 4.04-3.99 (m, 2H), 3.67-3.56 (m, 3H), 3.39-3.36 (m, 1H), 3.25-3.12 (m, 7H), 3.00 (d, J = 14.4 Hz, 1H), 2.78 (d, J = 11.7 Hz, 3H), 2.64-2.59 (m, 4H), 2.44-2.38 (m, 2H), 2.31 - 2.27 (m, 1H), 2.15-2.08 (m, 5H), 1.99 (s, 1H), 1.85-1.64 (m, 7H), 1.43 - 1.34 (m, 6H), 1.08 - 1.03 (m, 1H), 0.95 (s, 3H), 0.85 (s, 1H), 0.32 (s, 3H).

### Example 105: Synthesis of compound (1S,2S)-2-(difluoromethyl)-N-((1⁵S,6³S,4S,Z)-1²-(4-(dimethylamino)piperidin-1-yl)-1⁵,10,10-tri methyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5] oxazonino[5,6-a]indola-2(4,2)-thiazola-6(1,3)-pyridazinacycloundecaphane-4-yl)cyclopropane-1 -carboxamide

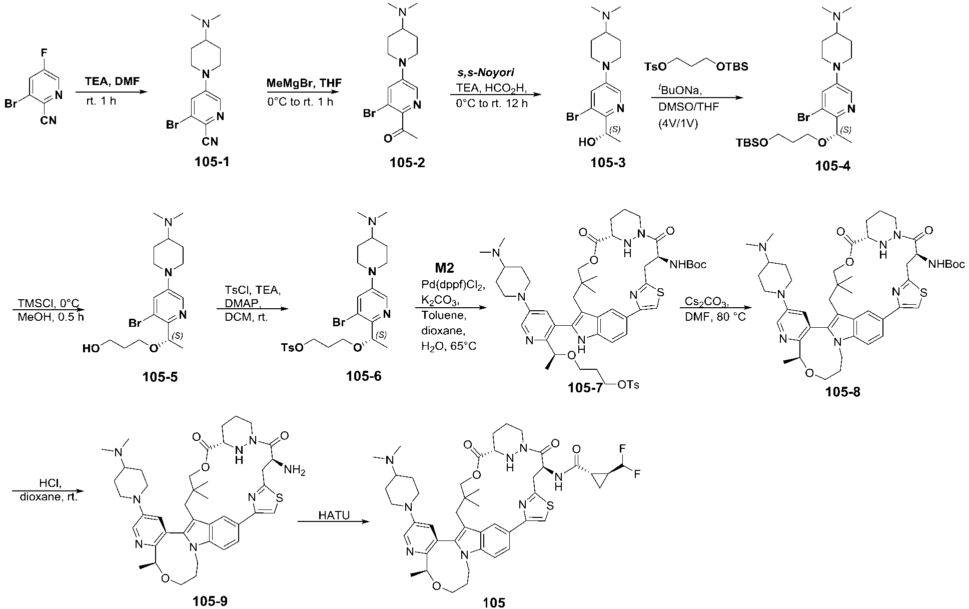

### Step 1: Synthesis of compound 105-1

Compound 3-bromo-5-fluoro-pyridine-2-carbonitrile (3.0 g) was added to a 50 mL single-necked flask, anhydrous DMF (30 mL) was added, TEA (10 mL) and 4-dimethylaminopiperidine (3.5 mL) were weighed and added respectively, and the reaction was carried out at room temperature for 0.5 h. After the reaction was complete, saturated brine (25 mL) was added, extracted with EA, and the organic phase was concentrated to dryness to obtain the target compound 105-1 (5.14 g, crude product). ESI-MS m/z: 309.2 [M+H]⁺.

### Step 2: Synthesis of compound 105-2

Compound 105-1 (4.8 g) and anhydrous THF (100 mL) were added to a 250 mL three-necked flask, the temperature was lowered to 0 °C under N₂ protection, MeMgBr (3 M in THF, 15.6 mL) was slowly added dropwise, and the mixture was heated to room temperature and reacted for 2 h. After the reaction was complete, HCl (1N, 30 mL) was added to the reaction solution, extracted with EA, the organic phase was collected, the aqueous phase was further extracted twice with EA (30 mL), the organic phases were collected and combined, dried over anhydrous sodium sulfate, and the concentrate was purified by column chromatography to obtain the target compound 105-2 (3.33 g, 65% yield). ESI-MS m/z: 326.4 [M+H]⁺.

### Step 3: Synthesis of compound 105-3

Compound 105-2 (3.33 g) was added to a 25 mL single-necked flask, TEA (17 mL) and s,s-Noyori catalyst (0.32 g) were added, the temperature was lowered to 0 °C under N₂ protection, formic acid (2 mL) was slowly added dropwise, and the reaction was carried out at room temperature for 12 h. After the reaction was complete, ethyl acetate (50 mL) and water (50 mL) were added to the reaction solution, extracted, the organic phase was collected. The aqueous phase was further extracted twice with ethyl acetate (25 mL), the organic phases were collected and combined, dried over anhydrous sodium sulfate, concentrated, and the concentrate was purified by column chromatography to obtain the target axially chiral compound 105-3 (3.0 g, 90% yield). ESI-MS m/z: 328.3 [M+H]⁺.

### Step 4: Synthesis of compound 105-4

Compound 105-3 (1.3 g) and 3-((tert-butyldimethylsilyl)oxy)propyl-4-methylbenzenesulfonate (2.23 g) and DMSO/THF (10 mL/2.5 mL) were weighed and added into the reaction flask respectively, and the temperature was lowered to 10 °C under N₂ protection; sodium tert-butoxide (0.80 g) was added, and the reaction was carried out at room temperature for 1.0 h; the reaction was complete, the temperature was lowered to 0 °C, tap water (50 mL) was added for dilution, extracted with EA, the organic phase was dried over anhydrous sodium sulfate, concentrated, and the concentrate was purified by column chromatography to obtain compound 105-4 (1.69 g, 85% yield). ESI-MS m/z: 500.1 [M+H]⁺.

### Step 5: Synthesis of compound 105-5

Compound 105-4 (1.69 g) was added to a 50 mL single-necked flask, anhydrous methanol (20 mL) was added, the temperature was lowered to 0 °C under N₂ protection, TMSCl (0.2 mL) was slowly added dropwise, and the reaction was carried out at room temperature for 1.0 h. After the reaction was complete, it was directly concentrated to dryness to obtain the target compound 105-5 (1.31 g, 100% yield). ESI-MS m/z: 385.3 [M+H]⁺.

### Step 6: Synthesis of compound 105-6

Compound 105-5 (1.31 g) was added to a 50 mL single-necked flask, anhydrous DCM (15 mL) was added, TEA (6 mL) and DMAP (21 mg) were weighed and added respectively, the temperature was lowered to 0 C under N₂ protection, TsCl (1.4 g) was added, and the reaction was carried out at room temperature for 1.0 h. After the reaction was complete, saturated brine (25 mL) was added, extracted with DCM, the organic phase was concentrated to dryness, and the concentrate was purified by column chromatography to obtain the target compound 105-6 (1.80 g, 98% yield). ESI-MS m/z: 540.5 [M+H]⁺.

### Step 7: Synthesis of compound 105-7

Compound 105-6 (468 mg), M2 (300 mg), Pd(dppf)Cl₂ (71 mg), and potassium carbonate (180 mg) were weighed and added respectively to a 100 mL single-necked flask, toluene (12 mL), 1,4-dioxane (4 mL) and 4 mL of water were added, N₂ was purged 3 times, and the mixture was heated to 65 °C and reacted for 5 h. After the reaction was complete, 20 mL of EA and 20 mL of water were added to the reaction solution, extracted, the aqueous phase was further extracted 2 times with EA. The organic phases were collected and combined, dried over anhydrous sodium sulfate, concentrated, and the concentrate was purified by column chromatography to obtain the target compound 105-7 (310 mg, 70% yield). ESI-MS m/z: 514.6 [M+H]⁺.

### Step 8: Synthesis of compound 105-8

Compound 105-7 (310 mg) was added to a 25 mL single-necked flask, Cs₂CO₃ (295 mg) and anhydrous DMF (15 mL) were weighed in, and the reaction was carried out at 80 °C for 1 h under N₂ protection. After the reaction was complete, tap water (50 mL) was added for dilution, extracted with EA, the organic phase was dried over anhydrous sodium sulfate, concentrated, and the concentrate was purified by column chromatography to obtain the target compound 105-8 (260 mg, 100% yield). ESI-MS m/z: 854.3 [M+H]⁺.

### Step 9: Synthesis of compound 105-9

Compound 105-8 (260 mg) was added to a 25 mL single-necked flask, 15 mL of hydrochloric acid (4 M solution in dioxane) was added, and the reaction was carried out at room temperature for 1 h. After the reaction was complete, it was concentrated under reduced pressure to dryness to obtain the target compound 105-9 hydrochloride (276 mg, crude product). ESI-MS m/z: 754.2 [M+H]⁺.

### Step 10: Synthesis of compound 105

Compound (1S,2S)-2-(difluoromethyl)cyclopropane-1-carboxylic acid (108 mg) was added to a 25 mL single-necked flask, HATU (302 mg), N,N-diisopropylethylamine (0.66 mL) and anhydrous acetonitrile (20 mL) were weighed in, and the reaction was carried out at room temperature for 10 min; a pre-liberated acetonitrile solution (20 mL) of compound 105-9 (260 mg) was added dropwise at room temperature, and the reaction was carried out at room temperature for 15 min. After the reaction was complete, it was directly concentrated, and the concentrate was purified by preparation (60% acetonitrile/water) to obtain the target compound 105 (61.8 mg, 20.5% yield). ESI-MS m/z: 873.4 [M+H]⁺.

¹H NMR (500 MHz, CDCl₃)δ 8.59 (d, J = 1.1 Hz, 1H), 8.44 (d, J = 2.9 Hz, 1H), 7.57 (dd, J = 8.6, 1.4 Hz, 1H), 7.31 (d, J = 8.6 Hz, 1H), 7.28 (d, J = 3.7 Hz, 1H), 7.10 (d, J = 2.9 Hz, 1H), 6.98 (d, J = 9.4 Hz, 1H), 5.95 - 5.87 (m, 1H), 5.77 (dd, J = 57.6, 2.9 Hz, 1H), 4.60 (d, J = 11.8 Hz, 1H), 4.36 (td, J = 12.1, 3.2 Hz, 1H), 4.27 - 4.17 (m, 2H), 4.10 (dd, J = 23.7, 9.7 Hz, 1H), 4.00 (dd, J = 14.5, 4.4 Hz, 1H), 3.85 - 3.75 (m, 5H), 3.50 - 3.43 (m, 1H), 3.32 (td, J = 12.3, 2.8 Hz, 1H), 3.22 - 3.12 (m, 2H), 2.85 (td, J = 14.0, 2.1 Hz, 2H), 2.70 (td, J = 12.9, 2.6 Hz, 1H), 2.47 - 2.39 (m, 1H), 2.36 (d, J = 13.4 Hz, 7H), 2.31 - 2.22 (m, 2H), 2.03 - 1.94 (m, 4H), 1.91 - 1.79 (m, 3H), 1.74 - 1.56 (m, 3H), 1.52 (d, J = 6.4 Hz, 3H), 1.28 (ddd, J = 14.3, 8.1, 4.9 Hz, 2H), 1.14 (dt, J = 8.6, 5.4 Hz, 1H), 0.97 (s, 3H).

### Example 106: Synthesis of compound (1S,2S)-2-(difluoromethyl)-N-((1⁵S,6³S,4S,Z)-1⁵,10,10-trimethyl-1²-(4-morpholinopiperidin-1-y l)-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxaz onino[5,6-a]indola-2(4,2)-thiazola-6(1,3)-pyridazinacycloundecaphane-4-yl)cyclopropane-1-carb oxamide

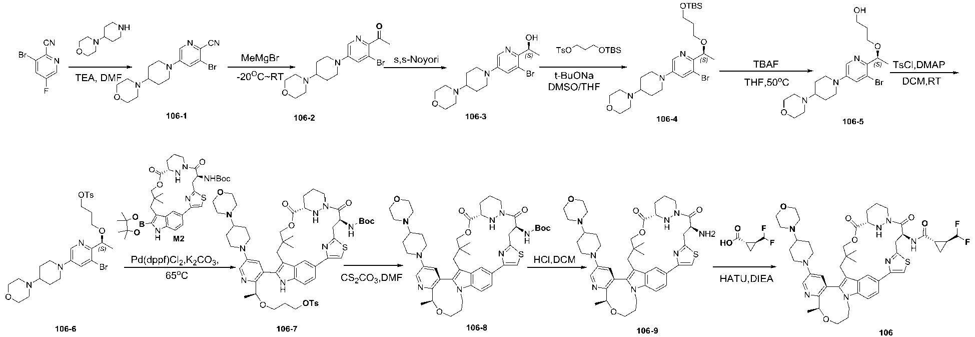

### Step 1: Synthesis of compound 106-1

3-Bromo-5-fluoro-pyridine-2-carbonitrile (1.50 g, 7.46 mmol), N,N-dimethylformamide (17.00 mL), and 4-morpholinopiperidine (1.65 g, 9.70 mmol) were added sequentially to a 50 mL single-necked flask. Triethylamine (2.59 mL, 18.66 mmol) was added dropwise at 0 °C, and the mixture was stirred at room temperature for 1 h. The reaction was complete as monitored by LC-MS, the reaction solution was poured into water, a solid precipitated, filtered, and the filter cake was washed with water. The filter cake was dried to obtain the target compound 106-1 (2.50 g). ESI-MS m/z: 351.1 [M+H]⁺.

### Step 2: Synthesis of compound 106-2

Compound 106-1 (2.50 g, 7.12 mmol) and tetrahydrofuran (25.00 mL) were added sequentially to a 100 mL three-necked flask. Under nitrogen protection, the temperature was lowered to -20 °C, and then methylmagnesium bromide (7.12 mL, 3.00 mol/L, 21.35 mmol) was added dropwise. The mixture was stirred at room temperature for 2 h. The reaction was complete as monitored by LC-MS, quenched with saturated NH₄Cl solution, adjusted to pH = 4 with dilute hydrochloric acid, and stirred for 10 min. The mixture was then adjusted to pH = 8 with saturated NaHCO₃ solution, extracted twice with ethyl acetate, and the organic layer was washed with saturated brine and dried over anhydrous sodium sulfate. The mixture was concentrated under reduced pressure and purified by column chromatography to obtain the target compound 106-2 (2.34 g). ESI-MS m/z: 368.1 [M+H]⁺.

### Step 3: Synthesis of compound 106-3

Triethylamine (22.08 mL, 158.85 mmol) was added to a 100 mL three-necked flask. Under nitrogen protection, the temperature was lowered to 0 °C, formic acid (1.20 mL, 31.77 mmol) was added dropwise, and then (S,S)-N-(p-toluenesulfonyl)-1,2-diphenylethane-1,2-diamine (p-cymene)ruthenium(II) chloride (0.08 g, 0.13 mmol) was added. The reaction was heated to 40 °C and stirred for 15 min. After cooling to room temperature, compound 106-2 (2.34 g, 6.35 mmol) was finally added, and the mixture was stirred at room temperature for 30 h. The reaction was complete as monitored by LC-MS, extracted twice with dichloromethane, and the organic layer was washed with saturated brine and dried over anhydrous sodium sulfate. The mixture was concentrated under reduced pressure and purified by column chromatography to obtain the target compound 106-3 (1.11 g). ESI-MS m/z: 370.1 [M+H]⁺.

### Step 4: Synthesis of compound 106-4

Compound 106-3 (1.11 g, 3.00 mmol), dimethyl sulfoxide (10.00 mL), tetrahydrofuran (2.00 mL), and 3-((tert-butyldimethylsilyl)oxy)propyl-4-methylbenzenesulfonate (3.61 g, 10.49 mmol) were added sequentially to a 50 mL single-necked flask. Sodium tert-butoxide (0.72 g, 7.49 mmol) was added in batches at 10 °C, and the mixture was stirred at room temperature for 1 h. The reaction was complete as monitored by LC-MS, and the reaction mixture was added to a saturated aqueous ammonium chloride solution for quenching. The mixture was extracted twice with ethyl acetate, washed three times with saturated brine, concentrated under reduced pressure, and purified by column chromatography to obtain the target compound 106-4 (1.44 g). ESI-MS m/z: 542.2 [M+H]⁺.

### Step 5: Synthesis of compound 106-5

Compound 106-4 (1.44 g, 2.65 mmol) and tetrahydrofuran (15.00 mL) were added sequentially to a 50 mL single-necked flask. Tetrabutylammonium fluoride (5.31 mL, 1.00 mol/L, 5.31 mmol) was added dropwise at 0 °C, and the reaction was heated to 50 °C and stirred for 1 h. The reaction was complete as monitored by LC-MS, concentrated under reduced pressure, and purified by column chromatography to obtain the target compound 106-5 (1.25 g). ESI-MS m/z: 428.1 [M+H]⁺.

### Step 6: Synthesis of compound 106-6

Compound 106-5 (1.25 g, 2.92 mmol), dichloromethane (13.00 mL), and 4-dimethylaminopyridine (0.71 g, 5.84 mmol) were added sequentially to a 50 mL single-necked flask. Then p-toluenesulfonyl chloride (1.11 g, 5.84 mmol) was added in batches, and the mixture was stirred at room temperature for 6 h. The reaction was complete as monitored by LC-MS, concentrated under reduced pressure, and purified by column chromatography to obtain the target compound 106-6 (0.16 g). ESI-MS m/z: 582.2 [M+H]⁺.

### Step 7: Synthesis of compound 106-7

Compound 106-6 (100.43 mg, 0.17 mmol), M2 (100.00 mg, 0.14 mmol), toluene (1.50 mL), 1,4-dioxane (0.50 mL), water (0.50 mL), potassium carbonate (49.66 mg, 0.36 mmol), and [1,1'-bis(diphenylphosphino)ferrocene]dichloropalladium(II) dichloromethane complex (11.76 mg, 0.01 mmol) were added sequentially to a 10 mL single-necked flask. Nitrogen was purged three times, and under nitrogen protection, the reaction was heated to 65 °C and stirred for 3 h. The reaction was complete as monitored by LC-MS, extracted twice with ethyl acetate, washed with saturated brine, concentrated under reduced pressure, and purified by column chromatography to obtain the target compound 106-7 (88.00 mg). ESI-MS m/z: 535.3 1/2[M+2H]⁺.

### Step 8: Synthesis of compound 106-8

Compound 106-7 (88.00 mg, 0.08 mmol), N,N-dimethylformamide (5.00 mL), and cesium carbonate (80.44 mg, 0.25 mmol) were added sequentially to a 10 mL single-necked flask, and the reaction was heated to 80 °C and stirred for 2 h. The reaction was complete as monitored by LC-MS, the reaction solution was diluted with ethyl acetate, washed sequentially with water and brine three times, concentrated under reduced pressure, and purified by column chromatography to obtain the target compound 106-8 (67.00 mg). ESI-MS m/z: 897.5 [M+H]⁺.

### Step 9: Synthesis of compound 106-9

Compound 106-8 (67.00 mg, 0.07 mmol), dichloromethane (2.00 mL), and hydrochloric acid (4M solution in dioxane) (1.50 mL) were added sequentially to a 10 mL single-necked flask, and the mixture was stirred at room temperature for 1 h. The reaction was complete as monitored by LC-MS, and the mixture was concentrated under reduced pressure. The crude product was directly used in the next step to obtain the crude product compound 106-9 (59.00 mg). ESI-MS m/z: 797.4 [M+H]⁺.

### Step 10: Synthesis of compound 106

(1S,2S)-2-(difluoromethyl)cyclopropane-1-carboxylic acid (15.11 mg, 0.11 mmol) and N,N-dimethylformamide (1.00 mL) were added sequentially to a 10 mL single-necked flask. At 0 °C, N,N,N',N'-tetramethyl-O-(7-azabenzotriazol-1-yl)uranium hexafluorophosphate (42.21 mg, 0.11 mmol) and N,N-diisopropylethylamine (0.12 mL, 0.74 mmol) were added, and the mixture was stirred at 0 °C for 10 min. Finally, compound 106-9 (59.00 mg, 0.07 mmol) was added, and the mixture was stirred at 0 °C for 10 min. The reaction was complete as monitored by LC-MS, and the reaction solution was directly purified by pre-HPLC to obtain the target compound 106 (35.20 mg). ESI-MS m/z: 915.4 [M+H]⁺.

¹H NMR (500 MHz, Chloroform-d)δ 8.58 (d, J = 1.6 Hz, 1H), 8.42 (d, J = 2.9 Hz, 1H), 7.58 (dd, J = 8.6, 1.7 Hz, 1H), 7.33 - 7.28 (m, 2H), 7.09 (d, J = 3.0 Hz, 1H), 6.72 (d, J = 9.5 Hz, 1H), 5.96 - 5.70 (m, 2H), 4.59 (d, J = 12.3 Hz, 1H), 4.36 (td, J = 12.1, 3.3 Hz, 1H), 4.22 (dq, J = 12.9, 6.1 Hz, 2H), 4.07 - 3.95 (m, 2H), 3.83-3.75 (m, 8H), 3.47 (d, J = 14.9 Hz, 1H), 3.31 (td, J = 12.4, 3.2 Hz, 1H), 3.22 - 3.16 (m, 1H), 3.16 - 3.06 (m, 4H), 2.88-2.81 (m, 2H), 2.73 - 2.66 (m, 4H), 2.42 (d, J = 14.4 Hz, 1H), 2.36 - 2.20 (m, 2H), 2.04 - 1.99 (m, 2H), 1.85-1.81 (m, 2H), 1.68-1.59 (m, 1H), 1.51 (d, J = 6.4 Hz, 2H), 1.35 (t, J = 7.3 Hz, 6H), 1.32 - 1.29 (m, 1H), 1.17 - 1.12 (m, 1H), 0.97 (s, 3H), 0.41 (s, 3H).

### Example 108: Synthesis of compound (1S,2S)-2-(difluoromethyl)-N-((1⁵S,6³S,4S,Z)-1⁵,10,10-trimethyl-1²-(4-(1-methylazetidin-3-yl)pi perazin-1-yl)-5,7-dioxo-1⁸, 1⁹,6¹,6²,6³,6⁴,6⁵ ,6⁶-octahydro-1⁵H,1⁷H-8-oxa-1(13,15)-pyrido[3',2':7,8 ][1,5]oxazonino[5,6-a]indola-2(4,2)-thiazola-6(1,3)-pyridazinacycloundecaphane-4-yl)cycloprop ane-1-carboxamide

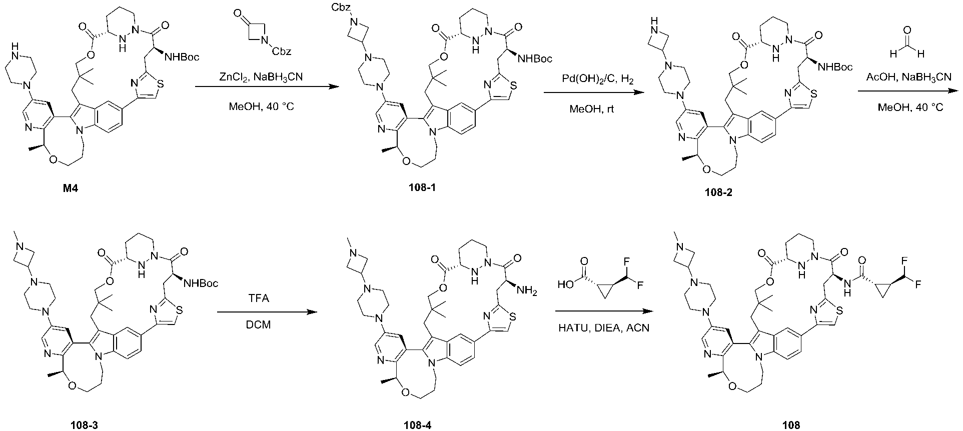

### Step 1: Synthesis of compound 108-1

Under a nitrogen atmosphere, 1-benzyloxycarbonylazetidin-3-one (50.5 mg) and ZnCl₂ (0.25 mL, 1M in THF) were added sequentially to a solution of M4 (100.0 mg) in MeOH (4.0 mL), and then the system was stirred in a 40 °C oil bath for 1 h. Then sodium cyanoborohydride (38.6 mg) was added, and the reaction was continued stirring in the 40 °C oil bath for 2 h. Additional 1-benzyloxycarbonylazetidin-3-one (50.5 mg) and ZnCl₂ (0.25 mL, 1M in THF) were added, and then the system was stirred in a 40 °C oil bath for 1 h. Then sodium cyanoborohydride (38.6 mg) was added, and the reaction was continued stirring in the 40 °C oil bath for 2 h. After the reaction was complete as monitored by LC-MS, the reaction was quenched with saturated sodium bicarbonate solution, extracted with DCM, and separated; the aqueous phase was further extracted twice with DCM, the organic phases were combined, dried over anhydrous sodium sulfate, and concentrated to obtain a crude product of compound 108-1 (100 mg). ESI-MS m/z: 502.0, [M+2H]²⁺/2.

### Step 2: Synthesis of compound 108-2

Pd(OH)₂/C (50.0 mg, 10% Pd) was added to a solution of compound 108-1 (100 mg) in MeOH (4.0 mL). After repeatedly purged with hydrogen, the reaction was allowed to stir at room temperature for 3 h. After the reaction was complete as monitored by LC-MS, the mixture was filtered to remove the catalyst, and the filtrate was concentrated to obtain a crude product of compound 108-2 (off-white solid powder, 50 mg), which was directly used in the next reaction. ESI-MS m/z: 435.0, [M+2H]²⁺/2.

### Step 3: Synthesis of compound 108-3

HOAc (9.9 µL) was added to a solution of compound 108-2 (50.0 mg) in MeOH (2.0 mL). The above system was stirred at room temperature for 10 min, then 37% aqueous formaldehyde solution (11 µL) and sodium cyanoborohydride (4.3 mg) were added, and the reaction was allowed to stir at room temperature for 20 min. After the reaction was complete as monitored by LC-MS, saturated sodium bicarbonate solution was added to quench the reaction. The mixture was extracted with DCM and separated, the aqueous phase was further extracted twice with DCM, the organic phases were combined, dried over anhydrous sodium sulfate, and further concentrated to obtain a crude product of compound 108-3 (40 mg), which was directly used in the next reaction. ESI-MS m/z: 442.0, [M+2H]²⁺/2.

### Step 4: Synthesis of compound 108-4

Trifluoroacetic acid (1.0 mL) was added to a solution of compound 108-3 (40 mg) in DCM (2.0 mL), and the reaction was stirred at room temperature for 1 h. After the reaction was complete as monitored by LC-MS, the reaction solution was concentrated to dryness. Then dichloromethane and saturated sodium carbonate solution were added for extraction and separation, and the aqueous phase was further extracted twice with dichloromethane. The organic phases were combined, dried over anhydrous sodium sulfate, and further concentrated to obtain a crude product of compound 108-4 (40 mg, hydrochloride salt), which was directly used in the next reaction. ESI-MS m/z: 782.5, [M+H]⁺.

### Step 5: Synthesis of compound 108

At room temperature, HATU (38.9 mg) and DIEA (42 µL) were added to a solution of compound (1S,2S)-2-(difluoromethyl)cyclopropane-1-carboxylic acid (13.9 mg) in acetonitrile (1.0 mL). The above solution was stirred at room temperature for 10 min, and then a solution of compound 108-4 and DIEA (42 µL) in acetonitrile (1.0 mL) was added to the above system, and the system was still stirred at room temperature for 30 min. After the reaction was complete, the system was concentrated, and the concentrate was purified by preparative chromatography (acetonitrile:water (containing 0.05% trifluoroacetic acid) = 3:2). Then an appropriate amount of sodium bicarbonate solution was added to the prepared solution to neutralize the trifluoroacetic acid, further concentrated to remove acetonitrile, then dichloromethane was added for extraction and separation, the aqueous phase was further extracted twice with dichloromethane, the organic phases were combined, dried over anhydrous sodium sulfate, and further concentrated to obtain the target compound 108 (13.0 mg). ESI-MS m/z: 900.5 [M+H]⁺.

¹H NMR (500 MHz, CDCl₃)δ 8.58 (d, J = 1.3 Hz, 1H), 8.42 (d, J = 2.9 Hz, 1H), 7.58 (dd, J = 8.6, 1.5 Hz, 1H), 7.31 (d, J = 8.5 Hz, 1H), 7.29 (s, 1H), 7.08 (d, J = 2.9 Hz, 1H), 6.64 (d, J = 9.5 Hz, 1H), 5.90 (t, J = 8.8 Hz, 1H), 5.86 (td, 1H), 4.60 (d, J = 11.9 Hz, 1H), 4.36 (td, J = 12.1, 3.2 Hz, 1H), 4.27 - 4.16 (m, 2H), 4.00 (m, 2H), 3.82 (s, 2H), 3.81 - 3.75 (m, 1H), 3.72 - 3.63 (m, 2H), 3.47 (d, J = 14.8 Hz, 1H), 3.35 - 3.23 (m, 5H), 3.22 - 3.02 (m, 5H), 2.73 - 2.65 (m, 1H), 2.50 (d, J = 4.3 Hz, 4H), 2.45 (s, 3H), 2.41 (d, J = 14.4 Hz, 1H), 2.37 - 2.18 (m, 3H), 1.85 - 1.80 (m, 2H), 1.51 (d, J = 6.5 Hz, 3H), 1.35 - 1.29 (m, 3H), 1.18 - 1.12 (m, 1H), 0.96 (s, 3H), 0.88 (t, J = 6.8 Hz, 1H), 0.40 (s, 3H).

### Example 109: Synthesis of compound (1S,2S)-2-(difluoromethyl)-N-((1⁵S,6³S,4S,Z)-1⁵,10,10-trimethyl-5,7-dioxo-1²-(4-(tetrahydro-2H -pyran-4-yl)piperazin-1-yl)-18, 1⁹,6¹,6²,6³ ,6⁴,6⁵,6⁶-octahydro-1⁵H,1⁷H-8-oxa-1(13,15)-pyrido[3',2' :7,8][1,5]oxazonino[5,6-a]indola-2(4,2)-thiazola-6(1,3)-pyridazinacycloundecaphane-4-yl)cyclo propane-1-carboxamide

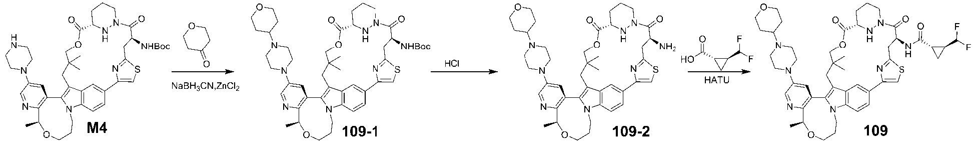

### Step 1: Synthesis of compound 109-1

Tetrahydropyranone (9 µL, 0.10 mmol), tetrahydrofuran (5.00 mL), and zinc chloride (0.15 mL, 1 mol/L in THF, 0.15 mmol) were added sequentially to a 25 mL single-necked flask, and the mixture was stirred at 50 °C for 1 h. M4 (80 mg, 0.10 mmol) was added, and the mixture was stirred at 50 °C for 1 h. Sodium cyanoborohydride (13.6 mg, 0.22 mmol) was added, and the reaction was stirred at 50 °C. After the reaction was complete as monitored by LC-MS, saturated ammonium chloride solution was added to quench the reaction, extracted twice with dichloromethane, the organic phases were combined, washed with saturated brine, and concentrated to obtain a crude product of compound 109-1 (white solid, 52.0 mg). ESI-MS m/z: 897.48 [M+H]⁺.

### Step 2: Synthesis of compound 109-2

Compound 109-1 (52.0 mg, 0.06 mmol), DCM (1.00 mL), and HCl (4M solution in dioxane) (1.00 mL) were added sequentially to a 10 mL vial, and the reaction was stirred at room temperature. After the reaction was complete as monitored by LC-MS, the reaction solution was neutralized with saturated sodium bicarbonate solution, extracted with DCM, washed with saturated brine, and concentrated to obtain a crude product of compound 109-2 (yellow solid, 50.0 mg). ESI-MS m/z: 796.97 [M+H]⁺.

### Step 3: Synthesis of compound 109

(1S,2S)-2-(difluoromethyl)cyclopropane-1-carboxylic acid (7 µL, 0.08 mmol), HATU (71.5 mg, 0.19 mmol), and DIEA (42 µL, 0.25 mmol) were added sequentially to a 10 mL single-necked flask, and the mixture was stirred at room temperature for 10 min. The mixture was cooled in an ice-water bath, and then a solution of compound 109-2 (50.0 mg, 0.06 mmol) in acetonitrile was added. After the reaction was complete as monitored by LC-MS, the reaction solution was directly purified by Prep-HPLC basic method, and the eluate was lyophilized to finally obtain the target compound 109 (6.6 mg). ESI-MS m/z: 915.10, [M+H]⁺.

¹H NMR (500 MHz, CDCl₃)δ 8.58 (s, 1H), 8.40 (d, J = 1.9 Hz, 1H), 7.57 (d, J = 8.5 Hz, 1H), 7.34 - 7.28 (m, 2H), 7.09 (d, J = 2.1 Hz, 1H), 6.85 (d, J = 9.4 Hz, 1H), 5.96 - 5.67 (m, 2H), 4.59 (d, J =12.5 Hz, 1H), 4.41 - 4.30 (m, 1H), 4.28 - 4.16 (m, 2H), 4.11 - 4.03 (m, 3H), 4.03 - 3.95 (m, 1H), 3.82 (s, 3H), 3.50 - 3.28 (m, 8H), 3.23 - 3.12 (m, 3H), 2.76 - 2.57 (m, 2H), 2.41 (d, J = 14.4 Hz, 1H), 2.36 - 2.20 (m, 2H), 2.11 - 1.77 (m, 10H), 1.74 - 1.57 (m, 4H), 1.51 (d, J = 6.2 Hz, 3H), 0.97 (s, 3H), 0.40 (s, 3H).

### Example 110: Synthesis of compound (1S,2S)-2-(difluoromethyl)-N-((1⁵S,6³S,4S,Z)-1²-((S)-hexahydropyrazino[2,1-c][1,4]oxazin-8(1 H)-yl)-1⁵,10,10-trimethyl-5,7-dioxo-1⁸,1⁹,6¹,6²,6³,6⁴,6⁵,6⁶-octahydro-1⁵H,1⁷H-8-oxa-1(13,15)-py rido[3',2':7,8][1,5]oxazonino[5,6-a]indola-2(4,2)-thiazola-6(1,3)-pyridazinacycloundecaphane-4-yl)cyclopropane-1-carboxamide

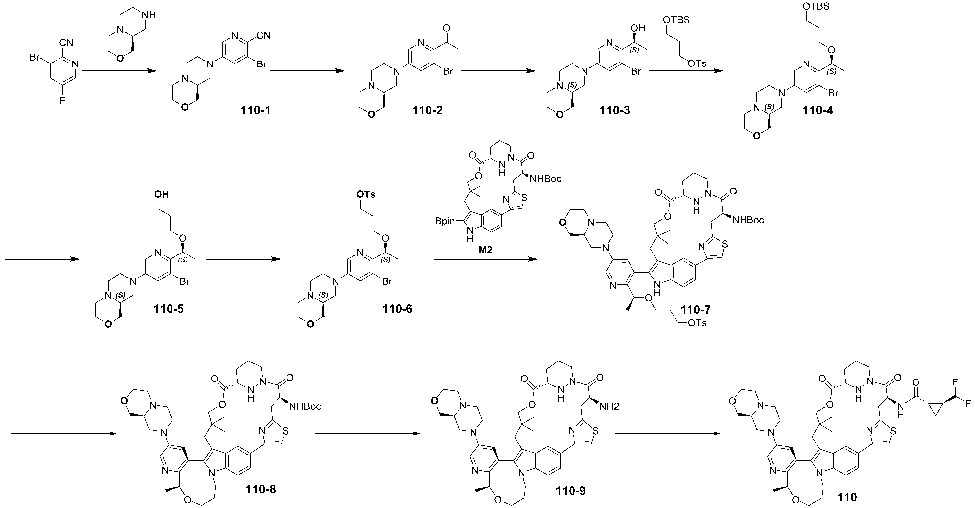

### Step 1: Synthesis of compound 110-1

The substrate 3-bromo-5-fluoro-pyridine-2-carbonitrile (1.3 g) was added to a 100 mL single-necked flask, THF (20 mL) was added, DIEA (2.67 mL) and (S)-octahydropyrazino[2,1-c][1,4]oxazine (0.92 g) were weighed and added respectively, and the reaction was carried out at room temperature for 0.5 h. After the reaction was complete, saturated NaCl (25 mL) was added, extracted with EA, and purified by column chromatography to obtain compound 110-1 (1.58 g). ESI-MS m/z: 323.24 [M+H]⁺.

### Step 2: Synthesis of compound 110-2

Compound 110-1 (1.58 g) and anhydrous THF (16 mL) were added to a 100 mL three-necked flask, the temperature was lowered to -20 °C under N₂ protection, MeMgBr (3 M in THF, 4.89 mL) was slowly added dropwise, and the mixture was heated to room temperature and reacted for 2 h. After the reaction was complete, HCl (1N, 30 mL) was added to the reaction solution, extracted with EA, the organic phase was collected, the aqueous phase was further extracted twice with EA (30 mL), the organic phases were collected and combined, dried over anhydrous sodium sulfate, and the concentrate was purified by column chromatography to obtain the target compound 110-2 (0.87 g). ESI-MS m/z: 340.26 [M+H]⁺.

### Step 3: Synthesis of compound 110-3

Compound 110-2 (0.87 g) was added to a 25 mL single-necked flask, TEA (10 mL) and s,s-Noyori catalyst (0.16 g) were added, the temperature was lowered to 0 °C under N₂ protection, formic acid (0.59 g) was slowly added dropwise, and the reaction was carried out at room temperature for 12 h. After the reaction was complete, ethyl acetate (50 mL) and water (50 mL) were added to the reaction solution, extracted, the organic phase was collected, the aqueous phase was further extracted twice with ethyl acetate (25 mL), the organic phases were collected and combined, dried over anhydrous sodium sulfate, concentrated, and the concentrate was purified by column chromatography to obtain the target axially chiral compound 110-3 (0.66 g). ESI-MS m/z: 342.27 [M+H]⁺.

### Step 4: Synthesis of compound 110-4

Compound 110-3 (0.95 g), 3-((tert-butyldimethylsilyl)oxy)propyl-4-methylbenzenesulfonate (1.99 g) and DMSO/THF (7 mL/1.5 mL) were weighed and added into the reaction flask respectively, and the temperature was lowered to 10 °C under N₂ protection; sodium tert-butoxide (0.46 g) was added, and the reaction was carried out at room temperature for 1.0 h; the reaction was complete, the temperature was lowered to 0 °C, tap water (50 mL) was added for dilution, extracted with EA, the organic phase was dried over anhydrous sodium sulfate, concentrated, and the concentrate was purified by column chromatography to obtain compound 110-4 (0.52 g). ESI-MS m/z: 514.40 [M+H]⁺.

### Step 5: Synthesis of compound 110-5

Compound 110-4 (0.52 g) was added to a 50 mL single-necked flask, anhydrous methanol (10 mL) was added, the temperature was lowered to 0 °C under N₂ protection, TMSCl (0.1 mL) was slowly added dropwise, and the reaction was carried out at room temperature for 1.0 h. After the reaction was complete, it was directly concentrated to dryness to obtain the target compound 110-5 (0.52 g). ESI-MS m/z: 400.32 [M+H]⁺.

### Step 6: Synthesis of compound 110-6

Compound 110-5 (0.52 g) was added to a 50 mL single-necked flask, anhydrous DCM (6 mL) was added, DIEA (0.64 mL) and DMAP (0.48 g) were weighed and added respectively, the temperature was lowered to 0 °C under N₂ protection, TsCl (0.50 g) was added, and the reaction was carried out at room temperature for 1.0 h. After the reaction was complete, saturated NaCl (25 mL) was added, extracted with DCM, the organic phase was concentrated to dryness, and the concentrate was purified by column chromatography to obtain the target compound 110-6 (0.2 g). ESI-MS m/z: 554.32 [M+H]⁺.

### Step 7: Synthesis of compound 110-7

Compound 110-6 (96 mg), M2 (100 mg), Pd(dppf)Cl₂ (11 mg), and potassium carbonate (50 mg) were weighed and added respectively to a 100 mL single-necked flask, toluene (1.5 mL), 1,4-dioxane (0.5 mL) and 0.5 mL of water were added, N₂ was purged 3 times, and the mixture was heated to 65 °C and reacted for 5 h. After the reaction was complete, 20 mL of EA and 20 mL of water were added to the reaction solution to extract. The aqueous phase was further extracted 2 times with EA, the organic phases were collected and combined, dried over anhydrous sodium sulfate, concentrated, and the concentrate was purified by column chromatography to obtain the target compound 110-7 (87 mg). ESI-MS m/z: 522.42 [M+H]⁺.

### Step 8: Synthesis of compound 110-8

Compound 110-7 (87 mg) was added to a 25 mL single-necked flask, Cs₂CO₃ (82 mg) and anhydrous DMF (9 mL) were weighed in, and the reaction was carried out at 80 C for 1 h under N₂ protection. After the reaction was complete, tap water (50 mL) was added for dilution, extracted with EA, the organic phase was dried over anhydrous sodium sulfate, concentrated, and the concentrate was purified by column chromatography to obtain the target compound 110-8 (64 mg). ESI-MS m/z: 867.65 [M+H]⁺.

### Step 9: Synthesis of compound 110-9

Compound 110-8 (64 mg) was added to a 25 mL single-necked flask, 2 mL of DCM was added, then 2 mL of hydrochloric acid (4 M solution in dioxane) was slowly added dropwise, and the reaction was carried out at room temperature for 1 h. After the reaction was complete, it was concentrated under reduced pressure to dryness to obtain the target compound 110-9 hydrochloride (64 mg). ESI-MS m/z: 767.6 [M+H]⁺.

### Step 10: Synthesis of compound 110

Compound (1S,2S)-2-(difluoromethyl)cyclopropane-1-carboxylic acid (14 mg) was added to a 10 mL single-necked flask, HATU (63 mg), N,N-diisopropylethylamine (0.08 mL) and anhydrous acetonitrile (2 mL) were weighed in, and the reaction was carried out at room temperature for 10 min; a pre-liberated acetonitrile solution (4 mL) of compound 110-9 (64 mg) was added dropwise at room temperature, and the reaction was carried out at room temperature for 15 min. After the reaction was complete, it was directly concentrated, and the concentrate was purified by preparation (60% acetonitrile/water) to obtain the target compound 110 (27.7 mg). ESI-MS m/z: 887.6 [M+H]⁺.

¹H NMR (500 MHz, CDCl₃)δ 8.58 (s, 1H), 8.40 (s, 1H), 7.58 (d, J = 8.7 Hz, 1H), 7.33 - 7.28 (m, 2H), 7.07 (s, 1H), 6.66 (d, J = 9.4 Hz, 1H), 5.87 (dt, J = 79.2, 37.9 Hz, 2H), 4.60 (d, J = 12.7 Hz, 1H), 4.35 (t, J = 10.6 Hz, 1H), 4.22 (dd, J = 14.7, 9.2 Hz, 2H), 4.01 (t, J = 10.9 Hz, 2H), 3.90 (d, J = 10.3 Hz, 1H), 3.74 (dd, J = 22.6, 11.6 Hz, 2H), 3.65 (d, J = 11.4 Hz, 1H), 3.54 - 3.39 (m, 2H), 3.33 (dd, J = 23.0, 11.9 Hz, 2H), 3.22 - 3.09 (m, 2H), 3.03 (t, J = 10.5 Hz, 1H), 2.90 (d, J = 10.9 Hz, 1H), 2.71 (dd, J = 27.2, 12.2 Hz, 2H), 2.54 (q, J = 10.8 Hz, 2H), 2.48 - 2.39 (m, 2H), 2.26 (dd, J = 29.7, 17.0 Hz, 2H), 1.98 (d, J = 14.1 Hz, 2H), 1.83 (d, J = 8.4 Hz, 2H), 1.51 (d, J = 6.2 Hz, 3H), 1.28 (d, J = 27.3 Hz, 6H), 1.16 (s, 1H), 0.97 (s, 3H), 0.41 (s, 3H).

### Example 112: Synthesis of compound (1S,2S)-2-(difluoromethyl)-N-((1⁵S,6³S,4S,Z)-1⁵,10,10-trimethyl-5,7-dioxo-1²-(piperazin-1-yl)-1 ⁸,1⁹,6¹,6²,6³,6⁴,6^{5,}6⁶-octahydro-1⁵H,1⁷H-8-oxa-1(13,15)-pyrido[3',2':7,8][1,5]oxazonino[5,6-a]in dola-2(4,2)-thiazola-6(1,3)-pyridazinacycloundecaphane-4-yl)cyclopropane-1-carboxamide

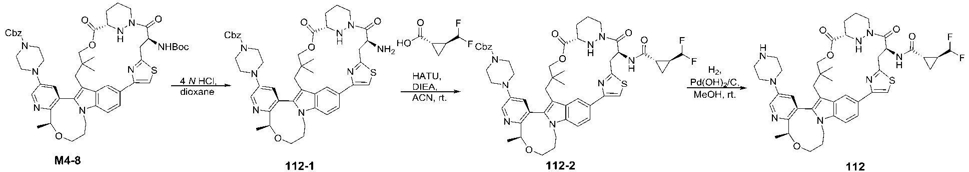

### Step 1: Synthesis of compound 112-1

Compound M4-8 (132 mg) was added to a 25 mL single-necked flask, 8 mL of hydrochloric acid (4 M solution in dioxane) was added, and the reaction was carried out at room temperature for 1 h. After the reaction was complete, it was concentrated under reduced pressure to dryness to obtain the target compound 112-1 hydrochloride (120 mg, 95.5% yield). ESI-MS m/z: 847.1 [M+H]⁺.

### Step 2: Synthesis of compound 112-2

Compound (1S,2S)-2-(difluoromethyl)cyclopropane-1-carboxylic acid (58 mg) was added to a 10 mL single-necked flask, HATU (108 mg), N,N-diisopropylethylamine (230 µL) and anhydrous acetonitrile (5 mL) were weighed in, and the reaction was carried out at room temperature for 10 min; a pre-liberated acetonitrile solution (5 mL) of 112-1 (120 mg) was added dropwise at room temperature, and the reaction was carried out at room temperature for 15 min. After the reaction was complete, it was directly concentrated, and the concentrate was purified by preparative TLC to obtain the target compound 112-2 (210 mg).

### Step 3: Synthesis of compound 112

Compound 112-2 (210 mg) was added to a 25 mL single-necked flask, palladium hydroxide on carbon (105 mg) and anhydrous methanol (10 mL) were weighed in, hydrogen was purged 3 times, and the reaction was carried out at room temperature for 2 h. After the reaction was complete, the reaction solution was filtered through a 0.45 µm filter membrane, the filtrate was concentrated under reduced pressure to dryness, and purified by preparative HPLC (60% ACN-H₂O) to obtain the target compound 112 (29.1 mg, 16% yield). ESI-MS m/z: 831.2 [M+H]⁺.

¹H NMR (500 MHz, MeOD)δ 8.56 (s, 1H), 8.41 (d, J = 2.9 Hz, 1H), 7.69 (dd, J = 8.6, 1.5 Hz, 1H), 7.56 (s, 1H), 7.45 (d, J = 8.6 Hz, 1H), 7.37 (d, J = 2.9 Hz, 1H), 5.98 - 5.67 (m, 2H), 4.90 (s, 3H), 4.88-4.82 (m, 4H), 4.61 (s, 1H), 4.43 (d, J = 10.3 Hz, 1H), 4.34 - 4.26 (m, 2H), 4.16 (dd, J = 12.5, 5.8 Hz, 1H), 4.06 (dd, J = 14.5, 4.5 Hz, 1H), 3.79-3.67 (m, 3H), 3.52 - 3.43 (m, 4H), 3.28- 3.25 (m, 3H),3.20 (d, J = 14.5 Hz, 1H), 2.81-2.75 (m, 1H), 2.48 (d, J = 14.3 Hz, 1H), 2.41-2.34 (m, 1H), 2.26-2.21 (m, 1H), 2.11 - 2.04 (m, 1H), 2.00 - 1.69 (m, 3H), 1.64-1.58 (m, 1H), 1.48 (d, J = 6.5 Hz, 3H), 1.20-1.10 (m, 2H), 1.00 (s, 3H), 0.41 (s, 3H).

### Example 113: Synthesis of compound (1S,2S)-2-(difluoromethyl)-N-((1⁵S,6³S,4S,Z)-1²-(4-methoxypiperidin-1-yl)-1⁵,10,10-trimethyl-5,7-dioxo-1⁸,1⁹,6¹,6²,6³,6⁴,6^{5,}6⁶-octahydro-1⁵H, 1⁷H-8-oxa-1(13,15)-pyrido[3',2':7,8][1,5]oxazoni no[5,6-alindola-2(4,2)-thiazola-6(1,3)-pyridazinacycloundecaphane-4-yl)cyclopropane-1-carbox amide

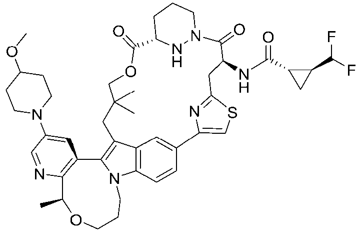

Compound 113 was prepared with reference to the synthetic procedure of compound 114. ESI-MS m/z: 860.6 [M+H]⁺. ¹H NMR (500 MHz, CDCl₃)δ 8.58 (d, J = 1.3 Hz, 1H), 8.44 (d, J = 2.9 Hz, 1H), 7.57 (dd, J = 8.5, 1.5 Hz, 1H), 7.31 (d, J = 8.6 Hz, 1H), 7.28 (s, 1H), 7.10 (d, J = 2.8 Hz, 1H), 6.64 (d, J = 9.5 Hz, 1H), 5.90 (t, J = 8.9 Hz, 1H), 5.84 (td, J = 57.6, 2.9 Hz, 1H), 4.59 (d, J = 12.0 Hz, 1H), 4.36 (td, J = 12.1, 3.3 Hz, 1H), 4.26 - 4.15 (m, 2H), 4.04 - 3.95 (m, 2H), 3.78 (m, 3H), 3.60 - 3.51 (m, 2H), 3.50 - 3.40 (m, 2H), 3.39 (s, 3H), 3.31 (td, J = 12.3, 3.5 Hz, 1H), 3.22 - 3.04 (m, 4H), 2.68 (td, J = 12.9, 2.7 Hz, 1H), 2.42 (d, J = 14.4 Hz, 1H), 2.36 - 2.19 (m, 2H), 2.06 - 1.88 (m, 5H), 1.85 - 1.79 (m, 2H), 1.79 - 1.70 (m, 2H), 1.51 (d, J = 6.4 Hz, 3H), 1.36 - 1.29 (m, 2H), 1.18 - 1.11 (m, 1H), 0.97 (s, 3H), 0.41 (s, 3H).

### Example 114: Synthesis of compound (1S,2S)-2-(difluoromethyl)-N-((1⁵S,6³S,4S,Z)-1²-(4,4-difluoropiperidin-1-yl)-1⁵,10,10-trimethyl-5,7-dioxo-1⁸,1⁹,6¹,6²,6³,6⁴,6⁵,6⁶-octahydro-1⁵H, 1⁷H-8-oxa-1(13,15)-pyrido[3',2':7,8][1,5]oxazoni no[5,6-alindola-2(4,2)-thiazola-6(1,3)-pyridazinacycloundecaphane-4-yl)cyclopropane-1-carbox amide

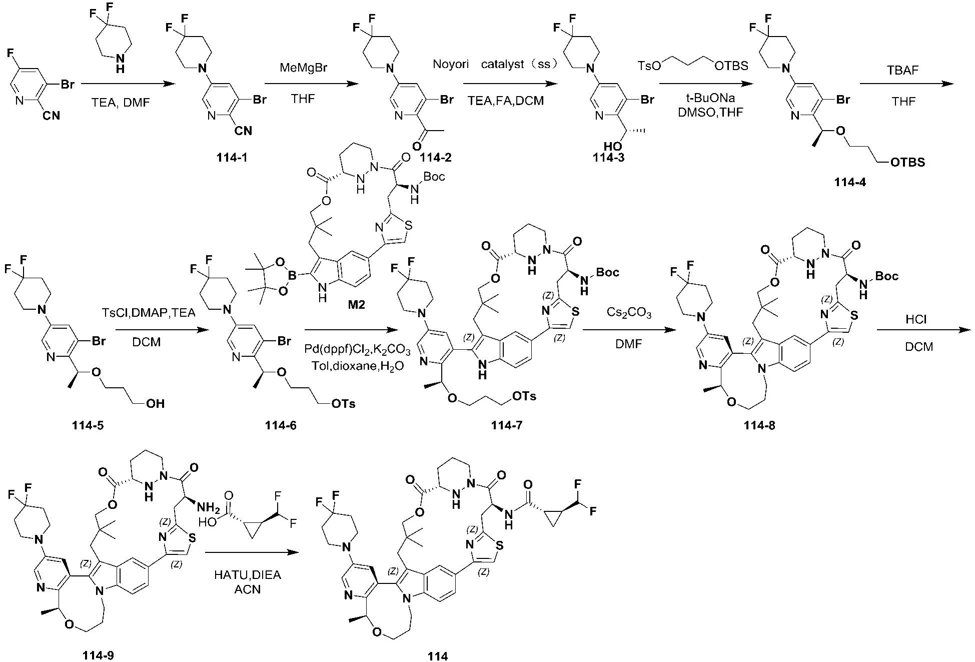

### Step 1: Synthesis of compound 114-1

Under ice-water bath conditions, 3-bromo-5-fluoro-pyridine-2-carbonitrile (2.00 g, 9.95 mmol), triethylamine (4.15 mL, 29.85 mmol), and compound 4,4-difluoropiperidine (1.33 g, 10.95 mmol) were added sequentially to DMF (20.00 mL), and the reaction was carried out at room temperature for 1 h. 50 mL of water was added to the reaction solution, extracted with 40 mL*2 of EA, washed with 40 mL*2 of saturated brine, dried over anhydrous sodium sulfate, and concentrated to dryness to obtain a crude product of compound 114-1 (2.60 g, 86.49%), which was directly used in the next reaction. ESI-MS m/z: 302.12 [M+H]⁺.

### Step 2: Synthesis of compound 114-2

Compound 114-1 (2.60 g, 8.61 mmol) was dissolved in tetrahydrofuran (30.00 mL), the atmosphere was purged with nitrogen, methylmagnesium bromide (8.61 mL, 3.00 mol/L, 25.82 mmol) was added dropwise at -20 °C, and the mixture was heated to room temperature and reacted for 2 h. The reaction solution was added dropwise to 40 mL of ice-cold saturated aqueous ammonium chloride solution, adjusted to pH = 3 with 1N aqueous hydrochloric acid solution, stirred for 10 min, then adjusted to pH = 9 with saturated aqueous sodium bicarbonate solution, extracted with 40 mL*2 of EA, washed with 40 mL*2 of saturated brine, dried over anhydrous sodium sulfate, and separated by column chromatography to obtain compound 114-2 (1.76 g, 64.08%). ESI-MS m/z: 319.03, [M+H]⁺.

### Step 3: Synthesis of compound 114-3

Compound 114-2 (1.76 g, 5.51 mmol), (S,S)-N-(p-toluenesulfonyl)-1,2-diphenylethane-1,2-diamine (p-cymene)ruthenium(II) chloride (0.17 g, 0.28 mmol), and TEA (7.67 mL, 55.15 mmol) were added to DCM (17.00 mL). Formic acid (1.27 g, 25.57 mmol) was added in an ice-water bath, and the reaction was carried out at room temperature for 16 h. 50 mL of water was added to the reaction solution, extracted with 40 mL*2 of DCM, washed with 40 mL*2 of saturated brine, dried over anhydrous sodium sulfate, and separated by column chromatography (PE:EA = 85%:15%) to obtain compound 114-3 (0.88 g, 49.69%). ESI-MS m/z: 321.07 [M+H]⁺.

### Step 4: Synthesis of compound 114-4

Compound 114-3 (880.00 mg, 2.74 mmol) and compound 3-((tert-butyldimethylsilyl)oxy)propyl-4-methylbenzenesulfonate (2.83 g, 8.22 mmol) were added to DMSO (9.00 mL), and then a solution of sodium tert-butoxide (660.00 mg, 6.85 mmol) in tetrahydrofuran (2.00 mL) was added, and the reaction was carried out at 40 °C for 1 h. 50 mL of water was added to the reaction solution, extracted with 40 mL*2 of EA, washed with 40 mL*2 of saturated brine, dried over anhydrous sodium sulfate, and separated by column chromatography to obtain compound 114-4 (1000.00 mg, 73.95%). ESI-MS m/z: 493.29, [M+H]⁺.

### Step 5: Synthesis of compound 114-5

Compound 114-4 (1000.00 mg, 2.03 mmol) and tetrabutylammonium fluoride (3.05 mL, 1.00 mol/L, 3.05 mmol) were added to tetrahydrofuran (10.00 mL), and the reaction was carried out at 50 °C for 1 h. The mixture was concentrated to dryness and separated by column chromatography to obtain compound 114-5 (700.00 mg, 96.09%). ESI-MS m/z: 379.15, [M+H]⁺.

### Step 6: Synthesis of compound 114-6

Compound 114-5 (700.00 mg, 1.85 mmol), DMAP (20.00 mg, 0.20 mmol), and TEA (0.56 mL, 4.06 mmol) were added to DCM (8.00 mL). p-Toluenesulfonyl chloride (580.00 mg, 4.05 mmol) was added in batches in an ice-water bath, and the reaction was carried out at room temperature for 2 h. 50 mL of water was added to the reaction solution, extracted with 40 mL*2 of DCM, washed with 40 mL*2 of saturated brine, dried over anhydrous sodium sulfate, and separated by column chromatography to obtain compound 114-6 (490.00 mg, 49.77%). ESI-MS m/z: 533.025, [M+H]⁺.

### Step 7: Synthesis of compound 114-7

Compound 114-6 (80.00 mg, 0.15 mmol), compound M2 (104.05 mg, 0.15 mmol), [1,1'-bis(diphenylphosphino)ferrocene]dichloropalladium(II) (10.98 mg, 0.01 mmol), potassium carbonate (41.46 mg, 0.30 mmol), water (0.50 mL), and 1,4-dioxane (1.00 mL) were added to toluene (3.00 mL), and the reaction was carried out at 65 °C for 2 h under a nitrogen atmosphere. The mixture was concentrated and separated by column chromatography to obtain compound 114-7 (80.00 mg, 52.28%). ESI-MS m/z: 1020.69, [M+H]⁺.

### Step 8: Synthesis of compound 114-8

Compound 114-7 (80.00 mg, 0.08 mmol) and cesium carbonate (51.09 mg, 0.16 mmol) were added to DMF (3.00 mL), and the reaction was carried out at 85 °C for 1 h. 50 mL of water was added to the reaction solution, extracted with 40 mL*2 of EA, washed with 40 mL*2 of saturated brine, dried over anhydrous sodium sulfate, and separated by column chromatography to obtain compound 114-8 (60 mg, 90.25%). ESI-MS m/z: 848.48, [M+H]⁺.

### Step 9: Synthesis of compound 114-9

Compound 114-8 (60.00 mg, 0.07 mmol) was dissolved in DCM (1.00 mL), then hydrochloric acid (4M solution in dioxane) (2.00 mL) was added, and the reaction was carried out at room temperature for 1 h. The reaction solution was concentrated to dryness, 5 mL of toluene was added for dilution, and concentrated to dryness again to obtain a crude product of compound 114-9 (50 mg, 94.43%), which was directly used in the next reaction. ESI-MS m/z: 748.45, [M+H]⁺.

### Step 10: Synthesis of compound 114

Compound (1S,2S)-2-(difluoromethyl)cyclopropane-1-carboxylic acid (10.95 mg, 0.08 mmol) was dissolved in acetonitrile (3.00 mL). DIEA (0.11 mL, 0.67 mmol) and HATU (38.18 mg, 0.10 mmol) were added sequentially in an ice-water bath. After reacting in the ice-water bath for 10 min, a solution of compound 114-9 (50.00 mg, 0.07 mmol) in acetonitrile (3.00 mL) and DIEA (0.11 mL, 0.67 mmol) was added, and the reaction was carried out at room temperature for 1 h. The mixture was concentrated to dryness and purified by preparative separation to obtain compound 114 (19.00 mg, 32.68%). ESI-MS m/z: 866.51, [M+H]⁺.

¹H NMR (500 MHz, DMSO)δ 8.90 (d, J = 9.0 Hz, 1H), 8.49 (dd, J = 10.0, 1.9 Hz, 2H), 7.82 (s, 1H), 7.72 (dd, J = 8.6, 1.4 Hz, 1H), 7.50 (d, J = 8.6 Hz, 1H), 7.34 (d, J = 2.9 Hz, 1H), 5.95 (td, J = 56.6, 5.1 Hz, 1H), 5.56 (t, J = 9.4 Hz, 1H), 5.13 (d, J = 12.2 Hz, 1H), 4.23 (dd, J = 16.5, 7.6 Hz, 2H), 4.15 (q, J = 6.3 Hz, 1H), 4.08 - 3.98 (m, 2H), 3.71 - 3.56 (m, 3H), 3.52 - 3.43 (m, 4H), 3.38 (d, J = 14.5 Hz, 1H), 3.21 - 3.10 (m, 2H), 3.01 (d, J = 14.4 Hz, 1H), 2.79-2.72 (m, 1H), 2.41 (d, J = 14.4 Hz, 1H), 2.31-2.26 (m, 1H), 2.13 - 2.02 (m, 6H), 1.81 (s, 3H), 1.67-1.61 (m, 1H), 1.55-1.49 (m, 1H), 1.38 (d, J = 6.4 Hz, 3H), 1.09-0.98 (m, 2H), 0.95 (s, 3H), 0.32 (s, 3H).

The synthetic procedure for the following intermediates is the same as in Example 114, using 3-bromo-5-fluoro-pyridine-2-carbonitrile and H-R₄ to synthesize the corresponding intermediate X-1. The specific synthetic steps are as follows:

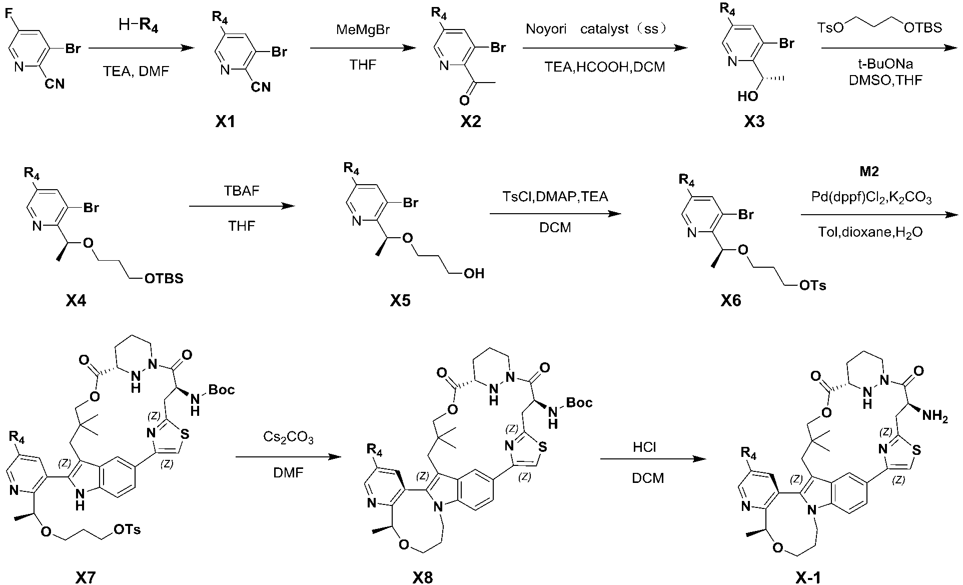

The required starting materials (H-R₄) are shown in the following table:

| **Example No.** | **Required Starting Material (H-R₄)** | **Corresponding Intermediate (X-1)** |
|---|---|---|
| **115** | 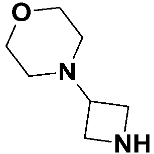 | 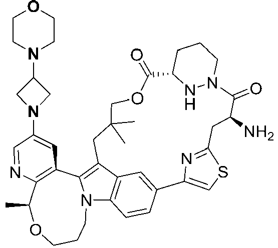 |
| **123** | 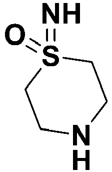 | 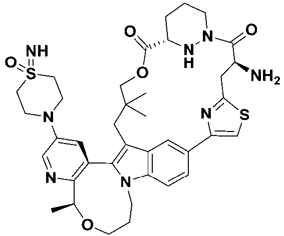 |
| **125** | 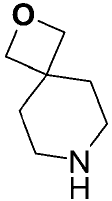 | 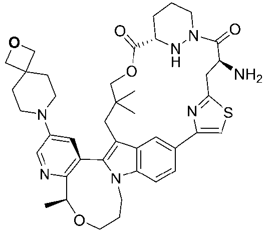 |
| **128** | 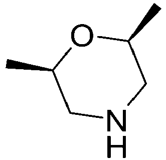 | 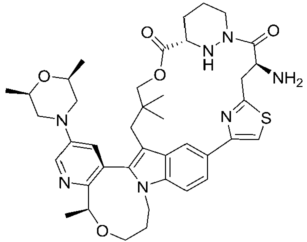 |
| **130** | 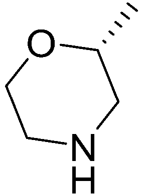 | 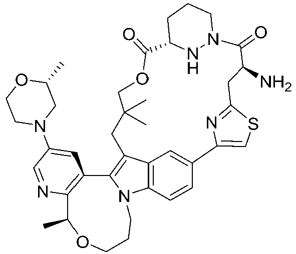 |
| **131** | 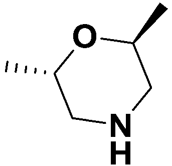 | 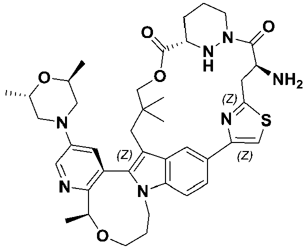 |
| **133** | 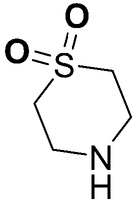 | 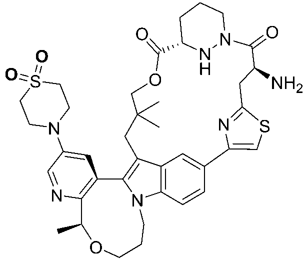 |
| **134** | 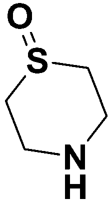 | 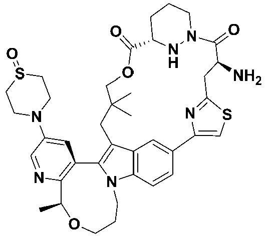 |
| **135** | 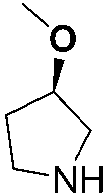 | 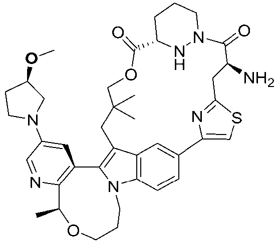 |
| **136** | 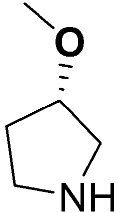 | 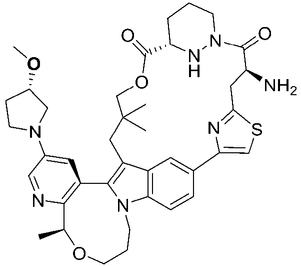 |
| **138** | 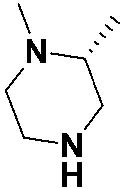 | 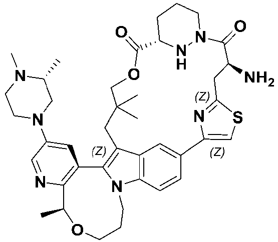 |
| **142, 172** | 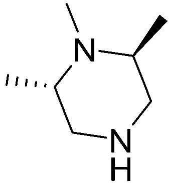 | 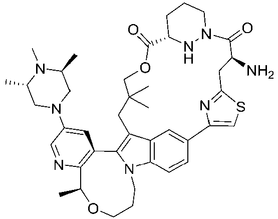 |
| **150** | 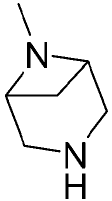 | 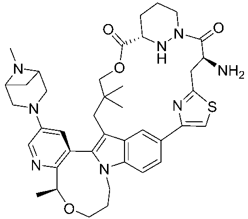 |
| **152** | 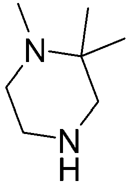 | 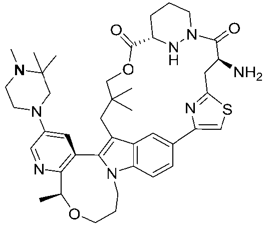 |
| **153** | 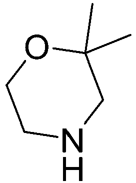 | 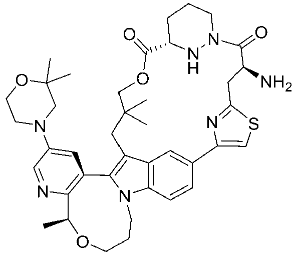 |
| **155** | 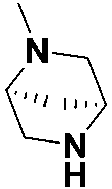 | 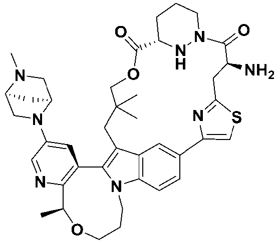 |
| **160** | 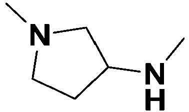 | 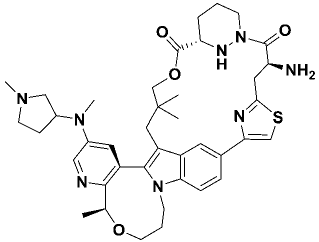 |
| **163** | 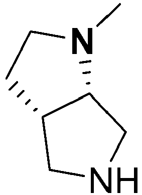 | 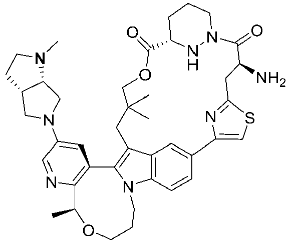 |
| **166** | 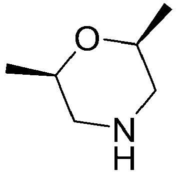 | 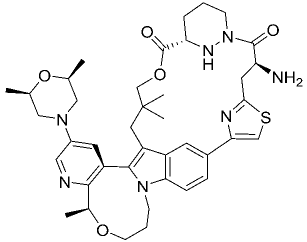 |

### Example 115: Synthesis of compound (1S,2S)-2-(difluoromethyl)-N-((1⁵S,6³S,4S,Z)-1⁵,10,10-trimethyl-1²-(3-morpholinoazetidin-1-yl) -5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxazon ino[5,6-a]indola-2(4,2)-thiazola-6(1,3)-pyridazinacycloundecaphane-4-yl)cyclopropane-1-carbox amide

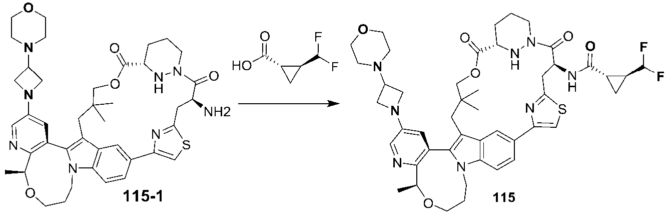

(1S,2S)-2-(difluoromethyl)cyclopropane-1-carboxylic acid (29 mg, 0.21 mmol), HATU (108 mg, 0.28 mmol), and DIEA (0.14 mL, 0.85 mmol) were added sequentially to a 10 mL single-necked flask, and the mixture was stirred at room temperature for 30 min. The mixture was cooled in an ice-water bath, and then a solution of compound 115-1 (109 mg, 0.21 mmol) in acetonitrile was added. After the reaction was complete as monitored by LC-MS, the reaction solution was directly purified by Prep-HPLC acidic method, and the eluate was lyophilized to finally obtain the target compound 115 (43 mg). ESI-MS m/z: 887.61, [M+H]⁺.

¹H NMR (500 MHz, DMSO)δ 8.89 (d, J = 9.0 Hz, 1H), 8.50 (s, 1H), 8.01 (d, J = 2.7 Hz, 1H), 7.82 (s, 1H), 7.73 (dd, J = 8.6, 1.4 Hz, 1H), 7.50 (d, J = 8.5 Hz, 1H), 6.86 (d, J = 2.7 Hz, 1H), 5.56 (t, J = 9.4 Hz, 1H), 5.12 (d, J = 12.2 Hz, 1H), 4.28 - 4.21 (m, 5H), 4.17 (d, J = 6.5 Hz, 4H), 4.07 - 3.98 (m, 4H), 3.68 (d, J = 11.1 Hz, 2H), 3.58 (dd, J = 22.8, 10.4 Hz, 3H), 3.36 (d, J = 14.4 Hz, 1H), 3.21 - 3.09 (m, 3H), 3.02 (d, J = 14.2 Hz, 2H), 2.77 (ddd, J = 12.8, 7.5, 4.5 Hz, 1H), 2.44 - 2.25 (m, 4H), 2.14 - 2.06 (m, 2H), 1.81 (s, 3H), 1.69 - 1.61 (m, 1H), 1.37 (d, J = 6.4 Hz, 3H), 1.18 (dd, J = 26.7, 19.3 Hz, 1H), 1.13 - 0.98 (m, 2H), 0.96 (s, 3H), 0.33 (s, 3H).

### Example 116: Synthesis of compound 3-methyl-N-((1⁵S6³S4S,Z)-1⁵,10,10-trimethyl-1²-morpholino-5,7-dioxo-1⁸,1⁹,6¹,6²,6³,6⁴,6⁵,6⁶-o ctahydro-1⁵H,1⁷H-8-oxa-1(13,15)-pyrido[3',2':7,8][1,5]oxazonino[5,6-a]indola-2(4,2)-thiazola-6 (1,3)-pyridazinacycloundecaphane-4-yl)bicyclo[1.1.1]pentane-1-carboxamide

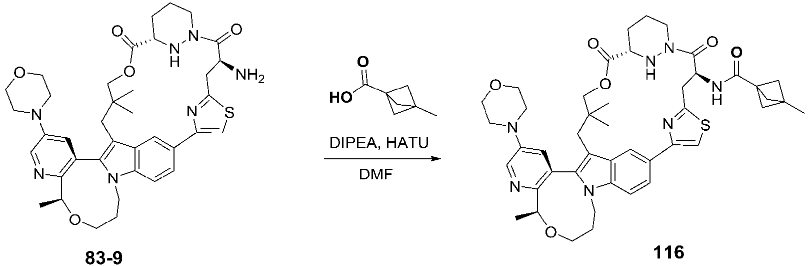

### Step 1: Synthesis of compound 116

Compound 1-methylbicyclo[1.1.1]pentane-3-carboxylic acid (10.60 mg, 0.08 mmol) was dissolved in DMF (1.00 mL), the temperature was lowered to 0 °C, then DIPEA (0.10 mL, 0.56 mmol) and HATU (42.59 mg, 0.11 mmol) were added, and the reaction was carried out at 0 °C for 5 min. Then compound 83-9 (40.00 mg, 0.06 mmol) was added, and the reaction was carried out at 0 °C for 5 min. After the reaction was complete as monitored by LC-MS, the resultant was purified by preparative chromatography (acetonitrile:water (containing 0.05% diethylamine) = 5:1) to obtain the target compound 116 (21.5 mg). ESI-MS m/z: 821.39, 822.40 [M+H]⁺

¹H NMR (500 MHz, Chloroform-d)δ 8.61 (d, J = 1.6 Hz, 1H), 8.44 (d, J = 3.0 Hz, 1H), 7.60 (dd, J = 8.5, 1.6 Hz, 1H), 7.36 - 7.27 (m, 2H), 7.10 (d, J = 3.0 Hz, 1H), 6.36 (d, J = 9.7 Hz, 1H), 5.85 (t, J = 9.0 Hz, 1H), 4.60 (d, J = 12.0 Hz, 1H), 4.40-4.33 (m, 1H), 4.27-4.20 (m, 2H), 4.03 (d, J = 12.2 Hz, 2H), 3.93-3.91 (m, 4H), 3.85 (s, 2H), 3.82-3.76 (m, 1H), 3.48 (d, J = 14.9 Hz, 1H), 3.36-3.30 (m, 1H), 3.29 - 3.18 (m, 5H), 3.15-3.09 (m, 1H), 2.74 - 2.65 (m, 1H), 2.44 (d, J = 14.4 Hz, 1H), 2.36-2.24 (m, 2H), 1.97 (s, 5H), 1.64 - 1.60 (m, 5H), 1.54 (d, J = 6.5 Hz, 3H), 1.25 (s, 3H), 0.99 (s, 3H), 0.44 (s, 3H).

### Example 117: Synthesis of compound (1S,2S)-2-(difluoromethyl)-N-((1⁵S,6³S,4S,Z)-1²-(3-(dimethylamino)piperidin-1-yl)-1⁵,10,10-tri methyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5] oxazonino[5,6-a]indola-2(4,2)-thiazola-6(1,3)-pyridazinacycloundecaphane-4-yl)cyclopropane-1 -carboxamide

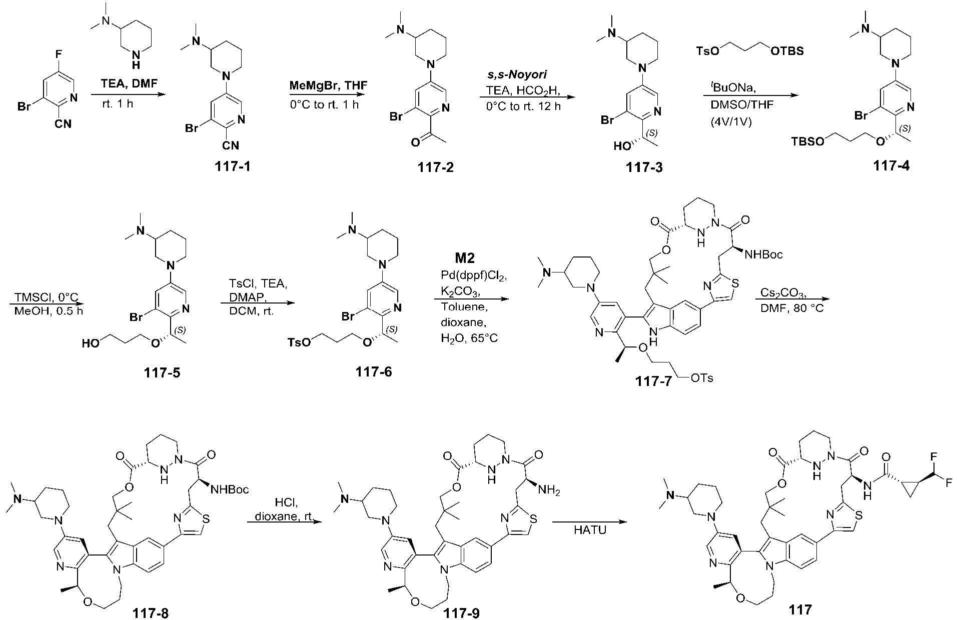

### Step 1: Synthesis of compound 117-1

The substrate 3-bromo-5-fluoropentanenitrile (1.5 g) was added to a 100 mL single-necked flask, THF (20 mL) was added, DIEA (2.67 mL) and 3-isopropylpiperidine (0.96 g) were weighed and added respectively, and the reaction was carried out at room temperature for 0.5 h. After the reaction was complete, saturated NaCl (25 mL) was added, extracted with EA, and purified by column chromatography to obtain compound 117-1 (2.07 g). ESI-MS m/z: 309.2 [M+H]⁺.

### Step 2: Synthesis of compound 117-2

Compound 117-1 (2.07 g) and anhydrous THF (21 mL) were added to a 100 mL three-necked flask, the temperature was lowered to -20 °C under N₂ protection, MeMgBr (3 M in THF, 6.69 mL) was slowly added dropwise, and the mixture was heated to room temperature and reacted for 2 h. After the reaction was complete, HCl (IN, 30 mL) was added to the reaction solution, extracted with EA, the organic phase was collected, the aqueous phase was further extracted twice with EA (30 mL), the organic phases were collected and combined, dried over anhydrous sodium sulfate, and the concentrate was purified by column chromatography to obtain the target compound 117-2 (1.58 g). ESI-MS m/z: 326.4 [M+H]⁺.

### Step 3: Synthesis of compound 117-3

Compound 117-2 (1.58 g) was added to a 25 mL single-necked flask, TEA (15 mL) and s,s-Noyori catalyst (0.31 g) were added, the temperature was lowered to 0 °C under N₂ protection, formic acid (1.11 g) was slowly added dropwise, and the reaction was carried out at room temperature for 12 h. After the reaction was complete, ethyl acetate (50 mL) and water (50 mL) were added to the reaction solution, extracted, the organic phase was collected, the aqueous phase was further extracted twice with ethyl acetate (25 mL), the organic phases were collected and combined, dried over anhydrous sodium sulfate, concentrated, and the concentrate was purified by column chromatography to obtain the target axially chiral compound 117-3 (0.95 g). ESI-MS m/z: 328.3 [M+H]⁺.

### Step 4: Synthesis of compound 117-4

Compound 117-3 (0.95 g), 3-((tert-butyldimethylsilyl)oxy)propyl-4-methylbenzenesulfonate (2.87 g) and DMSO/THF (10 mL/2.5 mL) were weighed and added into the reaction flask respectively, and the temperature was lowered to 10 °C under N₂ protection; sodium tert-butoxide (0.67 g) was added, and the reaction was carried out at room temperature for 1.0 h; the reaction was complete, the temperature was lowered to 0 °C, tap water (50 mL) was added for dilution, extracted with EA. The organic phase was dried over anhydrous sodium sulfate, concentrated, and the concentrate was purified by column chromatography to obtain compound 117-4 (1.03 g). ESI-MS m/z: 500.1 [M+H]⁺.

### Step 5: Synthesis of compound 117-5

Compound 117-4 (1.03 g) was added to a 50 mL single-necked flask, anhydrous methanol (20 mL) was added, the temperature was lowered to 0 °C under N₂ protection, TMSCl (0.2 mL) was slowly added dropwise, and the reaction was carried out at room temperature for 1.0 h. After the reaction was complete, it was directly concentrated to dryness to obtain the target compound 117-5 (0.84 g). ESI-MS m/z: 385.3 [M+H]⁺.

### Step 6: Synthesis of compound 117-6

Compound 117-5 (1.03 g) was added to a 50 mL single-necked flask, anhydrous DCM (15 mL) was added, DIEA (1.08 mL) and DMAP (0.8 g) were weighed and added respectively, the temperature was lowered to 0 °C under N₂ protection, TsCl (0.83 g) was added, and the reaction was carried out at room temperature for 1.0 h. After the reaction was complete, saturated NaCl (25 mL) was added, extracted with DCM, the organic phase was concentrated to dryness, and the concentrate was purified by column chromatography to obtain the target compound 117-6 (0.61 g). ESI-MS m/z: 540.5 [M+H]⁺.

### Step 7: Synthesis of compound 117-7

Compound 117-6 (94 mg), M2 (100 mg), Pd(dppf)Cl₂ (11 mg), and potassium carbonate (50 mg) were weighed and added respectively to a 100 mL single-necked flask, toluene (1.5 mL), 1,4-dioxane (0.5 mL) and 0.5 mL of water were added, N₂ was purged 3 times, and the mixture was heated to 65 °C and reacted for 5 h. After the reaction was complete, 20 mL of EA and 20 mL or water were added to the reaction solution, extracted, the aqueous phase was further extracted twice with EA, the organic phases were collected and combined, dried over anhydrous sodium sulfate, concentrated, and the concentrate was purified by column chromatography to obtain the target compound 117-7 (61 mg). ESI-MS m/z: 514.6 [M+H]⁺.

### Step 8: Synthesis of compound 117-8

Compound 117-7 (61 mg) was added to a 25 mL single-necked flask, Cs₂CO₃ (58 mg) and anhydrous DMF (6 mL) were weighed in, and the reaction was carried out at 80 C for 1 h under N₂ protection. After the reaction was complete, tap water (50 mL) was added for dilution, extracted with EA, the organic phase was dried over anhydrous sodium sulfate, concentrated, and the concentrate was purified by column chromatography to obtain the target compound 117-8 (30 mg). ESI-MS m/z: 854.3 [M+H]⁺.

### Step 9: Synthesis of compound 117-9

Compound 117-8 (30 mg) was added to a 25 mL single-necked flask, 15 mL of hydrochloric acid (4 M solution in dioxane) was added, and the reaction was carried out at room temperature for 1 h. After the reaction was complete, it was concentrated under reduced pressure to dryness to obtain the target compound 117-9 hydrochloride (30 mg). ESI-MS m/z: 754.2 [M+H]⁺.

### Step 10: Synthesis of compound 117

Compound (1S,2S)-2-(difluoromethyl)cyclopropane-1-carboxylic acid (6.4 mg) was added to a 10 mL single-necked flask, HATU (30 mg), N,N-diisopropylethylamine (0.04 mL) and anhydrous acetonitrile (2 mL) were weighed in, and the reaction was carried out at room temperature for 10 min; a pre-liberated acetonitrile solution (4 mL) of 117-9 (30 mg) was added dropwise at room temperature, and the reaction was carried out at room temperature for 15 min. After the reaction was complete as monitored by LC-MS, the reaction solution was directly purified by Prep-HPLC acidic method, and the eluate was lyophilized to finally obtain the target compound 117 (12.9 mg). ESI-MS m/z: 873.4 [M+H]⁺.

¹H NMR (500 MHz, DMSO)δ 8.90 (d, J = 9.1 Hz, 1H), 8.50 (s, 1H), 8.40 (dd, J = 5.8, 2.8 Hz, 1H), 8.19 (s, 1H), 7.81 (s, 1H), 7.71 (d, J = 7.4 Hz, 1H), 7.50 (d, J = 8.6 Hz, 1H), 7.20 (d, J = 2.5 Hz, 1H), 5.95 (td, J = 56.6, 5.0 Hz, 1H), 5.56 (t, J = 9.2 Hz, 1H), 5.12 (d, J = 12.0 Hz, 1H), 4.23 (t, J = 11.8 Hz, 2H), 4.13 (d, J = 4.8 Hz, 1H), 4.03 (d, J = 9.7 Hz, 2H), 3.95 - 3.79 (m, 2H), 3.63 (dt, J = 18.2, 10.7 Hz, 4H), 3.14 (dd, J = 14.7, 9.3 Hz, 2H), 3.01 (d, J = 14.0 Hz, 1H), 2.83 - 2.67 (m, 3H), 2.44 - 2.28 (m, 3H), 2.26 (d, J = 2.1 Hz, 6H), 2.14 - 2.05 (m, 2H), 1.92 (s, 1H), 1.81 (s, 2H), 1.65 (dd, J = 9.4, 4.5 Hz, 1H), 1.58 - 1.46 (m, 2H), 1.37 (d, J = 6.2 Hz, 3H), 1.24 (s, 1H), 1.08 - 0.99 (m, 2H), 0.95 (d, J = 3.0 Hz, 3H), 0.32 (s, 3H).

### Example 118: Synthesis of compound (1S,2S)-2-methyl-N-((1⁵S,6³S,4S,Z)-1⁵,10,10-trimethyl-1²-morpholino-5,7-dioxo-1⁸,1⁹,6¹,6²,6³,6 ⁴,6⁵,6⁶-octahydro-1⁵H,1⁷H-8-oxa-1(13,15)-pyrido[3',2':7,8][1,5]oxazonino[5,6-a]indola-2(4,2)-th iazola-6(1,3)-pyridazinacycloundecaphane-4-yl)cyclopropane-1-carboxamide

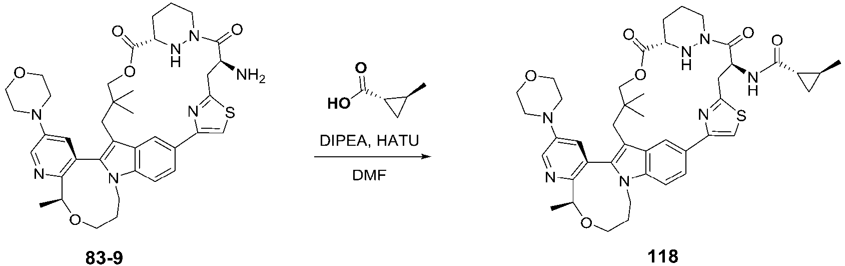

### Step 1: Synthesis of compound 118

Compound (1S,2S)-2-methylcyclopropane-1-carboxylic acid (8.41 mg, 0.08 mmol) was dissolved in DMF (1.00 mL), the temperature was lowered to 0 °C, then DIPEA (0.10 mL, 0.56 mmol) and HATU (42.59 mg, 0.11 mmol) were added, and the reaction was carried out at 0 °C for 5 min. Then compound 83-9 (40.00 mg, 0.06 mmol) was added, and the reaction was carried out at 0 °C for 5 min. After the reaction was complete as monitored by LC-MS, purification by preparative chromatography (acetonitrile:water (containing 0.05% diethylamine) = 5:1) yielded the target compound 118 (22.3 mg). ESI-MS m/z: 795.38, 796.36 [M+H]⁺

¹H NMR (500 MHz, Chloroform-d)δ 8.59 (d, J = 1.7 Hz, 1H), 8.42 (d, J = 2.9 Hz, 1H), 7.58 (dd, J = 8.5, 1.7 Hz, 1H), 7.34 - 7.27 (m, 3H), 7.08 (d, J = 3.0 Hz, 1H), 6.44 (d, J = 9.6 Hz, 1H), 5.90 (t, J = 9.0 Hz, 1H), 4.60 (d, J = 12.2 Hz, 1H), 4.35 (td, J = 12.1, 3.3 Hz, 1H), 4.23 (dq, J = 17.7, 6.1 Hz, 2H), 4.05 - 3.96 (m, 2H), 3.90 (d, J = 3.4 Hz, 2H), 3.89 (d, J = 3.8 Hz, 2H), 3.87 - 3.73 (m, 3H), 3.45 (d, J = 14.9 Hz, 1H), 3.36 - 3.27 (m, 1H), 3.25 (s, 1H), 3.24 (s, 3H), 3.25 - 3.16 (m, 2H), 3.12 (dd, J = 14.9, 8.6 Hz, 1H), 2.68 (td, J = 12.8, 3.0 Hz, 1H), 2.41 (d, J = 14.4 Hz, 1H), 2.28 (dt, J = 36.0, 10.0 Hz, 2H), 1.97 (d, J = 14.3 Hz, 2H), 1.52 (d, J = 6.5 Hz, 3H), 1.34 (s, 1H), 1.26 (s, 2H), 1.29 - 1.17 (m, 2H), 1.10 (d, J = 6.0 Hz, 3H), 0.97 (s, 3H), 0.68 - 0.61 (m, 1H), 0.41 (s, 3H).

### Example 120: Synthesis of compound (1S,2S)-2-(difluoromethyl)-N-((1⁵S,6³S,4S,Z)-1²-(3-(dimethylamino )pyrrolidin-1-yl)-1⁵,10,10-tr imethyl-5,7-dioxo-1⁸,1⁹,6¹,6²6³,6⁴,6⁵,6⁶-octahydro-1⁵H,1⁷H-8-oxa-1(13,15)-pyrido[3',2':7,8][1,5 ]oxazonino[5,6-a]indola-2(4,2)-thiazola-6(1,3)-pyridazinacycloundecaphane-4-yl)cyclopropane-1-carboxamide

CN

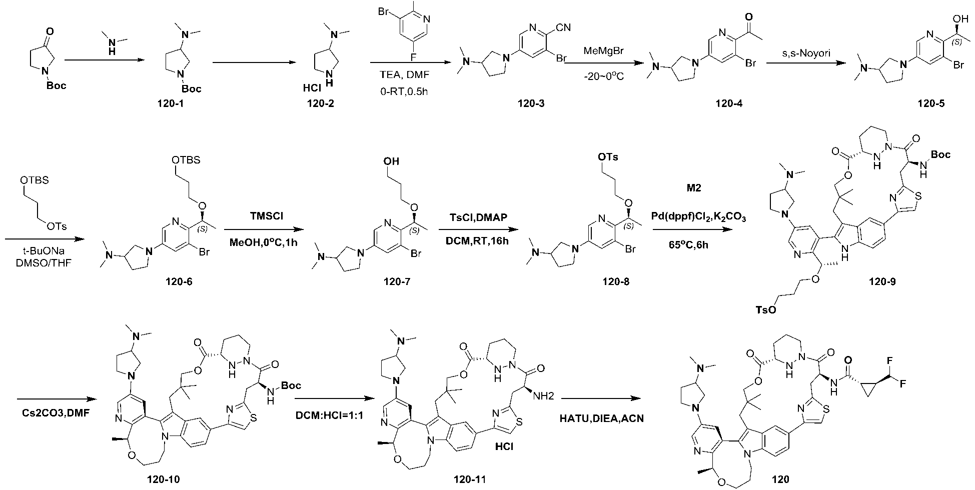

### Step 1: Synthesis of compound 120-1

1-Tert-butoxycarbonyl-3-pyrrolidinone (5 g, 26.99 mmol), N-methylmethylamine (20.25 mL, 40.49 mmol), and 1,2-dichloroethane (50.00 mL) were sequentially added to a 100 mL single-necked flask. At 0 °C, acetic acid (0.15 mL, 2.70 mmol) was added, and the mixture was kept stirring for 10 min. Sodium triacetoxyborohydride (17.16 g, 80.98 mmol) was added in batches, and after the addition was complete, the mixture was transferred to room temperature and stirred for 16 h. After the reaction was complete as monitored by LC-MS, the mixture was basified with 1M aqueous NaOH solution, extracted with ethyl acetate, washed sequentially with water and brine, separated, and the organic phase was concentrated to obtain a crude product, which was purified by silica gel column chromatography to finally obtain the target compound 120-1 (5.7 g, off-white solid powder). ESI-MS m/z: 215 [M+H]⁺.

### Step 2: Synthesis of compound 120-2

Compound 120-1 (5.7 g, 26.6 mmol), dichloromethane (60.00 mL), and hydrochloric acid (solution in dioxane, 4M) (96.00 mL) were added sequentially to a 100 mL single-necked flask, and the reaction was carried out at 25 °C for 3 h. After the reaction was complete as monitored by LC-MS, the reaction solution was concentrated to dryness, and evaporated twice with dichloromethane to obtain a crude product of the target compound 120-2 (3.99 g, off-white solid). It was directly used in the next step without purification. ESI-MS m/z: 115 [M+H]⁺.

### Step 3: Synthesis of compound 120-3

3-Bromo-5-fluoro-pyridine-2-carbonitrile (2 g, 9.95 mmol) and N,N-dimethylformamide (20.00 mL) were added sequentially to a 100 mL single-necked flask. At 0 °C, compound 120-2 (1.49 g, 9.95 mmol) and triethylamine (4.15 mL, 29.85 mmol) were added dropwise. After the addition was complete, the mixture was transferred to room temperature and stirred for 0.5 h. After the reaction was complete as monitored by LC-MS, the mixture was extracted three times with water and ethyl acetate. The organic phases were combined, washed sequentially with water and brine, separated, and concentrated to obtain a crude product, which was purified by silica gel column chromatography to finally obtain the target product 120-3 (1.3 g, off-white solid). ESI-MS m/z: 295, 297 [M+H]⁺.

### Step 4: Synthesis of compound 120-4

Compound 120-3 (1.3 g, 4.4 mmol) and THF (15 mL) were added sequentially to a 50 mL three-necked flask. Nitrogen was replaced three times, the temperature was lowered to -20 °C, methylmagnesium bromide (4.11 mL, 3.00 mol/L, 12.33 mmol) was added dropwise. After the addition was complete, the mixture was transferred to 0 C and stirred continuously for 2 h. After the reaction was complete as monitored by LC-MS, 1M dilute hydrochloric acid was added dropwise to adjust the pH to about 3-4; the mixture was stirred at room temperature for 15 min, basified with saturated aqueous sodium bicarbonate solution, extracted with ethyl acetate, washed with brine, separated, and the organic phase was concentrated to obtain a crude product, which was purified by silica gel column chromatography to finally obtain the target compound 120-4 (0.98 g, reddish-brown mucus). ESI-MS m/z: 312, 314 [M+H]⁺.

### Step 5: Synthesis of compound 120-5

Triethylamine (5.24 mL, 37.67 mmol) was added sequentially to a 500 mL single-necked flask. At 0 °C, formic acid (0.3 mL, 7.85 mmol) was added, then (S,S)-N-(p-toluenesulfonyl)-1,2-diphenylethane-1,2-diamine (p-cymene)ruthenium(II) chloride (0.02 g, 0.03 mmol) was added, and the mixture was stirred at 40 °C for 15 min. After cooling to room temperature, compound 120-4 (0.98 g, 3.14 mmol) was added, and the reaction was carried out at room temperature (oil temperature 28 °C) for 20 h. After the reaction was complete as monitored by LC-MS, the reaction mixture was added to an aqueous solution, extracted with ethyl acetate, washed with brine, separated, and the organic phase was concentrated to obtain a crude product, which was purified by silica gel column chromatography to finally obtain the target compound 120-5 (0.49 g, reddish-brown mucus). ESI-MS m/z: 314, 316 [M+H]⁺.

### Step 6: Synthesis of compound 120-6

Compound 120-5 (490.00 mg, 1.56 mmol) and 3-((tert-butyldimethylsilyl)oxy)propyl-4-methylbenzenesulfonate (1070 mg, 3.12 mmol) were added sequentially to a 50 mL single-necked flask, dissolved in DMSO (5.00 mL). Under ice-water cooling, a solution of sodium tert-butoxide (370 mg, 3.9 mmol) in tetrahydrofuran (1.00 mL) was added, and the mixture was transferred to room temperature and stirred for 0.5 h. After the reaction was complete as monitored by LC-MS, the reaction mixture was added to an aqueous solution, extracted with dichloromethane, washed with brine, separated, and the organic phase was concentrated to obtain a crude product, which was purified by silica gel column chromatography to finally obtain the target compound 120-6 (480 mg, reddish-brown mucus). ESI-MS m/z: 486, 488 [M+H]⁺.

### Step 7: Synthesis of compound 120-7

Compound 120-6 (380 mg, 0.78 mmol), tetrahydrofuran (5.00 mL), and TBAF (0.97 mL, 1M solution in THF) were added to a 50 mL single-necked flask, and the mixture was stirred at 50 °C for 1 h. After the reaction was complete as monitored by LC-MS, silica gel was added for sample preparation, followed by purification by silica gel column chromatography to finally obtain the target compound 120-7 (360 mg, reddish-brown mucus). ESI-MS m/z: 372, 374 [M+H]⁺.

### Step 8: Synthesis of compound 120-8

Compound 120-7 (360 mg, 0.7 mmol), 4-dimethylaminopyridine (11.81 mg, 0.1 mmol), dichloromethane (3.00 mL), and triethylamine (0.34 mL, 2.42 mmol) were added sequentially to a 50 mL single-necked flask. At 0 °C under nitrogen protection, p-toluenesulfonyl chloride (368.71 mg, 1.93 mmol) was added. After the addition was complete, the mixture was transferred to room temperature and stirred overnight. After the reaction was complete as monitored by LC-MS, the reaction mixture was added to an aqueous solution, extracted with dichloromethane, washed with brine, separated, and the organic phase was concentrated to obtain a crude product, which was purified by silica gel column chromatography to finally obtain the target compound 120-8 (210 mg, reddish-brown mucus). ESI-MS m/z: 526, 528 [M+H]⁺.

### Step 9: Synthesis of compound 120-9

Compound 120-8 (120 mg, 0.23 mmol), compound M2 (158 mg, 0.18 mmol), potassium carbonate (78.75 mg, 0.57 mmol), [PdCl₂(dppf)]CH₂Cl₂ (18.62 mg, 0.02 mmol), toluene (3.00 mL), 1,4-dioxane (1.00 mL), and water (1.00 mL) were added sequentially. Nitrogen was replaced three times, and the mixture was stirred at 60-65 °C for 1.5 h. After the reaction was complete as monitored by LC-MS, the reaction solution was diluted with ethyl acetate, washed sequentially with water and brine, separated, the organic phases were combined, and concentrated to obtain a crude product, which was purified by silica gel column chromatography to finally obtain the target compound 120-9 (122 mg, reddish-brown mucus). ESI-MS m/z: 507 [M+2H]²⁺/2.

### Step 10: Synthesis of compound 120-10

Compound 120-9 (122 mg, 0.12 mmol), DMF (3 mL), and Cs₂CO₃ (78.46 mg, 0.24 mmol) were added sequentially to a 10 mL single-necked flask. Under nitrogen protection, the mixture was heated in an oil bath and kept at 60 °C to 65 °C for reaction for 2 h. After the reaction was complete as monitored by LC-MS, the reaction solution was diluted with ethyl acetate, washed sequentially with water and brine, separated, and the organic phase was concentrated to obtain a crude product of compound 120-10, which was purified by silica gel column chromatography to finally obtain the target compound 120-10 (69 mg, reddish-brown mucus). ESI-MS m/z: 841 [M+H]⁺.

### Step 11: Synthesis of compound 120-11

Compound 120-10 (69 mg, 0.08 mmol), DCM (2.00 mL), and HCl (solution in dioxane, 4M) (2.00 mL) were added sequentially to a 10 mL single-necked flask, and the mixture was stirred and reacted at room temperature for 0.5 h. After the reaction was complete as monitored by LC-MS, the reaction solution was concentrated to dryness, DCM was added again, and the mixture was concentrated to dryness again. A crude product of compound 120-11 (82 mg, hydrochloride salt) was obtained as a slightly yellow solid powder, which was directly used in the next reaction. ESI-MS m/z: 741 [M+H]⁺.

### Step 12: Synthesis of compound 120

2-(difluoromethyl)cyclopropane-1-carboxylic acid (72 mg, 0.55 mmol), HATU (301 mg, 0.79 mmol), acetonitrile (3.00 mL), and N,N-diisopropylethylamine (0.17 mL, 1.06 mmol) were added sequentially to a 10 mL single-necked flask. After stirring at 0 °C for 15 min. A solution of compound 120-11 (82.00 mg, 0.11 mmol) in acetonitrile (3.00 mL) (DIEA was added dropwise until dissolved) was added, and the mixture was stirred continuously at 0 °C for 15 min. After the reaction was complete as monitored by LC-MS, the reaction solution was directly purified by Prep-HPLC acidic method, and the eluate was lyophilized to finally obtain the target compound 120 (7.6 mg). ESI-MS m/z: 859.41 [M+H]+.

¹H NMR (500 MHz, MeOD)δ 8.56 (d, J = 1.7 Hz, 1H), 8.10 (d, J = 2.8 Hz, 1H), 7.68 (dd, J = 8.6, 1.7 Hz, 1H), 7.56 (s, 1H), 7.46-7.43 (m, 1H), 6.99 (t, J = 2.8 Hz, 1H), 5.90-5.67 (m, 2H), 4.59 (s, 1H), 4.43 (d, J = 12.8 Hz, 1H), 4.30-4.26 (m, 2H), 4.18 - 4.02 (m, 2H), 3.95 (s, 1H), 3.84 - 3.73 (m, 2H), 3.76 - 3.66 (m, 2H), 3.49 - 3.40 (m, 1H), 3.24-3.16 (m, 2H), 2.93-2.90 (m, 7H), 2.83 - 2.74 (m, 1H), 2.60 - 2.50 (m, 2H), 2.40-2.33 (m, 1H), 2.28-2.20 (m, 3H), 2.09-2.04 (m, 1H), 1.98 - 1.76 (m, 3H), 1.65-1.57 (m, 1H), 1.48 (d, J = 6.5 Hz, 3H), 1.35 - 1.27 (m, 1H), 1.21 - 1.09 (m, 3H), 1.00 (d, J = 2.7 Hz, 3H), 0.42 (s, 3H).

### Example 123: Synthesis of compound (1S,2S)-2-(difluoromethyl)-N-(((1⁵S,6³S,4S,Z)-1²-(1-imino-1-oxido-1λ⁶-thiomorpholino)-1⁵,10,1 0-trimethyl-5,7-dioxo-1⁸, 1⁹,6¹,6²,6³,6⁴,6⁵,6⁶-octahydro-1⁵H,1⁷H-8-oxa-1(13, 15)-pyrido[3',2':7,8][ 1,5]oxazonino[5,6-a]indola-2(4,2)-thiazola-6(1,3)-pyridazinacycloundecaphane-4-yl)cyclopropa ne-1-carboxamide

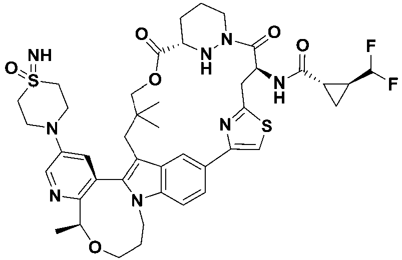

ESI-MS m/z: 879.5 [M+H]⁺.

¹H NMR (500 MHz, MeOD)δ 8.59 (s, 1H), 8.44 (d, J = 2.8 Hz, 1H), 7.59 (d, J = 7.6 Hz, 1H), 7.32 (d, J = 8.6 Hz, 1H), 7.29 (s, 1H), 7.14 (d, J = 2.9 Hz, 1H), 6.70 (d, J = 9.5 Hz, 1H), 5.90 (t, J = 8.9 Hz, 1H), 5.84 (td, J = 57.5, 2.9 Hz, 1H), 4.60 (d, J = 12.0 Hz, 1H), 4.36 (td, J = 12.1, 3.0 Hz, 1H), 4.28 - 4.19 (m, 2H), 4.05 - 3.98 (m, 2H), 3.96 - 3.73 (m, 7H), 3.47 (d, J = 14.8 Hz, 1H), 3.32 (td, J = 12.1, 2.9 Hz, 1H), 3.24 - 3.11 (m, 6H), 2.74 - 2.60 (m, 2H), 2.39 - 2.20 (m, 3H), 2.03 - 1.79 (m, 6H), 1.52 (m, 3H), 1.15 (dt, J = 10.6, 5.5 Hz, 1H), 0.98 (s, 3H), 0.88 (t, J = 6.8 Hz, 1H), 0.41 (s, 3H).

### Example 124: Synthesis of compound (1S,4r)-N-((1⁵S,6³S,4S,Z)-1²-(4-ethylpiperazin-1-yl)-1⁵,10,10-trimethyl-5,7-dioxo-1⁸,1⁹,6¹,6²,6³, 6⁴,6⁵,6⁶-octahydro-1⁵H,1⁷H-8-oxa-1(13,15)-pyrido[3',2':7,8][1,5]oxazonino[5,6-a]indola-2(4,2)-t hiazola-6(1,3)-pyridazinacycloundecaphane-4-yl)-1-methyl-2-oxabicyclo[2.1.1]hexane-4-carbox amide

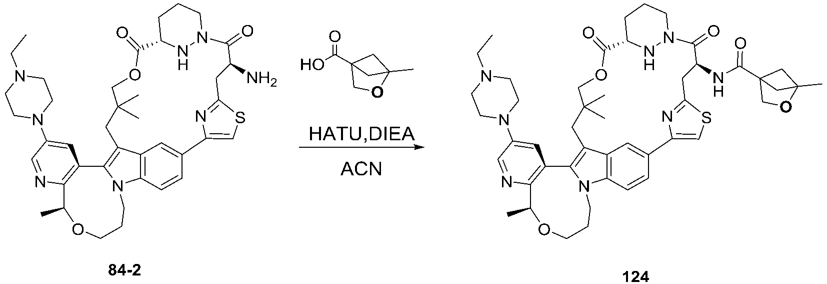

### Step 1: Synthesis of compound 124

1-Methyl-2-oxabicyclo[2.1.1]hexane-4-carboxylic acid (11.51 mg, 0.08 mmol) and acetonitrile (2.00 mL) were added sequentially to a 10 mL single-necked flask. At 0 °C, N,N,N',N'-tetramethyl-O-(7-azabenzotriazol-1-yl)uranium hexafluorophosphate (30.80 mg, 0.08 mmol) and N,N-diisopropylethylamine (0.09 mL, 0.54 mmol) were added, and the mixture was stirred at 0 °C for 10 min. Finally, compound 84-2 (40.00 mg, 0.05 mmol) was added, and the mixture was stirred at room temperature for 30 min. The reaction was complete as monitored by LC-MS, and the reaction solution was directly purified by pre-HPLC to obtain the target compound 124 (15.4 mg). ESI-MS m/z: 865.9 [M+H]⁺.

¹H NMR (500 MHz, CDCl₃)δ 8.59 (s, 1H), 8.43 (d, J = 2.8 Hz, 1H), 7.60 - 7.55 (m, 1H), 7.31 (d, J = 8.6 Hz, 1H), 7.28 (s, 1H), 7.09 (d, J = 2.8 Hz, 1H), 6.45 (d, J = 9.6 Hz, 1H), 5.90 (t, J = 9.0 Hz, 1H), 4.57 (d, J = 11.9 Hz, 1H), 4.36 (td, J = 12.0, 3.0 Hz, 1H), 4.26 - 4.17 (m, 2H), 4.04 - 3.97 (m, 4H), 3.85 - 3.75 (m, 3H), 3.48 (d, J = 14.9 Hz, 1H), 3.30 (d, J = 12.7 Hz, 5H), 3.19 (d, J = 14.4 Hz, 1H), 3.11 (dd, J = 15.0, 8.6 Hz, 1H), 2.74 - 2.62 (m, 4H), 2.56 - 2.47 (m, 2H), 2.42 (d, J = 14.4 Hz, 1H), 2.36 - 2.19 (m, 2H), 1.96 (dtd, J = 17.6, 9.8, 6.0 Hz, 6H), 1.87 - 1.78 (m, 1H), 1.65 - 1.59 (m, 1H), 1.51 (d, J = 6.4 Hz, 3H), 1.49 (s, 3H), 1.15 (t, J = 7.1 Hz, 3H), 0.96 (s, 3H), 0.88 (t, J = 6.8 Hz, 1H), 0.40 (s, 3H).

### Example 125: Synthesis of compound (1S,2S)-2-(difluoromethyl)-N-((1⁵S,6³S,4SZ)-1⁵,10,10-trimethyl-5,7-dioxo-1²-(2-oxa-7-azaspiro [3.5]nonan-7-yl)-1⁸,1⁹,6¹,6²,6³,6⁴,6⁵,6⁶-octahydro-1⁵H,1⁷H-8-oxa-1(13,15)-pyrido[3',2':7,8][1,5]o xazonino[5,6-a]indola-2(4,2)-thiazola-6(1,3)-pyridazinacycloundecaphane-4-yl)cyclopropane-1-carboxamide

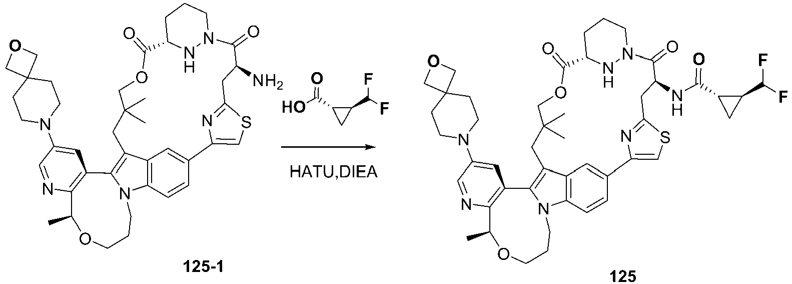

### Step 1: Synthesis of compound 125

(1S,2S)-2-(difluoromethyl)cyclopropane-1-carboxylic acid (23.83 mg, 0.18 mmol) and N,N-dimethylformamide (1.50 mL) were added sequentially to a 10 mL single-necked flask. At 0 °C, N,N,N',N'-tetramethyl-O-(7-azabenzotriazol-1-yl)uronium hexafluorophosphate (66.58 mg, 0.18 mmol) and N,N-diisopropylethylamine (0.15 mL, 0.93 mmol) were added, and the mixture was stirred at 0 °C for 10 min. Finally, compound 125-1 (88.00 mg, 0.12 mmol) was added, and the mixture was stirred at 0 °C for 10 min. The reaction was complete as monitored by LC-MS, and the reaction solution was directly purified by pre-HPLC to obtain the target product 125 (46.6 mg). ESI-MS m/z: 872.4 [M+H]⁺.

¹H NMR (500 MHz, Chloroform-d)δ 8.58 (d, J = 1.4 Hz, 1H), 8.42 (dd, J = 3.0, 1.1 Hz, 1H), 7.57 (dt, J = 8.6, 1.3 Hz, 1H), 7.32 - 7.28 (m, 2H), 7.09 (dd, J = 2.9, 1.0 Hz, 1H), 6.72 (d, J = 9.5 Hz, 1H), 5.97 - 5.70 (m, 2H), 4.60 (d, J = 13.2 Hz, 1H), 4.49 (d, J = 1.1 Hz, 4H), 4.36 (td, J = 12.1, 3.4 Hz, 1H), 4.21 (dq, J = 12.3, 6.1 Hz, 2H), 4.06 - 3.96 (m, 2H), 3.85 - 3.74 (m, 3H), 3.47 (d, J = 15.0 Hz, 1H), 3.31 (td, J = 12.4, 3.2 Hz, 1H), 3.23 - 3.12 (m, 6H), 2.69 (td, J = 12.8, 3.0 Hz, 1H), 2.41 (d, J = 14.4 Hz, 1H), 2.34 - 2.22 (m, 2H), 2.03 (t, J = 5.6 Hz, 4H), 1.98 (d, J = 13.9 Hz, 2H), 1.83 (dt, J = 9.3, 4.8 Hz, 2H), 1.61 (dd, J = 12.8, 3.9 Hz, 1H), 1.51 (d, J = 6.4 Hz, 3H), 1.36 - 1.28 (m, 2H), 1.15 (dt, J = 10.2, 5.5 Hz, 1H), 0.97 (s, 3H), 0.40 (s, 3H).

### Example 128: Synthesis of compound (1S,2S)-2-(difluoromethyl)-N-((1⁵S,6³S,4S,Z)-1²-((2S,6R)-2,6-dimethylmorpholino)-1⁵,0,10-tri methyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5] oxazonino[5,6-a]indola-2(4,2)-thiazola-6(1,3)-pyridazinacycloundecaphane-4-yl)cyclopropane-1 -carboxamide

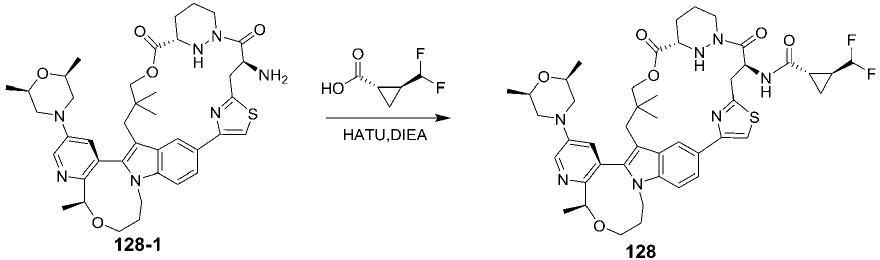

### Step 1: Synthesis of compound 128

(1S,2S)-2-(difluoromethyl)cyclopropane-1-carboxylic acid (55.05 mg, 0.40 mmol) and acetonitrile (5.00 mL) were added sequentially to a 10 mL single-necked flask. At 0 °C, N,N,N',N'-tetramethyl-O-(7-azabenzotriazol-1-yl)uronium hexafluorophosphate (184.53 mg, 0.49 mmol) and N,N-diisopropylethylamine (0.27 mL, 1.62 mmol) were added, and the mixture was stirred at 0 °C for 10 min. Finally, compound 128-1 (200.00 mg, 0.27 mmol) was added, and the mixture was stirred at 0 °C for 10 min. The reaction was complete as monitored by LC-MS, and the reaction solution was directly purified by pre-HPLC to obtain the target compound 128 (50.30 mg). ESI-MS m/z: 860.4 [M+H]⁺.

¹H NMR (500 MHz, CDCl₃)δ 8.58 (s, 1H), 8.41 (d, J = 2.7 Hz, 1H), 7.58 (dd, J = 8.4, 1.4 Hz, 1H), 7.32 (d, J = 8.6 Hz, 1H), 7.29 (s, 1H), 7.11 (d, J = 2.7 Hz, 1H), 6.74 (d, J = 9.7 Hz, 1H), 5.90 (t, J = 9.0 Hz, 1H), 5.83 (td, J = 57.6, 7.0 Hz, 1H), 4.59 (d, J = 12.8 Hz, 1H), 4.35 (td, J = 12.1, 3.1 Hz, 1H), 4.26 (q, J = 6.5 Hz, 1H), 4.21 (dd, J = 12.2, 5.7 Hz, 1H), 4.07 - 3.96 (m, 2H), 3.83 (s, 4H), 3.79 - 3.72 (m, 1H), 3.52 (d, J = 11 Hz, 2H), 3.46 (d, J = 15 Hz, 1H), 3.31 (td, J = 12.2, 2.8 Hz, 1H), 3.22 (d, J = 14.4 Hz, 1H), 3.15 (dd, J = 14.9, 8.5 Hz, 1H), 2.69 (td, J = 12.7, 2.5 Hz, 1H), 2.53 (dd, J = 21.8, 10.5 Hz, 2H), 2.41 (d, J = 14.3 Hz, 1H), 2.36 - 2.29 (m, 1H), 2.29 - 2.24 (m, 1H), 2.02 - 1.92 (m, 3H), 1.86 - 1.80 (m, 2H), 1.61 (qd, J = 12.8, 3.7 Hz, 1H), 1.53 (d, J = 6.5 Hz, 3H), 1.34 - 1.30 (m, 1H), 1.28 (t, J = 6.3 Hz, 6H), 1.18 - 1.13 (m, 1H), 0.99 (s, 3H), 0.42 (s, 3H).

### Example 130: Synthesis of compound (1S,2S)-2-(difluoromethyl)-N-((1⁵S,6³S,4S,Z)-1⁵,10,10-trimethyl-1²-((R)-2-methylmorpholino)-5,7-dioxo-1⁸,1⁹,6¹,6²,6³,6⁴,6⁵,6⁶-octahydro-1⁵H,⁷H-8-oxa-1(13,15)-pyrido[3',2':7,8][1,5]oxazoni no[5,6-a]indola-2(4,2)-thiazola-6(1,3)-pyridazinacycloundecaphane-4-yl)cyclopropane-1-carbox amide

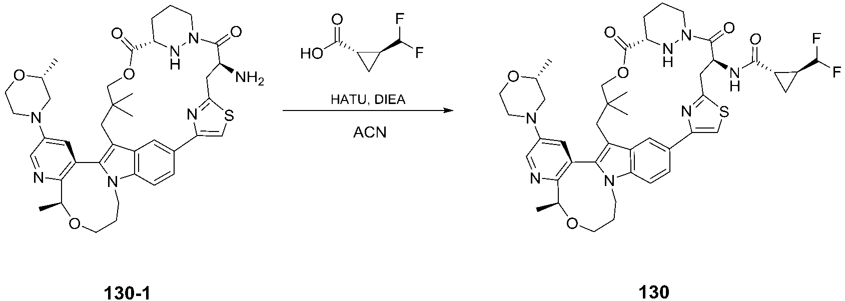

### Step 1: Synthesis of compound 130

(1S,2S)-2-(difluoromethyl)cyclopropane-1-carboxylic acid (29.53 mg, 0.22 mmol), HATU (82.51 mg, 0.22 mmol), DIEA (0.09 mL, 0.54 mmol), and acetonitrile (5.00 mL) were added sequentially to a 10 mL single-necked flask, and the mixture was stirred at room temperature for 15 min. The mixture was cooled in an ice-water bath, and then a solution of compound 130-1 (79.00 mg, 0.11 mmol) in acetonitrile was added. After the reaction was complete as monitored by LC-MS, the reaction solution was directly purified by Prep-HPLC basic method, and the eluate was lyophilized to finally obtain the target compound 130 (55.40 mg). ESI-MS m/z: 846.21, [M+H]⁺.

¹H NMR (500 MHz, Chloroform-d)δ 8.58 (d, J = 1.7 Hz, 1H), 8.41 (d, J = 2.9 Hz, 1H), 7.58 (m, 1H), 7.35-7.28 (m, 2H), 7.06 (d, J = 3.0 Hz, 1H), 6.63 (d, J = 9.5 Hz, 1H), 5.90 (t, J = 8.9 Hz, 1H), 5.84 (d, J = 3.0 Hz, 1H), 4.60 (d, J = 13.0 Hz, 1H), 4.36 (m, 1H), 4.22 (m, 2H), 4.02 (q, J = 12.3, 11.8 Hz, 3H), 3.87-3.74 (m, 5H), 3.52 (d, J = 11.7 Hz, 1H), 3.47 (d, J = 13.9 Hz, 2H), 3.31 (m, 1H), 3.20 (d, J = 14.4 Hz, 1H), 3.14 (m, 1H), 2.91 (m, 1H), 2.73-2.66 (m, 1H), 2.64-2.56 (m, 1H), 2.42 (d, J = 14.3 Hz, 1H), 2.35-2.22 (m, 2H), 1.98 (d, J = 15.6 Hz, 3H), 1.82 (m, 2H), 1.52 (d, J = 6.4 Hz, 3H), 1.28-1.24 (m, 7H), 1.16 (q, J = 6.4 Hz, 1H), 0.98 (s, 3H), 0.41 (s, 3H).

### Example 131: Synthesis of compound (1S,2S)-2-(difluoromethyl)-N-((1⁵S,6³S,4S,Z)-1²-((2S,6S)-2,6-dimethylmorpholino)-1⁵,10,10-tri methyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5] oxazonino[5,6-a]indola-2(4,2)-thiazola-6(1,3)-pyridazinacycloundecaphane-4-yl)cyclopropane-1 -carboxamide

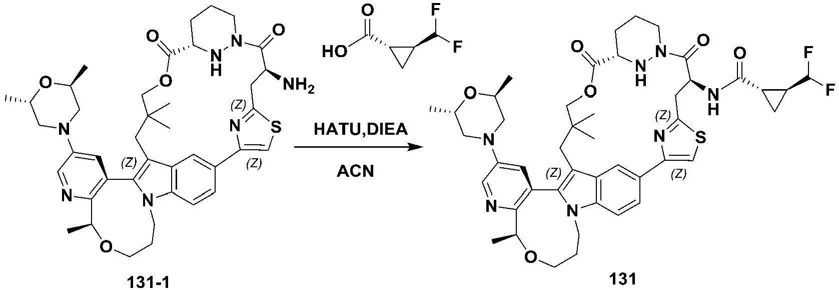

### Step 1: Synthesis of compound 131

Compound (1S,2S)-2-(difluoromethyl)cyclopropane-1-carboxylic acid (30.28 mg, 0.22 mmol) was dissolved in acetonitrile (2.00 mL). DIEA (0.25 mL, 1.48 mmol) and HATU (84.60 mg, 0.22 mmol) were added sequentially in an ice-water bath. After reacting in the ice-water bath for 10 min, a solution of compound 131-1 (110.00 mg, 0.15 mmol) in acetonitrile (2.00 mL) and DIEA (0.25 mL, 1.48 mmol) was added, and the reaction was carried out at room temperature for 1 h. The mixture was concentrated to dryness and purified by preparative separation to obtain compound 131 (47.30 mg, 36.22%). ESI-MS m/z: 860.65, [M+H]⁺.

¹H NMR (500 MHz, DMSO)δ 8.91 (d, J = 9.0 Hz, 1H), 8.50 (s, 1H), 8.39 (d, J = 2.7 Hz, 1H), 7.82 (s, 1H), 7.74 - 7.68 (m, 1H), 7.50 (d, J = 8.6 Hz, 1H), 7.24 (d, J = 2.7 Hz, 1H), 5.95 (td, J = 56.6, 5.0 Hz, 1H), 5.56 (t, J = 9.3 Hz, 1H), 5.13 (d, J = 12.2 Hz, 1H), 4.24 (t, J = 10.2 Hz, 2H), 4.16 (q, J = 6.1 Hz, 1H), 4.12 - 3.97 (m, 4H), 3.68 (d, J = 10.9 Hz, 1H), 3.59 (d, J = 10.5 Hz, 2H), 3.38 (d, J = 14.5 Hz, 1H), 3.31 (dd, J = 11.9, 2.9 Hz, 2H), 3.14 (dd, J = 14.4, 9.2 Hz, 2H), 3.06 - 2.94 (m, 3H), 2.76 (dt, J = 12.6, 8.8 Hz, 1H), 2.44 - 2.36 (m, 1H), 2.29 (s, 1H), 2.15 - 2.05 (m, 4H), 1.79 (d, J = 22.6 Hz, 3H), 1.65 (dd, J = 9.3, 4.3 Hz, 1H), 1.58 - 1.47 (m, 1H), 1.38 (d, J = 6.3 Hz, 3H), 1.19 (d, J = 6.4 Hz, 6H), 0.96 (s, 3H), 0.34 (s, 3H).

### Example 133: Synthesis of compound (1S,2S)-2-(difluoromethyl)-N-((1⁵S,6³S,4S,Z)-1²-(1,1-dioxidothiomorpholino)-1⁵,10,10-trimethy 1-5,7-dioxo-1⁸,1⁹,6¹,6,²6³,6⁴,6⁵,6⁶-octahydro-1⁵H,1⁷H-8-oxa-1(13,15)-pyrido[3',2':7,8][1,5]oxazo nino[5,6-alindola-2(4,2)-thiazola-6(1,3)-pyridazinacycloundecaphane-4-yl)cyclopropane-1-carbo xamide

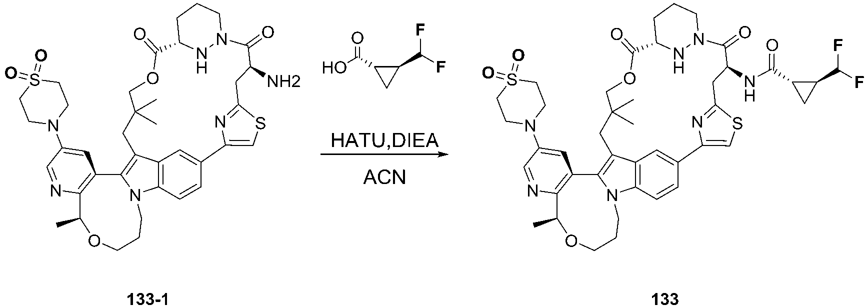

(1S,2S)-2-(difluoromethyl)cyclopropane-1-carboxylic acid (11.79 mg, 0.09 mmol) and acetonitrile (2.00 mL) were added sequentially to a 10 mL single-necked flask. At 0 °C, N,N,N',N'-tetramethyl-O-(7-azabenzotriazol-1-yl)uronium hexafluorophosphate (32.93 mg, 0.09 mmol) and N,N-diisopropylethylamine (0.09 mL, 0.58 mmol) were added, and the mixture was stirred at 0 °C for 10 min. Finally, compound 133-1 (44.00 mg, 0.06 mmol) was added, and the mixture was stirred at room temperature for 30 min. The reaction was complete as monitored by LC-MS, and the reaction solution was directly purified by pre-HPLC to obtain the target compound 133 (38.10 mg). ESI-MS m/z: 880.5 [M+H]⁺.

¹H NMR (500 MHz, CDCl₃)δ 8.58 (s, 1H), 8.44 (d, J = 2.9 Hz, 1H), 7.62 - 7.56 (m, 1H), 7.32 (d, J = 8.6 Hz, 1H), 7.29 (s, 1H), 7.14 (d, J = 2.9 Hz, 1H), 6.69 (d, J = 9.5 Hz, 1H), 5.90 (t, J = 8.9 Hz, 1H), 5.84 (td, J = 57.5, 2.9 Hz, 1H), 4.60 (d, J = 11.8 Hz, 1H), 4.35 (td, J = 12.0, 3.0 Hz, 1H), 4.28 - 4.20 (m, 2H), 4.02 (d, J = 12.3 Hz, 2H), 3.96 - 3.88 (m, 4H), 3.86 - 3.73 (m, 3H), 3.46 (d, J = 14.9 Hz, 1H), 3.32 (td, J = 12.4, 2.9 Hz, 1H), 3.22 (d, J = 14.4 Hz, 1H), 3.18 - 3.10 (m, 5H), 2.69 (td, J = 12.9, 2.5 Hz, 1H), 2.37 - 2.30 (m, 2H), 2.23 (dd, J = 19.1, 11.3 Hz, 1H), 2.00 (dd, J = 14.6, 10.2 Hz, 6H), 1.52 (d, J = 6.4 Hz, 3H), 1.18 - 1.12 (m, 1H), 0.97 (s, 3H), 0.88 (t, J = 6.6 Hz, 1H), 0.40 (s, 3H).

### Example 134: Synthesis of compound (1S,2S)-2-(difluoromethyl)-N-(((1⁵S,6³S,4S,Z)-1⁵,10,10-trimethyl-1²-(1-oxidothiomorpholino)-5, 7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxazonin o[5,6-alindola-2(4,2)-thiazola-6(1,3)-pyridazinacycloundecaphane-4-yl)cyclopropane-1-carboxa mide

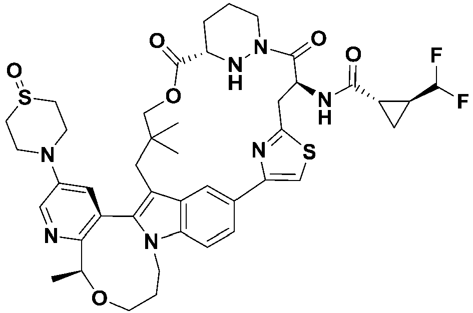

The specific synthetic steps are the same as in Example 133. ESI-MS m/z: 864.66 [M+H]⁺.

¹H NMR (500 MHz, MeOD)δ 8.59 (s, 1H), 8.47 (d, J = 2.9 Hz, 1H), 7.59 (d, J = 8.5 Hz, 1H), 7.31 (t, J = 7.2 Hz, 1H), 7.29 (s, 1H), 7.16 (d, J = 2.9 Hz, 1H), 6.66 (d, J = 9.5 Hz, 1H), 5.90 (t, J = 8.9 Hz, 1H), 5.84 (td, J = 57.5, 3.0 Hz, 1H), 4.60 (d, J = 11.0 Hz, 1H), 4.36 (td, J = 12.1, 2.9 Hz, 1H), 4.28 - 4.20 (m, 2H), 4.10 - 3.97 (m, 4H), 3.86 - 3.75 (m, 3H), 3.73 - 3.60 (m, 2H), 3.47 (d, J = 15.0 Hz, 1H), 3.32 (t, J = 10.9 Hz, 1H), 3.21 (d, J = 14.4 Hz, 1H), 3.14 (dd, J = 15.0, 8.6 Hz, 1H), 2.95 - 2.82 (m, 4H), 2.69 (t, J = 11.5 Hz, 1H), 2.38 (d, J = 14.5 Hz, 1H), 2.26 - 2.20 (m, 3H), 2.04 - 1.94 (m, 5H), 1.86 - 1.78 (m, 2H), 1.52 (d, J = 6.4 Hz, 3H), 0.98 (s, 3H), 0.41 (s, 3H).

### Example 135: Synthesis of compound (1S,2S)-2-(difluoromethyl)-N-((1⁵S,6³S,4S,Z)-1²-((R)-3-methoxypyrrolidin-1-yl)-1⁵,10,10-trimet hyl-5,7-dioxo-1⁸, 1⁹,6¹ ,6²,6³,6⁴,6⁵ ,6⁶-octahydro-1⁵H,1⁷H-8-oxa-1(13,15)-pyrido[3',2':7,8][1,5]oxa zonino[5,6-a]indola-2(4,2)-thiazola-6(1,3)-pyridazinacycloundecaphane-4-yl)cyclopropane-1-car boxamide

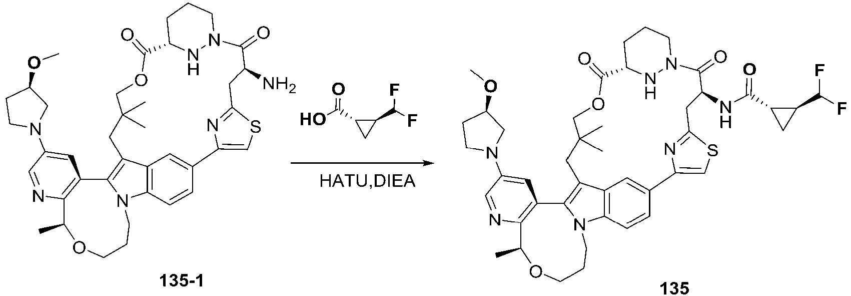

### Step 1: Synthesis of compound 135

(1S,2S)-2-(difluoromethyl)cyclopropane-1-carboxylic acid (21.60 mg, 0.18 mmol) and N,N-dimethylformamide (1.50 mL) were added sequentially to a 10 mL single-necked flask. At 0 °C, N,N,N',N'-tetramethyl-O-(7-azabenzotriazol-1-yl)uronium hexafluorophosphate (60.34 mg, 0.16 mmol) and N,N-diisopropylethylamine (0.14 mL, 0.85 mmol) were added, and the mixture was stirred at 0 °C for 10 min. Finally, 135-1 (77.00 mg, 0.11 mmol) was added, and the mixture was stirred at 0 °C for 10 min. The reaction was complete as monitored by LC-MS, and the reaction solution was directly purified by pre-HPLC to obtain the target product 135 (31.7 mg). ESI-MS m/z: 846.4 [M+H]⁺.

### Example 136: Synthesis of compound (1S,2S)-2-(difluoromethyl)-N-((1⁵S,6³S,4S,Z)-1²-((S)-3-methoxypyrrolidin-1-yl)-1⁵,10,10-trimet hyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxa zonino[5,6-alindola-2(4,2)-thiazola-6(1,3)-pyridazinacycloundecaphane-4-yl)cyclopropane-1-car boxamide

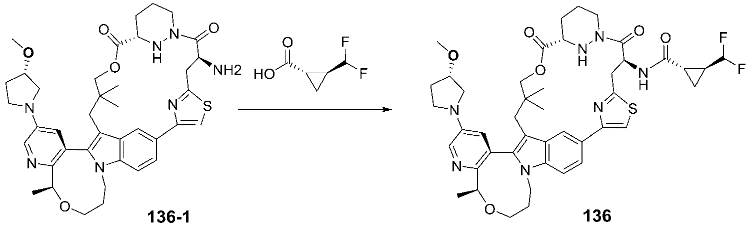

### Step 1: Synthesis of compound 136

(1S,2S)-2-(difluoromethyl)cyclopropane-1-carboxylic acid (29 mg, 0.21 mmol), HATU (108 mg, 0.28 mmol), and DIEA (0.14 mL, 0.85 mmol) were added sequentially to a 10 mL single-necked flask, and the mixture was stirred at room temperature for 30 min. The mixture was cooled in an ice-water bath, and then a solution of compound 136-1 (155 mg, 0.21 mmol) in acetonitrile was added. After the reaction was complete as monitored by LC-MS, the reaction solution was directly purified by Prep-HPLC, and the eluate was lyophilized to finally obtain the target compound 136 (70.6 mg). ESI-MS m/z: 846.55, [M+H]⁺.

### Example 138: Synthesis of compound (1S,2S)-2-(difluoromethyl)-N-((1⁵S,6³S,4S,Z)-1²-((R)-3,4-dimethylpiperazin-1-yl)-1⁵,10,10-trim ethyl-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]o xazonino[5,6-a]indola-2(4,2)-thiazola-6(1,3)-pyridazinacycloundecaphane-4-yl)cyclopropane-1-carboxamide

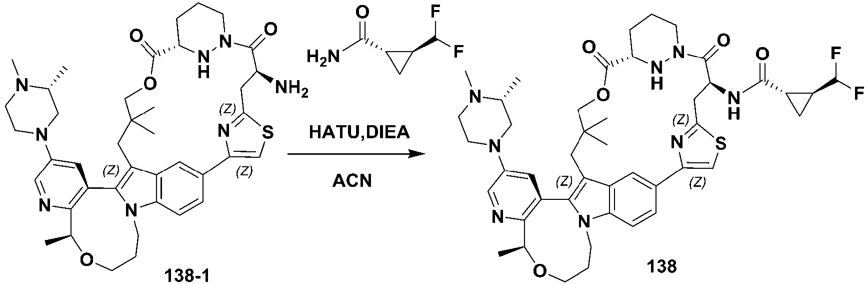

Compound (1S,2S)-2-(difluoromethyl)cyclopropane-1-carboxylic acid (22.05 mg, 0.16 mmol) was dissolved in acetonitrile (4.00 mL). DIEA (0.22 mL, 1.36 mmol) and HATU (77.00 mg, 0.20 mmol) were added sequentially in an ice-water bath. After reacting in the ice-water bath for 10 min, a solution of compound 138-1 (100.00 mg, 0.14 mmol) in acetonitrile (4.00 mL) and DIEA (0.22 mL, 1.36 mmol) was added, and the reaction was carried out at room temperature for 1 h. The mixture was concentrated to dryness and purified by preparative separation to obtain compound 138 (24.00 mg, 20.47%). ESI-MS m/z: 859.52, [M+H]⁺.

¹H NMR (500 MHz, DMSO)δ 8.90 (d, J = 8.9 Hz, 1H), 8.50 (s, 1H), 8.41 (s, 1H), 7.81 (s, 1H), 7.71 (d, J = 8.4 Hz, 1H), 7.49 (d, J = 8.5 Hz, 1H), 7.21 (s, 1H), 5.95 (td, J = 56.7, 4.8 Hz, 1H), 5.56 (t, J = 9.1 Hz, 1H), 5.12 (d, J = 12.1 Hz, 1H), 4.24 (d, J = 11.9 Hz, 2H), 4.15 (d, J = 6.2 Hz, 1H), 4.03 (d, J = 10.3 Hz, 2H), 3.72 - 3.65 (m, 3H), 3.59 (d, J = 10.8 Hz, 2H), 3.40 - 3.34 (m, 2H), 3.19 - 3.10 (m, 2H), 3.01 (d, J = 14.3 Hz, 1H), 2.83 (dd, J = 20.6, 11.3 Hz, 4H), 2.47 - 2.36 (m, 3H), 2.33 - 2.17 (m, 6H), 2.10 (s, 3H), 2.00 (d, J = 8.5 Hz, 1H), 1.81 (s, 3H), 1.65 (s, 1H), 1.49 (d, J = 29.7 Hz, 2H), 1.37 (d, J = 6.0 Hz, 3H), 0.96 (s, 3H), 0.33 (s, 3H).

### Example 140: Synthesis of compound (1S,2S)-2-(difluoromethyl)-N-((1⁵S,6³S,4S,Z)-1⁵,10,10-trimethyl-5,7-dioxo-1²-((3S,5R)-3,4,5-tri methylpiperazin-1-yl)-1⁸,1⁹,6¹,6²,6³,6⁴,6⁵,6⁶-octahydro-1⁵H,1⁷H-8-oxa-1(13,15)-pyrido[3',2':7,8] [1,5]oxazonino[5,6-a]indola-2(4,2)-thiazola-6(1,3)-pyridazinacycloundecaphane-4-yl)cyclopropa ne-1-carboxamide

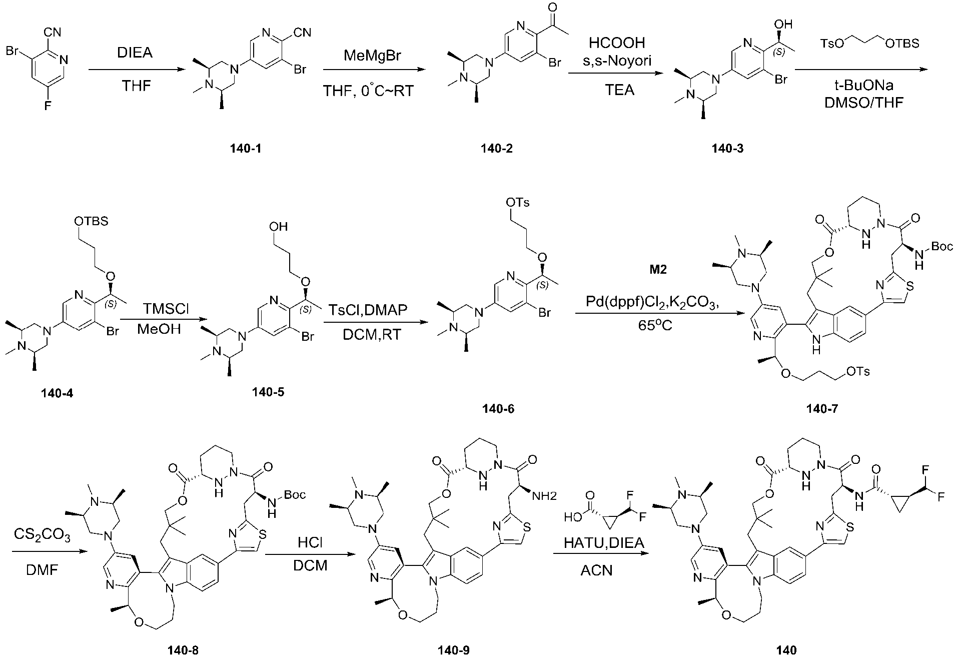

### Step 1: Synthesis of compound 140-1

3-Bromo-5-fluoro-pyridine-2-carbonitrile (2.00 g, 9.95 mmol), tetrahydrofuran (20.00 mL), 1-methyl-4-(piperidin-4-yl)piperazine (1.40 g, 10.95 mmol), and N,N-diisopropylethylamine (2.47 mL, 14.93 mmol) were added sequentially to a 50 mL single-necked flask, and the mixture was stirred at room temperature for 2 h. The reaction was complete as monitored by LC-MS, the system became a suspension, filtered, and the filter cake was washed with a small amount of EA. The filter cake was dried to obtain the target compound 140-1 (3.08 g). ESI-MS m/z: 309.0 [M+H]⁺.

### Step 2: Synthesis of compound 140-2

Compound 140-1 (2.13 g, 6.89 mmol) and tetrahydrofuran (20.00 mL) were added sequentially to a 100 mL three-necked flask. Under nitrogen protection, the temperature was lowered to 0 °C, and then methylmagnesium bromide (6.89 mL, 3.00 mol/L, 20.67 mmol) was added dropwise. The mixture was stirred at room temperature for 30 min. The reaction was complete as monitored by LC-MS, quenched with saturated NH₄Cl solution, adjusted to pH=4 with dilute hydrochloric acid, and stirred for 10 min. The mixture was then adjusted to pH=8 with saturated NaHCO₃ solution, extracted twice with ethyl acetate, and the organic layer was washed with saturated brine and dried over anhydrous sodium sulfate. The mixture was concentrated under reduced pressure and purified by column chromatography to obtain the target compound 140-2 (1.33 g). ESI-MS m/z: 326.1 [M+H]⁺.

### Step 3: Synthesis of compound 140-3

Triethylamine (8.5 mL, 61.15 mmol) was added to a 50 mL three-necked flask. Under nitrogen protection, the temperature was lowered to 0 °C, formic acid (0.77 mL, 20.38 mmol) was added dropwise, and then (S,S)-N-(p-toluenesulfonyl)-1,2-diphenylethane-1,2-diamine (p-cymene)ruthenium(II) chloride (0.26 g, 0.41 mmol) was added. The reaction was warmed to 40 C and stirred for 15 min. After cooling to room temperature, compound 140-2 (1.33 g, 4.08 mmol) and dichloromethane (2.00 mL) were finally added, and the mixture was stirred at room temperature for 24 h. The reaction was complete as monitored by LC-MS, extracted twice with dichloromethane, and the organic layer was washed with saturated brine and dried over anhydrous sodium sulfate. The mixture was concentrated under reduced pressure and purified by column chromatography to obtain the target compound 140-3 (0.94 g). ESI-MS m/z: 328.1 [M+H]⁺.

### Step 4: Synthesis of compound 140-4

Compound 140-3 (0.94 g, 2.86 mmol), dimethyl sulfoxide (10.00 mL), tetrahydrofuran (2.50 mL), and 3-((tert-butyldimethylsilyl)oxy)propyl-4-methylbenzenesulfonate (2.96 g, 8.59 mmol) were added sequentially to a 50 mL single-necked flask. Sodium tert-butoxide (0.83 g, 8.59 mmol) was added in batches at 10 °C, and the mixture was stirred at room temperature for 1 h. The reaction was complete as monitored by LC-MS, and the reaction mixture was added to a saturated aqueous ammonium chloride solution for quenching. The mixture was extracted twice with ethyl acetate, washed three times with saturated brine, concentrated under reduced pressure, and purified by column chromatography to obtain the target compound 140-4 (1.24 g). ESI-MS m/z: 500.4 [M+H]⁺.

### Step 5: Synthesis of compound 140-5

Compound 140-4 (1.24 g, 2.48 mmol) and methanol (10.00 mL) were added sequentially to a 50 mL single-necked flask. Trimethylchlorosilane (0.63 mL, 4.95 mmol) was added dropwise at 0 °C, and then the reaction was allowed to stir at room temperature for 1 h. The reaction was complete as monitored by LC-MS, and concentrated under reduced pressure. The concentrate compound 140-5 (1.23 g) was used directly in the next step without purification. ESI-MS m/z: 386.3 [M+H]⁺.

### Step 6: Synthesis of compound 140-6

Compound 140-5 (1.23 g, 3.18 mmol), dichloromethane (10.00 mL), and 4-dimethylaminopyridine (77.77 mg, 0.64 mmol) were added sequentially to a 50 mL single-necked flask. Then p-toluenesulfonyl chloride (1.21 g, 6.37 mmol) and triethylamine (2.21 mL, 15.92 mmol) were added in batches, and the mixture was stirred at room temperature for 2 h. The reaction was complete as monitored by LC-MS, concentrated under reduced pressure, and purified by column chromatography to obtain the target compound 140-6 (1.33 g). ESI-MS m/z: 540.1 [M+H]⁺.

### Step 7: Synthesis of compound 140-7

Compound 140-6 (81.83 mg, 0.15 mmol), compound M2 (100.00 mg, 0.14 mmol), toluene (1.20 mL), 1,4-dioxane (0.40 mL), water (0.40 mL), potassium carbonate (59.79 mg, 0.43 mmol), and [1,1'-bis(diphenylphosphino)ferrocene]dichloropalladium(II) dichloromethane complex (10.54 mg, 0.01 mmol) were added sequentially to a 10 mL single-necked flask. Nitrogen was replaced three times, and under nitrogen protection, the reaction was heated to 65 °C and stirred for 3 h. The reaction was complete as monitored by LC-MS, extracted twice with ethyl acetate, washed with saturated brine, concentrated under reduced pressure, and purified by column chromatography to obtain the target compound 140-7 (50.00 mg). ESI-MS m/z: 514.6 1/2[M+2H]⁺.

### Step 8: Synthesis of compound 140-8

Compound 140-7 (50.00 mg, 0.05 mmol), N,N-dimethylformamide (4.00 mL), and cesium carbonate (47.60 mg, 0.15 mmol) were added sequentially to a 10 mL single-necked flask, and the reaction was warmed to 70 °C and stirred for 2 h. The reaction was complete as monitored by LC-MS, the reaction solution was diluted with ethyl acetate, washed sequentially with water and brine three times, concentrated under reduced pressure, and purified by column chromatography to obtain the target compound 140-8 (41.00 mg). ESI-MS m/z: 855.6 [M+H]⁺.

### Step 9: Synthesis of compound 140-9

Compound 140-8 (41.00 mg, 0.05 mmol), dichloromethane (2.00 mL), and hydrochloric acid (dioxane solution, 4M) (2.00 mL) were added sequentially to a 10 mL single-necked flask, and the mixture was stirred at room temperature for 1 h. The reaction was complete as monitored by LC-MS, and the mixture was concentrated under reduced pressure. The crude product was directly used in the next step to obtain the crude product compound 140-9 (50.00 mg). ESI-MS m/z: 755.0 [M+H]⁺.

### Step 10: Synthesis of compound 140

(1S,2S)-2-(difluoromethyl)cyclopropane-1-carboxylic acid (13.51 mg, 0.1 mmol) and acetonitrile (1.00 mL) were added sequentially to a 10 mL single-necked flask. At 0 °C, N,N,N',N'-tetramethyl-O-(7-azabenzotriazol-1-yl)uronium hexafluorophosphate (37.76 mg, 0.11 mmol) and N,N-diisopropylethylamine (0.11 mL, 0.66 mmol) were added, and the mixture was stirred at 0 °C for 10 min. Finally, compound 140-9 (50.00 mg, 0.07 mmol) was added, and the mixture was stirred at room temperature for 30 min. The reaction was complete as monitored by LC-MS, and the reaction solution was directly purified by pre-HPLC to obtain the target compound 140 (5.2 mg). ESI-MS m/z: 873.5 [M+H]⁺.

¹H NMR (500 MHz, CDCl₃)δ 8.59 (s, 1H), 8.41 (d, J = 2.8 Hz, 1H), 7.58 (d, J = 7.1 Hz, 1H), 7.31 (d, J = 8.5 Hz, 1H), 7.29 (s, 1H), 7.07 (d, J = 2.8 Hz, 1H), 6.63 (d, J = 9.6 Hz, 1H), 5.90 (t, J = 8.9 Hz, 1H), 5.85 (td, J = 57.5, 2.9 Hz, 2H), 5.35 (d, J = 1.8 Hz, 1H), 4.60 (d, J = 12.2 Hz, 1H), 4.40 - 4.32 (m, 1H), 4.27 - 4.17 (m, 2H), 4.04 - 3.97 (m, 2H), 3.85 - 3.74 (m, 3H), 3.58 - 3.50 (m, 2H), 3.47 (d, J = 14.9 Hz, 1H), 3.35 - 3.27 (m, 1H), 3.20 (d, J = 14.2 Hz, 1H), 3.14 (dd, J = 15.0, 8.5 Hz, 1H), 2.74 - 2.59 (m, 3H), 2.30 (ddd, J = 35.6, 23.4, 11.1 Hz, 8H), 2.04 - 1.77 (m, 6H), 1.51 (d, J = 6.5 Hz, 3H), 1.31 (s, 5H), 0.98 (s, 3H), 0.88 (t, J = 6.9 Hz, 2H), 0.41 (s, 3H).

### Example 142: Synthesis of compound (1S,2S)-2-(difluoromethyl)-N-((1⁵S,6³S,4S,Z)-1⁵,10,10-trimethyl-5,7-dioxo-1²-((3S,5S)-3,4,5-tri methylpiperazin-1-yl)-1⁸,1⁹,6¹,6²,6³,6⁴,6⁵,6⁶-octahydro-1⁵H,1⁷H-8-oxa-1(13,15)-pyrido[3',2':7,8] [1,5]oxazonino[5,6-a]indola-2(4,2)-thiazola-6(1,3)-pyridazinacycloundecaphane-4-yl)cyclopropa ne-1-carboxamide

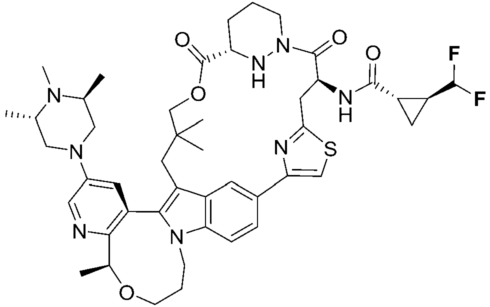

The synthetic steps are the same as in Example 140. ESI-MS m/z: 873.9 [M+H]⁺.

¹H NMR (500 MHz, CDCl₃)δ 8.58 (d, J = 1.3 Hz, 1H), 8.40 (d, J = 2.9 Hz, 1H), 7.57 (dd, J = 8.6, 1.5 Hz, 1H), 7.31 (d, J = 8.5 Hz, 1H), 7.28 (s, 1H), 7.04 (d, J = 2.9 Hz, 1H), 6.62 (d, J = 9.5 Hz, 1H), 5.90 (t, J = 8.9 Hz, 1H), 5.84 (td, J = 57.6, 3.0 Hz, 1H), 4.60 (d, J = 12.0 Hz, 1H), 4.36 (td, J = 12.1, 3.2 Hz, 1H), 4.26 - 4.17 (m, 2H), 4.04 - 3.97 (m, 2H), 3.86 - 3.74 (m, 3H), 3.47 (d, J = 14.9 Hz, 1H), 3.31 (td, J = 12.2, 3.1 Hz, 2H), 3.20 (d, J = 14.3 Hz, 1H), 3.13 (dd, J = 15.0, 8.5 Hz, 1H), 3.02 (s, 3H), 2.69 (td, J = 12.9, 2.8 Hz, 1H), 2.47 - 2.19 (m, 7H), 2.04 - 1.86 (m, 4H), 1.85 - 1.79 (m, 2H), 1.51 (d, J = 6.5 Hz, 3H), 1.35 - 1.30 (m, 4H), 1.18 - 1.11 (m, 5H), 0.98 (s, 3H), 0.42 (s, 3H).

### Example 144: Synthesis of compound (1S,2S)-2-(difluoromethyl)-N-((SS,18S,26S)-2-(4-ethylpiperazin-1-yl)-5,30,30-trimethyl-21,27-dioxo-5,8,9,15,16,18,19,20,21,24,25,26,27,29,30,31-hexadecahydro-7H,23H-22,26-epimino-11, 13-etheno-14,18-methanopyrido[3",2":7',8'][1,5]oxazonino[5',6':1,5]pyrrolo[3,4-g][1,12]dioxa[4, 18]diazacyclodocosin-20-yl)cyclopropane-1-carboxamide

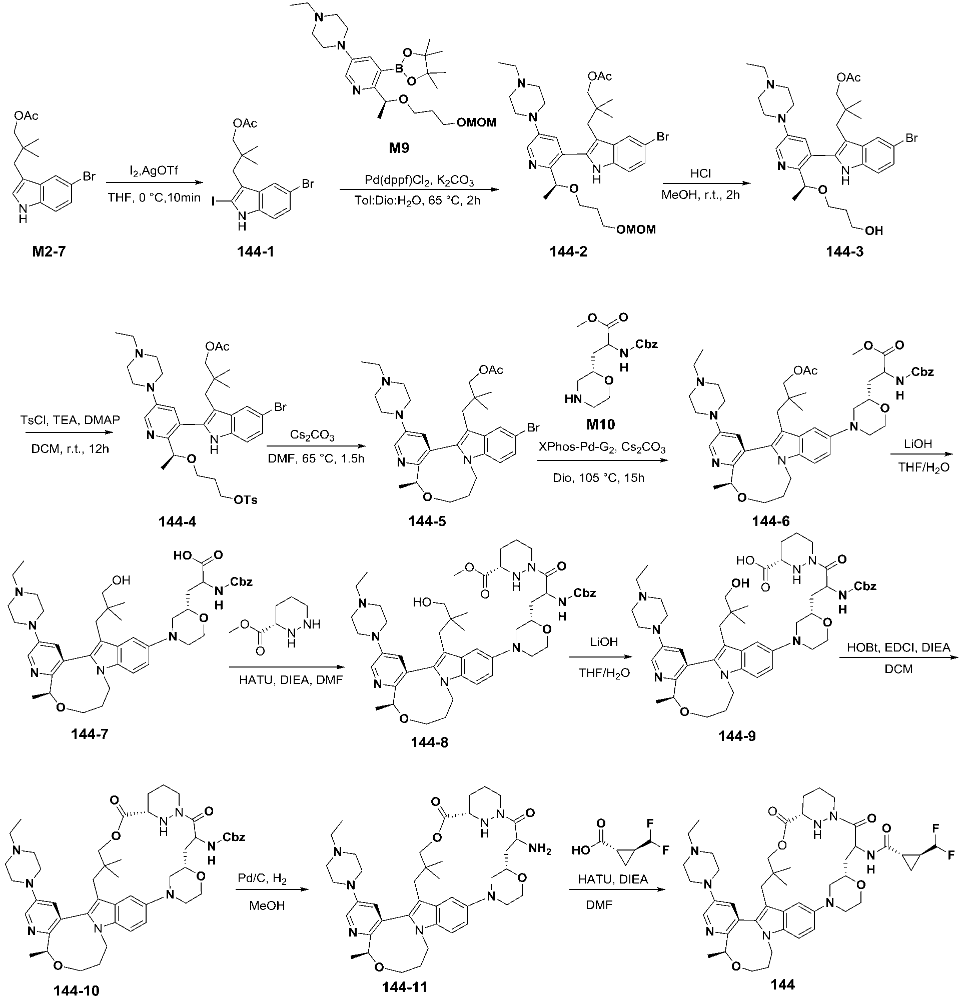

### Step 1: Synthesis of compound 144-1

Compound M2-7 (3.90 g, 12.03 mmol) was dissolved in THF (50.00 mL), the temperature was lowered to 0 °C. Then AgOTf (3.71 g, 14.43 mmol) was added, followed by a solution of I₂ (3.05 g, 12.03 mmol) in THF (5.00 mL), and the reaction was carried out at 0 °C for 10 min. LC-MS showed the reaction was complete. Saturated sodium sulfite solution was added to the reaction system for quenching, the reaction solution was diluted with EA, washed sequentially with water and brine, separated, and the organic phase was concentrated to obtain a crude product, which was purified by silica gel column chromatography to obtain the target compound 144-1 (3.5 g). ESI-MS m/z: 448.95, 449.89 [M+H]⁺.

### Step 2: Synthesis of compound 144-2

Compound 144-1 (3.50 g, 7.78 mmol), compound M9 (7.21 g, 15.55 mmol), [PdCl₂(dppf)]CH₂Cl₂ (0.64 g, 0.78 mmol), and K₂CO₃ (3.22 g, 23.33 mmol) were dissolved in toluene (60.00 mL), 1,4-dioxane (20.00 mL) and H₂O (20.00 mL) under nitrogen protection, and reacted at 65 °C for 5 h. LC-MS showed the reaction was complete. The reaction solution was diluted with EA, washed sequentially with water and brine, separated, and the organic phase was concentrated to obtain a crude product, which was purified by silica gel column chromatography to obtain the target compound 144-2 (4.2 g). ESI-MS m/z: 658.27, 659.30 [M+H]⁺.

### Step 3: Synthesis of compound 144-3

Compound 144-2 (4.20 g, 6.37 mmol) was dissolved in DCM (20.00 mL), then HCl (4M/dioxane) (20.00 mL) was added, and the reaction was carried out at room temperature for 30 min. LC-MS showed the reaction was complete. The mixture was concentrated under reduced pressure, sodium carbonate hydrogen and DCM were added for extraction, dried over anhydrous sodium sulfate, and the organic phase was concentrated to obtain a crude product, which was purified by silica gel column chromatography to obtain the target compound 144-3 (1.4 g). ESI-MS m/z: 614.25, 615.24 [M+H]⁺.

### Step 4: Synthesis of compound 144-4

Compound 144-3 (1.40 g, 2.27 mmol) was dissolved in DCM (20.00 mL) under nitrogen protection, and the temperature was lowered to 0 °C. Then TEA (0.95 mL, 6.82 mmol) and DMAP (0.03 g, 0.23 mmol) were added, followed by TosCl (1.30 g, 6.82 mmol), and the reaction was carried out at room temperature overnight. LC-MS showed the reaction was complete. Saturated sodium bicarbonate was added for quenching, the reaction solution was diluted with DCM, washed sequentially with water and brine, separated, and the organic phase was concentrated to obtain a crude product, which was purified by silica gel column chromatography to obtain the target compound 144-4 (1.25 g). ESI-MS m/z: 768.26, 769.28 [M+H]⁺.

### Step 5: Synthesis of compound 144-5

Compound 144-4 (1.25 g, 1.62 mmol) was dissolved in DMF (10.00 mL), then Cs₂CO₃ (1.59 g, 4.87 mmol) was added, and the reaction was carried out at 65 °C for 1 h. LC-MS showed the reaction was complete. The reaction solution was diluted with EA, washed sequentially with water and brine, separated, and the organic phase was concentrated to obtain a crude product, which was purified by silica gel column chromatography to obtain the target compound 144-5 (0.67 g). ESI-MS m/z: 596.24, 597.30 [M+H]⁺.

### Step 6: Synthesis of compound 144-6

Compound 144-5 (668.00 mg, 1.12 mmol), compound M10 (720.66 mg, 2.24 mmol), XphosPdG2 (87.96 mg, 0.11 mmol), and Cs₂CO₃ (1092.60 mg, 3.35 mmol) were dissolved in 1,4-dioxane (10.00 mL) under nitrogen protection, and reacted at 105 °C for 5 h. LC-MS showed the reaction was complete. The reaction solution was diluted with EA, washed sequentially with water and brine, separated, and the organic phase was concentrated to obtain a crude product, which was purified by silica gel column chromatography to obtain the target compound 144-6 (1.01 g). ESI-MS m/z: 838.46, 839.39 [M+H]⁺.

### Step 7: Synthesis of compound 144-7

Compound 144-6 (1.01 g, 1.20 mmol) was dissolved in THF (12.00 mL) and H₂O (4.00 mL). Then lithium hydroxide monohydrate (0.15 g, 3.61 mmol) was added, and the reaction was carried out at room temperature overnight. LC-MS showed the reaction was complete. The pH was adjusted to about 6 with 1M hydrochloric acid, and the mixture was extracted with EA and water, then extracted twice with a mixed solution of DCM/MEOH = 10:1 and water. The organic phases were combined and concentrated to obtain a crude product of the target compound 144-7 (0.84 g), which was directly used in the next reaction. ESI-MS m/z: 782.44, 783.41 [M+H]⁺.

### Step 8: Synthesis of compound 144-8

Compound 144-7 (800.00 mg, 1.02 mmol) was dissolved in DMF (5.00 mL). Then compound (S)-tert-butyl hexahydropyridazine-3-carboxylate (176.78 mg, 1.23 mmol), HATU (466.24 mg, 1.23 mmol), and DIEA (0.89 mL, 5.11 mmol) were added, and the reaction was carried out at room temperature for 2 h. LC-MS showed the reaction was complete. The reaction solution was diluted with EA, washed sequentially with water and brine, separated, and the organic phase was concentrated to obtain a crude product, which was purified by silica gel column chromatography to obtain the target compound 144-8 (0.84 g). ESI-MS m/z: 908.52, 909.49 [M+H]⁺.

### Step 9: Synthesis of compound 144-9

Compound 144-8 (630.00 mg, 0.69 mmol) was dissolved in THF (6.00 mL). Then a solution of lithium hydroxide monohydrate (72.70 mg, 1.73 mmol) in H₂O (2.00 mL) was added, and the reaction was carried out at room temperature for 30 min. LC-MS showed the reaction was complete. The reaction solution was diluted with EA, washed sequentially with saturated ammonium chloride, water, and brine, separated, and the organic phase was concentrated to obtain a crude product, which was purified by silica gel column chromatography to obtain the target compound 144-9 (447 mg). ESI-MS m/z: 894.50, 895.52 [M+H]⁺.

### Step 10: Synthesis of compound 144-10

Compound 144-9 (447.00 mg, 0.50 mmol) was dissolved in DCM (50.00 mL). Then HOBt (202.44 mg, 1.50 mmol), EDCI (478.68 mg, 2.50 mmol), and DIEA (0.52 mL, 3.00 mmol) were added, and the reaction was carried out at room temperature overnight. LC-MS showed the reaction was complete. The reaction solution was diluted with DCM, washed sequentially with water and brine, separated, and the organic phase was concentrated to obtain a crude product, which was purified by silica gel column chromatography to obtain the target compound 144-10 (190 mg). ESI-MS m/z: 876.49, 877.50 [M+H]⁺.

### Step 11: Synthesis of compound 144-11

Compound 144-10 (110.00 mg, 0.13 mmol) was dissolved in MeOH (3.00 mL). Then Pd/C (133.45 mg, 1.25 mmol) was added, hydrogen was replaced 5 times, and the reaction was carried out at 40 C for 1 h. LC-MS showed the reaction was complete. The mixture was filtered, concentrated, dissolved in EA, washed sequentially with water and brine, separated, and the organic phase was concentrated to obtain a crude product of the target compound 144-11 (93 mg), which was directly used in the next reaction. ESI-MS m/z: 742.45, 743.44 [M+H]⁺.

### Step 12: Synthesis of compound 144

Compound (1S,2S)-2-(difluoromethyl)cyclopropane-1-carboxylic acid (5.50 mg, 0.04 mmol) was dissolved in DMF (1.00 mL), the temperature was lowered to 0 °C. Then DIEA (0.05 mL, 0.27 mmol) and HATU (20.46 mg, 0.05 mmol) were added, and the reaction was carried out at 0 C for 5 min. Then compound 144-11 (20.00 mg, 0.03 mmol) was added, and the reaction was carried out at 0 °C for 5 min. LC-MS showed the reaction was complete, and purification by preparative chromatography yielded the target compound 144 (3.1 mg). ESI-MS m/z: 860.48, 861.49 [M+H]⁺.

¹H NMR (500 MHz, DMSO-d₆) δ 8.38 (d, J = 2.9 Hz, 1H), 8.34 (d, J = 7.6 Hz, 1H), 7.37 (dd, J = 9.1, 4.0 Hz, 1H), 7.17 (d, J = 2.9 Hz, 1H), 7.10 (d, J = 2.2 Hz, 1H), 7.06 (dd, J = 8.8, 2.1 Hz, 1H), 5.87 (td, J = 56.7, 5.2 Hz, 1H), 5.51 (q, J = 7.0 Hz, 1H), 5.05 (d, J = 12.2 Hz, 1H), 4.32 (d, J = 12.1 Hz, 1H), 4.12 (dq, J = 13.1, 6.7 Hz, 1H), 3.96 (d, J = 14.5 Hz, 2H), 3.85 (t, J = 10.0 Hz, 2H), 3.71 - 3.61 (m, 1H), 3.59 - 3.50 (m, 4H), 3.24 (q, J = 4.7 Hz, 5H), 3.17 (d, J = 11.5 Hz, 1H), 3.11 (d, J = 11.9 Hz, 1H), 2.91 (d, J = 14.3 Hz, 1H), 2.86 - 2.77 (m, 1H), 2.69 (t, J = 12.3 Hz, 1H), 2.64 (p, J = 1.9 Hz, 1H), 2.57 (t, J = 10.5 Hz, 5H), 2.43 (s, 1H), 2.37 - 2.35 (m, 2H), 2.20 (d, J = 13.4 Hz, 1H), 2.13 (d, J = 11.9 Hz, 1H), 1.97 (dt, J = 7.8, 4.6 Hz, 1H), 1.90 - 1.81 (m, 3H), 1.75 (s, 1H), 1.67 (d, J = 12.5 Hz, 1H), 1.61 (d, J = 11.6 Hz, 1H), 1.55 - 1.50 (m, 1H), 1.37 (d, J = 6.3 Hz, 3H), 1.24 (s, 1H), 1.03 (d, J = 7.2 Hz, 3H), 0.92 (d, J = 6.6 Hz, 5H), 0.28 (s, 2H).

### Example 145: Synthesis of compound (1S,2S)-2-(difluoromethyl)-N-((1⁵S,6³S,4S)-2⁵-methoxy-1⁵,10,10-trimethyl-1²-(4-methylpiperazi n-1-yl)-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5] oxazonino[5,6-a]indola-6(1,3)-pyridazina-2(1,3)-benzenacycloundecaphane-4-yl)cyclopropane-1 -carboxamide

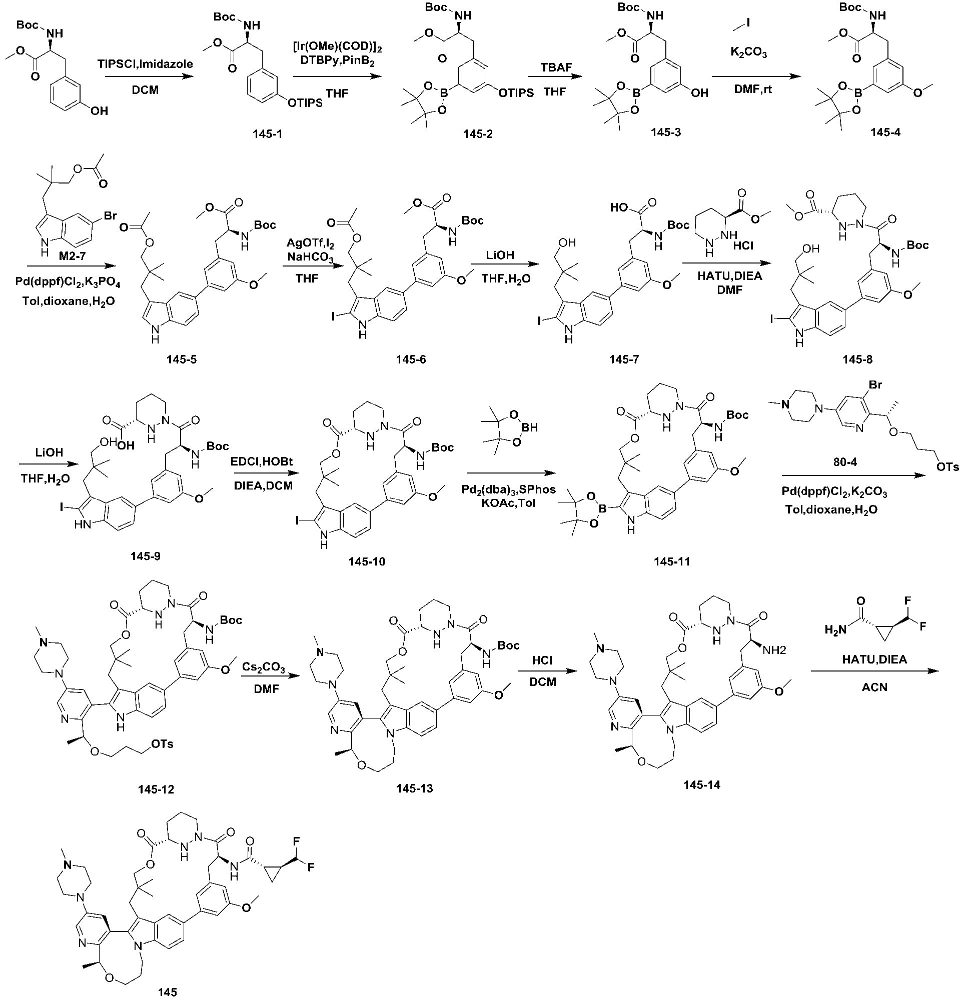

### Step 1: Synthesis of compound 145-1

Compound methyl (S)-2-((tert-butoxycarbonyl)amino)-3-(3-hydroxyphenyl)propanoate (4.00 g, 13.54 mmol), imidazole (1.84 g, 27.09 mmol), and triisopropylchlorosilane (3.48 mL, 16.25 mmol) were added sequentially to DCM (10.00 mL), and the reaction was carried out at room temperature for 2 h. Water (50 mL) was added to the reaction solution, and the resultant was extracted twice with 40 mL of DCM, washed twice with 40 mL of saturated brine, dried over anhydrous sodium sulfate, concentrated to dryness, and separated by column chromatography (PE:EA = 95%:5%) to obtain compound 145-1 (6.10 g, 99.71%). ESI-MS m/z: 352.33 [M+H]⁺.

### Step 2: Synthesis of compound 145-2

Compound 145-1 (6.10 g, 13.51 mmol), bis(pinacolato)diboron (6.86 g, 27.01 mmol), 4,4'-di-tert-butyl-2,2'-bipyridine (1.70 g, 4.05 mmol), and methoxy(cyclooctadiene)iridium(I) dimer (0.90 g, 1.35 mmol) were added sequentially to THF (60.00 mL), the atmosphere was replaced with nitrogen, and the reaction was carried out at 80 °C for 10 h. The reaction solution was concentrated to dryness and separated by column chromatography (PE:EA = 97%:3%) to obtain compound 145-2 (5.00 g, 64.09%). ESI-MS m/z: 477.62 [M+H]+.

### Step 3: Synthesis of compound 145-3

Compound 145-2 (5.00 g, 8.66 mmol) and tetrabutylammonium fluoride (10.39 mL, 1.00 mol/L, 10.39 mmol) were added to THF (50.00 mL), and the reaction was carried out at room temperature for 10 min. The reaction solution was diluted by adding 50 mL of ethyl acetate, then washed twice with 40 mL of saturated brine, dried over anhydrous sodium sulfate, and separated by column chromatography (PE:EA = 70%:30%) to obtain compound 145-3 (3.00 g, 82.27%). ESI-MS m/z: 322.32 [M+H]⁺.

### Step 4: Synthesis of compound 145-4

Compound 145-3 (2900.00 mg, 6.88 mmol) and potassium carbonate (1902.76 mg, 13.77 mmol) were added to DMF (30.00 mL). In an ice-water bath, iodomethane (0.64 mL, 10.33 mmol) was added dropwise, and the reaction was carried out at room temperature for 2 h. Water (50 mL) was added to the reaction solution, and the resultant was extracted twice with 40 mL of EA, washed twice with 40 mL of saturated brine, dried over anhydrous sodium sulfate, and separated by column chromatography (PE:EA = 90%:10%) to obtain compound 145-4 (2500.00 mg, 83.43%). ESI-MS m/z: 336.27 [M+H]⁺.

### Step 5: Synthesis of compound 145-5

Compound 145-4 (2300.00 mg, 5.28 mmol), intermediate compound M2-7 (1712.98 mg, 5.28 mmol), PdCl₂(dppf) (385.82 mg, 0.53 mmol), potassium phosphate (2804.14 mg, 13.21 mmol), water (2.00 mL), and 1,4-dioxane (5.00 mL) were added to toluene (20.00 mL). The atmosphere was replaced with nitrogen, and the reaction was carried out at 70 °C for 10 h. Water (50 mL) was added to the reaction solution, extracted twice with 40 mL of EA, washed twice with 40 mL of saturated brine, dried over anhydrous sodium sulfate, and separated by column chromatography (PE:EA = 70%:30%) to obtain compound 145-5 (2000.00 mg, 68.49%). ESI-MS m/z: 453.50 [M+H]⁺.

### Step 6: Synthesis of compound 145-6

Compound 145-5 (2000.00 mg, 3.62 mmol) and sodium bicarbonate (364.83 mg, 4.34 mmol) were added to THF (2.00 mL). In an ice-water bath, a solution of silver trifluoromethanesulfonate (1115.81 mg, 4.34 mmol) in THF (2 mL) and a solution of iodine (826.66 mg, 3.26 mmol) in THF (2 mL) were added dropwise sequentially, and the reaction was kept in an ice-water bath for 10 min. The reaction solution was added dropwise to 50 mL of saturated aqueous sodium sulfite solution, and the resultant was extracted twice with 40 mL of EA, washed twice with 40 mL of saturated brine, dried over anhydrous sodium sulfate, and concentrated to dryness to obtain compound 145-6 (2350.00 mg, 95.70%). ESI-MS m/z: 579.20 [M+H]⁺.

### Step 7: Synthesis of compound 145-7

Compound 145-6 (2.35 g, 3.46 mmol) and lithium hydroxide monohydrate (0.87 g, 20.78 mmol) were added to tetrahydrofuran (20.00 mL) and water (20.00 mL), and the reaction was carried out at room temperature for 16 h. Water (50 mL) was added to the reaction solution, and the resultant was extracted twice with 40 mL of EA, washed twice with 40 mL of saturated brine, dried over anhydrous sodium sulfate, and concentrated to dryness to obtain compound 145-7 (1.60 g, 74.22%). ESI-MS m/z: 523.21 [M+H]⁺.

### Step 8: Synthesis of compound 145-8

Compound 145-7 (1.60 g, 2.57 mmol), compound (S)-tert-butyl hexahydropyridazine-3-carboxylate hydrochloride (0.44 g, 3.08 mmol), and N,N-diisopropylethylamine (2.55 mL, 15.42 mmol) were added to DMF (16.00 mL). In an ice-water bath, HATU (1.47 g, 3.86 mmol) was added, and the reaction was carried out in an ice-water bath for 1 h. Water (50 mL) was added to the reaction solution, and the mixture was extracted twice with 40 mL of EA, washed twice with 40 mL of saturated brine, dried over anhydrous sodium sulfate, and concentrated to dryness to obtain compound 145-8 (1.92 g, 99.78%). ESI-MS m/z: 649.27 [M+H]⁺.

### Step 9: Synthesis of compound 145-9

Compound 145-8 (1.92 g, 2.56 mmol) was added to THF (20.00 mL) and water (10.00 mL). In an ice-water bath, lithium hydroxide monohydrate (0.65 g, 15.39 mmol) was added, and the reaction was carried out at room temperature for 2 h. Water (50 mL) was added to the reaction solution, and the resultant was extracted twice with 40 mL of EA, washed twice with 40 mL of saturated brine, dried over anhydrous sodium sulfate, and concentrated to dryness to obtain compound 145-9 (1.80 g, 95.54%). ESI-MS m/z: 635.40, [M+H]⁺.

### Step 10: Synthesis of compound 145-10

Compound 145-9 (1.80 g, 2.45 mmol) and DIEA (4.86 mL, 29.40 mmol) were added to DCM (60.00 mL). In an ice-water bath, EDCI (2.82 g, 14.70 mmol) and HOBt (3.31 g, 24.50 mmol) were added sequentially, and the reaction was carried out at room temperature for 16 h. Water (50 mL) was added to the reaction solution, and the resultant was extracted twice with 40 mL of EA, washed twice with 40 mL of saturated brine, dried over anhydrous sodium sulfate, and separated by column chromatography (PE:EA = 70%:30%) to obtain compound 145-10 (1.10 g, 62.65%). ESI-MS m/z: 617.20 [M+H]⁺.

### Step 11: Synthesis of compound 145-11

Compound 145-10 (1.10 g, 1.53 mmol), Pd₂(dba)₃ (0.17 g, 0.18 mmol), SpHos (0.19 g, 0.46 mmol), and potassium acetate (0.53 g, 5.37 mmol) were added to toluene (10.00 mL). The atmosphere was replaced with nitrogen, pinacolborane (1.57 g, 12.28 mmol) was added in an ice-water bath, and then the reaction was carried out at 60 °C for 4 h. The reaction solution was concentrated to dryness and separated by column chromatography (PE:EA = 70%:30%) to obtain compound 145-11 (0.44 g, 40.00%). ESI-MS m/z: 617.47 [M+H]⁺.

### Step 12: Synthesis of compound 145-12

Intermediate 80-4 (251.67 mg, 0.49 mmol), compound 145-11 (440.00 mg, 0.61 mmol), PdCl₂(dppf) (44.93 mg, 0.06 mmol), and potassium carbonate (254.54 mg, 1.84 mmol) were added to toluene (5.00 mL), 1,4-dioxane (2.00 mL), and water (0.50 mL). The atmosphere was replaced with nitrogen, and the reaction was carried out at 65 °C for 2 h. The reaction solution was concentrated to dryness and separated by column chromatography (DCM:MeOH = 95%:5%) to obtain compound 145-12 (400.00 mg, 63.74%). ESI-MS m/z: 483.94 [M+H]⁺.

### Step 13: Synthesis of compound 145-13

Compound 145-12 (400.00 mg, 0.39 mmol) and cesium carbonate (382.48 mg, 1.17 mmol) were added to DMF (5.00 mL), and the reaction was carried out at 65 °C for 1 h. Water (50 mL) was added to the reaction solution, and the resultant was extracted twice with 40 mL of EA, washed twice with 40 mL of saturated brine, dried over anhydrous sodium sulfate, and separated by column chromatography (DCM:MeOH = 95%:5%) to obtain compound 145-13 (320.00 mg, 96.20%). ESI-MS m/z: 850.98 [M+H]⁺.

### Step 14: Synthesis of compound 145-14

Compound 145-13 (320.00 mg, 0.38 mmol) and HCl (4M in dioxane) (1.88 mL, 4.00 mol/L, 7.53 mmol) were added to dichloromethane (1.00 mL), and the reaction was carried out at room temperature for 1 h. The reaction solution was concentrated to dryness to obtain compound 145-14 (280.00 mg, 99.19%). ESI-MS m/z: 750.54 [M+H]⁺.

### Step 15: Synthesis of compound 145

Compound (1S,2S)-2-(difluoromethyl)cyclopropane-1-carboxylic acid (60.99 mg, 0.45 mmol) and DIEA (0.62 mL, 3.73 mmol) were added to acetonitrile (3.00 mL). In an ice-water bath, HATU (212.97 mg, 0.56 mmol) was added. After stirring at this temperature for 10 min, a solution of compound 145-14 (280.00 mg, 0.37 mmol) in DIEA (0.62 mL, 3.73 mmol) and acetonitrile (3.00 mL) was added, and the reaction was carried out at room temperature for 20 min. The mixture was concentrated to dryness and purified by preparative separation to obtain compound 145 (133.00 mg, 40.61%). ESI-MS m/z: 868.62 [M+H]⁺.

¹H NMR (500 MHz, DMSO)δ 8.78 (d, J = 8.6 Hz, 1H), 8.41 (d, J = 2.9 Hz, 1H), 7.94 (s, 1H), 7.64 (dd, J = 8.8, 1.5 Hz, 1H), 7.53 (d, J = 8.6 Hz, 1H), 7.25 (s, 1H), 7.20 (d, J = 2.8 Hz, 1H), 7.14 (s, 1H), 6.76 (s, 1H), 5.93 (td, J = 56.7, 5.1 Hz, 1H), 5.44 (td, J = 9.1, 2.2 Hz, 1H), 5.11 (d, J = 12.3 Hz, 1H), 4.27 (d, J = 11.0 Hz, 1H), 4.16 (q, J = 6.3 Hz, 1H), 4.05 - 3.99 (m, 3H), 3.79 (s, 3H), 3.63 (ddd, J = 19.9, 16.9, 7.5 Hz, 3H), 3.30 - 3.21 (m, 4H), 3.15 (dd, J = 12.4, 9.7 Hz, 1H), 3.04 (d, J = 14.5 Hz, 1H), 2.89 - 2.69 (m, 3H), 2.48 - 2.42 (m, 5H), 2.27 (t, J = 13.0 Hz, 1H), 2.22 (s, 3H), 2.10 (dd, J = 8.7, 4.5 Hz, 1H), 2.05 (d, J = 10.0 Hz, 1H), 1.86 - 1.76 (m, 2H), 1.71 - 1.58 (m, 2H), 1.54 (d, J = 9.1 Hz, 1H), 1.39 (d, J = 6.4 Hz, 3H), 0.99 (dt, J = 9.5, 4.6 Hz, 1H), 0.93 (s, 4H), 0.35 (s, 3H).

### Example 146: Synthesis of compound (1S,2S)-2-(difluoromethyl)-N-((1⁵S,6³S,4S,Z)-1⁵,10,10-trimethyl-1²-morpholino-5,7-dioxo-1⁸,1⁹, 6¹,6²,6³,6⁴,6^{5 6}-octahydro-1⁵H,1⁷H-8-oxa-1(13,15)-pyrido[3',2':7,8][1,5]oxazonino[5,6-a]indola -2(4,2)-oxazola-6(1,3)-pyridazinacycloundecaphane-4-yl)cyclopropane-1-carboxamide

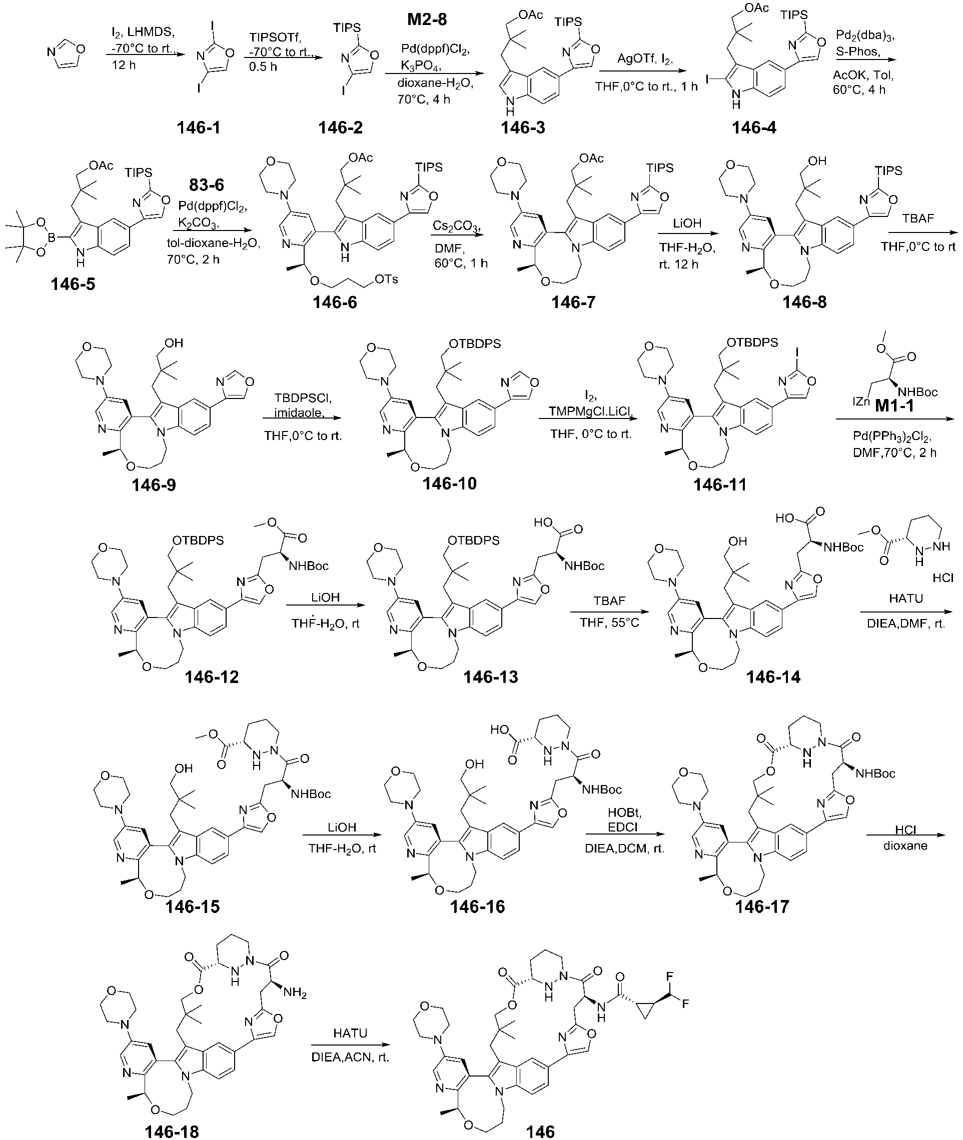

### Step 1: Synthesis of compound 146-1

Compound oxazole (2.1 g) was added to a 100 mL three-necked flask, anhydrous THF (13 mL) and DMPU (10.4 mL) were added, the temperature was lowered to -78 °C under N₂ protection, LHMDS (67 mL, 1 mol/L solution in THF) was slowly added dropwise, and the reaction was carried out at < -75 °C for 1.0 h. Iodine (15.4 g) was added in batches, the reaction was carried out at < -75 °C for 30 min, then transferred to room temperature and reacted for 8.0 h. After the reaction was complete, it was diluted with saturated aqueous sodium thiosulfate solution (20 mL), extracted with Et₂O, the organic phase was dried over anhydrous sodium sulfate, and purified by column chromatography (PE: EA = 9: 1) to obtain compound 146-1 (6.54 g, 67.3% yield). ESI-MS m/z: 321.1 [M+H]⁺.

### Step 2: Synthesis of compound 146-2

The above compound 146-1 (6.5 g) was added to a 500 mL three-necked flask, anhydrous THF (150 mL) was added, the temperature was lowered to -78 °C under N₂ protection, n-butyllithium (10 mL, 2.5 mol/L) was slowly added dropwise, and the reaction was carried out at < -75 °C for 10 min. Triisopropylsilyl trifluoromethanesulfonate (5.8 mL) was added dropwise, and after 15 min, the mixture was transferred to room temperature and reacted for 0.5 h. After the reaction was complete, it was diluted with saturated aqueous sodium chloride solution (20 mL), extracted with DCM, the organic phase was dried over anhydrous sodium sulfate, and purified by column chromatography (PE: DCM = 4: 1) to obtain compound 146-2 (3.29 g, 46.0% yield). ESI-MS m/z: 351.3 [M+H]⁺.

### Step 3: Synthesis of compound 146-3

Compound M2-8 (4.17 g), compound 146-2 (3.29 g), Pd(dppf)Cl₂ (1.37 g), and potassium phosphate (5.96 g) were weighed to a 100 mL single-necked flask, 1,4-dioxane (50 mL) and water (10 mL) were added, N₂ was replaced 3 times, and the mixture was warmed to 70 °C and reacted for 4 h. After the reaction was complete, EA and water were added to the reaction solution, extracted, the aqueous phase was further extracted twice with EA, the organic phases were collected and combined, dried over anhydrous sodium sulfate, concentrated, and the concentrate was purified by column chromatography (PE: EA = 4: 1) to obtain the target compound 146-3 (3.98 g, 90.7% yield). ESI-MS m/z: 468.7 [M+H]⁺.

### Step 4: Synthesis of compound 146-4

Intermediate 146-3 (3.58 g) and anhydrous THF (100 mL) were added to a 250 mL three-necked flask, the temperature was lowered to 0 °C under N₂ protection, silver trifluoromethanesulfonate (2.55 g) was added, then iodine (2.13 g dissolved in 40 mL of THF) was slowly added dropwise, and the mixture was warmed to room temperature and reacted for 30 min. After the reaction was complete, saturated sodium thiosulfate (40 mL) was added to the reaction solution, extracted with EA, the organic phase was collected, the aqueous phase was further extracted twice with EA (25 mL), the organic phases were collected and combined, dried over anhydrous sodium sulfate, and the concentrate was purified by column chromatography (PE: EA = 4: 1) to obtain the target compound 146-4 (3.42 g, 75.4% yield). ESI-MS m/z: 594.6 [M+H]⁺.

### Step 5: Synthesis of compound 146-5

Compound 146-4 (3.42 g), Pd₂(dba)₃ (1.05 g), and potassium acetate (1.70 g) were weighed to a 100 mL single-necked flask, toluene (30 mL) was added, N₂ was replaced 3 times, the temperature was lowered to 0 °C, pinacolborane (10 mL) was added dropwise, and the mixture was warmed to 60 °C and reacted for 2 h. After the reaction was complete, EA and water were added to the reaction solution, extracted, the aqueous phase was further extracted twice with EA, the organic phases were collected and combined, dried over anhydrous sodium sulfate, concentrated, and the concentrate was purified by column chromatography (PE: EA = 4: 1) to obtain the target compound 146-5 (2.07 g, 60.5% yield). ESI-MS m/z: 594.7 [M+H]⁺.

### Step 6: Synthesis of compound 146-6

Compound 146-5 (2.07 g), compound 83-6 (2.09 g), Pd(dppf)Cl₂ (0.2 g), and potassium carbonate (1.44 g) were weighed to a 100 mL single-necked flask, toluene (36 mL)-1,4-dioxane (12 mL) and water (12 mL) were added, N₂ was replaced 3 times, and the mixture was warmed to 70 °C and reacted for 2 h. After the reaction was complete, EA and water were added to the reaction solution, extracted, the aqueous phase was further extracted twice with EA, the organic phases were collected and combined, dried over anhydrous sodium sulfate, concentrated, and the concentrate was purified by column chromatography (DCM:MeOH = 12:1) to obtain the target compound 146-6 (2.0 g, 64.8% yield). ESI-MS m/z: 887.3 [M+H]⁺.

### Step 7: Synthesis of compound 146-7

Intermediate 146-6 (2.0 g) was added, Cs₂CO₃ (2.2 g) and anhydrous DMF (75 mL) were weighed to a 250 mL single-necked flask, and the reaction was carried out at 50 °C for 1 h under N₂ protection. After the reaction was complete, tap water (50 mL) was added for dilution, and the resultant was extracted with EA, the organic phase was dried over anhydrous sodium sulfate, and concentrated to obtain the target compound 146-7 (1.7 g, 100% yield). ESI-MS m/z: 715.1 [M+H]⁺.

### Step 8: Synthesis of compound 146-8

Intermediate 146-7 (1.05 g) was added, lithium hydroxide (0.36 g), THF (24 mL), and tap water (12 mL) were weighed to a 100 mL single-necked flask, and the reaction was carried out at 40 °C for 12 h. After the reaction was complete, tap water (50 mL) was added for dilution, extracted with EA, the organic phase was dried over anhydrous sodium sulfate, and concentrated to obtain the target compound 146-8 (1.1 g, 110% yield). ESI-MS m/z: 673.1 [M+H]⁺.

### Step 9: Synthesis of compound 146-9

Intermediate 146-8 (1.1 g) was added to a 50 mL single-necked flask, anhydrous THF (15 mL) was added, the temperature was lowered to 0 °C under N₂ protection, tetrabutylammonium fluoride (3.21 mL, 1 mol/L solution in THF) was slowly added dropwise, and the reaction was carried out at room temperature for 1.0 h. After the reaction was complete, calcium carbonate (1.53 g), DOWEX(R)50WX4 HYDROGEN FORM resin (2 g), and methanol (15 mL) were added respectively, stirred at room temperature for 1 h, the resultant was filtered, the filtrate was concentrated to dryness, and purified by column chromatography (10% NH₃ in MeOH/DCM) to obtain the target compound 146-9 (0.73 g, 87.7% yield). ESI-MS m/z: 516.7 [M+H]⁺.

### Step 10: Synthesis of compound 146-10

Intermediate 146-9 (0.73 g) was added to a 100 mL single-necked flask, anhydrous THF (15 mL) was added, the temperature was lowered to 0 °C under N₂ protection, imidazole (0.72 g) and tert-butylchlorodiphenylsilane (2.27 mL) were added respectively, and the reaction was carried out at room temperature for 2.0 h. After the reaction was complete, tap water (20 mL) was added for dilution, extracted with EA, the organic phase was dried over anhydrous sodium sulfate, and purified by column chromatography (PE:EA = 1:1) to obtain the target compound 146-10 (0.87 g, 81.7% yield). ESI-MS m/z: 755.2 [M+H]⁺.

### Step 11: Synthesis of compound 146-11

Intermediate 146-10 (0.83 g) was added to a 250 mL three-necked flask, anhydrous THF (50 mL) was added, the temperature was lowered to 0 °C under N₂ protection, TMP MgCl.LiCl (4.2 mL, 1 mol/L solution in THF) was slowly added dropwise, and the reaction was carried out at room temperature for 1.0 h. The temperature was lowered to 0 °C, iodine (0.42 g dissolved in 10 mL of THF) was added dropwise, and the reaction was carried out at room temperature for 1.0 h. After the reaction was complete, it was diluted with saturated aqueous ammonium chloride solution (20 mL), extracted with EA, the organic phase was dried over anhydrous sodium sulfate, and purified by column chromatography (PE:EA = 1:1) to obtain the target compound 146-11 (0.52 g, 53.6% yield). ESI-MS m/z: 881.1 [M+H]⁺.

### Step 12: Synthesis of compound 146-12

Compound 146-11 (0.47 g), bis(triphenylphosphine)palladium(II) dichloride (75 mg) and anhydrous DMF (10 mL) were weighed to a respective 100 mL single-necked flask, N₂ was replaced 3 times, finally compound M1-1 (4.85 mL, 0.33 mol/L solution in DMF) was added, and the mixture was warmed to 70 °C and reacted for 2.5 h. After the reaction was complete, the mixture was cooled to room temperature, EA and water were added to the reaction solution, extracted, the aqueous phase was further extracted twice with EA, the organic phases were collected and combined, dried over anhydrous sodium sulfate, concentrated, and the concentrate was purified by column chromatography (PE:EA = 2:3) to obtain the target compound 146-12 (0.54 g, 106% yield). ESI-MS m/z: 956.5 [M+H]⁺.

### Step 13: Synthesis of compound 146-13

In to a 50 mL single-necked flask, intermediate 146-12 (542 mg) was added, lithium hydroxide (58 mg), THF (8 mL), and tap water (4 mL) were weighed, and the reaction was carried out at room temperature for 1 h. After the reaction was complete, tap water (50 mL) was added for dilution, extracted with EA, the organic phase was dried over anhydrous sodium sulfate, and concentrated to obtain the target compound 146-13 (520 mg, 100% yield). ESI-MS m/z: 942.3 [M+H]⁺.

### Step 14: Synthesis of compound 146-14

Intermediate 146-13 (0.52 g) was added to a 25 mL single-necked flask, anhydrous THF (15 mL) was added, the temperature was lowered to 0 °C under N₂ protection, tetrabutylammonium fluoride (2 mL, 1 mol/L solution in THF) was slowly added dropwise, and the mixture was warmed to 55 °C and reacted for 10.0 h. After the reaction was complete, calcium carbonate (1.3 g), DOWEX(R)50WX4 HYDROGEN FORM resin (1.5 g), and methanol (10 mL) were added respectively, stirred at room temperature for 1 h, filtered, the filtrate was concentrated to dryness, and purified by column chromatography (DCM/MeOH = 6:1) to obtain the target compound 146-14 (0.29 g, 71.4% yield). ESI-MS m/z: 703.8 [M+H]⁺.

### Step 15: Synthesis of compound 146-15

Intermediate 146-14 (285 mg) and (S)-tert-butyl hexahydropyridazine-3-carboxylate (70 mg) were added to a 25 mL single-necked flask, N,N-diisopropylethylamine (0.4 mL) and anhydrous DMF (5 mL) were weighed; the temperature was lowered to 0 °C, HATU (231 mg) was added, and the reaction was carried out at room temperature for 1 h. After the reaction was complete, tap water (50 mL) was added for dilution, extracted with EA, the organic phase was dried over anhydrous sodium sulfate, and concentrated to obtain the target compound 146-15 (496 mg, 147% yield). ESI-MS m/z: 830.3 [M+H]⁺.

### Step 16: Synthesis of compound 146-16

Intermediate 146-15 (496 mg) was added, lithium hydroxide (90 mg), THF (5 mL), and tap water (2.5 mL) were weighed to a 25 mL single-necked flask, and the reaction was carried out at room temperature for 1 h. After the reaction was complete, it was acidified with saturated aqueous ammonium chloride solution (50 mL), extracted with EA, the organic phase was dried over anhydrous sodium sulfate, and concentrated to obtain the target compound 146-16 (363 mg, 74.4% yield). ESI-MS m/z: 816.0 [M+H]⁺.

### Step 17: Synthesis of compound 146-17

Intermediate 146-16 (363 mg) was added, N,N-diisopropylethylamine (0.5 mL) and anhydrous DCM (40 mL) were weighed to a 100 mL single-necked flask, the temperature was lowered to 0 °C under nitrogen protection, HOBt (300 mg) and EDCI (256 mg) were added, and the reaction was carried out at room temperature for 18 h. After the reaction was complete, it was diluted with saturated brine (50 mL), extracted with DCM/MeOH (10:1), the organic phase was dried over anhydrous sodium sulfate, and purified by column chromatography (DCM/MeOH = 9:1) to obtain the target compound 146-17 (0.24 g, 57.4% yield). ESI-MS m/z: 798.1 [M+H]⁺.

### Step 18: Synthesis of compound 146-18

Intermediate 146-17 (240 mg) was added to a 50 mL single-necked flask, hydrogen chloride in dioxane solution (30 mL) was weighed in, and the reaction was carried out at room temperature for 1 h. After the reaction was complete, it was directly concentrated under reduced pressure to obtain the hydrochloride salt of the target compound 146-18 (252 mg, 120% yield). ESI-MS m/z: 697.8 [M+H]⁺.

### Step 19: Synthesis of compound 146

Compound (1S,2S)-2-(difluoromethyl)cyclopropane-1-carboxylic acid (32 mg) was added, HATU (68 mg), N,N-diisopropylethylamine (0.2 mL) and anhydrous acetonitrile (25 mL) were weighed to a 25 mL single-necked flask, and the reaction was carried out at room temperature for 10 min; a pre-liberated acetonitrile solution (20 mL) of compound 146-18 (252 mg) was added dropwise at room temperature, and the reaction was carried out at room temperature for 15 min. After the reaction was complete, it was directly concentrated, and the concentrate was purified by preparative TLC (dichloromethane/7M ammonia in methanol = 15:1) to obtain the target compound 146 (26.7 mg, 26.9% yield). ESI-MS m/z: 816.6 [M+H]⁺.

¹H NMR (500 MHz, CDCl₃)δ 8.90 (d, J = 18.6 Hz, 1H), 8.36 (s, 1H), 7.80 (d, J = 12.5 Hz, 1H), 7.43 - 7.36 (m, 2H), 7.30 (d, J = 8.5 Hz, 1H), 6.87 (d, J = 7.8 Hz, 1H), 5.83 (dd, J = 10.8, 3.1 Hz, 2H), 4.62 (d, J = 12.4 Hz, 1H), 4.49 (d, J = 11.2 Hz, 1H), 4.31 - 4.21 (m, 7H), 4.05 (dd, J = 14.6, 4.7 Hz, 2H), 3.91 (s, 6H), 3.78 (d, J = 11.0 Hz, 2H), 3.70 (d, J = 11.3 Hz, 2H), 3.36 (s, 5H), 3.30 - 3.19 (m, 3H), 2.99 (dd, J = 15.5, 5.0 Hz, 1H), 2.67 (t, J = 11.6 Hz, 1H), 2.03 - 1.96 (m, 3H), 1.90 - 1.82 (m, 3H), 1.80 - 1.76 (m, 1H), 1.63 (d, J = 6.5 Hz, 4H), 1.58 (dd, J = 12.7, 3.8 Hz, 1H), 1.36 (dd, J = 9.5, 4.8 Hz, 1H), 1.31 (d, J = 6.6 Hz, 9H), 1.25 (s, 2H), 1.16 - 1.10 (m, 1H), 1.05 - 0.99 (m, 4H), 0.96 - 0.86 (m, 1H).

### Example 149: Synthesis of compound N-((2S)-1-(((1⁵S,6³S,4S,Z)-1²-(4-ethylpiperazin-1-yl)-1⁵,10,10-trimethyl-5,7-dioxo-1⁸,1⁹,6¹,6²,6³ ,6⁴,6⁵,6⁶-octahydro-1⁵H,1⁷H-8-oxa-1(13,15)-pyrido[3',2':7,8][1,5]oxazonino[5,6-a]indola-2(4,2)-thiazola-6(1,3)-pyridazinacycloundecaphane-4-yl)amino)-3-methyl-1-oxobutan-2-yl)-N-methyl morpholine-4-carboxamide

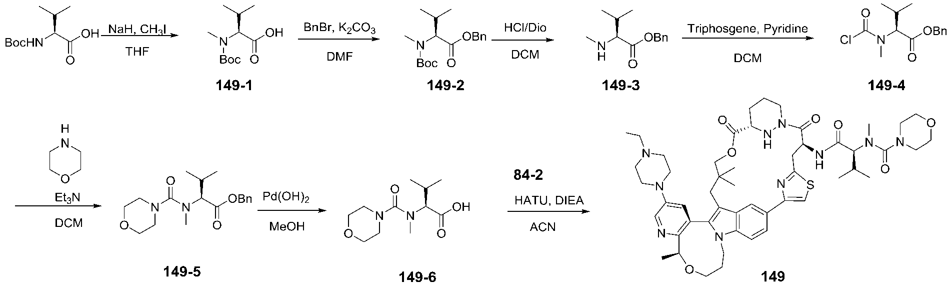

### Step 1: Synthesis of compound 149-1

(Tert-butoxycarbonyl)-L-valine (5.00 g, 23.01 mmol), iodomethane (32.67 g, 230.14 mmol), and THF (100.00 mL) were added sequentially to a 250 mL three-necked flask. Under nitrogen protection, with ice-water bath cooling, NaH (9.2 g, 60% content, 230.14 mmol) was slowly added. The reaction was kept in an ice-water bath for 1 h, and then reacted at room temperature for 20 h. After the reaction was complete as monitored by LC-MS, the reaction solution was slowly added to 150 mL of ice water to quench the reaction, then extracted three times with 60 mL of EA. The organic phases were combined, washed with saturated sodium chloride, dried over anhydrous sodium sulfate, and concentrated to dryness to obtain a crude product of compound 149-1, which was purified by silica gel column chromatography (PE to PE/EA = 90/10) to finally obtain the target product 149-1 (5.30 g, light yellow solid). ESI-MS m/z: 232.34 [M+H]⁺.

### Step 2: Synthesis of compound 149-2

Compound 149-1 (5.30 g, 22.92 mmol), benzyl bromide (4.70 g, 27.50 mmol), potassium carbonate (4.75 g, 34.37 mmol), and DMF (50.00 mL) were added sequentially to a 250 mL three-necked flask. Under nitrogen protection, the reaction was carried out at room temperature for 2 h. After the reaction was complete as monitored by LC-MS, the reaction solution was slowly added to 60 mL of ice water to quench the reaction, then extracted three times with 50 mL of EA. The organic phases were combined, washed with saturated sodium chloride, dried over anhydrous sodium sulfate, and concentrated to dryness to obtain a crude product of compound 149-2, which was purified by silica gel column chromatography (PE to PE/EA = 90/10) to finally obtain the target product 149-2 (6.80 g, light yellow liquid). ESI-MS m/z: 352.43 [M+H]⁺.

### Step 3: Synthesis of compound 149-3

Compound 149-2 (6.80 g, 19.35 mmol), DCM (15.00 mL), and HCl (4M in dioxane) (24.19 mL) were added sequentially to a 100 mL single-necked flask. Under nitrogen protection, the reaction was carried out at room temperature for 2 h. After the reaction was complete as monitored by LC-MS, the reaction solution was slowly added to 60 mL of ice water to quench the reaction. Then saturated sodium bicarbonate was added to adjust the pH to 8-9, followed by extraction three times with 50 mL of EA. The organic phases were combined, washed with saturated sodium chloride, dried over anhydrous sodium sulfate, and concentrated to dryness to obtain a crude product of compound 149-3, which was purified by silica gel column chromatography (PE to PE/EA = 50/50) to finally obtain the target product 149-3 (4.30 g, light yellow liquid). ESI-MS m/z: 252.31 [M+H]⁺.

### Step 4: Synthesis of compound 149-4

Compound 149-3 (1.00 g, 4.52 mmol) and DCM (10.00 mL) were added sequentially to a 100 mL three-necked flask. Under nitrogen protection, with ice-water bath cooling, a mixed solution of triphosgene (0.47 g, 1.58 mmol), pyridine (1.07 g, 13.56 mmol), and DCM (10.00 mL) was slowly added, and the mixture was warmed to room temperature and reacted for 3 h. After the reaction was complete as monitored by LC-MS, the reaction was quenched by slowly adding the reaction solution to 30 mL of EA. The mixture was stirred and reacted at room temperature for 0.5 h, a solid precipitated, which was directly filtered, and the filter cake was washed three times with 5 mL of EA. The organic phases were combined to obtain the target product 149-4 (1.25 g, light yellow liquid). ESI-MS m/z: 284.71 [M+H]⁺.

### Step 5: Synthesis of compound 149-5

Compound 149-4 (6.80 g, 19.35 mmol), DCM (15.00 mL), and triethylamine (1.56 g, 15.42 mmol) were added sequentially to a 100 mL single-necked flask. Under nitrogen protection, with ice-water bath cooling, morpholine (0.42 g, 4.85 mmol) was slowly added, and then the mixture was warmed to room temperature and reacted for 1 h. After the reaction was complete as monitored by LC-MS, the reaction solution was slowly added to 60 mL of ice water to quench the reaction, then extracted three times with 50 mL of EA. The organic phases were combined, washed with saturated sodium chloride, dried over anhydrous sodium sulfate, and concentrated to dryness to obtain a crude product of compound 149-5, which was purified by silica gel column chromatography (PE to PE/EA = 50/50) to finally obtain the target product 149-5 (1.20 g, light yellow liquid). ESI-MS m/z: 335.12 [M+H]⁺.

### Step 6: Synthesis of compound 149-6

Compound 149-5 (1.20 g, 3.59 mmol), Pd(OH)₂ (0.50 g, 3.59 mmol), and MeOH (25.00 mL) were added sequentially to a 100 mL single-necked flask. Under a hydrogen atmosphere, morpholine (0.42 g, 4.85 mmol) was slowly added, and the reaction was carried out at room temperature for 4 h. After the reaction was complete as monitored by LC-MS, the reaction solution was directly filtered through celite, and the filter cake was washed three times with 10 mL of MeOH. The organic phase was concentrated to dryness to obtain a crude product of compound 149-6, which was purified by silica gel column chromatography (DCM to DCM/MeOH = 85/15) to finally obtain the target product 149-6 (0.50 g, light yellow liquid). ESI-MS m/z: 245.33 [M+H]⁺.

### Step 7: Synthesis of compound 149

Compound 149-6 (52.77 mg, 0.22 mmol), HATU (82.13 mg, 0.22 mmol), and DIEA (0.09 mL, 0.54 mmol) were added sequentially to a 10 mL single-necked flask, and the mixture was stirred at room temperature for 15 min. The mixture was cooled in an ice-water bath, and then a solution of compound 84-2 (80.00 mg, 0.11 mmol) in acetonitrile was added. After the reaction was complete as monitored by LC-MS, the reaction solution was directly purified by Prep-HPLC basic method, and the eluate was lyophilized to finally obtain the target compound 149 (45.00 mg). ESI-MS m/z: 968.08, [M+H]⁺.

¹H NMR (500 MHz, Chloroform-d) δ 8.60 (s, 1H), 8.43 (d, J = 2.9 Hz, 1H), 8.00 (s, 1H), 7.56 (d, J = 8.5 Hz, 1H), 7.31 (d, J = 8.5 Hz, 1H), 7.09 (d, J = 2.9 Hz, 1H), 5.76 (t, J = 8.9 Hz, 1H), 4.60 (d, J = 13.0 Hz, 1H), 4.37-4.30 (m, 1H), 4.28-4.17 (m, 2H), 4.02-3.90 (m, 5H), 3.85-3.68 (m, 5H), 3.46-3.25 (m, 10H), 3.23-3.11 (m, 2H), 2.91 (s, 3H), 2.73-2.64 (m, 2H), 2.41 (d, J = 14.4 Hz, 1H), 2.33 (d, J = 13.2 Hz, 2H), 2.25 (d, J = 13.0 Hz, 1H), 1.96 (d, J = 12.4 Hz, 2H), 1.82-1.77 (m, 1H), 1.73-1.57 (m, 5H), 1.51 (d, J = 6.4 Hz, 3H), 1.25-1.23 (m, 3H), 0.98 (d, J = 6.3 Hz, 6H), 0.89 (d, J = 6.7 Hz, 3H), 0.40 (s, 3H).

### Example 150: Synthesis of compound (1S,2S)-2-(difluoromethyl)-N-((1⁵S,6³S,4S,Z)-1⁵,10,10-trimethyl-1²-(6-methyl-3,6-diazabicyclo[ 3.1.1]heptan-3-yl)-5,7-dioxo-1⁸,1⁹,6¹,6²,6³,6⁴,6⁵,6⁶-octahydro-1⁵H,1⁷H-8-oxa-1(13,15)-pyrido[3', 2':7,8][1,5]oxazonino[5,6-a]indola-2(4,2)-thiazola-6(1,3)-pyridazinacycloundecaphane-4-yl)cycl opropane-1-carboxamide

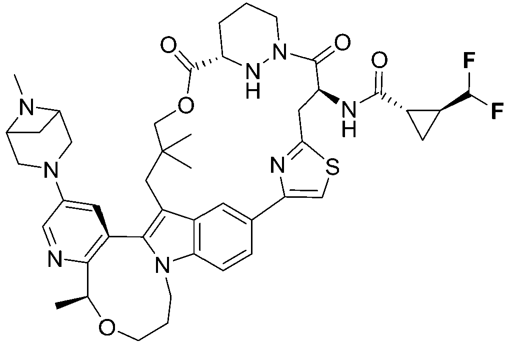

ESI-MS m/z:851.7 [M+H]⁺.

¹H NMR (500 MHz, CDCl₃)δ 8.59 (s, 1H), 8.31 (s, 1H), 7.57 (d, J = 8.5 Hz, 1H), 7.32 (d, J = 8.5 Hz, 1H), 7.29 (s, 1H), 6.88 (s, 1H), 6.68 (d, J = 9.4 Hz, 1H), 5.90 (t, J = 8.8 Hz, 1H), 5.83 (t, J = 57.6 Hz, 1H), 4.59 (d, J = 12.6 Hz, 1H), 4.36 (t, J = 10.9 Hz, 1H), 4.28 - 4.17 (m, 2H), 4.05 - 3.98 (m, 2H), 3.86 - 3.69 (m, 5H), 3.59 (t, J = 11.5 Hz, 2H), 3.47 (d, J = 14.9 Hz, 1H), 3.41 - 3.29 (m, 3H), 3.22 - 3.11 (m, 2H), 2.66 (dd, J = 26.6, 13.9 Hz, 2H), 2.49 (d, J = 14.2 Hz, 1H), 2.35 - 2.17 (m, 4H), 1.98 (d, J = 12.6 Hz, 2H), 1.86 - 1.79 (m, 2H), 1.71 (d, J = 8.0 Hz, 2H), 1.61 (d, J = 12.6 Hz, 2H), 1.53 (d, J = 6.1 Hz, 3H), 1.33-1.25 (m, 2H), 0.98 (s, 3H), 0.44 (s, 3H).

### Example 152: Synthesis of compound (1S,2S)-2-(difluoromethyl)-N-((1⁵S,6³S,4S,Z)-1⁵,10,10-trimethyl-5,7-dioxo-1²-(3,3,4-trimethylpi perazin-1-yl)-1⁸,1⁹,6¹,6²,6³ ,6⁴,6⁵,6⁶-octahydro-1⁵H,1⁷H-8-oxa-1(13,15)-pyrido[3',2':7,8][1,5]oxaz onino[5,6-a]indola-2(4,2)-thiazola-6(1,3)-pyridazinacycloundecaphane-4-yl)cyclopropane-1-carb oxamide

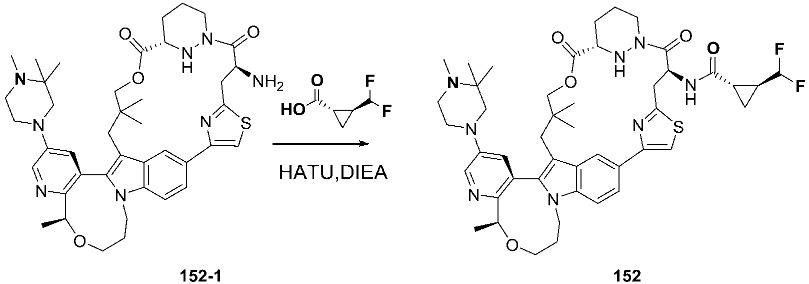

(1S,2S)-2-(difluoromethyl)cyclopropane-1-carboxylic acid (43.27 mg, 0.32 mmol) and N,N-dimethylformamide (3.00 mL) were added sequentially to a 10 mL single-necked flask. At 0 °C, N,N,N',N'-tetramethyl-O-(7-azabenzotriazol-1-yl)uronium hexafluorophosphate (120.88 mg, 0.32 mmol) and N,N-diisopropylethylamine (0.28 mL, 1.70 mmol) were added, and the mixture was stirred at 0 °C for 10 min. Finally, compound 152-1 (160.00 mg, 0.21 mmol) was added, and the mixture was stirred at 0 °C for 10 min. The reaction was complete as monitored by LC-MS, and the reaction solution was directly purified by pre-HPLC to obtain the target product 152 (103.4 mg). ESI-MS m/z: 873.4 [M+H]⁺.

¹H NMR (500 MHz, Chloroform-d)δ 8.52 (d, J = 1.6 Hz, 1H), 8.32 (d, J = 2.9 Hz, 1H), 7.50 (dd, J = 8.5, 1.7 Hz, 1H), 7.26 - 7.20 (m, 2H), 6.97 (d, J = 2.9 Hz, 1H), 6.64 (d, J = 9.5 Hz, 1H), 5.95 - 5.61 (m, 2H), 4.53 (d, J = 12.4 Hz, 1H), 4.29 (td, J = 12.1, 3.4 Hz, 1H), 4.14 (dq, J = 15.3, 6.1 Hz, 2H), 3.98 - 3.89 (m, 2H), 3.79 - 3.68 (m, 3H), 3.40 (d, J = 14.9 Hz, 1H), 3.24 (dq, J = 13.2, 4.7, 4.0 Hz, 3H), 3.15 - 3.04 (m, 2H), 2.96 (s, 2H), 2.64 (ddd, J = 25.8, 11.9, 4.3 Hz, 3H), 2.35 (d, J = 14.4 Hz, 1H), 2.28 - 2.16 (m, 5H), 1.94 - 1.81 (m, 3H), 1.76 (dt, J = 9.0, 4.6 Hz, 2H), 1.54 (qd, J = 12.9, 4.0 Hz, 1H), 1.44 (d, J = 6.5 Hz, 3H), 1.25 (dt, J = 9.5, 4.9 Hz, 1H), 1.06 (d, J = 4.3 Hz, 7H), 0.91 (s, 3H), 0.35 (s, 3H).

### Example 153: Synthesis of compound (1S,2S)-2-(difluoromethyl)-N-((1⁵S,6³S,4S,Z)-1²-(2,2-dimethylmorpholino)-1⁵,10,10-trimethyl-5 ,7-dioxo-1⁸,1⁹,6¹,6²,6³,6⁴,6⁵,6⁶-octahydro-1⁵H,¹⁷H-8-oxa-1(13,15)-pyrido[3',2':7,8][1,5]oxazonin o[5,6-a]indola-2(4,2)-thiazola-6(1,3)-pyridazinacycloundecaphane-4-yl)cyclopropane-1-carboxa mide

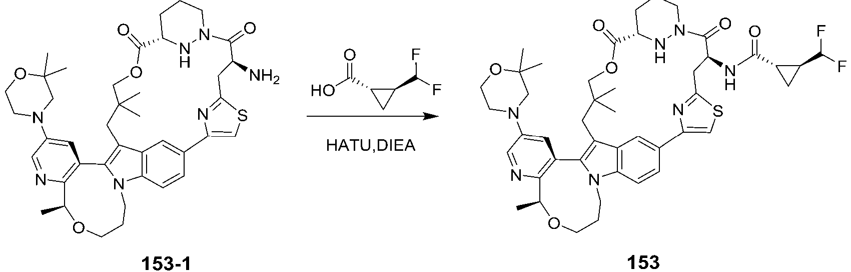

### Step 1: Synthesis of compound 153

(1S,2S)-2-(difluoromethyl)cyclopropane-1-carboxylic acid (38.41 mg, 0.28 mmol) and acetonitrile (5.00 mL) were added sequentially to a 10 mL single-necked flask. At 0 °C, N,N,N',N'-tetramethyl-O-(7-azabenzotriazol-1-yl)uronium hexafluorophosphate (122.66 mg, 0.32 mmol) and N,N-diisopropylethylamine (0.13 mL, 0.81 mmol) were added, and the mixture was stirred at 0 °C for 10 min. Finally, compound 153-1 (150.00 mg, 0.20 mmol) was added, and the mixture was stirred at 0 °C for 10 min. The reaction was complete as monitored by LC-MS, and the reaction solution was directly purified by pre-HPLC to obtain the target compound 153 (63.00 mg). ESI-MS m/z: 860.4 [M+H]⁺.

¹H NMR (500 MHz, CDCl₃)δ 8.57 (s, 1H), 8.39 (d, J = 2.8 Hz, 1H), 7.57 (dd, J = 8.5, 1.1 Hz, 1H), 7.31 (d, J = 8.1 Hz, 1H), 7.29 (s, 1H), 7.03 (d, J = 2.7 Hz, 1H), 6.69 (d, J = 9.7 Hz, 1H), 5.90 (t, J = 8.9 Hz, 1H), 5.83 (td, J = 57.6, 2.9 Hz, 1H), 4.62 - 4.56 (m, 1H), 4.35 (td, J = 12.1, 3.3 Hz, 1H), 4.24 (q, J = 6.4 Hz, 1H), 4.21 (dd, J = 12.3, 5.7 Hz, 1H), 4.05 - 3.96 (m, 2H), 3.94 - 3.89 (m, 2H), 3.83 (s, 3H), 3.47 (d, J = 15 Hz, 1H), 3.31 (td, J = 12.4, 2.9 Hz, 1H), 3.23 - 3.11 (m, 4H), 3.04 (td, J = 17.0, 12.1 Hz, 2H), 2.68 (td, J = 12.8, 2.5 Hz, 1H), 2.42 (d, J = 15.6 Hz, 1H), 2.36 - 2.20 (m, 2H), 2.02 - 1.94 (m, 2H), 1.85 - 1.80 (m, 2H), 1.63 - 1.56 (m, 2H), 1.52 (d, J = 6.4 Hz, 3H), 1.34 (s, 6H), 1.32 - 1.29 (m, 1H), 1.18 - 1.12 (m, 1H), 0.98 (s, 3H), 0.42 (s, 3H).

### Example 154: Synthesis of compound (1S,2S)-2-(difluoromethyl)-N-((1⁵S,6³S,4S,Z)-1⁵,10,10-trimethyl-1²-(5-methyl-2,5-diazabicyclo[ 2.2.2]octan-2-yl)-5,7-dioxo-1⁸,1⁹,6¹,6²,6³,6⁴,6⁵,6⁶-octahydro-1⁵H,1⁷H-8-oxa-1(13,15)-pyrido[3',2 ':7,8][1,5]oxazonino[5,6-a]indola-2(4,2)-thiazola-6(1,3)-pyridazinacycloundecaphane-4-yl)cyclo propane-1-carboxamide

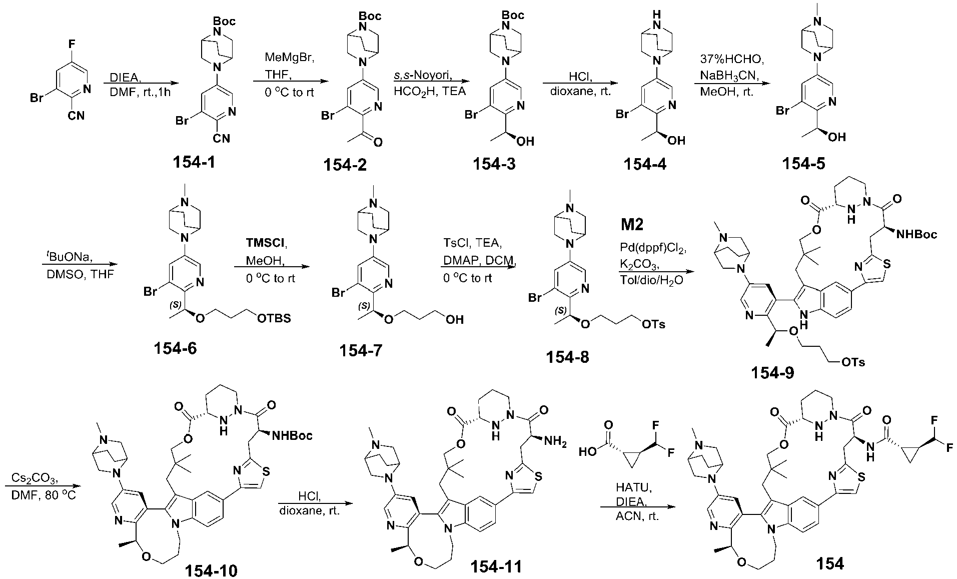

### Step 1: Synthesis of compound 154-1

Compound 3-bromo-5-fluoro-pyridine-2-carbonitrile (1.82 g) was added to a 250 mL single-necked flask, anhydrous DMF (20 mL) was added, TEA (10 mL) and tert-butyl 2,5-diazabicyclo[2.2.1]heptane-2-carboxylate (2.02 g) were weighed and added respectively, and the reaction was carried out at room temperature for 0.5 h. After the reaction was complete, tap water (200 mL) was added to precipitate a solid, stirred for 0.5 h, filtered with suction to obtain a solid, and dried to obtain the white target compound 154-1 (3.45 g, 96% yield). ESI-MS m/z: 393.6 [M+H]⁺.

### Step 2: Synthesis of compound 154-2

Intermediate 154-1 (3.45 g) and anhydrous THF (100 mL) were added to a 250 mL three-necked flask, the temperature was lowered to 0 °C under N₂ protection, MeMgBr (3 M in THF, 11 mL) was slowly added dropwise, and the mixture was warmed to room temperature and reacted for 2 h. After the reaction was complete, HCl (1N, 30 mL) was added to the reaction solution, extracted with EA, the organic phase was collected, the aqueous phase was further extracted twice with EA (30 mL), the organic phases were collected and combined, dried over anhydrous sodium sulfate, and the concentrate was purified by column chromatography (PE:EA = 1:1) to obtain the target compound 154-2 (1.46 g, 41% yield). ESI-MS m/z: 410.4 [M+H]⁺.

### Step 3: Synthesis of compound 154-3

Intermediate 154-2 (1.46 g) was added to a 25 mL single-necked flask, TEA (6 mL) and s,s-Noyori catalyst (0.11 g) were added, the temperature was lowered to 0 °C under N₂ protection, formic acid (0.82 g) was slowly added dropwise, and the reaction was carried out at room temperature for 12 h. After the reaction was complete, ethyl acetate (50 mL) and water (50 mL) were added to the reaction solution, extracted, the organic phase was collected, the aqueous phase was further extracted twice with ethyl acetate (25 mL), the organic phases were collected and combined, dried over anhydrous sodium sulfate, concentrated, and the concentrate was purified by column chromatography (PE:EA = 1:1) to obtain the target compound 154-3 (1.34 g, 91.2% yield). ESI-MS m/z: 412.4 [M+H]⁺.

### Step 4: Synthesis of compound 154-4

Compound 154-3 (1.34 g) was added to a 25 mL single-necked flask, 10 mL of hydrochloric acid (4N in dioxane) was added, and the reaction was carried out at room temperature for 1 h. After the reaction was complete, it was concentrated under reduced pressure to dryness to obtain the target compound 154-4 hydrochloride (1.63 g, 144% yield). ESI-MS m/z: 312.3 [M+H]⁺.

### Step 5: Synthesis of compound 154-5

Intermediate 154-4 (1.63 g) was added to a 100 mL single-necked flask, acetic acid (3 drops) and methanol (15 mL) were added, and after reacting at room temperature for 10 min, 37% aqueous formaldehyde solution (1.27 g) and NaBH₃CN (0.98 g) were added respectively, and the reaction was carried out at room temperature for 20 min. After the reaction was complete, dichloromethane (20 mL) and water (10 mL) were added to the reaction solution, extracted, the organic phase was collected, the aqueous phase was further extracted twice with dichloromethane (25 mL), the organic phases were collected and combined, dried over anhydrous sodium sulfate, concentrated under reduced pressure, and the concentrate was purified by column chromatography (dichloromethane:7 M ammonia in methanol solution = 10:1) to obtain the target axially chiral compound 154-5 (0.98 g, 58% yield). ESI-MS m/z: 326.5 [M+H]⁺.

### Step 6: Synthesis of compound 154-6

Compound 154-5 (1.3 g), 3-((tert-butyldimethylsilyl)oxy)propyl-4-methylbenzenesulfonate (2.23 g) and DMSO/THF (10 mL/2.5 mL) were weighed and added to the reaction flask respectively, and the temperature was lowered to 10 °C under N₂ protection; tBuONa (1.16 g) was added, and the reaction was carried out at room temperature for 0.5 h; the reaction was complete, the temperature was lowered to 10 °C, tap water (50 mL) was added for dilution, extracted with EA, the organic phase was dried over anhydrous sodium sulfate, concentrated, and the concentrate was purified by column chromatography to obtain compound 154-6 (1.93 g, crude product). ESI-MS m/z: 499.1 [M+H]⁺.

### Step 7: Synthesis of compound 154-7

Intermediate 154-6 (1.93 g) was added to a 50 mL single-necked flask, anhydrous methanol (20 mL) was added, the temperature was lowered to 0 °C under N₂ protection, TMSCl (0.98 mL) was slowly added dropwise, and the reaction was carried out at room temperature for 0.5 h. After the reaction was complete, it was directly concentrated to dryness to obtain the target compound 154-7 (1.78 g, crude product). ESI-MS m/z: 384.5 [M+H]⁺.

### Step 8: Synthesis of compound 154-8

Intermediate 154-7 (1.78 g) was added to a 50 mL single-necked flask, anhydrous DCM (20 mL) was added, TEA (6 mL) and DMAP (30 mg) were weighed and added respectively, the temperature was lowered to 0 °C under N₂ protection, TsCl (2.64 g) was added, and the reaction was carried out at room temperature for 2.0 h. After the reaction was complete, saturated NaCl (25 mL) was added, extracted with DCM, the organic phase was concentrated to dryness, and the concentrate was purified by column chromatography (dichloromethane:methanol = 6:1) to obtain the target compound 154-8 (1.24 g, 50% yield). ESI-MS m/z: 539.1 [M+H]⁺.

### Step 9: Synthesis of compound 154-9

Compound 154-8 (291 mg), compound M2 (100 mg), Pd(dppf)Cl₂ (20 mg), and potassium carbonate (60 mg) were weighed to a respective 100 mL single-necked flask, toluene (6 mL), 1,4-dioxane (2 mL) and 2 mL of water were added, N₂ was replaced 3 times, and the mixture was warmed to 70 °C and reacted for 3 h. After the reaction was complete, EA and water (20 mL each) were added to the reaction solution, extracted, the aqueous phase was further extracted twice with DCM, the organic phases were collected and combined, dried over anhydrous sodium sulfate, concentrated, and the concentrate was purified by column chromatography (dichloromethane:methanol = 10:1) to obtain the target compound 154-9 (114.9 mg, 77.8% yield). ESI-MS m/z: 1025.4 [M+H]⁺.

### Step 10: Synthesis of compound 154-10

Intermediate 154-9 (114.9 mg) was added to a 25 mL single-necked flask, Cs₂CO₃ (110 mg) and anhydrous DMF (10 mL) were weighed in, and the reaction was carried out at 80 °C for 1 h under N₂ protection. After the reaction was complete, tap water (50 mL) was added for dilution, extracted with EA, the organic phase was dried over anhydrous sodium sulfate, concentrated, and the concentrate was purified by column chromatography (dichloromethane:7 M ammonia in methanol solution = 10:1) to obtain the target compound 154-10 (85.9 mg, 89.7% yield). ESI-MS m/z: 853.3 [M+H]⁺.

### Step 11: Synthesis of compound 154-11

Compound 154-10 (85.9 mg) was added to a 25 mL single-necked flask, 10 mL of hydrochloric acid (4M in Dioxane) was added, and the reaction was carried out at room temperature for 1 h. After the reaction was complete, it was concentrated under reduced pressure to dryness to obtain the target compound 154-11 hydrochloride (83 mg, 109% yield). ESI-MS m/z: 753.2 [M+H]⁺.

### Step 12: Synthesis of compound 154

Compound (1S,2S)-2-(difluoromethyl)cyclopropane-1-carboxylic acid (27 mg) was added to a 25 mL single-necked flask, HATU (55 mg), N,N-diisopropylethylamine (0.16 mL) and anhydrous acetonitrile (10 mL) were weighed, and the reaction was carried out at room temperature for 10 min; a pre-liberated acetonitrile solution (10 mL) of compound 154-11 (83 mg) was added dropwise at room temperature, and the reaction was carried out at room temperature for 30 min. After the reaction was complete, it was directly concentrated, and the concentrate was purified by preparation (60% acetonitrile/water) to obtain the target compound 154 (41.3 mg, 48.5% yield). ESI-MS m/z: 871.6 [M+H]⁺.

¹H NMR (500 MHz, CDCl₃)δ 8.58 (d, J = 1.3 Hz, 1H), 8.20 (dd, J = 2.7, 1.4 Hz, 1H), 7.57 (dd, J = 8.6, 1.4 Hz, 1H), 7.33 - 7.27 (m, 2H), 6.83 - 6.73 (m, 2H), 6.00 - 5.67 (m, 2H), 4.60 (d, J = 11.8 Hz, 1H), 4.36 (td, J = 12.1, 3.0 Hz, 1H), 4.21 (dt, J = 12.4, 6.0 Hz, 2H), 4.01 (dd, J = 22.6, 8.2 Hz, 2H), 3.96 - 3.87 (m, 2H), 3.86 - 3.76 (m, 3H), 3.47 (d, J = 14.8 Hz, 1H), 3.30 (dd, J = 24.6, 14.7 Hz, 3H), 3.22 - 3.09 (m, 4H), 2.69 (td, J = 13.1, 2.9 Hz, 1H), 2.63 (d, J = 2.9 Hz, 3H), 2.44 (dd, J = 14.4, 5.0 Hz, 1H), 2.29 (dd, J = 31.8, 15.1 Hz, 3H), 2.12 - 2.01 (m, 1H), 1.93 (dt, J = 12.4, 11.8 Hz, 4H), 1.83 (td, J = 8.9, 4.5 Hz, 2H), 1.77 (d, J = 11.8 Hz, 1H), 1.66 - 1.56 (m, 1H), 1.51 (d, J = 6.4 Hz, 3H), 1.37 - 1.24 (m, 3H), 1.19 - 1.11 (m, 1H).

### Example 155: Synthesis of compound (1S,2S)-2-(difluoromethyl)-N-((1⁵S,6³S,4S,Z)-1⁵,10,10-trimethyl-1²-((1S,4S)-5-methyl-2,5-diaza bicyclo[2.2.1]heptan-2-yl)-5,7-dioxo-1⁸,1⁹,6¹,6²6³,6⁴,6⁵,6⁶-octahydro-1⁵H,1⁷H-8-oxa-1(13,15)-p yrido[3',2':7,8][1,5]oxazonino[5,6-a]indola-2(4,2)-thiazola-6(1,3)-pyridazinacycloundecaphane-4 -yl)cyclopropane-1-carboxamide

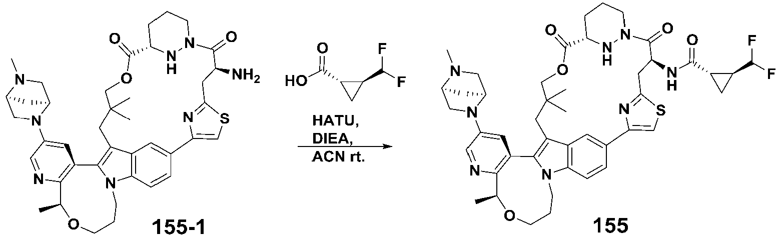

Compound (1S,2S)-2-(difluoromethyl)cyclopropane-1-carboxylic acid (130 mg) was added to a 25 mL single-necked flask, HATU (273 mg), N,N-diisopropylethylamine (0.8 mL) and anhydrous acetonitrile (10 mL) were weighed, and the reaction was carried out at room temperature for 10 min; a pre-liberated acetonitrile solution (10 mL) of compound 155-1 (354 mg) was added dropwise at room temperature, and the reaction was carried out at room temperature for 45 min. After the reaction was complete, it was directly concentrated, and the concentrate was purified by preparation (60% acetonitrile/water) to obtain the target compound 155 (49.8 mg, 11.9% yield). ESI-MS m/z: 857.8 [M+H]⁺.

¹H NMR (500 MHz, CDCl₃)δ 8.58 (s, 1H), 7.61 (d, J = 8.1 Hz, 1H), 7.47 (d, J = 8.0 Hz, 2H), 7.30 (s, 2H), 7.02 (d, J = 8.0 Hz, 2H), 6.84 (d, J = 9.5 Hz, 1H), 5.97 - 5.67 (m, 2H), 4.59 (d, J = 10.9 Hz, 2H), 4.27 (d, J = 55.8 Hz, 9H), 4.01 (d, J = 10.8 Hz, 3H), 3.73 (d, J = 59.8 Hz, 5H), 3.48 (d, J = 14.9 Hz, 1H), 3.36 - 3.11 (m, 3H), 2.97 (s, 4H), 2.69 (t, J = 11.5 Hz, 1H), 2.44 (s, 1H), 2.35 - 2.24 (m, 6H), 1.89 (ddd, J = 33.0, 12.6, 8.8 Hz, 5H), 1.61 (s, 4H), 1.40 - 1.22 (m, 3H), 1.15 (s, 1H), 0.99 (s, 3H).

### Example 156: Synthesis of compound (1S,2S)-2-(difluoromethyl)-N-((1⁵S,6³S,4S,Z)-1⁵,10,10-trimethyl-1²-(((S)-1-methylpiperidin-2-y l)methoxy)-5,7-dioxo-1⁸,1⁹,6¹,6²6³6⁴,6⁵,6⁶-octahydro-1⁵H,1⁷H-8-oxa-1(13,15)-pyrido[3',2':7,8][ 1,5]oxazonino[5,6-a]indola-2(4,2)-thiazola-6(1,3)-pyridazinacycloundecaphane-4-yl)cyclopropa ne-1-carboxamide

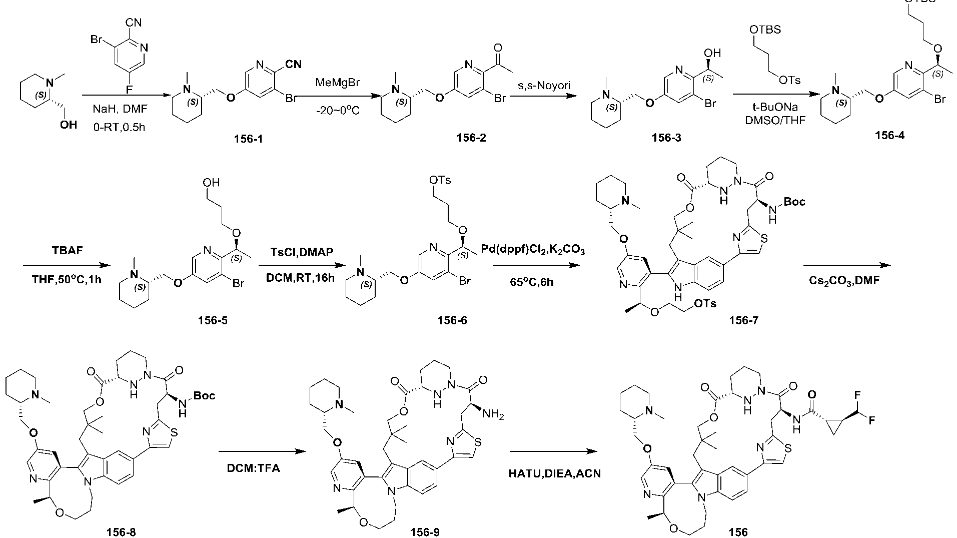

### Step 1: Synthesis of compound 156-1

(S)-(1-methylpiperidin-2-yl)methanol (1.29 g, 9.95 mmol) and N,N-dimethylformamide (20.00 mL) and sodium hydride (0.8 g, 19.9 mmol) were added sequentially to a 50 mL single-necked flask, the mixture was kept stirring for 15 min. At 0 °C, 3-bromo-5-fluoropicolinonitrile (2.00 g, 9.95 mmol) was added dropwise, and the mixture was stirred at room temperature for 1 h. The reaction was complete as monitored by LC-MS, the reaction solution was poured into water, a solid precipitated, filtered, and the filter cake was washed with water. The filter cake was dried to obtain the target compound 156-1 (2.00 g). ESI-MS m/z: 310 [M+H]⁺.

### Step 2: Synthesis of compound 156-2

Compound 156-1 (2.00 g, 6.68 mmol) and tetrahydrofuran (20.00 mL) were added sequentially to a 100 mL three-necked flask. Under nitrogen protection, the temperature was lowered to -20 °C, and then methylmagnesium bromide (6.24 mL, 3.00 mol/L, 18.72 mmol) was added dropwise. After the addition was complete, the mixture was transferred to 0 °C and stirred for 2 h. The reaction was complete as monitored by LC-MS, quenched with saturated NH₄Cl solution, adjusted to pH=4 with dilute hydrochloric acid, and stirred for 10 min. The mixture was then adjusted to pH=8 with saturated NaHCO₃ solution, extracted twice with ethyl acetate, and the organic layer was washed with saturated brine and dried over anhydrous sodium sulfate. The mixture was concentrated under reduced pressure and purified by column chromatography to obtain the target compound 156-2 (1.22 g). ESI-MS m/z: 327 [M+H]⁺.

### Step 3: Synthesis of compound 156-3

Compound 156-2 (1.22 g, 4.08 mmol), triethylamine (14.17 mL, 101.94 mmol), and (S,S)-N-(p-toluenesulfonyl)-1,2-diphenylethane-1,2-diamine (p-cymene)ruthenium(II) chloride (0.03 g, 0.04 mmol) were added sequentially to a 100 mL three-necked flask. Under nitrogen protection, the temperature was lowered to 0 °C, formic acid (0.39 mL, 10.19 mmol) was added dropwise. The mixture was stirred at room temperature for 24 h. The reaction was complete as monitored by LC-MS, extracted twice with dichloromethane, and the organic layer was washed with saturated brine and dried over anhydrous sodium sulfate. The mixture was concentrated under reduced pressure and purified by column chromatography to obtain the target compound 156-3 (1.06 g). ESI-MS m/z: 329 [M+H]⁺.

### Step 4: Synthesis of compound 156-4

Compound 156-3 (0.58 g, 1.76 mmol), dimethyl sulfoxide (10.00 mL), tetrahydrofuran (2.00 mL), and 3-((tert-butyldimethylsilyl)oxy)propyl-4-methylbenzenesulfonate (1.82 g, 5.29 mmol) were added sequentially to a 50 mL single-necked flask. Sodium tert-butoxide (0.42 g, 4.4 mmol) was added in batches at 0 °C, and the mixture was transferred to room temperature and stirred for 1 h. The reaction was complete as monitored by LC-MS, and the reaction mixture was added to a saturated ammonium chloride aqueous solution for quenching. The mixture was extracted twice with ethyl acetate, washed three times with saturated brine, concentrated under reduced pressure, and purified by column chromatography to obtain the target compound 156-4 (0.6 g). ESI-MS m/z: 501 [M+H]⁺.

### Step 5: Synthesis of compound 156-5

Compound 156-4 (0.66 mg, 1.32 mmol) and tetrahydrofuran (7.00 mL) were added sequentially to a 50 mL single-necked flask. Tetrabutylammonium fluoride (1.97 mL, 1.00 mol/L, 1.97 mmol) was added dropwise at 0 °C, and the reaction was heated to 50 °C and stirred for 1 h. The reaction was complete as monitored by LC-MS, concentrated under reduced pressure, and purified by column chromatography to obtain the target compound 156-5 (0.27 g). ESI-MS m/z: 387 [M+H]⁺.

### Step 6: Synthesis of compound 156-6

Compound 156-5 (267 mg, 0.69 mmol), dichloromethane (10.00 mL), and 4-dimethylaminopyridine (8.42 mg, 0.07 mmol) were added sequentially to a 50 mL single-necked flask. Then p-toluenesulfonyl chloride (262.82 mg, 1.38 mmol) and triethylamine (0.24 mL, 1.72 mmol) were added in batches, and the mixture was stirred at room temperature for 4 h. The reaction was complete as monitored by LC-MS, concentrated under reduced pressure, and purified by column chromatography to obtain the target compound 156-6 (216 mg). ESI-MS m/z: 541 [M+H]⁺.

### Step 7: Synthesis of compound 156-7

Compound 156-6 (160 mg, 0.30 mmol), M3 (205 mg, 0.30 mmol), toluene (3.00 mL), 1,4-dioxane (1.00 mL), water (1.00 mL), potassium carbonate (102.11 mg, 0.74 mmol), and [1,1'-bis(diphenylphosphino)ferrocene]dichloropalladium(II) dichloromethane complex (24.17 mg, 0.03 mmol) were added sequentially to a 10 mL single-necked flask. The atmosphere was replaced by nitrogen for three times, and under the protection of nitrogen, the reaction was heated to 65 °C and stirred for 3 h. The reaction was complete as monitored by LC-MS, extracted twice with ethyl acetate, washed with saturated brine, concentrated under reduced pressure, and purified by column chromatography to obtain the target compound 156-7 (160.00 mg). ESI-MS m/z: 508 1/2[M+2H]⁺.

### Step 8: Synthesis of compound 156-8

Compound 156-7 (160.00 mg, 0.16 mmol), N,N-dimethylformamide (5.00 mL), and cesium carbonate (102 mg, 0.32 mmol) were added sequentially to a 10 mL single-necked flask, and the reaction was heated to 65 °C and stirred for 1 h. The reaction was complete as monitored by LC-MS, the reaction solution was diluted with ethyl acetate, washed sequentially with water and brine three times, concentrated under reduced pressure, and purified by column chromatography to obtain the target compound 156-8 (120.00 mg). ESI-MS m/z: 856 [M+H]⁺.

### Step 9: Synthesis of compound 156-9

Compound 156-8 (120.00 mg, 0.14 mmol), dichloromethane (2.00 mL), and hydrochloric acid (4M solution in dioxane) (2.00 mL) were added sequentially to a 10 mL single-necked flask, and the mixture was stirred at room temperature for 1 h. The reaction was complete as monitored by LC-MS, and the mixture was concentrated under reduced pressure. The crude product was directly used in the next step to obtain the crude product compound 156-9 (121.00 mg). ESI-MS m/z: 756 [M+H]⁺.

### Step 10: Synthesis of compound 156

(1S,2S)-2-(difluoromethyl)cyclopropane-1-carboxylic acid (20.00 mg, 0.17 mmol) and acetonitrile (2.00 mL) were added sequentially to a 10 mL single-necked flask. At 0 °C, N,N,N',N'-tetramethyl-O-(7-azabenzotriazol-1-yl)uronium hexafluorophosphate (110.19 mg, 0.29 mmol) and N,N-diisopropylethylamine (0.12 mL, 0.70 mmol) were added, and the mixture was stirred at 0 °C for 10 min. Finally, compound 156-9 (126.00 mg, 0.14 mmol) was added, and the mixture was stirred at 0 °C for 10 min. The reaction was complete as monitored by LC-MS, and the reaction solution was directly purified by pre-HPLC to obtain the target compound 156 (40.5 mg). ESI-MS m/z: 874 [M+H]⁺.

¹H NMR (500 MHz, MeOD)δ 8.57 (s, 1H), 8.40 (d, J = 2.8 Hz, 1H), 7.68 (dd, J = 8.6, 1.3 Hz, 1H), 7.55 (s, 1H), 7.45 (d, J = 8.6 Hz, 1H), 7.36 (d, J = 2.8 Hz, 1H), 5.93 (d, J = 4.0 Hz, 0.24H), 5.81 (d, J = 4.1 Hz, 0.5H), 5.69 (t, J = 7.2 Hz, 1.26H), 4.43 (d, J = 11.3 Hz, 1H), 4.36 - 4.27 (m, 2H), 4.22 - 4.12 (m, 3H), 4.06 (dd, J = 14.6, 4.5 Hz, 1H), 3.79-3.66 (m, 3H), 3.45 (d, J = 14.8 Hz, 1H), 2.92 (d, J = 11.7 Hz, 1H), 2.81-2.74 (m, 1H), 2.49 (d, J = 14.4 Hz, 1H), 2.37 (s, 5H), 2.25-2.17 (m, 2H), 2.10 - 2.05 (m, 1H), 1.98 - 1.74 (m, 7H), 1.71 - 1.54 (m, 5H), 1.48 (d, J = 6.5 Hz, 3H), 1.44 - 1.24 (m, 3H), 1.22 - 1.08 (m, 2H), 1.00 (s, 3H), 0.40 (s, 3H).

### Example 157: Synthesis of compound (1S,2S)-2-(difluoromethyl)-N-((1⁵S,6³S,4S,Z)-1⁵,10,10-trimethyl-1²-(2-morpholinoethoxy)-5,7-d ioxo-1⁸,1⁹,6¹,6²,6³,6⁴,6⁵,6⁶-octahydro-1⁵H,1⁷H-8-oxa-1(13,15)-pyrido[3',2':7,8][1,5]oxazonino[5, 6-a]indola-2(4,2)-thiazola-6(1,3)-pyridazinacycloundecaphane-4-yl)cyclopropane-1-carboxamid e

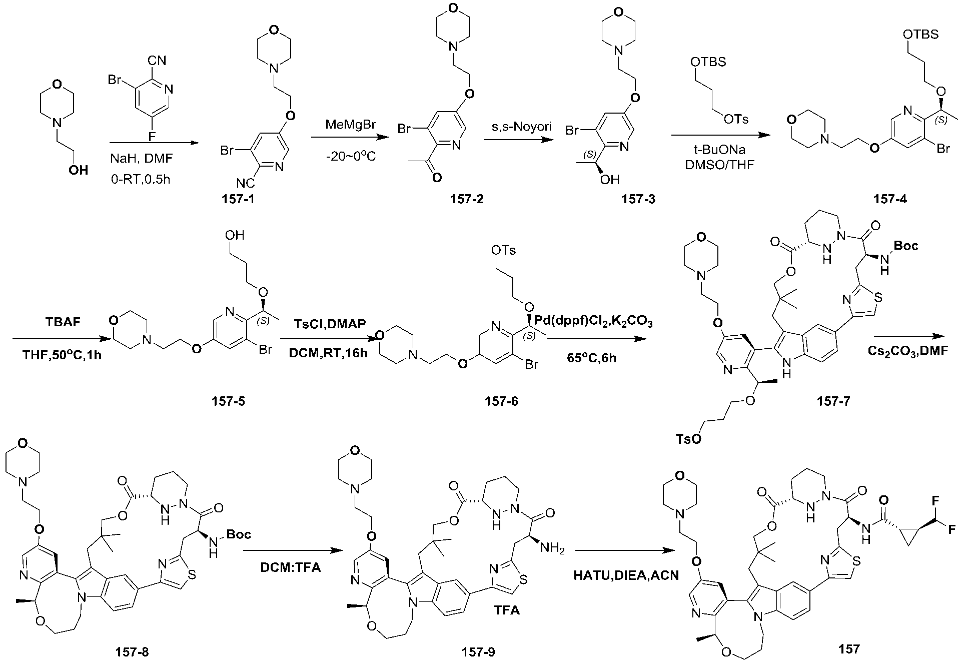

### Step 1: Synthesis of compound 157-1

2-Morpholinoethan-1-ol (2.35 g, 17.91 mmol), N,N-dimethylformamide (20.00 mL) and sodium hydride (1.19 g, 29.85 mmol) were added sequentially to a 50 mL single-necked flask, and the mixture was stirred insulated for 15 min. At 0 °C, 3-bromo-5-fluoropicolinonitrile (3.00 g, 14.93 mmol) was added dropwise, and the mixture was stirred at room temperature for 1 h. The reaction was complete as monitored by LC-MS, the reaction solution was poured into water, a solid precipitated, filtered, and the filter cake was washed with water. The filter cake was dried to obtain the target compound 157-1 (3.06 g). ESI-MS m/z: 312 [M+H]⁺.

### Step 2: Synthesis of compound 157-2

Compound 157-1 (3.06 g, 10.16 mmol) and tetrahydrofuran (31.00 mL) were added sequentially to a 100 mL three-necked flask. Under nitrogen protection, the temperature was lowered to -20 °C, and then methylmagnesium bromide (9.48 mL, 3.00 mol/L, 28.45 mmol) was added dropwise. After the addition was complete, the mixture was transferred to 0 °C and stirred for 2 h. The reaction was complete as monitored by LC-MS, quenched with saturated NH₄Cl solution, adjusted to pH=4 with dilute hydrochloric acid, and stirred for 10 min. The mixture was then adjusted to pH=8 with saturated NaHCO₃ solution, extracted twice with ethyl acetate, and the organic layer was washed with saturated brine and dried over anhydrous sodium sulfate. The mixture was concentrated under reduced pressure and purified by column chromatography to obtain the target compound 157-2 (3.06 g). ESI-MS m/z: 329 [M+H]⁺.

### Step 3: Synthesis of compound 157-3

Compound 157-2 (2.16 g, 6.56 mmol), triethylamine (22.8 mL, 164.04 mmol), and (S,S)-N-(p-toluenesulfonyl)-1,2-diphenylethane-1,2-diamine (p-cymene)ruthenium(II) chloride (0.04 g, 0.07 mmol) were added sequentially to a 100 mL three-necked flask. Under nitrogen protection, the temperature was lowered to 0 °C, formic acid (0.63 mL, 16.4 mmol) was added dropwise. The mixture was stirred at room temperature for 24 h. The reaction was complete as monitored by LC-MS, extracted twice with dichloromethane, and the organic layer was washed with saturated brine and dried over anhydrous sodium sulfate. The mixture was concentrated under reduced pressure and purified by column chromatography to obtain the target compound 157-3 (1.76 g). ESI-MS m/z: 331 [M+H]⁺.

### Step 4: Synthesis of compound 157-4

Compound 157-3 (0.8 g, 2.42 mmol), dimethyl sulfoxide (10.00 mL), tetrahydrofuran (2.00 mL), and 3-((tert-butyldimethylsilyl)oxy)propyl-4-methylbenzenesulfonate (2.50 g, 7.25 mmol) were added sequentially to a 50 mL single-necked flask. Sodium tert-butoxide (0.58 g, 6.04 mmol) was added in batches at 0 °C, and the mixture was transferred to room temperature and stirred for 1 h. The reaction was complete as monitored by LC-MS, and the reaction mixture was added to a saturated ammonium chloride aqueous solution for quenching. The mixture was extracted twice with ethyl acetate, washed three times with saturated brine, concentrated under reduced pressure, and purified by column chromatography to obtain the target compound 157-4 (1.03 g). ESI-MS m/z: 503 [M+H]⁺.

### Step 5: Synthesis of compound 157-5

Compound 157-4 (1.03 g, 1.32 mmol) and tetrahydrofuran (10.00 mL) were added sequentially to a 50 mL single-necked flask. Tetrabutylammonium fluoride (3.07 mL, 1.00 mol/L, 3.07 mmol) was added dropwise at 0 °C, and the reaction was heated to 50 °C and stirred for 1 h. The reaction was complete as monitored by LC-MS, concentrated under reduced pressure, and purified by column chromatography to obtain the target compound 157-5 (0.56 g). ESI-MS m/z: 389 [M+H]⁺.

### Step 6: Synthesis of compound 157-6

Compound 157-5 (560 mg, 1.44 mmol), dichloromethane (6.00 mL), and 4-dimethylaminopyridine (17.58 mg, 0.14 mmol) were added sequentially to a 50 mL single-necked flask. And then, p-toluenesulfonyl chloride (548.49 mg, 2.88 mmol) and triethylamine (0.50 mL, 3.60 mmol) were added in batches, and the mixture was stirred at room temperature for 4 h. The reaction was complete as monitored by LC-MS, concentrated under reduced pressure, and purified by column chromatography to obtain the target compound 157-6 (690 mg). ESI-MS m/z: 543 [M+H]⁺.

### Step 7: Synthesis of compound 157-7

Compound 157-6 (400 mg, 0.74 mmol), M3 (510 mg, 0.74 mmol), toluene (6.00 mL), 1,4-dioxane (2.00 mL), water (2.00 mL), potassium carbonate (254.31 mg, 1.84 mmol), and [1,1'-bis(diphenylphosphino)ferrocene]dichloropalladium(II) dichloromethane complex (60.1 mg, 0.07 mmol) were added sequentially to a 50 mL single-necked flask. The atmosphere was replaced by nitrogen for three times, and under nitrogen protection, the reaction was heated to 65 °C and stirred for 3 h. The reaction was complete as monitored by LC-MS, extracted twice with ethyl acetate, washed with saturated brine, concentrated under reduced pressure, and purified by column chromatography to obtain the target compound 157-7 (428.00 mg). ESI-MS m/z: 516 1/2[M+2H]⁺.

### Step 8: Synthesis of compound 157-8

Compound 157-7 (420.00 mg, 0.41 mmol), N,N-dimethylformamide (5.00 mL), and cesium carbonate (265 mg, 0.82 mmol) were added sequentially to a 10 mL single-necked flask, and the reaction was heated to 65 °C and stirred for 1 h. The reaction was complete as monitored by LC-MS, the reaction solution was diluted with ethyl acetate, washed sequentially with water and brine three times, concentrated under reduced pressure, and purified by column chromatography to obtain the target compound 157-8 (325.00 mg). ESI-MS m/z: 858 [M+H]⁺.

### Step 9: Synthesis of compound 157-9

Compound 157-8 (160.00 mg, 0.19 mmol), dichloromethane (2.00 mL), and hydrochloric acid (4M solution in dioxane) (2.00 mL) were added sequentially to a 10 mL single-necked flask, and the mixture was stirred at room temperature for 1 h. The reaction was complete as monitored by LC-MS, and the mixture was concentrated under reduced pressure. The crude product was directly used in the next step to obtain the crude product compound 157-9 (162.00 mg). ESI-MS m/z: 758 [M+H]⁺.

### Step 10: Synthesis of compound 157

(1S,2S)-2-(difluoromethyl)cyclopropane-1-carboxylic acid (30.36 mg, 0.22 mmol) and acetonitrile (3.00 mL) were added sequentially to a 10 mL single-necked flask. At 0 °C, N,N,N',N'-tetramethyl-O-(7-azabenzotriazol-1-yl)uronium hexafluorophosphate (141.45 mg, 0.37 mmol) and N,N-diisopropylethylamine (0.15 mL, 0.93 mmol) were added, and the mixture was stirred at 0 °C for 10 min. Finally, compound 157-9 (162.00 mg, 0.19 mmol) was added, and the mixture was stirred at 0 °C for 10 min. The reaction was complete as monitored by LC-MS, and the reaction solution was directly purified by pre-HPLC to obtain the target compound 157 (55.70 mg). ESI-MS m/z: 876 [M+H]⁺.

¹H NMR (500 MHz, MeOD)δ 8.59 (d, J = 1.3 Hz, 1H), 8.42 (d, J = 2.9 Hz, 1H), 7.71 (d, J = 8.5 Hz, 1H), 7.57 (s, 1H), 7.47 (d, J = 8.6 Hz, 1H), 7.37 (d, J = 2.0 Hz, 1H), 5.95 (d, J = 4.0 Hz, 0.26H), 5.84 (d, J = 4.1 Hz, 0.5H), 5.71 (t, J = 7.7 Hz, 1.24H), 4.45 (d, J = 12.4 Hz, 1H), 4.36 - 4.25 (m, 4H), 4.17 (dd, J = 11.9, 4.8 Hz, 1H), 4.08 (d, J = 10.8 Hz, 1H), 3.82 - 3.67 (m, 7H), 3.51-3.47 (m, 1H), 3.37 (s, 5H), 2.89 - 2.75 (m, 3H), 2.62 (s, 4H), 2.52 (d, J = 14.5 Hz, 1H), 2.40 (t, J = 12.3 Hz, 1H), 2.25 (t, J = 13.1 Hz, 1H), 2.14 - 2.07 (m, 1H), 2.01 - 1.75 (m, 4H), 1.67 - 1.58 (m, 1H), 1.50 (d, J = 6.4 Hz, 3H), 1.24 - 1.10 (m, 2H), 1.03 (s, 3H), 0.42 (s, 3H).

### Example 158: Synthesis of compound (1r,2R,3S)-2,3-dimethyl-N-((1⁵S,6³S,4S,Z)-1⁵,10,10-trimethyl-1²-(2-morpholinoethoxy)-5,7-dio xo-1⁸,1⁹,6¹,6²6³,6⁴,6⁵,6⁶-octahydro-1⁵H,1⁷H-8-oxa-1(13,15)-pyrido[3',2':7,8][1,5]oxazonino[5,6-a]indola-2(4,2)-thiazola-6(1,3)-pyridazinacycloundecaphane-4-yl)cyclopropane-1-carboxamide

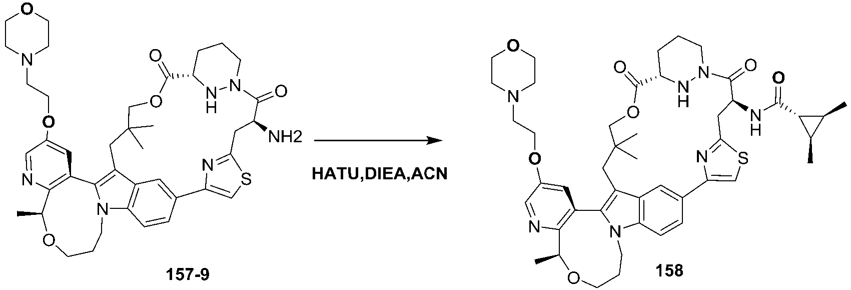

(1R,2R,3S)-2,3-dimethylcyclopropane-1-carboxylic acid (25.15 mg, 0.22 mmol) and acetonitrile (3.00 mL) were added sequentially to a 10 mL single-necked flask. At 0 °C, N,N,N',N'-tetramethyl-O-(7-azabenzotriazol-1-yl)uronium hexafluorophosphate (141.45 mg, 0.37 mmol) and N,N-diisopropylethylamine (0.15 mL, 0.93 mmol) were added, and the mixture was stirred at 0 °C for 10 min. Finally, compound 157-9 (160.00 mg, 0.19 mmol) was added, and the mixture was stirred at 0 °C for 10 min. The reaction was complete as monitored by LC-MS, and the reaction solution was directly purified by pre-HPLC to obtain the target compound 158 (58.10 mg). ESI-MS m/z: 854 [M+H]⁺.

¹H NMR (500 MHz, MeOD)δ 8.56 (s, 1H), 8.39 (d, J = 2.9 Hz, 1H), 7.67 (dd, J = 8.6, 1.5 Hz, 1H), 7.53 (s, 1H), 7.43 (t, J = 6.4 Hz, 1H), 7.34 (d, J = 2.9 Hz, 1H), 5.65 (d, J = 8.9 Hz, 1H), 4.78 (d, J = 10.7 Hz, 1H), 4.42 (d, J = 11.2 Hz, 1H), 4.32-4.28 (m, 4H), 4.17 - 4.00 (m, 2H), 3.79 - 3.67 (m, 7H), 3.45-3.40 (m, 1H), 3.27-3.16 (m, 3H), 2.85 - 2.71 (m,3H), 2.58 (s, 4H), 2.52 - 2.41 (m, 1H), 2.36 (t, J = 13.2 Hz, 1H), 2.24-2.17 (m, 1H), 1.97 - 1.75 (m, 3H), 1.64 - 1.55 (m, 1H), 1.47 (d, J = 8.9 Hz, 3H), 1.41 - 1.26 (m, 3H), 1.18 - 1.09 (m, 7H), 1.00 (s, 3H), 0.39 (s, 3H).

### Example 159: Synthesis of compound (1r,2R,3S)-N-((1⁵S,6³S,4S,Z)-1²-(4-ethylpiperazin-1-yl)-1⁵,10,10-trimethyl-5,7-dioxo-1⁸,1⁹,6¹,6² ,6³,6⁴,6⁵,6⁶-octahydro-1⁵H,1⁷H-8-oxa-1(13,15)-pyrido[3',2':7,8][1,5]oxazonino[5,6-a]indola-2(4, 2)-thiazola-6(1,3)-pyridazinacycloundecaphane-4-yl)-2,3-dimethylcyclopropane-1-carboxamide

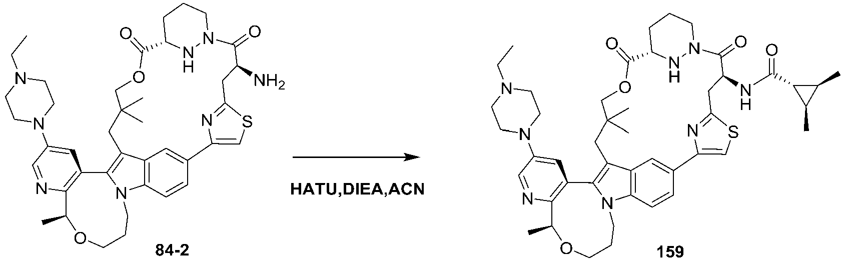

(1R,2R,3S)-2,3-dimethylcyclopropane-1-carboxylic acid (12.35 mg, 0.11 mmol) and acetonitrile (3.00 mL) were added sequentially to a 10 mL single-necked flask. At 0 °C, N,N,N',N'-tetramethyl-O-(7-azabenzotriazol-1-yl)uronium hexafluorophosphate (68.59 mg, 0.18 mmol) and N,N-diisopropylethylamine (0.07 mL, 0.45 mmol) were added, and the mixture was stirred at 0 °C for 10 min. Finally, compound 84-2 (70.00 mg, 0.09 mmol) was added, and the mixture was stirred at 0 °C for 10 min. The reaction was complete as monitored by LC-MS, and the reaction solution was directly purified by pre-HPLC to obtain the target compound 159 (32.40 mg). ESI-MS m/z: 837 [M+H]⁺.

¹H NMR (500 MHz, MeOD) δ 8.55 (d, J = 1.1 Hz, 1H), 8.36 (d, J = 2.9 Hz, 1H), 7.67 (dd, J = 8.6, 1.4 Hz, 1H), 7.54 (s, 1H), 7.44 (d, J = 8.6 Hz, 1H), 7.28 (d, J = 2.9 Hz, 1H), 5.66 (d, J = 8.5 Hz, 1H), 4.42-4.44 (m, 1H), 4.30-4.28 (m, 2H), 4.14 (dd, J = 12.3, 5.4 Hz, 1H), 4.06 (dd, J = 14.5, 4.3 Hz, 1H), 3.81-3.67 (m, 3H), 3.43 (d, J = 15 Hz, 1H), 3.38-3.34 (m, 4H), 3.26-3.23 (m, 2H), 3.19 (d, J = 14.5 Hz, 1H), 2.79-2.73 (m, 1H), 2.70-2.63 (m, 4H), 2.58-2.46 (m, 3H), 2.40-2.35 (m, 1H), 2.27-2.15 (m, 1H), 1.97-1.88 (m, 2H), 1.84-1.78 (m, 1H), 1.66-1.55 (m, 1H), 1.47 (d, J = 6.5 Hz, 3H), 1.42 - 1.36 (m, 3H), 1.18-1.12 (m, 9H), 1.00 (s, 3H), 0.41 (s, 3H).

### Example 160: Synthesis of compound (1S,2S)-2-(difluoromethyl)-N-((1⁵S,6³S,4S,Z)-1⁵,10,10-trimethyl-1²-(methyl(1-methylpyrrolidin -3-yl)amino)-5,7-dioxo-1⁸,1⁹,6¹,6²,6³,6⁴,6⁵,6⁶-octahydro-1⁵H,1⁷H-8-oxa-1(13,15)-pyrido[3',2':7,8 ][1,5]oxazonino[5,6-a]indola-2(4,2)-thiazola-6(1,3)-pyridazinacycloundecaphane-4-yl)cycloprop ane-1-carboxamide

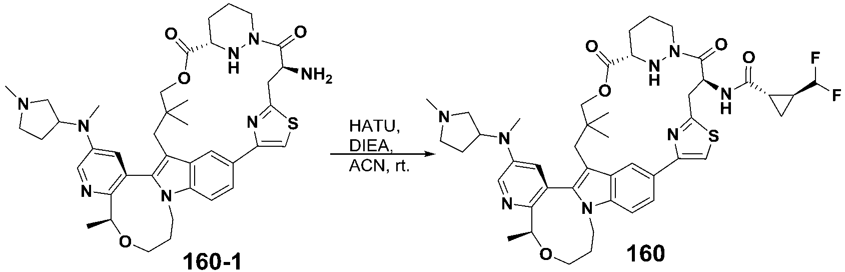

Compound (1S,2S)-2-(difluoromethyl)cyclopropane-1-carboxylic acid (42 mg) was added to a 25 mL single-necked flask, HATU (88 mg), N,N-diisopropylethylamine (0.26 mL) and anhydrous acetonitrile (5 mL) were weighed and added in, and the reaction was carried out at room temperature for 10 min; a pre-liberated acetonitrile solution (5 mL) of 160-1 (115 mg) was added dropwise at room temperature, and the reaction was carried out at room temperature for 20 min. After the reaction was complete, it was directly concentrated, and the concentrate was purified by preparation (60% acetonitrile/water) to obtain the target compound 160 (59.0 mg, 44.1% yield). ESI-MS m/z: 859.6 [M+H]⁺.

¹H NMR (500 MHz, CDCl₃)δ 8.57 (d, J = 13.8 Hz, 1H), 8.35 (s, 1H), 7.77 (d, J = 8.2 Hz, 1H), 7.58 (d, J = 7.4 Hz, 1H), 7.36 - 7.31 (m, 1H), 7.29 (d, J = 10.8 Hz, 1H), 7.01 (s, 1H), 6.76 (d, J = 9.4 Hz, 1H), 5.99 - 5.65 (m, 2H), 4.86 - 4.71 (m, 2H), 4.59 (d, J = 12.3 Hz, 1H), 4.36 (td, J = 12.1, 3.1 Hz, 1H), 4.22 (dt, J = 12.8, 6.0 Hz, 2H), 4.01 (dd, J = 18.8, 8.4 Hz, 3H), 3.86 - 3.71 (m, 4H), 3.52 - 3.37 (m, 2H), 3.32 (t, J = 10.8 Hz, 2H), 3.25 - 3.09 (m, 3H), 2.95 (s, 4H), 2.89 (d, J = 9.5 Hz, 6H), 2.69 (t, J = 11.5 Hz, 1H), 2.48 - 2.21 (m, 7H), 2.05 - 1.94 (m, 3H), 1.89 - 1.81 (m, 4H), 1.61 (d, J = 12.5 Hz, 2H), 1.51 (d, J = 6.4 Hz, 3H), 1.39 (d, J = 6.4 Hz, 1H), 1.30 (dd, J = 18.9, 14.5 Hz, 3H), 1.15 (dt, J = 10.8, 5.5 Hz, 1H), 0.98 (s, 3H).

### Example 161: Synthesis of compound (1S,2S)-2-(difluoromethyl)-N-((1⁵S,1⁸R,6³S,4S,Z)-1²-(4-ethylpiperazin-1-yl)-1⁵,1⁸,10,10-tetrame thyl-5,7-dioxo- 1⁸,1⁹,6¹,6²,6³,6⁴,6⁵,6⁶-octahydro-1⁵H,1⁷H-8-oxa-1(13,15)-pyrido[3',2':7,8][1,5]ox azonino[5,6-a]indola-2(4,2)-thiazola-6(1,3)-pyridazinacycloundecaphane-4-yl)cyclopropane-1-c arboxamide

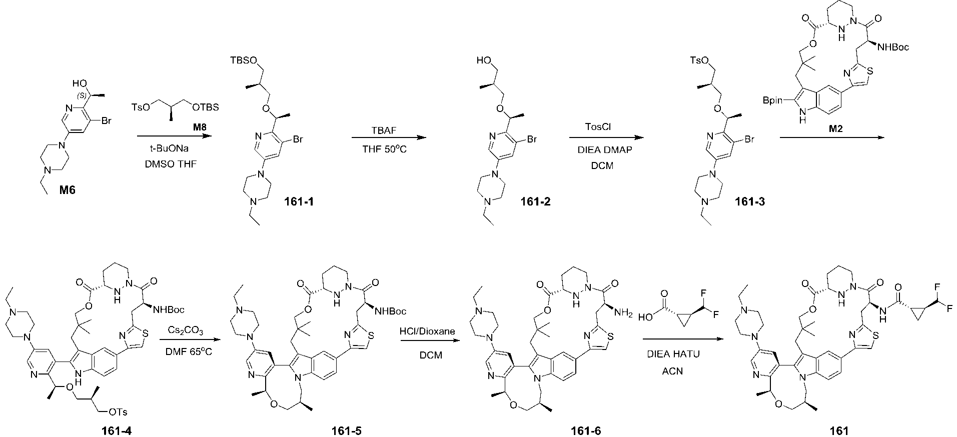

### Step 1: Synthesis of compound 161-1

Intermediate M6 (0.3 g, 0.95 mmol), dimethyl sulfoxide (5.00 mL), tetrahydrofuran (1.00 mL), and M8 (1.03 g, 2.86 mmol) were added sequentially to a 50 mL single-necked flask. Sodium tert-butoxide (0.23 g, 2.39 mmol) was added in batches at 0 °C, and the mixture was transferred to room temperature and stirred for 1 h. The reaction was complete as monitored by LC-MS, and the reaction mixture was added to a saturated ammonium chloride aqueous solution for quenching. The mixture was extracted twice with ethyl acetate, washed three times with saturated brine, concentrated under reduced pressure, and purified by column chromatography to obtain the target compound 161-1 (0.16 g). ESI-MS m/z: 500 [M+H]⁺.

### Step 2: Synthesis of compound 161-2

Compound 161-1 (154 mg, 0.31 mmol) and tetrahydrofuran (3.00 mL) were added sequentially to a 50 mL single-necked flask. Tetrabutylammonium fluoride (0.46 mL, 1.00 mol/L, 0.46 mmol) was added dropwise at 0 °C, and the reaction was heated to 50 °C and stirred for 1 h. The reaction was complete as monitored by LC-MS, concentrated under reduced pressure, and purified by column chromatography to obtain the target compound 161-2 (119 mg). ESI-MS m/z: 386 [M+H]⁺.

### Step 3: Synthesis of compound 161-3

Compound 161-2 (119 mg, 0.31 mmol), dichloromethane (3.00 mL), and 4-dimethylaminopyridine (3.76 mg, 0.03 mmol) were added sequentially to a 50 mL single-necked flask. Then p-toluenesulfonyl chloride (117.44 mg, 0.62 mmol) and triethylamine (0.11 mL, 0.77 mmol) were added in batches, and the mixture was stirred at room temperature for 4 h. The reaction was complete as monitored by LC-MS, concentrated under reduced pressure, and purified by column chromatography to obtain the target compound 161-3 (150 mg). ESI-MS m/z: 540 [M+H]⁺.

### Step 4: Synthesis of compound 161-4

Compound 161-3 (150 mg, 0.28 mmol), M2 (192.49 mg, 0.28 mmol), toluene (3.00 mL), 1,4-dioxane (1.00 mL), water (1.00 mL), potassium carbonate (95.89 mg, 0.69 mmol), and [1,1'-bis(diphenylphosphino)ferrocene]dichloropalladium(II) dichloromethane complex (22.7 mg, 0.03 mmol) were added sequentially to a 10 mL single-necked flask. The atmosphere was replaced by N₂ for 3 times, and under nitrogen protection, the reaction was heated to 65 °C and stirred for 3 h. The reaction was complete as monitored by LC-MS, extracted twice with ethyl acetate, washed with saturated brine, concentrated under reduced pressure, and purified by column chromatography to obtain the target compound 161-4 (256.00 mg). ESI-MS m/z: 514 1/2[M+2H]⁺.

### Step 5: Synthesis of compound 161-5

Compound 161-4 (260.00 mg, 0.25 mmol), N,N-dimethylformamide (5.00 mL), and cesium carbonate (165 mg, 0.51 mmol) were added sequentially to a 10 mL single-necked flask, and the reaction was heated to 65 °C and stirred for 1 h. The reaction was complete as monitored by LC-MS, the reaction solution was diluted with ethyl acetate, washed sequentially with water and brine three times, concentrated under reduced pressure, and purified by column chromatography to obtain the target compound 161-5 (200.00 mg). ESI-MS m/z: 855 [M+H]⁺.

### Step 6: Synthesis of compound 161-6

Compound 161-5 (200.00 mg, 0.23 mmol), dichloromethane (2.00 mL), and hydrochloric acid (4M solution in dioxane) (2.00 mL) were added sequentially to a 10 mL single-necked flask, and the mixture was stirred at room temperature for 1 h. The reaction was complete as monitored by LC-MS, and the mixture was concentrated under reduced pressure. The crude product was directly used in the next step to obtain the crude product compound 161-6 (172.00 mg). ESI-MS m/z: 755 [M+H]⁺.

### Step 7: Synthesis of compound 161

(1S,2S)-2-(difluoromethyl)cyclopropane-1-carboxylic acid (37.21 mg, 0.27 mmol) and acetonitrile (3.00 mL) were added sequentially to a 10 mL single-necked flask. At 0 °C, N,N,N',N'-tetramethyl-O-(7-azabenzotriazol-1-yl)uranium hexafluorophosphate (173.45 mg, 0.46 mmol) and N,N-diisopropylethylamine (0.19 mL, 1.14 mmol) were added, and the mixture was stirred at 0 °C for 10 min. Finally, compound 161-6 (172.00 mg, 0.23 mmol) was added, and the mixture was stirred at 0 °C for 10 min. The reaction was complete as monitored by LC-MS, and the reaction solution was directly purified by pre-HPLC to obtain the target compound 161 (68.00 mg). ESI-MS m/z: 873 [M+H]⁺.

¹H NMR (500 MHz, MeOD)δ 8.55 (s, 1H), 8.35 (d, J = 2.7 Hz, 1H), 7.66 (dd, J = 8.6, 1.1 Hz, 1H), 7.53 (s, 1H), 7.42 (d, J = 8.6 Hz, 1H), 7.26 (d, J = 2.7 Hz, 1H), 5.92 (d, J = 4.0 Hz, 0.2H), 5.81 (d, J = 4.0 Hz, 0.5H), 5.69 (t, J = 7.8 Hz, 1.3H), 4.83 (d, J = 12.3 Hz, 1H), 4.42 (d, J = 11.2 Hz, 1H), 4.33 - 4.20 (m, 2H), 3.93 (d, J = 12.1 Hz, 1H), 3.86 (dd, J = 14.3, 5.0 Hz, 1H), 3.75 (q, J = 10.0 Hz, 2H), 3.44 (d, J = 14.8 Hz, 1H), 3.38-3.35 (m, 6H), 3.26 (q, J = 10.0 Hz, 1H), 3.19 (d, J = 14.5 Hz, 1H), 2.82 - 2.72 (m, 1H), 2.66 (s, 4H), 2.58 - 2.45 (m, 4H), 2.23 (d, J = 10.2 Hz, 1H), 2.11 - 2.05 (m, 1H), 1.99 - 1.90 (m, 1H), 1.87 - 1.73 (m, 2H), 1.60 (dt, J = 12.6, 9.1 Hz, 1H), 1.45 (d, J = 15.0 Hz, 3H), 1.22 - 1.08 (m, 6H), 0.99 (s, 3H), 0.96 (d, J = 6.8 Hz, 3H), 0.40 (s, 3H).

### Example 162: Synthesis of compound (3R,4r,5S)-N-((1⁵S,6³S,4S,Z)-1²-(4-ethylpiperazin-1-yl)-1⁵,10,10-trimethyl-5,7-dioxo-1⁸,1⁹,6¹,6² ,6³,6⁴,6⁵,6⁶-octahydro-1⁵H,1⁷H-8-oxa-1(13,15)-pyrido[3',2':7,8][1,5]oxazonino[5,6-a]indola-2(4, 2)-thiazola-6(1,3)-pyridazinacycloundecaphane-4-yl)tetrahydro-2H-pyran-4-carboxamide

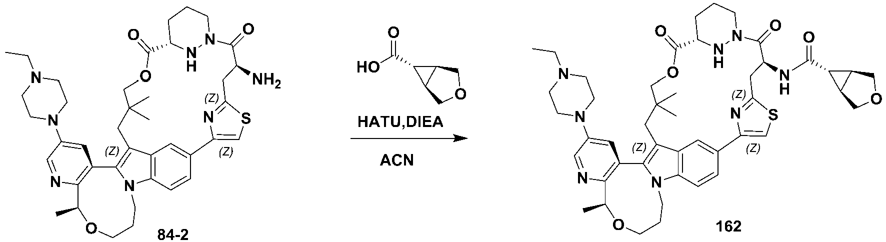

Compound (1R,5S,6R)-3-oxabicyclo[3.1.0]hexane-6-carboxylic acid (9.02 mg, 0.07 mmol) was dissolved in acetonitrile (2.00 mL). DIEA (0.10 mL, 0.59 mmol) and HATU (33.50 mg, 0.09 mmol) were added sequentially in an ice-water bath. After reacting in the ice-water bath for 10 min, a solution of compound 84-2 (50.00 mg, 0.06 mmol) in acetonitrile (2.00 mL) and DIEA (0.10 mL, 0.59 mmol) was added, and the reaction was carried out at room temperature for 1 h. The mixture was concentrated to dryness and purified by preparative separation to obtain compound 162 (21.00 mg, 41.13%). ESI-MS m/z: 851.58, [M+H]⁺.

¹H NMR (500 MHz, DMSO)δ 8.61 (d, J = 8.9 Hz, 1H), 8.49 (s, 1H), 8.41 (d, J = 2.8 Hz, 1H), 7.81 (s, 1H), 7.71 (dd, J = 8.6, 1.4 Hz, 1H), 7.49 (d, J = 8.6 Hz, 1H), 7.21 (d, J = 2.5 Hz, 1H), 5.51 (t, J = 9.3 Hz, 1H), 5.09 (d, J = 12.2 Hz, 1H), 4.28 - 4.19 (m, 2H), 4.15 (q, J = 6.3 Hz, 1H), 4.03 (dd, J = 11.9, 4.7 Hz, 2H), 3.84 (dd, J = 11.1, 8.6 Hz, 2H), 3.70 - 3.55 (m, 5H), 3.37 (s, 1H), 3.26 (s, 4H), 3.19 - 3.09 (m, 2H), 3.00 (d, J = 14.5 Hz, 1H), 2.80 - 2.70 (m, 1H), 2.47 - 2.24 (m, 5H), 2.11 (d, J = 10.1 Hz, 1H), 1.98 (ddd, J = 11.1, 10.1, 5.2 Hz, 2H), 1.92 - 1.88 (m, 1H), 1.80 (s, 3H), 1.66 (t, J = 3.1 Hz, 1H), 1.57 - 1.45 (m, 1H), 1.37 (d, J = 6.4 Hz, 3H), 1.23 (s, 2H), 1.03 (t, J = 7.1 Hz, 3H), 0.95 (s, 3H), 0.32 (s, 3H).

### Example 163: Synthesis of compound (1S,2S)-2-(difluoromethyl)-N-((1⁵S,6³S,4S,Z)-1⁵,10,10-trimethyl-1²-((3aS,6aS)-1-methylhexahy dropyrrolo[3,4-b]pyrrol-5(1H)-yl)-5,7-dioxo-1⁸,1⁹,6¹,6²,6³,6⁴,6⁵,6⁶-octahydro-1⁵H,1⁷H-8-oxa-1(1 3,15)-pyrido[3',2':7,8][1,5]oxazonino[5,6-a]indola-2(4,2)-thiazola-6(1,3)-pyridazinacycloundeca phane-4-yl)cyclopropane-1-carboxamide

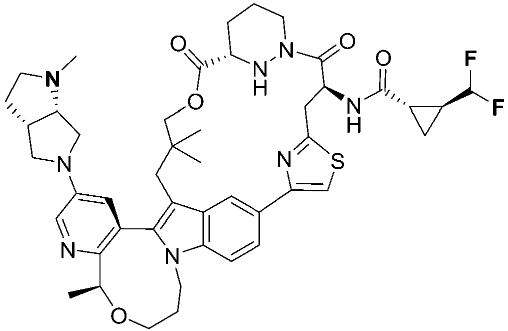

ESI-MS m/z:871.5 [M+H]⁺.

¹H NMR (500 MHz, CDCl₃)δ 8.58 (s, 1H), 8.16 (t, J = 3.0 Hz, 1H), 7.56 (dd, J = 8.6, 1.5 Hz, 1H), 7.30 (d, J = 8.5 Hz, 1H), 7.28 (s, 1H), 6.78 (dd, J = 12.3, 2.7 Hz, 1H), 6.68 (d, J = 9.5 Hz, 1H), 5.89 (t, J = 8.9 Hz, 1H), 5.84 (td, J = 57.6, 3.0 Hz, 1H), 4.59 (d, J = 11.4 Hz, 1H), 4.35 (td, J = 12.2, 3.3 Hz, 1H), 4.19 (ddd, J = 17.8, 9.3, 4.3 Hz, 2H), 4.02 (d, J = 12.2 Hz, 1H), 3.98 (dd, J = 14.4, 4.8 Hz, 1H), 3.82 (s, 2H), 3.80 - 3.72 (m, 1H), 3.60 - 3.52 (m, 1H), 3.49 - 3.24 (m, 5H), 3.22 - 3.11 (m, 3H), 3.03 (dd, J = 21.2, 13.9 Hz, 2H), 2.69 (dd, J = 12.8, 10.2 Hz, 1H), 2.51 - 2.40 (m, 4H), 2.29 - 2.18 (m, 3H), 1.97 (dd, J = 32.6, 16.2 Hz, 3H), 1.86 - 1.79 (m, 3H), 1.50 (d, J = 6.5 Hz, 3H), 1.44 (t, J = 7.2 Hz, 1H), 1.35 - 1.29 (m, 3H), 1.17 - 1.12 (m, 1H), 0.98 (s, 3H), 0.88 (t, J = 6.9 Hz, 1H), 0.42 (d, J = 2.1 Hz, 3H).

### Example 165: Synthesis of compound (1r,2R,3S)-2,3-dimethyl-N-((1⁵S,6³S,4S,Z)-1⁵,10,10-trimethyl-5,7-dioxo-1²-((3S,5R)-3,4,5-trime thylpiperazin-1-yl)-1⁸,1⁹,6¹,6²,6³,6⁴,6⁵,6⁶ -octahydro-1⁵H,1⁷H-8-oxa-1(13,15)-pyrido[3',2':7,8][1, 5]oxazonino[5,6-a]indola-2(4,2)-thiazola-6(1,3)-pyridazinacycloundecaphane-4-yl)cyclopropane -1-carboxamide

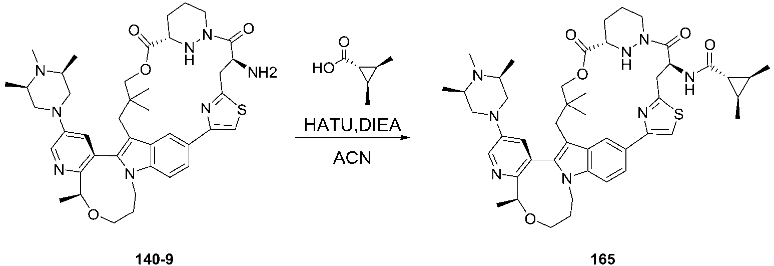

REL-(1R,2R,3S)-2,3-dimethylcyclopropane-1-carboxylic acid (18.15 mg, 0.16 mmol) and acetonitrile (2.00 mL) were added sequentially to a 10 mL single-necked flask. At 0 °C, N,N,N',N'-tetramethyl-O-(7-azabenzotriazol-1-yl)uranium hexafluorophosphate (60.46 mg, 0.16 mmol) and N,N-diisopropylethylamine (0.17 mL, 1.06 mmol) were added, and the mixture was stirred at 0 °C for 10 min. Finally, compound 140-9 (80.00 mg, 0.11 mmol) was added, and the mixture was stirred at room temperature for 30 min. The reaction was complete as monitored by LC-MS, and the reaction solution was directly purified by pre-HPLC to obtain the target compound 165 (33.5 mg). ESI-MS m/z: 851.7 [M+H]⁺.

¹H NMR (500 MHz, CDCl₃)δ 8.58 (s, 1H), 8.40 (d, J = 2.9 Hz, 1H), 7.57 (dd, J = 8.6, 1.4 Hz, 1H), 7.30 (d, J = 8.5 Hz, 1H), 7.28 (s, 1H), 7.06 (d, J = 2.9 Hz, 1H), 6.38 (d, J = 9.6 Hz, 1H), 5.88 (t, J = 8.9 Hz, 1H), 4.59 (d, J = 12.9 Hz, 1H), 4.34 (td, J = 12.1, 3.2 Hz, 1H), 4.26 - 4.16 (m, 2H), 4.04 - 3.96 (m, 2H), 3.85 - 3.74 (m, 3H), 3.52 (t, J = 10.4 Hz, 2H), 3.44 (d, J = 14.8 Hz, 1H), 3.31 (td, J = 12.4, 2.9 Hz, 1H), 3.20 (d, J = 14.4 Hz, 1H), 3.11 (dd, J = 15.0, 8.6 Hz, 1H), 2.71 = 2.61 (m, 3H), 2.47 - 2.37 (m, 3H), 2.33 (s, 3H), 2.23 (dd, J = 17.3, 9.9 Hz, 2H), 2.04 - 1.91 (m, 3H), 1.87 - 1.76 (m, 1H), 1.51 (d, J = 6.4 Hz, 3H), 1.46 - 1.39 (m, 1H), 1.21 - 1.13 (m, 10H), 1.09 (d, J = 6.2 Hz, 3H), 0.97 (s, 3H), 0.90 (t, J = 4.3 Hz, 1H), 0.40 (s, 3H).

### Example 166: Synthesis of compound (1r,2R,3S)-N-((1⁵S,6³S,4S,Z)-1²-((2S,6R)-2,6-dimethylmorpholino)-1⁵,10,10-trimethyl-5,7-diox o-1⁸,1⁹,6¹,6²,6³,6⁴,6⁵,6⁶-octahydro-1⁵H,1⁷H-8-oxa-1(13,15)-pyrido[3',2':7,8][1,5]oxazonino[5,6-a ]indola-2(4,2)-thiazola-6(1,3)-pyridazinacycloundecaphane-4-yl)-2,3-dimethylcyclopropane-1-ca rboxamide

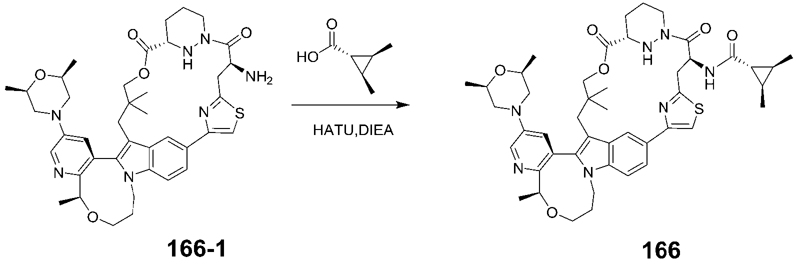

(1r,2R,3S)-2,3-dimethylcyclopropane-1-carboxylic acid (32.31 mg, 0.28 mmol) and acetonitrile (5.00 mL) were added sequentially to a 10 mL single-necked flask. At 0 °C, N,N,N',N'-tetramethyl-O-(7-azabenzotriazol-1-yl)uranium hexafluorophosphate (143.50 mg, 0.38 mmol) and N,N-diisopropylethylamine (0.12 mL, 0.75 mmol) were added, and the mixture was stirred at 0 °C for 10 min. Finally, compound 166-1 (140.00 mg, 0.19 mmol) was added, and the mixture was stirred at 0 °C for 10 min. The reaction was complete as monitored by LC-MS, and the reaction solution was directly purified by pre-HPLC to obtain the target compound 166 (60.9 mg). ESI-MS m/z: 838.4 [M+H]⁺.

¹H NMR (500 MHz, CDCl₃)δ 8.52 (d, J = 1.4 Hz, 1H), 8.33 (d, J = 2.9 Hz, 1H), 7.51 (dd, J = 8.5, 1.6 Hz, 1H), 7.25 (d, J = 8.7 Hz, 1H), 7.21 (s, 1H), 7.01 - 6.98 (m, 1H), 6.30 (d, J = 9.7 Hz, 1H), 5.81 (t, J = 9.0 Hz, 1H), 4.57 - 4.48 (m, 1H), 4.27 (td, J = 12.0, 3.3 Hz, 1H), 4.20 - 4.09 (m, 2H), 3.98 - 3.89 (m, 2H), 3.78-3.67 (m, 5H), 3.43 (dd, J = 10.2, 1.9 Hz, 2H), 3.38 (d, J = 15.0 Hz, 1H), 3.24 (td, J = 12.4, 3.5 Hz, 1H), 3.15 (d, J = 14.4 Hz, 1H), 3.01 (dd, J = 15.1, 8.5 Hz, 1H), 2.60 (td, J = 12.9, 2.9 Hz, 1H), 2.43 (dd, J = 22.0, 10.5 Hz, 2H), 2.34 (d, J = 14.4 Hz, 1H), 2.30 - 2.20 (m, 1H), 2.19 - 2.12 (m, 1H), 1.95 - 1.87 (m, 2H), 1.81 - 1.70 (m, 1H), 1.55 - 1.48 (m, 1H), 1.45 (d, J = 6.5 Hz, 3H), 1.43 - 1.33 (m, 2H), 1.19 - 1.18 (m, 7H), 1.09 (d, J = 6.2 Hz, 3H), 1.02 (d, J = 6.2 Hz, 3H), 0.91 (s, 3H), 0.34 (s, 3H).

### Example 171: Synthesis of compound (1r,2R,3S)-2,3-dimethyl-N-((1⁵S,6³S,4S,Z)-15,10,10-trimethyl-5,7-dioxo-1²-((3S,5S)-3,4,5-trime thylpiperazin-1-yl)-1⁸,1⁹,6¹,6²,6³,6⁴,6⁵,6⁶-octahydro-1⁵H,1⁷H-8-oxa-1(13,15)-pyrido[3',2':7,8][1, 5]oxazonino[5,6-a]indola-2(4,2)-thiazola-6(1,3)-pyridazinacycloundecaphane-4-yl)cyclopropane -1-carboxamide

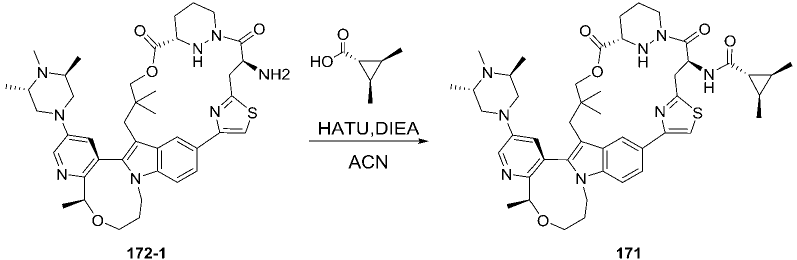

3-methylbicyclo[1.1.1]pentane-1-carboxylic acid (21.62 mg, 0.19 mmol) and acetonitrile (3.00 mL) were added sequentially to a 10 mL single-necked flask. At 0 °C, N,N,N',N'-tetramethyl-O-(7-azabenzotriazol-1-yl)uranium hexafluorophosphate (78.56 mg, 0.21 mmol) and N,N-diisopropylethylamine (0.28 mL, 1.72 mmol) were added, and the mixture was stirred at 0 °C for 10 min. Finally, compound 172-1 (100.00 mg, 0.13 mmol) was added, and the mixture was stirred at room temperature for 30 min. The reaction was complete as monitored by LC-MS, and the reaction solution was directly purified by preparative thin layer chromatography to obtain the target compound 171 (90.0 mg). ESI-MS m/z: 426.4 [M+2H]2⁺/2.

¹H NMR (500 MHz, CDCl₃) δ 8.57 (d, J = 1.3 Hz, 1H), 8.39 (d, J = 2.9 Hz, 1H), 7.58 (dd, J = 8.6, 1.5 Hz, 1H), 7.31 (d, J = 8.6 Hz, 1H), 7.28 (s, 1H), 7.05 (d, J = 2.9 Hz, 1H), 6.41 (d, J = 9.6 Hz, 1H), 5.89 (t, J = 8.9 Hz, 1H), 4.59 (m, 1H), 4.33 (td, J = 12.1, 3.3 Hz, 1H), 4.26 - 4.17 (m, 2H), 4.00 (d, J = 12.2 Hz, 2H), 3.85 - 3.73 (m, 3H), 3.73 - 3.66 (m, 1H), 3.44 (d, J = 14.8 Hz, 1H), 3.38 (m, 2H), 3.34 - 3.27 (m, 1H), 3.21 (d, J = 14.5 Hz), 3.14 - 3.07 (m, 3H), 2.68 (td, J = 12.9, 2.8 Hz, 1H), 2.52 (s, 3H), 2.41 (d, J = 14.4 Hz, 1H), 2.35 - 2.21 (m, 2H), 2.00 - 1.91 (m, 2H), 1.87 - 1.74 (m, 3H), 1.55-1.64 (m, 2H), 1.51 (d, J = 6.5 Hz, 3H), 1.34-1.29 (m, 1H), 1.21 (d, J = 6.3 Hz, 6H), 1.16 (d, J = 6.2 Hz, 3H), 1.10 (d, J = 6.2 Hz, 3H), 0.98 (s, 3H), 0.42 (s, 3H).

### Example 172: Synthesis of compound 3-methyl-N-((1⁵S,6³S,4S,Z)-1⁵,10,10-trimethyl-5,7-dioxo-1²-((3S,5S)-3,4,5-trimethylpiperazin-1 -y)-1⁸,1⁹,6¹,6²,6³,6⁴,6⁵,6⁶-octahydro-1⁵H,1⁷H-8-oxa-1(13,15)-pyrido[3',2':7,8][1,5]oxazonino[5, 6-a]indola-2(4,2)-thiazola-6(1,3)-pyridazinacycloundecaphane-4-yl)bicyclo[1.1.1]pentane-1-carb oxamide

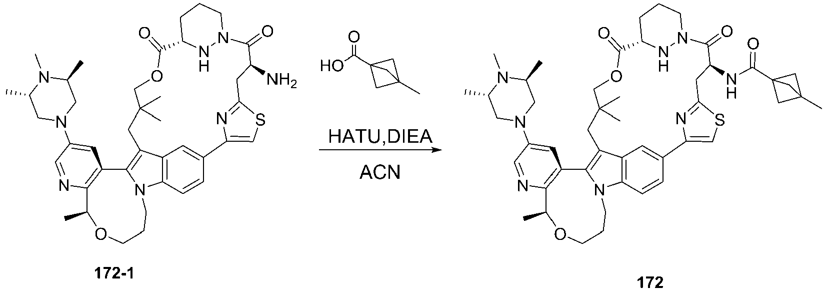

3-Methylbicyclo[1.1.1]pentane-1-carboxylic acid (6.77 mg, 0.05 mmol) and acetonitrile (1.00 mL) were added sequentially to a 10 mL single-necked flask. At 0 °C, N,N,N',N'-tetramethyl-O-(7-azabenzotriazol-1-yl)uranium hexafluorophosphate (20.42 mg, 0.05 mmol) and N,N-diisopropylethylamine (0.06 mL, 0.36 mmol) were added, and the mixture was stirred at 0 °C for 10 min. Finally, compound 172-1 (27.00 mg, 0.04 mmol) was added, and the mixture was stirred at room temperature for 30 min. The reaction was complete as monitored by LC-MS, and the reaction solution was directly purified by pre-HPLC to obtain the target compound 172 (12.0 mg). ESI-MS m/z: 863.1 [M+H]⁺.

¹H NMR (500 MHz, CDCl₃) δ 8.59 (s, 1H), 8.40 (d, J = 2.9 Hz, 1H), 7.60 - 7.54 (m, 1H), 7.31 (d, J = 8.5 Hz, 1H), 7.28 (s, 1H), 7.04 (d, J = 2.9 Hz, 1H), 6.34 (d, J = 9.9 Hz, 1H), 5.83 (t, J = 9.0 Hz, 1H), 4.57 (d, J = 13.0 Hz, 1H), 4.34 (td, J = 12.0, 4.3 Hz, 1H), 4.26 - 4.15 (m, 2H), 4.05 - 3.95 (m, 2H), 3.87 - 3.74 (m, 3H), 3.46 (d, J = 15.0 Hz, 1H), 3.35 - 3.26 (m, 2H), 3.20 (d, J = 14.3 Hz, 1H), 3.10 (dd, J = 15.0, 8.5 Hz, 1H), 3.01 (s, 4H), 2.72 - 2.63 (m, 1H), 2.46 - 2.19 (m, 6H), 2.03 - 1.90 (m, 4H), 1.96 (s, 6H), 1.51 (d, J = 6.5 Hz, 3H), 1.37 - 1.27 (m, 7H), 1.22 (s, 3H), 0.97 (s, 3H), 0.42 (s, 3H).

### Example 183: Synthesis of compound (1S,2S)-2-methyl-N-((1⁵S,6⁴S,4S,Z)-1⁵,10,10-trimethyl-1²-(4-methylpiperazin-1-yl)-5,7-dioxo-1 ⁸,1⁹-dihydro-1⁵H,1⁷H-8-oxa-6²,6³-diaza-1(13,15)-pyrido[3',2':7,8][1,5]oxazonino[5,6-a]indola-2( 4,2)-thiazola-6(2,4)-bicyclo[3.1.1]heptanacycloundecaphane-4-yl)cyclopropane-1-carboxamide

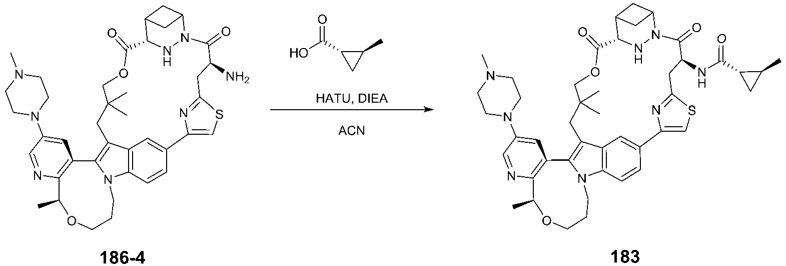

Compound (1S,2S)-2-methylcyclopropane-1-carboxylic acid (3.52 mg, 0.04 mmol) was dissolved in ACN (1.00 mL), the temperature was lowered to 0 °C. Then DIEA (0.03 mL, 0.16 mmol) and HATU (20.61 mg, 0.05 mmol) were added, and the reaction was carried out at 0 °C for 15 min. Then compound 186-4 (20.00 mg, 0.03 mmol) was added, and the reaction was carried out at 0 C for 0.5 h. After the reaction was complete as monitored by LC-MS, purification by preparative chromatography yielded the target compound 183 (13.8 mg). ESI-MS m/z: 821.38 [M+H]⁺.

¹H NMR (500 MHz, DMSO-d₆) δ 8.55 (d, J = 9.0 Hz, 1H), 8.41 (dd, J = 7.5, 2.3 Hz, 2H), 7.81 (s, 1H), 7.71 (dd, J = 8.6, 1.6 Hz, 1H), 7.49 (d, J = 8.6 Hz, 1H), 7.21 (d, J = 3.0 Hz, 1H), 5.92 (d, J = 11.1 Hz, 1H), 5.33 (t, J = 8.2 Hz, 1H), 4.74 (d, J = 11.1 Hz, 1H), 4.50 (q, J = 5.0 Hz, 1H), 4.14 (t, J = 6.5 Hz, 1H), 4.03 (dd, J = 12.8, 5.2 Hz, 2H), 3.68-3.52 (m, 3H), 3.26 (s, 5H), 3.15 (d, J = 8.4 Hz, 2H), 3.00 (d, J = 14.4 Hz, 1H), 2.66 (d, J = 5.9 Hz, 1H), 2.43 (d, J = 20.1 Hz, 7H), 2.22 (s, 3H), 2.16 (t, J = 9.9 Hz, 1H), 1.64 (t, J = 9.4 Hz, 1H), 1.50 (s, 1H), 1.37 (d, J = 6.4 Hz, 3H), 1.11 (s, 3H), 1.08 (d, J = 1.8 Hz, 4H), 0.93 (s, 3H), 0.30 (s, 3H).

### Example 184: Synthesis of compound (1S,2S)-2-(difluoromethyl)-N-((1⁵S,6⁴S,4S,Z)-1⁵,10,10-trimethyl-1²-(4-methylpiperazin-1-yl)-5, 7-dioxo-1⁸,1⁹-dihydro-1⁵H,1⁷H-8-oxa-6²,6³-diaza-1(13,15)-pyrido[3',2':7,8][1,5]oxazonino[5,6-a ]indola-2(4,2)-thiazola-6(2,4)-bicyclo[3.1.1]heptanacycloundecaphane-4-yl)cyclopropane-1-carb oxamide

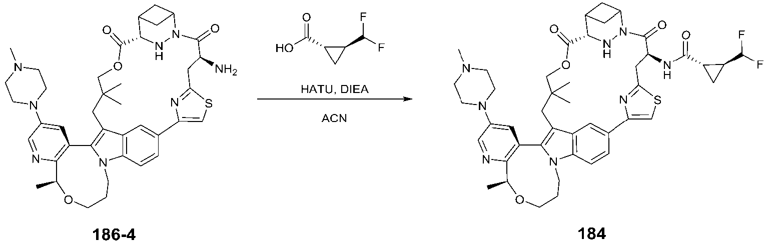

Compound (1S,2S)-2-(difluoromethyl)cyclopropane-1-carboxylic acid (7.19 mg, 0.05 mmol) was dissolved in ACN (4.00 mL), the temperature was lowered to 0 °C. Then DIEA (0.04 mL, 0.24 mmol) and HATU (30.87 mg, 0.08 mmol) were added, and the reaction was carried out at 0 °C for 15 min. Then compound 186-4 (30.00 mg, 0.04 mmol) was added, and the reaction was carried out at 0 °C for 0.5 h. After the reaction was complete as monitored by LC-MS, the reaction solution was directly added to 30 mL of water to quench the reaction, then extracted with DCM (20 mL * 3), washed with saturated sodium chloride solution (20 mL * 2), dried over anhydrous sodium sulfate, filtered, and concentrated to dryness to obtain a crude product of the target product. The crude product was separated by column chromatography (DCM:MeOH = 97:3 to 90:10) to obtain the target compound 184 (27.3 mg). ESI-MS m/z: 853.45, [M+H]⁺.

¹H NMR (500 MHz, DMSO-d₆) δ 8.90 (d, J = 9.0 Hz, 1H), 8.48-8.39 (m, 2H), 7.82 (s, 1H), 7.71 (d, J = 8.5 Hz, 1H), 7.50 (d, J = 8.5 Hz, 1H), 7.21 (d, J = 2.9 Hz, 1H), 5.98-5.94 (m, 1H), 5.84 (d, J = 5.0 Hz, 1H), 5.36 (t, J = 8.3 Hz, 1H), 4.74 (d, J = 11.1 Hz, 1H), 4.53-4.46 (m, 1H), 4.15 (d, J = 6.5 Hz, 1H), 4.03 (dd, J = 13.0, 5.3 Hz, 2H), 3.69-3.54 (m, 4H), 3.25 (s, 5H), 3.14 (t, J = 7.7 Hz, 2H), 3.00 (d, J = 14.4 Hz, 1H), 2.66 (d, J = 5.9 Hz, 1H), 2.45 (t, J = 5.3 Hz, 5H), 2.38-2.31 (m, 2H), 2.22 (s, 3H), 2.16 (t, J = 9.5 Hz, 1H), 2.09 (q, J = 5.9, 5.4 Hz, 1H), 1.81-1.75 (m, 3H), 1.65 (t, J = 9.6 Hz, 2H), 1.37 (d, J = 6.4 Hz, 3H), 1.09 (dt, J = 16.3, 5.7 Hz, 4H), 0.93 (s, 3H).

### Example 186: Synthesis of compound (1r,2R,3S)-2,3-dimethyl-N-((1⁵S,6⁴S,4S,Z)-1⁵,10,10-trimethyl-1²-(4-methylpiperazin-1-yl)-5,7-d ioxo-1⁸,1⁹-dihydro-1⁵H,1⁷H-8-oxa-6²,6³ -diaza-1(13,15)-pyrido[3',2':7,8][1,5]oxazonino[5,6-a]in dola-2(4,2)-thiazola-6(2,4)-bicyclo[3.1.1]heptanacycloundecaphane-4-yl)cyclopropane-1-carbox amide

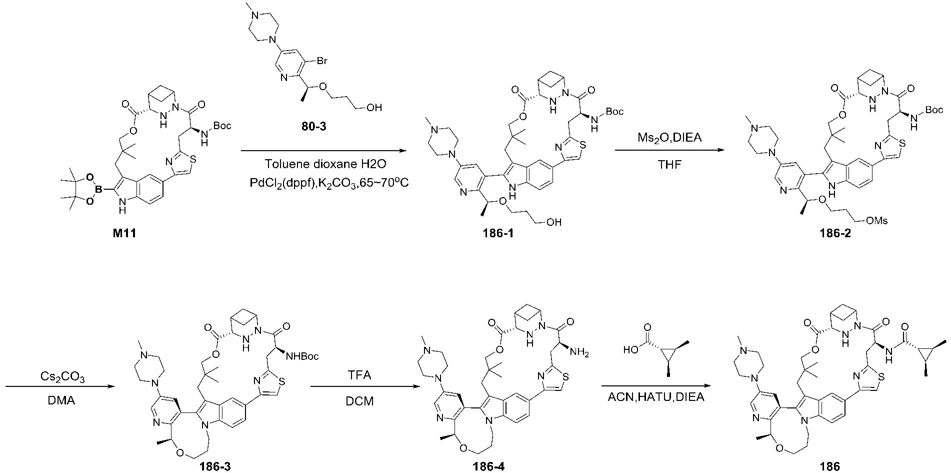

### Step 1: Synthesis of compound 186-1

Compound 80-3 (42.67 mg, 0.12 mmol), M11 (80.00 mg, 0.11 mmol), PdCl₂(dppf) (8.34 mg, 0.01 mmol), and K₂CO₃ (47.02 mg, 0.34 mmol) were added to toluene (4.50 mL), 1,4-dioxane (1.00 mL) and water (1.00 mL). Then, under N₂ protection, the reaction was carried out at 70 °C for 3 h. After the reaction was complete, 30 mL of water was added to the reaction solution to quench the reaction, then extracted three times with 30 mL of DCM, washed twice with 30 mL of saturated sodium chloride solution, and the combined organic phases were concentrated to obtain a crude product. The crude product was separated by column chromatography to obtain the target compound 186-1 (95.00 mg, white solid). ESI-MS m/z: 858.22 [M+H]⁺.

### Step 2: Synthesis of compound 186-2

186-1 (95.00 mg, 0.11 mmol), N,N-diisopropylethylamine (0.05 mL, 0.33 mmol), and THF (3.00 mL) were added sequentially to a 100 mL three-necked flask. Under nitrogen protection and ice-water bath conditions, methanesulfonic anhydride (25.08 mg, 0.14 mmol) was slowly added, and then the mixture was heated to room temperature and reacted for 3 h. After the reaction was complete as monitored by LC-MS, the reaction solution was slowly added to 20.00 mL of water to quench the reaction, then extracted three times with 50 mL of DCM. The organic phases were combined, washed with saturated sodium chloride solution, dried over anhydrous sodium sulfate, and concentrated to dryness to obtain compound 186-2 (100.00 mg, light yellow solid). ESI-MS m/z: 936.21 [M+H]⁺.

### Step 3: Synthesis of compound 186-3

Compound 186-2 (100.00 mg, 0.11 mmol) and Cs₂CO₃ (104.49 mg, 0.32 mmol) were added to DMA (10.00 mL). Then, under N₂ protection, the reaction was carried out at 65 °C for 2 h. After the reaction was complete, 50 mL of water was added to the reaction solution to quench the reaction, then extracted three times with 40 mL of DCM, washed four times with 30 mL of saturated sodium chloride solution, and the combined organic phases were concentrated to obtain a crude product. The crude product was separated by column chromatography to obtain the target compound 186-3 (65.00 mg, light yellow solid). ESI-MS m/z: 840.22 [M+H]⁺.

### Step 4: Synthesis of compound 186-4

Compound 186-3 (65.00 mg, 0.08 mmol) was added to DCM (3.00 mL). Then, under N₂ protection and ice-water bath conditions, trifluoroacetic acid (1.50 mL) was slowly added, and the mixture was heated to room temperature and reacted for 1 h. After the reaction was complete, the reaction solution was concentrated to obtain a crude product. Saturated sodium bicarbonate solution was added to the reaction solution to adjust the pH to 8-9, then extracted three times with 15 mL of DCM, washed with saturated sodium chloride solution, the organic phases were combined, dried over anhydrous sodium sulfate, filtered, and concentrated to dryness to obtain the target product 186-4 (50.00 mg, light yellow solid). ESI-MS m/z: 739.25 [M+H]⁺.

### Step 5: Synthesis of compound 186

Compound REL-(1R,2R,3S)-2,3-dimethylcyclopropane-1-carboxylic acid (10.00 mg, 0.09 mmol) was dissolved in acetonitrile (1.00 mL), the temperature was lowered to 0 °C. Then DIEA (0.07 mL, 0.41 mmol) and HATU (51.48 mg, 0.14 mmol) were added, and the reaction was carried out at 0 °C for 15 min. Then compound 186-4 (50.00 mg, 0.07 mmol) was added, and the reaction was carried out at 0 °C for 0.5 h. After the reaction was complete as monitored by LC-MS, purification by preparative chromatography yielded the target compound 186 (26.90 mg). ESI-MS m/z: 835.38 [M+H]⁺.

¹H NMR (500 MHz, DMSO-d₆) δ 8.45-8.38 (m, 3H), 7.81 (s, 1H), 7.71 (dd, J = 8.7, 1.7 Hz, 1H), 7.49 (d, J = 8.6 Hz, 1H), 7.21 (d, J = 2.9 Hz, 1H), 5.90 (d, J = 11.1 Hz, 1H), 5.36-5.28 (m, 1H), 4.73 (d, J = 11.1 Hz, 1H), 4.50-4.47 (m, 1H), 4.15 (q, J = 6.4 Hz, 1H), 4.05-4.01 (m, 2H), 3.68-3.53 (m, 3H), 3.29-3.23 (m, 5H), 3.16-3.12 (m, 2H), 3.00 (d, J = 14.5 Hz, 1H), 2.67-2.63 (m, 1H), 2.47-2.43 (m, 8H), 2.36-2.31 (m, 2H), 2.22 (s, 3H), 2.15 (t, J = 9.8 Hz, 1H), 1.82-1.76 (m, 1H), 1.63 (t, J = 9.4 Hz, 1H), 1.37 (d, J = 6.4 Hz, 3H), 1.11-1.06 (m, 7H), 0.93 (s, 3H), 0.31 (s, 3H).

### Example 192: Synthesis of compound (1R,5S,6r)-N-((1⁵S,6⁴S,4S,Z)-1⁵,10,10-trimethyl-1²-(4-methylpiperazin-1-yl)-5,7-dioxo-1⁸,1⁹-dih ydro-1⁵H,1⁷H-8-oxa-6²,6³-diaza-1(13,15)-pyrido[3',2':7,8][1,5]oxazonino[5,6-a]indola-2(4,2)-thi azola-6(2,4)-bicyclo[3.1.1]heptanacycloundecaphane-4-yl)-3-oxabicyclo[3.1.0]hexane-6-carboxa mide

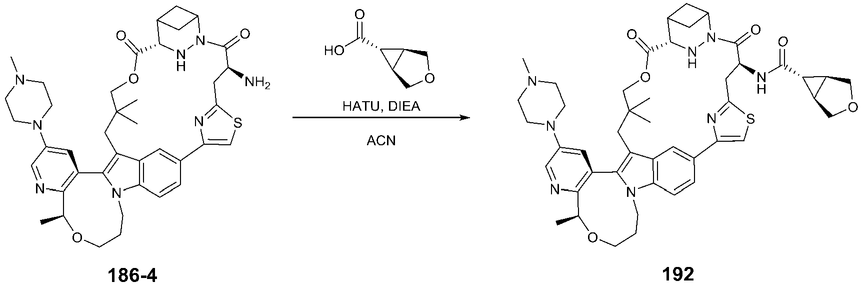

Compound (1R,5S,6r)-rel-3-oxabicyclo[3.1.0]hexane-6-carboxylic acid (7.89 mg, 0.06 mmol) was dissolved in ACN (4.00 mL), the temperature was lowered to 0 °C. Then DIEA (0.05 mL, 0.28 mmol) and HATU (36.05 mg, 0.09 mmol) were added, and the reaction was carried out at 0 °C for 15 min. Then compound 186-4 (35.00 mg, 0.05 mmol) was added, and the reaction was carried out at 0 °C for 0.5 h. After the reaction was complete as monitored by LC-MS, the reaction solution was directly added to 30 mL of water to quench the reaction, then extracted three times with 20 mL of DCM, washed twice with 20 mL of saturated sodium chloride solution, dried over anhydrous sodium sulfate, filtered, and concentrated to dryness to obtain a crude product of the target product. The crude product was separated by column chromatography (DCM:MeOH = 97:3 to 90:10) to obtain the target compound 192 (36.8 mg). ESI-MS m/z: 849.36, [M+H]⁺.

¹H NMR (500 MHz, DMSO-d₆) δ 8.61 (d, J = 8.9 Hz, 1H), 8.42 (d, J = 4.4 Hz, 2H), 7.82 (s, 1H), 7.72 (d, J = 8.5 Hz, 1H), 7.50 (d, J = 8.7 Hz, 1H), 7.28 (s, 1H), 5.94 (d, J = 11.1 Hz, 1H), 5.32 (t, J = 8.2 Hz, 1H), 4.73 (d, J = 11.1 Hz, 1H), 4.49 (q, J = 5.0 Hz, 1H), 4.17 (q, J = 6.6 Hz, 1H), 3.84 (dt, J = 13.5, 6.7 Hz, 2H), 3.69-3.52 (m, 11H), 3.17-3.10 (m, 7H), 3.00 (d, J = 14.4 Hz, 1H), 2.65 (d, J = 6.1 Hz, 2H), 2.42 (d, J = 14.4 Hz, 3H), 2.15 (d, J = 9.9 Hz, 1H), 1.99 (s, 3H), 1.91 (s, 2H), 1.66 (t, J = 3.5 Hz, 2H), 1.38 (d, J = 6.3 Hz, 4H), 1.17 (t, J = 7.1 Hz, 3H), 0.93 (s, 3H).

### Example 193: Synthesis of compound (1r,2R,3S)-2,3-dimethyl-N-((1⁵S,6³S,4S,Z)-1⁵,10,10-trimethyl-1²-((4-methylpiperazin-1-yl)meth yl)-5,7-dioxo-1⁸,1⁹,6¹,6²,6³,6⁴,6⁵,6⁶-octahydro-1⁵H,1⁷H-8-oxa-1(13,15)-pyrido[3',2':7,8][1,5]oxa zonino[5,6-a]indola-2(4,2)-thiazola-6(1,3)-pyridazinacycloundecaphane-4-yl)cyclopropane-1-car boxamide

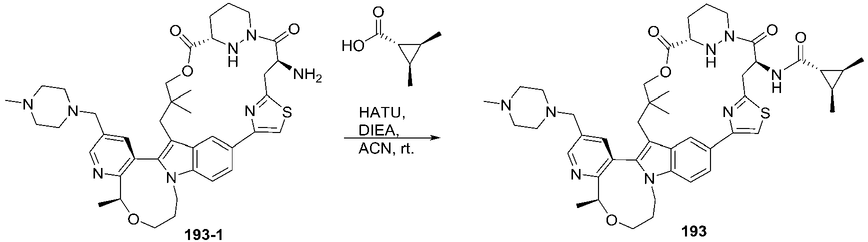

Compound REL-(1R,2R,3S)-2,3-dimethylcyclopropane-1-carboxylic acid (33.9 mg) was added to a 25 mL single-necked flask, HATU (112.9 mg), N,N-diisopropylethylamine (0.15 mL) and anhydrous acetonitrile (2 mL) were added, and the reaction was carried out at room temperature for 10 min. Then an acetonitrile solution (2 mL) of 193-1 (110 mg, synthesized from intermediate M12 with reference to the synthetic procedure of compound 186) was added, and the reaction was carried out at room temperature for 15 min. After the reaction was complete, it was directly concentrated, and the concentrate was purified by preparation to obtain the target compound 193 (71.1 mg). ESI-MS m/z: 837.44 [M+H]⁺.

### Example 194: Synthesis of compound (1r,2R,3S)-2,3-dimethyl-N-((1⁵S,6³S,4S,Z)-1⁵,10,10-trimethyl-1²-(morpholinomethyl)-5,7-dioxo -1⁸,1⁹,6¹,6²,6³,6⁴,6⁵,6⁶-octahydro-1⁵H,1⁷H-8-oxa-1(13,15)-pyrido[3',2':7,8][1,5]oxazonino[5,6-a]i ndola-2(4,2)-thiazola-6(1,3)-pyridazinacycloundecaphane-4-yl)cyclopropane-1-carboxamide

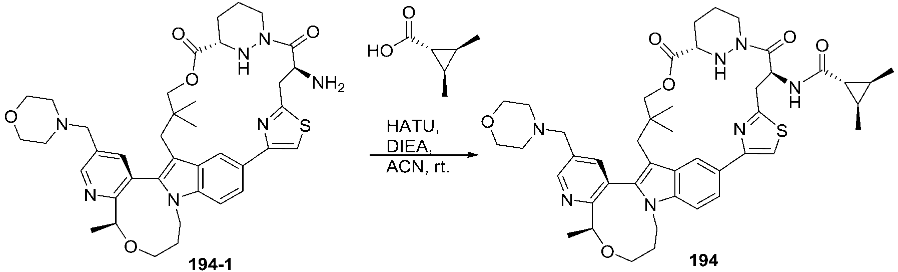

Compound REL-(1R,2R,3S)-2,3-dimethylcyclopropane-1-carboxylic acid (30 mg) was added to a 25 mL single-necked flask, HATU (65 mg), N,N-diisopropylethylamine (120 µL) and anhydrous acetonitrile (10 mL) were added, and the reaction was carried out at room temperature for 10 min; a pre-liberated acetonitrile solution (5 mL) of 194-1 (100 mg) with DIEA was added dropwise at room temperature, and the reaction was carried out at room temperature for 15 min. After the reaction was complete, it was directly concentrated, and the concentrate was purified by preparation to obtain the target compound 194 (36.2 mg). ESI-MS m/z: 824.2 [M+H]⁺.

¹H NMR (500 MHz, CDCl₃). δ 8.55 (dd, J = 34.9, 1.6 Hz, 2H), 7.60 (s, 1H), 7.51 (dd, J = 8.6, 1.5 Hz, 1H), 7.25 - 7.19 (m, 3H), 6.32 (d, J = 9.6 Hz, 1H), 5.82 (t, J = 8.8 Hz, 1H), 4.52 (d, J = 12.3 Hz, 1H), 4.32 - 4.14 (m, 4H), 4.00 - 3.89 (m, 2H), 3.81 - 3.50 (m, 11H), 3.45 - 3.24 (m, 3H), 3.15 (d, J = 14.5 Hz, 1H), 3.05 (dd, J = 14.9, 8.6 Hz, 1H), 2.60 (td, J = 12.9, 2.8 Hz, 2H), 2.42 (s, 5H), 2.30 (d, J = 14.5 Hz, 2H), 2.21 - 2.12 (m, 1H), 1.92 (dd, J = 22.8, 8.4 Hz, 3H), 1.75 (dd, J = 9.1, 3.9 Hz, 2H), 1.54 (ddd, J = 13.4, 12.1, 7.4 Hz, 6H), 1.49 - 1.34 (m, 6H), 1.30 (s, 1H), 1.24 - 1.21 (m, 2H), 1.08 (d, J = 6.2 Hz, 3H), 1.05 (dd, J = 6.1, 2.6 Hz, 1H), 1.02 (d, J = 6.2 Hz, 3H), 0.85 (d, J = 3.5 Hz, 3H), 0.84 - 0.76 (m, 2H), 0.27 (s, 3H).

### Example 195: Synthesis of compound (1r,2R,3S)-2,3-dimethyl-N-((1⁵S,6⁴S,4S,Z)-1⁵,10,10-trimethyl-1²-(4-methylpiperazin-1-yl)-5,7-d ioxo-1⁸,1⁹ -dihydro-1⁵H,1⁷H-8-oxa-6²,6³-diaza-1(13,15)-pyridazino[4',3':7,8][1,5]oxazonino[5,6-a]indola-2(4,2)-thiazola-6(2,4)-bicyclo[3.1.1]heptanacycloundecaphane-4-yl)cyclopropane-1-car boxamide

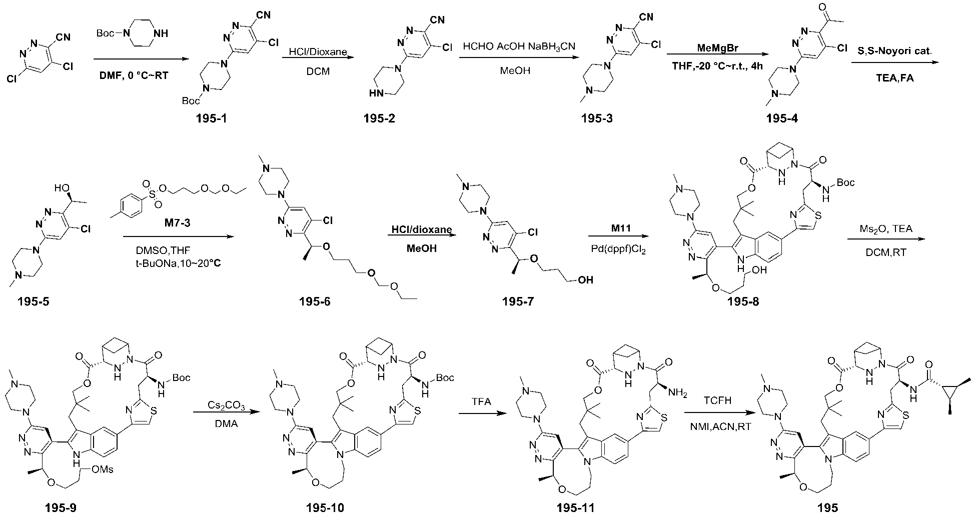

### Step 1: Synthesis of compound 195-1

Compound 4,6-dichloropyridazine-3-carbonitrile (5 g, 28.74 mmol) was dissolved in DMF (100 mL), the temperature was lowered to 0 °C, 1-Boc-piperazine (5.35 g, 28.74 mmol) and N,N-diisopropylethylamine (7.34 g, 57.48 mmol) were added. After the addition was complete, the reaction was continued at 0 °C. After the reaction was complete, the reaction solution was quenched with saturated ammonium chloride solution, extracted twice with EA, washed twice with saturated brine, dried over anhydrous sodium sulfate, filtered, rotary evaporated, and purified by column chromatography to obtain compound 195-1 (2.15 g) (the less polar compound was the product, the more polar one was the isomer, separated by column). ESI-MS m/z: 324.31 [M+H]⁺.

### Step 2: Synthesis of compound 195-2

Compound 195-1 (2.05 g, 6.33 mmol) was dissolved in dichloromethane (20 mL), hydrochloric acid (4M in dioxane, 15 mL) was added. After the addition was complete, the reaction was carried out at room temperature. After the reaction was complete as monitored, it was directly rotary evaporated to dryness to obtain compound 195-2 (1.42 g). ESI-MS m/z: 224.16 [M+H]⁺.

### Step 3: Synthesis of compound 195-3

Compound 195-2 (1.42 g, 6.35 mmol) was dissolved in methanol (20 mL), triethylamine (2.65 mL, 19.05 mmol) was added to liberate the compound. Then 37% aqueous formaldehyde solution (5.15 g, 37%, 63.49 mmol) and acetic acid (1.82 mL, 31.74 mmol) were added, and finally NaBH₃CN/sodium cyanoborohydride (1.20 g, 19.05 mmol) was added, and the reaction was carried out at room temperature for 0.5 h. After the reaction was complete, it was quenched with saturated ammonium chloride solution, extracted twice with DCM, and purified by column chromatography to obtain compound 195-3 (1.25 g). ESI-MS m/z: 238.18 [M+H]⁺.

### Step 4: Synthesis of compound 195-4

Compound 195-3 (1.25 g, 3.49 mmol) was dissolved in tetrahydrofuran (15 mL). Under N₂ protection, the temperature was lowered to -20 °C, and methylmagnesium bromide (3.49 mL, 3.00 mol/L, 10.48 mmol) was slowly added dropwise. After the addition was complete and the reaction was complete at room temperature, the reaction solution was added to ice-cold dilute hydrochloric acid to pH=3-4, stirred for ten min, the imine intermediate was completely converted to the ketone, adjusted to pH=9 with saturated sodium carbonate solution, extracted twice with EA, dried, filtered, rotary evaporated, and purified by column chromatography to obtain compound 195-4 (0.45 g). ESI-MS m/z: 255.19 [M+H]⁺.

### Step 5: Synthesis of compound 195-5

Compound 195-4 (0.45 g, 1.75 mmol) was dissolved in triethylamine (6 mL), (S,S)-N-(p-toluenesulfonyl)-1,2-diphenylethane-1,2-diamine (p-cymene)ruthenium(II) chloride (44 mg, 0.07 mmol) and FA/formic acid (0.32 g, 7 mmol) were added, and the reaction was carried out at room temperature for 6 h. After the reaction was complete, it was directly mixed with silica gel and purified by column chromatography to obtain compound 195-5 (0.26 g). ESI-MS m/z: 257.31 [M+H]⁺.

### Step 6: Synthesis of compound 195-6

Compound 195-5 (260 mg, 1.01 mmol) and compound M7-3 (584 mg, 2.03 mmol) were dissolved in dimethyl sulfoxide (5 mL) and tetrahydrofuran (1 mL), the temperature was lowered to 0 °C, sodium tert-butoxide (195 mg, 2.03 mmol) was added, and then the mixture was returned to room temperature and reacted for 3 h. After the reaction was complete, the temperature was lowered to 0 °C, quenched with saturated ammonium chloride solution, extracted twice with EA, washed three times with saturated brine, dried, filtered, and rotary evaporated. The crude product was directly used in the next step to obtain compound 195-6 (377 mg). ESI-MS m/z: 373.39 [M+H]⁺.

### Step 7: Synthesis of compound 195-7

Compound 195-6 (300 mg, 0.61 mmol) was dissolved in methanol (3 mL), hydrochloric acid (4M in dioxane) (3 mL) was added, and the reaction was carried out at room temperature for 10 min. After the reaction was complete, the reaction solution was cooled to 0 °C, the compound was liberated using saturated sodium bicarbonate solution, extracted with EA, washed once with saturated brine, and purified by column chromatography to obtain compound 195-7 (121 mg). ESI-MS m/z: 315.35 [M+H]⁺.

### Step 8: Synthesis of compound 195-8

Compound 195-7 (95 mg, 0.3 mmol), M11 (214 mg, 0.3 mmol), CataCXium A Pd G3 (22 mg, 0.03 mmol), and potassium phosphate (193 mg, 0.91 mmol) were dissolved in 1,4-dioxane (3 mL) and water (0.30 mL). After nitrogen replacement, the reaction was carried out at 80 °C for 2 h. After the reaction was complete, the sample was directly mixed with silica gel and purified by column chromatography to obtain compound 195-8 (195 mg). ESI-MS m/z: 858.53 [M+H]⁺.

### Step 9: Synthesis of compound 195-9

Compound 195-8 (195 mg, 0.23 mmol) was dissolved in tetrahydrofuran (4 mL), N,N-diisopropylethylamine (149 mg, 1.15 mmol) was added, and finally methanesulfonic anhydride (80 mg, 0.46 mmol) was added. After the addition was complete, the reaction was carried out at room temperature. After the reaction was complete, the reaction solution was diluted with EA and water, extracted once with EA, washed with saturated brine, dried, filtered, and rotary evaporated to obtain compound 195-9 (226 mg). ESI-MS m/z: 936.50 [M+H]⁺.

### Step 10: Synthesis of compound 195-10

Compound 195-9 (226 mg, 0.24 mmol) was dissolved in DMA/N,N-dimethylacetamide (3 mL), cesium carbonate (234 mg, 0.72 mmol) was added. After the addition was complete, the reaction was carried out at 60 °C for 2 h. After the reaction was complete, the reaction solution was diluted with EA and water, extracted once with EA, washed three times with saturated brine, and purified by column chromatography to obtain compound 195-10 (130 mg). ESI-MS m/z: 840.52 [M+H]⁺.

### Step 11: Synthesis of compound 195-11

Compound 195-10 (130 mg, 0.16 mmol) was dissolved in DCM/dichloromethane (2 mL), TFA/trifluoroacetic acid (1 mL) was added, and the reaction was carried out at room temperature for 0.5 h. After the reaction was complete, the reaction solution was rotary evaporated, dissolved in EA and water, the compound was liberated with saturated sodium bicarbonate solution, extracted twice with EA, washed once with saturated brine, dried, filtered, and rotary evaporated to obtain compound 195-11 (100 mg), which was directly used in the next step. ESI-MS m/z: 740.46 [M+H]⁺.

### Step 12: Synthesis of compound 195

Compound 195-11 (100 mg, 0.14 mmol) and REL-(1R,2R,3S)-2,3-dimethylcyclopropane-1-carboxylic acid (23.14 mg, 0.20 mmol) were dissolved in acetonitrile (4 mL), N-methylimidazole (55.46 mg, 0.68 mmol) was added, and finally N,N,N',N'-tetramethylchloroformamidinium hexafluorophosphate (56.87 mg, 0.20 mmol) was added. After the addition was complete, the reaction was carried out at room temperature for 10 min. The reaction solution was quenched with saturated ammonium chloride solution, extracted twice with EA, washed twice with saturated brine, dried over anhydrous sodium sulfate, filtered, rotary evaporated, and purified by preparation to obtain compound 195 (71.3 mg). ESI-MS m/z: 836.52 [M+H]⁺.

¹H NMR (500 MHz, DMSO-d₆) δ 8.42-8.44 (m, 2H), 7.84 (s, 1H), 7.75 (dd, J=8.5, 1.5Hz, 1H), 7.53 (d, J=8.5Hz, 1H), 7.25 (s, 1H), 5.91 (d, J=11.0Hz, 1H), 5.33-5.30 (m, 1H), 4.73 (d, J=11.0Hz, 1H), 4.51-4.48 (m, 1H), 4.27 (q, J=6.5Hz, 1H), 4.09-4.05 (m, 2H), 3.69-3.60 (m, 6H), 3.56 (d, J=10.5Hz, 1H), 3.26 (d, J=14.5Hz, 1H), 3.21-3.18 (m, 1H), 3.15-3.11 (m, 1H), 3.00 (d, J=14.5Hz, 1H), 2.67-2.64 (m, 1H), 2.52-2.51 (m, 1H), 2.44-2.42 (m, 5H), 2.37-2.32 (m, 2H), 2.22 (s, 3H), 2.17-2.13 (m, 1H), 1.88-1.83 (m, 1H), 1.65-1.61 (m, 1H), 1.49 (d, J=6.5Hz, 3H), 1.26-1.23 (m, 1H), 1.18-1.14 (m, 2H), 1.10 (d, J=6.5Hz, 3H), 1.07 (d, J=5.5Hz, 3H), 0.95 (s, 3H), 0.32 (s, 3H).

### Example 197: Synthesis of compound (1r,2R,3S)-N-((1⁵S,6⁴S,4S,Z)-1²-(4-ethylpiperazin-1-yl)-1⁵,10,10-trimethyl-5,7-dioxo-1⁸,1⁹-dihy dro-1⁵H,1⁷H-8-oxa-6²,6³-diaza-1(13,15)-pyrido[3',2':7,8][1,5]oxazonino[5,6-a]indola-2(4,2)-oxa zola-6(2,4)-bicyclo[3.1.1]heptanacycloundecaphane-4-yl)-2,3-dimethylcyclopropane-1-carboxa mide

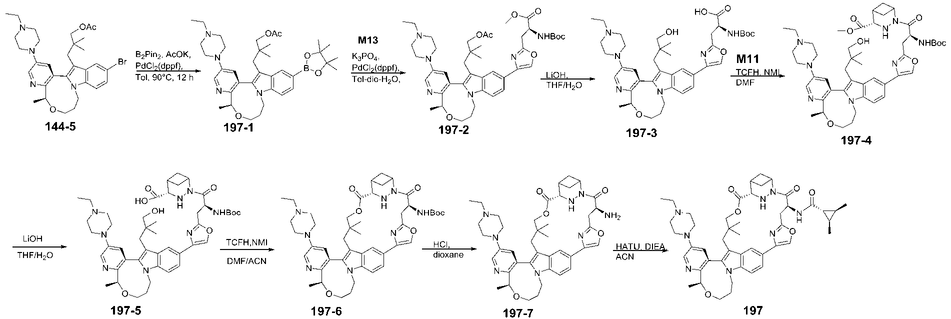

### Step 1: Synthesis of compound 197-1

Compound 144-5 (500 mg), B₂Pin₂ (320 mg), Pd(dppf)Cl₂ (104 mg), and potassium acetate (246 mg) were weighed and added respectively into a 100 mL three-necked flask, toluene (20 mL) was added, the atmosphere was replaced by N₂ for 3 times, the mixture was heated to 90 °C and reacted for 8 h. After the reaction was complete, EA and water were added to the reaction solution, extracted, the aqueous phase was further extracted twice with EA, the organic phases were collected and combined, dried over anhydrous sodium sulfate, concentrated, and the concentrate was purified by column chromatography (DCM:MeOH = 10:1) to obtain the target compound 197-1 (525 mg, 97.3% yield). ESI-MS m/z: 645.4 [M+H]⁺.

### Step 2: Synthesis of compound 197-2

Compound 197-1 (410 mg), M13 (630 mg), Pd(dppf)Cl₂ (70 mg), and potassium phosphate (405 mg) were weighed and added respectively into a 100 mL two-necked flask, toluene (10 mL), 1,4-dioxane (3 mL) and water (3 mL) were added, the atmosphere was replaced by N₂ for 3 times, and the mixture was heated to 70 °C and reacted for 24 h. After the reaction was complete, EA and water were added to the reaction solution, extracted, the aqueous phase was further extracted twice with EA, the organic phases were collected and combined, dried over anhydrous sodium sulfate, concentrated, and the concentrate was purified by column chromatography (DCM:MeOH = 10:1) to obtain the target compound 197-2 (318 mg, 63.6% yield). ESI-MS m/z: 787.4 [M+H]⁺.

### Step 3: Synthesis of compound 197-3

Intermediate 197-2 (318 mg) was added to a 100 mL single-necked flask, lithium hydroxide (24 mg), THF (10 mL), and water (2.5 mL) were weighed and added in, and the reaction was carried out at 25 °C for 12 h. After the reaction was complete, it was diluted with saturated brine (20 mL), extracted with EA, the organic phase was dried over anhydrous sodium sulfate, and concentrated to obtain a crude product of the target compound 197-3 (323 mg, 110% yield). ESI-MS m/z: 731.1 [M+H]⁺.

### Step 4: Synthesis of compound 197-4

Intermediate 197-3 (323 mg) and M11 (143 mg) were added to a 50 mL single-necked flask, anhydrous DMF (15 mL) was added; under nitrogen protection, the temperature was lowered to 0°C, NMI (208 µL) and TCFH (161 mg) were added, and the reaction was carried out at 0 °C for 15 min. After the reaction was complete, it was diluted with water (150 mL), extracted with EA, the organic phase was dried over anhydrous sodium sulfate, concentrated, and the concentrate was purified by column chromatography (DCM:MeOH = 9:1) to obtain the target compound 197-4 (239 mg, 62.4% yield). ESI-MS m/z: 870.2 [M+H]⁺.

### Step 5: Synthesis of compound 197-5

Intermediate 197-4 (239 mg) was added to a 50 mL single-necked flask, lithium hydroxide (16.5 mg), THF (10 mL), and water (2.5 mL) were weighed and added in, and the reaction was carried out at 0 °C for 0.5 h. After the reaction was complete, it was adjusted to neutral with saturated ammonium chloride aqueous solution (15 mL), extracted with EA, the organic phase was dried over anhydrous sodium sulfate, and concentrated to obtain the target compound 197-5 (227 mg, 97% yield). ESI-MS m/z: 854.5 [M+H]⁺.

### Step 6: Synthesis of compound 197-6

NMI (230 µL) and anhydrous ACN (20 mL) were added to a 50 mL single-necked flask, under nitrogen protection the temperature was lowered to 0 °C, TCFH (300 mg) was added, and the mixture was transferred to 25 °C and reacted for 15 min; a solution of intermediate 197-5 (227 mg) in 5 mL of anhydrous DMF was added dropwise, and the reaction was carried out at room temperature for 0.5 h; after the reaction was complete, it was diluted with water (200 mL), extracted with EA, the organic phase was dried over anhydrous sodium sulfate, and concentrated to obtain the target compound 197-6 (529 mg, >100% yield, containing DMF). ESI-MS m/z: 837.2 [M+H]⁺.

### Step 7: Synthesis of compound 197-7

Intermediate 197-6 (529 mg) was added to a 50 mL single-necked flask, anhydrous DCM and hydrogen chloride in dioxane solution (4 N) (10 mL each) were added, and the reaction was carried out at room temperature for 1 h. After the reaction was complete, it was directly concentrated under reduced pressure to obtain the hydrochloride salt of the target compound 197-7 (282 mg, 120% yield). ESI-MS m/z: 737.1 [M+H]⁺.

### Step 8: Synthesis of compound 197

REL-(1R,2R,3S)-2,3-dimethylcyclopropane-1-carboxylic acid (66 mg) was added to a 100 mL single-necked flask, HATU (175 mg), N,N-diisopropylethylamine (0.31 mL) and anhydrous acetonitrile (25 mL) were weighed and added in, and the reaction was carried out at room temperature for 10 min; a pre-liberated acetonitrile solution (10 mL) of 197-7 (282 mg) was added dropwise at room temperature, and the reaction was carried out at room temperature for 15 min. After the reaction was complete, it was directly concentrated, and the concentrate was purified by preparative TLC (dichloromethane/7M ammonia in methanol = 13:1) to obtain the target compound 197 (71 mg, 22.3% yield). ESI-MS m/z: 833.7 [M+H]⁺.

¹H NMR (500 MHz, DMSO) δ 8.45 (d, J = 8.7 Hz, 1H), 8.41 - 8.38 (m, 1H), 8.34 (s, 1H), 8.18 (d, J = 1.4 Hz, 1H), 7.48 (d, J = 1.5 Hz, 2H), 7.22 (d, J = 2.8 Hz, 1H), 5.84 (d, J = 10.9 Hz, 1H), 5.32 (dd, J = 8.5, 4.2 Hz, 1H), 4.83 (d, J = 10.9 Hz, 1H), 4.49 (d, J = 5.1 Hz, 1H), 4.15-4.10 (m, 1H), 4.06 - 3.98 (m, 2H), 3.64 - 3.56 (m, 2H), 3.48 (d, J = 10.8 Hz, 1H), 3.27-3.23 (m, 4H), 3.17-3.11 (m, 1H), 2.99-2.97 (m, 3H), 2.69-2.65 (m, 1H), 2.53-2.50 (m, 5H), 2.42 (d, J = 14.5 Hz, 1H), 2.37-2.34 (m, 2H), 2.33 - 2.29 (m, 1H), 2.28-2.24 (m, 1H), 2.08 (t, J = 9.8 Hz, 1H), 1.83-1.77 (m, 1H), 1.61 (t, J = 9.4 Hz, 1H), 1.37 (d, J = 6.2 Hz, 3H), 1.24 (d, J = 7.0 Hz, 1H), 1.16-1.13 (m, 2H), 1.10 - 1.06 (m, 3H), 1.06-1.04 (m, 3H), 1.03-1.00 (m, 3H), 0.96 (s, 3H), 0.31 (s, 3H).

### Example 198: Synthesis of compound (1r,2R,3S)-N-((1⁵S,6⁴S,4S,Z)-1²-(4-(oxetan-3-yl)piperazin-1-yl)-1⁵,10,10-trimethyl-5,7-dioxo-1⁸ ,1⁹-dihydro-1⁵H,1⁷H-8-oxa-6²,6³-diaza-1(13,15)-pyrido[3',2':7,8][1,5]oxazonino[5,6-a]indola-2(4 ,2)-thiazola-6(2,4)-bicyclo[3.1.1]heptanacycloundecaphane-4-yl)-2,3-dimethylcyclopropane-1-ca rboxamide

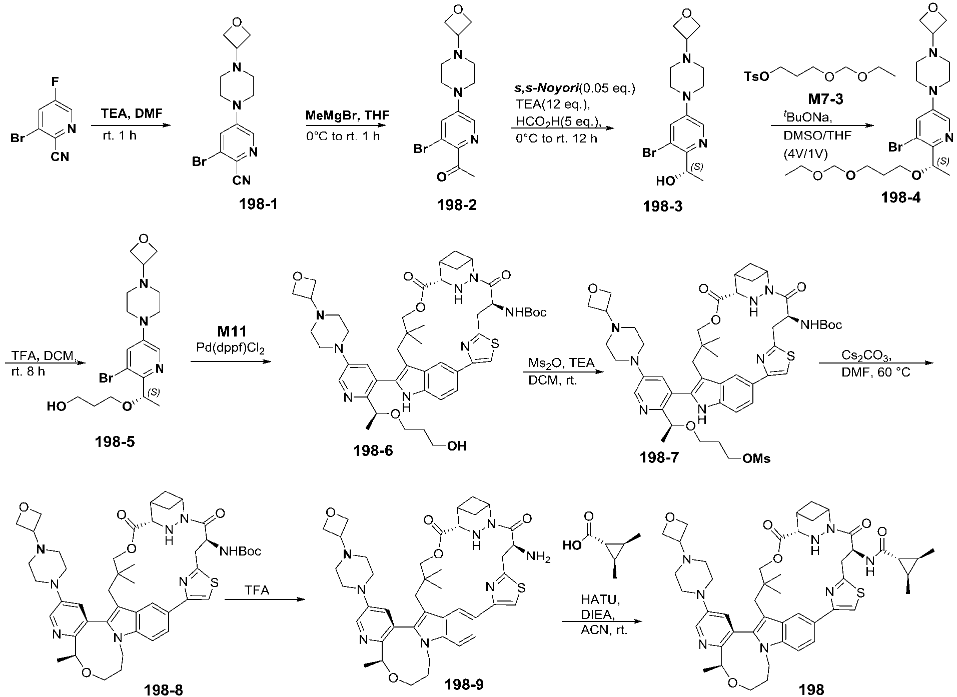

### Step 1: Synthesis of compound 198-1

Compound 5-bromo-3-fluoropicolinonitrile (3.0 g) was added to a 100 mL single-necked flask, anhydrous DMF (20 mL) was added, TEA (8 mL) and 1-(oxetan-3-yl)piperazine (2.33 g) were added respectively, and the reaction was carried out at room temperature for 0.5 h. After the reaction was complete, water (200 mL) was added for precipitation, stirred for 0.5 h, filtered with suction to obtain a filter cake, and dried to obtain the target compound 198-1 (4.65 g, 96.8% yield). ESI-MS m/z: 322.2 [M+H]⁺.

### Step 2: Synthesis of compound 198-2

Intermediate 198-1 (2.65 g) and anhydrous THF (50 mL) were added to a 250 mL three-necked flask, the temperature was lowered to 0 °C under N₂ protection, MeMgBr (3 M in THF, 10 mL) was slowly added dropwise, and the mixture was heated to room temperature and reacted for 1 h. After the reaction was complete, saturated ammonium chloride aqueous solution (50 mL) was added to the reaction solution, and the mixture was stirred at room temperature overnight; the reaction was complete, 50 mL of EA was added for extraction, the organic phase was collected, the aqueous phase was extracted twice with EA (30 mL), the organic phases were collected and combined, dried over anhydrous sodium sulfate, and concentrated to obtain the target compound 198-2 (2.82 g). ESI-MS m/z: 340.4 [M+H]⁺.

### Step 3: Synthesis of compound 198-3

Intermediate 198-2 (2.82 g) was added to a 50 mL two-necked flask, TEA (14 mL) and s,s-Noyori catalyst (0.26 g) were added, the temperature was lowered to 0 °C under N₂ protection, formic acid (1.6 mL) was slowly added dropwise, and the reaction was carried out at room temperature for 12 h. After the reaction was complete, ethyl acetate (50 mL) and water (50 mL) were added to the reaction solution, the mixture was extracted, the organic phase was collected, the aqueous phase was further extracted twice with ethyl acetate (25 mL), the organic phases were collected and combined, dried over anhydrous sodium sulfate, and concentrated to obtain the target compound 198-3 (2.75 g, 97% yield). ESI-MS m/z: 342.1 [M+H]⁺.

### Step 4: Synthesis of compound 198-4

198-3 (2.56 g), M7-3 (5.4 g) and DMSO/THF (20 mL/5 mL) were weighed and added to the reaction flask respectively, and the temperature was lowered to 10 °C under N₂ protection; tBuONa (2.16 g) was added, and the reaction was carried out at room temperature for 1.0 h; the reaction was complete, the temperature was lowered to 10 °C, water (100 mL) was added for dilution, extracted with EA, the organic phase was dried over anhydrous sodium sulfate, concentrated, and the concentrate was purified by column chromatography (dichloromethane:methanol = 10:1) to obtain 198-4 (3.26 g, 95.3% yield). ESI-MS m/z: 458.6 [M+H]⁺.

### Step 5: Synthesis of compound 198-5

Intermediate 198-4 (3.26 g) was added to a 50 mL single-necked flask, anhydrous DCM (50 mL) and TFA (10 mL) were added, and the reaction was carried out at room temperature for 3.0 h. After the reaction was complete, it was concentrated under reduced pressure to dryness. The residue was basified with saturated aqueous sodium bicarbonate solution, extracted with EA, the organic phase was dried over anhydrous sodium sulfate, and concentrated to obtain a crude red oily product 198-5 (3.58 g). ESI-MS m/z: 401.3 [M+H]⁺.

### Step 6: Synthesis of compound 198-6

Compound 198-5 (231 mg), M11 (136 mg), Pd(dppf)Cl₂ (21 mg), and potassium carbonate (80 mg) were weighed and added respectively to a 100 mL two-necked flask, toluene (10 mL), 1,4-dioxane (3 mL) and 3 mL of water were added, the atmosphere was replaced by N₂ for 3 times, and the mixture was heated to 65 °C and reacted for 5 h. After the reaction was complete, EA and water (20 mL each) were added to the reaction solution, the mixture was extracted, the aqueous phase was further extracted twice with EA, the organic phases were collected and combined, dried over anhydrous sodium sulfate, concentrated, and the concentrate was purified by column chromatography (dichloromethane:methanol = 10:1) to obtain the target compound 198-6 (337 mg). ESI-MS m/z: 899.5 [M+H]⁺.

### Step 7: Synthesis of compound 198-7

Intermediate 198-6 (337 mg) was added to a 50 mL three-necked flask, anhydrous DCM (10 mL) and TEA (0.3 mL) were added, and the reaction was carried out at room temperature under nitrogen protection. A solution of methanesulfonic anhydride (105 mg) in ACN (1 mL) was added dropwise. After the reaction was complete, it was diluted with saturated brine solution, extracted with DCM/MeOH (10:1), the organic phase was dried over anhydrous sodium sulfate, and concentrated to obtain the target compound 198-7 (380 mg). ESI-MS m/z: 977.5 [M+H]⁺.

### Step 8: Synthesis of compound 198-8

Intermediate 198-7 (380 mg) was added to a 50 mL single-necked flask, Cs₂CO₃ (380 mg) and anhydrous DMF (14 mL) were added, and the reaction was carried out at 60 °C for 1 h under N₂ protection. After the reaction was complete, it was diluted with water (140 mL), extracted with EA, the organic phase was dried over anhydrous sodium sulfate, and concentrated to obtain crude 198-8 (410 mg). ESI-MS m/z: 881.2 [M+H]⁺.

### Step 9: Synthesis of compound 198-9

Compound 198-8 (410 mg) was added to a 25 mL single-necked flask, anhydrous DCM (11 mL) and TFA (4 mL) were added, and the reaction was carried out at room temperature for 0.5 h. After the reaction was complete, it was concentrated under reduced pressure to dryness. The residue was basified with saturated sodium bicarbonate solution, extracted with DCM/MeOH (10:1), the organic phase was dried, and concentrated to obtain the target compound 198-9 (295 mg). ESI-MS m/z: 781.4 [M+H]⁺.

### Step 10: Synthesis of compound 198

REL-(1R,2R,3S)-2,3-dimethylcyclopropane-1-carboxylic acid (65 mg) was added to a 25 mL single-necked flask, HATU (174 mg), N,N-diisopropylethylamine (0.2 mL) and anhydrous acetonitrile (10 mL) were weighed and added in, and the reaction was carried out at room temperature for 10 min; a pre-liberated acetonitrile solution (8 mL) of 198-9 (295 mg) was added dropwise at room temperature, and the reaction was carried out at room temperature for 15 min. After the reaction was complete, it was directly concentrated, and the concentrate was purified by preparation (60% acetonitrile/water) to obtain the target compound 198 (81.8 mg). ESI-MS m/z: 877.4 [M+H]⁺.

¹H NMR (500 MHz, CDCl₃) δ 8.45 - 8.34 (m, 2H), 7.58 (dd, J = 8.6, 1.5 Hz, 1H), 7.35 - 7.28 (m, 2H), 7.10 (d, J = 2.9 Hz, 1H), 6.71 (d, J = 8.5 Hz, 1H), 5.56 - 5.47 (m, 1H), 5.28 (d, J = 10.7 Hz, 1H), 4.87 (d, J = 10.8 Hz, 1H), 4.75-4.70 (m, 3H), 4.65 (t, J = 6.1 Hz, 2H), 4.23-4.18 (m, 2H), 4.00 (dd, J = 14.9, 4.3 Hz, 1H), 3.81-3.76 (m, 2H), 3.63 (d, J = 10.6 Hz, 1H), 3.60 - 3.52 (m, 2H), 3.45 (d, J = 13.0 Hz, 1H), 3.37 - 3.24 (m, 5H), 3.23 - 3.10 (m, 2H), 2.76 (dd, J = 11.6, 5.8 Hz, 1H), 2.63 - 2.56 (m, 1H), 2.55-2.50 (m, 4H), 2.46 - 2.37 (m, 2H), 2.34-2.28 (m, 1H), 2.14 - 2.05 (m, 1H), 2.01-1.91 (m, 1H), 1.73 (t, J = 9.6 Hz, 1H), 1.54-1.44 (m, 6H), 1.15 (d, J =6.3 Hz, 3H), 1.10 (d, J =6.3 Hz, 3H), 0.97 (s, 3H), 0.38 (s, 3H).

### Example 199: Synthesis of compound N-((1⁵S,6⁴S,4S,Z)-1⁵,10,10-trimethyl-1²-(4-methylpiperazin-1-yl)-5,7-dioxo-1⁸,1⁹-dihydro-1⁵H, 1 ⁷H-8-oxa-6²,6³-diaza-1(13,15)-pyrido[3',2':7,8][1,5]oxazonino[5,6-a]indola-2(4,2)-thiazola-6(2,4 )-bicyclo[3.1.1]heptanacycloundecaphane-4-yl)oxetane-3-carboxamide

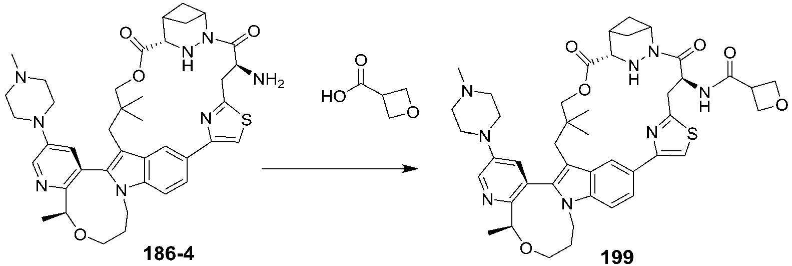

### Step 1: Synthesis of compound 199

Compound 186-4 (30 mg, 0.04 mmol) and compound oxetane-3-carboxylic acid (6.22 mg, 0.06 mmol) were dissolved in acetonitrile (2.00 mL). N-methylimidazole (16.67 mg, 0.20 mmol) was added, and finally N,N,N',N'-tetramethylchloroformamidinium hexafluorophosphate (17.09 mg, 0.06 mmol) was added. After the addition was complete, the reaction was carried out at room temperature for 10 min. The reaction was monitored by LCMS and confirmed complete. The reaction was quenched with saturated ammonium chloride solution, extracted twice with EA, washed once with saturated brine, dried over anhydrous sodium sulfate, filtered, rotary evaporated, and purified by preparation to obtain compound 199 (16.7 mg). ESI-MS m/z: 823.47 [M+H]⁺.

### Example 203: Synthesis of compound (1S,2S)-N-((1⁵S,1⁸R,6³S,4S,Z)-1²-(4-ethylpiperazin-1-yl)-1⁸-methoxy-1⁵,10,10-trimethyl-5,7-dio xo-1⁸,1⁹,6¹,6²6³,6⁴,6⁵,6⁶-octahydro-1⁵H,1⁷H-8-oxa-1(13,15)-pyrido[3',2':7,8][1,5]oxazonino[5,6-a]indola-2(4,2)-thiazola-6(1,3)-pyridazinacycloundecaphane-4-yl)-2-methylcyclopropane-1-carb oxamide

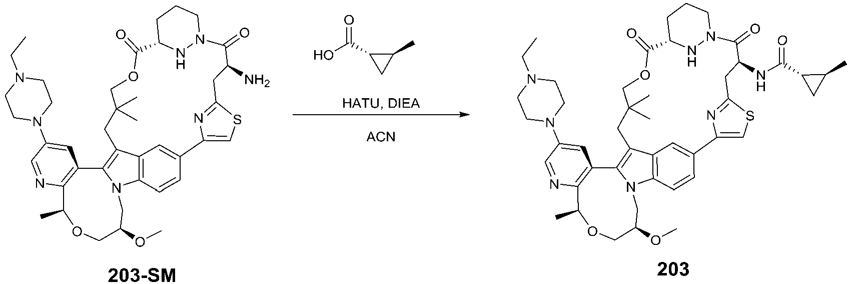

(1S,2S)-2-methylcyclopropane-1-carboxylic acid (12.47 mg, 0.12 mmol), HATU (78.94 mg, 0.21 mmol), DIEA (0.10 mL, 0.62 mmol), and acetonitrile (2.00 mL) were added sequentially to a 50 mL single-necked flask, and the mixture was stirred at room temperature for 15 min. The mixture was cooled in an ice-water bath, and then a solution of 203-SM (80.00 mg, 0.10 mmol) in acetonitrile (2.00 mL) was added. After the reaction was complete as monitored by LC-MS, the reaction solution was directly added to 30 mL of water to quench the reaction, then extracted three times with 20 mL of DCM, washed twice with 20 mL of saturated sodium chloride solution, dried over anhydrous sodium sulfate, filtered, and concentrated to dryness to obtain a crude product of the target product. The crude product was separated by column chromatography (DCM:MeOH = 97:3 to 90:10) to finally obtain the target compound 203 (76.2 mg). ESI-MS m/z: 853.45, [M+H]⁺. (203-SM was synthesized with reference to compound 161-6).

¹H NMR (500 MHz, DMSO-d₆) δ 8.55-8.48 (m, 2H), 8.44 (d, J = 2.8 Hz, 1H), 7.82 (s, 1H), 7.74 (dd, J = 8.6, 1.7 Hz, 1H), 7.55 (d, J = 8.6 Hz, 1H), 7.25 (s, 1H), 5.53 (t, J = 9.2 Hz, 1H), 5.05 (d, J = 12.2 Hz, 1H), 4.30-4.19 (m, 3H), 4.15 (q, J = 6.3 Hz, 1H), 4.12-4.03 (m, 2H), 3.71-3.56 (m, 8H), 3.52 (dd, J = 13.3, 10.2 Hz, 1H), 3.37 (s, 4H), 3.27 (s, 3H), 3.16 (dd, J = 13.6, 8.3 Hz, 7H), 3.01 (d, J = 14.4 Hz, 1H), 2.75 (dd, J = 11.3, 4.9 Hz, 1H), 2.42-2.35 (m, 2H), 2.12 (d, J = 12.1 Hz, 1H), 1.81 (s, 2H), 1.55-1.47 (m, 2H), 1.39 (d, J = 6.3 Hz, 3H), 1.0-0.93 (m, 5H), 0.88 (q, J = 3.8 Hz, 1H), 0.57 (dd, J = 8.0, 5.3 Hz, 1H), 0.31 (s, 3H).

### Example 204: Synthesis of compound (2S)-3-methyl-N-((1⁵S,6⁴S,4S,Z)-1⁵,10,10-trimethyl-1²-(4-methylpiperazin-1-yl)-5,7-dioxo-1⁸,1⁹ -dihydro-1⁵H,1⁷H-8-oxa-6²,6³-diaza-1(13,15)-pyrido[3',2':7,8][1,5]oxazonino[5,6-a]indola-2(4,2) -thiazola-6(2,4)-bicyclo[3.1.1]heptanacycloundecaphane-4-yl)-2-(1,3,3-trimethylureido)butanam ide

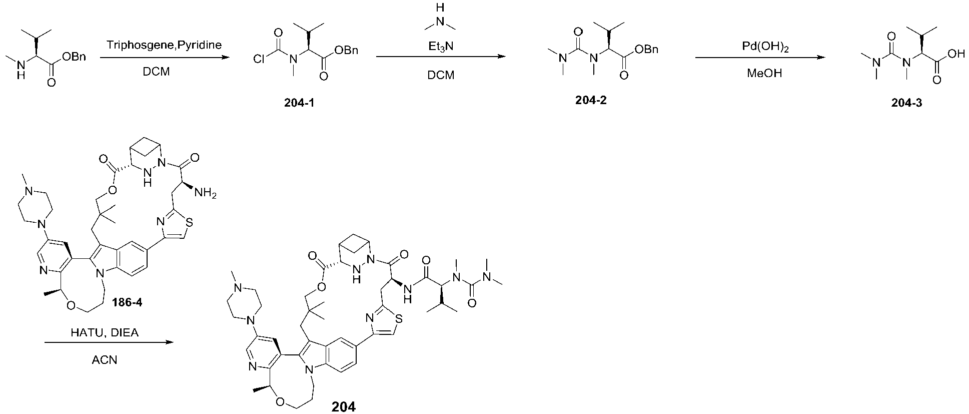

### Step 1: Synthesis of compound 204-1

Benzyl L-valinate (1.00 g, 4.52 mmol) and DCM (10.00 mL) were added sequentially to a 100 mL three-necked flask. Under nitrogen protection, with ice-water bath cooling, a mixed solution of triphosgene (0.54 g, 1.81 mmol), pyridine (1.07 g, 13.56 mmol), and DCM (10.00 mL) was slowly added, and the mixture was heated to room temperature and reacted for 3 h. After the reaction was complete as monitored by LC-MS, the reaction solution was concentrated to dryness to obtain the target product 204-1 (1.28 g, red liquid). ESI-MS m/z: 284.71 [M+H]⁺.

### Step 2: Synthesis of compound 204-2

Compound 204-1 (1.28 g, 4.51 mmol) and DIEA (1.75 g, 13.53 mmol) were added sequentially to a 100 mL single-necked flask. Under nitrogen protection, with ice-water bath cooling, dimethylamine (6.77 mL, 2M in THF, 13.53 mmol) was slowly added, and then the mixture was heated to room temperature and reacted for 1 h. After the reaction was complete as monitored by LC-MS, the reaction solution was slowly added to 60 mL of ice water to quench the reaction, then extracted three times with 50 mL of EA. The organic phases were combined, washed with saturated sodium chloride solution, dried over anhydrous sodium sulfate, and purified by silica gel column chromatography to obtain the target product 204-2 (0.75 g, light yellow liquid). ESI-MS m/z: 293.41 [M+H]⁺.

### Step 3: Synthesis of compound 204-3

Compound 204-2 (0.75 g, 2.57 mmol), Pd(OH)₂ (0.36 g, 2.57 mmol), and MeOH (15.00 mL) were added sequentially to a 100 mL single-necked flask. Under a hydrogen atmosphere, the reaction was carried out at room temperature for 2 h. After the reaction was complete as monitored by LC-MS, the reaction solution was directly filtered through celite, and the filter cake was washed three times with 10 mL of MeOH. The organic phase was rotary evaporated and purified by silica gel column chromatography to obtain the target product 204-3 (0.30 g, light yellow liquid). ESI-MS m/z: 203.27 [M+H]⁺.

### Step 4: Synthesis of compound 204

Compound 204-3 (10.68 mg, 0.05 mmol), 186-4 (30.00 mg, 0.04 mmol), N-methylimidazole (0.02 mL, 0.20 mmol), and ACN (15.00 mL) were added sequentially to a 25 mL single-necked flask. Then, under nitrogen protection and ice-water bath cooling, TCFH/N,N,N',N'-tetramethylchloroformamidinium hexafluorophosphate (17.09 mg, 0.06 mmol) was added. After the reaction was complete as monitored by LC-MS, the reaction solution was directly added to 30 mL of water to quench the reaction, then extracted three times with 20 mL of DCM, washed twice with 20 mL of saturated sodium chloride solution, dried over anhydrous sodium sulfate, filtered, and concentrated. The crude product was separated by column chromatography to obtain the target compound 204 (29.80 mg). ESI-MS m/z: 923.36 [M+H]⁺.

¹H NMR (500 MHz, DMSO-d₆) δ 8.47 (d, J = 8.7 Hz, 1H), 8.40 (t, J = 2.6 Hz, 2H), 7.83 (s, 1H), 7.71 (dd, J = 8.7, 1.7 Hz, 1H), 7.49 (d, J = 8.6 Hz, 1H), 7.21 (d, J = 2.9 Hz, 1H), 5.97 (d, J = 11.0 Hz, 1H), 5.30 (t, J = 8.2 Hz, 1H), 4.76 (d, J = 11.0 Hz, 1H), 4.51 (d, J = 5.2 Hz, 1H), 4.15 (d, J = 6.4 Hz, 1H), 4.02 (d, J = 11.7 Hz, 2H), 3.86 (d, J = 10.9 Hz, 1H), 3.67-3.53 (m, 3H), 3.26 (d, J = 5.4 Hz, 3H), 3.18-3.14 (m, 1H), 3.07 (d, J = 9.4 Hz, 1H), 3.02 (s, 3H), 2.89 (s, 2H), 2.82 (d, J = 5.1 Hz, 9H), 2.69-2.65 (m, 1H), 2.22 (s, 5H), 2.16 (t, J = 9.5 Hz, 2H), 1.81 (s, 2H), 1.64 (t, J = 9.4 Hz, 1H), 1.37 (d, J = 6.4 Hz, 3H), 1.11 (s, 1H), 0.91 (d, J = 6.6 Hz, 5H), 0.87 (dd, J = 6.7, 4.0 Hz, 5H).

Other compounds of the present invention can be prepared according to similar synthetic methods as described in the above examples.

### Biological Testing

### Pharmacological Experiment 1: Cell Proliferation Inhibition Assay (AGS)

KRAS-G12D mutant tumor cells AGS were seeded in low-attachment 96-well plates at a cell density of 1×10³ cells/well and cultured overnight in a cell culture incubator. After the cells adhered, the compounds for the assay were added to the 96-well plates at final concentrations of 10000, 2000, 400, 80, 16, 3.2, 0.64, 0.13, 0.03, 0 nM (final concentration of DMSO for all was 0.5%). After incubation at 37°C for 96 h, 50 µL of Cell-titer GLO working solution was added to each well, mixed by shaking, incubated at room temperature for 10 min, and the Luminescence value was read on a multi-function microplate reader. The luminescence data were calculated and converted into inhibition percent. The inhibition percent of cell proliferation was calculated according to the following formula: $Inhibition Percent = \left(Max Value-Measured Value\right) / \left(Max Value-Blank\right) \times 100 %$

("Max Value" comes from the 0.5% DMSO control well, "Blank" comes from the blank control well, and "Measured Value" comes from the compound-treated well).

Curve fitting was performed using GraphPad Prism software to obtain IC₅₀ values, see Table 1.

### Pharmacological Experiment 2: Cell Proliferation Inhibition Assay (SW620)

KRAS-G12V mutant tumor cells SW620 were seeded in low-attachment 96-well plates at a cell density of 1×10³ cells/well and cultured overnight in a cell culture incubator. After the cells adhered, the test compounds were added to the 96-well plates at final concentrations of 10000, 2000, 400, 80, 16, 3.2, 0.64, 0.13, 0.03, 0 nM (final concentration of DMSO was 0.5% for all). After incubation at 37°C for 96 h, 50 µL of Cell-titer GLO working solution was added to each well, mixed by shaking, incubated at room temperature for 10 min, and the Luminescence value was read on a multi-function microplate reader. The luminescence data were calculated and converted into inhibition percent. The inhibition percent of cell proliferation was calculated according to the following formula: $Inhibition Percent = \left(Max Value-Measured Value\right) / \left(Max Value-Blank\right) \times 100 %$

("Max Value" comes from the 0.5% DMSO control well, "Blank" comes from the blank control well, and "Measured Value" comes from the compound-treated well).

Curve fitting was performed using GraphPad Prism software to obtain IC₅₀ values, see Table 1.

### Pharmacological Experiment 3: Cellular KRAS G12D-cRAF Binding Inhibition Assay

NanoLuc^{®} luciferase was used as the energy donor, and NanoBRET^{™} 618 fluorescent ligand labeled with the HaloTag^{®} protein was used as the energy acceptor to detect protein-protein interactions in living cells.

AD293 stable cells transfected with KRAS G12D-NanoLuc^{®} luciferase and cRAF RBD-HaloTag^{®} tag protein were seeded in 96-well plates at a cell density of 1×10⁴ cells/well. Except for wells with medium added as a blank control, NanoBRET^{™} 618 fluorescent ligand was added to all other wells, and the plates were cultured overnight in a cell culture incubator. After the cells adhered, the test compounds were added to the 96-well plates at final concentrations of 1000 nM, 250 nM, 62.5 nM, 15.6 nM, 3.9 nM, 0.98 nM, 0.24 nM, and 0.1% DMSO. 0.1% DMSO was added to the blank control wells. After incubation for 3 h, NanoBRET^{™} Nano-Glo^{®} substrate was added to the 96-well plates, mixed by shaking, incubated at room temperature for 10 min, and the luminescence value was read on a multi-function microplate reader. The luminescence data were calculated and converted into inhibition percent. The inhibition percent of KRAS G12D-cRAF binding was calculated according to the following formula: $Inhibition Percent = \left(Max Value-Measured Value\right) / \left(Max Value-Blank\right) \times 100 %$

("Max" comes from the 0.1% DMSO control well, "Blank" comes from the blank control well, and "Measured Value" comes from the compound-treated well).

Curve fitting was performed using GraphPad Prism software to obtain IC₅₀ values, see Table 1.

### Pharmacological Experiment 4: Cellular KRAS G12V-cRAF Binding Inhibition Assay

NanoLuc^{®} luciferase was used as the energy donor, and NanoBRET^{™} 618 fluorescent ligand labeled with the HaloTag^{®} protein was used as the energy acceptor to detect protein-protein interactions in living cells.

AD293 stable cells transfected with KRAS G12V-NanoLuc^{®} luciferase and cRAF RBD-HaloTag^{®} tag protein were seeded in 96-well plates at a cell density of 1×10⁴ cells/well. Except for wells with medium added as a blank control, NanoBRET^{™} 618 fluorescent ligand was added to all other wells, and the plates were cultured overnight in a cell culture incubator. After the cells adhered, the test compounds were added to the 96-well plates at final concentrations of 1000 nM, 250 nM, 62.5 nM, 15.6 nM, 3.9 nM, 0.98 nM, 0.24 nM, and 0.1% DMSO. 0.1% DMSO was added to the blank control wells. After incubation for 3 h, NanoBRET^{™} Nano-Glo^{®} substrate was added to the 96-well plates, mixed by shaking, incubated at room temperature for 10 min, and the luminescence value was read on a multi-function microplate reader. The luminescence data were calculated and converted into inhibition percent. The inhibition percent of KRAS G12V-cRAF binding was calculated according to the following formula: $Inhibition Percent = \left(Max Value-Measured Value\right) / \left(Max Value-Blank\right) \times 100 %$

("Max Value" comes from the 0.1% DMSO control well, "Blank" comes from the blank control well, and "Measured Value" comes from the compound-treated well).

Curve fitting was performed using GraphPad Prism software to obtain IC₅₀ values, see Table 1.

**Table 1**

| Example No. | AGS(G12D) (nM) | SW620(G12V) (nM) | NanoBRET KRAS G12D (nM) | NanoBRET KRAS G12V (nM) |
|---|---|---|---|---|
| 1 | 7.86 | 0.54 | 6.1 | 29.9 |
| 2 | 8.7 | 1.56 | 29.1 | 205.6 |
| 4 | 51.9 | 6.2 | 155.5 | 650.5 |
| 5 | 6.92 | 1.08 | 25.91 | 78.79 |
| 21 | 2.19 | 1.46 | 3.95 | 15.13 |
| 22 | 2.62 | 1.03 | 6.6 | 22.86 |
| 37 | 4.28 | 1.02 | 5.32 | 22.37 |
| 38 | 4.2 | 1.72 | 8.6 | 31.86 |
| 39 | 33.72 | 8.17 | 22.13 | 86.4 |
| 40 | 22.36 | 4.29 | 15.75 | 61.1 |
| 80 | 1.76 | 0.30 | 5.7 | 21.3 |
| 81 | 0.79 | 0.11 | 3.14 | 9.6 |
| 83 | 2.04 | 0.33 | 2.88 | 11.9 |
| 84 | 1.15 | 0.19 | 2.76 | 9.17 |
| 85 | 3.72 | 0.63 | 5.56 | 19.88 |
| 86 | 1.19 | 0.17 | 2.19 | 10.98 |
| 87 | 8.3 | 2.75 | 38.8 | 183.8 |
| 88 | 1.1 | 0.32 | 5.9 | 20.4 |
| 91 | 1.51 | 0.21 | 2.7 | 10.29 |
| 92 | 1 | 0.33 | 3.7 | 12.7 |
| 94 | 2.68 | 0.34 | 6.55 | 22.75 |
| 96 | 2.4 | 1.07 | 5.99 | 19.81 |
| 97 | 2.69 | 0.42 | 11.93 | 61.82 |
| 98 | 13.24 | 3.87 | 14.8 | 61.7 |
| 99 | 0.74 | 0.14 | 4.25 | 13.32 |
| 100 | 0.44 | 0.1 | 4.55 | 21.7 |
| 101 | 1.4 | 0.26 | 2.74 | 12.01 |
| 102 | 0.38 | 0.17 | 4.13 | 14.7 |
| 103 | 0.85 | 0.21 | 7.74 | 22.16 |
| 104 | 0.63 | 0.2 | 3.2 | 11.79 |
| 105 | 0.69 | 0.24 | 4.55 | 16.63 |
| 106 | 0.86 | 0.24 | 6.39 | 27.4 |
| 107 | 0.93 | 0.45 | 3.7 | 19.9 |
| 108 | 0.58 | 0.28 | 6.1 | 37.9 |
| 109 | 1.02 | 0.2 | 6.2 | 14.87 |
| 110 | 1.12 | 0.15 | 4.17 | 14.02 |
| 112 | 2.68 | 0.34 | 8.39 | 34.23 |
| 113 | 3.6 | 0.54 | 7.1 | 23.88 |
| 114 | 15.7 | 3.4 | 19.4 | 65.75 |
| 115 | 1.72 | 0.9 | 4.28 | 16.33 |
| 116 | 8.27 | 4.1 | 9.57 | 37.77 |
| 117 | 0.86 | 0.21 | 1.8 | 8.1 |
| 118 | 3.77 | 1.5 | 7.2 | 30.56 |
| 124 | 4.67 | 1.45 | 8.38 | 17.98 |
| 125 | 2.8 | 0.6 | 4.55 | 14.67 |
| 127 | 4.5 | 1.2 | 4.51 | 17.68 |
| 128 | 1.28 | 0.29 | 2.5 | 11.4 |
| 130 | 1.8 | 0.85 | 3.4 | 16.3 |
| 131 | 1.31 | 0.51 | 2.57 | 11.04 |
| 132 | 1.27 | 0.34 | 1.37 | 5.38 |
| 133 | 1.25 | 0.79 | 9.2 | 40.9 |
| 134 | 1.7 | 1.44 | 13.7 | 59 |
| 135 | 2.2 | 0.7 | 2.39 | 10.62 |
| 136 | 5.6 | 1.5 | 4.59 | 19.61 |
| 137 | 0.79 | 0.21 | 1.11 | 5.86 |
| 138 | 1.59 | 0.63 | 3.2 | 14.7 |
| 140 | 0.38 | 0.14 | 1.8 | 9.3 |
| 142 | 0.3 | 0.09 | 1.88 | 5.61 |
| 146 | 1.4 | 0.6 | 1.97 | 8.52 |
| 149 | 3.19 | 1.09 | 3.48 | 10.94 |
| 150 | 0.93 | 0.16 | 1.61 | 8.1 |
| 151 | / | / | 3.29 | 24.27 |
| 153 | 1.33 | 0.28 | / | / |
| 154 | 1.95 | 0.91 | 4.2 | 19.3 |
| 155 | 1.38 | 0.62 | / | / |
| 156 | 8.27 | / | / | / |
| 157 | 3.31 | 0.58 | / | / |
| 158 | 0.58 | 0.11 | 1.01 | 5.06 |
| 159 | 0.21 | 0.06 | 0.38 | 3.83 |
| 160 | 2.25 | 0.98 | / | / |
| 161 | 0.71 | 0.24 | 0.92 | 4.52 |
| 162 | 1.08 | / | / | / |
| 163 | 1.44 | 0.2 | / | / |
| 165 | 0.19 | 0.05 | 0.76 | 3.8 |
| 166 | 0.36 | 0.14 | / | / |
| 171 | 0.11 | 0.04 | 0.24 | 3.04 |
| 172 | 1.1 | 0.32 | 2.02 | 6.7 |
| 183 | 2.0 | 0.6 | 2.7 | 17.6 |
| 184 | 0.9 | 0.65 | / | / |
| 186 | 0.42 | 0.15 | 0.8 | 5.4 |
| 191 | 0.45 | 0.28 | / | / |
| 192 | 1.5 | 0.9 | 4.3 | 16.3 |
| 193 | 0.9 | 0.2 | 1.2 | 27.1 |
| 194 | 3.0 | 1.4 | / | / |
| 195 | 0.6 | 0.06 | / | / |
| 197 | 0.03 | 0.01 | 0.3 | 2.0 |
| 198 | 0.37 | 0.09 | 2.0 | 8.4 |
| 199 | 39 | 4.1 | / | / |
| 200 | 3.9 | 1.0 | 13.8 | 37.6 |
| 203 | 2.1 | 3.3 | / | / |
| 204 | 0.4 | 0.2 | / | / |

### Pharmacological Experiment 5: PK Study of Compounds in Mice

The test compound was mixed with the vehicle (10% DMSO/5% Solutol/85% physiological saline), vortexed and sonicated to prepare a clear 0.2 mg/mL intravenous injection solution. The test compound was mixed with the vehicle (20% PEG400/10% Solutol/70% pH 4.5 acetate buffer), vortexed and sonicated to prepare a clear 1 mg/mL oral solution. Female ICR mice aged 6 to 7 weeks were selected and administered the test compound via intravenous (IV) and oral (PO) routes, respectively. Whole blood samples were collected at 5 min, 15 min, 30 min, 1 h, 2 h, 4 h, 7 h, and 24 h for the intravenous route, and at 15 min, 30 min, 1 h, 2 h, 4 h, 7 h, and 24 h for the oral route. The administration doses and experimental protocols are shown in Table 2 below. Drug concentrations were analyzed by LC-MS/MS method, and pharmacokinetic parameters were calculated using Phoenix WinNonlin software (Pharsight, USA). The PK results are shown in Table 3.

**Table 2**

| Group | Number | Test | Administr | Dose | Administr | Administr | Vehicle |
|---|---|---|---|---|---|---|---|
| | of Animals | Compound | ation Route | (mg/kg) | ation Volume (ml/kg) | ation Concentration (mg/ml) | |
| G1 | 3 | 186 | IV | 1 | 5 | 0.2 | 10% DMSO/5% Solutol/85% Physiological Saline |
| G2 | 3 | | PO | 10 | 10 | 1 | 20% PEG400/10% Solutol/70% pH 4.5 Acetate Buffer |
| G3 | 3 | 198 | IV | 1 | 5 | 0.2 | 10% DMSO/5% Solutol/85% Physiological Saline |
| G4 | 3 | | PO | 10 | 10 | 1 | 20% PEG400/10% Solutol/70% pH4.5 Acetate Buffer |

**Table 3**

| PK parameters (Mean) | IV 1 mg/kg (n=3) | PO 10 mg/kg (n=3) | IV 1 mg/kg (n=3) | PO 10 mg/kg (n=3) |
|---|---|---|---|---|
| | Compound 186 | | Compound 198 | |
| T_{1/2}(h) | 9.31 | NA | 8.9 | NA |
| Tₘₐₓ (h) | / | 4 | / | 2 |
| C₀/Cₘₐₓ (ng/mL) | 571 | 679 | 462 | 1300 |
| AUC₀₋ₜ (h*ng/mL) | 3258 | 10138 | 2453 | 13249 |
| Vₛₛ (L/kg) | 3.09 | / | 4.66 | / |
| CL(mL/min/kg) | 4.45 | / | 5.75 | / |
| F | / | 31.1 | / | 54.0 |

### Pharmacological Experiment 6: PK Study of Compounds in Rats

The test compound was mixed with the vehicle (10% DMSO/5% Solutol/85% physiological saline), vortexed and sonicated to prepare a clear 0.5 mg/mL intravenous injection solution. The test compound was mixed with the vehicle (20% PEG400/10% Solutol/70% pH 4.5 acetate buffer), vortexed and sonicated to prepare a clear 1 mg/mL oral solution. Male SD rats aged 6 to 7 weeks were selected and administered the test compound via intravenous (IV) and oral (PO) routes, respectively. Whole blood samples were collected at 5 min, 15 min, 30 min, 1 h, 2 h, 4 h, 7 h, and 24 h for the intravenous route, and at 15 min, 30 min, 1 h, 2 h, 4 h, 7 h, and 24 h for the oral route. The administration doses and experimental protocols are shown in Table 4 below. Drug concentrations were analyzed by LC-MS/MS method, and pharmacokinetic parameters were calculated using Phoenix WinNonlin software (Pharsight, USA). The PK results are shown in Table 5.

**Table 4**

| Group | Number of Animals | Test Compound | Administration Route | Dose (mg/kg) | Administration Volume (ml/kg) | Administration Concentration (mg/ml) | Vehicle |
|---|---|---|---|---|---|---|---|
| G1 | 3 | 186 | IV | 1 | 2 | 0.5 | 10%DMSO/5% Solutol/85% Physiological Saline |
| G2 | 3 | | PO | 10 | 10 | 1 | 20% PEG400/10% Solutol/70% pH4.5Acetate Buffer |
| G3 | 3 | 184 | IV | 1 | 2 | 0.5 | 10%DMSO/5% Solutol/85% Physiological Saline |
| G4 | 3 | | PO | 10 | 10 | 1 | 20% PEG400/10% Solutol/70% pH4.5Acetate Buffer |
| G5 | 3 | 192 | IV | 1 | 2 | 0.5 | 10%DMSO/5% Solutol/85% Physiological Saline |
| G6 | 3 | | PO | 10 | 10 | 1 | 20% PEG400/10% Solutol/70% pH4.5Acetate Buffer |
| G7 | 3 | 198 | IV | 1 | 2 | 0.5 | 10%DMSO/5% Solutol/85% Physiological Saline |
| G8 | 3 | | PO | 10 | 10 | 1 | 20% PEG400/10% Solutol/70% pH4.5Acetate Buffer |

**Table 5**

| PK parameters (Mean) | IV 1 mg/kg (n=3) | | | | PO 10 mg/kg (n=3) | | | |
|---|---|---|---|---|---|---|---|---|
| | Compound 186 | Compound 184 | Compound 192 | Compound 198 | Compound 186 | Compound 184 | Compound 192 | Compound 198 |
| T_{1/2} (h) | 8.40 | 9.07 | 4.93 | 7.22 | NA | NA | 5.13 | NA |
| Tₘₐₓ (h) * | / | / | / | / | 4.00 | 3.33 | 3.33 | 3.33 |
| C₀/Cₘₐₓ (ng/mL) | 4246 | 2210 | 4221 | 6059 | 1367 | 796 | 472 | 2060 |
| AUC₀₋ₜ (h*ng/mL) | 7507 | 5558 | 4505 | 6940 | 14624 | 10910 | 5350 | 22484 |
| Vₛₛ (L/kg) | 1.20 | 1.71 | 1.11 | 1.08 | / | / | / | / |
| CL(mL/min/kg) | 1.93 | 2.56 | 3.59 | 2.22 | / | / | / | / |
| F | / | / | / | / | 19.5 | 19.6 | 11.9 | 32.4 |

### Pharmacological Experiment 6: PK Study of Compounds in Dogs

The test compound was mixed with the vehicle (10% DMSO/5% Solutol/85% physiological saline), vortexed and sonicated to prepare a clear 1 mg/mL intravenous injection solution. The test compound was mixed with the vehicle (10% DMSO + 5% HS15 + 85% 10 mM aqueous citric acid solution), vortexed and sonicated to prepare a clear 0.6 mg/mL oral solution. Beagle dogs (two males, one female) were selected and administered the test compound via intravenous (IV) and oral (PO) routes, respectively. Whole blood samples were collected at 0 min, 5 min, 15 min, 30 min, 1 h, 2 h, 4 h, 6 h, 8 h, 12 h, 24 h, 48 h, and 72 h for the intravenous route, and at 0 min, 15 min, 30 min, 1 h, 2 h, 4 h, 6 h, 8 h, 12 h, 24 h, 48 h, and 72 h for the oral route. The administration doses and experimental protocols are shown in Table 6 below. Drug concentrations were analyzed by LC-MS/MS method, and pharmacokinetic parameters were calculated using Phoenix WinNonlin software (Pharsight, USA). The results are shown in Table 7.

**Table 6**

| Group | Number of Animals | Test Compound | Administration Route | Dose (mg/kg) | Administration Volume (ml/kg) | Administration Concentration (mg/ml) | Vehicle |
|---|---|---|---|---|---|---|---|
| G1 | 3 | 186 | IV | 1 | 1 | 1 | 10% DMSO/5% Solutol/ 85% Physiological Saline |
| G2 | 3 | | PO | 3 | 5 | 0.6 | 10%DMSO+5%HS15+ 85% 10mMaqueous citric acid solution |

**Table 7**

| PK parameters (Mean) | IV 1 mg/kg (n=3) | PO 3 mg/kg (n=3) |
|---|---|---|
| | Compound 186 | |
| T_{1/2} (h) | 34.8 | NA |
| Tₘₐₓ (h) | / | 1.17 |
| C₀/Cₘₐₓ (ng/mL) | 2015 | 608 |
| AUC₀₋ₜ (h*ng/mL) | 16625 | 20528 |
| Vₛₛ (L/kg) | 2.62 | / |
| CL (mL/min/kg) | 0.659 | / |
| F | / | 41.2 |

Although the present invention has been fully described through its embodiments, it is important to note that various changes and modifications will be apparent to those skilled in the art. Such changes and modifications should all be included within the scope of the appended claims of the present invention.

## Claims

1. A compound represented by formula (I), or a stereoisomer, a tautomer, a deuterated compound or a pharmaceutically acceptable salt thereof: 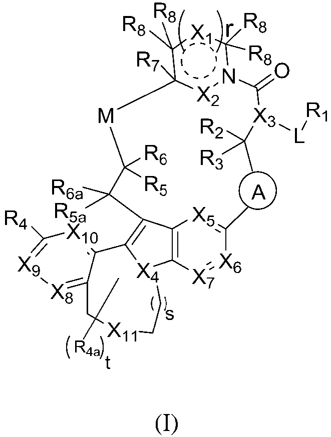 wherein, 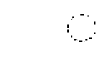 represents that a bond in a ring is a single bond or a double bond;
each X₁ is the same or different, and each X₁ is independently selected from the group consisting of CR₁₀, CR₁₀R₁₀, N, NR₁₀, O, S and S(O)₁₋₂;
X₂ is selected from the group consisting of O, S, S(O)₁₋₂, N and NR₁₀;
X₃ is selected from the group consisting of N and CR₁₀
X₄ is selected from the group consisting of CR₁₀ and N;
X₅ is selected from the group consisting of CR₁₁ and N;
X₆ is selected from the group consisting of CR₁₂ and N;
X₇ is selected from the group consisting of CR₁₃ and N;
X₈ is selected from the group consisting of CR₁₄ and N;
X₉ is selected from the group consisting of CR₁₅ and N;
X₁₀ is selected from the group consisting of CR₁₆ and N;
X₁₁ is selected from the group consisting of C(R₁₇)₂, O and NR₁₇;
L is selected from the group consisting of 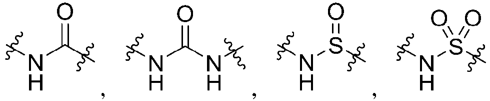 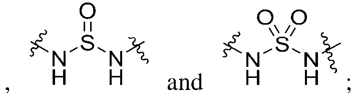
ring A is selected from the group consisting of C₅₋₁₄ cycloalkyl, 5- to 14-membered heterocyclyl, C₆₋₁₈ aryl and 5- to 18-membered heteroaryl; wherein the C₅₋₁₄ cycloalkyl, 5- to 14-membered heterocyclyl, C₆₋₁₈ aryl or 5- to 18-membered heteroaryl is optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₁ is selected from the group consisting of H, hydroxyl, C₁₋₆ hydroxyalkyl, amino, cyano, halogen, C₁₋₆ alkyl, C₁₋₆ haloalkyl, C₁₋₆ alkoxy, C₁₋₆ haloalkoxy, -C₀₋₃ alkylene-N(R_{c})₂, -C₀₋₃ alkylene-C₂₋₆ alkenyl, -C₀₋₃ alkylene-C₂₋₆ alkynyl, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl and -C₀₋₃ alkylene-5- to 18-membered heteroaryl; wherein the hydroxyl, C₁₋₆ hydroxyalkyl, amino, C₁₋₆ alkyl, C₁₋₆ haloalkyl, C₁₋₆ alkoxy, C₁₋₆ haloalkoxy, -C₀₋₃ alkylene-N(R_{c})₂, -C₀₋₃ alkylene-C₂₋₆ alkenyl, -C₀₋₃ alkylene-C₂₋₆ alkynyl, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl or -C₀₋₃ alkylene-5- to 18-membered heteroaryl is optionally further substituted by one or more Rₐ, or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₂ is selected from the group consisting of H, hydroxyl, halogen, C₁₋₆ alkyl, C₁₋₆ haloalkyl, C₁₋₆ alkoxy and C₁₋₆ haloalkoxy;
R₃ is selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; or,
R₂ and R₃ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b}; or two R_{b} on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₄ is selected from the group consisting of H, hydroxyl, C₁₋₆ hydroxyalkyl, amino, cyano, halogen, C₁₋₆ alkyl, C₁₋₆ haloalkyl, C₁₋₆ alkoxy, C₁₋₆ haloalkoxy, C₂₋₆ alkenyl, C₂₋₆ alkynyl, -C₀₋₃ alkylene-N(R_{c})₂, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl and -C₀₋₃ alkylene-5- to 18-membered heteroaryl; wherein the hydroxyl, C₁₋₆ hydroxyalkyl, amino, C₁₋₆ alkyl, C₁₋₆ haloalkyl, C₁₋₆ alkoxy, C₁₋₆ haloalkoxy, C₂₋₆ alkenyl, C₂₋₆ alkynyl, -C₀₋₃ alkylene-N(R_{c})₂, -C₀₋₃ alkylene-C₃₋₁₄ cycloalkyl, -C₀₋₃ alkylene-3- to 14-membered heterocyclyl, -C₀₋₃ alkylene-C₆₋₁₈ aryl or -C₀₋₃ alkylene-5- to 18-membered heteroaryl is optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b};
R₄ₐ is selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; wherein the amino, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl or C₁₋₆ hydroxyalkyl is optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b}; or,
two R₄ₐ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b};
R₅ is selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl;
R₆ is selected from the group consisting of H, halogen, hydroxyl, cyano, C₁₋₃ alkoxy, C₁₋₃ alkyl, C₂₋₆ alkenyl, C₂₋₆ alkynyl, C₃₋₈ cycloalkyl, 3- to 14-membered heterocyclyl, 5- to 10-membered heteroaryl and C₆₋₁₀ aryl; wherein the hydroxyl, C₁₋₃ alkoxy, C₁₋₃ alkyl, C₂₋₆ alkenyl, C₂₋₆ alkynyl, C₃₋₈ cycloalkyl, 3- to 14-membered heterocyclyl, 5- to 10-membered heteroaryl or C₆₋₁₀ aryl is optionally further substituted by one or more Rₐ; or,
R₅ and R₆ together with the atom to which they are attached form carbonyl, C₃₋₈ cycloalkyl or 3- to 14-membered heterocyclyl; wherein the C₃₋₈ cycloalkyl or 3- to 14-membered heterocyclyl is optionally further substituted by one or more Rₐ;
R₅ₐ and R₆ₐ are each independently selected from the group consisting of H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; or,
R₅ₐ and R₆ₐ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b};
R₇ is selected from the group consisting of absent, H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl, C₁₋₆ hydroxyalkyl, C₃₋₈ cycloalkyl, 3- to 14-membered heterocyclyl and 5-to 10-membered heteroaryl;
R₈ is selected from the group consisting of absent, H, halogen, cyano, amino, nitro, C₁₋₆ alkyl, C₁₋₆ alkoxy, C₃₋₁₄ cycloalkyl-O-, 3- to 14-membered heterocyclyl-O-, C₁₋₆ haloalkyl, C₁₋₆ haloalkoxy, hydroxyl and C₁₋₆ hydroxyalkyl; or,
R₇ and R₈ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
two R₈ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b};
M is selected from the group consisting of C₁₋₆ alkylene, C₂₋₆ alkenylene, C₂₋₆ alkynylene, C₁₋₆ heteroalkylene, -C(O)O-CH(R₉)-, -C(O)NH-CH(R₉)- and 5- to 8-membered heteroarylene; R₉ is selected from the group consisting of hydrogen and methyl;
when M is -C(O)O-CH(R₉)- or -C(O)NH-CH(R₉)-, CH(R₉) therein is bonded to -C(R₅R₆)-; or,
R₆ and R₉ together with the atom to which they are attached form C₃₋₈ cycloalkyl or 3- to 14-membered heterocyclyl; wherein the C₃₋₈ cycloalkyl or 3- to 14-membered heterocyclyl is optionally further substituted by one or more R_{b};
R₁₀ is selected from the group consisting of H, halogen, cyano, C₁₋₆ alkyl, C₁₋₆ alkoxy, C₁₋₆ haloalkoxy, C₃₋₆ cycloalkyl, 3- to 8-membered heterocyclyl and C₁₋₆ haloalkyl; or,
R₁₀ and R₈ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
two R₁₀ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b};
R₁₁, R₁₂, R₁₃, R₁₄, R₁₅, R₁₆, R₁₇ are each independently selected from the group consisting of H, halogen, C₁₋₆ alkyl, C₁₋₆ alkoxy, C₁₋₆ haloalkoxy and C₁₋₆ haloalkyl; or,
two R₁₇ together with the atom to which they are attached form carbonyl, C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
R₁₃ and R₄ₐ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
R₁₆ and R₅ₐ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
R₁₇ and R₄ₐ together with the atom to which they are attached form C₃₋₈ cycloalkyl or 3- to 8-membered heterocyclyl; wherein the C₃₋₈ cycloalkyl or 3- to 8-membered heterocyclyl is optionally further substituted by one or more R_{b}; or,
any two of R₁₄, R₁₅, R₁₆, R₄ and R₄ₐ together with the atom to which they are attached form C₃₋₈ cycloalkyl, 3- to 8-membered heterocyclyl, C₆₋₁₀ aryl or 5- to 10-membered heteroaryl; wherein the C₃₋₈ cycloalkyl, 3- to 8-membered heterocyclyl, C₆₋₁₀ aryl or 5- to 10-membered heteroaryl is optionally further substituted by one or more R_{b};
Rₐ is independently selected from the group consisting of absent, H, hydroxyl, oxo, amino, imino, cyano, halogen, C₁₋₆ alkyl, C₁₋₆ haloalkyl, C₁₋₆ alkoxy, C₁₋₆ haloalkoxy, -C₀₋₃ alkylene-OR_{b}, -C₀₋₃ alkylene-SR_{b}, -C₀₋₃ alkylene-N(R_{b})₂, -C₀₋₃ alkylene-S(O)₁₋₂R_{b}, -C₀₋₃ alkylene-S(R_{b})₅, =C(R_{b})₂, C₂₋₆ alkenyl, C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl and -C₀₋₃ alkylene-(5- to 18-membered heteroaryl); wherein the amino, imino, C₁₋₆ alkyl, C₁₋₆ haloalkyl, C₁₋₆ alkoxy, C₁₋₆ haloalkoxy, C₂₋₆ alkenyl, C₂₋₆ alkynyl, -C₀₋₃ alkylene-, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl or -C₀₋₃ alkylene-(5- to 18-membered heteroaryl) is optionally further substituted by one or more R_{b};
each R_{b} is independently selected from the group consisting of H, halogen, hydroxyl, oxo, amino, cyano, C₁₋₆ alkyl, C₁₋₆ alkoxy, C₃₋₆ cycloalkyl, 3- to 8-membered heterocyclyl, C₁₋₆ haloalkoxy and C₁₋₆ haloalkyl; wherein the amino, C₁₋₆ alkyl, C₁₋₆ alkoxy, C₃₋₆ cycloalkyl, 3- to 8-membered heterocyclyl, C₁₋₆ haloalkoxy or C₁₋₆ haloalkyl is optionally further substituted by one or more H, halogen, hydroxyl, cyano, C₁₋₆ alkyl, C₁₋₆ haloalkyl, C₁₋₆ alkoxy, C₁₋₆ haloalkoxy, amino, -NHC₁₋₃ alkyl or -N(C₁₋₃ alkyl)₂;
each R_{c} is independently selected from the group consisting of H, hydroxyl, halogen, C₁₋₆ alkyl, C₃₋₆ cycloalkyl, 3- to 8-membered heterocyclyl and C₁₋₆ haloalkyl;
r is 1 to 3; preferably 1, 2 or 3;
s is 1 to 3, preferably 1, 2 or 3; and
t is 0 to 4, preferably 0, 1, 2, 3 or 4, more preferably 0 to 3.

2. The compound, or the stereoisomer, the tautomer, the deuterated compound or the pharmaceutically acceptable salt thereof according to claim 1, wherein formula (I) is formula (I-1): 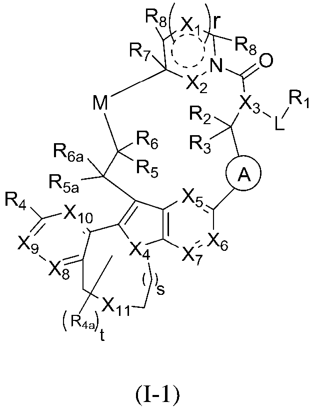 wherein, R₁, R₂, R₃, R₄, R₅, R₆, R₇, R₈, R₄ₐ, R₅ₐ, R₆ₐ, X₁, X₂, X₃, X₄, X₅, X₆, X₇, X₈, X₉, X₁₀, X₁₁, L, M, ring A, r, s, t are as defined in claim 1.

3. The compound, or the stereoisomer, the tautomer, the deuterated compound or the pharmaceutically acceptable salt thereof according to claim 1 or 2, wherein formula (I) is selected from the group consisting of formula (II-1) and formula (II-2): 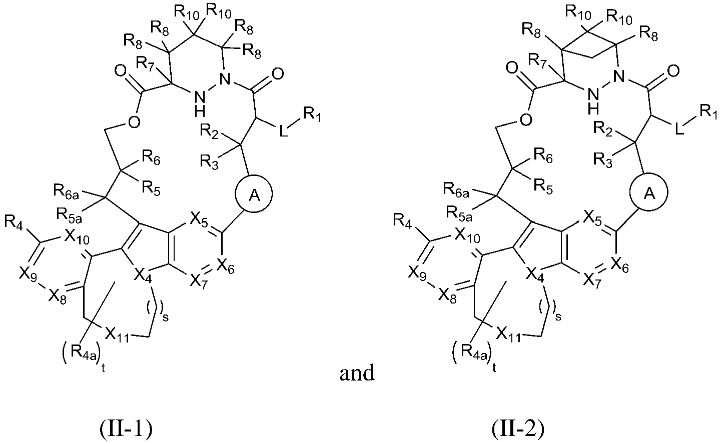 wherein, R₁, R₂, R₃, R₄, R₅, R₆, R₇, R₈, R₁₀, R₄ₐ, R₅ₐ, R₆ₐ, X₄, X₅, X₆, X₇, X₈, X₉, X₁₀, X₁₁, L, ring A, s, t are as defined in any one of claims 1 to 2.

4. The compound, or the stereoisomer, the tautomer, the deuterated compound or the pharmaceutically acceptable salt thereof according to any one of claims 1 to 3, wherein formula (I) is selected from the group consisting of formula (II-1a) and formula (II-2a): 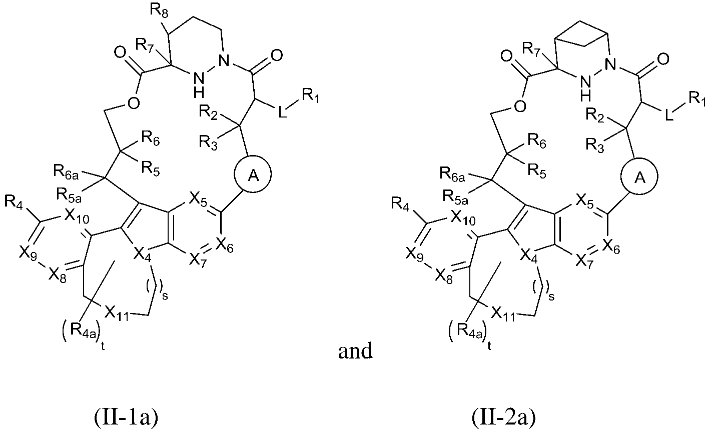 wherein, R₁, R₂, R₃, R₄, R₅, R₆, R₇, R₈, R₄ₐ, R₅ₐ, R₆ₐ, X₄, X₅, X₆, X₇, X₈, X₉, X₁₀, X₁₁, L, ring A, s, t are as defined in any one of claims 1 to 3.

5. The compound, or the stereoisomer, the tautomer, the deuterated compound or the pharmaceutically acceptable salt thereof according to any one of claims 1 to 4, wherein formula (I) is selected from the group consisting of formulas (IIIa) to (IIIc) and (IIIa') to (IIIc'): 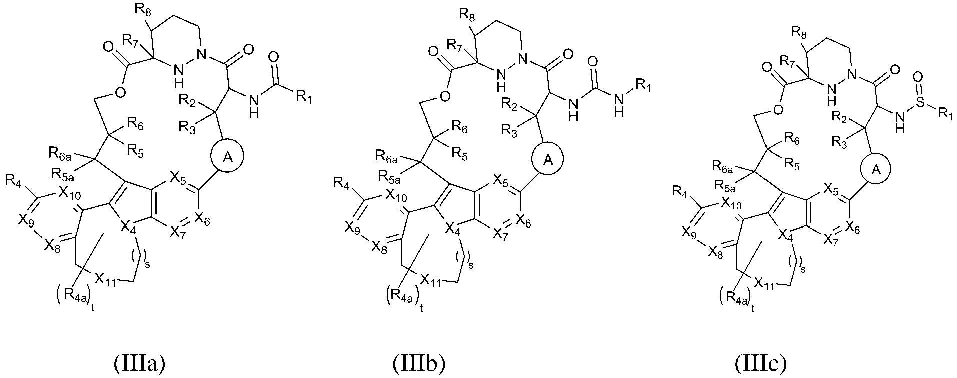 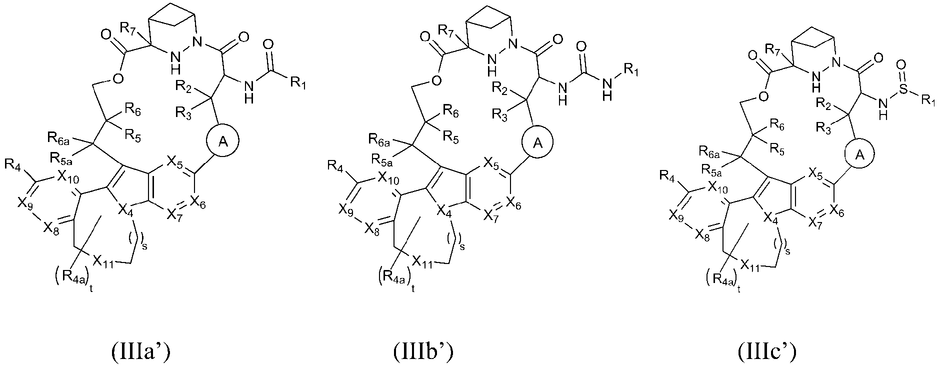 wherein, R₁, R₂, R₃, R₄, R₅, R₆, R₇, R₈, R₄ₐ, R₅ₐ, R₆ₐ, X₄, X₅, X₆, X₇, X₈, X₉, X₁₀, X₁₁, ring A, s, t are as defined in any one of claims 1 to 4.

6. The compound, or the stereoisomer, the tautomer, the deuterated compound or the pharmaceutically acceptable salt thereof according to any one of claims 1 to 5, wherein ring A is selected from the group consisting of C₆₋₁₈ aryl and 5- to 18-membered heteroaryl; wherein the C₆₋₁₈ aryl or 5- to 18-membered heteroaryl is optionally further substituted by one or more Rₐ, wherein Rₐ is selected from the group consisting of H, hydroxyl, cyano, halogen, C₁₋₆ alkyl, C₁₋₆ haloalkyl, C₁₋₆ alkoxy and C₁₋₆ haloalkoxy, wherein the C₁₋₆ alkyl, C₁₋₆ haloalkyl, C₁₋₆ alkoxy or C₁₋₆ haloalkoxy is optionally further substituted by one or more R_{b}, wherein R_{b} is selected from the group consisting of H, halogen and C₁₋₆ alkyl.

7. The compound, or the stereoisomer, the tautomer, the deuterated compound or the pharmaceutically acceptable salt thereof according to claim 6, wherein the C₆₋₁₈ aryl is phenyl.

8. The compound, or the stereoisomer, the tautomer, the deuterated compound or the pharmaceutically acceptable salt thereof according to claim 6, wherein the 5- to 18-membered heteroaryl is selected from the group consisting of thiazolyl and oxazolyl.

9. The compound, or the stereoisomer, the tautomer, the deuterated compound or the pharmaceutically acceptable salt thereof according to any one of claims 1 to 8, wherein X₄ is N.

10. The compound, or the stereoisomer, the tautomer, the deuterated compound or the pharmaceutically acceptable salt thereof according to any one of claims 1 to 9, wherein X₅ is selected from the group consisting of CH and N.

11. The compound, or the stereoisomer, the tautomer, the deuterated compound or the pharmaceutically acceptable salt thereof according to any one of claims 1 to 10, wherein X₆ is selected from the group consisting of CH and N.

12. The compound, or the stereoisomer, the tautomer, the deuterated compound or the pharmaceutically acceptable salt thereof according to any one of claims 1 to 11, wherein X₇ is selected from the group consisting of CH and N.

13. The compound, or the stereoisomer, the tautomer, the deuterated compound or the pharmaceutically acceptable salt thereof according to any one of claims 1 to 12, wherein X₈ is selected from the group consisting of CH and N.

14. The compound, or the stereoisomer, the tautomer, the deuterated compound or the pharmaceutically acceptable salt thereof according to any one of claims 1 to 13, wherein X₉ is selected from the group consisting of CH and N.

15. The compound, or the stereoisomer, the tautomer, the deuterated compound or the pharmaceutically acceptable salt thereof according to any one of claims 1 to 14, wherein X₁₀ is CH.

16. The compound, or the stereoisomer, the tautomer, the deuterated compound or the pharmaceutically acceptable salt thereof according to any one of claims 1 to 15, wherein X₁₁ is O.

17. The compound, or the stereoisomer, the tautomer, the deuterated compound or the pharmaceutically acceptable salt thereof according to any one of claims 1 to 16, wherein formula (I) is selected from the group consisting of formulas (IVa) to (IVc) and formulas (IVa') to (IVc'): 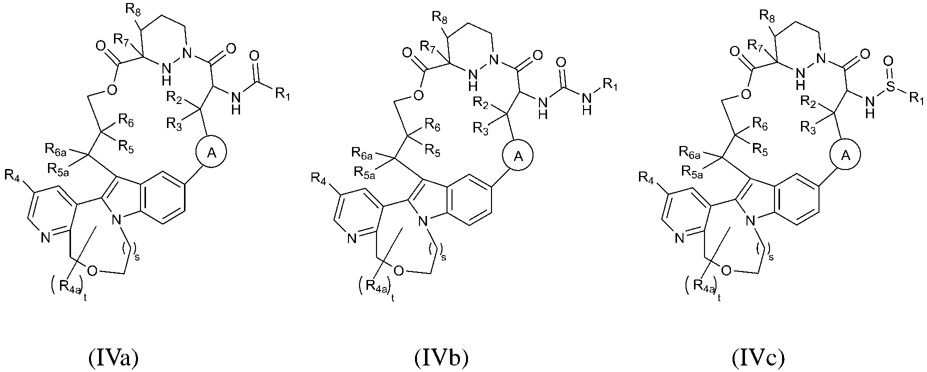 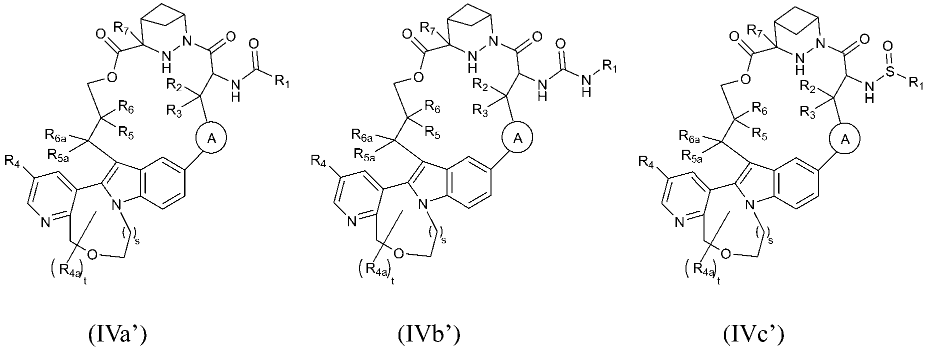 wherein, R₁, R₂, R₃, R₄, R₅, R₆, R₇, R₈, R₄ₐ, R₅ₐ, R₆ₐ, ring A, s, t are as defined in any one of claims 1 to 16.

18. The compound, or the stereoisomer, the tautomer, the deuterated compound or the pharmaceutically acceptable salt thereof according to claim 17, wherein formula (I) is selected from the group consisting of formulas (IVa-1) to (IVc-1) and (IVa-1') to (IVc-1'): 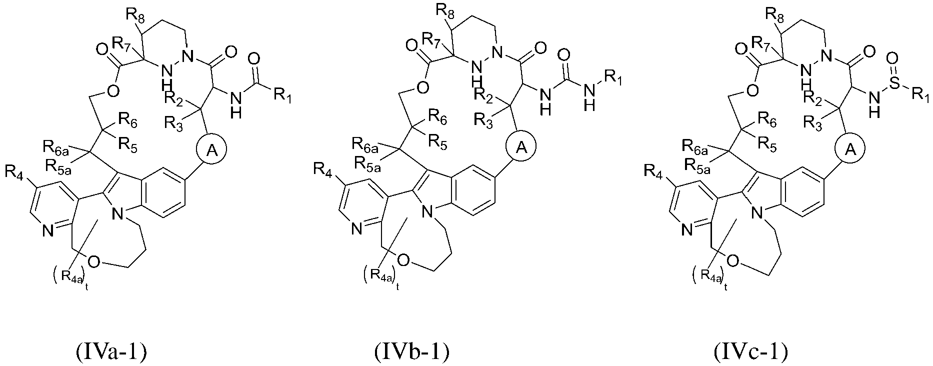 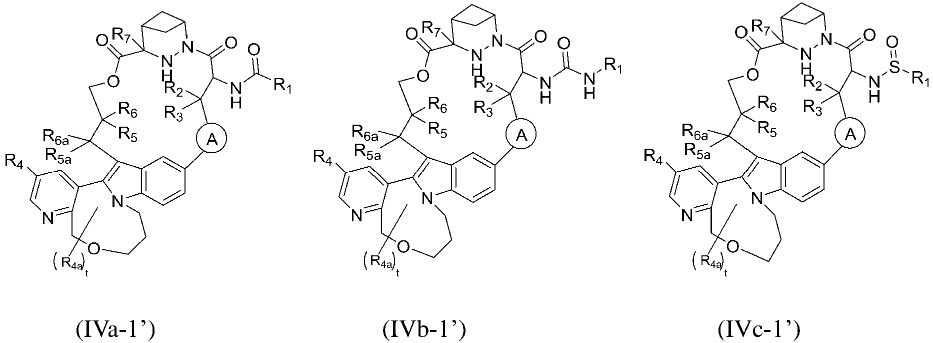 wherein, R₁, R₂, R₃, R₄, R₅, R₆, R₇, R₈, R₄ₐ, R₅ₐ, R₆ₐ, ring A, t are as defined in claim 17.

19. The compound, or the stereoisomer, the tautomer, the deuterated compound or the pharmaceutically acceptable salt thereof according to any one of claims 1 to 6 and 8 to 15, wherein formula (I) is selected from the group consisting of formulas (Va) to (Vc) and formulas (Va') to (Vc'): 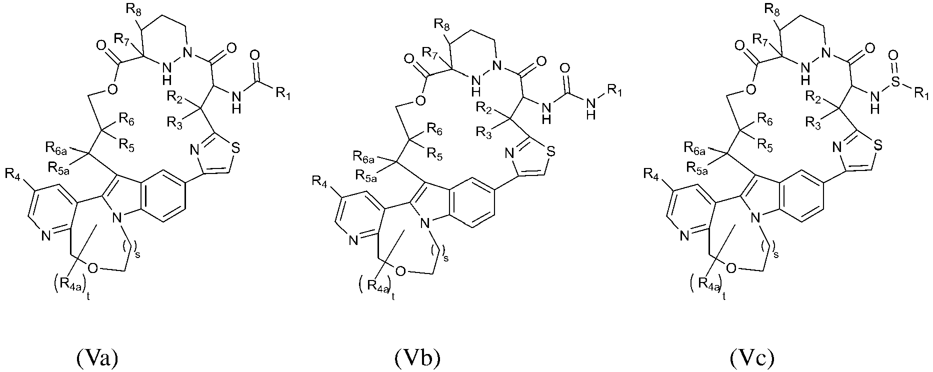 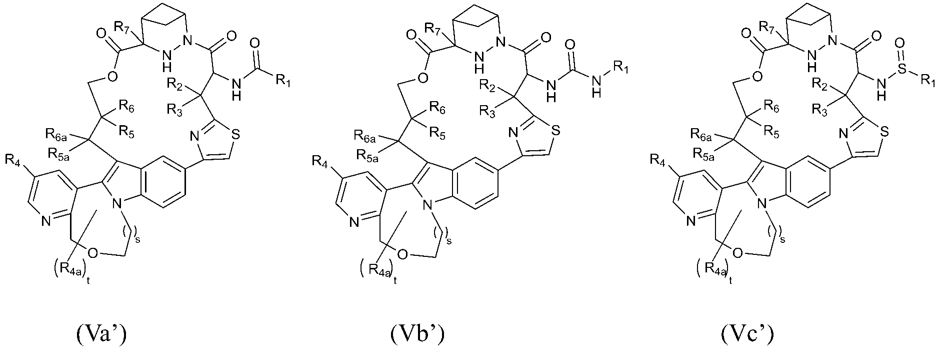 wherein, R₁, R₂, R₃, R₄, R₅, R₆, R₇, R₈, R₄ₐ, R₅ₐ, R₆ₐ, s, t are as defined in any one of claims 1 to 6 and 8 to 15.

20. The compound, or the stereoisomer, the tautomer, the deuterated compound or the pharmaceutically acceptable salt thereof according to claim 19, wherein formula (I) is selected from the group consisting of formulas (Va-1) to (Vc-1) and formulas (Va-1') to (Vc-1'): 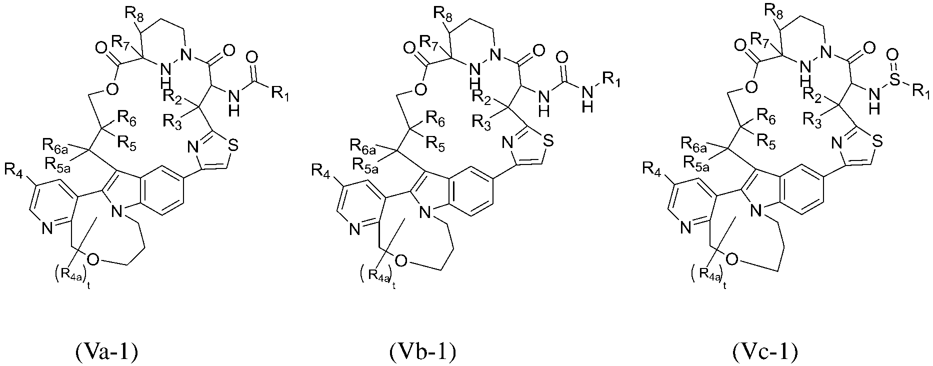 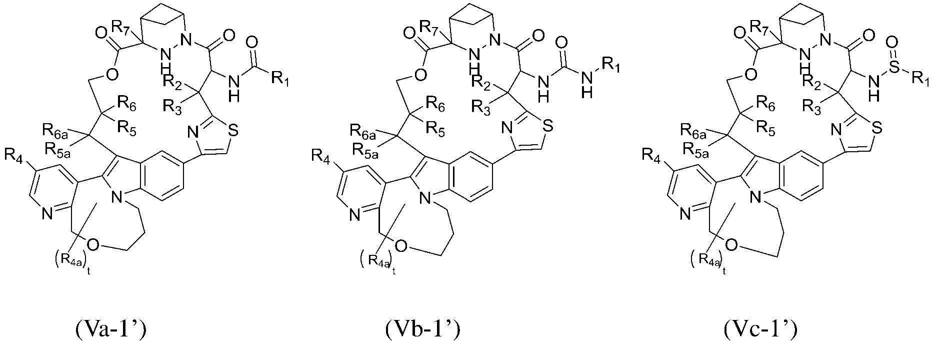 wherein, R₁, R₂, R₃, R₄, R₅, R₆, R₇, R₈, R₄ₐ, R₅ₐ, R₆ₐ, t are as defined in claim 19.

21. The compound, or the stereoisomer, the tautomer, the deuterated compound or the pharmaceutically acceptable salt thereof according to claim 1, wherein formula (I) is selected from the group consisting of formula (VI) and formula (VI'): 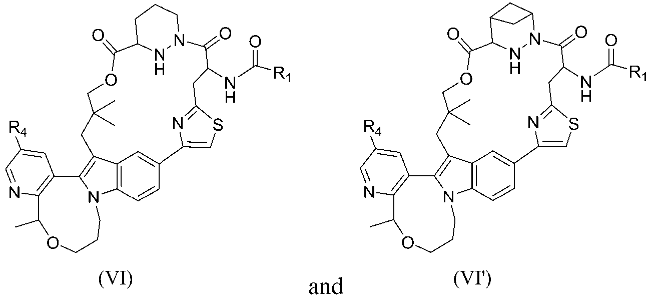 wherein, R₁, R₄ are as defined in claim 1.

22. The compound, or the stereoisomer, the tautomer, the deuterated compound or the pharmaceutically acceptable salt thereof according to any one of claims 1 to 21, wherein R₁ is selected from the group consisting of C₁₋₆ alkyl, -C₀₋₃ alkylene-C₃₋₁₄ cycloalkyl, -C₀₋₃ alkylene-3- to 14-membered heterocyclyl, -C₀₋₃ alkylene-N(R_{c})₂, -C₀₋₃ alkylene-C₂₋₆ alkenyl and -C₀₋₃ alkylene-C₂₋₆ alkynyl; wherein the C₁₋₆ alkyl, -C₀₋₃ alkylene-C₃₋₁₄ cycloalkyl, -C₀₋₃ alkylene-3- to 14-membered heterocyclyl, -C₀₋₃ alkylene-N(R_{c})₂, -C₀₋₃ alkylene-C₂₋₆ alkenyl or -C₀₋₃ alkylene-C₂₋₆ alkynyl is optionally further substituted by one or more Rₐ, each R_{c} is independently selected from the group consisting of H, hydroxyl, halogen, C₁₋₆ alkyl, C₃₋₆ cycloalkyl and C₁₋₆ haloalkyl; wherein Rₐ is selected from the group consisting of halogen, C₁₋₆ alkyl, =C(R_{b})₂, C₁₋₆ haloalkyl, C₁₋₆ alkoxy and -C₀₋₃ alkylene-N(R_{b})₂, R_{b} is selected from the group consisting of H, halogen and C₁₋₆ alkyl; or two Rₐ on the same atom together with the atom to which they are attached form C₃₋₈ cycloalkyl or 3- to 8-membered heterocyclyl.

23. The compound, or the stereoisomer, the tautomer, the deuterated compound or the pharmaceutically acceptable salt thereof according to any one of claims 1 to 22, wherein R₁ is selected from the group consisting of

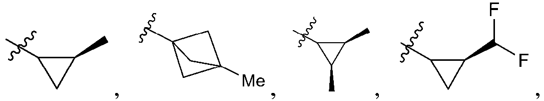

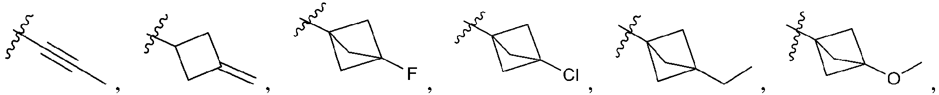

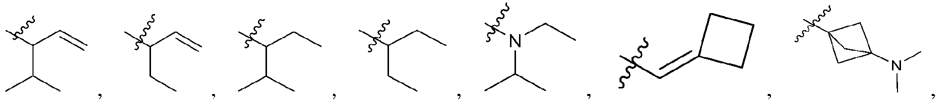

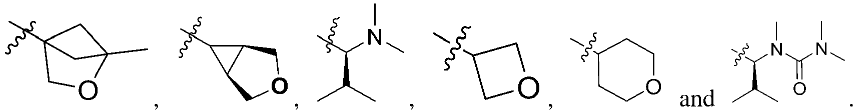

24. The compound, or the stereoisomer, the tautomer, the deuterated compound or the pharmaceutically acceptable salt thereof according to any one of claims 1 to 23, wherein R₂ is H.

25. The compound, or the stereoisomer, the tautomer, the deuterated compound or the pharmaceutically acceptable salt thereof according to any one of claims 1 to 24, wherein R₃ is H.

26. The compound, or the stereoisomer, the tautomer, the deuterated compound or the pharmaceutically acceptable salt thereof according to any one of claims 1 to 25, wherein R₄ is selected from the group consisting of H, C₁₋₆ alkyl, C₁₋₆ alkoxy, -C₀₋₃ alkylene-3- to 14-membered heterocyclyl and -C₀₋₃ alkylene-N(R_{c})₂; wherein the C₁₋₆ alkyl, C₁₋₆ alkoxy, -C₀₋₃ alkylene-3- to 14-membered heterocyclyl or -C₀₋₃ alkylene-N(R_{c})₂ is optionally further substituted by one or more Rₐ, wherein Rₐ is independently selected from the group consisting of absent, H, hydroxyl, oxo, amino, imino, cyano, halogen, C₁₋₆ alkyl, -C₀₋₃ alkylene-N(R_{b})₂, C₁₋₆ haloalkyl, -C₀₋₃ alkylene-C₃₋₁₄ cycloalkyl and -C₀₋₃ alkylene-3- to 14-membered heterocyclyl; wherein the amino, imino, C₁₋₆ alkyl, -C₀₋₃ alkylene-, C₁₋₆ haloalkyl or -C₀₋₃ alkylene-3- to 14-membered heterocyclyl is optionally further substituted by one or more R_{b}, wherein each R_{b} is independently selected from the group consisting of H, halogen, hydroxyl, cyano, C₁₋₆ alkyl, C₁₋₆ alkoxy, C₃₋₆ cycloalkyl and C₁₋₆ haloalkyl.

27. The compound, or the stereoisomer, the tautomer, the deuterated compound or the pharmaceutically acceptable salt thereof according to any one of claims 1 to 26, wherein R₄ is selected from the group consisting of

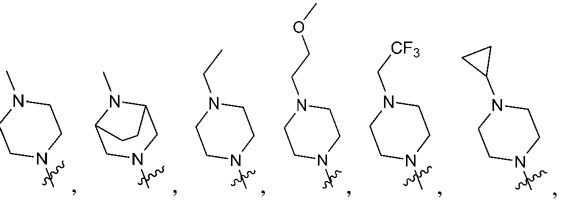

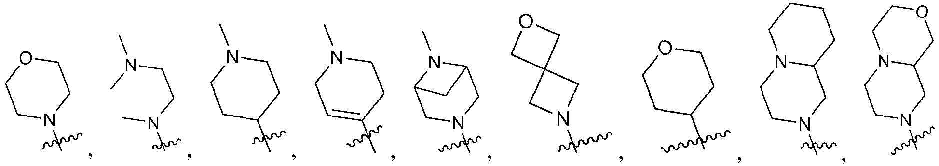

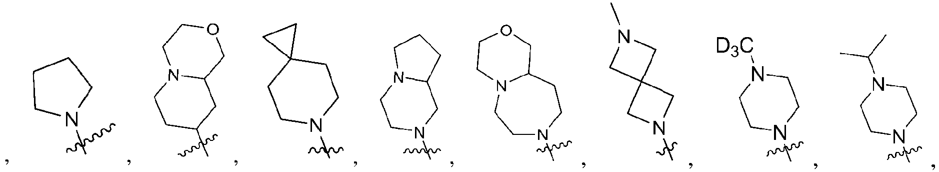

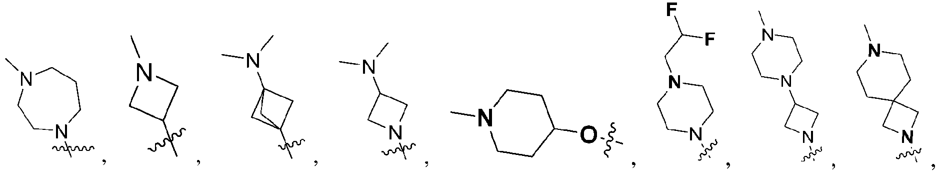

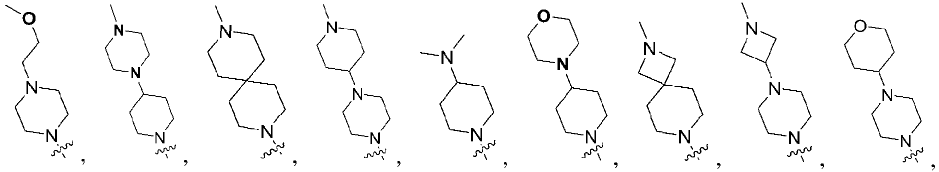

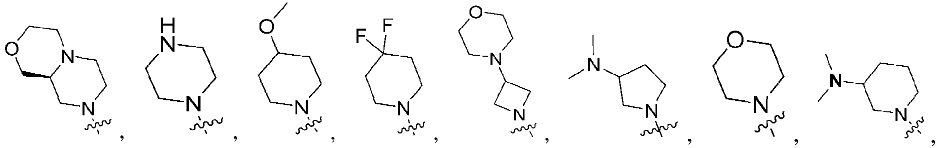

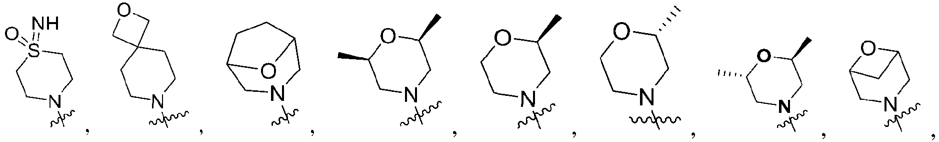

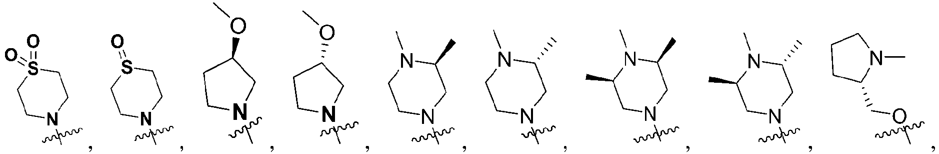

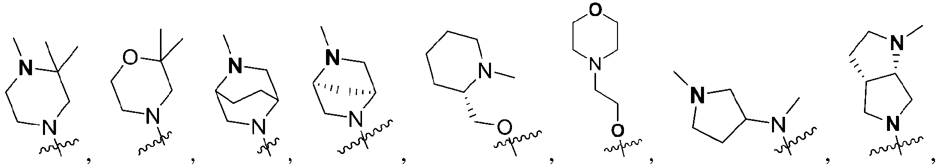

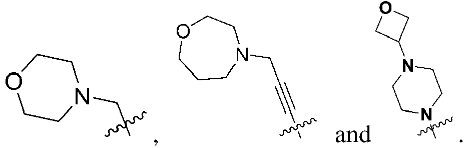

28. The compound, or the stereoisomer, the tautomer, the deuterated compound or the pharmaceutically acceptable salt thereof according to any one of claims 1 to 27, wherein R₄ₐ is selected from the group consisting of H and C₁₋₆ alkyl.

29. The compound, or the stereoisomer, the tautomer, the deuterated compound or the pharmaceutically acceptable salt thereof according to any one of claims 1 to 28, wherein R₅ is selected from the group consisting of H and C₁₋₃ alkyl.

30. The compound, or the stereoisomer, the tautomer, the deuterated compound or the pharmaceutically acceptable salt thereof according to any one of claims 1 to 29, wherein R₆ is selected from the group consisting of H and C₁₋₃ alkyl.

31. The compound, or the stereoisomer, the tautomer, the deuterated compound or the pharmaceutically acceptable salt thereof according to any one of claims 1 to 30, wherein R₅ₐ is selected from the group consisting of H and C₁₋₃ alkyl.

32. The compound, or the stereoisomer, the tautomer, the deuterated compound or the pharmaceutically acceptable salt thereof according to any one of claims 1 to 31, wherein R₆ₐ is selected from the group consisting of H and C₁₋₃ alkyl.

33. The compound, or the stereoisomer, the tautomer, the deuterated compound or the pharmaceutically acceptable salt thereof according to any one of claims 1 to 32, wherein R₇ is selected from the group consisting of H and C₁₋₃ alkyl, preferably H.

34. The compound, or the stereoisomer, the tautomer, the deuterated compound or the pharmaceutically acceptable salt thereof according to any one of claims 1 to 33, wherein R₈ is selected from the group consisting of H and C₁₋₃ alkyl, preferably H.

35. The compound, or the stereoisomer, the tautomer, the deuterated compound or the pharmaceutically acceptable salt thereof according to any one of claims 1 to 18 and 22 to 34, wherein ring A is selected from the group consisting of 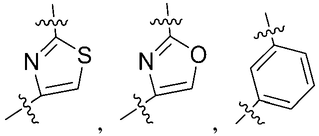 and 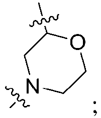 wherein these groups are optionally further substituted by one or more Rₐ; or two Rₐ on the same atom together with the atom to which they are attached form C₃₋₈ cycloalkyl or 3- to 8-membered heterocyclyl, wherein the C₃₋₈ cycloalkyl or 3- to 8-membered heterocyclyl is optionally further substituted by one or more R_{b}.

36. The compound, or the stereoisomer, the tautomer, the deuterated compound or the pharmaceutically acceptable salt thereof according to any one of claims 1 to 35, the compound represented by formula (I) is selected from the group consisting of:

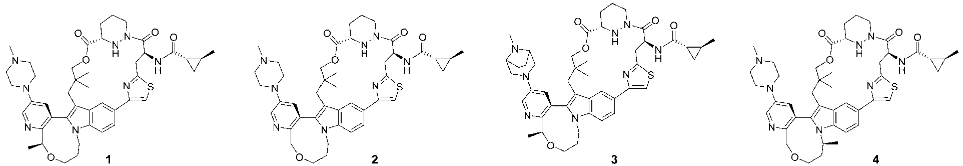

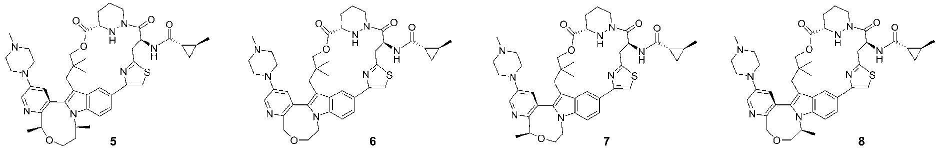

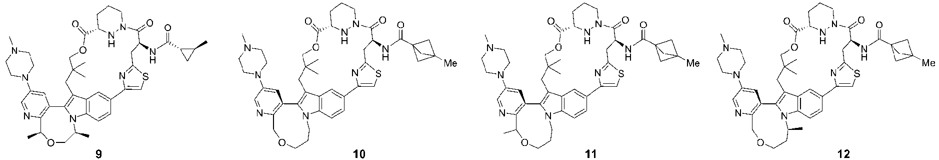

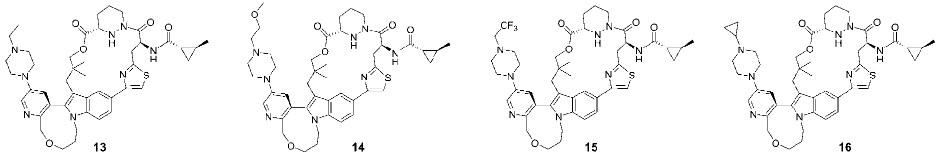

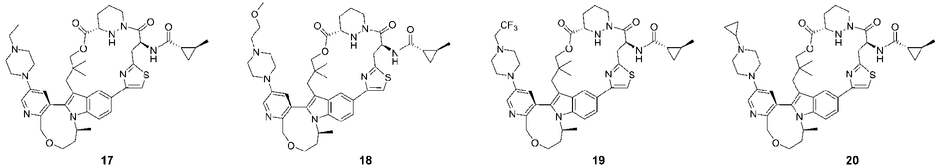

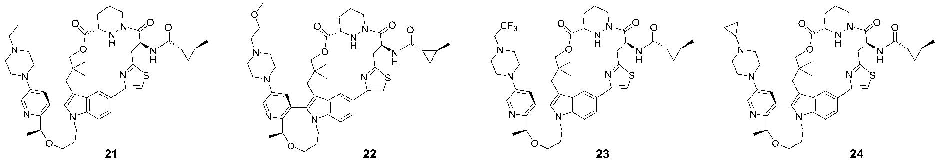

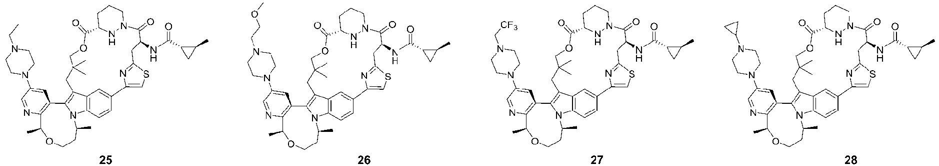

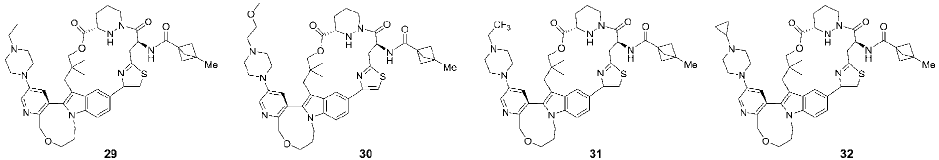

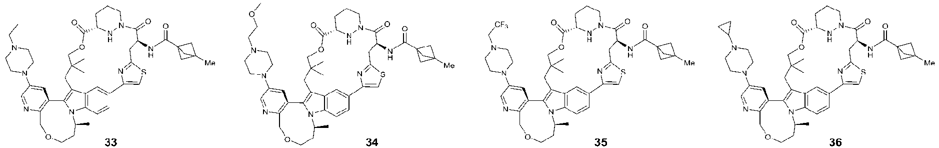

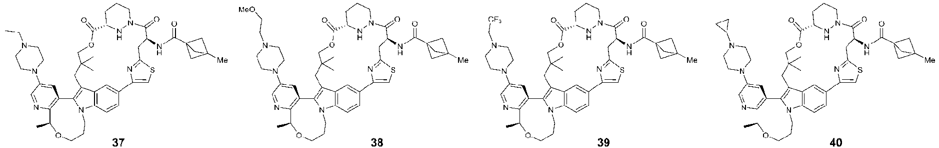

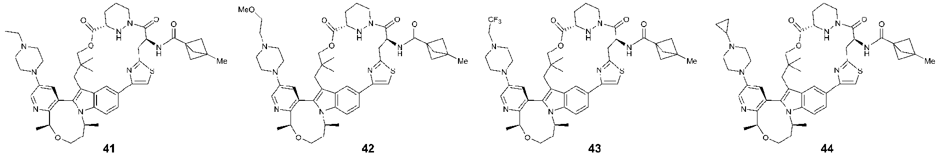

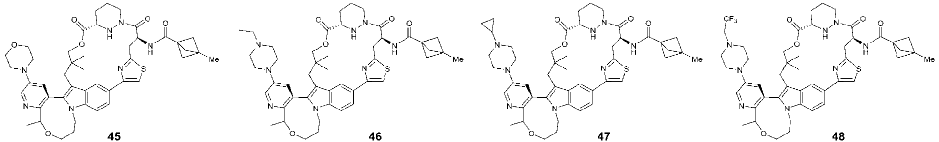

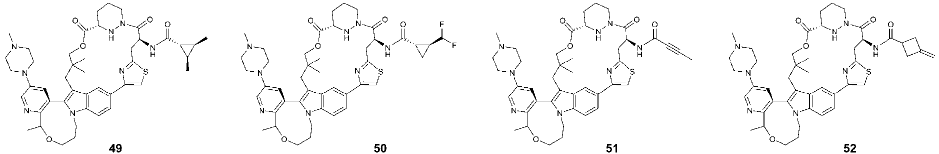

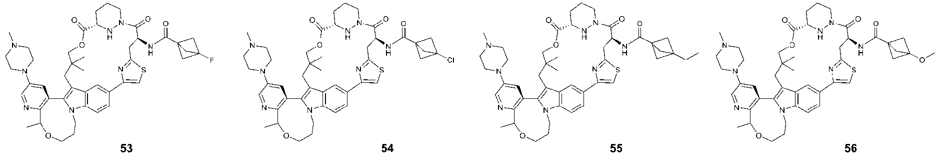

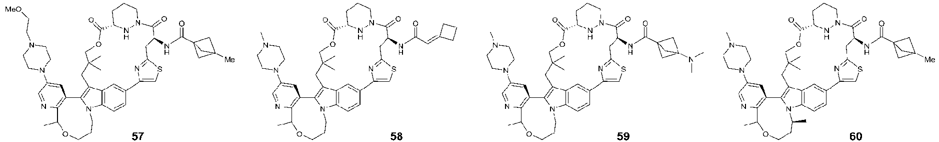

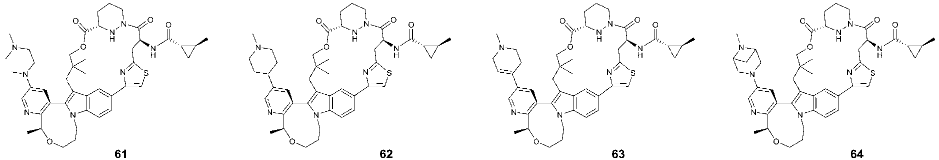

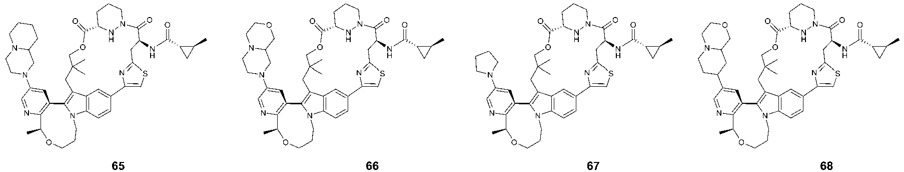

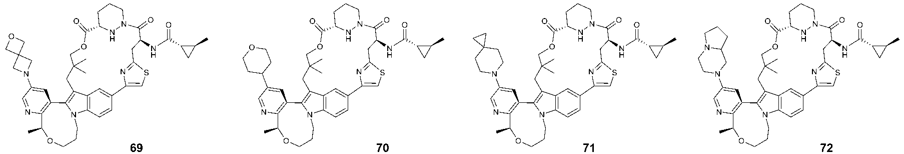

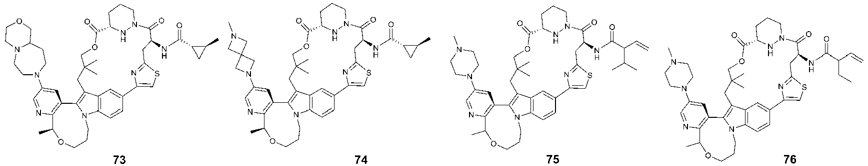

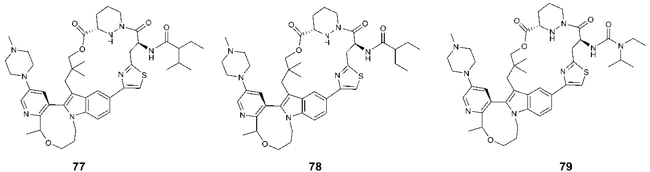

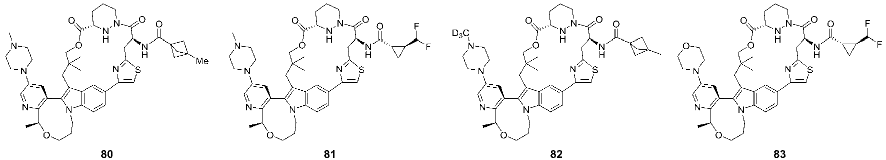

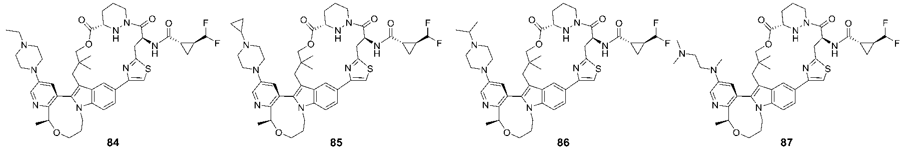

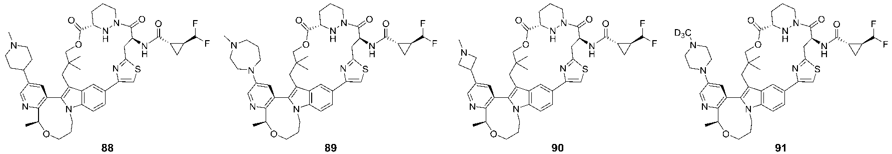

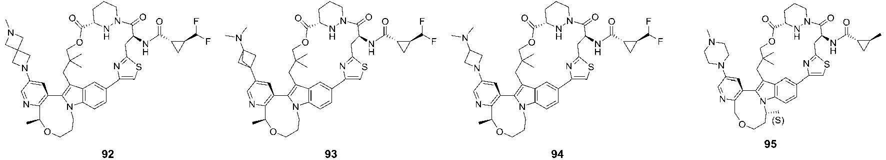

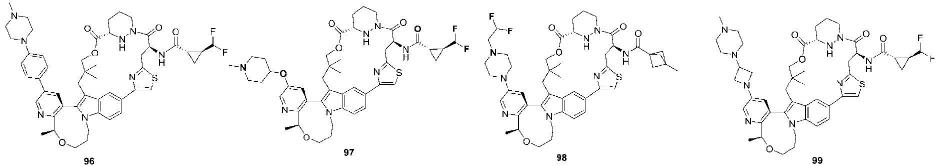

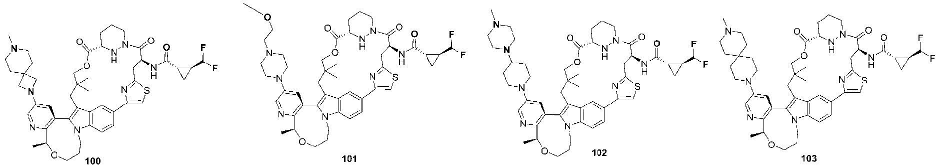

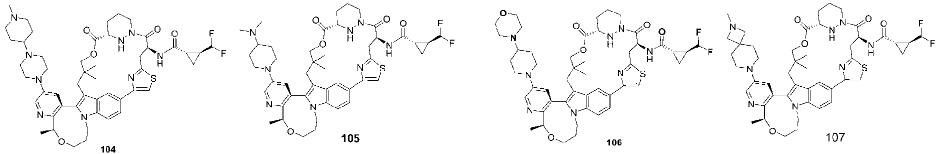

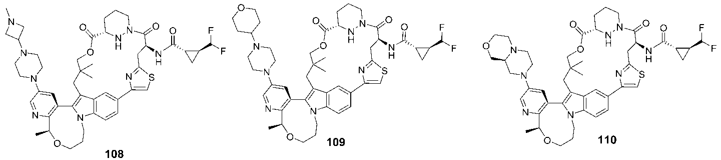

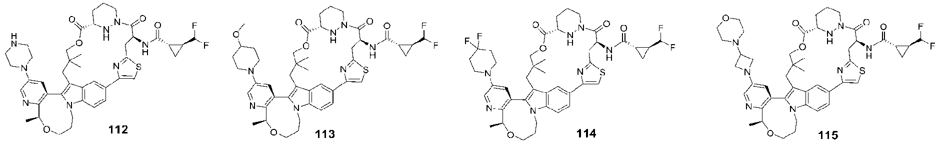

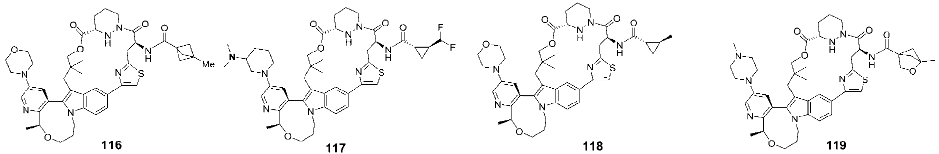

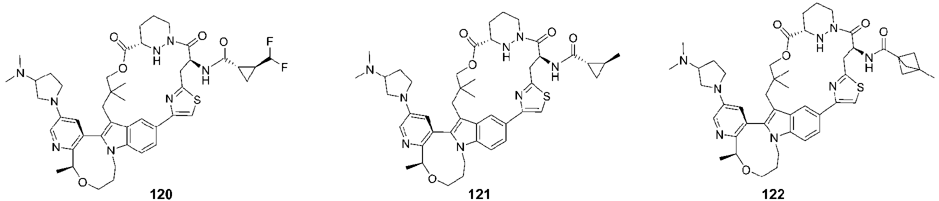

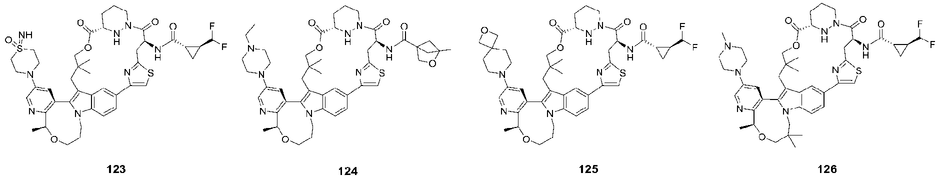

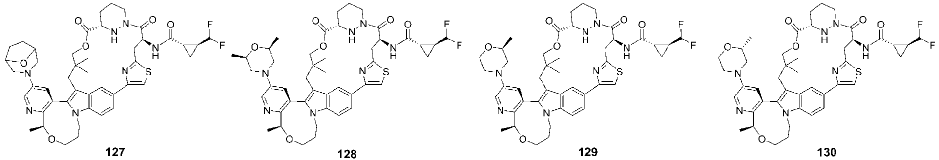

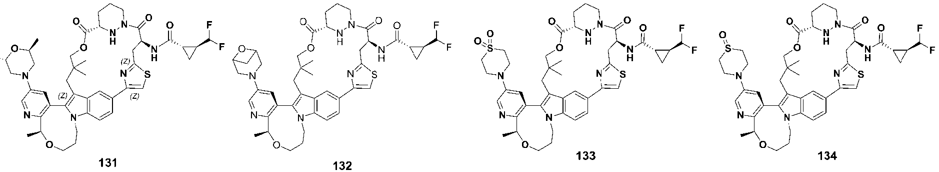

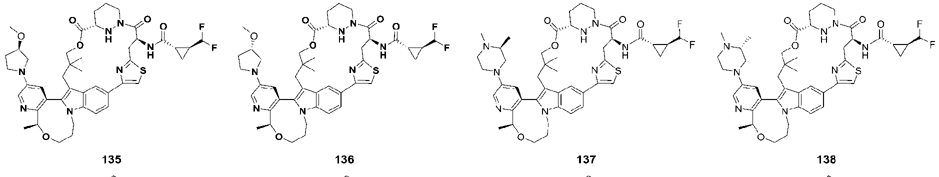

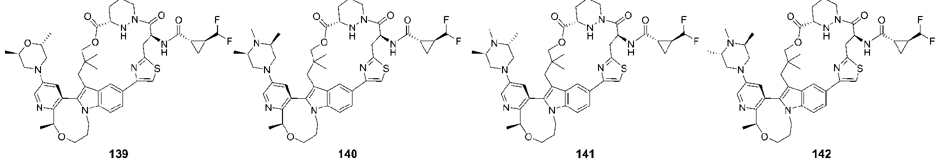

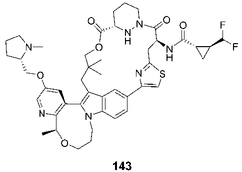

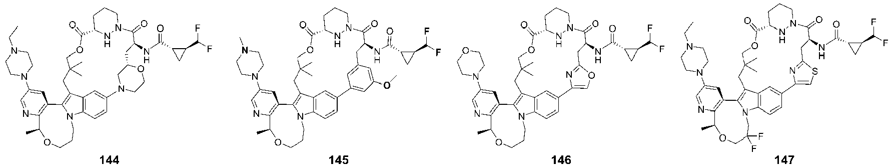

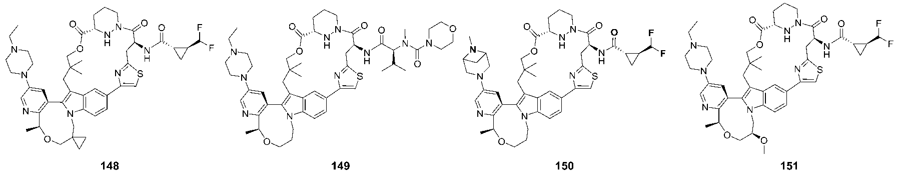

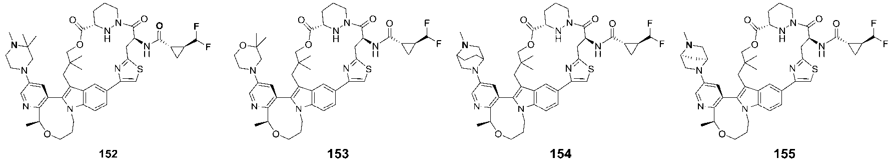

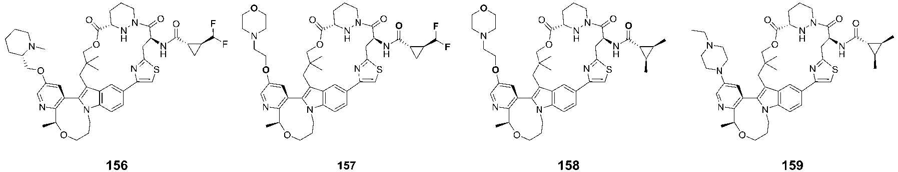

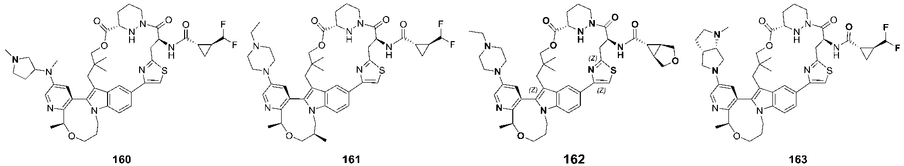

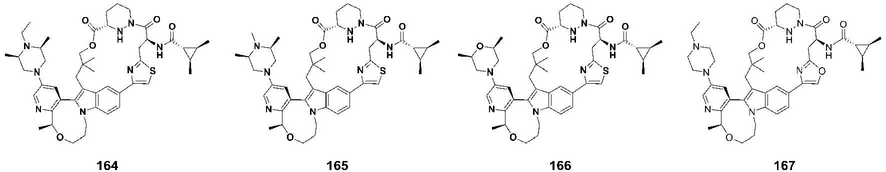

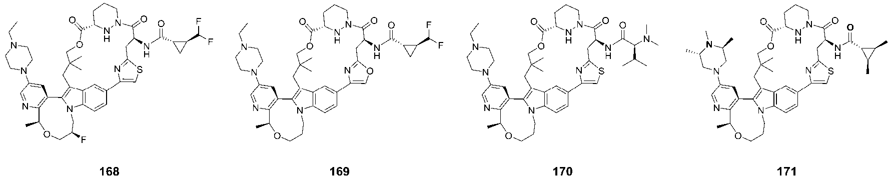

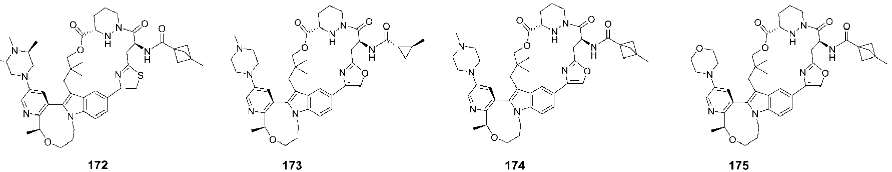

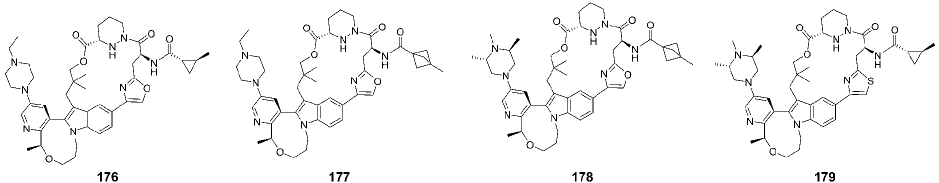

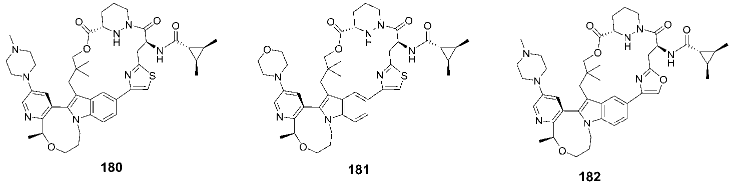

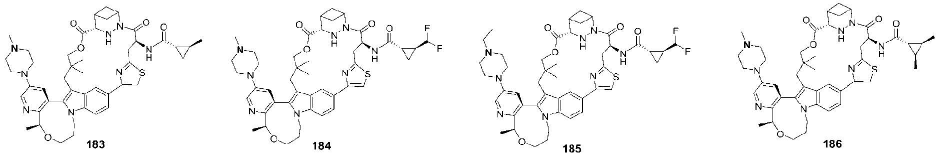

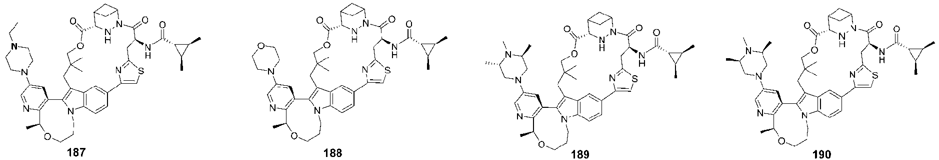

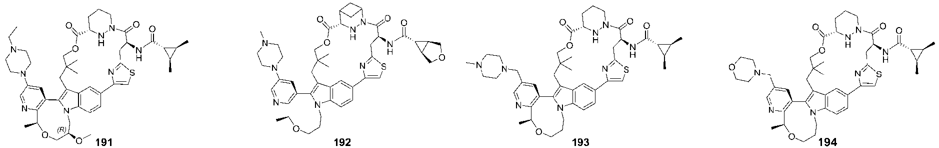

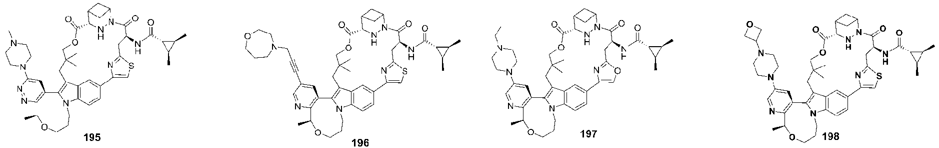

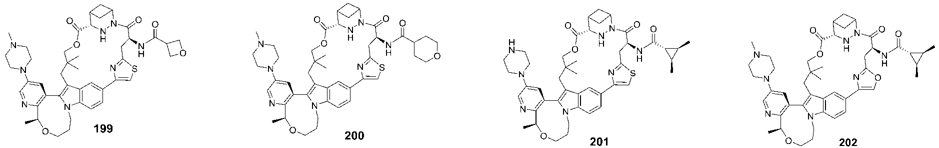

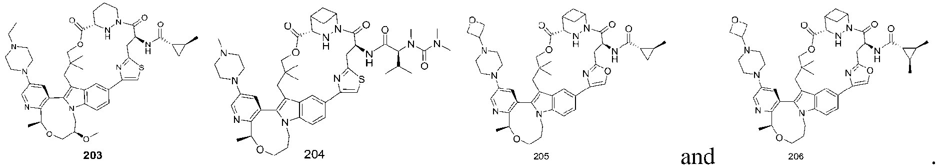

37. A pharmaceutical composition, comprising a therapeutically effective amount of the compound, or the stereoisomer, the tautomer, the deuterated compound or the pharmaceutically acceptable salt thereof according to any one of claims 1 to 36.

38. Use of the compound, or the stereoisomer, the tautomer, the deuterated compound or the pharmaceutically acceptable salt thereof according to any one of claims 1 to 36, or the pharmaceutical composition according to claim 37 in the manufacture of a medicament for treating a KRAS-mediated disease.
